# EUROPEAN PATENT APPLICATION

(11) **EP 1 975 253 A1**
(43) Date of publication of application: **01.10.2008**
(21) Application number: 07290388.3
(22) Date of filing: 30.03.2007
(51) Int. Cl.: C12Q 1/68

(54) **Method of discrimination of the species of the enterobacteriaceae family by genotyping and its applications**

(71) Applicant: INSTITUT PASTEUR, 75015 Paris (FR)
(72) Inventor: Deletoile, Alexis, 95130 Franconville (FR); Brisse, Sylvain, 92140 Clamart (FR); Grimont, Patrick, 75015 Paris (FR)
(74) Representative: Vialle-Presles, Marie José

(57) **Abstract**

Said method comprises for detecting specifically one bacterium among at least 148 species of the 171 species of the *Enterobacteriaceae* family listed in figure 1 and in the herewith attached sequence listing:
A- the isolation of at least a sequence, or a fragment thereof, comprising a target region having a length comprised between 300 and 650 bp and selected among internal portions of a set of genes selected in the group consisting of *gyrB, rpoB, fusA, leus, rplB* and *pyrG* of *Enterobacteriaceae,* or a fragment of said target region of at least 15 bp, preferably of 40-150 bp and
B- determining the species of said bacterium.

## Description

The invention relates to a method of accurate discrimination of the species of the *Enterobacteriaceae* family by genotyping and its applications.

The *Enterobacteriaceae* family contains 186 validly-described species and subspecies, considered validly-published according to the latest version of the Bergey's manual (2), many of which are human pathogens and/or pathogens of animals or plants of economic importance. *Enterobacteriaceae* are Gram-negative bacillus, oxidase-negative, motile or non-motile depending on species, facultative anaerobic bacteria, which almost all grow on common agar, ferment glucose and reduce nitrates in nitrites (2). This family contains a very heterogeneous variety of species that are very miscellaneous in terms of biochemical characters, ecology and genetic. *Enterobacteriaceae* are ubiquist, as members of the family are found in many biotopes and have been isolated from many different sources (from soil and water to animals, insects and plants). Nevertheless, distribution of particular species is very variable *(e.g., Salmonella enterica* subsp. *enterica* serotype Typhi is restricted to humans, *Escherichia coli* is found in the digestive tract of warm-blooded vertebrates, and *Klebsiella pneumoniae* is a real ubiquist found in animals, plants, water and soil).

Pathogenicity of *Enterobacteriacae* is also very different according to species and even sometimes according to strains within a species. Human diseases range from diarrhoea to plague. This family includes highly dangerous pathogens (e.g. *Yersinia pestis, Salmonella* serotype Typhi, *Shigella);* therefore, this family is of major clinical, veterinary, industrial and economic importance. It is responsible of approximately 50% of nosocomial infections: 43% of nosocomial infection in France in 2001 (13), and the annual cost of illness due to *Escherichia coli* 0157 in US was $405 million in 2003 (6). The most frequently isolated species in hospital infections are *Escherichia coli, Klebsiella pneumoniae, Klebsiella oxytoca, Enterobacter cloacae, Enterobacter aerogenes, Citrobacter freundii, Morganella, Hafnia alvei* and *some Serratia.*

More precisely, among *Enterobacteriaceae,* there are strict pathogens in the family *Enterobacteriaceae,* for example (1) *Yersinia pestis,* the causative agent of plague, (2) species belonging to the genus *Shigella,* including *Shigella dysenteriae.* Some particular clones or serotypes of several species are also strict pathogens, such as *Salmonella enterica* subsp. *enterica* serotype Typhi, the causative agent of typhoid fever, or *Escherichia coli* O157:H7, which is responsible for food-borne epidemics of severe hemolytic-uremic syndrome.

Some members of the family *Enterobacteriaceae* are pathogens of animals (for example bovine, ovine, horses). For example, *Klebsiella pneumoniae* causes a venereal disease which can lead to abortion in mares, a serious cause of concern in the race horse breeding and reproduction.

Some members of the family *Enterobacteriaceae* are pathogens of economically important plants, for example species belonging to genera *Erwinia, Pectobacterium* and *Pantoea.*

Finally, some *Enterobacteriaceae* species can have applications in pest biocontrol. For example, *Rahnella aquatilis* can be used to control pathogenic fungi on apple trees. Members of *Photorhabdus* and *Xenorhabdus* are used as biological insecticides in agriculture.

Hereafter, species and subspecies will be referred to as species.

Rapid and precise identification of members of this family is needed in many settings (hospitals, medical laboratories, food industry, veterinary practice, pharma-production, etc).

Currently, species of this family are routinely identified by phenotypic tests (implemented using biochemical strips or manual tests) but these methods are slow and imprecise, and often fail to yield correct identification, especially for rare or emerging strains/species.

To obtain a comprehensive view of the diversity of this family and be able to quickly discriminate them, DNA sequence comparisons and phylogenetic approaches are needed.

Molecular identification intends to exploit nucleotide sequence variation at the genomic level to deduce the species to which a strain most probably belongs.

The 16S rDNA gene sequence is most often chosen for identification, but this gene lacks resolution, making it imprecise or unreliable: some pairs of distinct species share exactly the same sequence or have a very similar sequence. As regards the *Enterobacteriaceae* family, the first phylogenetic studies were based on 16S rRNA (5), because this marker is highly conserved, making it easy to derive broad-range PCR primers for DNA amplification and sequencing. However, the low evolution rate of 16S rRNA makes this gene not informative enough for closely-related species.

Therefore, other markers that evolve more quickly, such as protein coding genes, are needed for precise characterization of strains and identification of closely-related species.

Several protein-coding genes have been explored for their potential to discriminate species of the *Enterobacteriaceae.* However, most studies have explored only one or two genes on a limited number of species. This is mainly due to the difficulty to design universal primers for the family. Use of a single gene or only two genes can lead, depending on the selected gene, to misidentification or inconclusive results owing to horizontal gene transfer.

The candidate genes which have already been used are, for instance *rpoB* (18), *infB* (11) and *gyrB* (3):
- Mollet, C. et al. (18) have performed a *rpoB* sequence analysis for bacterial identification of 16 species: *Escherichia coli, Shigella dysenteriae, Escherichia fergusonii, Kluyvera ascorbata, Citrobacter freundii, Enterobacter cloacae, Klebsiella pneumoniae, Klebsiella ornithinolytica, Klebsiella oxytoca, Salmonella enterica subsp. Enterica, Salmonella enterica subsp. Arizonae, Serratia marcescens, Hafnia alvei, Yersinia enterocolitica, Providencia stuartii* and *Proteus mirabilis,*
- Dauga, C. (3) has studied the *gyrB* gene for bacterial identification of 23 species: *Citrobacter freundii, Enterobacter aerogenes, Enterobacter cloacae, Hafnia alvei, Klebsiella pneumoniae* Subsp. *pneumoniae, Klebsiella terrigena, Morganella morganii, Pectobacterium carotovorum* Subsp. *Carotovorum, Plesiomonas shigelloides, Proteus mirabilis, Proteus vulgaris, Providencia alcalifaciens, Salmonella enterica* Subsp. *enterica* serotype Typhimurium, *Serratia entomophila, Serratia ficaria, Serratia fonticola, Serratia grimesii, Serratia liquefaciens, Serratia marcescens, Serratia odorifera, Serratia plymuthica, Serratia proteamaculans* and *Serratia rubidaea.*

In most cases, well-defined clusters were observed, generally corresponding to described genera. However, for some genera such as *Klebsiella* or *Enterobacter,* relationships are imprecise and contradictory, and species of these genera do not cluster in a single, genus-specific branch (in phylogenetic terms, these genera are polyphyletic). The effect of homologous recombination could be an explanation of these problems. It has been shown in a highly recombinant genus, *Neisseria,* that the use of a single gene is insufficient to clearly distinguish the species inside this genus; in contrast, the tree based on the sequence of seven concatenated genes shows well separated phylogenetic clusters corresponding to three species (*N. meningitidis, N. gonorrhoeae* and *N. lactamica*) (8).

Currently the two gold standards for species delineation are DNA-DNA hybridization and 16S rRNA that have respectively practical and discrimination limitations. Protein coding gene sequencing can overcome these limitations and display many advantages such as a high discriminatory power; further more it buffers the deleterious effects of homologous recombination. However, the limitation, in the use of such protein coding gene sequencing is that it cannot be expanded to species with no genomic data (e.g. emerging pathogens). Generally, studies are made on closely-related species (8, 9). Only few studies are published for entire family or kingdom (14, 15).

Currently, two studies based on protein coding gene sequencing for *Enterobacteriaceae* have been performed. Wertz *et al.* used five genes but included only 8 species (23). Paradis *et al.* sequence 96 strains representing 78 species but only for two genes (19).

However, these studies are only limited to a small number of species.

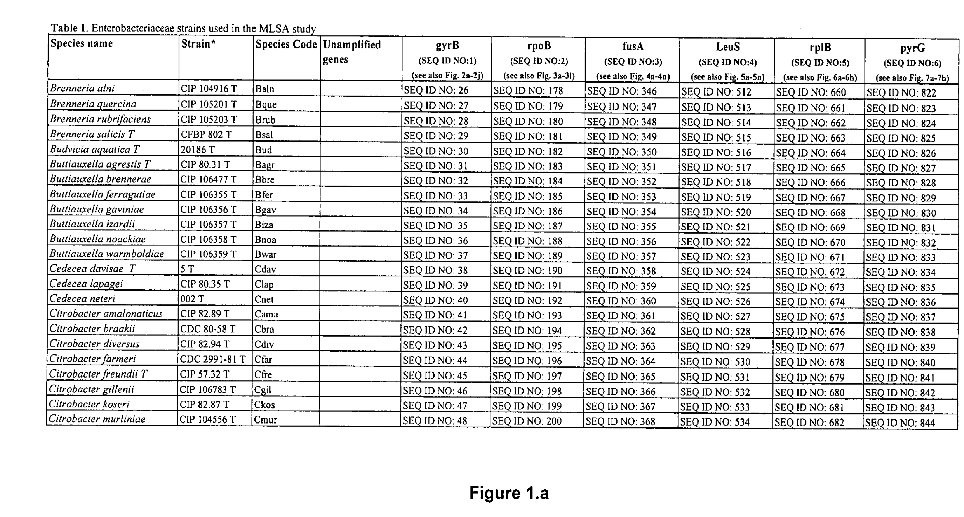

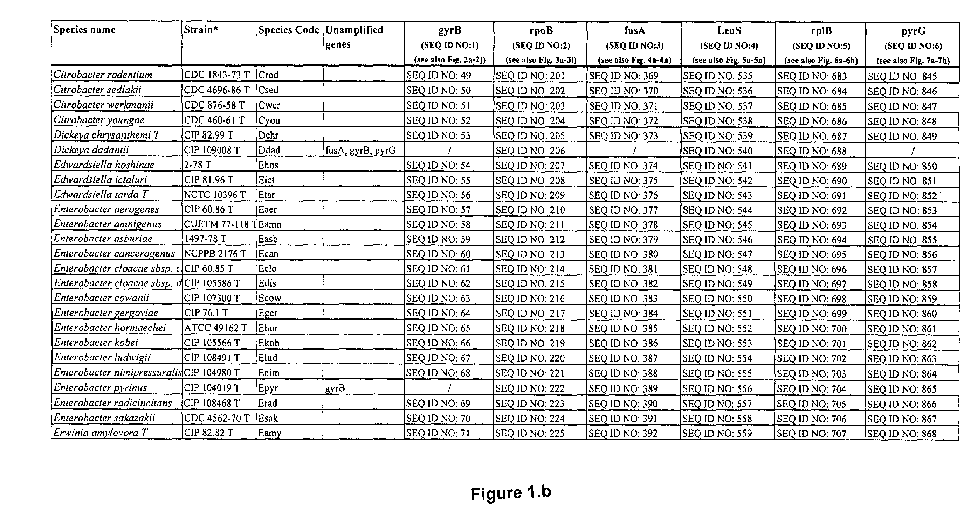

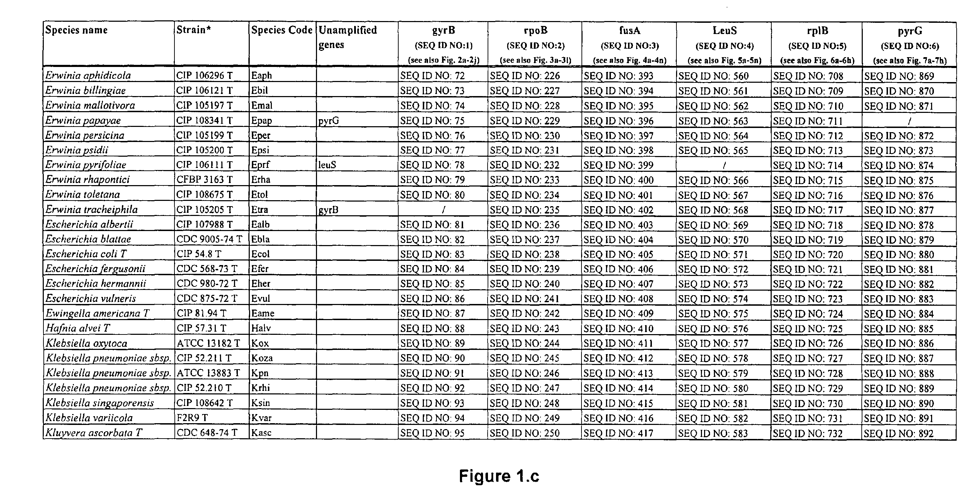

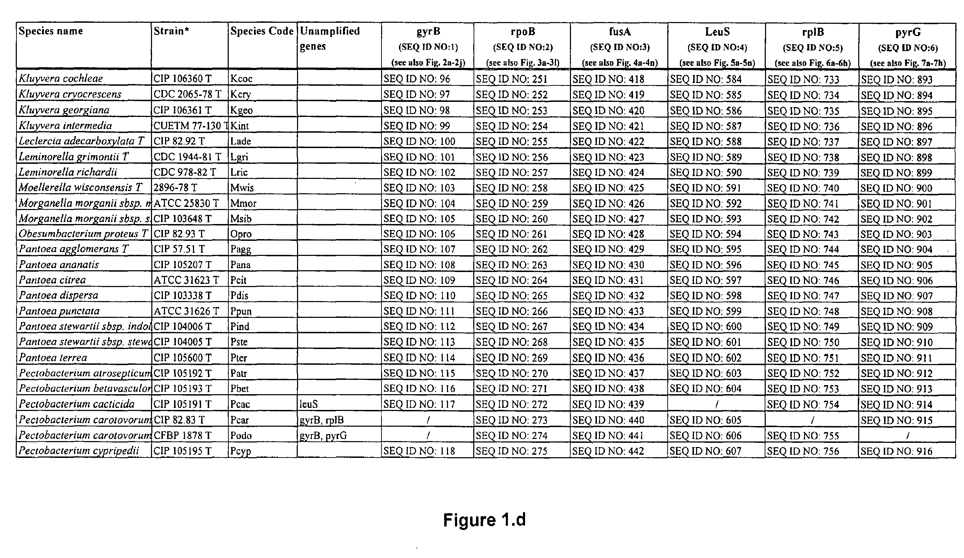

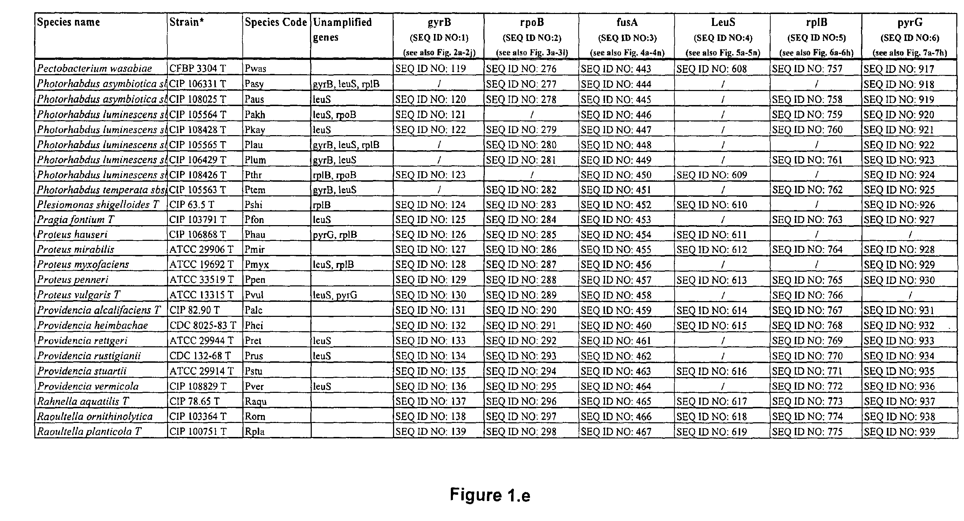

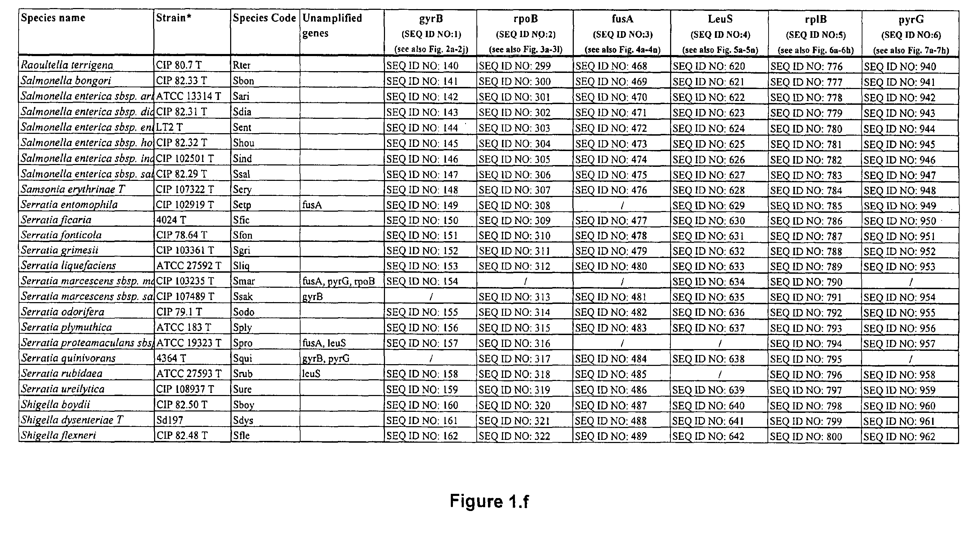

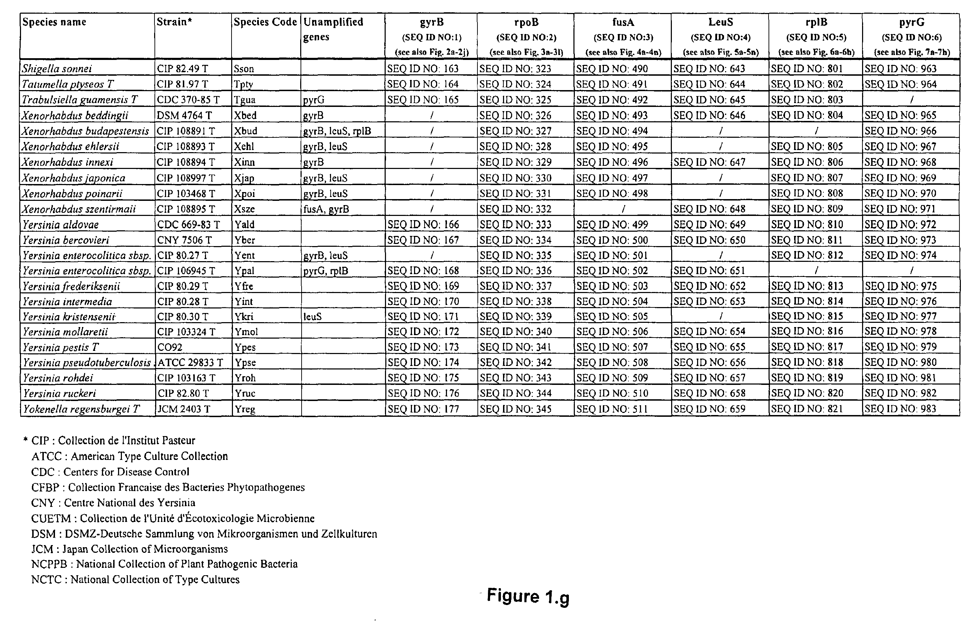

Among the 186 species and subspecies of validly published *Enterobacteriaceae,* the 171 species listed in figure 1 are considered important economically or clinically.

Therefore, there is a need for an efficient and costless method of discriminating, determining and identifying, with accuracy and rapidly one species among the 171 most important species of *Enterobacteriaceae.*

Thus, the aim of the instant invention is to provide such a method.

The Inventors have found that unexpectedly the six genes *fusA, gyrB, leuS, pyrG, rplB* and *rpoB* of *Enterobacteriaceae* improve discrimination, precision, and robustness against gene transfer.

By sequencing internal portions of said six protein-coding genes in the 171 species listed in figure 1 and in the sequence listing, the Inventors have found that the selection of these specific genes and more specifically fragments thereof are unexpectedly sufficient to discriminate all said 171 species of *Enterobacteriaceae.* More precisely, the Inventors obtained reliable sequences of internal portions of the six genes for 130 species, of 5 genes for an additional 19 species, of 4 genes for an additional 17 species and of 3 genes for an additional 5 species (figure 1).

On the 186 species, the data could not be obtained for 11 species: for 9 of them, *Alterococcus agarolyticus, Dickeya dianthicola, Dickeya dieffenbachiae, Dickeya paradisiaca, Dickeya zeae, Saccharobacter fermentatus, Salmonella subterranea, Xenorhabdus nematophila* and *Yersinia aleksiciae,* the type strain was not available; for two of them, *Brenneria nigrifluens* and *Xenorhabdus bovienii,* it was not possible to amplify any gene. In addition, four endosymbiotic species, *Arsenophonus nasoniae, Buchnera aphidicola, Sodalis glossinidus* and *Wigglesworthia glossinida,* were excluded because their sequences are AT rich due to intracellular lifestyle adaptation, and thus introduce a disproportionate number of polymorphisms. However, the excluded strains do not present any major economical or clinical interest.

### Definitions

- "Target region" corresponds to the region of the CDS (coding sequence) of the genes that were determined sufficient for discriminating a species of *Enterobacteriaceae* among said family, alone or in combination with at least another target region or a fragment thereof.
- "Fragment of a target region" corresponds to a fragment of the region of the CDS of the genes, that were determined sufficient for discriminating a species of *Enterobacteriaceae* among said family, alone or in combination with at least (i) another fragment of the same target region, (ii) a second target region different from the target region from which the fragment is derived or (iii) a fragment of said second target region.
- Nucleic acid and DNA are equivalent in the instant invention and encompass both single-stranded and double-stranded nucleic acids.
- The term "label" or "detectable label" refers to any atom or molecule which can be used to provide a detectable (possibly quantifiable) signal, and which can be operatively linked to a nucleic acid. Labels may provide signals detectable by fluorescence, radioactivity, colorimetry, gravimetry, X-ray diffraction or absorption, magnetism, enzymatic activity, mass spectrometry, binding affinity, hybridization radiofrequency, nanocrystals and the like. The target regions or fragments thereof, and the oligonucleotides (probes or primers) of the present invention may be labelled so that the amplification or hybridization reaction product may be "detected" by "detecting" the detectable label.
- A "primer" refers to an oligonucleotide capable of annealing to a complementary nucleic acid template and providing a 3' end to produce an extension product which is complementary to the nucleic acid template. The conditions for initiation and extension usually include the presence of four different deoxyribonucleoside triphosphates and a polymerization-inducing agent such as DNA polymerase or eventually reverse transcriptase, in a suitable buffer ("buffer" includes substituents which are cofactors, or which affect pH, ionic strength, etc.) and at a suitable temperature. Primers may be single- or double-stranded, and preferably single-stranded for maximum efficiency in amplification. "Primers" useful in the present invention are preferably less than or equal to 50 nucleotides in length, e.g., less than or equal to 50, 40, 30, 20, 15, or 10 nucleotides in length.
- A "probe" refers to a nucleic acid molecule having the above characteristics, except that the probe does not have to provide a 3' end for extension. The term "probe" refers to any molecule which is capable of selectively binding to a specifically intended target molecule. Probes can be either synthesized by one skilled in the art, or derived from appropriate biological preparations. For purposes of detection of the target molecule, probes may be specifically designed to be labelled. Examples of molecules that can be utilized as probes include, but are not limited to DNA. Probes useful in the present invention are preferably less that or equal to 150 nucleotides in length, e.g. less that or equal to 140, 130, 120, 110, 100, 90, 80, 70, 60, 50, 40, 30, 20 or 15 nucleotides in length.
- Primers and probes as described above may be also generally referred to as "oligonucleotides" in the present invention.
- Specific hybridization conditions: a nucleic acid molecule "hybridizes" to another nucleic acid molecule when the hybridization occurs under "stringent conditions" as described herein or as understood by those of skill in the art (25).
- "Stringent hybridization conditions" are known to those of ordinary skill in the Art (25; 26); stringent conditions typically include (1) employ low ionic strength and high temperature for washing, for example 0.015 M sodium chloride/0.0015 M sodium citrate/0.1% sodium dodecyl sulphate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) employ 50% formamide, 5xSSC (0.75 M NaCl, 0.075 M sodium citrate), 50 mM sodium phosphate (pH 6.8), 0.1% sodium pyrophosphate, 5x Denhardt's solution, sonicated salmon sperm DNA (50 µg/ml), 0.1% SDS, and 10% dextran sulphate at 42°C, with washes at 42°C in 0.2xSSC (sodium chloride/sodium citrate) and 50% formamide at 55°C, followed by a high-stringency wash consisting of 0.1xSSC containing EDTA at 55°C. For instance gDNA may be resuspended in a hybridization solution containing 40% desionized formamide, 5X Denhardt's solution 50 mM Tris pH 7.4, 0.1% SDS, 1 mM Na pyrophosphate, and 5X SSC, denatured at 95°C for 2 min, and hybridized to the SSC (sodium chloride/sodium citrate) microarray for 16h at 42°C. After hybridization, microarrays are washed twice over a 10 min period at 42°C with wash buffer (2X SSC, 0.1% SDS), and then in 0.2X SSC and in 0.1X SSC, each for 2 min at room temperature.
- The term "plurality" refers to more than one. Plurality, according to the invention, can be 2, 3, 4, 5, 6 or more, 10 or more, 20 or more, 50 or more, 100 or more, or 1000 or more.
- Arrays and microarrays refer to a set of oligonucleotides localized by deposition or synthesis into an array of discrete test zones (spots or registers) on the surface of a solid substrate (or support). Multispot arrays are known by a number of names depending on the oligonucleotides (probes) arrayed or the analytical application (gene chip, DNA chip, DNA array). Spots range in size from a few millimetres to a few micrometres, and a single array can contain several to many thousand spots of different probes. Usually the immobilized molecule is termed probe (or oligonucleotide) while the solution-phase molecule that reacts with the probe is the target. A multispot array may also comprise control samples. In the basic multispot array, the array is exposed to the test sample and specific binding or hybridization occurs between targets (isolated products in the present invention) and the individual probe spots on the array. A label or detection reaction reveals the pattern of binding and provides information on the reactivity and hence the composition of the sample.

Accordingly, a first object of the invention is a method for discriminating accurately a bacterium of the *Enterobacteriaceae* family by genotyping, characterized in that said method comprises, for detecting specifically one bacterium among at least 148 species of the 171 species of the *Enterobacteriaceae* family listed in figure 1:
A- the isolation of at least a sequence or a fragment thereof comprising a target region having a length comprised between 300 and 650 bp and selected among internal portions of a set of genes selected in the group consisting of *gyrB, rpoB, fusA, leuS, rplB* and *pyrG* of *Enterobacteriaceae* or a fragment of said target region of at least 15 bp, preferably of 40-150 bp and
B- determining the species of said bacterium.

According to a first advantageous embodiment of carrying out said method, step A comprises, for detecting specifically one bacterium among said 148 species, the isolation of a plurality of sequences, or fragments thereof, comprising target regions having a length comprised between 300 and 650 bp, said target regions being selected among internal portions of a set of at least two genes selected in the group consisting of *gyrB* and *leuS* of *Enterobacteriaceae.*

According to said first advantageous embodiment of carrying out said method, step A comprises for detecting specifically one bacterium among the said 171 main species of the *Enterobacteriaceae* family listed in figure 1, the further isolation of a plurality of sequences, or fragments thereof, comprising target regions having a length comprised between 300 and 650 bp, said target regions being selected among at least one further internal portion of a gene selected in the group consisting of *rpoB, fusA, rplB* and *pyrG* of *Enterobacteriaceae.*

According to a second advantageous embodiment of carrying out said method, step A comprises, for detecting specifically one bacterium among said 171 main species of *Enterobacteriaceae* listed in figure 1, the isolation of a plurality of sequences, or fragments thereof, comprising target regions having a length comprised between 300 and 650 bp, said target regions being selected among internal portions of *leuS* and *rplB* genes of *Enterobacteriaceae* and a set of at least two genes selected in the group consisting of *fusA* and *pyrG* of *Enterobacteriaceae.*

According to a third advantageous embodiment of carrying out said method, said target regions are selected in the group consisting of:
* the target region of *gyrB* corresponding to a fragment of 417-420 bp of *gyrB* CDS (consensus sequence SEQ ID NO:1 and SEQ ID NO: 26-177) corresponding, in reference to *E. coli* K12 genome (NCBI accession number NC_000913) to the predicted positions 3,877,887 to 3,877,471 (Reverse Complement) included in the CDS corresponding to the predicted positions 256 to 672 of *gyrB* CDS (3,875,728-3,878,142).
* the target region of *rpoB* corresponding to a fragment of 501 bp of *rpoB* CDS (consensus sequence SEQ ID NO:2 and SEQ ID NO:178-345) corresponding, in reference to *E*. *coli* K12 (NCBI accession number NC_000913), to the predicted positions 4,180,924 to 4,181,424 (Reverse Complement) included in the CDS corresponding to the predicted positions 1657 to 2157 of *rpoB* CDS (4,179,268-4,183,296).
* the target region of *fusA* corresponding to a fragment of 633 bp of *fusA* CDS (consensus sequence SEQ ID NO:3 and SEQ ID NO:346-511) corresponding, in reference to *E. coli* K12 genome (NCBI accession number NC_000913) to the predicted positions 3,471,434 to 3,470,802 (Reverse complement) included in the CDS corresponding to the predicted positions 103 to 735 of *fusA* CDS (3469422-3471536).
* the target region of *leuS* corresponding to a fragment of 642 bp of *leuS* CDS (consensus sequence SEQ ID NO:4 and SEQ ID NO:512-659), corresponding, in reference to *E*. *coli* K12 genome (NCBI accession number NC_000913) to the positions 673,439 to 672,798 (Reverse Complement) included in the CDS corresponding to the predicted positions 568 to 1209 of *leus* CDS (671,424-674,006).
* the target region of *rplB* corresponding to a fragment of 333-339 bp of *rplB* CDS (consensus sequence SEQ ID NO:5 and SEQ ID NO:660-821) corresponding, in reference to *E*. *coli* K12 genome (NCBI accession number NC_000913), to the positions 3,449,083 to 3,448,751 (Reverse Complement) included in the CDS corresponding to the predicted positions 304 to 636 of *rplB* CDS (3,448,565-3,449,386) and
* the target region of *pyrG* corresponding to a fragment of 306 bp of *pyrG* gene (consensus sequence SEQ ID NO:6 and SEQ ID NO:822-983), corresponding, in reference to *E. coli* K12 genome (NCBI accession number NC_000913), to the positions 2,907,607 to 2,907,302 (Reverse Complement) included in the CDS corresponding to the predicted positions 82 to 387 of *pyrG* CDS (2,906,051-2,907,688).

According to a fourth advantageous embodiment of carrying out said method, said fragments of said target regions are selected in the group consisting of fragments of said target regions of at least 15 bp, preferably of 40-150 bp.

Preferably, said fragments of said target regions are selected in the group consisting of:
- a fragment of 40 bp defined by the positions 336 to 375 of *gyrB* sequences SEQ ID NO:1 or SEQ ID NO:26-177.
- a fragment 40 bp defined by the positions 416 to 455 of *rpoB* sequences SEQ ID NO:2 or SEQ ID NO:178-345.
- a fragment of 40 bp defined by the positions 480 to 519 of *fusA* sequences of SEQ ID NO:3 or SEQ ID NO:346-511.
- a fragment of 40 bp defined by the positions 336 to 375 of *leus* sequences of SEQ ID NO:4 or SEQ ID NO:512-659 and
- a fragment of 40 bp defined by the positions 194 to 233 of *pyrG* sequences of SEQ ID NO:6 or SEQ ID NO:822-983.

Unexpectedly, the selection of said target regions or fragments thereof and specific combinations thereof allow effectively the specific detection of one bacterium among said at least 148 species and preferably among said 171 species of the *Enterobacteriaceae* family listed in figure 1 and in the here with attaché sequence listing. For instance, the different sets listed bellow may advantageously be selected:
- **Set N°1** (for detecting at least 148 species of the 171 listed in figure 1 and in the here with attached sequence listing) consisting of target regions as defined above of any of the *gyrB, rpoB, fusA, leuS, rplB* and *pyrG* CDS,
- **Set N°2** (for detecting at least 148 species of the 171 listed in figure 1 and in the here with attached sequence listing) consisting of the combination of target regions as defined above of the couple *gyrB* and *rpoB,*
- **Set N°3** (for detecting the 171 species listed in figure 1 and in the here with attached sequence listing) consisting of the combination of target regions as defined above of the triplet *leuS, rplB* and *fusA,*
- **Set N°4** (for detecting the 171 species listed in figure 1 and in the here with attached sequence listing) consisting of the combination of target regions of the triplet *leuS, rplB* and *pyrG,*
- **Set N° 5** (for detecting the 171 species listed in figure 1 and in the here with attached sequence listing) consisting of the combination of target regions of the quadruplet *leuS, rpl*B*, pyrG* and *fusA,*
- **Set N°6** (for detecting the 171 species listed in figure 1 and in the here with attached sequence listing) consisting of the combination of target regions of the sextuplet *gyrB, rpoB, fusA, leuS, rplB* and *pyrG* or fragments thereof of 40-150 bp,
- **Set N°7** (for detecting the 171 species listed in figure 1 and in the here with attached sequence listing) consisting of combination of the fragments of 40 bp of the target regions of the quintuplet *gyrB, rpoB, fusA, leuS,* and *pyrG,* said fragments of 40 bp corresponding to the following positions: *gyrB:* from positions 336 to 375 of SEQ ID NO:1 and SEQ ID NO:26-177, *rpoB* from positions 416 to 455 of SEQ ID NO:2 and SEQ ID NO:178-345, *fusA* from positions 480 to 519 of SEQ ID NO:3 and SEQ ID NO:346-511, *leuS* from positions 336 to 375 of SEQ ID NO:4 and SEQ ID NO:512-659 and *pyrG* from positions 194 to 233 of SEQ ID NO:6 and SEQ ID NO:822-983.

Unexpectedly:
- The combination of set N°7, i.e. said five fragments of 40 nucleotides of the defined target regions allow distinguishing the 171 species listed in figure 1.

The combinations of the target regions defined in sets N°3-6, i.e. *leuS*/*rplB*/*fusA, leuS*/*rplB*/*pyrG, leuS*/*rplB*/*pyrG*/*fusA* and *gyrB*/*rpoB*/*fusA*/*leuS*/*rplB*/*pyrG* are combinations for which all species are distinguished and each of the 171 species are represented by at least one gene. These target regions constitute good discriminatory markers for effectively discriminating said 171 species of *Enterobacteriaceae.*
- Each one of the target regions defined in sets N°1-2 allows distinguishing 148 species of the 171 listed in figure 1.

Other minimal marker sets may be advantageously selected among said target regions. The rules for selecting said marker sets of discriminatory markers are as follows:

There are different possible methods to select markers and the minimum number of markers (single nucleotide polymorphisms SNPs or oligonucleotides or restriction sites) required to discriminate all species and subspecies or to choose a set of markers that distinguishes one species (or subspecies) from all others.

The analysis performed by the Inventors allowed them to derive specific nucleotide signatures for each species. Based on the invention, oligonucleotide (probes or PCR primers) were deduced to develop identification tests based eg on microarray (DNA chips), real-time PCR or SNP (single Nucleotide Polymorphism) detection technologies known to those of ordinary skill in the art (25, 26, 27, 28, 29, 30, 31).

This is possible on the basis of figures 2-7 in conjunction with figures 10 and 11 in which the % of polymorphic sites is specified.

One possible way is to first calculate the discrimination score (Simpson's Index) of all the combined markers *gyrB, rpoB, fusA, leuS, rplB* and *pyrG.* Then, this discrimination score is calculated for each individual marker. Finally, the last operation consists in combining the markers with the highest score (one being added at a time) until the discrimination score of the combination equals the discrimination score of all the combined markers.

Another method consists in grouping the markers *gyrB, rpoB, fusA, leuS, rplB* and *pyrG* according to the species that they contain, meaning that two markers that are specific for the same species are in the same group. Groups of markers are then selected depending on the species to be distinguished. For example, taken four species A, B, C and D, several markers (markers Group 1) are specific of species A, B and C; other markers (Group 2) are specific of species B, C, and D, and other markers (Group 3) are specific of species A and D. If one chooses one marker in group 1 and one marker in group 3, species A can be distinguished from the all other species just by using these two markers.

Isolation of genomic DNA (gDNA) of the bacterium may be performed by different methods known to those of ordinary skill in the art.

For instance:
- for specific isolation, amplification techniques with specific primers will be used (27).
- a global amplification of the genome may be performed by known methods (whole genome amplification, for instance), optionally followed by a specific amplification.

According to a fifth advantageous embodiment of carrying out said method, step A of isolation comprises:
(A1) Obtaining a test sample that contains genomic nucleic acids from bacteria present in said sample. Examples of the sample include, but are not limited to, tissues, body fluids, water, food, plants, etc...
(A2) Amplifying at least one target region or a fragment thereof, as defined here above, to generate isolated products.

The step (A2) of amplifying said at least one target region, or a fragment thereof, is performed by a method selected from the group consisting of polymerase chain reaction, (PCR), ligase chain reaction (LCR), nucleic acid-sequence-based amplification (NASBA), cycling probe technology (CPT), nested PCR and multiplex PCR; for instance, said multiplex PCR is conducted to partially amplify the DNA sequences of the target regions of said sets, according to methods known by those of skill in the art.

According to said fifth embodiment, the step (A2) of amplifying said at least one target region or a fragment thereof is performed with primers having a length comprised between 10 and 40 nucleotides, preferably of 18-35 nucleotides, said primers, optionally labelled or modified (said primers have U nucleotides instead of T nucleotides), being able to amplify the target region or a fragment thereof.

Preferably, the primers are selected in the group consisting of the primers listed in Table I below.

| Genes | Primers | Primer sequences | Primer positions on CDS |
|---|---|---|---|
| *gyrB* | gyrB3 | 5'-GCG TAA GCG CCC GGG TAT GTA- 3' (SEQ ID NO: 12) | 57 to 77 |
| | gyrB4 | 5'-CCG TCG ACG TCC GCA TCG GTC AT- 3' (SEQ ID NO: 13) | 1486 to 1508 |
| *rpoB* | Vic3 | 5'-GGC GAA ATG GCW GAG AAC CA- 3' (SEQ ID NO: 16) | 1369 to 1388 |
| | Vic2 | 5'-GAG TCT TCG AAG TTG TAA CC- 3' (SEQ ID NO: 17) | 2425 to 2444 |
| *fusA* | fusA3 | 5'-CAT CGG TAT CAG TGC KCA CAT CGA- 3' (SEQ ID NO: 18) | 36 to 59 |
| | fusA4 | 5'-CAG CAT CGC CTG AAC RCC TTT GTT- 3' (SEQ ID NO: 19) | 814 to 837 |
| *leuS* | leuS3 | 5'-CAG ACC GTG CTG GCC AAC GAR CAR GT- 3' (SEQ ID NO: 20) | 487 to 512 |
| | leuS4 | 5'-CGG CGC GCC CCA RTA RCG CT- 3' (SEQ ID NO: 21) | 1274 to 1293 |
| *rplB* | rplB3 | 5'-CAG TTG TTG AAC GTC TTG AGT ACG ATC C- 3' (SEQ ID NO: 22) | 227 to 254 |
| | rplB4 | 5'-CAC CAC CAC CAT GYG GGT GRT C- 3' (SEQ ID NO: 23) | 685 to 706 |
| *pyrG* | pyrG3 | 5'-GGG GTC GTA TCC TCT CTG GGT AAA GG- 3' (SEQ ID NO: 24) | 31 to 56 |
| | pyrG4 | 5'-GGA ACG GCA GGG ATT CGA TAT CNC CKA- 3' (SEQ ID NO: 25) | 434 to 460 |

According to a sixth advantageous embodiment of carrying out said method, the step B of determining the species of said bacterium comprises:
(B1) Analysing the isolated product obtained in step A, in order to discriminate among possible sequences and
(B2) Determining the species of the *Enterobacteriaceae.*

The invention represents the first exhaustive database of reference sequences of multiple genes used for *Enterobacteriaceae* identification and species discrimination.

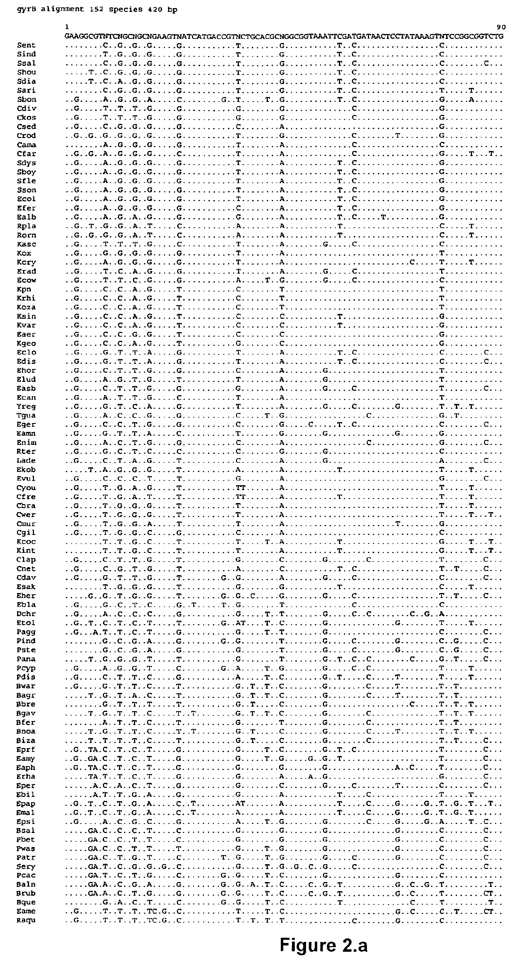

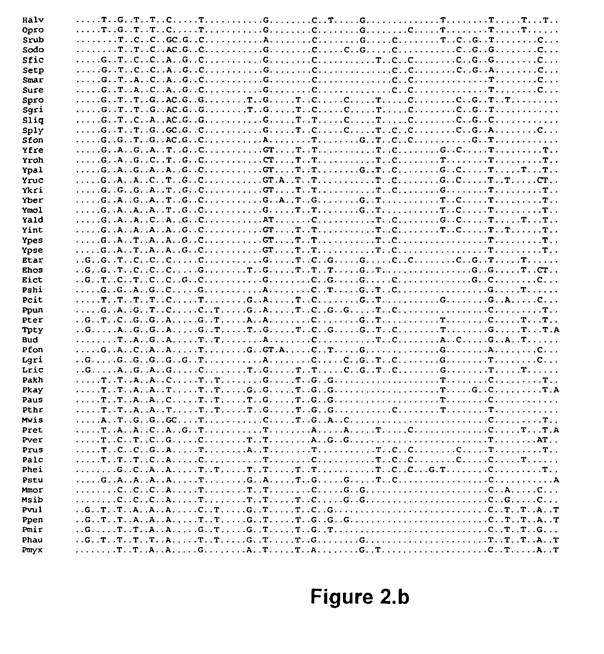

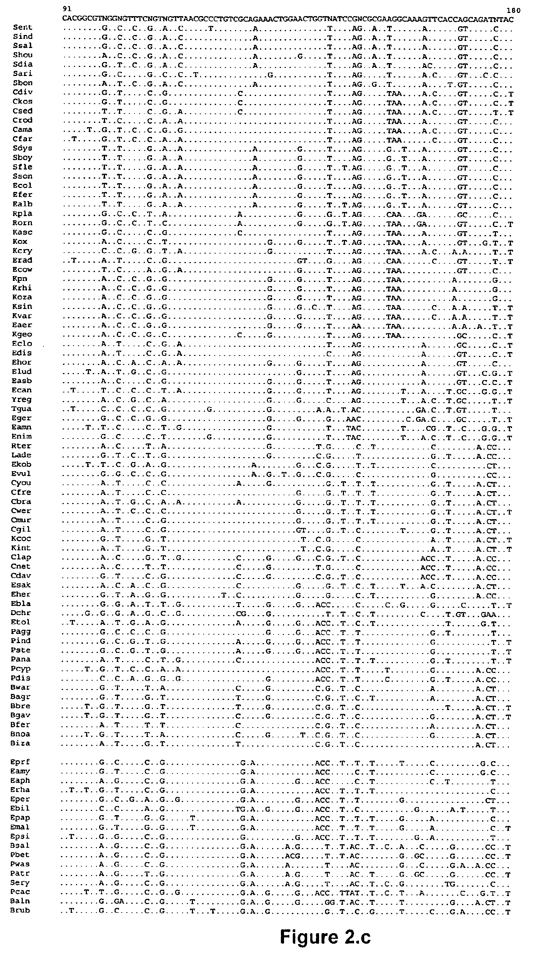

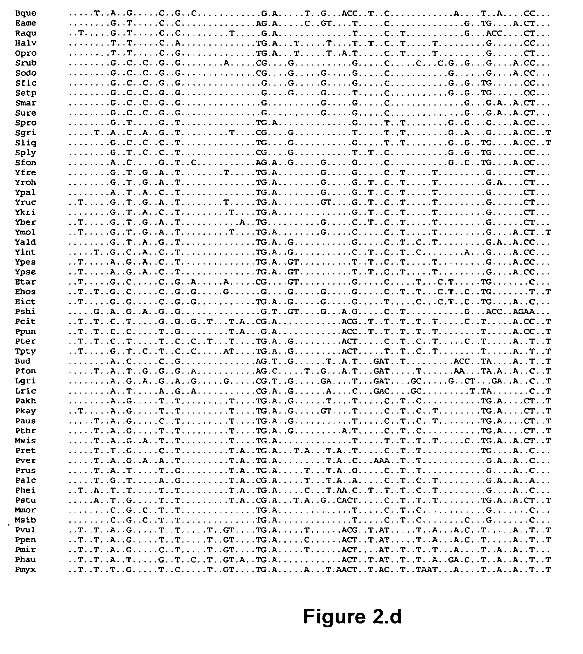

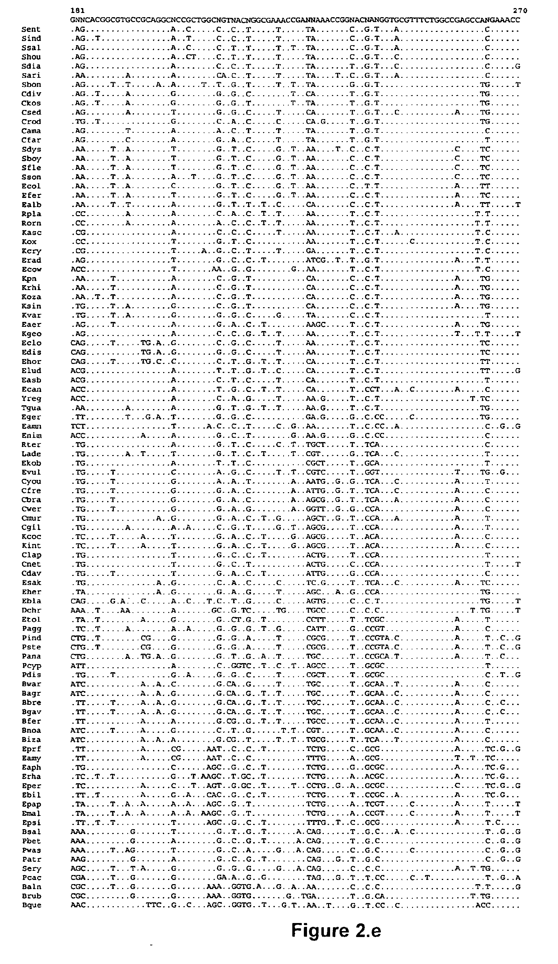

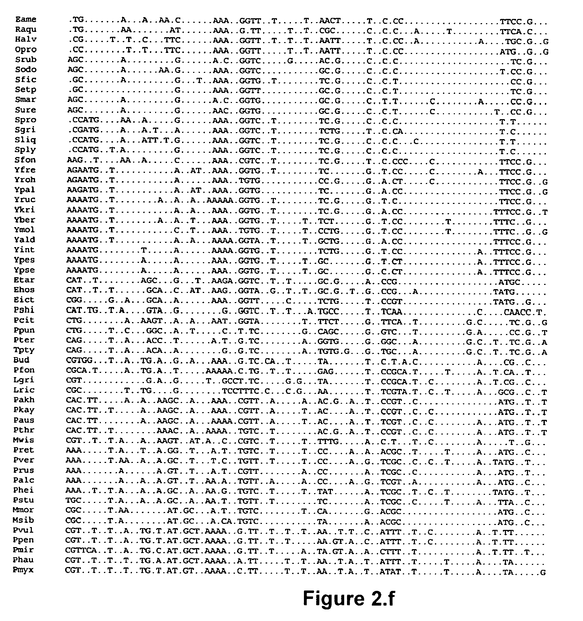

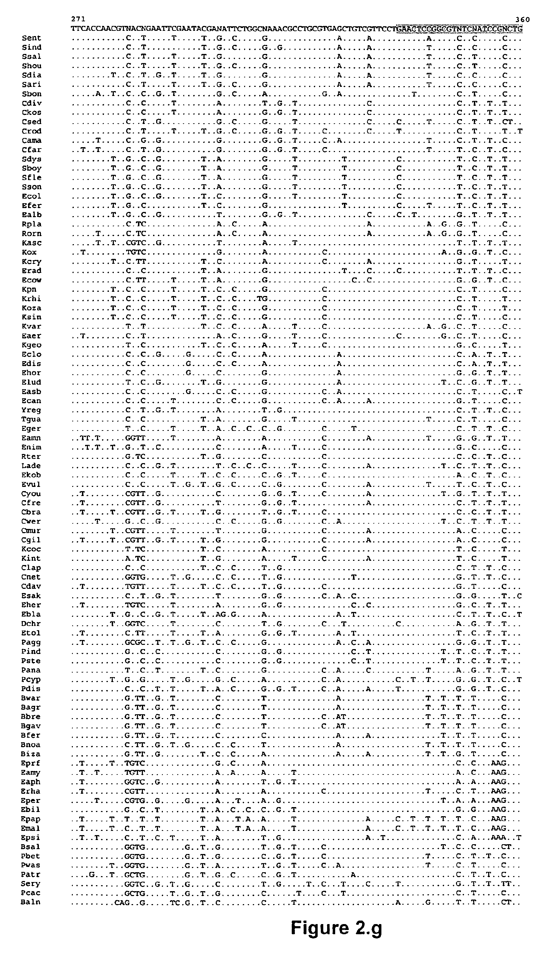

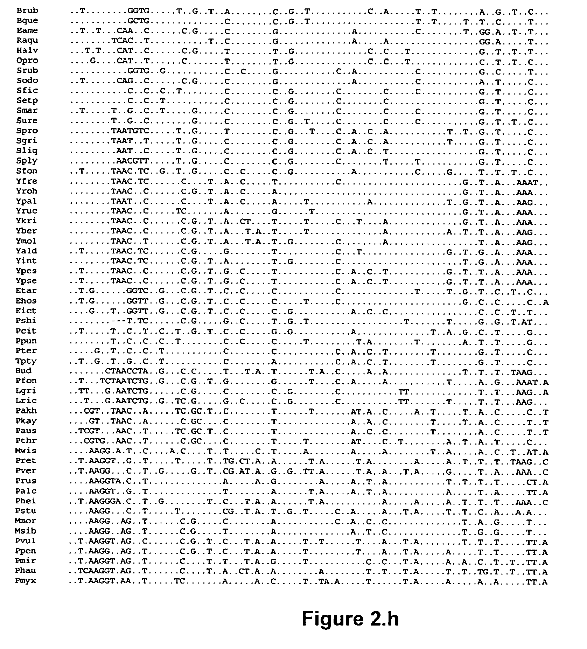

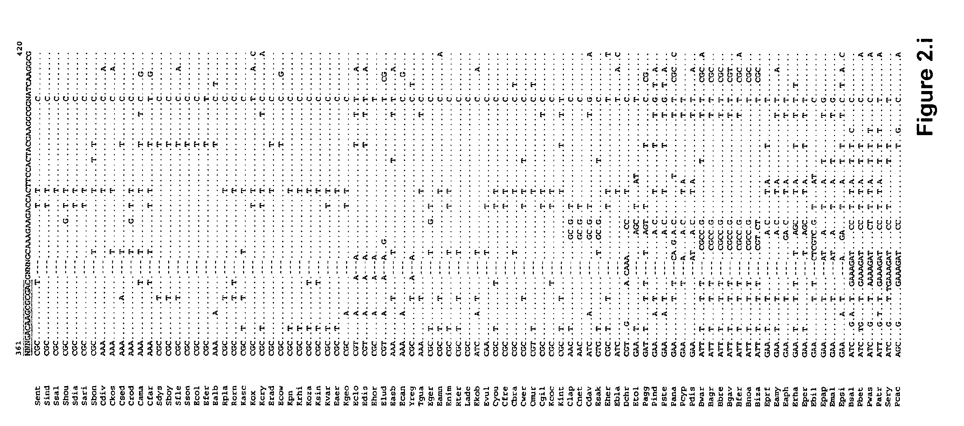

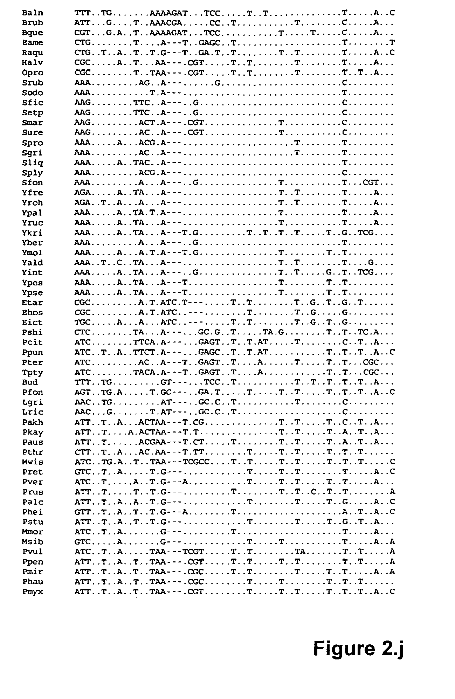

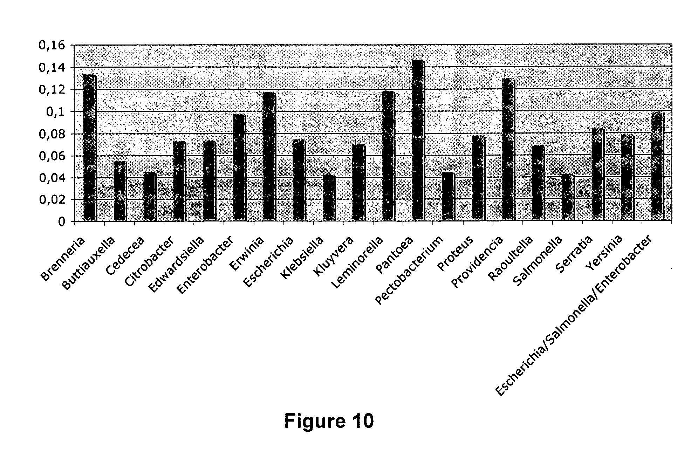

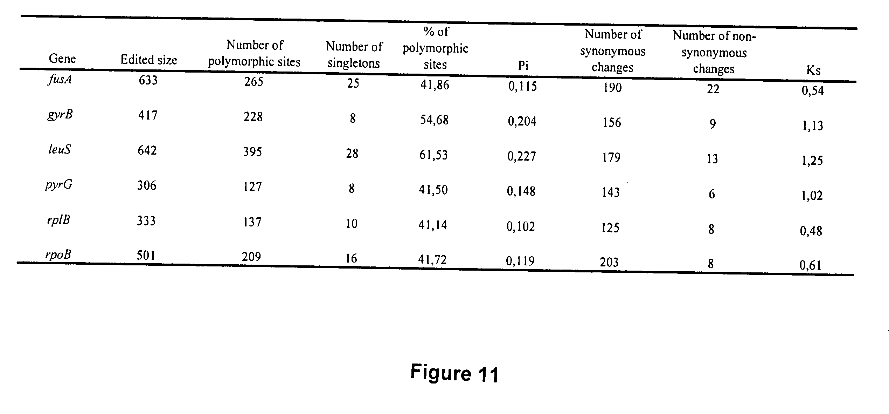

The analysis of the isolated products obtained in step A may be performed by any method known by those of skill in the art and for instance by:
1) sequencing (Sanger method, pyrosequencing...)
2) hybridization (on solid supports: plates or beads, resequencing or sequencing by hybridization (SBH)...) followed by, depending on the choice of the oligonucleotides used (probes, or primers), determining the base composition (mass spectrometry) or performing a SNP analysis (minisequencing, SNaP shot, iPLEX, TaqMan...), considering that in the six selected sequenced target regions, the different variable positions or sites (SNPs), as it emerges from figures 2-7 in conjunction with figures 10 and 11, provide a lot of potential discriminative sites between all species of *Enterobacteriaceae.* Specific sets of variable sites can therefore be selected on the basis of the information given in said cited figures, depending on the sites that are most appropriate for the identification assay under consideration (eg, clustered SNPs for oligonucleotide-probe based assays, versus isolated SNPs for primer annealing-based methods) or
3) Restriction fragment length polymorphism (RFLP).

Therefore, step B comprises (but is not limited to) the following possibilities:
1) sequencing method: step B of determining the species of *Enterobacteriaceae* comprises the step (B1) of analysing the isolated product obtained in step A by sequencing at least one target region or a fragment thereof and the step (B2) of determining the species of the *Enterobacteriaceae* by comparing said at least one target region or a fragment thereof with the sequences of the repertory of nucleic acids of the 171 species of *Enterobacteriaceae* family listed in figure 1 (SEQ ID NO:1-6 and SEQ ID NO:26 to SEQ ID NO:983).
   Examples of use of such a method for step B are specified below:
   * step (B1): analysis of a fragment of 43 nucleotides between positions 282 and 324 of the *gyrB* target region (SEQ ID NO:1) and step (B2): comparing said fragment with the corresponding fragments of the sequences of the repertory of nucleic acids of the 171 species of the *Enterobacteriaceae* family listed in figure 1 (SEQ ID NO:26-177); the analysis of said fragment allows the discrimination of the species *Enterobacter cloacae.*
   * step (B1): analysis of a fragment of 39 nucleotides between positions 91 and 129 of the *fusA* target region (SEQ ID NO:3) and analysis of a fragment between the positions 378 and 402 of the *leuS* target region (SEQ ID NO:4) and step (B2): comparing said two fragments with the corresponding fragments of the sequences of the repertory of nucleic acids of the 171 species of the *Enterobacteriaceae* family listed in figure 1 (SEQ ID NO:346-511 and SEQ ID NO:512-659); the analysis of said two fragments allows the discrimination of *Klebsiella pneumoniae* from the other *K. pneumoniae* subspecies.
2) by hybridization: a set or collection of a plurality of oligonucleotides is immobilized on solid supports (or substrates), which may be in the form of plates or in the form of beads and are for instance made of the following components: glass, nitrocellulose, nylon, polyacrylamide-coated glass, polypropylene, polystyrene and silicon. In the basic multispot array experiment, the array is exposed to the isolated products obtained in step A and specific binding or hybridization occurs between said isolated products and the individual oligonucleotide spots on the array; a label or detection reaction reveals the pattern of binding and provides information on the reactivity and hence on the composition of the isolated products applied to the array. Depending on the choice of the oligonucleotides, it will be possible to perform a DNA sequence analysis (resequencing) or a SNP analysis.

### For instance:

- for sequencing by hybridization (resequencing), step B of determining the species of *Enterobacteriaceae* comprises the step (B1) of analysing said isolated products (corresponding to at least one selected target region or a fragment thereof) by hybridization using all possible oligonucleotides in a tiling strategy. Each position in each of the selected target sequence (isolated products as defined here above) is interrogated by a plurality of sets of four or eight (four for each of the two strands of DNA) oligonucleotides differing only at the nucleotide being interrogated, which is generally positioned in the middle of the oligonucleotide (probe) sequence. The sets of four oligonucleotides (or probes) are tiled across the target sequence in increments of one nucleotide. The number of oligonucleotides (or probes) required to interrogate a target sequence of length N is 4^{N} or 8^{N}. The step (B2) comprises detecting the isolated products which are hybridized with the different oligonucleotides and determining the species by comparing with the sequences of the repertory of nucleic acids of the 171 species of the *Enterobacteriaceae* family listed in figure 1.
- for detection of SNP by hybridization, known methods by those skilled in the art may be used (24, 32, 33). For instance step B of determining the species of *Enterobacteriaceae* comprises the step (B1) of analysing said isolated products (comprising at least one selected target region or a fragment thereof) by hybridization using oligonucleotides, the 3' end of which is one nucleotide upstream the SNP position (or site) to be analysed; and step (B2) for determining the species comprises, for instance, extending immobilized oligonucleotides that anneal to their template sequences (isolated products comprising the target regions or fragments thereof) immediately adjacent to the SNP site using a DNA polymerase to specifically extend the 3' end of the oligonucleotide with a labelled nucleotide analogue complementary to the nucleotide at the variable site and determining the species by comparing the extension products obtained with the sequences of the repertory of nucleic acids of the 171 species of the *Enterobacteriaceae* family listed in figure 1. In such a case, the arrays are prepared by coupling one to four oligonucleotides per SNP to be detected on a small glass area for instance. Thus, the different target regions as defined here above are amplified, for example in PCR reactions (step A) and used as templates (24).

Globally, in one embodiment, the method of the invention comprises in step B, i.e. after having isolated and optionally amplified and/or labelled at least a fragment of at least one target region as defined here above:
- selecting the appropriate oligonucleotides (probes or primers),
- preparing an appropriate array,
- performing the assay including a hybridization step and carrying out known methods for those skilled in the Art (ligase, base addition, electric, MS, electrophoresis, fluocytometry PCR, TaqMan...),
- reading out the result (fluorescence, conductance, MS, electrophoresis...), and
- computing the results in reference to the data of figure 1.

Therefore, the oligonucleotides to be used may vary, depending on the method of analysing the hybridized products:
- in a general point of view, the oligonucleotides are fragments of 15-150 nucleotides corresponding to consecutive nucleotides of the target regions as defined above; they preferably comprise up to 25 nucleotides for DNA array applications or up to 80 nucleotides for pyrosequencing or up to 150 nucleotides for ESI-MS.
- in the more general case of hybridization, each oligonucleotide consists of a fragment of at least 15 nucleotides, preferably of 15-30 nucleotides and most preferably of 18-25 nucleotides corresponding to consecutive nucleotides of the target regions as defined here above and more precisely of sequences SEQ ID NO:1-6 and SEQ ID NO:26-983.
- for resequencing, each oligonucleotide consists of a fragment of an odd number of nucleotides, has a sequence of a length comprised between preferably of 19-29 nucleotides and corresponding to consecutive nucleotides of the sequences SEQ ID NO:1-6 and SEQ ID NO:26-983; a set of four or eight of complementary oligonucleotides being necessary for analysing a nucleotide, each oligonucleotide of the set differing only at the nucleotide being interrogated in the target region, which is generally positioned in the middle of the oligonucleotide (probe) sequence.
- for the detection of a SNP, each oligonucleotide consists of a fragment of 15-30 nucleotides corresponding to consecutive nucleotides of the sequences SEQ ID NO:1-6 and SEQ ID NO:26-983; in a particular embodiment, each oligonucleotide ends (3'extremity) either one nucleotide upstream a SNP site, or at a SNP site as defined on the basis of figures 2-7, in conjunction with figures 10-11.
- for the detection by mass spectrometry, known methods by those skilled in the art may be used, such as ESI-MS (34, 35).

According to the method of detection chosen, either the isolated products or the oligonucleotides will be labelled as specified here above.

Thus the invention encompasses at least:
- a method in which step B of determining the species of *Enterobacteriaceae* comprises the step (B1) of analysing the isolated product obtained in step A by hybridizing at least one target region or a fragment thereof with at least one oligonucleotide and preferably a plurality of oligonucleotides corresponding to fragments of the target regions as defined above, each of said oligonucleotides having a length of at least 15 consecutive nucleotides, and preferably of 18-25 nucleotides corresponding to consecutive nucleotides of the sequences SEQ ID NO:1-6 or SEQ ID NO:26-983 and the step (B2) of determining the species of the *Enterobacteriaceae* by determining the identity of the isolated products hybridized to the different oligonucleotides in reference to the sequences of the repertory of nucleic acids of the 171 species of the *Enterobacteriaceae* family listed in figure 1.
- a method in which step B of determining the species of *Enterobacteriaceae* comprises the step (B1) of analysing said isolated products obtained in step A (comprising at least one selected target region or a fragment thereof) by hybridizing at least one isolated product or a fragment thereof with at least one set of four oligonucleotides, each oligonucleotide corresponding to a fragment of an odd number of consecutive nucleotides of the target regions as defined above, having a sequence of a length comprised between preferably 19-29 nucleotides and corresponding to consecutive nucleotides of the sequences SEQ ID NO:1-6 or SEQ ID NO:26-983, each set of four oligonucleotides differing only at the nucleotide being interrogated in the target region, which is generally positioned in the middle of the oligonucleotide sequence and the step (B2) comprises detecting the isolated products which are hybridized with the different oligonucleotides and determining the species by comparing with the sequences of the repertory of nucleic acids of the 171 species of the *Enterobacteriaceae* family listed in figure 1.
- a method in which step B of determining the species of the *Enterobacteriaceae* family comprises the step (B1) of analysing the isolated products obtained in step A by hybridizing at least one isolated product or a fragment thereof with at least one oligonucleotide and preferably a plurality of oligonucleotides, each of said oligonucleotides corresponding to a fragment of the target regions as defined above, having a sequence of a length of at least 15 nucleotides, and preferably of 18-25 corresponding to consecutive nucleotides of the sequences SEQ ID NO:1-6 or SEQ ID NO:26-983 and ending (3'extremity) either one nucleotide upstream a SNP site, or at a SNP site as defined here above and the step (B2) comprises detecting the isolated products by extending immobilized oligonucleotides that anneal to their template sequences (isolated products comprising the target regions or fragments thereof) immediately adjacent to the SNP site using a DNA polymerase and labelled nucleotide analogues complementary to the nucleotide at the variable SNP site and determining the species by comparing the extension products obtained with the sequences of the repertory of nucleic acids of the 171 species of the *Enterobacteriaceae* family listed in figure 1.

In one mode of carrying out said last method, the step B consists, for discriminating the species *E. coli,* in identifying the nucleotide at position 87 of the *leuS* target region (SEQ ID NO:4); the nucleotide A at said position 87 is specific of the species *E. coli.*

In a second mode of carrying out said last method, the step B consists, for discriminating the genus *Buttiauxella* from other *Enterobacteriaceae,* in identifying the nucleotide at position 232 of the *leuS* target region (SEQ ID NO:4); the nucleotide A at said position 232 is specific of a *Buttiauxella* species.

In a third mode of carrying out said last method, step B consists, for discriminating *Y. pestis,* in identifying both the nucleotide at position 384 of the *fusA* target region (SEQ ID NO:3) and the nucleotide at position 240 of the *gyrB* target region (SEQ ID NO:1). The nucleotide T in position 384 of the *fusA* target region is specific to *Y. pestis* and *Y. pseudotuberculosis.* To separate these two species, the position 240 of the *gyrB* target region can be targeted because the nucleotide is a T for *Y. pestis* and a C for *Y. pseudotuberculosis.*

Indeed, for many species, the use of multiple positions is necessary to distinguish one species from the others, because there is no unique specific polymorphism allowing the identification. This is the case, for example, for *Yersinia pestis.* Nonetheless, two markers are enough to distinguish this species from the rest of the family.

In a fourth mode of carrying out said last method, step B consists, for discriminating *Salmonella enterica* subsp. *enterica,,* in identifying both the nucleotide at position 6 of the *pyrG* target region (SEQ ID NO:6) and the nucleotide at position 120 of the *gyrB* target region (SEQ ID NO:1). The G at position 6 of the *pyrG* target region (SEQ ID NO:6) is specific of the 6 subspecies of *Salmonella enterica. S. enterica* subsp. *enterica* can be distinguished from the other subspecies because it is the only *S. enterica* subspecies with a T at position 120 of the *gyrB* target region.

Therefore, it is possible to use a SNP genotyping method with an assay design that targets two or more SNPs, located either on the same gene or on different genes. For example, it is possible to use xMAP (36; BioPlex of BioRad) with, for each SNP to be determined, one or a pair of oligonucleotides that are complementary to both nucleotides of SNPs.

The present invention also relates to a DNA array comprising at least one polynucleotide selected among:
a) the polynucleotides of sequences SEQ ID NO:1-6, 7-11 and 26-983 or fragments thereof of at least 15 consecutive nucleotides and their reverse-complementary sequences
b) a fragment of at least 15-30 consecutive nucleotides of a polynucleotide defined in a)
c) a polynucleotide of at least 15-30 base pairs that hybridizes selectively under stringent conditions with a polynucleotide of a)
d) a plurality of fragments having an odd number of nucleotides having a sequence of a length comprised between 19-29 nucleotides and corresponding to consecutive nucleotides of the polynucleotides defined in a) except that their central nucleotide is N and their reverse-complementary sequences
e) a plurality of fragments having a sequence of a length comprised between 15 and 30 nucleotides and corresponding to consecutive nucleotides of the polynucleotides defined in a), said fragments ending either one nucleotide upstream a SNP site or at a SNP site.

The present invention also relates to nucleic acids selected among: nucleic acids of sequences SEQ ID NO:1-6 and 26-983 and their fragments of SEQ ID NO:7-11 and reverse-complementary sequences thereof.

The present invention also relates to nucleic acids selected among: nucleic acids of sequences SEQ ID NO:12-25.

These different oligonucleotides and target regions or fragments thereof may be parts of a kit for discriminating the species of the *Enterobacteriaceae* family.

More precisely, the kit for discriminating a bacterium of the *Enterobacteriaceae* family, comprises at least:
- primers able to amplify at least a sequence, or a fragment thereof, comprising a target region having a length comprised between 300 and 650 bp and selected among internal portions of a set of genes selected in the group consisting of *gyrB, rpoB, fusA, leuS, rplB* and *pyrG* of *Enterobacteriaceae,* or a fragment of said target region of at least 15 bp, preferably of 40-150 bp,
- a DNA array as defined above, and/or
- oligonucleotides as defined above, and
- the repertory of nucleic acids of the 171 species of *Enterobacteriaceae* family listed in figure 1 (SEQ ID NO:1-6 and SEQ ID NO:26 to SEQ ID NO:983).

Besides the above provisions, the invention also comprises other provisions which would emerge from the following description, which refers to examples of implementation of the invention and also to the attached drawings, in which:
- Figure 1: List of the 171 *Enterobacteriaceae* species
- Figure 2: Alignment of *gyrB* fragment of 420 bp in 148 of the species listed in figure 1

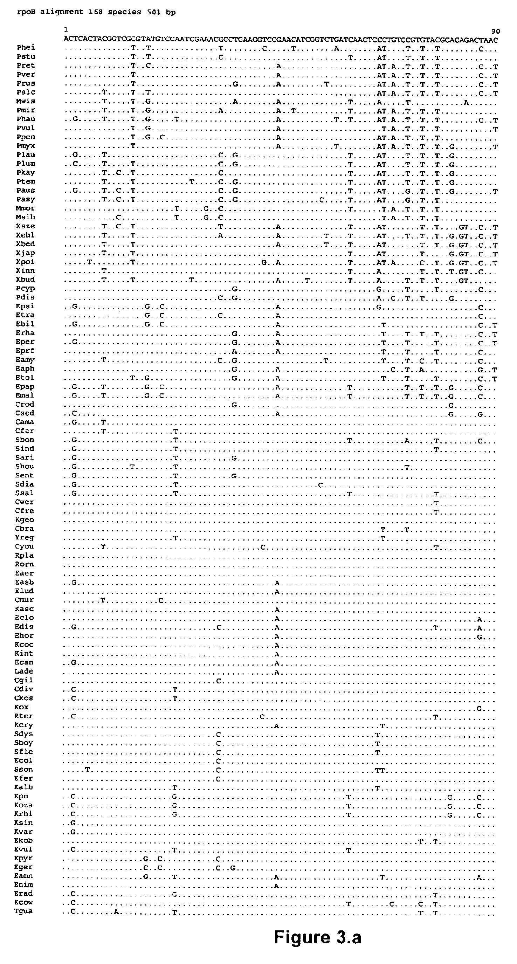

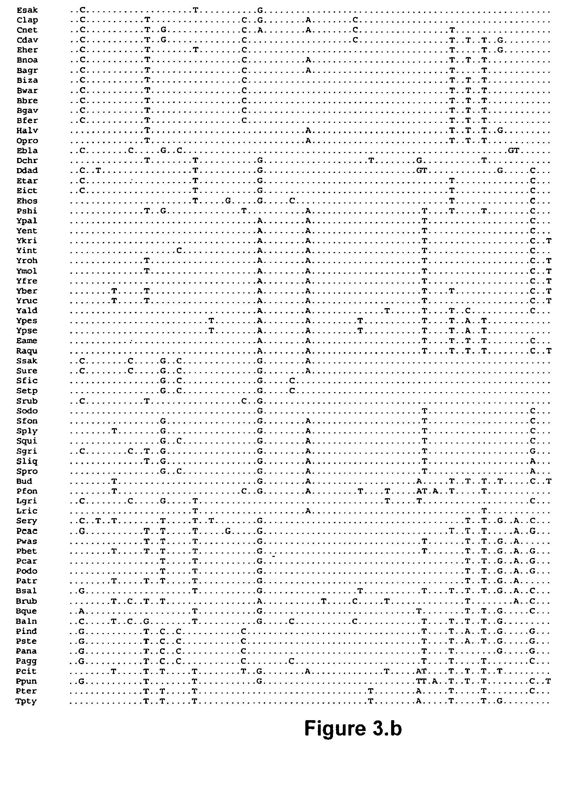

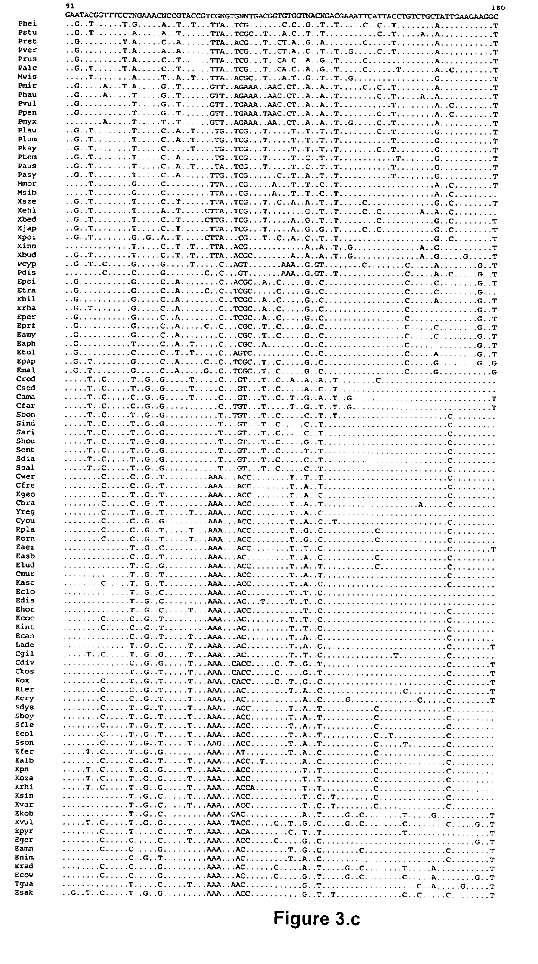

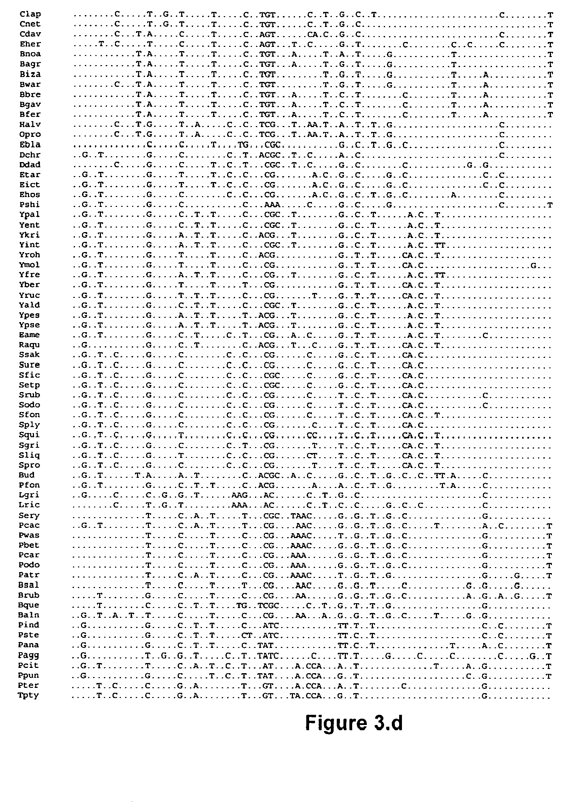

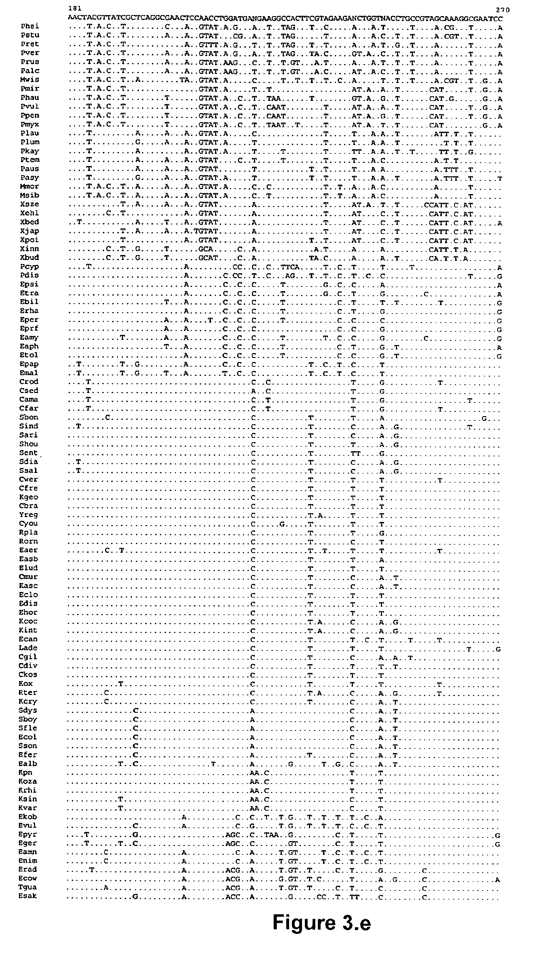

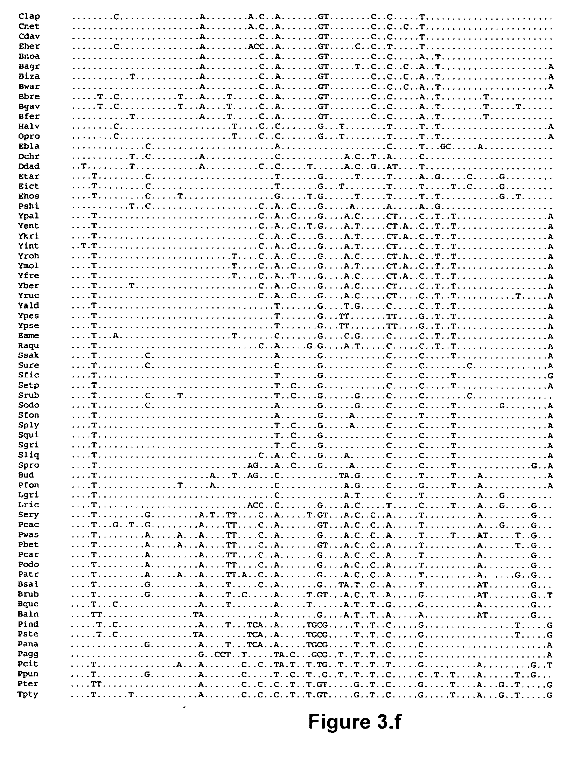

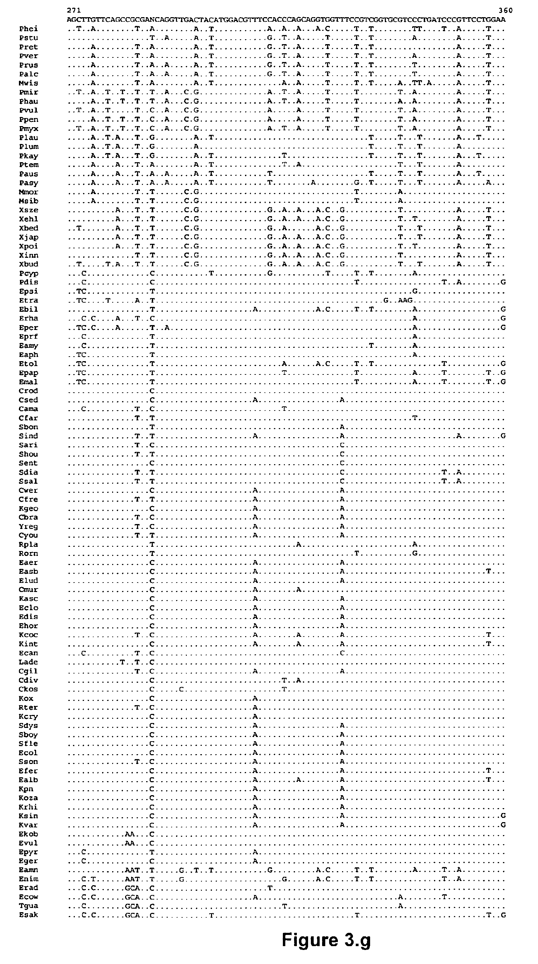

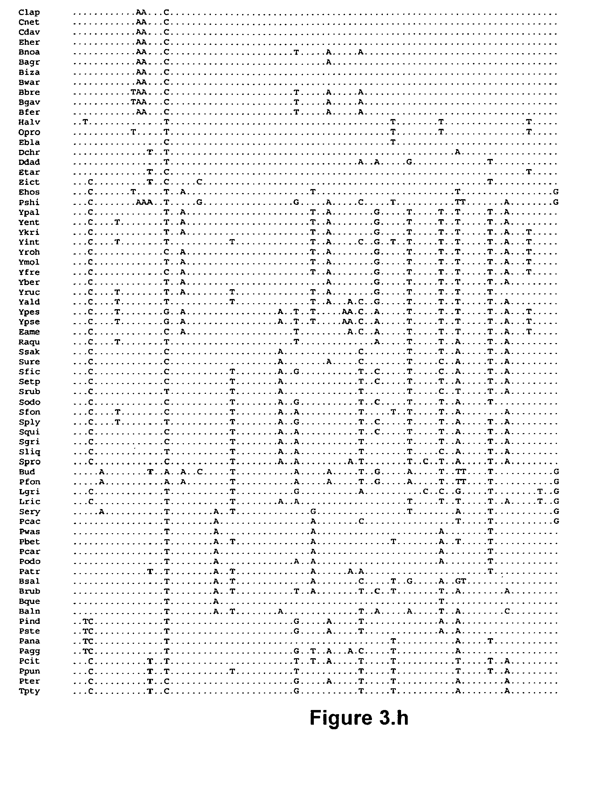

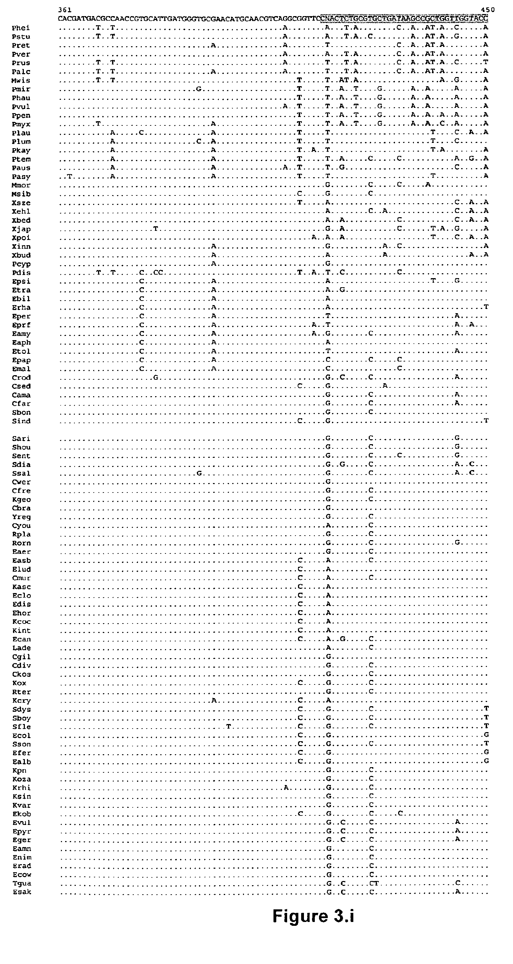

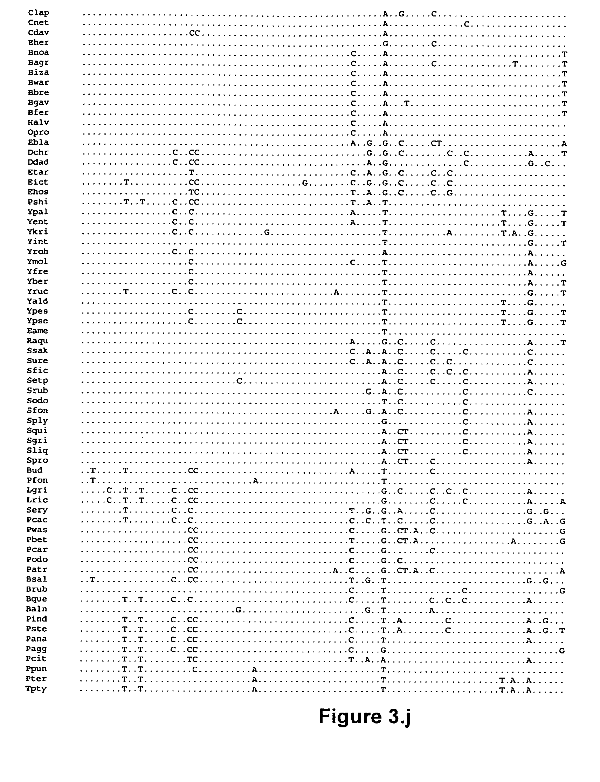

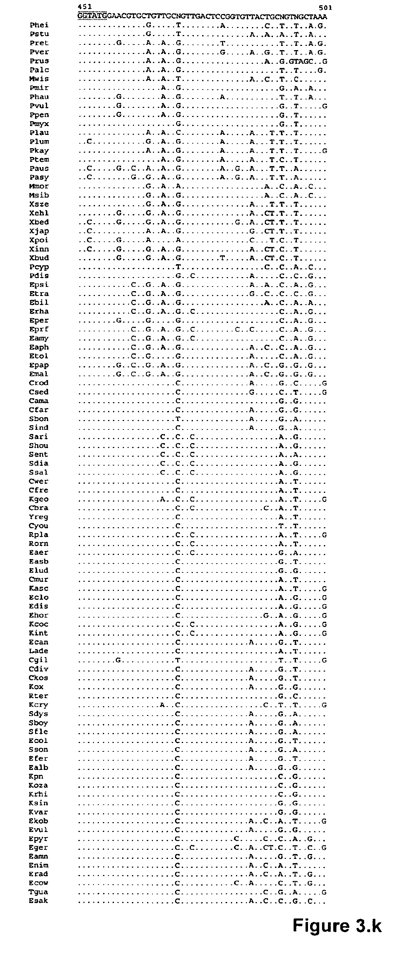

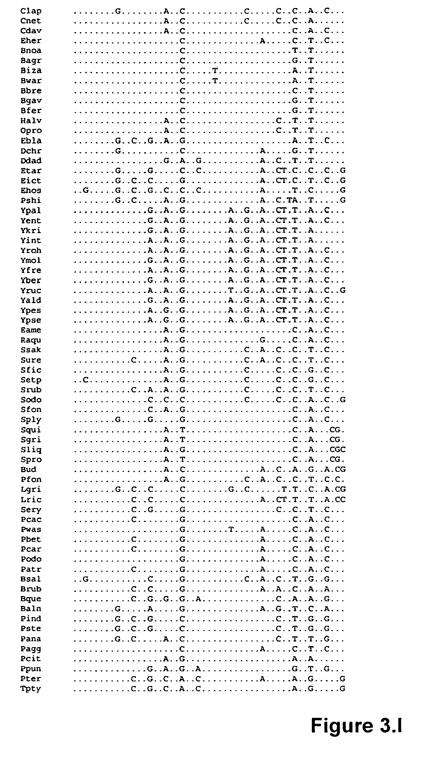

- Figure 3: Alignment of *rpoB* fragment of 501 bp in 168 species listed in Figure 1

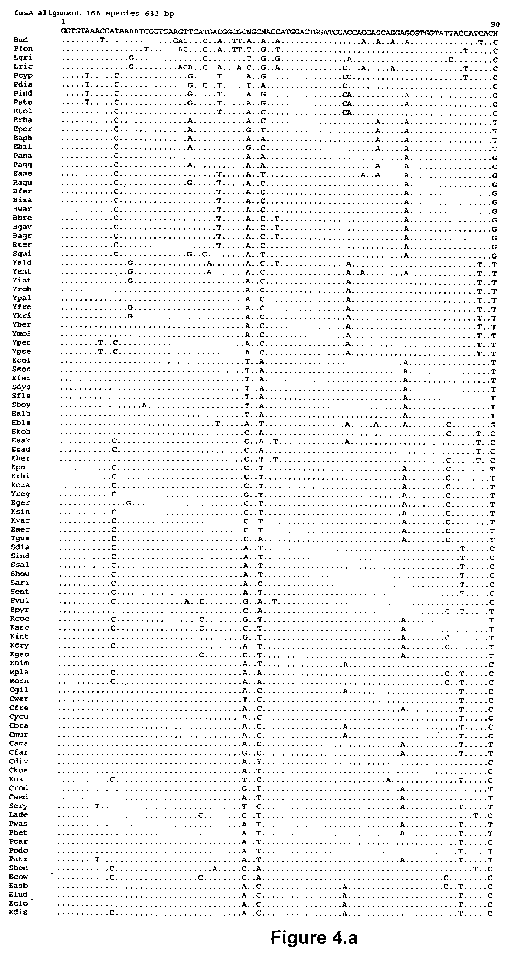

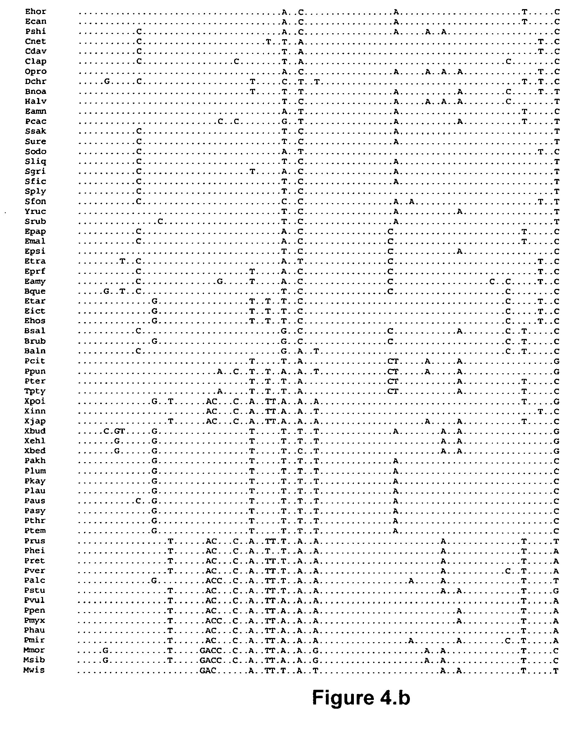

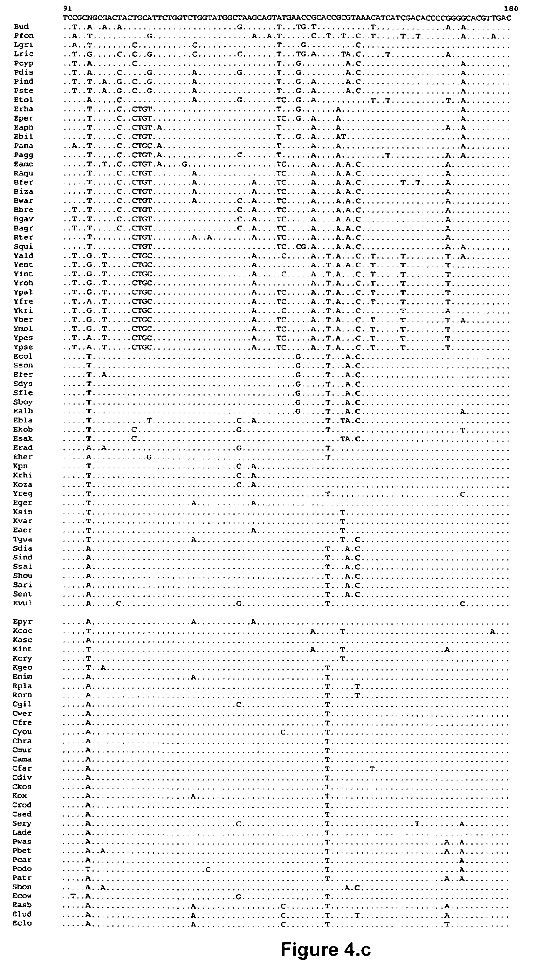

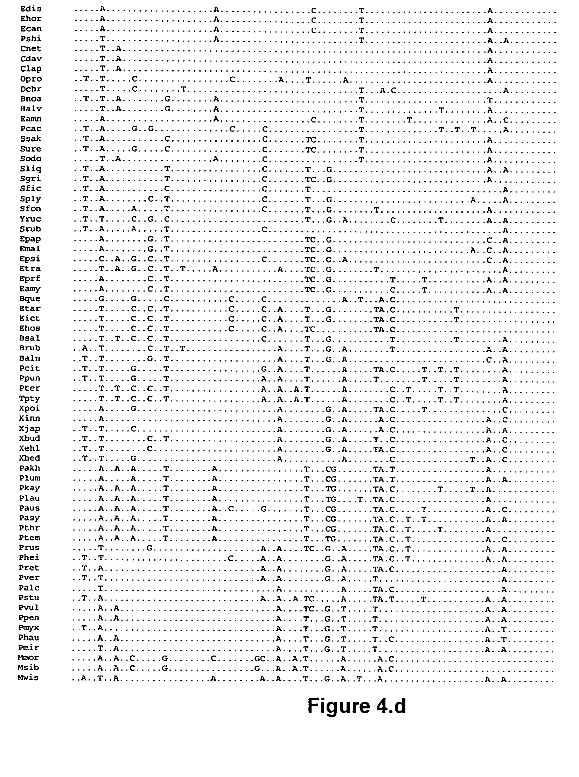

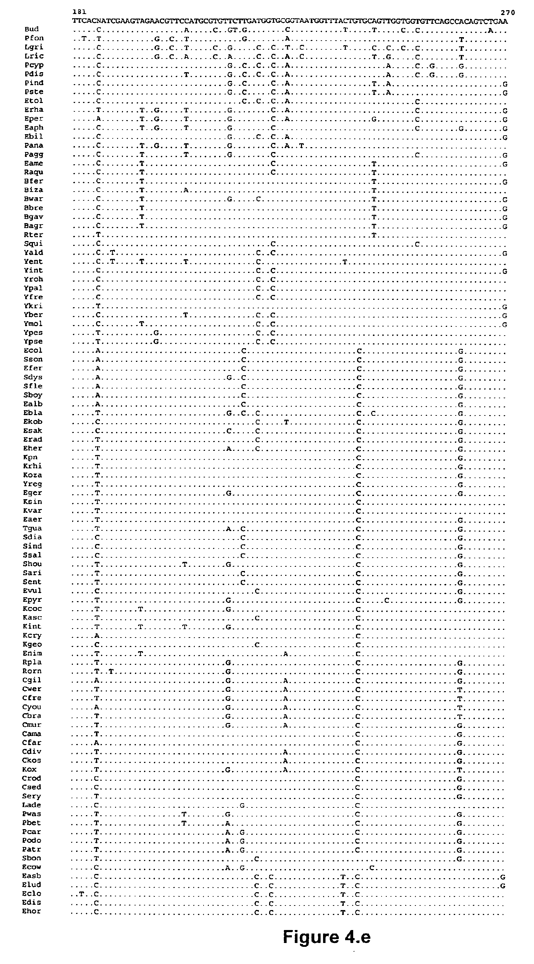

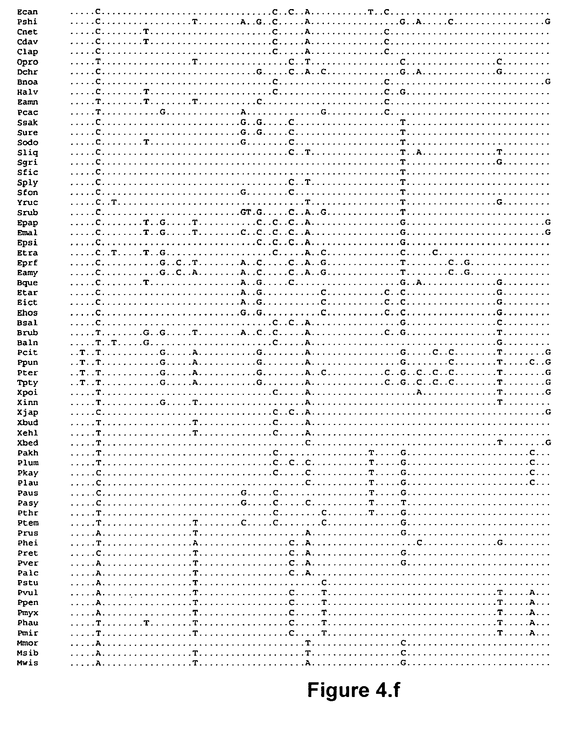

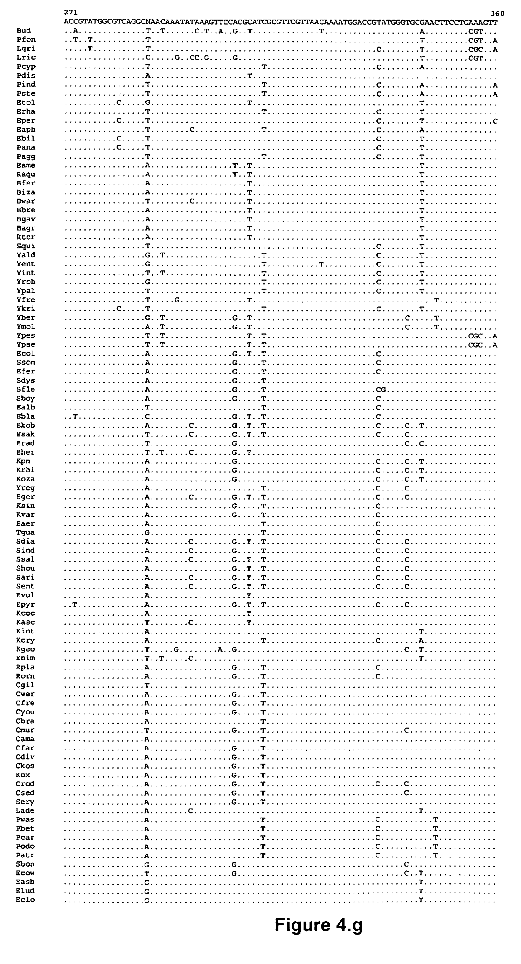

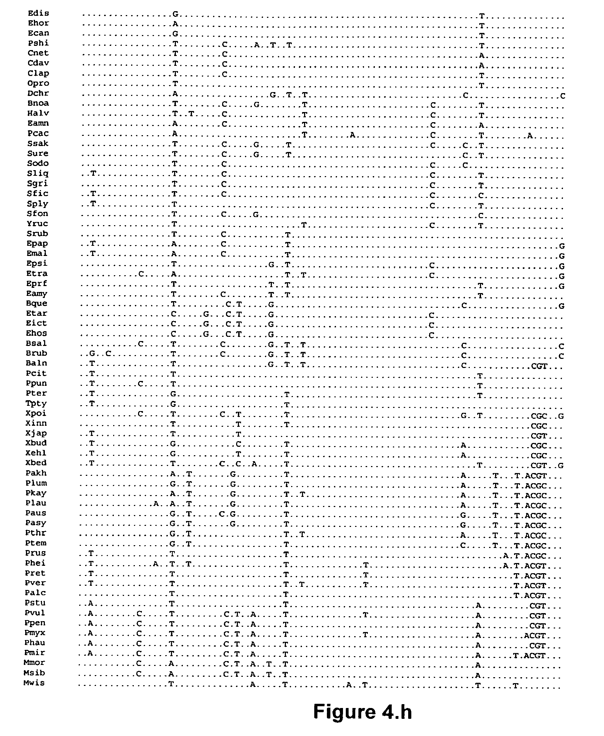

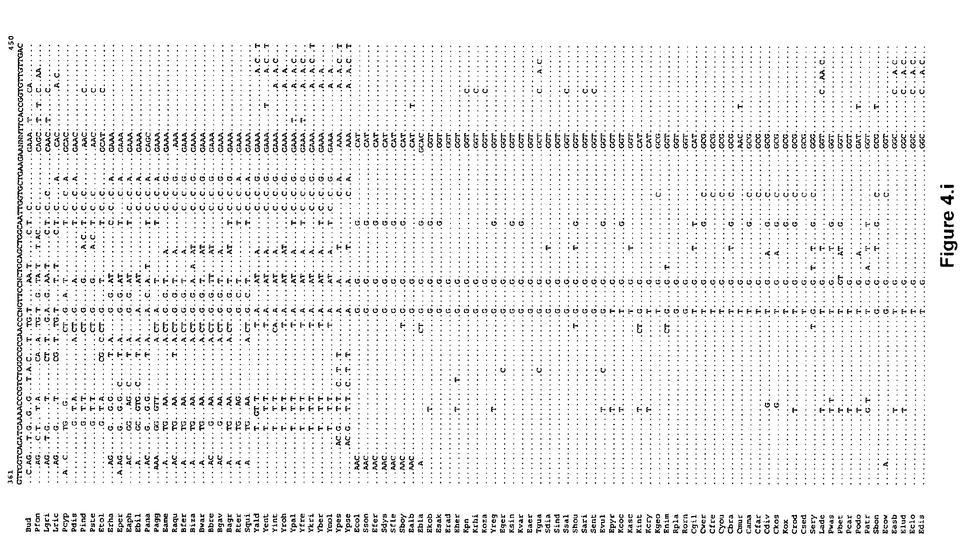

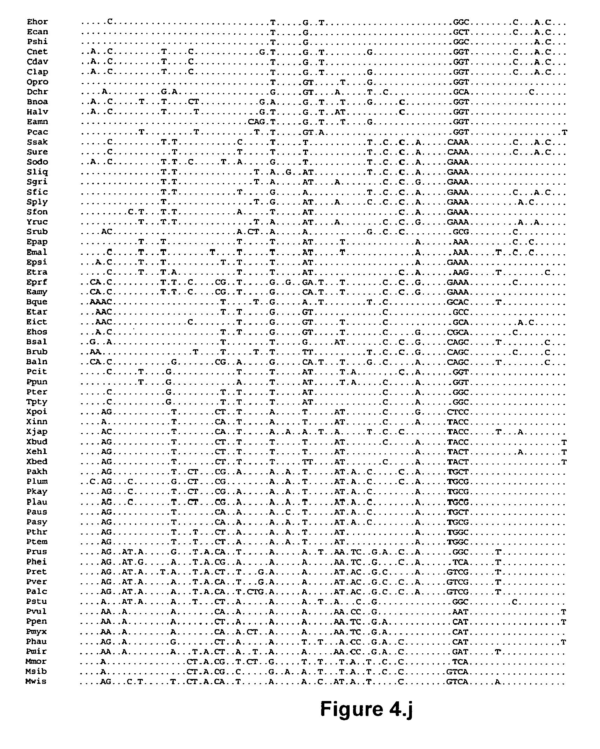

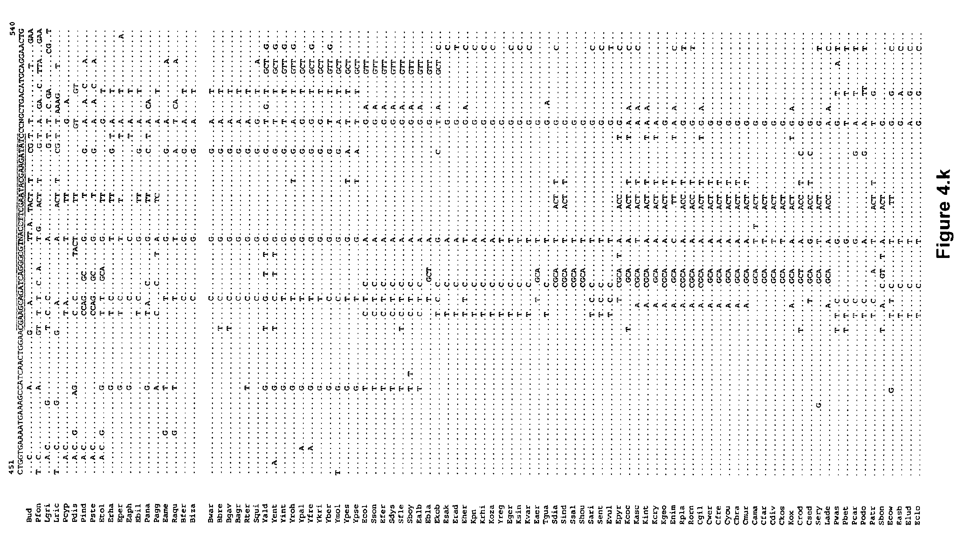

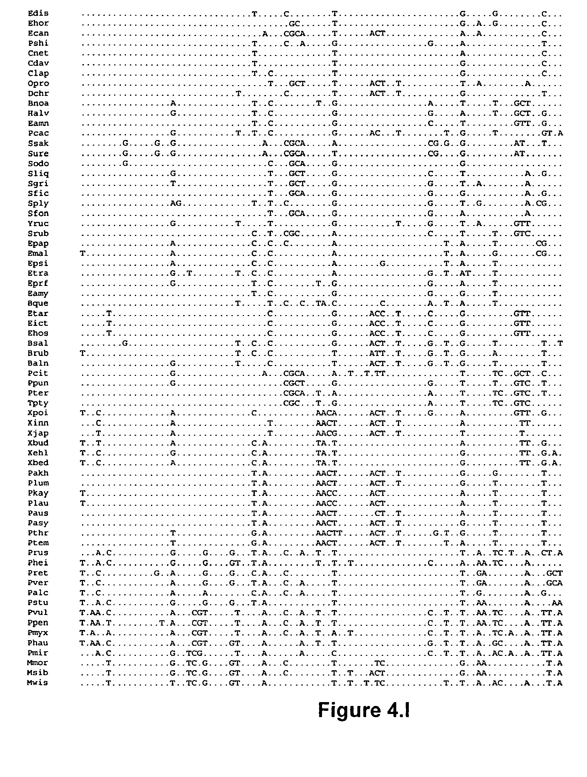

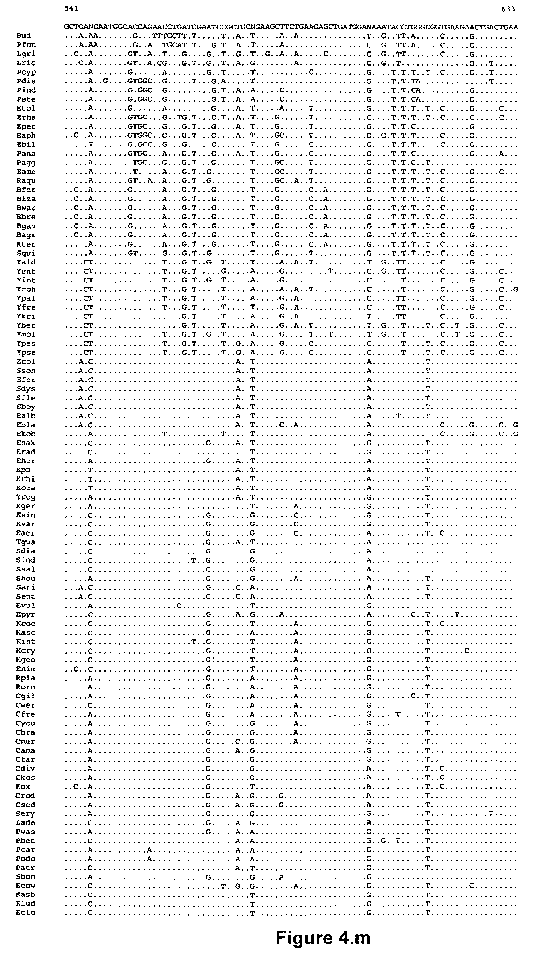

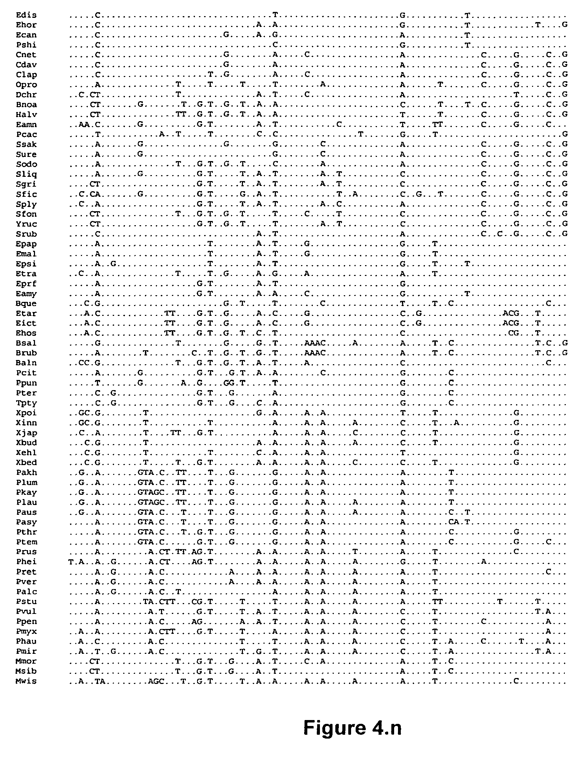

- Figure 4: Alignment of *fusA* fragment of 633 bp in 166 species listed in Figure 1

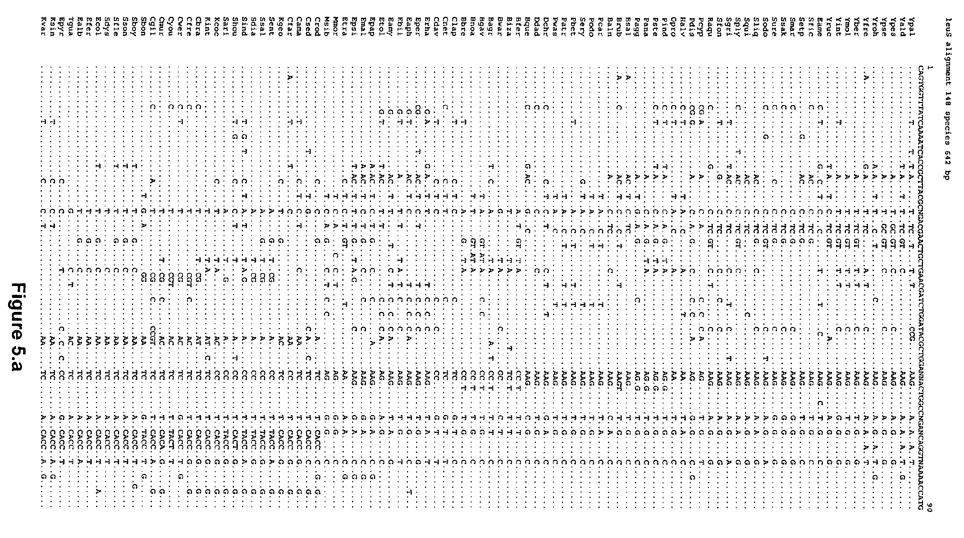

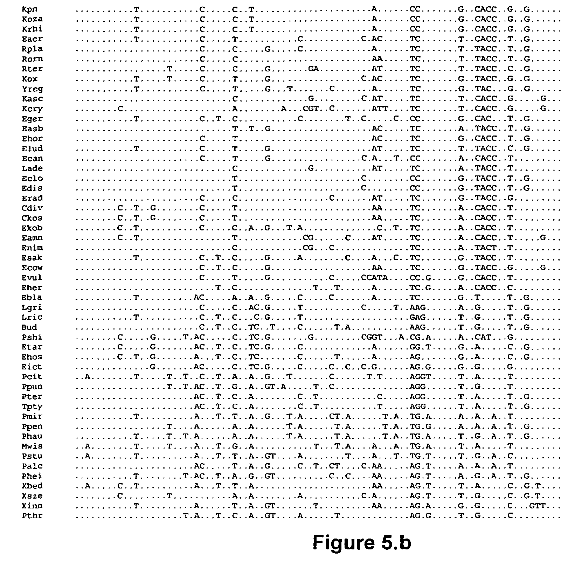

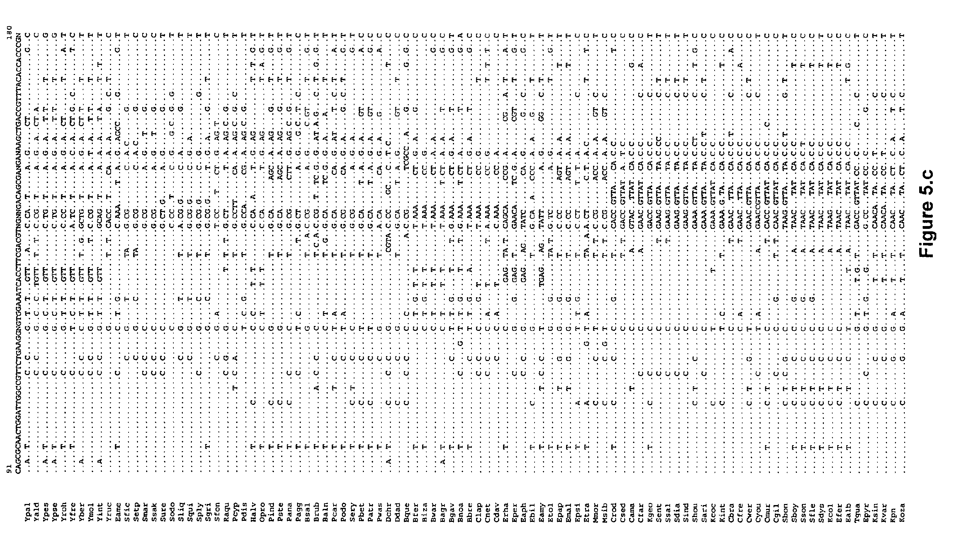

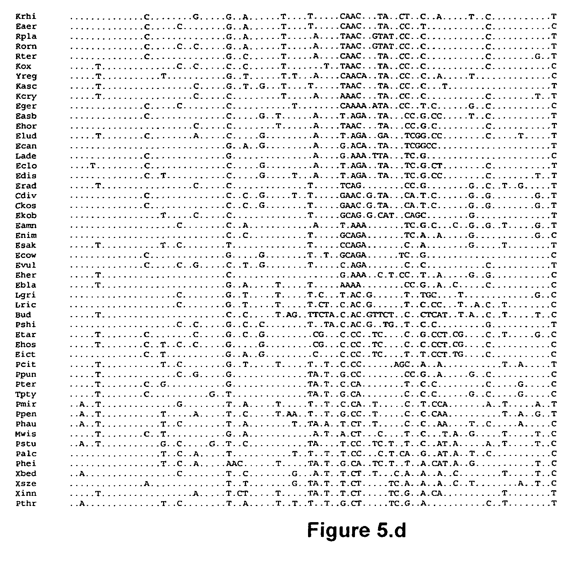

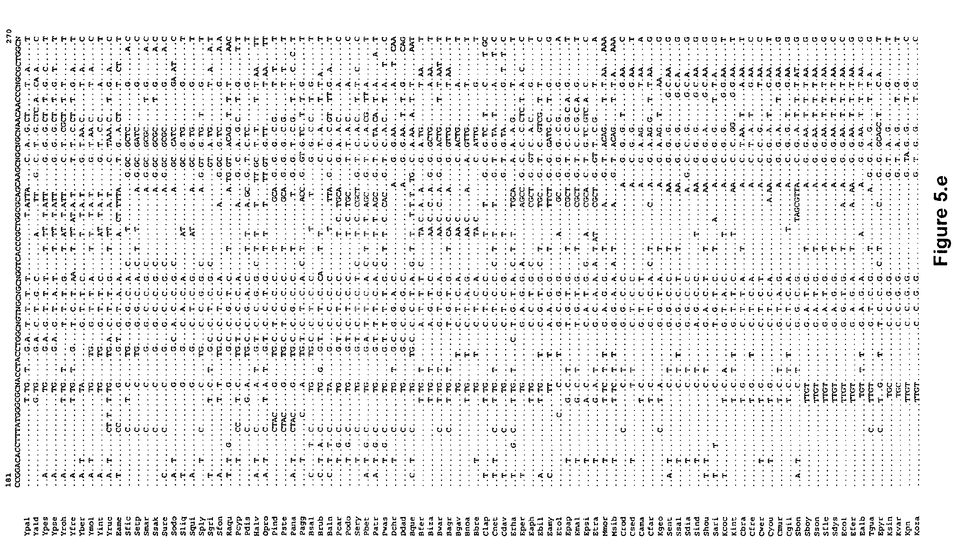

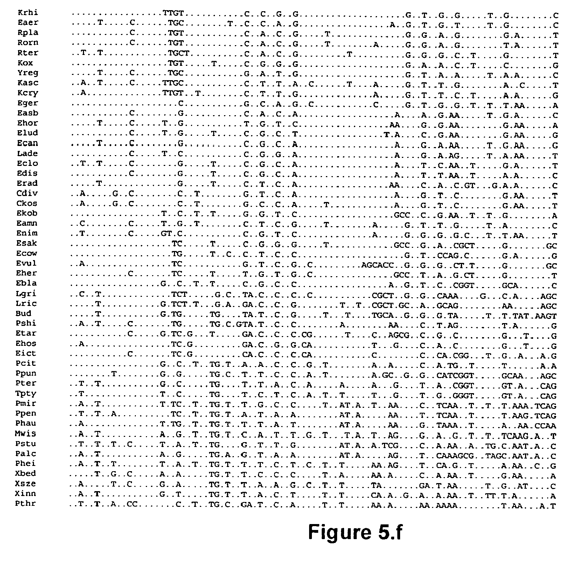

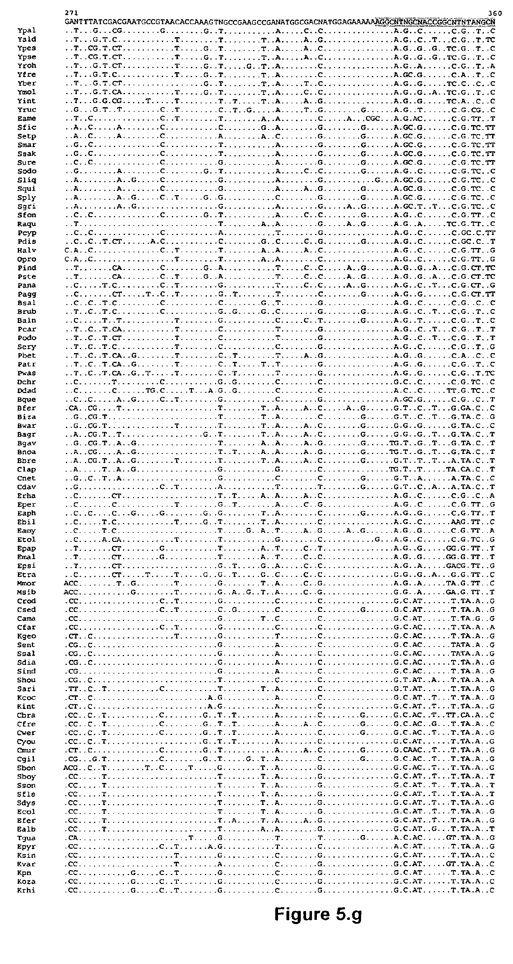

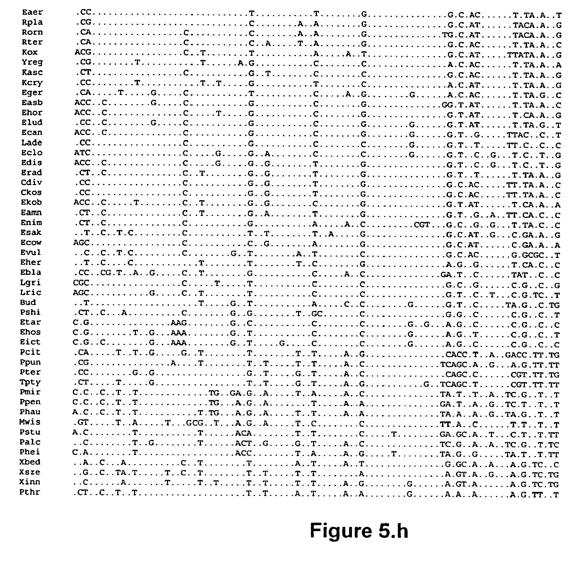

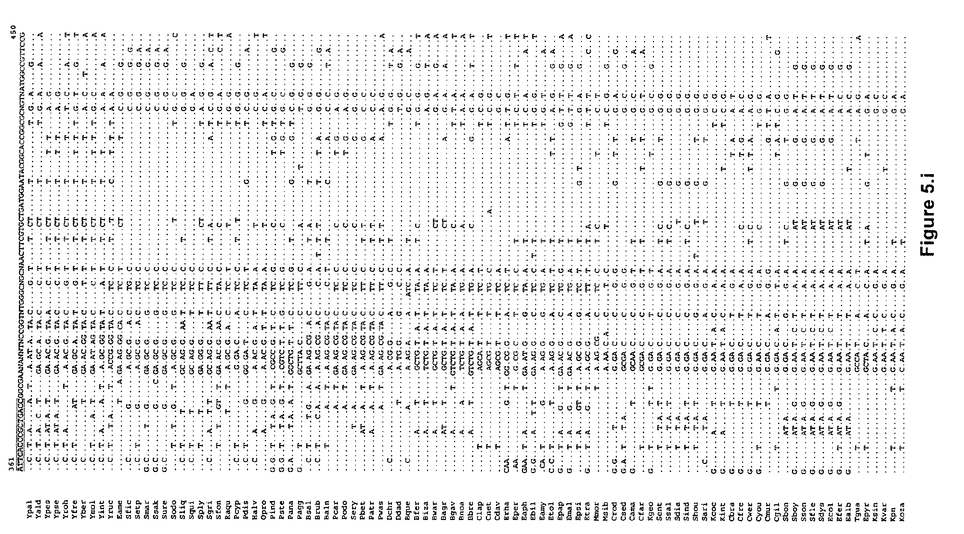

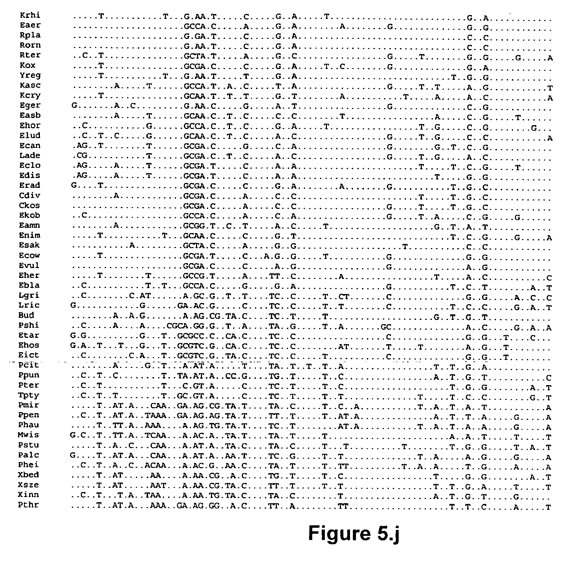

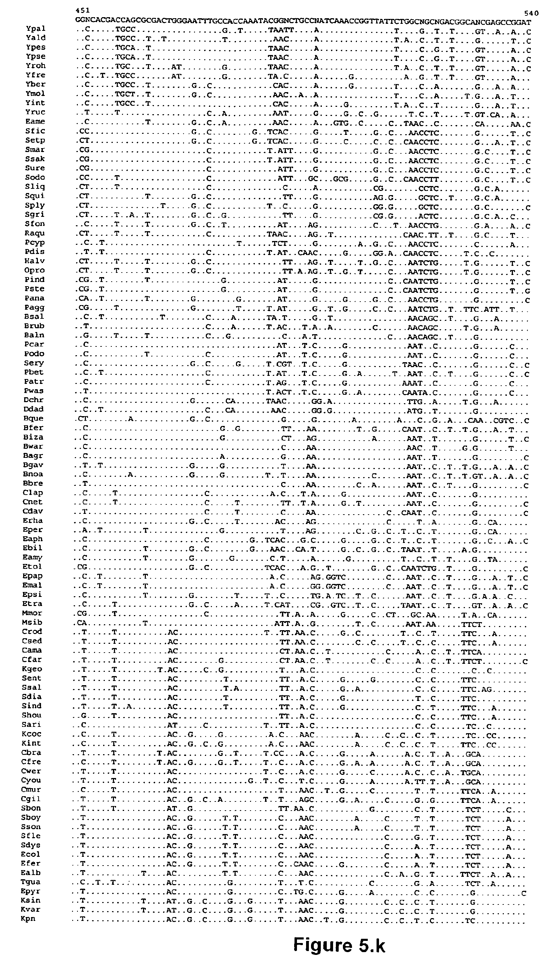

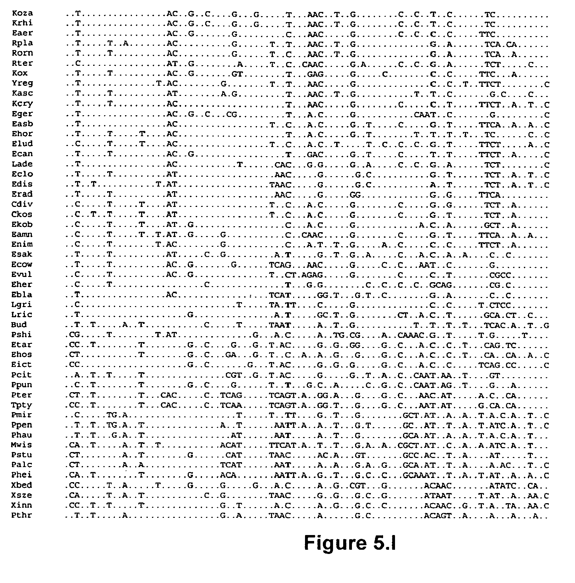

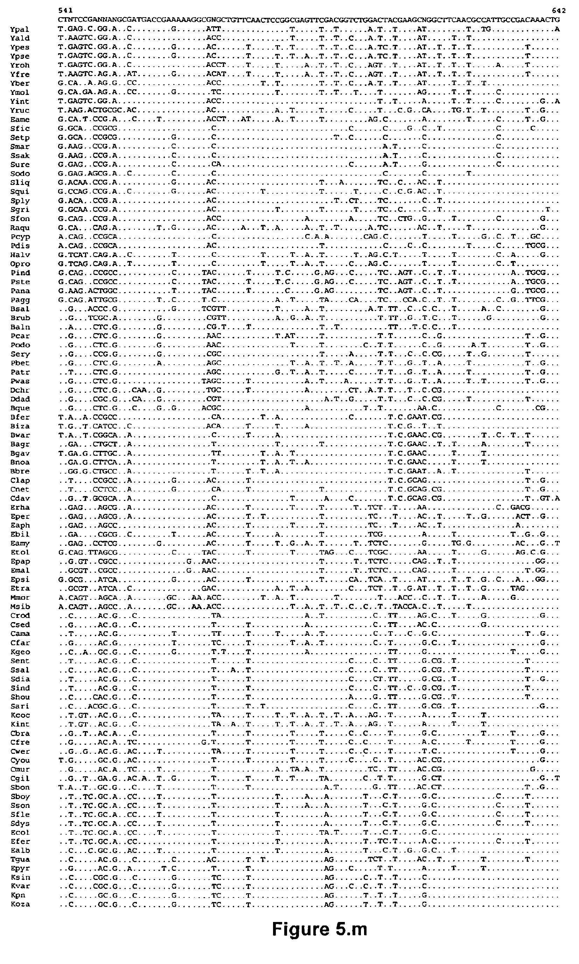

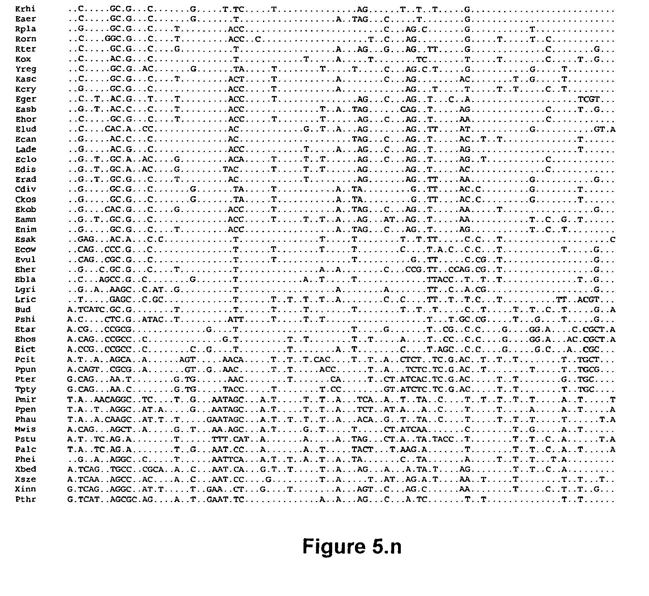

- Figure 5: Alignment of *leus* fragment of 642 bp in 148 species listed in Figure 1

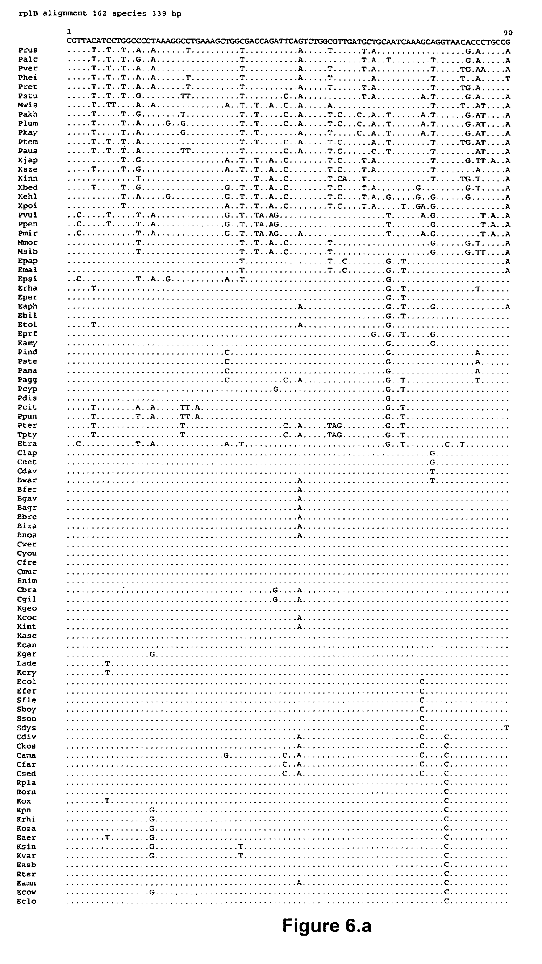

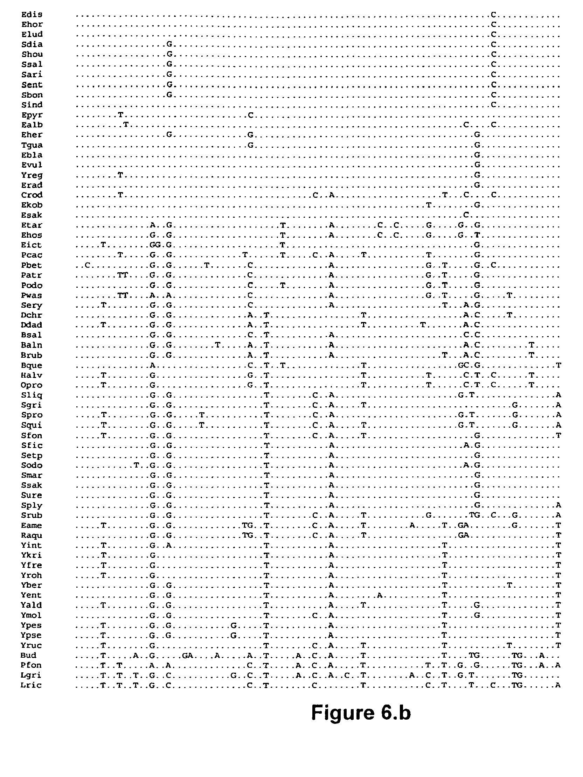

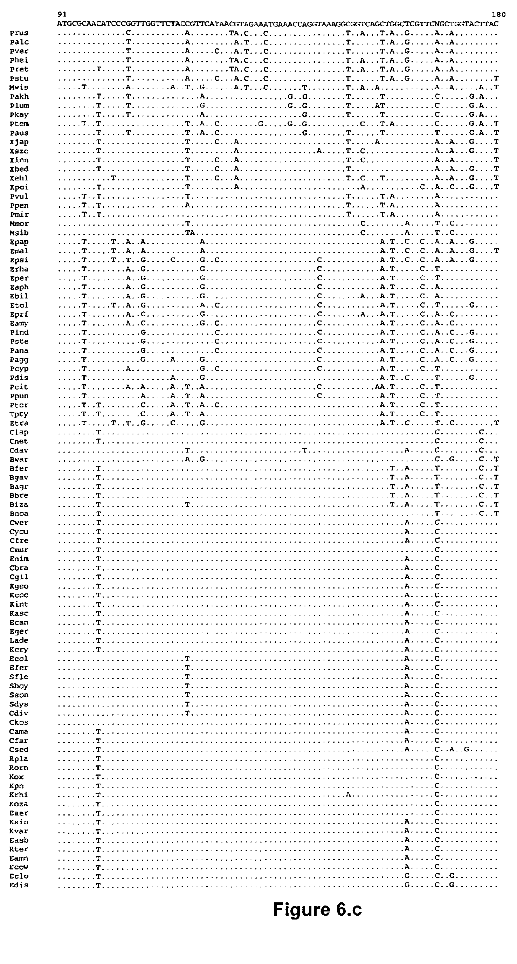

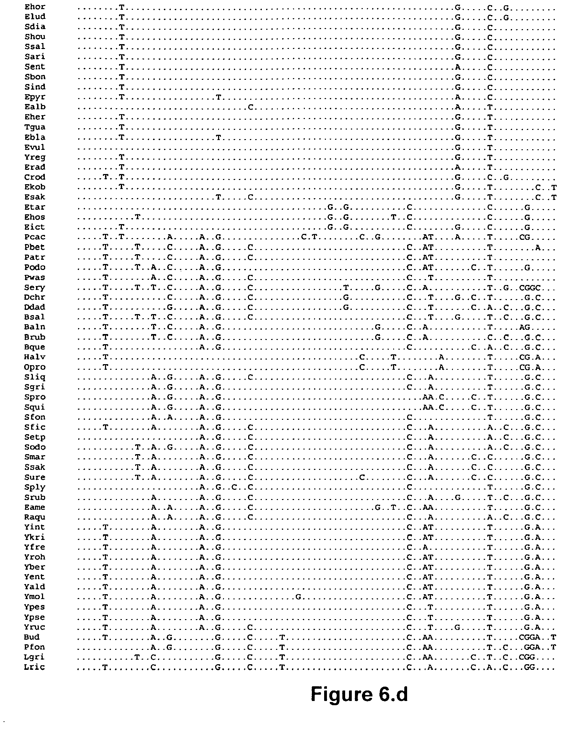

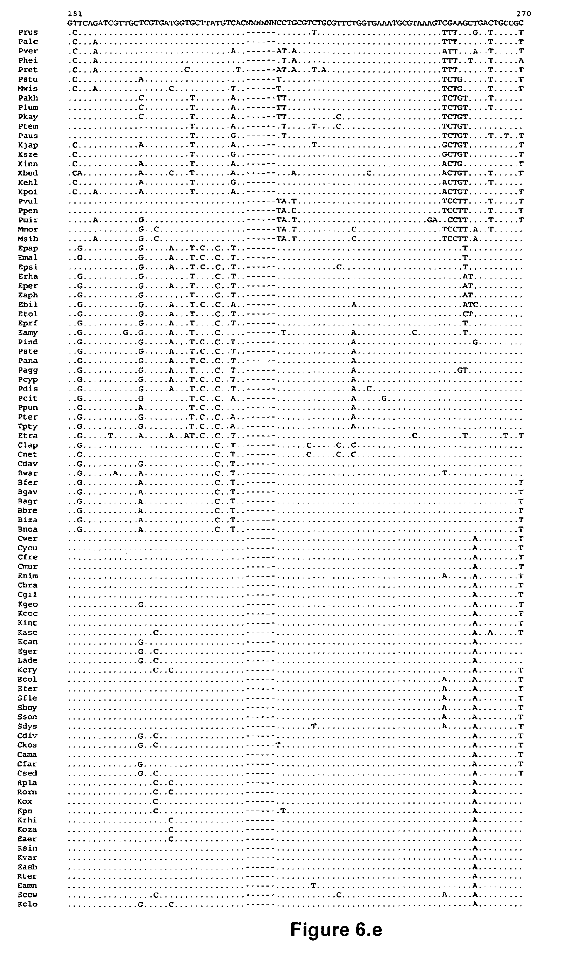

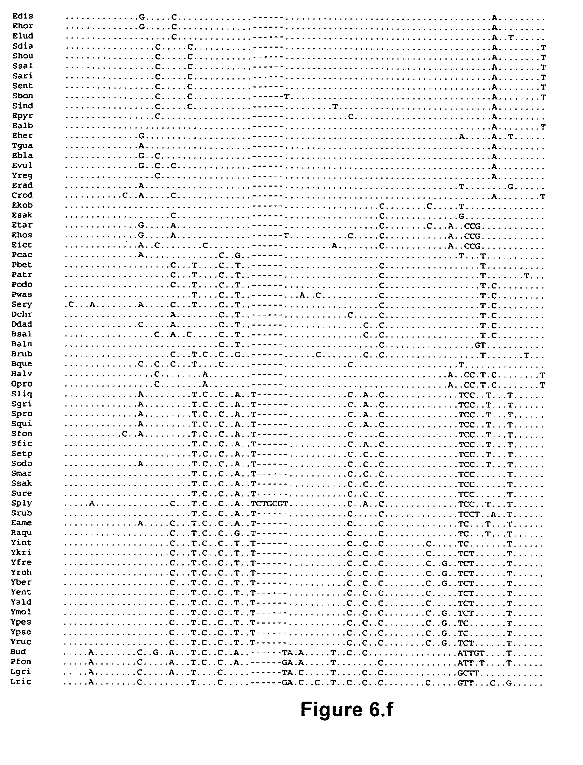

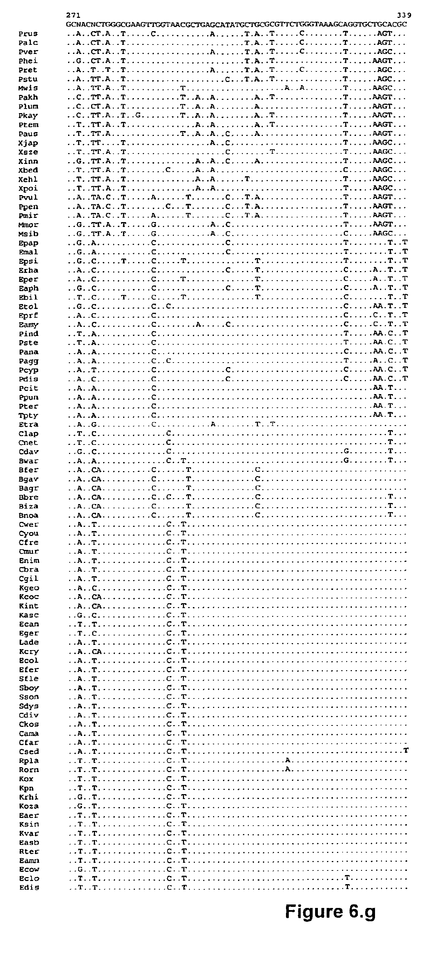

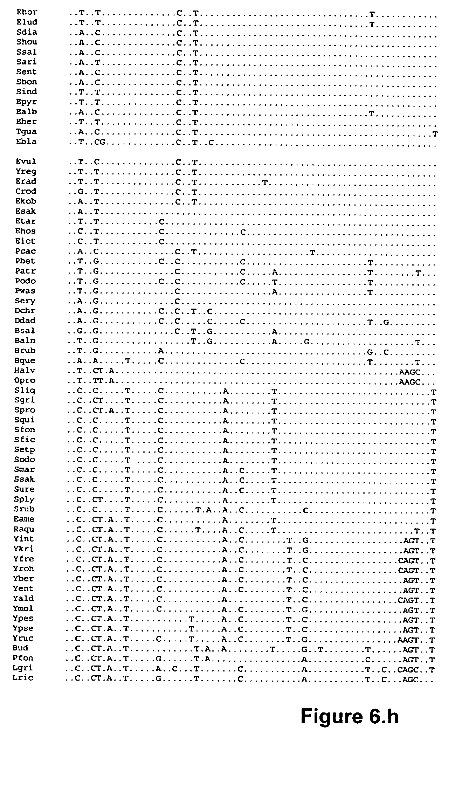

- Figure 6: Alignment of *rplB* fragment of 339 bp in 162 species listed in Figure 1

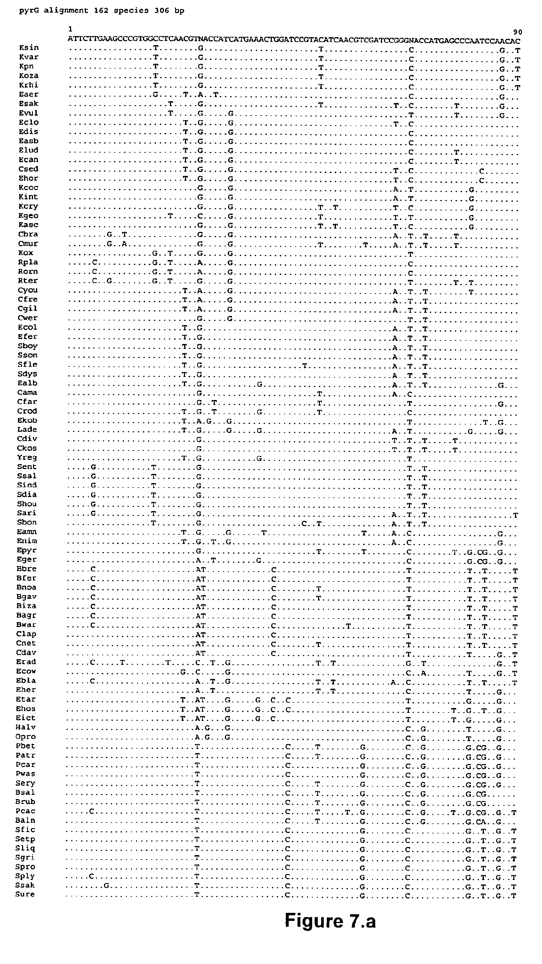

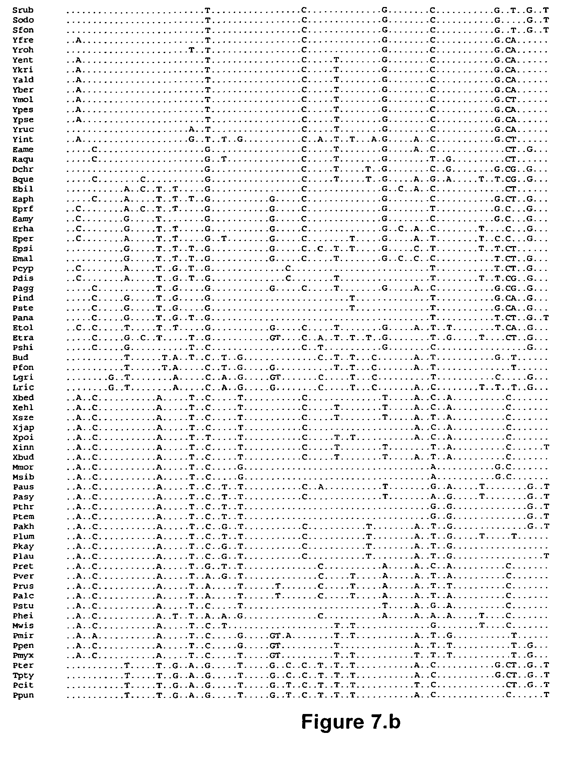

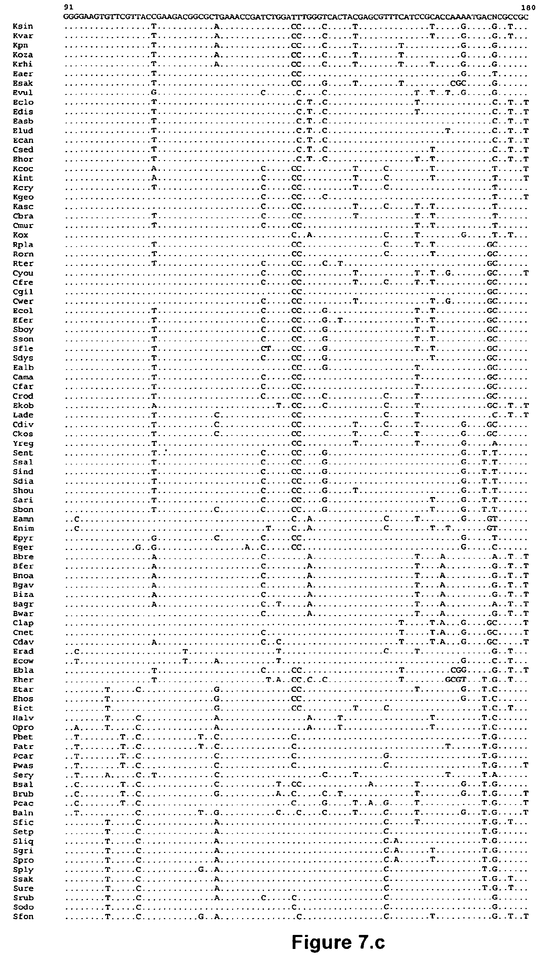

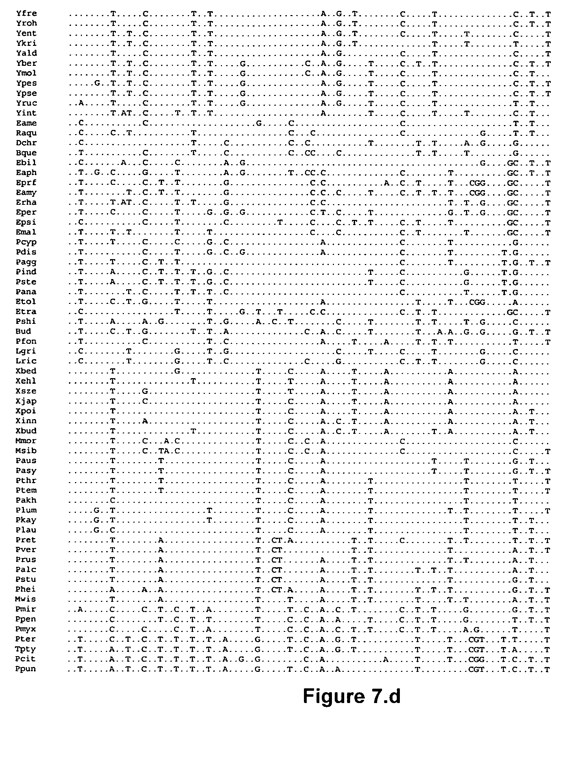

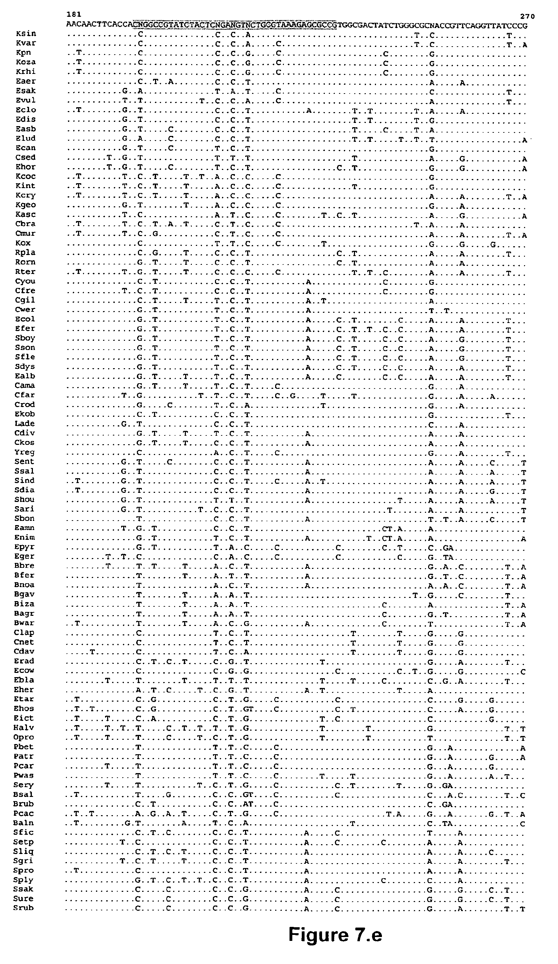

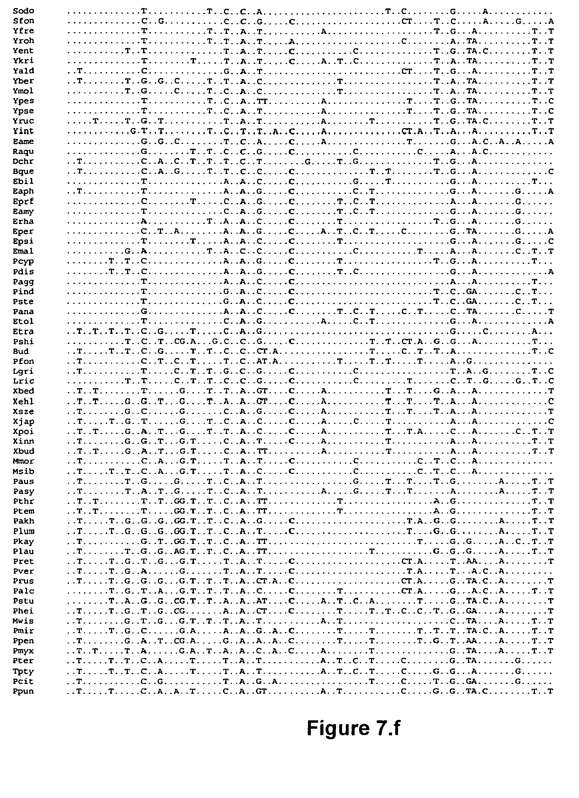

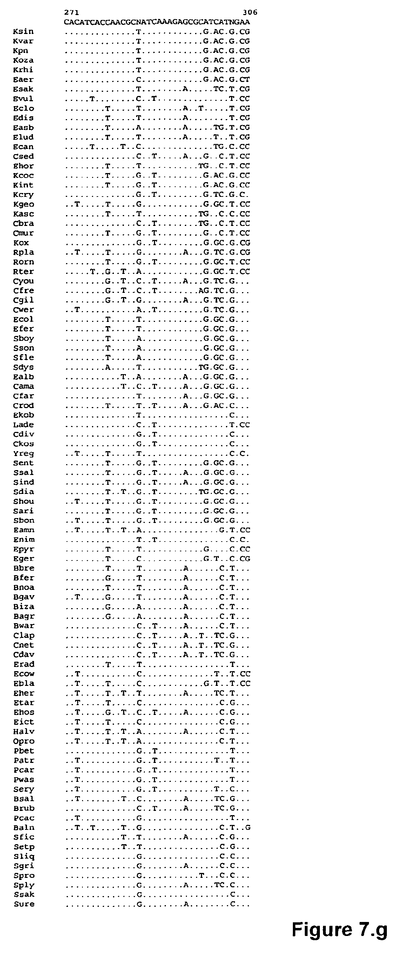

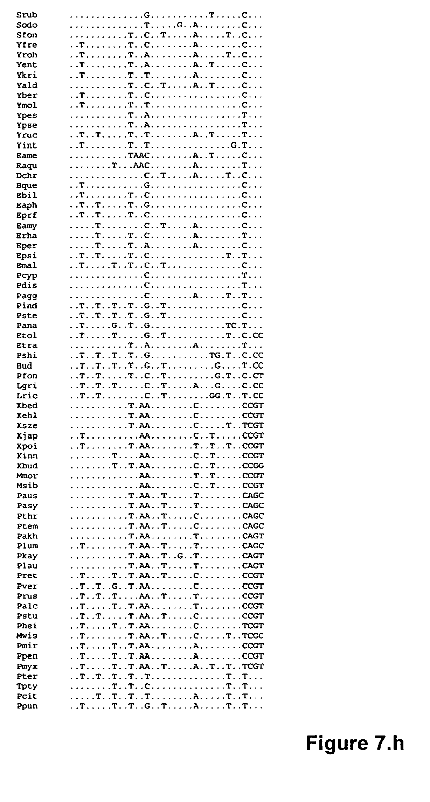

- Figure 7: Alignment of pyrG fragment of 306 bp in 162 species listed in Figure 1.

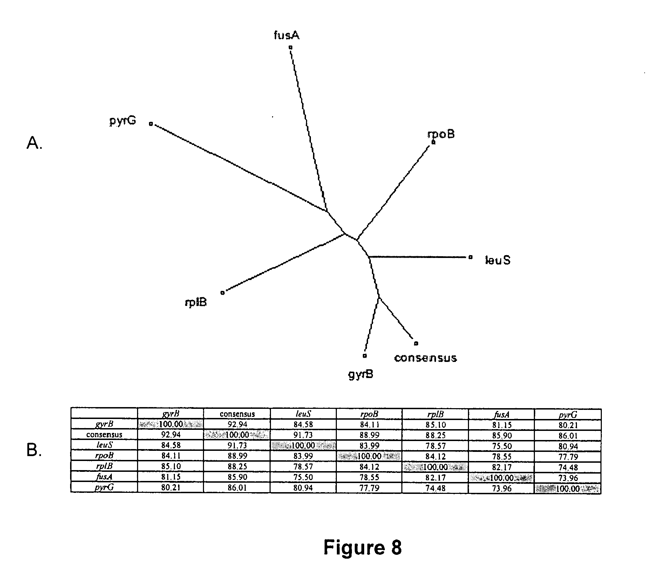

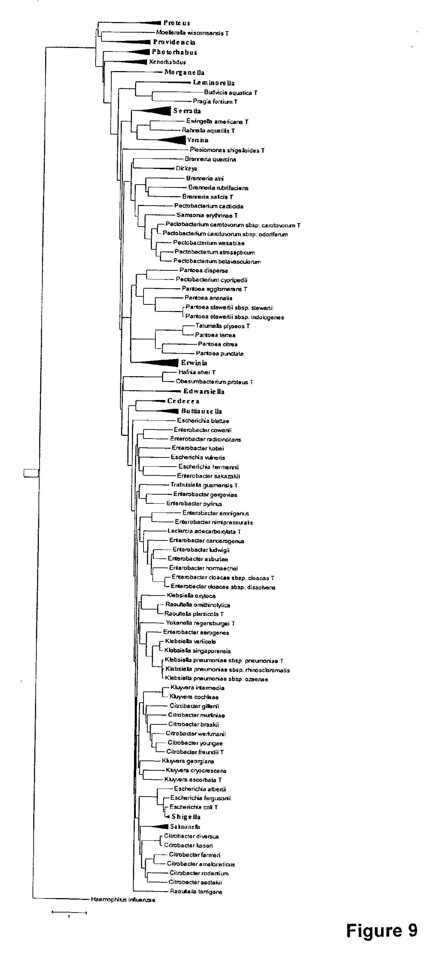

- Figure 8: Tree of trees (A.) derived from the matrix of the Pearson correlation coefficients (B.)- Figure 9: Consensus tree based on the distance average of six genes. Genera represented by a black arrow are monophyletic and were condensed into a single triangle.
- Figure 10. Histogram of the nucleotidic diversity average of the 6 genes (Pi) for given genera.
- Figure 11: Diversity and polymorphism of the 6 genes in the Enterobacteriaceae family

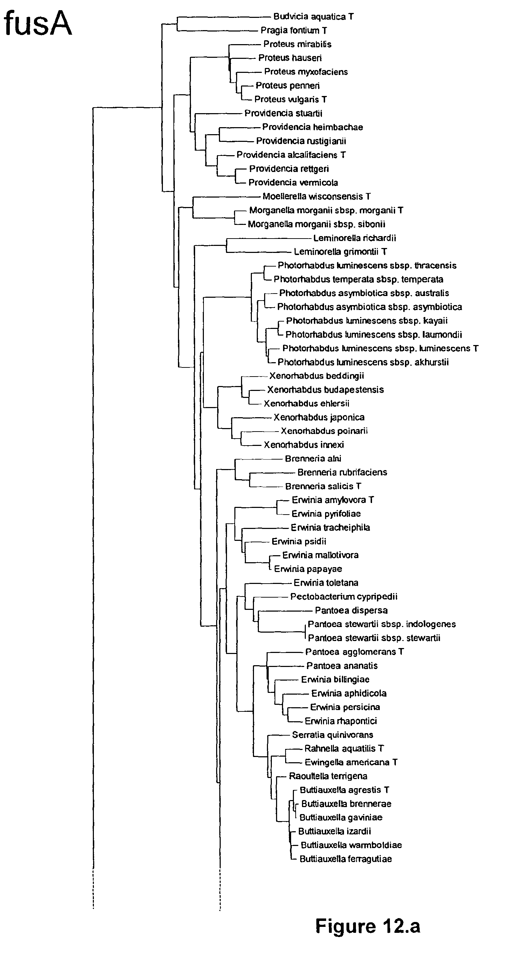

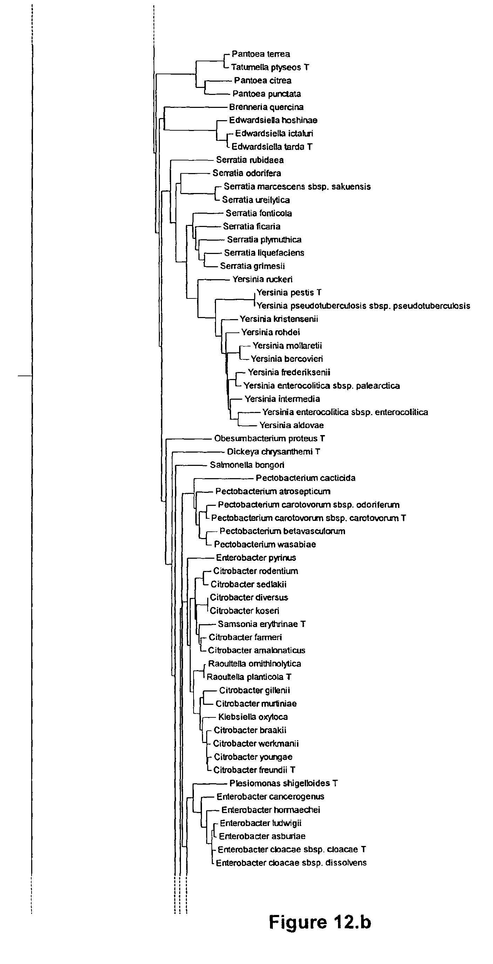

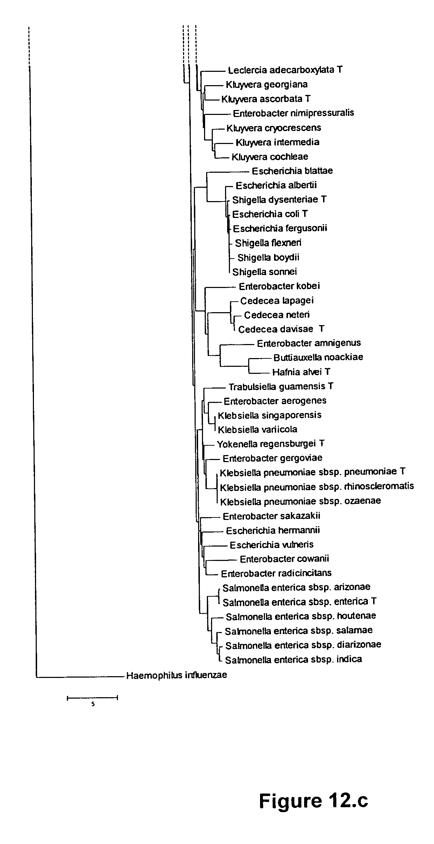

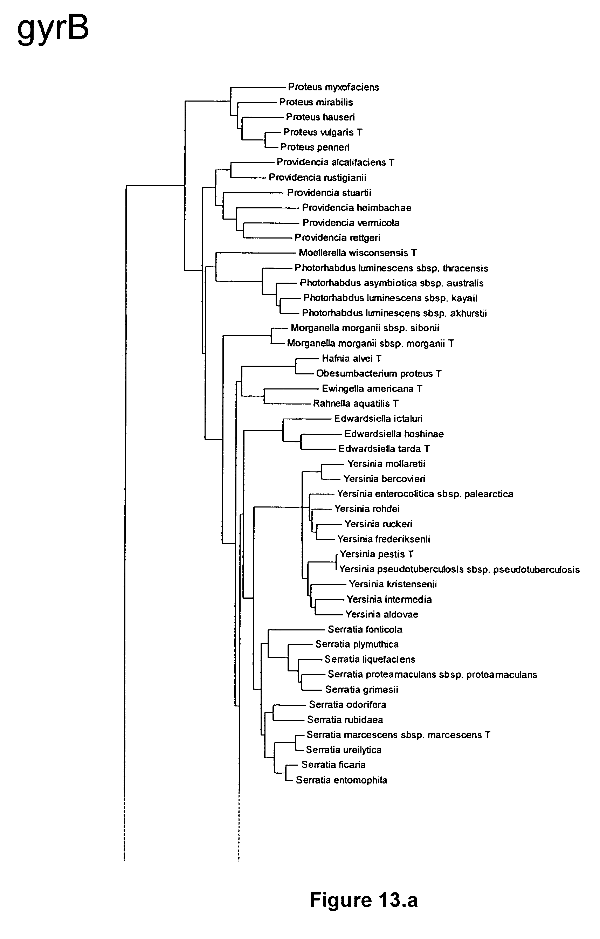

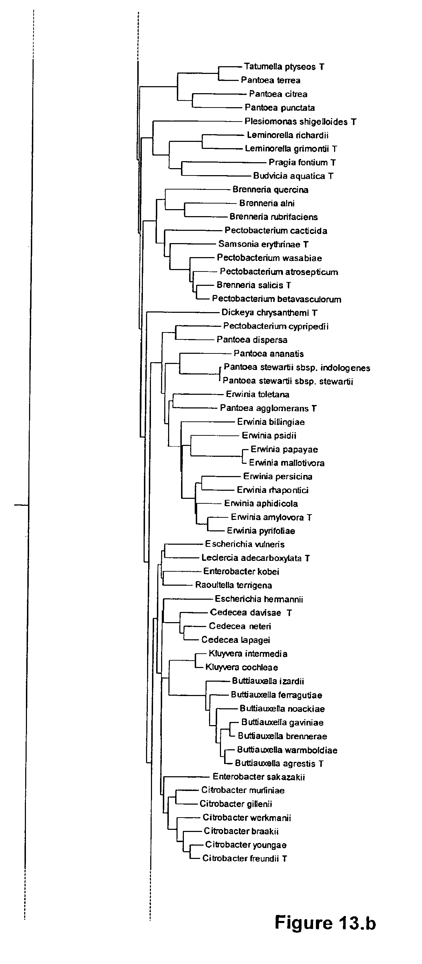

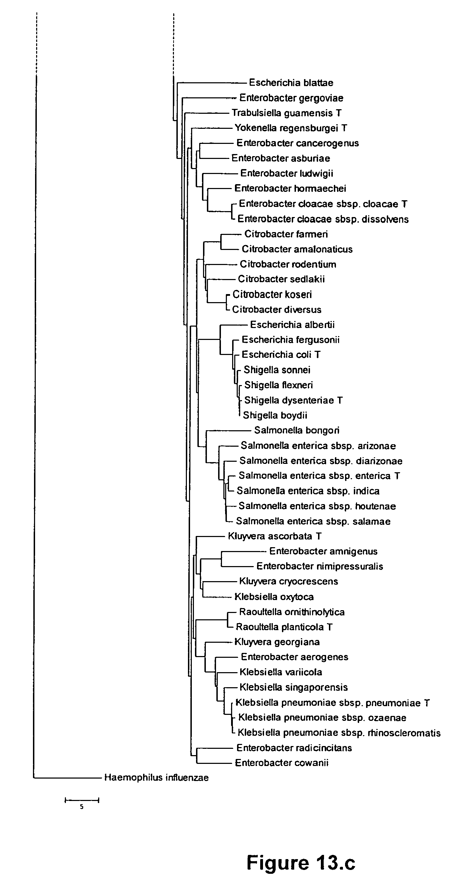

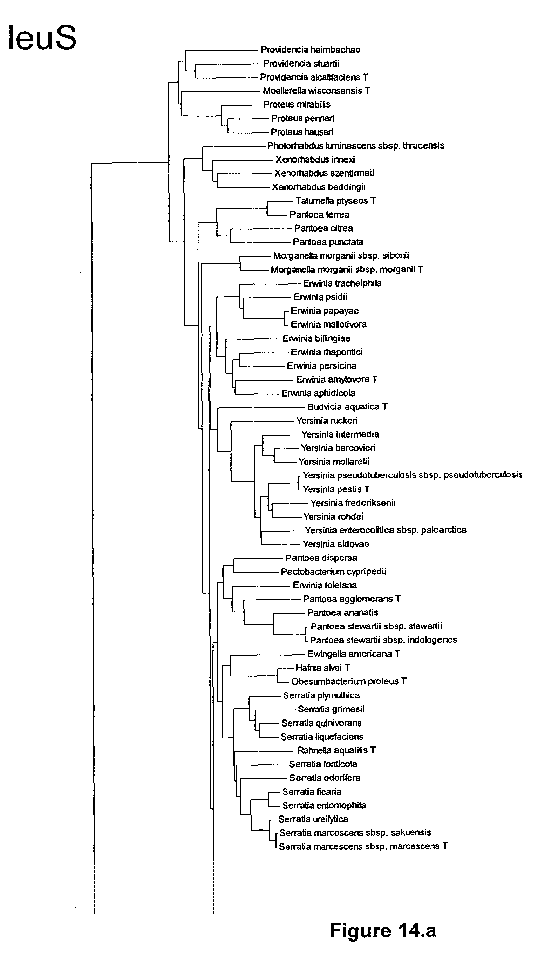

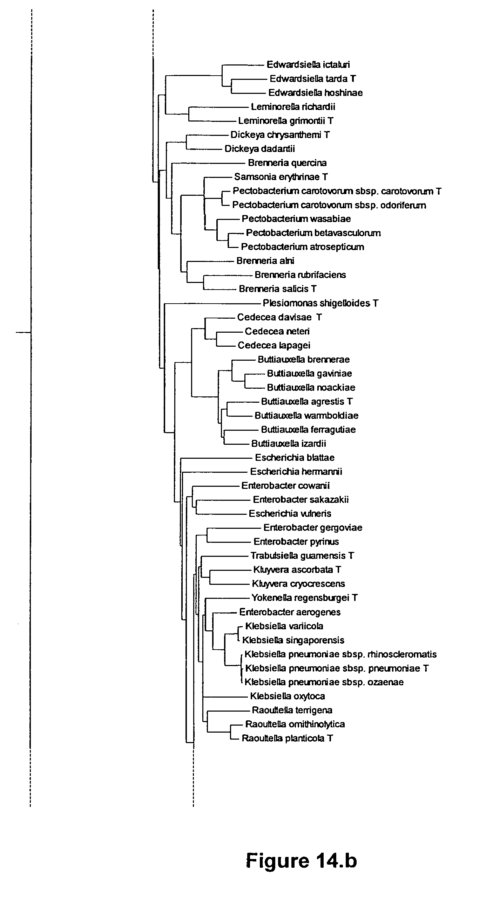

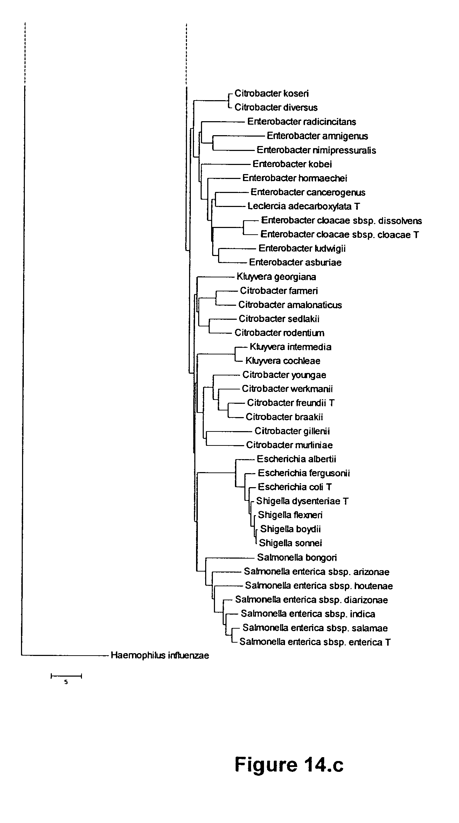

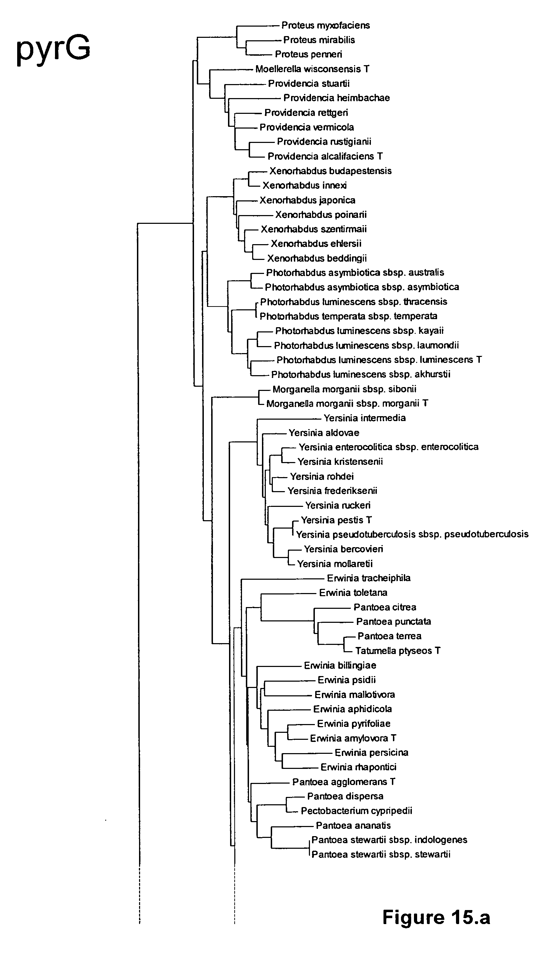

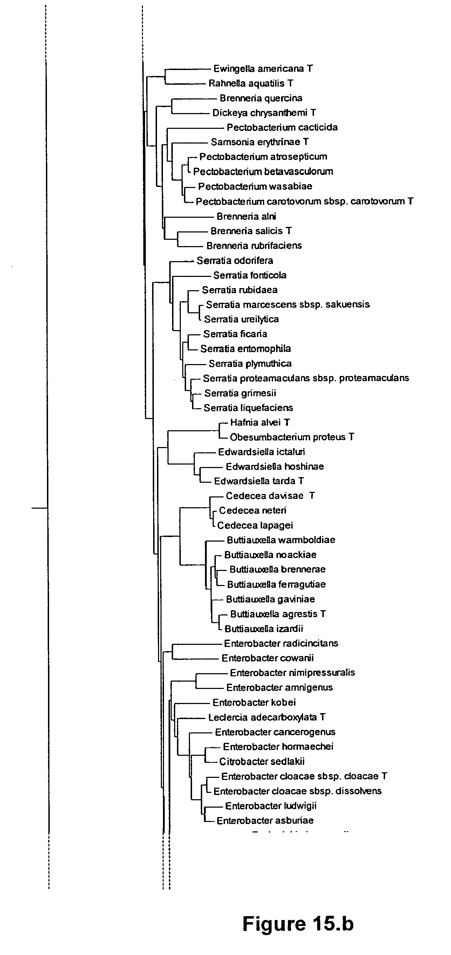

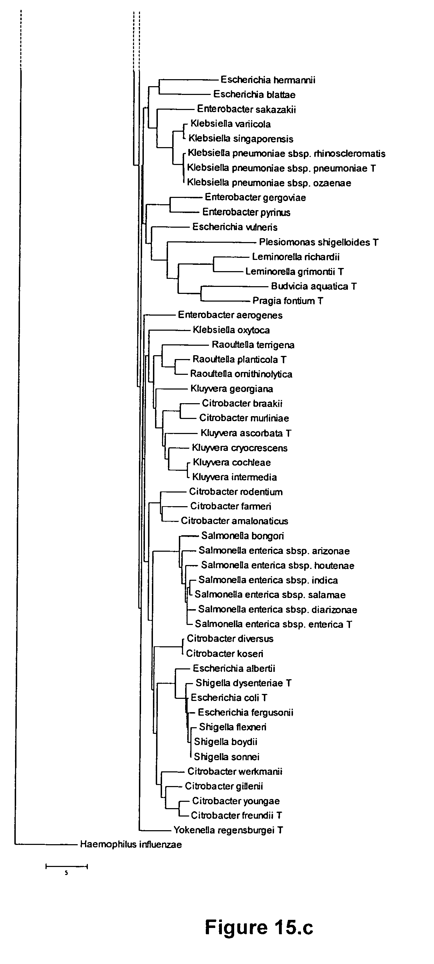

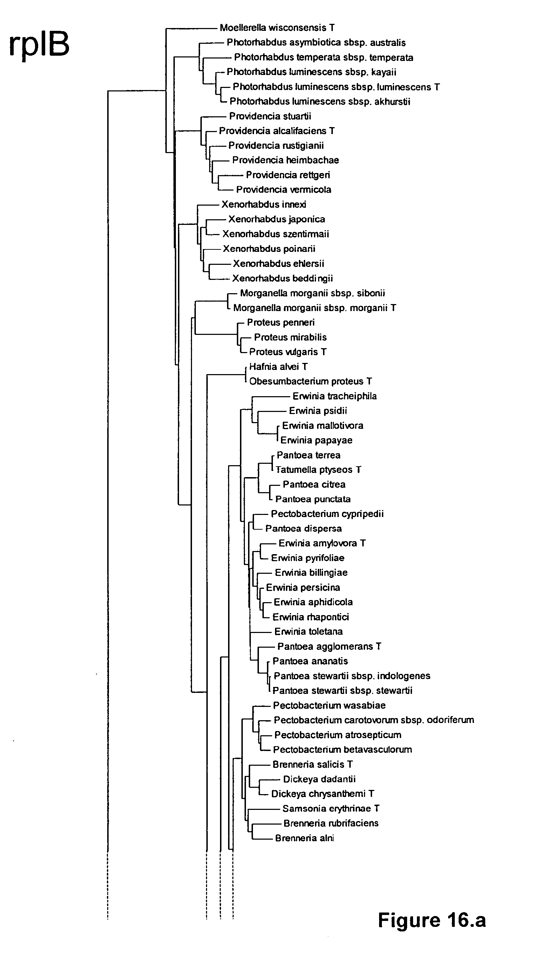

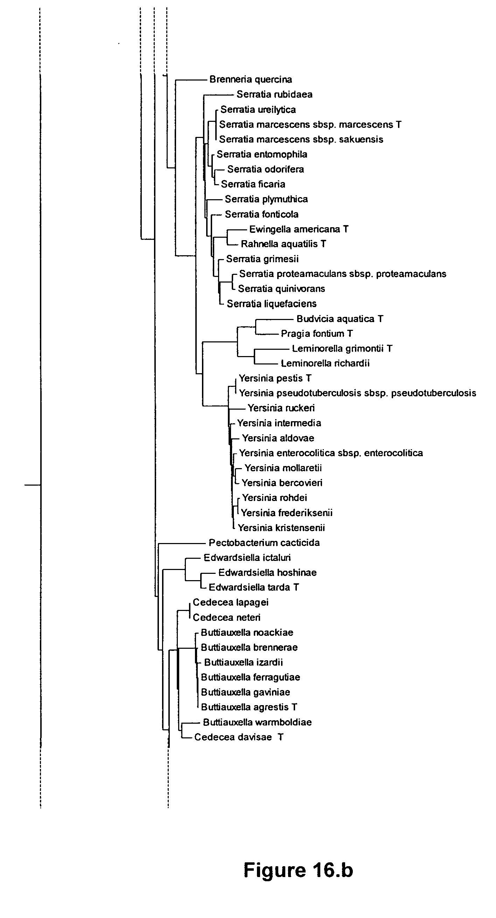

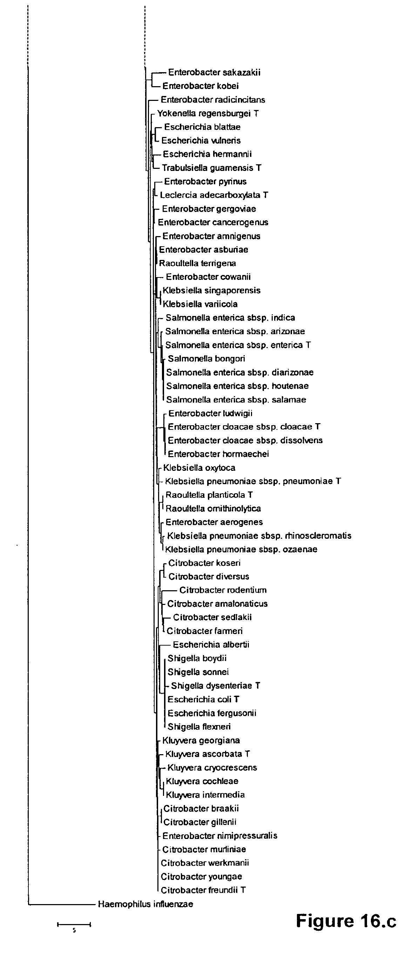

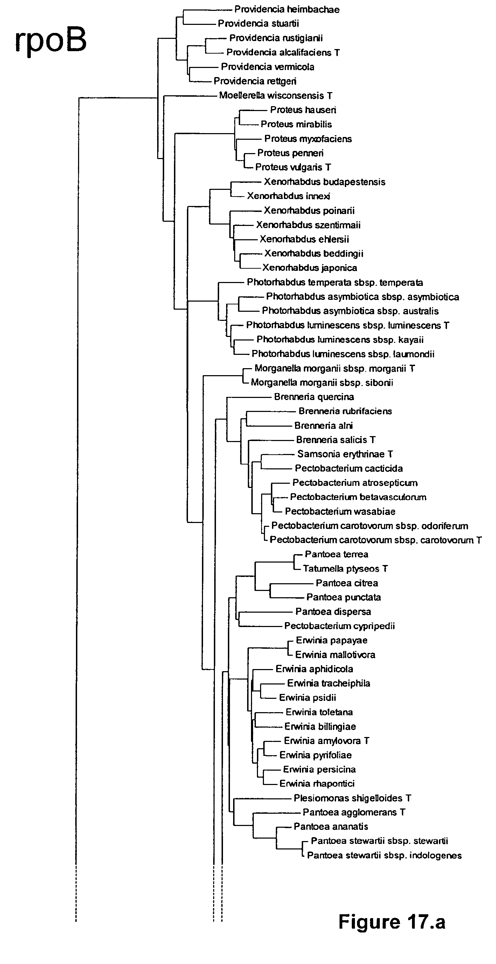

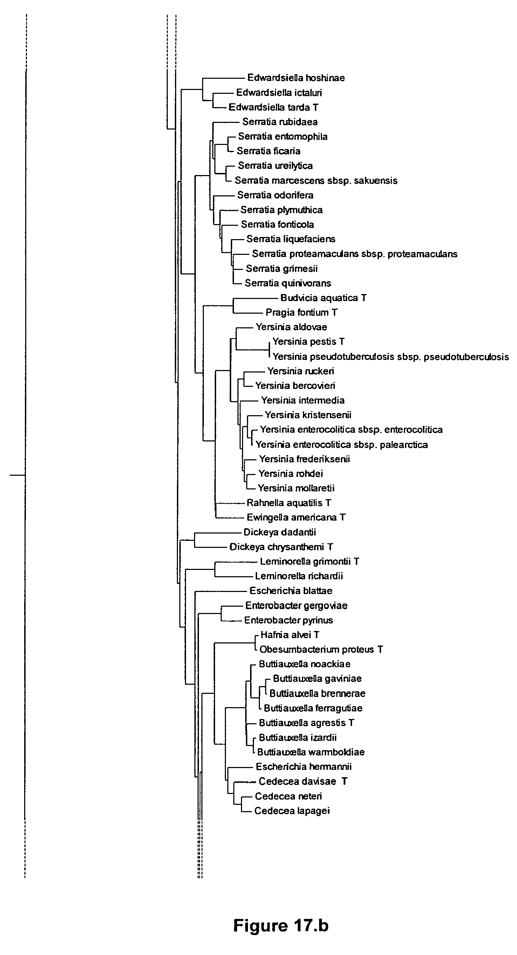

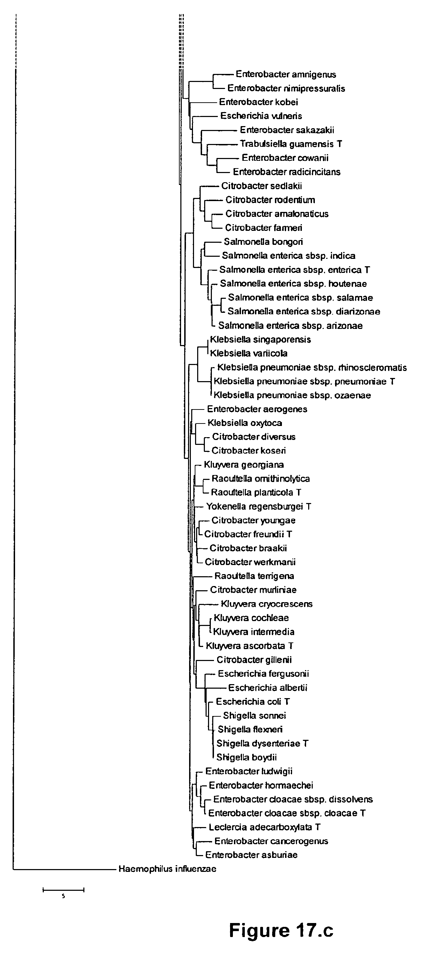

- Figures 12-17 Neighbor-joining trees based on sequences of *fusA* (Figure 12, *gyrB* (Figure 13), *leuS* (Figure 14), *pyrG* (Figure 15), *rplB* (Figure 16) and *rpoB* (Figure 17).

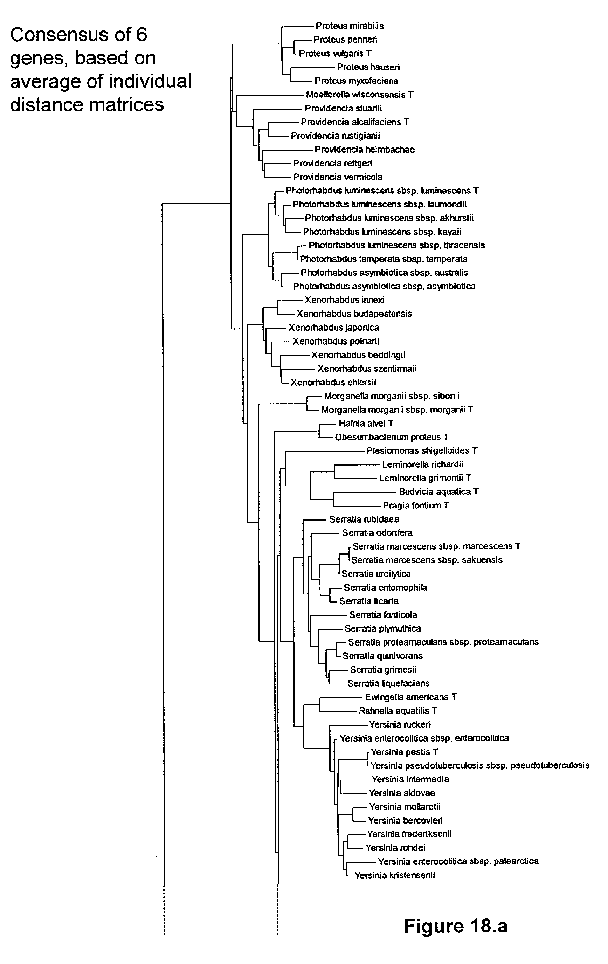

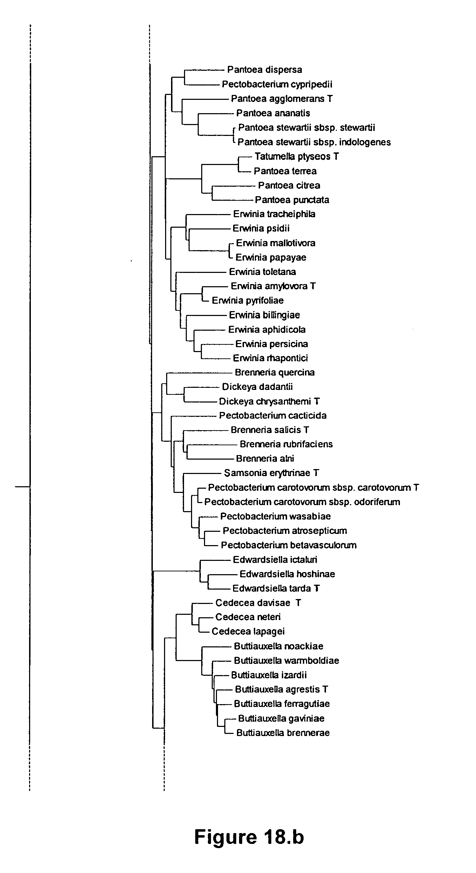

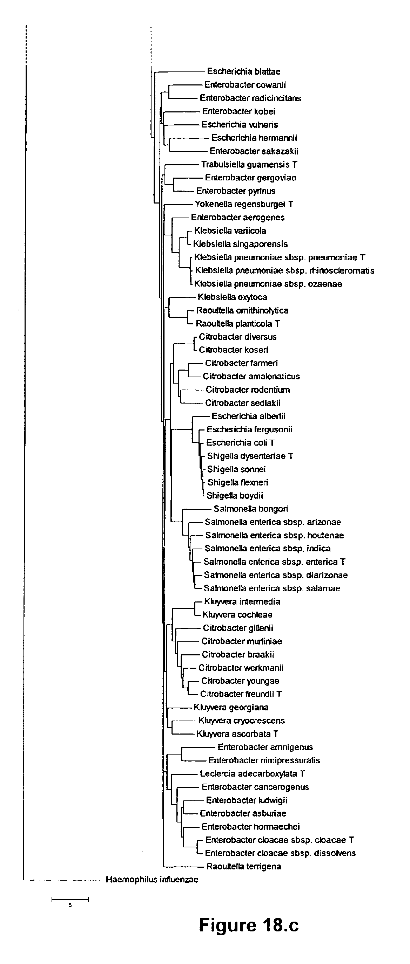

- Figure 18: The consensus tree was generated with a matrix of an average of genetic distances of the six genes.

The following examples illustrate the invention but in no way limit it.

### EXAMPLE 1: Material and Methods

**Bacterial strains.** A collection of 171 strains representing 171 species and subspecies and belonging to 37 genera has been studied (Figure 1). All of these strains are the type strain of the species or subspecies except for two strains (*Yersinia pestis* CO92 and *Shigella dysenteriae* Sd197), for which the genome is sequenced and the sequences obtained from the NCBI website.

**Total DNA extraction.** Bacterial strains were grown on tryptone-casein-soy broth agar during 18 hours at 37°C. One colony was resuspended in 200 µl of purify water and the suspension placed at 96°C for ten min in order to lyse cells. After a centrifugation at 13,000 rpm for 5 min, supernatants was collected and frozen until use. For *Serratia* species, DNA is extracted by PROMEGA kit according to the recommendations of the manufacturers.

**PCR primers.** Primers are derived from those designed by Santos and Ochman (21). These primers correspond to conserved parts of genes that are present in single copy in 95% of sequenced genomes. They are divided in two parts, in 5' the consensus part with no degeneracy and in 3' the degenerated part. To make these primers more robust in amplifying *Enterobacteriaceae,* the number of degeneracy has be decreased by fine-tuning the primer sequences based on the alignment of the corresponding gene of four completely sequenced genomes of *Enterobacteriaceae (Escherichia coli, Salmonella enterica, Klebsiella pneumoniae, Y. pestis)* (Figure 1). For gene *rpoB,* primers VIC4 and VIC6 were designed (Table I)

The conditions of the amplifications are specified in the Table II bellow.

**Table II: PCR conditions for amplification with the primers of Table I**

| Genes | PCR cycles |
|---|---|
| *GyrB* | 2 min 94°C, (1 min 94°C, 1 min 60°C, 1 min 72°C)x10, (1 min 94°C, 1 min 50°C, 1 min 72°C)x21, 5 min 72°C primers only used for sequencing |
| *RpoB* | 4 min 94°C, (30s 94°C, 30s 50°C, 30s 72°C)x30, 5 min 72°C |
| *FusA* | 2 min 94°C, (1 min 94°C, 1 min 60°C, 1 min 72°C)x10, (1 min 94°C, 1 min 50°C, 1 min 72°C)x21, 5 min 72°C |
| *LeuS* | 2 min 94°C, (1 min 94°C, 1 min 60°C, 1 min 72°C)x10, (1 min 94°C, 1 min 50°C, 1 min 72°C)x21, 5 min 72°C |
| *RplB* | 2 min 94°C, (1 min 94°C, 1 min 60°C, 1 min 72°C)x10, (1 min 94°C, 1 min 50°C, 1 min 72°C)x21, 5 min 72°C |
| *PyrG* | 2 min 94°C, (1 min 94°C, 1 min 60°C, 1 min 72°C)x10, (1 min 94°C, 1 min 50°C, 1 min 72°C)x21, 5 min 72°C |

**DNA sequencing.** PCR cycles used for amplification are shown in Table 2. The 50 µl PCR mixtures contained 0.2 µM of each primer, 100 µM of each dNTP, 20 mM Tris-HCl (pH 8.4), 50 mM KCI, 1.5 mM MgCl₂, 0.85 U Taq DNA polymerase (Invitrogen) and 2 µl of total DNA. Amplification products were checked by agarose gel electrophoresis (1%) and ethidium bromide staining. Two molecular size markers were used, 1 kb and 100 bp DNA ladder (Biolabs). PCR products are purified by ultrafiltration (Millipore).

Both strands of the purified PCR products were sequenced by using the Big Dye Terminator Ready Reaction version 3.1 (Perkin-Elmer). The 10 µl sequence reaction solutions contained 1.5 µl of Big Dye Sequencing buffer, 0.3 µM of each primer and 1 µl of Ready Reaction Premix. The reaction cycle was 1 min 96°C and 25 cycles of 10 s at 96°C, 5 s at 50°C and 4 min at 60°C. Reagent excess was eliminated by ethanol precipitation. Analysis of the sequence reaction products were performed on a ABI-3700 automated DNA sequencer. Primers used for sequencing were the same as for PCR amplification except for *gyrB,* for which internal primers were used (SEQ ID NO:14 and SEQ ID NO:15).

**Data analysis**. Edition and analysis of chromatogram traces was performed using BioNumerics v4.5. Each base of the selected template region was confirmed by at least two chromatograms (forward and reverse); if there were ambiguities for a sequence, this sequence was repeated. Neighbour-joining trees and Pearson correlation analyses were done using BioNumerics v4.6. Nucleotide diversity and amounts of polymorphisms were calculated using DNAsp version 4 (20). The matrix used to design the consensus tree was an average of the matrix of the single gene and was calculated with BioNumerics v4.5.

### EXAMPLE 2: Results and Discussion

**Sequence details.** Multiple sequence alignments revealed no insertion/deletion (indel) events for *fusA, leuS, pyrG* and *rpoB.* For gene *rplB,* an insertion of two codons for *Serratia plymuthica* was identifed. For *gyrB,* there was one deletion of one codon in *Plesiomonas shigelloides,* and one insertion of two codons for 10 strains that are *Brenneria alni, Brenneria quercina, Brenneria rubrifaciens, Brenneria salicis, Dickeya chrysanthemi, Pectobacterium atrosepticum, Pectobacterium betavasculorum, Pectobacterium cacticida, Pectobacterium wasabiae* and *Samsonia erythrinae.* Among these 10 strains, nine are grouped in *gyrB* tree and *D. chrysanthemi* forms a single branch.

The most conserved gene was *rplB,* with 41.14% polymorphic sites, whereas the most variable was *leuS,* with 61.53% polymorphic sites. The nucleotide diversity indices (average number of nucleotide differences per site between two randomly-selected strains, Pi) were 10.2% and 22.7% for *rplB* and *leuS,* respectively. The average nucleotide diversity over the six genes was 15.25%. The maximal divergence among species ranged from 18.03% (for *rpoB*) to 28.48% (*leuS*). In view of the great ecological behaviour differences of *Enterobacteriaceae* species, this very high amount of nucleotide diversity was expected. Non-synonymous changes were rare, with a Ks/Ka ratio upper to 10 for all genes, revealing a strong selection against amino acid changes, as typically observed for housekeeping genes (Figure 11).

**Congruence of gene trees.** The congruence between single gene trees and the consensus was tested. For this purpose, a Pearson correlation coefficient between the genetic distances of the six genes and the consensus tree was calculated. A tree of trees derived from this matrix was then designed (Figure 8). The consensus is taken as the reference tree, the tree based on *gyrB* sequences is the closest tree of consensus tree, following by *leuS, rpoB* and *rplB* trees, finally the less congruent trees are trees based on *fusA* and *pyrG* sequences. Despite this very good correlation between the trees, if the topology of each tree was looked to, some incongruences were found. These incongruences are characterized by the different positions of some species in the trees and they can be explained by possible events of homologous recombination (Figures 12-18). For example, in *fusA* tree (figure 12), *E. amnigenus, B. noackiae* and *H. alvei* formed a cluster, whereas, they were separated and were respectively close to *E. nimipressularis, Buttiauxella* genus and *O. proteus* in almost all other genes. Nonetheless, phylogenies of each single gene are overall very congruent and wrong branching are eliminated in the consensus tree (Figure 9).

**Phylogeny of *Enterobacteriaceae.*** The phylogeny was based on the distance average of the six genes, with some species without sequence for some gene (but with sequence for three genes at least). The most external branches were formed by the *Proteus* genus, *M. wisconcensis* and *Providencia* genus. Remarkably, the plant associated bacteria, including, *Brenneria, Dickeya, Pectobacterium* and *Erwinia* genera, were all grouped into two neighbor clusters. On the 37 genera represented in this study, 15 appeared monophyletic, 13 were composed of one single species and 9 were polyphyletic. The genus *Brenneria* is polymorphic because the species B. *quercina* is closer to *Dickeya* genus than species of is own genus, and the *Dickeya* were absent in the publication that described *B. quercina* (10). In the same article, authors described *Pectobacterium* genus, but here *Pectobacterium* genus is polymorphic, *P. cypripedii* being closer to *Pantoea dispersa* (also absent in Hauben *et al.* paper). The Pantoea genus is polyphyletic because of two species *Pectobacterium cypripedii* and *Tatumella ptyseos.* These two species are respectively close to *Pantoea dispersa* and *Pantoea terrea.* The average percentage of similarity for the six genes between *P. terrea* and *T. ptyseos* is 96.7%. These two *Pantoea* species were absent in the article that described *Pectobacterium cypripedii* and *Tatumella ptyseos* (10, 12). The *Escherichia* genus is divided into two clusters. The first cluster contains the **species** *Escherichia vulneris* and *Escherichia hermanii,* which grouped with four species of the *Enterobacter* genus *(E. cowanii, E radicincitans, E. kobei* and *E*. *sakazakii). E. albertii, E. fergusonii, E. coli* and *the Shigella* genus compose the second cluster. Paradis et al. found the same two clusters with two different genes (19). The *Enterobacter* genus was very heterogeneous, with a high level of nucleotide diversity. It is separated into 4 groups. One group is the above-described group made of four *Enterobacter* cluster and two species of *Escherichia. Enterobacter gergoviae* and *Enterobacter pyrinus* form another group with *Trabulsiella guamensis* inside. The remaining species of this genus, including the type species *Enterobacter cloacae,* form a group in which we also found *Leclercia adecarboxylata.* Of note, *T. guamensis* and *L. adecarboxylata* (17, 22) were defined based on DNA-DNA hybridization and phenotypic characteristics, and these methods do not appear reliable to define relationships between unconnected taxa. Finally, *Enterobacter aerogenes,* in agreement with previous reports (1), was closer to *Klebsiella pneumoniae* than to *Enterobacter* species. In 2001, the genus *Klebsiella* was divided in two genera, *Klebsiella* and *Raoultella* (4). It is obvious that *Klebsiella* genus is polyphyletic but actually the new *Raoultella* genus also. In the consensus tree (figure 9), it is clearly seen that *K. variicola, K. singaporensis* and the three subspecies of *K. pneumoniae* (plus *E. aerogenes)* form a group separated by *Yokenella regensburgei* of the other group formed by *K. oxytoca, Raoultella ornithinolytica* and *R. planticola. Raoultella terrigena* form a single branch and this species was absent in the study of Paradis et al. and this is the reason why they wrongly concluded that the genus *Raoultella* is monophyletic (19). *Citrobacter* and *Kluyvera* contain respectively 12 and 5 species. *Citrobacter* genus is separated in two very distinct cluster both composed by six species. The cluster that contains the type species *C. freundii* is inside the *Kluyvera* genus. The second cluster is close to *Salmonella* genus

**Genus definition.** Some genera do not correspond to clear clusters. Another apparent problem in the definition of the genus is that the nucleotide heterogeneity level is very different among genera. To illustrate that, we calculated the nucleotidic diversity inside genus that contain more than two species with six sequenced genes was calculated (Figure 10). It clearly appeared that the nucleotidic diversity greatly varied among genera, ranging from 0.04 for *Klebsiella* to 0.15 for *Pantoea.* It was also observed that some genera had a higher nucleotidic diversity than a set of several genera. The set of genera *Enterobacter, Escherichia, Salmonella* taken together has a lower nucleotidic diversity than the four individual genera *Brenneria, Erwinia, Leminorella* and *Providencia.*

### Conclusion

The instant method of discriminating a single species among the *Enterobacteriaceae* family is very accurate and can be applied on related species of this family even if those species have any genomic data.

The phylogeny obtained was accurate and precise the relative positions of each species indicating, for example, that all plant associated bacteria are clustered.

Some genera were completely polyphyletic and heterogeneity of nucleotidic diversity level is very high. This results over lined the fact the genus definition in *Enterobacteriaceae* is totally unstandardized.

However the selected target regions allow an accurate discrimination and revealed an excellent correlation between single gene trees and can be used in different combinations, the minimum being the selection of only one of said target regions, with a high confidence in the identification results.

Indeed, only the following groups (pairs or triplets) of species or subspecies are not distinguished by the sequence of a single target region:

Remark: for most of these groups, it is known that the species or subspecies are very closely related or undistinguishable. Some of them are considered as strains within a single species and were elevated to species or subspecies status for medical reasons only (for example, *Klebsiella pneumoniae* subsp. *pneumoniae* / *Klebsiella pneumoniae* subsp. *ozaenae* / *Klebsiella pneumoniae* subsp. *rhinoscleromatis, or Yersinia pseudotuberculosis and Y pestis).*

For *fusA :*
- *Pantoea stewartii* subsp. *indologenes* / *Pantoea stewartii* subsp. *stewartii*
- *Citrobacter diversus* / *Citrobacter koseri*
- *Klebsiella singaporensis* /*Klebsiella variicola*
- *Klebsiella pneumoniae* subsp. *pneumoniae* / *Klebsiella pneumoniae* subsp. *ozaenae* / *Klebsiella pneumoniae* subsp. *rhinoscleromatis*

For *gyrB :* All sequenced species for this gene have different sequences.

*For leuS:* All sequenced species for this gene have different sequences.

*For pyrG :*
- *Pantoea stewartii* subsp. *indologenes* / *Pantoea stewartii* subsp. *stewartii*
- *Klebsiella pneumoniae* subsp. *Pneumoniael Klebsiella pneumoniae* subsp. *ozaenae*
- *Shigella boydii* / *Shigella sonnei*

For *rplB :*
- *Serratia marcescens* subsp. *marcescens* / *Serratia marcescens* subsp. *sakuensis*
- *Yersinia pestis* / *Yersinia pseudotuberculosis* subsp. *pseudotuberculosis*
- *Cedecea lapagei* / *Cedecea neteri*
- *Buttiauxellla agrestis* / *Buttiauxella ferragutiae* / *Buttiauxella gaviniae*
- *Enterobacter asburiae l Raoultella terrigena*
- *Klebsiella singaporensis* / *Klebsiella variicola*
- *Salmonella enterica* subsp. *diarizonae l Salmonella enterica* subsp. *houtanae l Salmonella enterica* subsp. *salamae*
- *Enterobacter cloacae* subsp. *cloacae* / *Enterobacter cloacae* subsp. *dissolvens* / *Enterobacter hormaechei*
- *Raoultella ornithinolytica* / *Raoultella planticola*
- *Shigella sonnei* / *Shigella flexneri* / *Shigella boydii* / *Escherichia coli* / *Escherichia fergusonii*
- *Kluyvera cochleae* / *Kluyvera intermedia*
- *Citrobacter braakii* / *Citrobacter gillenii*
- *Citrobacter freundii* / *Citrobacter werkmanii* / *Citrobacter youngae*

For *rpoB :*
- *Yersinia pestis* / *Yersinia pseudotuberculosis* subsp. *pseudotuberculosis*
- *Klebsiella pneumoniae* subsp. *pneumoniae* / *Klebsiella pneumoniae* subsp. *ozaenae*
- *Shigella boydii* / *Shigella dysenteriae*

### REFERENCES

1. Bascomb, S. et al., J Gen Microbiol, 1971, 66:279-95.
2. Brenner, D. 1984. Family I. Enterobacteriaceae Rahn 1937, nom. fam. cons. Opin. 15, Jud. Comm. 1958, 73; Ewing, Farmer, and Brenner 1980, 674; Judicial Commission 1981, 104, p. 408-420. *In* N. R. K. a. J. G. H. (ed.) (ed.), Bergey's manual of systematic bacteriology, vol. 1.
3. Dauga, C., Int JSyst Evol Microbiol, 2002, 52:531-47.
4. Drancourt, M. et al., Int JSyst Evol Microbiol, 2001, 51:925-32.
5. Fox, G. E. et al., Science, 1980, 209:457-63.
6. Frenzen, P. D. et al., J Food Prot, 2005, 68:2623-30.
7. Gevers, D. et al., Nat Rev Microbiol, 2005, 3:733-9.
8. Hanage, W. P. et al., BMC Biol, 2005, 3:6.
9. Hanage, W. P. et al., J Bacteriol, 2005, 187:6223-30.
10. Hauben, L. et al., Syst Appl Microbiol, 1998, 21:384-97.
11. Hedegaard, J. et al., Int J Syst Bacteriol, 1999, 49 Pt 4:1531-8.
12. Hollis, D. G. et al., J Clin Microbiol, 1981, 14:79-88.
13. InVS. 2003. Enquête nationale de prevalence des infections nosocomiales 2001. Institut national de Veille Sanitaire.
14. James, T. Y. et al., Nature, 2006, 443:818-22.
15. Kuhnert, P. et al., Microbiology, 2006, 152:2537-48.
16. Maiden, M. C. et al., Proc Natl Acad Sci USA, 1998, 95:3140-5.
17. McWhorter, A. C. et al., J Clin Microbiol, 1991, 29:1480-5.
18. Mollet, C. et al., Mol Microbiol, 1997, 26:1005-11.
19. Paradis, S. et al., Int J Syst Evol Microbiol, 2005, 55:2013-25.
20. Rozas, J. et al., Bioinformatics, 2003, 19:2496-7.
21. Santos, S. R. et al., Environ Microbiol, 2004, 6:754-9.
22. Tamura, K. et al., Curr Microbiol, 1986, 13:179-184.
23. Wertz, J. E. et al., J Evol Biol, 2003, 16:1236-48.
24. Syvänen A., Clinica Chimica Acta, 1994, 226, 225-236
25. Sambrook et al., "Molecular Cloning: A Laboratory Manual", (New York: Cold Spring Harbor Laboratory Press, 1989
26. Ausubel FM. et al., Current Protocols in Molecular Biology, Greene Publishing Assoc. and Wiley-Interscience 1987 & Suppl. 49, 2000
27. Erlich HA et al., Science, 1991, 252, 1643-1650
28. Mullis KB et al., eds, 1994, The polymerase chain reaction, Boston: Birkhäuser
29. Poitras E et al., Rev. Biol. Biotechnol., 2002, 2, 2, 2-11
30. Schena M ed., 2000, Microarray Biochip Technology. Natick, MA:Eaton Publishing
31. Lechner D et al., Curr. Opinion Chem. Biol., 2002, 6, 31-38
32. Twyman et al., Pharmacogenomics, 2003, 4, 67-79
33. Wang L. et al., Advances in experimental medicine and biology, 2007, 593, 105-116
34. Ecker J.A. et al., J. Clin. Microbiol., 2006, 44, 2921-2932
35. von Wintzingerode et al., Proc. Natl. Acad. Sci. USA, 2002, 99, 7039-7044
36. Dunbar S, Clin. Chim. Acta, 2006, 363, 71-82

## Claims

1. Method for discriminating accurately a bacterium of the *Enterobacteriaceae* family by genotyping, **characterized in that** said method comprises for detecting specifically one bacterium among at least 148 species of the 171 species of the *Enterobacteriaceae* family listed in figure 1 and in the herewith attached sequence listing:
A- the isolation of at least a sequence, or a fragment thereof, comprising a target region having a length comprised between 300 and 650 bp and selected among internal portions of a set of genes selected in the group consisting of *gyrB, rpoB, fusA, leuS, rplB* and *pyrG* of *Enterobacteriaceae,* or a fragment of said target region of at least 15 bp, preferably of 40-150 bp and
B- determining the species of said bacterium.

2. Method according to claim 1, **characterized in that** step A comprises for detecting specifically one bacterium among said 148 species, the isolation of a plurality of sequences or fragments thereof comprising target regions having a length comprised between 300 and 650 bp, said target regions being selected among an internal portion of a set of at least two genes selected in the group consisting of *gyrB* and *leuS* of *Enterobacteriaceae* or a fragment of said target regions of at least 15 bp, preferably of 40-150 bp.

3. Method according to claim 2, **characterized in that** step A comprises for detecting specifically one bacterium among said 171 species of the *Enterobacteriaceae* family listed in figure 1, the further isolation of a plurality of sequences, or fragments thereof, comprising target regions having a length comprised between 300 and 650 bp, said target regions being selected among an internal portion of at least one further gene selected in the group consisting of *rpoB, fusA, rplB* and *pyrG* of *Enterobacteriaceae* or a fragment of said target regions of at least 15 bp, preferably of 40-150 bp.

4. Method according to any one of claims 1 to 3, **characterized in that** the target regions are selected in the group consisting of:
* the target region of *gyrB* corresponding to a fragment of 417-420 bp of *gyrB* CDS (consensus sequence SEQ ID NO:1 and SEQ ID NO: 26-177),
* the target region of *rpoB* corresponding to a fragment of 501 bp of *rpoB* CDS (consensus sequence SEQ ID NO:2 and SEQ ID NO: 178-345),
* the target region of *fusA* corresponding to a fragment of 633 bp of *fusA* CDS (consensus sequence SEQ ID NO:3 and SEQ ID NO: 346-511),
* the target region of *leuS* corresponding to a fragment of 642 bp of *leuS* CDS (consensus sequence SEQ ID NO:4 and SEQ ID NO: 512-659),
* the target region of *rplB* corresponding to a fragment of 333-339 bp of *rplB* CDS (consensus sequence SEQ ID NO:5 and SEQ ID NO: 660-821) and
* the target region of *pyrG* corresponding to a fragment of 306 bp of *pyrG* gene (consensus sequence SEQ ID NO:6 and SEQ ID NO: 822-983).

5. Method according to any one of claims 1 to 4, **characterized in that** said fragments of said target regions are selected in the group consisting of fragments of said target regions of at least 15 bp, preferably of 40-150 bp.

6. Method according to claim 5, **characterized in that** said fragments of said target regions are selected in the group consisting of:
- a fragment of 40 bp defined by the positions 336 to 375 of *gyrB* sequences SEQ ID NO:1 or SEQ ID NO:26-177.
- a fragment 40 bp defined by the positions 416 to 455 of *rpoB* sequences SEQ ID NO:2 or SEQ ID NO: 178-345.
- a fragment of 40 bp defined by the positions 480 to 519 of *fusA* sequences of SEQ ID NO:3 or SEQ ID NO:346-511.
- a fragment of 40 bp defined by the positions 336 to 375 of *leuS* sequences of SEQ ID NO:4 or SEQ ID NO:512-659 and
- a fragment of 40 bp defined by the positions 194 to 233 of *pyrG* sequences of SEQ ID NO:6 or SEQ ID NO:822-983.

7. Method according to any one of claims 1 to 6, **characterized in that** the target regions or fragments thereof are selected in the group consisting of:
. Set N°1 (for detecting at least 148 species of the 171 listed in figure 1 and in the here with attached sequence listing) consisting of target regions as defined above of any of the *gyrB, rpoB, fusA, leuS, rplB and pyrG* CDS,
. **Set N°2** (for detecting at least **148** species of the 171 listed in figure 1 and in the here with attached sequence listing) consisting of the combination of target regions as defined above of the couple *gyrB* and *rpoB,*
. **Set N°3** (for detecting the 171 species listed in figure 1 and in the here with attached sequence listing) consisting of the combination of target regions as defined above of the triplet *leuS, rplB* and *fusA,*
. **Set N°4** (for detecting the 171 species listed in figure 1 and in the here with attached sequence listing) consisting of the combination of target regions of the triplet *leuS, rplB* and *pyrG,*
. **Set N° 5** (for detecting the 171 species listed in figure 1 and in the here with attached sequence listing) consisting of the combination of target regions of the quadruplet *leuS, rplB, pyrG* and *fusA,*
. **Set N°6** (for detecting the 171 species listed in figure 1 and in the here with attached sequence listing) consisting of the combination of target regions of the sextuplet *gyrB, rpoB, fusA, leuS, rplB* and *pyrG* or fragments thereof of 40-150 bp,
. **Set N°7** (for detecting the 171 species listed in figure 1 and in the here with attached sequence listing) consisting of combination of the fragments of 40 bp of the target regions of the quintuplet *gyrB, rpoB, fusA, IeuS,* and *pyrG,* said fragments of 40 bp corresponding to the following positions: *gyrB:* from positions 336 to 375 of SEQ ID NO:1 and SEQ ID NO:26-177, *rpoB* from positions 416 to 455 of SEQ ID NO:2 and SEQ ID NO:178-345, *fusA* from positions 480 to 519 of SEQ ID NO:3 and SEQ ID NO:346-511, *leuS* from positions 336 to 375 of SEQ ID NO:4 and SEQ ID NO:512-659 and *pyrG* from positions 194 to 233 of SEQ ID NO:6 and SEQ ID NO:822-983.

8. Method according to any one of claims 1 to 7, **characterized in that** step A of isolation comprises:
(A1) Obtaining a test sample that contains genomic nucleic acids from bacteria present in said sample and
(A2) Amplifying at least one target region as defined in claims 1 to 7 to generate isolated products.

9. Method according to claim 8, **characterized in that** the step (A2) of amplifying said at least one target region, or a fragment thereof, is performed by a method selected from the group consisting of polymerase chain reaction, (PCR), ligase chain reaction (LCR), nucleic acid-sequence-based amplification (NASBA), cycling probe technology (CPT), nested PCR and multiplex PCR.

10. The method according to claim 8 or to claim 9, **characterized in that** the step (A2) of amplifying said at least one target region, or a fragment thereof, is performed with primers having a length comprised between 10 and 40 nucleotides, preferably of 18-35 nucleotides, said primers, optionally labelled or modified, being able to amplify the target region or a fragment thereof.

11. The method according to claim 10, **characterized in that** the primers are selected in the group consisting of the primers of SEQ ID NO:12-13 and SEQ ID NO:16-25.

12. The method according to any one of claims 1 to 11, **characterized in that** the step B of determining the species of said bacterium comprises:
(B1) Analysing the isolated product obtained in step A, in order to discriminate among possible sequences and
(B2) Determining the species of the *Enterobacteriaceae.*

13. The method of claim 12, **characterized in that** step B of determining the species of *Enterobacteriaceae* comprises the step (B 1) of analysing the isolated product obtained in step A by sequencing at least one target region or a fragment thereof and the step (B2) of determining the species of the *Enterobacteriaceae* by comparing said at least one target region or a fragment thereof with the sequences of the repertory of nucleic acids of the 171 species of *Enterobacteriaceae* family listed in figure 1 (SEQ ID NO:1-6 and SEQ ID NO:26 to SEQ ID NO:983).

14. The method of claim 13, **characterized in that** for discriminating the species *Enterobacter cloacae,* the step (B1) is performed by analysis of a fragment of 43 nucleotides between positions 282 and 324 of the *gyrB* target region (SEQ ID NO:1) and step (B2) is performed by comparing said fragment with the corresponding fragments of the sequences of the repertory of nucleic acids of the 171 species of the *Enterobacteriaceae* family listed in figure 1 (SEQ ID NO:26-177).

15. The method of claim 13, **characterized in that** for discriminating the species *Klebsiella pneumoniae* from the other *K. pneumoniae* subspecies, the step (B1) is performed by analysis of a fragment of 39 nucleotides between positions 91 and 129 of the *fusA* target region (SEQ ID NO:3) and analysis of a fragment between the positions 378 and 402 of the *leuS* target region (SEQ ID NO:4) and step (B2) is performed by comparing said two fragments with the corresponding fragments of the sequences of the repertory of nucleic acids of the 171 species of the *Enterobacteriaceae* family listed in figure 1 (SEQ ID NO:346-511 and SEQ ID NO:512-659).

16. The method of claim 12, **characterized in that** step B of determining the species of *Enterobacteriaceae* comprises the step (B1) of analysing the isolated product obtained in step A by hybridizing at least one target region or a fragment thereof with at least one oligonucleotide and preferably a plurality of oligonucleotides corresponding to fragments of the target regions as defined in claims 1 to 7, each of said oligonucleotides having a sequence of a length of at least 15 nucleotides, and preferably of 18-25 nucleotides corresponding to consecutive nucleotides of the sequences SEQ ID NO:1-6 or SEQ ID NO:26-983 and the step (B2) of determining the species of the *Enterobacteriaceae* by determining the identity of the isolated products hybridized to the different oligonucleotides in reference to the sequences of the repertory of nucleic acids of the 171 species of the *Enterobacteriaceae* family listed in figure 1.

17. The method of claim 12, **characterized in that** step B of determining the species of *Enterobacteriaceae* comprises the step (B1) of analysing said isolated products obtained in step A by hybridizing at least one isolated product, or a fragment thereof, with at least one set of four oligonucleotides, each oligonucleotide corresponding to a fragment of an odd number of consecutive nucleotides of the target regions as defined in claims 1 to 7, having a sequence of a length comprised between preferably 19-29 and corresponding to consecutive nucleotides of the sequences SEQ ID NO:1-6 or SEQ ID NO:26-983, each set of four oligonucleotides differing only at the nucleotide being interrogated in the target region, which is generally positioned in the middle of the oligonucleotide sequence and the step (B2) comprises detecting the isolated products which are hybridized with the different oligonucleotides and determining the species by comparing with the sequences of the repertory of nucleic acids of the 171 species of the *Enterobacteriaceae* family listed in figure 1.

18. The method of claim 12, **characterized in that** step B of determining the species of the *Enterobacteriaceae* family comprises the step (B1) of analysing the isolated products obtained in step A by hybridizing at least one isolated product, or a fragment thereof, with at least one oligonucleotide and preferably a plurality of oligonucleotides, each of said oligonucleotides corresponding to a fragment of the target regions as defined in claims 1 to 7, having a sequence of a length of at least 15 nucleotides, and preferably of 18-25 nucleotides corresponding to consecutive nucleotides of the sequences SEQ ID NO:1-6 or SEQ ID NO:26-983 and ending (3'extremity) either one nucleotide upstream a SNP site, or at a SNP site and the step (B2) comprises detecting the isolated products by extending immobilized oligonucleotides that anneal to said isolated products, immediately adjacent to the SNP site using a DNA polymerase and labelled nucleotide analogues complementary to the nucleotide at the variable SNP site and determining the species by comparing the extension products obtained with the sequences of the repertory of nucleic acids of the 171 species of the *Enterobacteriaceae* family listed in figure 1.

19. The method according to claim 18, **characterized in that** the step B consists, for discriminating the species *E*. *coli,* in identifying the nucleotide at position 87 of the *leuS* target region (SEQ ID NO:4).

20. The method according to claim 18, **characterized in that** the step B consists, for discriminating the genus *Buttiauxella* from other *Enterobacteriaceae,* in identifying the nucleotide at position 232 of the *leuS* target region (SEQ ID NO:4).

21. The method according to claim 18, **characterized in that** step B consists, for discriminating *Y. pestis,* in identifying both the nucleotide at position 384 of the *fusA* target region (SEQ ID NO:3) and the nucleotide at position 240 of the *gyrB* target region (SEQ ID NO:1).

22. The method according to claim 18, **characterized in that** step B consists, for discriminating *Salmonella enterica* subsp. *enterica,,* in identifying both the nucleotide at position 6 of the *pyrG* target region (SEQ ID NO:6) and the nucleotide at position 120 of the *gyrB* target region (SEQ ID NO:1).

23. DNA array comprising at least one polynucleotide selected among:
a) the polynucleotides of sequences SEQ ID NO:1-6, 7-11 and 26-983 or fragments thereof of at least 15 consecutive nucleotides and their reverse-complementary sequences
b) a fragment of at least 15-30 consecutive nucleotides of a polynucleotide defined in a)
c) a polynucleotide of at least 15-30 base pairs that hybridizes selectively under stringent conditions with a polynucleotide of a)
d) a plurality of fragments having an odd number of nucleotides having a sequence of a length comprised between 19-29 nucleotides and corresponding to consecutive nucleotides of the polynucleotides defined in a) except that their central nucleotide is N and their reverse-complementary sequences
e) a plurality of fragments having a sequence of a length comprised between 15 and 30 nucleotides corresponding to consecutive nucleotides of the polynucleotides defined in a), said fragments ending either one nucleotide upstream a SNP site or at a SNP site.

24. Nucleic acids selected among: nucleic acids of sequences SEQ ID NO:1-6 and 26-983 and their fragments of SEQ ID NO:7-11 and reverse-complementary sequences thereof.

25. Oligonucleotide, **characterized in that** it consists of a fragment of 15-150 nucleotides corresponding to consecutive nucleotides of the target regions (SEQ ID NO:1-6 and 26-983).

26. Oligonucleotide according to claim 25, **characterized in that** it consists of a fragment of at least 15 nucleotides, preferably of 15-30 nucleotides and most preferably of 18-25 nucleotides corresponding to consecutive nucleotides of the target regions as defined in claims 1 to 7 and more precisely of sequences SEQ ID NO:1-6 and SEQ ID NO:26-983.

27. Oligonucleotide according to claim 25, **characterized in that** it consists of a fragment of an odd number of nucleotides, has a length comprised between preferably of 19-29 nucleotides corresponding to consecutive nucleotides of the sequences SEQ ID NO:26-983, the central nucleotide being N.

28. Oligonucleotide according to claim 25, **characterized in that** it consists of a fragment of 15-30 nucleotides corresponding to consecutive nucleotides of the sequences SEQ ID NO:1-6 and SEQ ID NO:26-983, each oligonucleotide ending (3'extremity) either one nucleotide upstream a SNP site, or at a SNP site.

29. Kit for discriminating a bacterium of the *Enterobacteriaceae* family, **characterized in that** it comprises at least:
- primers able to amplify at least a sequence, or a fragment thereof, comprising a target region having a length comprised between 300 and 650 bp and selected among internal portions of a set of genes selected in the group consisting of *gyrB, rpoB, fusA, leuS, rplB* and *pyrG* of *Enterobacteriaceae,* or a fragment of said target region of at least 15 bp, preferably of 40-150 bp,
- a DNA array as defined in claim 23, and/or
- oligonucleotides as defined in claims 25-28, and
- the repertory of nucleic acids of the 171 species of *Enterobacteriaceae* family listed in figure 1 (SEQ ID NO:1-6 and SEQ ID NO:26 to SEQ ID NO:983).


SEQUENCE LISTING

<110>  INSTITUT PASTEUR

<120>  METHOD OF DISCRIMINATION OF THE SPECIES OF THE ENTEROBACTERIACEAE
       FAMILY BY GENOTYPING AND ITS APPLICATIONS

<130>  BLOcp226/139EP

<160>  983

<170>  PatentIn version 3.3

<210>  1
<211>  420
<212>  DNA
<213>  Artificial

<220>
<223>  gyrB marker


<220>
<221>  misc_feature
<222>  (9)..(9)
<223>  n is a, c, g, or t

<220>
<221>  misc_feature
<222>  (12)..(12)
<223>  n is a, c, g, or t

<220>
<221>  misc_feature
<222>  (15)..(15)
<223>  n is a, c, g, or t

<220>
<221>  misc_feature
<222>  (18)..(18)
<223>  n is a, c, g, or t

<220>
<221>  misc_feature
<222>  (24)..(24)
<223>  n is a, c, g, or t

<220>
<221>  misc_feature
<222>  (36)..(36)
<223>  n is a, c, g, or t

<220>
<221>  misc_feature
<222>  (45)..(45)
<223>  n is a, c, g, or t

<220>
<221>  misc_feature
<222>  (78)..(78)
<223>  n is a, c, g, or t

<220>
<221>  misc_feature
<222>  (99)..(99)
<223>  n is a, c, g, or t

<220>

<221>  misc_feature
<222>  (102)..(102)
<223>  n is a, c, g, or t

<220>
<221>  misc_feature
<222>  (108)..(108)
<223>  n is a, c, g, or t

<220>
<221>  misc_feature
<222>  (111)..(111)
<223>  n is a, c, g, or t

<220>
<221>  misc_feature
<222>  (144)..(144)
<223>  n is a, c, g, or t

<220>
<221>  misc_feature
<222>  (150)..(150)
<223>  n is a, c, g, or t

<220>
<221>  misc_feature
<222>  (177)..(177)
<223>  n is a, c, g, or t

<220>
<221>  misc_feature
<222>  (182)..(183)
<223>  n is a, c, g, or t

<220>
<221>  misc_feature
<222>  (201)..(201)
<223>  n is a, c, g, or t

<220>
<221>  misc_feature
<222>  (210)..(210)
<223>  n is a, c, g, or t

<220>
<221>  misc_feature
<222>  (213)..(213)
<223>  n is a, c, g, or t

<220>
<221>  misc_feature
<222>  (216)..(216)
<223>  n is a, c, g, or t

<220>
<221>  misc_feature
<222>  (228)..(229)
<223>  n is a, c, g, or t

<220>
<221>  misc_feature
<222>  (237)..(237)
<223>  n is a, c, g, or t

<220>
<221>  misc_feature
<222>  (240)..(240)
<223>  n is a, c, g, or t

<220>
<221>  misc_feature
<222>  (242)..(242)
<223>  n is a, c, g, or t

<220>
<221>  misc_feature
<222>  (264)..(264)
<223>  n is a, c, g, or t

<220>
<221>  misc_feature
<222>  (282)..(282)
<223>  n is a, c, g, or t

<220>
<221>  misc_feature
<222>  (285)..(285)
<223>  n is a, c, g, or t

<220>
<221>  misc_feature
<222>  (300)..(300)
<223>  n is a, c, g, or t

<220>
<221>  misc_feature
<222>  (309)..(309)
<223>  n is a, c, g, or t

<220>
<221>  misc_feature
<222>  (348)..(348)
<223>  n is a, c, g, or t

<220>
<221>  misc_feature
<222>  (351)..(351)
<223>  n is a, c, g, or t

<220>
<221>  misc_feature
<222>  (357)..(357)
<223>  n is a, c, g, or t

<220>
<221>  misc_feature
<222>  (361)..(363)
<223>  n is a, c, g, or t

<220>
<221>  misc_feature
<222>  (377)..(379)
<223>  n is a, c, g, or t

<220>
<221>  misc_feature
<222>  (411)..(411)
<223>  n is a, c, g, or t

<400>  1
gaaggcgtnt cngcngcnga agtnatcatg accgtnctgc acgcnggcgg taaattcgat      60

gataactcct ataaagtntc cggcggtctg cacggcgtng gngtttcngt ngttaacgcc     120

ctgtcgcaga aactggaact ggtnatccgn cgcgaaggca aagttcacca gcagatntac     180

gnncacggcg tgccgcaggc nccgctggcn gtnacnggcg aaaccganna aaccggnacn 240

anggtgcgtt tctggccgag ccangaaacc ttcaccaacg tnacngaatt cgaatacgan 300

attctggcna aacgcctgcg tgagctgtcg ttcctgaact ccggcgtntc natccgnctg 360

nnngacaagc gcgacgnnng caaagaagac cacttccact acgaaggcgg natcaaggcg 420

<210> 2
<211> 501
<212> DNA
<213> Artificial

<220>
<223> rpoB marker

<220>
<221> misc_feature
<222> (105)..(105)
<223> n is a, c, g, or t

<220>
<221> misc_feature
<222> (111)..(111)
<223> n is a, c, g, or t

<220>
<221> misc_feature
<222> (123)..(123)
<223> n is a, c, g, or t

<220>
<221> misc_feature
<222> (127)..(128)
<223> n is a, c, g, or t

<220>
<221> misc_feature
<222> (141)..(141)
<223> n is a, c, g, or t

<220>
<221> misc_feature
<222> (144)..(144)
<223> n is a, c, g, or t

<220>
<221> misc_feature
<222> (219)..(219)
<223> n is a, c, g, or t

<220>
<221> misc_feature
<222> (240)..(240)
<223> n is a, c, g, or t

<220>
<221> misc_feature
<222> (246)..(246)
<223> n is a, c, g, or t

<220>
<221> misc_feature
<222> (288)..(288)
<223> n is a, c, g, or t

<220>
<221>  misc_feature
<222>  (417)..(417)
<223>  n is a, c, g, or t

<220>
<221>  misc_feature
<222>  (471)..(471)
<223>  n is a, c, g, or t

<220>
<221>  misc_feature
<222>  (492)..(492)
<223>  n is a, c, g, or t

<220>
<221>  misc_feature
<222>  (495)..(495)
<223>  n is a, c, g, or t

<400>  2
actcactacg gtcgcgtatg tccaatcgaa acgcctgaag gtccgaacat cggtctgatc      60

aactccctgt ccgtgtacgc acagactaac gaatacggtt tcctngaaac nccgtaccgt     120

cgngtgnntg acggtgtggt nacngacgaa attcattacc tgtctgctat tgaagaaggc     180

aactacgtta tcgctcaggc gaactccaac ctggatgang aaggccactt cgtagaagan     240

ctggtnacct gccgtagcaa aggcgaatcc agcttgttca gccgcganca ggttgactac     300

atggacgttt ccacccagca ggtggtttcc gtcggtgcgt ccctgatccc gttcctggaa     360

cacgatgacg ccaaccgtgc attgatgggt gcgaacatgc aacgtcaggc ggttccnact     420

ctgcgtgctg ataagccgct ggttggtacc ggtatggaac gtgctgttgc ngttgactcc     480

ggtgttactg cngtngctaa a                                               501

<210>  3
<211>  633
<212>  DNA
<213>  Artificial

<220>
<223>  fusA marker


<220>
<221>  misc_feature
<222>  (39)..(39)
<223>  n is a, c, g, or t

<220>
<221>  misc_feature
<222>  (42)..(42)
<223>  n is a, c, g, or t

<220>
<221>  misc_feature
<222>  (90)..(90)
<223>  n is a, c, g, or t

<220>
<221>  misc_feature
<222>  (96)..(96)
<223>  n is a, c, g, or t

<220>
<221>  misc_feature
<222>  (186)..(186)
<223>  n is a, c, g, or t

<220>
<221>  misc_feature
<222>  (288)..(288)
<223>  n is a, c, g, or t

<220>
<221>  misc_feature
<222>  (396)..(396)
<223>  n is a, c, g, or t

<220>
<221>  misc_feature
<222>  (402)..(402)
<223>  n is a, c, g, or t

<220>
<221>  misc_feature
<222>  (430)..(432)
<223>  n is a, c, g, or t

<220>
<221>  misc_feature
<222>  (498)..(498)
<223>  n is a, c, g, or t

<220>
<221>  misc_feature
<222>  (522)..(522)
<223>  n is a, c, g, or t

<220>
<221>  misc_feature
<222>  (546)..(546)
<223>  n is a, c, g, or t

<220>
<221>  misc_feature
<222>  (579)..(579)
<223>  n is a, c, g, or t

<220>
<221>  misc_feature
<222>  (603)..(603)
<223>  n is a, c, g, or t

<400>  3
ggtgtaaacc ataaaatcgg tgaagttcat gacggcgcng cnaccatgga ctggatggag      60

caggagcagg agcgtggtat taccatcacn tccgcngcga ctactgcatt ctggtctggt     120

atggctaagc agtatgaacc gcaccgcgta aacatcatcg acaccccggg gcacgttgac     180

ttcacnatcg aagtagaacg ttccatgcgt gttcttgatg gtgcggtaat ggtttactgt     240

gcagttggtg gtgttcagcc acagtctgaa accgtatggc gtcaggcnaa caaatataaa     300

gttccacgca tcgcgttcgt taacaaaatg gaccgtatgg gtgcgaactt cctgaaagtt     360

gttggtcaga tcaaaacccg tctgggcgcg aacccngttc cnctgcagct ggcaattggt     420

gctgaagaan nnttcaccgg tgttgttgac ctggtgaaaa tgaaagccat caactggaac     480

gaagcagatc agggcgtnac cttcgaatac gaagatatcc cngctgacat gcaggaactg     540
gctgangaat ggcaccagaa cctgatcgaa tccgctgcng aagcttctga agagctgatg     600
ganaaatacc tgggcggtga agaactgact gaa                                  633

<210>  4
<211>  642
<212>  DNA
<213>  Artificial

<220>
<223>  leuS marker


<220>
<221>  misc_feature
<222>  (30)..(30)
<223>  n is a, c, g, or t

<220>
<221>  misc_feature
<222>  (63)..(64)
<223>  n is a, c, g, or t

<220>
<221>  misc_feature
<222>  (72)..(72)
<223>  n is a, c, g, or t

<220>
<221>  misc_feature
<222>  (75)..(75)
<223>  n is a, c, g, or t

<220>
<221>  misc_feature
<222>  (81)..(81)
<223>  n is a, c, g, or t

<220>
<221>  misc_feature
<222>  (120)..(120)
<223>  n is a, c, g, or t

<220>
<221>  misc_feature
<222>  (141)..(141)
<223>  n is a, c, g, or t

<220>
<221>  misc_feature
<222>  (143)..(144)
<223>  n is a, c, g, or t

<220>
<221>  misc_feature
<222>  (153)..(153)
<223>  n is a, c, g, or t

<220>
<221>  misc_feature
<222>  (156)..(156)
<223>  n is a, c, g, or t

<220>
<221>  misc_feature

<222>  (180)..(180)
<223>  n is a, c, g, or t

<220>
<221>  misc_feature
<222>  (201)..(201)
<223>  n is a, c, g, or t

<220>
<221>  misc_feature
<222>  (213)..(213)
<223>  n is a, c, g, or t

<220>
<221>  misc_feature
<222>  (216)..(216)
<223>  n is a, c, g, or t

<220>
<221>  misc_feature
<222>  (219)..(219)
<223>  n is a, c, g, or t

<220>
<221>  misc_feature
<222>  (222)..(222)
<223>  n is a, c, g, or t

<220>
<221>  misc_feature
<222>  (246)..(246)
<223>  n is a, c, g, or t

<220>
<221>  misc_feature
<222>  (249)..(249)
<223>  n is a, c, g, or t

<220>
<221>  misc_feature
<222>  (252)..(252)
<223>  n is a, c, g, or t

<220>
<221>  misc_feature
<222>  (261)..(261)
<223>  n is a, c, g, or t

<220>
<221>  misc_feature
<222>  (270)..(270)
<223>  n is a, c, g, or t

<220>
<221>  misc_feature
<222>  (273)..(273)
<223>  n is a, c, g, or t

<220>
<221>  misc_feature
<222>  (303)..(303)
<223>  n is a, c, g, or t

<220>
<221>  misc_feature
<222>  (315)..(315)
<223>  n is a, c, g, or t

<220>
<221>  misc_feature
<222>  (324)..(324)
<223>  n is a, c, g, or t

<220>
<221>  misc_feature
<222>  (340)..(340)
<223>  n is a, c, g, or t

<220>
<221>  misc_feature
<222>  (342)..(342)
<223>  n is a, c, g, or t

<220>
<221>  misc_feature
<222>  (345)..(345)
<223>  n is a, c, g, or t

<220>
<221>  misc_feature
<222>  (352)..(352)
<223>  n is a, c, g, or t

<220>
<221>  misc_feature
<222>  (354)..(354)
<223>  n is a, c, g, or t

<220>
<221>  misc_feature
<222>  (357)..(357)
<223>  n is a, c, g, or t

<220>
<221>  misc_feature
<222>  (360)..(360)
<223>  n is a, c, g, or t

<220>
<221>  misc_feature
<222>  (382)..(382)
<223>  n is a, c, g, or t

<220>
<221>  misc_feature
<222>  (384)..(385)
<223>  n is a, c, g, or t

<220>
<221>  misc_feature
<222>  (387)..(387)
<223>  n is a, c, g, or t

<220>
<221>  misc_feature
<222>  (393)..(393)
<223>  n is a, c, g, or t

<220>
<221>  misc_feature
<222>  (399)..(399)
<223>  n is a, c, g, or t

<220>
<221>  misc_feature
<222>  (402)..(402)

<223>  n is a, c, g, or t

<220>
<221>  misc_feature
<222>  (435)..(435)
<223>  n is a, c, g, or t

<220>
<221>  misc_feature
<222>  (438)..(438)
<223>  n is a, c, g, or t

<220>
<221>  misc_feature
<222>  (453)..(453)
<223>  n is a, c, g, or t

<220>
<221>  misc_feature
<222>  (492)..(492)
<223>  n is a, c, g, or t

<220>
<221>  misc_feature
<222>  (498)..(498)
<223>  n is a, c, g, or t

<220>
<221>  misc_feature
<222>  (519)..(519)
<223>  n is a, c, g, or t

<220>
<221>  misc_feature
<222>  (522)..(522)
<223>  n is a, c, g, or t

<220>
<221>  misc_feature
<222>  (530)..(530)
<223>  n is a, c, g, or t

<220>
<221>  misc_feature
<222>  (543)..(543)
<223>  n is a, c, g, or t

<220>
<221>  misc_feature
<222>  (549)..(550)
<223>  n is a, c, g, or t

<220>
<221>  misc_feature
<222>  (552)..(552)
<223>  n is a, c, g, or t

<220>
<221>  misc_feature
<222>  (572)..(572)
<223>  n is a, c, g, or t

<220>
<221>  misc_feature
<222>  (615)..(615)
<223>  n is a, c, g, or t

<400>  4

cagtggttta tcaaaatcac cgcttacgcn gacgaactgc tgaacgatct ggatacgctg 60

gannactggc cngancaggt naaaaccatg cagcgcaact ggattggccg ttctgaaggn 120

gtggaaatca ccttcgacgt ngnngacagc ganganaagc tgaccgttta caccacccgn 180

ccggacacct ttatgggcgc nacctacctg gcngtngcng cnggtcaccc gctggcgcag 240

caagcngcng cnaacaaccc ngcgctggcn gantttatcg acgaatgccg taacaccaaa 300

gtngccgaag ccganatggc gacnatggag aaaaaaggcn tngcnaccgg cntntangcn 360

attcacccgc tgaccggcga ananntnccg gtntgggcng cnaacttcgt gctgatggaa 420

tacggcaccg gcgcngtnat ggccgttccg ggncacgacc agcgcgactg ggaatttgcc 480

accaaatacg gnctgccnat caaaccggtt attctggcng cngacggcan cgagccggat 540

ctntccgann angcgatgac cgaaaaaggc gngctgttca actccggcga gttcgacggt 600

ctggactacg aagcnggctt caacgccatt gccgacaaac tg 642

<210> 5
<211> 339
<212> DNA
<213> Artificial

<220>
<223> rplB marker


<220>
<221> misc_feature
<222> (168)..(168)
<223> n is a, c, g, or t

<220>
<221> misc_feature
<222> (216)..(221)
<223> n is a, c, g, or t

<220>
<221> misc_feature
<222> (273)..(273)
<223> n is a, c, g, or t

<220>
<221> misc_feature
<222> (276)..(276)
<223> n is a, c, g, or t

<400> 5
cgttacatcc tggcccctaa aggcctgaaa gctggcgacc agattcagtc tggcgttgat 60

gctgcaatca aagcaggtaa caccctgccg atgcgcaaca tcccggttgg ttctaccgtt 120

cataacgtag aaatgaaacc aggtaaaggc ggtcagctgg ctcgttcngc tggtacttac 180

gttcagatcg ttgctcgtga tggtgcttat gtcacnnnnn ncctgcgtct gcgttctggt 240

gaaatgcgta aagtcgaagc tgactgccgc gcnacnctgg gcgaagttgg taacgctgag 300

catatgctgc gcgttctggg taaagcaggt gctgcacgc 339


<210> 6
<211> 306

<212>  DNA
<213>  Artificial

<220>
<223>  pyrG marker

<220>
<221>  misc_feature
<222>  (27)..(27)
<223>  n is a, c, g, or t

<220>
<221>  misc_feature
<222>  (69)..(69)
<223>  n is a, c, g, or t

<220>
<221>  misc_feature
<222>  (174)..(174)
<223>  n is a, c, g, or t

<220>
<221>  misc_feature
<222>  (195)..(195)
<223>  n is a, c, g, or t

<220>
<221>  misc_feature
<222>  (210)..(210)
<223>  n is a, c, g, or t

<220>
<221>  misc_feature
<222>  (213)..(213)
<223>  n is a, c, g, or t

<220>
<221>  misc_feature
<222>  (216)..(216)
<223>  n is a, c, g, or t

<220>
<221>  misc_feature
<222>  (252)..(252)
<223>  n is a, c, g, or t

<220>
<221>  misc_feature
<222>  (285)..(285)
<223>  n is a, c, g, or t

<220>
<221>  misc_feature
<222>  (303)..(303)
<223>  n is a, c, g, or t

<400>  6
attcttgaag cccgtggcct caacgtnacc atcatgaaac tggatccgta catcaacgtc     60

gatccgggna ccatgagccc aatccaacac ggggaagtgt tcgttaccga agacggcgct    120

gaaaccgatc tggatttggg tcactacgag cgtttcatcc gcaccaaaat gacncgccgc    180

aacaacttca ccacnggccg tatctactcn gangtnctgc gtaaagagcg ccgtggcgac    240

tatctgggcg cnaccgttca ggttatcccg cacatcacca acgcnatcaa agagcgcatc    300

atngaa                                                                306

<210>  7
<211>  40
<212>  DNA
<213>  Artificial

<220>
<223>  gyrB 40 pb


<220>
<221>  misc_feature
<222>  (13)..(13)
<223>  n is a, c, g, or t

<220>
<221>  misc_feature
<222>  (16)..(16)
<223>  n is a, c, g, or t

<220>
<221>  misc_feature
<222>  (22)..(22)
<223>  n is a, c, g, or t

<220>
<221>  misc_feature
<222>  (26)..(28)
<223>  n is a, c, g, or t

<400>  7
gaactccggc gtntcnatcc gnctgnnnga caagcgcgac                              40


<210>  8
<211>  40
<212>  DNA
<213>  Artificial

<220>
<223>  rpoB 40 pb


<220>
<221>  misc_feature
<222>  (2)..(2)
<223>  n is a, c, g, or t

<400>  8
cnactctgcg tgctgataag ccgctggttg gtaccggtat                              40


<210>  9
<211>  40
<212>  DNA
<213>  Artificial

<220>
<223>  fusA 40pb


<220>
<221>  misc_feature
<222>  (19)..(19)
<223>  n is a, c, g, or t

<400>  9
cgaagcagat cagggcgtna ccttcgaata cgaagatatc                                40


<210>  10
<211>  40
<212>  DNA
<213>  Artificial

<220>
<223>  leuS 40 pb


<220>
<221>  misc_feature
<222>  (5)..(5)
<223>  n is a, c, g, or t

<220>
<221>  misc_feature
<222>  (7)..(7)
<223>  n is a, c, g, or t

<220>
<221>  misc_feature
<222>  (10)..(10)
<223>  n is a, c, g, or t

<220>
<221>  misc_feature
<222>  (17)..(17)
<223>  n is a, c, g, or t

<220>
<221>  misc_feature
<222>  (19)..(19)
<223>  n is a, c, g, or t

<220>
<221>  misc_feature
<222>  (22)..(22)
<223>  n is a, c, g, or t

<220>
<221>  misc_feature
<222>  (25)..(25)
<223>  n is a, c, g, or t

<400>  10
aggcntngcn accggcntnt angcnattca cccgctgacc                                40


<210>  11
<211>  40
<212>  DNA
<213>  Artificial

<220>
<223>  pyrG 40 pb


<220>
<221>  misc_feature
<222>  (2)..(2)
<223>  n is a, c, g, or t

<220>
<221>  misc_feature

<222>  (17)..(17)
<223>  n is a, c, g, or t

<220>
<221>  misc_feature
<222>  (20)..(20)
<223>  n is a, c, g, or t

<220>
<221>  misc_feature
<222>  (23)..(23)
<223>  n is a, c, g, or t

<400>  11
cnggccgtat ctactcngan gtnctgcgta aagagcgccg                              40


<210>  12
<211>  21
<212>  DNA
<213>  Artificial

<220>
<223>  gyrB3 primer

<400>  12
gcgtaagcgc ccgggtatgt a                                                  21


<210>  13
<211>  23
<212>  DNA
<213>  Artificial

<220>
<223>  gyrB4 primer

<400>  13
ccgtcgacgt ccgcatcggt cat                                                23


<210>  14
<211>  17
<212>  DNA
<213>  Artificial

<220>
<223>  gyrB3i primer

<400>  14
aacgcwatcg acgaagc                                                       17


<210>  15
<211>  18
<212>  DNA
<213>  Artificial

<220>
<223>  gyrB4i primer

<400>  15
tggaavccrt crttccac                                                      18


<210>  16
<211>  20
<212>  DNA

<213>  Artificial

<220>
<223>  Vic3 primer

<400>  16
ggcgaaatgg cwgagaacca                                                    20


<210>  17
<211>  20
<212>  DNA
<213>  Artificial

<220>
<223>  Vic2 primer

<400>  17
gagtcttcga agttgtaacc                                                    20


<210>  18
<211>  24
<212>  DNA
<213>  Artificial

<220>
<223>  fusA3 primer

<400>  18
catcggtatc agtgckcaca tcga                                               24


<210>  19
<211>  24
<212>  DNA
<213>  Artificial

<220>
<223>  fusA4 primer

<400>  19
cagcatcgcc tgaacrcctt tgtt                                               24


<210>  20
<211>  26
<212>  DNA
<213>  Artificial

<220>
<223>  leuS3 primer

<400>  20
cagaccgtgc tggccaacga rcargt                                             26


<210>  21
<211>  20
<212>  DNA
<213>  Artificial

<220>
<223>  leuS4 primer

<400>  21
cggcgcgccc cartarcgct                                                    20

<210>  22
<211>  28
<212>  DNA
<213>  Artificial

<220>
<223>  rplB3 primer

<400>  22
cagttgttga acgtcttgag tacgatcc                                          28


<210>  23
<211>  22
<212>  DNA
<213>  Artificial

<220>
<223>  rplB4 primer

<400>  23
caccaccacc atgygggtgr tc                                                22


<210>  24
<211>  26
<212>  DNA
<213>  Artificial

<220>
<223>  pyrG3 primer

<400>  24
ggggtcgtat cctctctggg taaagg                                            26


<210>  25
<211>  27
<212>  DNA
<213>  Artificial

<220>
<223>  pyrG4 primer


<220>
<221>  misc_feature
<222>  (23)..(23)
<223>  n is a, c, g, or t

<400>  25
ggaacggcag ggattcgata tcnccka                                           27


<210>  26
<211>  420
<212>  DNA
<213>  Brenneria alni

<400>  26
gaagggatat ccgcggcaga agtgatcatg acggtgctac atgccggcgg caagtttgat      60

gataactcgt acaaggtttc cggcggtttg cacggcgtgg ggatttccgt ggttaatgcc     120

ctgtcggaaa agctggagct ggtggttcac cgtgaaggta aagttcacga gcaaacttat     180

cgccacggtg tggcgcaggc gccgctgaaa gtggtggacg agacagaaaa aaccggcacc     240

acggtgcgtt tctggccgag ctatgaaacg ttcaccaacc agacggaatt tcagtatgac 300

attctggcca aacgtctgcg tgagctgtcg ttcctaaact cgggcgtttc tatccgcttg 360

tttgatgagc gcgaaaaaga taaatccgac cattttcact acgaaggcgg tatcaaagcc 420

<210> 27
<211> 420
<212> DNA
<213> Brenneria quercina

<400> 27
gaaggcgtgt cagccgctga agtcatcatg acggtgctgc atgccggcgg taaatttgat 60

gataactcct acaaagtttc cggcggtttg cacggtgtag gggtttccgt ggtcaacgcc 120

ctgtcggaaa aacttgagct gaccattcgc cgcgaaggca aaattcatca acagacctac 180

aaccacggcg tgccgttcgc gcccctgagc gtggtgggtg aagctgaaaa atccgggact 240

accgtccgtt tctggccgag caccgaaacc ttcaccaacg tgctggaatt cgaatacgac 300

attctggcca agcgtctgcg tgagctgtcg ttccttaatt ccggtgtttc catccgtctg 360

cgtgacgaac gtgaaaaaga taaatccgac cacttccatt acgatggcgg catcaaagcg 420

<210> 28
<211> 420
<212> DNA
<213> Brenneria rubrifaciens

<400> 28
gaagggatat ccgctgcgga agtgatcatg acggtgctgc atgccggcgg caagtttgat 60

gataactcgt acaaagtttc cggcggcttg catggcgtgg gcgtttccgt ggttaatgcc 120

ttgtcggaaa agctggaact ggtgattcgc cgggaaggta aagtccacga acagacctat 180

cgccacggcg tggcgcaggc gccgctgaaa gtggtgggcg aaagcgtgaa aaccggtacg 240

acagtgcgtt tctggccgag tctggaaacc tttaccaacg tggtggaatt tgagtatgaa 300

attctggcca agcgtctgcg tgagctctca ttccttaatt ccggcgtatc gattcgcctg 360

attgacgagc gtgaaaacga caaagccgat cacttccact atgaaggcgg catcaaagcg 420

<210> 29
<211> 420
<212> DNA
<213> Brenneria salicis T

<400> 29
gaagggatct ccgccgctga agtcatcatg accgtgctgc acgcgggcgg taagttcgat 60

gataactcgt ataaagtctc cggcggcctg cacggcgtag gggtttccgt ggttaacgcc 120

ctgtcggaaa aactgaagct ggttattcac cgtgacggaa aacttcacga gcagacctat 180

aaacacggcg tggcgcaggc tccgctggcg gttacgggtg aaaccaacag aaccggtacg 240

acggtacgct tctggccgag ccatgagacg ttcaccaacg tggtggaatt cgagtatgag 300

attctggcta agcgtctgcg cgagctgtcg ttcctgaact ccggtgtctc catccgcttg 360

atcgacgaac gtgagaaaga taaagccgat cattaccatt atgacggcgg catcaaggcg 420

<210> 30
<211> 417
<212> DNA
<213> Budvicia aquatica T

<400> 30
gaaggcgttt cagcggcaga agttattatg accgtactgc acgccggcgg taaatttgac 60

gataactcat acaaagtgtc aggtggtctg cacggcgtag gcgtttccgt ggttaacgcc 120

ctgtcagata agctggaatt gattatcgat cgtgaaggca aaacccataa gcaaatttat 180

cgtggcggtg tacctgaagc gccactgaaa gtgatcgcag ataccgataa aaccggtacc 240

atgatccgtt tctggccaag cccggacacc ttcaccacta acctagagtt ccactacgat 300

attttagcta aacgcttacg cgagctatca ttcctgaact ccggtgtttc tattaagctg 360

tttgatgagc gcgacggtaa atccgatcac ttccactatg atggtggtat taaagcg 417

<210> 31
<211> 417
<212> DNA
<213> Buttiauxella agrestis T

<400> 31
gaaggtgtgt ctgcagccga agttatcatg accgtgcttc atgccggcgg taagttcgat 60

gacaactctt ataaagtttc tggcggtctg cacggcgtgg gtgtttcggt tgttaacgcc 120

ctgtctcaga agctggaact gctgattcgc cgtgaaggca aagtacacca gcaaacttac 180

atccacggcg tgccacaagc gccgctggcg gcaacgggtg ataccgatgc aaccggtacg 240

caagtccgtt tctggccaag ccacgaaacc ttcaccaacg tgattgagtt tgaatacgac 300

attctggcta aacgcctgcg tgaactgtcg ttcctgaact ctggtgtttc tatccgcctg 360

attgataagc gtgatggccg ccaggaccac ttccactacg aaggtggtat ccgcgcg 417

<210> 32
<211> 417
<212> DNA
<213> Buttiauxella brennerae

<400> 32
gaaggcgtgt ctgctgctga agttatcatg accgtgcttc acgccggcgg taagttcgat 60

gataactcct acaaagtttc tggtggtctg cacggtgtgg gtgtttcggt tgttaacgcc 120

ctgtctcaga agctggaact gctgattcgc cgcgaaggca aagttcatca gcaaacttat 180

gttcacggtg tgccacaagc gccgctggcg gcaacgggtg ataccgatgc aaccggtacg 240

caagtccgtt tctggccaag ccacgacacc ttcaccaacg tgattgagtt tgaatacgac 300

attctggcta aacgcctgcg cgaattgtcg ttcctgaact ctggtgtttc tatccgcctg 360

attgataagc gtgatggccg ccaggaccac ttccactacg aaggtggtat ccgcgcg 417

<210> 33
<211> 417

<212>  DNA
<213>  Buttiauxella ferragutiae

<400>  33
gaaggcgtat ctgctgccga agttatcatg accgtgctgc acgctggcgg taagttcgat      60

gacaactcct ataaagtttc tggcggtctg cacggcgtag gtgtttctgt tgttaacgcc     120

ctgtcccaga aactggaact gctgattcgc cgcgaaggca aagtacacca gcaaacttac     180

gttcacggcg tgccacaggc accgctggcg gcgacgggtg ataccgatgc caccggtacg     240

caagtccgtt tctggccaag ccatgaaacc ttcaccaacg tgattgagtt tgaatacgac     300

attctggcca aacgcctgcg tgaactgtca ttcctgaact ccggtgtttc tatccgcctg     360

attgataagc gtgatggccg ccaggaccac ttccactacg aaggtggtat ccgcgca        417

<210>  34
<211>  417
<212>  DNA
<213>  Buttiauxella gaviniae

<400>  34
gaaggtgtgt ctgctgccga agttattatg accgtgcttc atgccggcgg taagttcgat      60

gacaactcct ataaagtttc tggtggtctg cacggtgtgg gtgtttcggt tgttaacgcc     120

ctgtcccaga agctggaact gctgattcgc cgcgaaggca aagtgcacca gcaaacttat     180

gttcacggtg tgccacaagc gccgctggcg gcaacgggtg ataccgatgc aaccggtacg     240

caagtccgtt tctggccaag ccacgacacc ttcaccaacg tgattgagtt tgaatacgac     300

attctggcta aacgcctgcg cgaattgtcg ttcctgaact ctggtgtttc tatccgcctg     360

attgataagc gtgatggccg ccaggaccac ttccactacg aaggtggtat ccgtgcg        417

<210>  35
<211>  417
<212>  DNA
<213>  Buttiauxella izardii

<400>  35
gaaggtgttt ctgctgccga agttatcatg accgtgcttc acgccggcgg taagttcgat      60

gacaactcct ataaagtttc tggcggcctg cacggcgtag gtgtttcggt tgttaacgcc     120

ctgtctcaga aactggaact gctgattcgc cgcgaaggca aagttcacca gcaaacttac     180

atccacggcg tgccacaagc accgctggcg gcgactggcg atactgatgc gaccggtact     240

caggttcgtt tctggccaag ccacgaaacc ttcaccaacg tgattgagtt cgaatatgac     300

atcctggcaa aacgcctgcg tgaactgtca ttcctgaact ctggtgtgtc tatccgcctg     360

attgataagc gtgatggccg tgctgaccac ttccactacg aaggcggtat ccgcgcg        417

<210>  36
<211>  417
<212>  DNA
<213>  Buttiauxella noackiae

<400>  36
gaaggtgtgt ctgctgccga agttattatg accgtgcttc acgccggcgg taagttcgat      60

gacaactctt ataaagtttc tggtggtctg cacggtgtgg gtgtttctgt agttaacgcc 120

ctgtcccaga agctggaatt gctgattcgc cgcgaaggca aagtgcacca gcaaacttac 180

atccacggtg tgccacaggc gccgctggcc gttacgggcg aatctgacgt aaccggtacg 240

caagtccgtt tctggccaag ccacgaaacc ttcaccaacg tcattgagtt tgagtacgac 300

atcctggcta aacgcctgcg tgaactgtcg ttcctgaact ctggtgtttc tatccgcctg 360

attgataagc gtgatggccg ccaggaccac ttccactacg aaggcggtat ccgcgcg 417

<210> 37
<211> 417
<212> DNA
<213> Buttiauxella warmboldiae

<400> 37
gagggcgtgt ctgctgccga agttatcatg accgtgcttc atgccggcgg taagttcgat 60

gacaactctt ataaagtttc tggcggtctg cacggcgtgg gtgtttctgt agttaacgcc 120

ctgtcccaga agctggaact gctgattcgc cgcgaaggca aagtacacca gcaaacttac 180

atccacggcg tgccacaagc cccgctggcg gcaacgggcg ataccgatgc aaccggtacg 240

caagttcgtt tctggccaag ccacgaaacc ttcaccaacg tgattgagtt tgaatacgac 300

attctggcta aacgcctgcg tgaactgtcg ttcctgaact ctggtgtttc tatccgcctg 360

attgataagc gtgatggccg ccaggaccac ttccattacg aaggtggtat ccgcgca 417

<210> 38
<211> 417
<212> DNA
<213> Cedecea davisae T

<400> 38
gagggcgtgt ctgctgcgga agttatcatg acggtgctgc acgcaggcgg taagttcgac 60

gataactcct ataaagtctc cggcggcctg cacggcgtgg gtgtttccgt ggttaacgcc 120

ctgtcccaga agctggaact gctgattcgc cgcgaaggca aaacccatca gcaaacctac 180

gtgcacggtg tgccgcaggc tccgctggcg gtaaccggtg aaaccgaatt gaccgggacc 240

caggtgcgtt tctggccgag ccacgaaacc tttaccaacg ttgttgaatt tgaatatgac 300

atcctggcta agcgcctgcg cgagctgtcg ttcctgaact ccggcgtgtc tatccgcctg 360

atcgacaaac gcgacggcaa gcaggatcac ttccactacg aaggtgggat caaggca 417

<210> 39
<211> 417
<212> DNA
<213> Cedecea lapagei

<400> 39
gagggcgtct ctgctgcgga agttatcatg accgtgctgc acgccggcgg taagttcgat 60

gacaactcct ataaagtttc cggcggcctg cacggcgtag gcgtttcggt tgtgaacgcc 120

ctgtcccaga agctggagct gctgattcgc cgcgaaggca aaacccatca gcaaacctac 180

gtgcacggcg tgccgcaggc tccgctggcg gtcaccggtg aaaccgaact gaccggtacc 240

caggtgcgtt tctggccgag ccatgaaacc ttcaccaacg tcaccgaatt cgaatatgac 300

atcctggcta agcgcctgcg tgagctgtcg ttcctgaact ccggcgtctc tattcgcctg 360

aacgataagc gcgacggcaa gcaggatcac ttccactacg aaggcggcat caaggcg 417

<210> 40
<211> 417
<212> DNA
<213> Cedecea neteri

<400> 40
gagggcgtct cggctgcgga agttatcatg accgtactgc acgccggcgg taaatttgac 60

gataactcct ataaagtttc tggcggcctg cacggcgtag gcgtttctgt tgttaacgcc 120

ctgtcccaga agctggaact gttgatccgc cgcgaaggca aaacccatca gcaaacctac 180

gtgcacggcg tgccgcaggc tccgctggcg gtcactggcg aaaccgaact gaccggcacc 240

caggtgcgtt tctggccgag ccatgaaact ttcaccaacg tggtggaatt tgagtacgac 300

atcctggcta agcgcctgcg tgagctttcg ttcctgaact ccggcgtgtc tattcgcctg 360

aacgacaagc gcgacggcaa gcaggatcac ttccactacg aaggcggcat caaggcg 417

<210> 41
<211> 417
<212> DNA
<213> Citrobacter amalonaticus

<400> 41
gaaggcgtat cggcggcgga agtgatcatg accgttctgc acgcaggcgg taaattcgac 60

gataactcct ataaagtctc cggcggtctg cacggtgtgg gtgtctccgt ggtgaacgcc 120

ctgtcgcaga aactggaact ggttatccag cgcgataaca aaatccaccg tcagatctac 180

gagcacggcg ttccgcaggc accgctggca gtcactggcg ataccgataa aaccggtacg 240

atggtgcgtt tctggccgag ccacgaaacc ttcactaacg tcacggagtt cgaatacgag 300

attctggcga agcgtctgcg cgaactgtcg ttcctgaact ctggcgtctc tattcgcctg 360

aaagacaagc gcgatggtaa agaagaccat ttccactatg aaggtggcat cagggcg 417

<210> 42
<211> 417
<212> DNA
<213> Citrobacter braakii

<400> 42
gagggcgttt cggcggcgga agttatcatg accgttctgc acgcaggcgg taaattcgat 60

gataactcct ataaagtttc cggtggtctg cacggcgtag gtgtgtccgt agtaaacgcc 120

ctgtcacaga aactggagct ggtgattcgt cgtgaaggca aagtgcatca gcaaacttac 180

gtgcacggtg tgccgcaggc gccgctggcg gtaaccggcg aaacagaagc gacgggtact 240

caggtacgtt tctggccaag ccacgaaacc tttaccaatg tcgttgagtt tgaatatgag 300

attctggcta agcgtctgcg cgagctgtcg ttcctgaact ccggcgtctc cattcgtctg 360

cgcgacaagc gcgacggtaa agaagaccat ttccactacg aaggcggcat taaggcg        417

<210>  43
<211>  417
<212>  DNA
<213>  Citrobacter diversus

<400>  43
gagggcgttt ctgctgcgga agtgatcatg accgtgctgc acgcgggcgg taaattcgac        60

gataactcct ataaagtgtc cggcggtctg cacggcgtgg gtgtttccgt ggttaacgcc       120

ctgtcccaga aactggaact ggttatccag cgcgataaca aaatccaccg tcagatctat       180

gagcatggcg taccgcaggc gccgctggcg gtgaccggcg aaactgacaa aaccggtacg       240

atggtgcgtt tctggccgag cctggaaacc ttcaccaacg tcaccgaatt tgaatacgaa       300

attctggcta agcgtctgcg tgagctgtcc ttcctgaact ccggcgtctc tattcgtctg       360

aaagacaagc gcgacggcaa agaagaccat ttccactacg aaggcggcat caaagcg          417

<210>  44
<211>  417
<212>  DNA
<213>  Citrobacter farmeri

<400>  44
gagggggtat cggcggcgga agtcatcatg accgttctgc acgcaggcgg taaattcgac        60

gataactcct ataaagtgtc cggtggtttg catggcgtgg gtgtctccgt agtaaacgcc       120

ctgtcgcaga aactggaact ggttatccag cgcgataaca aagtccaccg tcagatctac       180

gagcacggcg tcccgcaggc accgctggcg gtaaccggcg ataccgataa aaccggtacg       240

atggtgcgtt tctggccgag ccatgaaacc tttactaacg tcactgagtt cgaatacgag       300

attctggcga agcgtctgcg cgagctgtcg ttcctgaact ctggcgtttc cattcgcctg       360

aaagacaagc gcgatggtaa agaagatcat ttccactatg aaggcggtat cagggcg          417

<210>  45
<211>  417
<212>  DNA
<213>  Citrobacter freundii T

<400>  45
gagggcgttt cggcagctga agttatcatg accgttttgc acgcaggcgg taaatttgat        60

gataactcct ataaagtttc cggtggtctg cacggcgtag gtgtttccgt cgttaacgcc       120

ctgtcgcaga aactggagct ggtgattcgt cgtgaaggca aagtgcatca gcaaacttac       180

gtgcacggtg tgccgcaggc gccgctggcg gtaaccggcg aaacagaatt gacgggtact       240

caggtccgtt tctggccaag ccacgaaacc tttaccaacg tcgttgagtt cgaatacgat       300

attctggcga agcgtctgcg tgagctgtca ttcctgaact ccggcgtctc tattcgtctg       360

cgcgacaagc gcgacggcaa agaagatcat ttccactacg aaggcggcat caaggcg          417

<210>  46

<211>  417
<212>  DNA
<213>  Citrobacter gillenii

<400>  46
gagggcgttt cggcggccga agtgatcatg accgtcctgc acgcaggcgg taaattcgat      60

gataactcct ataaagtctc cggcggtctg cacggcgtag gtgtttccgt ggttaacgcc     120

ctgtcgcaga aactggagtt ggtgattcgc cgcgaaggta aagtgcatca gcaaacttac     180

gtgcacggcg taccgcaggc accactggcc gtgactggcg agactgaagc gaccggtacc     240

caggtgcgtt tctggccaag ccatgaaacc tttaccaatg tcgttgagtt tgaatatgag     300

attctggcga aacgcctgcg cgagctgtca ttcctgaact ccggcgtatc catccgcctg     360

cgcgacaagc gcgacggcaa agaagatcat ttccactacg aaggtggcat caaggcg        417

<210>  47
<211>  417
<212>  DNA
<213>  Citrobacter koseri

<400>  47
gagggcgttt ctgctgcgga agtgatcatg accgtgctgc acgcgggcgg taaattcgac      60

gataactcct ataaagtgtc cggcggtctg cacggcgtgg gtgtttccgt ggttaacgcc     120

ctgtcccaga aactggaact ggttatccag cgcgataaca aaatccaccg tcagatctat     180

gagcatggcg taccgcaggc gccgctggcg gtgactggcg aaactgataa aaccggtacg     240

atggtgcgtt tctggccgag cctggaaacc ttcaccaacg tcaccgaatt tgaatacgaa     300

attctggcga agcgtctgcg tgagctgtcc ttcctgaact ccggcgtctc tattcgtctg     360

aaagacaagc gcgacggtaa agaagaccat ttccactacg aaggcggcat caaagcg        417

<210>  48
<211>  417
<212>  DNA
<213>  Citrobacter murliniae

<400>  48
gagggcgttt cggcggcaga agttatcatg accgttctgc acgcaggcgg taaattcgat      60

gataactctt ataaagtgtc cggcggtctg cacggcgtag gtgtttccgt ggttaacgcc     120

ctgtcgcaga aactggagct ggtgattcgt cgtgaaggca aagtgcatca gcaaacttac     180

gtgcacggcg tgccgcaagc gccgctggcg gtaaccggtg agaccgaagc tacgggtacc     240

caggtacgtt tctggccaag ccatgaaacc ttcaccaatg tcgttgaatt tgaatacgat     300

attctggcga aacgcctgcg cgagctgtca ttcctgaact ccggcgtatc catccgcctg     360

cgtgataagc gcgacggcaa agaagaccat ttccactacg aaggcggcat taaggcg        417

<210>  49
<211>  417
<212>  DNA
<213>  Citrobacter rodentium

<400>  49

gagggggtgt cggcggcgga agtgatcatg accgttctgc acgcgggcgg taaattcgac 60

gataactctt ataaagtgtc cggcggtctg cacggcgttg gcgtttccgt agtaaacgcc 120

ctgtcgcaga aactggaact ggttatccag cgcgataaca aaattcaccg tcagatctac 180

gtgcatggcg tgccgcaggc gccgctggcc gtaaccggcg acaccgacaa gaccggtacg 240

atggtgcgtt tctggccgag cctggaaacc ttcaccaacg tcactgaatt tgaatatgag 300

atcctggcga agcgtctgcg cgagctgtcc ttccttaact ccggcgtctc tatccgtctt 360

aaagacaagc gcgacggtaa agaggatcat ttccactacg aaggcggcat caaggcg 417

<210> 50
<211> 417
<212> DNA
<213> Citrobacter sedlakii

<400> 50
gagggcgtct cggcggcgga agtgatcatg accgtcctgc acgcaggcgg taaattcgat 60

gataactcct ataaagtgtc cggcggtctg cacggcgttg gtgtttccgt ggtaaacgcc 120

ctgtcccaga aactggaact ggttatccag cgcgataaca aaatccaccg tcagatttat 180

gagcacggcg taccgcaggc tccgctggcg gtgaccggcg ataccgacaa aaccggtacg 240

atggtccgtt tctggccaag cctggaaacc ttcaccaacg tcactgagtt cgaatacgag 300

atcctggcga aacgtctgcg tgagctgtcc ttcctcaact ctggcgtctc tattcgcttg 360

aaagacaagc gagacggtaa agaagaccac ttccactatg aaggcggcat caaggcg 417

<210> 51
<211> 417
<212> DNA
<213> Citrobacter werkmanii

<400> 51
gagggcgttt cggcggcgga agttatcatg accgttctgc acgcaggcgg taaattcgat 60

gataactcct ataaagtttc cggtggtttg cacggcgtag gtgtctccgt cgttaacgcc 120

ctgtcgcaga aactggagct ggtgattcgt cgtgaaggca aagtgcatca gcaaacttat 180

gtgcacggtg tgccgcaggc gccgctggcg gtaacgggcg aaacagaggt tacggggacc 240

caggtgcgtt tctggccaag ccacgaaacc ttcactaacg tgaccgagtt cgaatacgac 300

atcctggcga agcgcctgcg cgaactgtcg ttcctgaact ccggtgtctc tattcgtctg 360

cgcgacaagc gtgacggcaa agaagatcat ttccattacg aaggcggcat caaggcg 417

<210> 52
<211> 417
<212> DNA
<213> Citrobacter youngae

<400> 52
gagggcgttt cggcagcgga agttatcatg accgttttgc acgcaggcgg taaattcgat 60

gataactcct ataaagtttc cggtggtctg cacggcgtag gtgtttccgt cgttaacgcc 120

ctgtcacaga agctggaact ggtgattcgt cgtgaaggca aagtgcatca gcaaacttac 180

gtgcacggtg tgccgcaggc gccgctggca gtaactggcg aaacagaaat gacggggact 240

caggtccgtt tctggccaag ccatgaaacc tttaccaacg tcgttgagtt cgaatacgac 300

attctggcga agcgtctgcg cgagctgtca ttcctgaact ccggtgtgtc tattcgtctg 360

cgcgataagc gcgatggcaa agaagatcat ttccactacg aaggcggcat caaggcg 417

<210> 53
<211> 420
<212> DNA
<213> Dickeya chrysanthemi T

<400> 53
gagggcgtat ccgccgccga agtgatcatg accgtgctgc atgctggcgg taagttcgac 60

gacaactcct acaaggtatc cggcggtctg cacggggtgg gggtatcggt cgtgaacgcc 120

ctgtccgaga agctggaact ggttattcgc cgtgaaggca aacttcatcg tcaggaatac 180

aaacatggcg aaccgcaggc accgctgggc gtgatcggcg atgccgatgc caccggcacc 240

acggtgcgtt tctggccgag tctggaaact ttcaccaatg tggtcgaatt tgaatacgac 300

attctggcta agcgcctgcg cgagttgtcg ttcctcaact ccggcgtatc gattcgtctg 360

cgtgacgagc gcgaagcaaa caaagccgac catttccact acgaaggcgg catcaaggcg 420

<210> 54
<211> 417
<212> DNA
<213> Edwardsiella hoshinae

<400> 54
gagggggttt ccgccgccga agtgatcatg actgtgctgc atgctggtgg taagtttgat 60

gataactcct ataaggtgtc cggtggcttg catggtgtgg gcgtttccgt ggtgaacgcg 120

ctgtcggaga agctggagct ggtgatccgc cgtgatggcc atgtccatga gcagatttat 180

catcatggtg tgccggcagc cccattgaag gtggtagggg atactgagca gactgggacc 240

cgggtacgtt tctggccgag tatggaaacc tttagcaacg tggttgagtt ccagtatgac 300

atcctggcca aacgtctgcg cgagctgtcg ttcctgaact ccggggtctc catccgccta 360

cgcgacaagc gcaatgatcg cgaagaccat ttccactatg agggcgggat caaggcg 417

<210> 55
<211> 417
<212> DNA
<213> Edwardsiella ictaluri

<400> 55
gagggtgtct ctgccgccga ggtcatcatg accgtgctgc acgccggggg taagttcgac 60

gataactcct ataaggtctc cggcggcctg catggcgtgg gggtttccgt ggtgaacgcc 120

ctgtctgaaa agctggagct ggtgatccgt cgcgacggcc atgtccatga gcaaatctac 180

cggcacgggg taccggcagc accgctgaaa gtggttggcg acaccgatct gaccggtacc 240

cgtgtgcgtt tctggccgag tatggagacc ttcagcaatg tggttgagtt ccagtatgac 300

atcctggcca agcgcctgcg tgaactctcc ttcctgaact ccggcgtctc cattcgtctg 360

tgcgacaaac gcaacgatcg cgaagatcat ttccactatg agggtgggat caaggcg 417

<210> 56
<211> 417
<212> DNA
<213> Edwardsiella tarda T

<400> 56
gagggggttt ccgccgccga agtcatcatg accgtgctgc atgccggggg taagttcgac 60

gacaactcct acaaggtttc cggtggtctg catggcgtgg gcgtttccgt ggtaaacgca 120

ctgtccgaga agttggaact ggtgatccgc cgcgatggcc atgttcatga gcagatctac 180

catcatggcg tgccagcggc gccgttgaag atggtcggtg ataccgagca gaccggaacc 240

cgggtgcgtt tctggccgag catgcaaacc tttagcaacg tggtcgagtt ccagtatgac 300

atcctggcca aacgcctgcg cgagctgtcg ttccttaact ctggggtttc cattcgcctg 360

cgcgacaagc gcaatgatcg tgaagatcat ttccactatg agggtgggat taaggcg 417

<210> 57
<211> 417
<212> DNA
<213> Enterobacter aerogenes

<400> 57
gagggcgtct ccgcggcgga agttatcatg accgtcctgc acgccggcgg taaattcgat 60

gataactcct ataaagtgtc cggcggtctg cacggcgtag gcgtctcggt ggttaacgcc 120

ctgtcgcaga agctggagct ggttatccaa cgcgataaca aagttcacaa acaaatttat 180

gagcacggtg tgccgcaggc accgctggcg gtaaccggtg aaaccgaaag caccggtacc 240

atggtgcgtt tctggccaag cctggaaacc tttaccaacg tcactgaatt cgaatacgaa 300

atcctggcga aacgtctgcg cgagctgtcg ttcctcaact ccggggtctc tatccgcctg 360

cgcgataagc gcgacggcaa agaagaccat ttccactacg aaggcggcat caaggcg 417

<210> 58
<211> 417
<212> DNA
<213> Enterobacter amnigenus

<400> 58
gagggcgtgt ctgctgcaga agtcatcatg accgttctgc acgcgggcgg taagttcgat 60

gataactcgt ataaagtgtc cggcggtctg cacggtgttg gcgtttccgt tgtgaacgcc 120

ctgtcgcaga agctggaact ggttatctac cgcgaaggta aagcgcatca ccaggtgtat 180

tctcacggcg tgccgcaggc tccgctgacc gtcactggcg acacggaaaa aaccggtacc 240

accgtacgtt tctggccgag ccacgagacg ttttctaacg tggttgaatt tgaatacgaa 300

attctggcaa aacgcctgcg cgagctgtca ttcctgaact ctggcgtgtc gattcgtctg 360

cgcgataagc gtgacggcaa agaagaccat ttccactacg aaggcggcat caaggca 417

<210>  59
<211>  417
<212>  DNA
<213>  Enterobacter asburiae

<400>  59
gagggcgtct ctgctgcgga agtgatcatg accgtgctgc acgcaggcgg taagttcgac      60

gataactcct ataaagtttc cggcggcctg cacggcgtag gcgtttccgt ggttaacgcc     120

ctgtcgcaga agctggagct ggttatccag cgcgaaggca aaattcaccg tcagctctat     180

acgcacggcg tgccgcaggc accgctggcc gttaccggcg ataccgacaa aaccggtacc     240

atggtgcgtt tctggccgag ccatgaaacc ttcaccaacg tcaccgaatt cgagtacgac     300

atcctggcga aacgcctgcg cgaactgtcg ttcctgaact ccggcgtctc tatccgcctt     360

aaagacaagc gtgacggtaa agaagaccac ttccattacg aaggtggtat caaagcg        417

<210>  60
<211>  417
<212>  DNA
<213>  Enterobacter cancerogenus

<400>  60
gagggcgtat ctgctgcgga agtcatcatg accgttctgc acgcaggcgg taaattcgat      60

gataactcct ataaagtttc cggcggtctg catggcgttg gcgtctccgt tgtaaacgcc     120

ctgtcgcaga agctggagct ggttatccag cgcgaaggta aaattcatcg ccaggtgtat     180

acccacggcg tgccgcaggc accgctggct gtgaccggtg ataccgacaa aaccggtacc     240

ctggtacgct tctggccaag ccacgaaacc ttcaccaacg tcaccgaatt tgaatacgac     300

atcctggcga aacgcctgcg cgaactgtca ttcctgaact ccggcgtgtc tatccgcctg     360

aaagacaaac gcgacggcaa agaagaccac ttccactacg aaggcggcat cagggcg        417

<210>  61
<211>  417
<212>  DNA
<213>  Enterobacter cloacae sbsp. cloacae T

<400>  61
gagggcgtgt ctgctgcaga agtgatcatg accgttctgc acgcaggcgg taaatttgac      60

gataactcct ataaagtctc cggcggcctg cacggcgtag gtgtttccgt ggtaaacgcc     120

ctgtcgcaga aactggaact ggttatccag cgcgaaggca aaattcaccg ccagatctat     180

cagcacggtg tgcctgaagc gccgctggcc gtgaccggcg ataccgaaaa aaccggtacc     240

atggtgcgtt tctggccgag cctcgaaacc ttcaccaacg tcaccgagtt cgagtacgac     300

atcctggcaa aacgcctgcg tgaactgtcg ttcctgaact ccggcgtctc aattcgtctg     360

cgtgacaaac gcgacaacaa agaagaccac ttccactatg aaggtggtat caaagcg        417

<210>  62
<211>  417
<212>  DNA
<213>  Enterobacter cloacae sbsp. dissolvens

<400> 62
gagggcgtgt ctgctgcaga agtgatcatg accgttctgc acgcaggcgg taaatttgac 60

gataactcct ataaagtctc cggcggcctg cacggcgtag gtgtttccgt ggtaaacgcc 120

ctgtcgcaga aactggaact ggtcatccag cgcgaaggca aaattcaccg tcagatctat 180

cagcacggcg tgcctgaagc gccgctggcg gtgaccggcg ataccgaaaa aaccggtacc 240

atggtgcgtt tctggccgag cctcgaaacc ttcaccaacg tcaccgaatt cgagtacgac 300

atcctggcaa aacgcctgcg tgaactgtcg ttcctgaact ccggcgtctc aattcgtctg 360

cgtgacaaac gcgacaacaa agaagaccac ttccactatg aaggtggtat caaagcg 417

<210> 63
<211> 417
<212> DNA
<213> Enterobacter cowanii

<400> 63
gagggcgttt ctgccgcgga agtgatcatg accgtactgc atgcgggcgg taagttcgac 60

gataactcct ataaagtctc cggcggtctg cacggcgttg gcgtttcagt ggtaaacgcc 120

ctgtcgcaga aactggagct ggttatccag cgcgataaca aagttcaccg tcagatctac 180

acccacggcg tgccgcaggc tccgctggaa gtgacgggcg aaacggaaaa aaccggtacc 240

atggtgcgtt tctggccgag ctacgaaacc ttcaccaacg tcattgaatt tgaatatgaa 300

attctggcga aacgcctgcg tgagctctcc ttcctgaact ccggcgtgtc gattcgcctg 360

cgcgacaagc gcgacggcaa agaagaccac ttccactatg aaggcggcat cagggcg 417

<210> 64
<211> 417
<212> DNA
<213> Enterobacter gergoviae

<400> 64
gagggcgtct ccgctgcgga agtgatcatg accgtcctgc acgcgggcgg caaatttgac 60

gataactcct ataaagtctc cggcggcctg cacggcgtgg gcgtctcggt ggttaacgcc 120

ctgtcgcaga agctggagct ggtgatcaac cgcgaaggcc agatccaccg ccagatttat 180

gttcacggcg ttccggaagc tccgctggcg gtgaccggcg aaaccgagaa gaccgggacc 240

accgtgcgct tctggccgag cctggaaacc ttcaccaacg ttaccgaatt tgaatatgaa 300

atcctcgcca agcgcctgcg cgagctttcg ttcctgaact ccggcgtctc tattcgcctg 360

cgcgataagc gcgacggtaa agaggatcac ttccactacg aaggcggcat caaggcg 417

<210> 65
<211> 417
<212> DNA
<213> Enterobacter hormaechei

<400> 65
gagggcgtct ctgctgcgga agtgatcatg accgtcctgc acgcaggcgg taagttcgat 60

gataactcct ataaagtttc cggcggtctg cacggcgtag gcgtatccgt agtaaacgcc 120

ctgtcgcaga agctggagct ggttatccag cgcgaaggca aaattcaccg tcagatctac 180

cagcacggtg tgcctgacgc cccgctggcc gttacgggtg ataccgacaa aaccggtacc 240

atggtgcgtt tctggccgag ccttgaaacc ttcaccaacg tcaccgaatt cgagtacgac 300

attctggcga aacgcctgcg tgaactgtcg ttcctgaact ccggcgtgtc gattcgtctg 360

cgcgacaaac gcgacaacaa agaagaccac ttccactacg aaggcggtat caaggcg 417

<210> 66
<211> 417
<212> DNA
<213> Enterobacter kobei

<400> 66
gaaggtgtat cggcggcgga agttatcatg accgtactgc acgcaggcgg taaatttgat 60

gataactcct ataaagtttc cggtggtctg cacggtgttg gcgtgtcagt ggttaacgcc 120

ctgtcgcaaa aactggagct gctgatccgt cgtgaaggca aagtccacca gcagacttac 180

gtgcacggcg tgccgcaggc accgctggct gttaccggcg aaaccgacgc taccggtacg 240

caggtgcgtt tctggccgag ccatgaaacc ttcaccaacg tcaccgaatt tgaatatgac 300

atcctggcca agcgtctgcg cgagctgtcg ttcctgaact ccggcgtatc cattcgcctg 360

atcgacaaac gtgacggtaa agaagaccac ttccactacg aaggcggcat caaagcg 417

<210> 67
<211> 417
<212> DNA
<213> Enterobacter ludwigii

<400> 67
gagggcgtat cggctgcgga agttatcatg accgttctgc acgcaggcgg taagttcgat 60

gataactcct ataaagtttc cggcggtctg cacggtgtag gtgtgtccgt ggttaacgcc 120

ctgtcgcaga aactggagct ggttatccag cgcgaaggca aaattcaccg tcagctgtat 180

acgcacggcg tgccgcaggc accgctggct gttacgggtg acaccgacaa aaccggtacc 240

atggtgcgtt tctggccgag ccttgaaacg ttcaccaacg ttaccgagtt cgaatatgag 300

attctggcga aacgcctgcg tgaactgtcg ttcctgaact ccggtgtctc gattcgtctg 360

cgtgacaaac gcgacaacag agaagaccac ttccactacg aaggcggtat ccgggcg 417

<210> 68
<211> 417
<212> DNA
<213> Enterobacter nimipressuralis

<400> 68
gagggcgtat ccgctgcgga agtgatcatg accgtcctgc acgcaggcgg taaattcgat 60

gacaactcct ataaagtgtc cggcggcctg cacggcgtgg gcgtttccgt tgttaacgcg 120

ctgtcgcaga agctggaact ggttatctac cgcgaaggta aaatccatca ccaggtgtat 180

acccacggcg tgccacaggc accgctggcg gtcactggcg aaacggaaaa gaccgggacc 240

accgtgcgtt tctggccgag ccacgaaacc ttctctaatg tgactgactt cgaatacgac 300

attctggcaa aacgtctgcg cgagctgtcg ttcctgaact ccggcgtgtc catccgcctg 360

cgcgataagc gcgacggtaa agaagatcat ttccactacg aaggcggcat caaggcg 417

<210> 69
<211> 417
<212> DNA
<213> Enterobacter radicincitans

<400> 69
gagggcgttt ccgcagcgga agtgatcatg accgttctgc acgcaggcgg taagttcgac 60

gataactcct ataaagtttc cggcggtctg catggcgtag gtgtttccgt ggttaacgcc 120

ctgtcgcaga aactggagtt ggtgatccag cgcgacaaca aagtccaccg tcagatttat 180

gagcacggcg tgccgcaggc tccgctggcg gtcaccggtg aaaccgaatc gactggtacg 240

atggtgcgtt tctggccaag ctatgaaacc ttcaccaacg tcaccgaatt cgaatatgaa 300

attctggcga aacgcctgcg tgagttgtcc ttcctcaact ccggcgtttc tattcgcctg 360

cgcgacaagc gcgacggcaa agaagaccat ttccactatg aaggcggcat caaggcg 417

<210> 70
<211> 417
<212> DNA
<213> Enterobacter sakazakii

<400> 70
gaaggcgttt cggcggcgga agttatcatg accgtgctgc acgcgggcgg taaatttgat 60

gataactcct ataaagtctc cggtggtctg cacggcgtag gcgtatccgt ggttaacgcc 120

ctgtcccaga aactggagct ggtgattcgc cgtgaaggta aaatccacca gcaaacttac 180

gtgcacggcg tgccgcaagc gccgctggcc gtaaccggcg acaccgatca gaccggtact 240

caggtccgtt tctggccaag cctcgaaacc ttcaccaacg tcactgagtt tgaatacgat 300

attctggcga agcgcctgcg cgaactctcg ttcctgaact ccggcgtgtc gatccgtctc 360

gtggataagc gcgacggtaa gcaggaccac ttccattacg aaggcggcat caaggcg 417

<210> 71
<211> 417
<212> DNA
<213> Erwinia amylovora T

<400> 71
gaggggatct ctgccgctga agtgatcatg accgtgctgc atgcgggcgg gaagtttgat 60

gataactcct ataaagtttc cggcggcctg cacggcgtgg gtgtttccgt ggttaacgcc 120

ctgtcggaaa aactggaact gaccatccgc cgtgaaggca aagtccacca gcaggtctac 180

gttcacggcg tgccacaggc cgcgctgaat gtcaccggcg aaaccgattt gaccggaacg 240

cgggtgcgtt tctggcctag tcatcaaacc tttactaacg ttgttgaatt cgaatacgaa 300

atactggcaa aacgtctgcg tgagctgtcg ttcctgaact ccggcgtatc catcaagctg 360

gaagataagc gtgacggcaa aaacgaccat taccactacg aaggtggtat caaagcg 417

<210> 72
<211> 417
<212> DNA
<213> Erwinia aphidicola

<400> 72
gagggtatct ctgccgctga agtgatcatg accgtgctgc acgcgggcgg taagttcgat 60

gataactcat acaaagtttc cggcggcctg cacggcgtag gggtttccgt ggttaacgcc 120

ctgtcggaaa aactggagct gaccatccgc cgtgaaggca aagtccatca gcagatttac 180

gtgcacggcg tgccgcaggc cccgctgagc gtgaccggtg aaaccgatct gaccgggacg 240

cgcgtgcgtt tctggccaag ccatcagacc tttaccaacg tggtcgagtt cgaatacgaa 300

attctggcta agcgtctgcg tgagctgtcg ttcctgaact ccggcgtatc aatcaagctg 360

gaagacaagc gtgacggcaa gaacgaccat taccactacg aaggtggtat caaggcg 417

<210> 73
<211> 417
<212> DNA
<213> Erwinia billingiae

<400> 73
gaaggcattt ctgcggcaga agtgatcatg accgtgctgc acgcaggcgg taaatttgat 60

gacaactcct ataaagtttc cggtggtctg cacggcgtcg gcgtttcagt ggttaacgcc 120

ctgtctgaaa agctggagct gaccattcgc cgcgaaggca aggttcacaa tcagatttac 180

gttcatggcg tgccacaggc gccactgcac gtgaccggtg aaaccgatct gaccggtacc 240

cgcgtacgtt tctggccaag ccatcagacc ttcaccaacg tgaccgattt cgaatatgaa 300

atcctcgcca agcgtctgcg tgagctgtcg ttcctgaact ccggcgtgtc gatcaagctg 360

gaagacaagc gtgatgctcg tcaggatcac tatcactacg aaggcggcat caaggcg 417

<210> 74
<211> 417
<212> DNA
<213> Erwinia mallotivora

<400> 74
gagggtgtct ctgcggcaga agtcattatg accgtactgc acgcaggcgg taaatttgat 60

gacaactcgt ataaggtttc gggtggtctg cacggcgtgg gtgtttcggt ggttaatgcc 120

ctgtcggaaa aactggaact gaccattcgt cgtgaaggga aagtacacca gcagatctat 180

gtacacggtg taccacaggc accactaagc gtgactggcg aaaccgatct gaccggaacc 240

cgtgtgcgct tctggccaag ccatgaaact tttaccaatg tcactgattt cgaatatgaa 300

attttagcaa aacgtctgcg tgagctgtca ttcctcaatt ctggtgtttc catcaagctg 360

gaagacaagc gtgacgataa aaaagatcac taccattacg aaggtgggat caaggcg 417

<210> 75
<211> 417
<212> DNA
<213> Erwinia papayae

<400> 75
gagggtgtct ctgcggcaga agtcattatg accgtattgc acgcaggcgg taaatttgat 60

gacaactcgt ataaggtttc gggtggtttg cacggcgtgg gtgtttcggt ggttaatgcc 120

ctgtcggaaa aactggaact gaccattcgt cgtgaaggga aagtacacca gcagatctac 180

gtacacggtg taccacaggc accactgagc gtgactggcg aaaccgatct gaccggaact 240

cgtgtgcgct tctggccaag ccatgaaact tttaccaatg ttactgattt cgaatatgaa 300

attttagcaa aacgtctgcg tgagctgtca ttcctcaatt ctggtgtttc catcaagctg 360

gaagacaagc gtgacgataa aaaagatcac taccattacg aaggtgggat caaggcg 417

<210> 76
<211> 417
<212> DNA
<213> Erwinia persicina

<400> 76
gaaggcatct cagccgctga agtgatcatg accgtgctgc acgccggcgg taagttcgac 60

gataactctt ataaagtctc cggcggcctg cacggcgtgg gcgtgtcagt ggtgaacgcc 120

ctgtcggaaa aactggagct gaccattcgt cgcgaaggga aagttcacca gcagacttac 180

gtccacggcg tgccacaggc cccgttgagt gtgagcggtg aaactgacct gacgggaacg 240

cgcgtgcgtt tctggcccag ccatcagacg ttcactaacg tcgtggagtt cgagtacgaa 300

attttggcaa agcgcctgcg tgagctgtcg ttcctgaact ccggtgtatc aatcaagctg 360

gaagataagc gcgacggtaa aagcgaccat taccactatg aaggtggtat caaggcg 417

<210> 77
<211> 417
<212> DNA
<213> Erwinia psidii

<400> 77
gagggcgtat ccgcggccga agtgatcatg accgtactgc atgcgggcgg taagttcgat 60

gacaactcgt ataaggtatc cggtggcctg catggcgtgg gggtttccgt ggttaacgcc 120

ctgtcggaga aactggagct gaccattcgt cgtgaaggca aggtacacca gcagatttac 180

gttcatggtg tgccgcaggc tccgctgagc gtgaccggtg aaaccgattt gactggcacg 240

cgggtgcgtt tctggccaag ccatgcaacc tttactaacg tcactgactt tgaatatgaa 300

attctggcta agcgcctgcg tgagctgtca tttctgaact ccggcgtctc aatcaaactt 360

gaagacaaac gtgacgacaa gaaagatcac taccattatg aaggtggcat taaagcc 417

<210> 78
<211> 417
<212> DNA
<213> Erwinia pyrifoliae

<400> 78
gagggtatct ctgccgctga agtgatcatg accgtgctgc atgccggcgg taagtttgac 60

gataactcct ataaagtttc cggcggcctg cacggcgtgg gcgtttccgt ggttaacgcc 120

ctgtcggaaa aactggaact gaccattcgt cgtgaaggta aagtccacca gcaggtctac 180

gttcacggcg tgccacaggc cgcgctgaat gtcaccggtg aaaccgatct gaccggcacg 240

cgggtgcgtt tctggccaag ccatcagacg tttaccaatg ttgtcgaatt cgaatacgag 300

atcctggcaa aacgcctgcg tgagctgtcg ttcctgaact ccggcgtctc catcaagctg 360

gaagataagc gtgacggcaa aaacgaccat taccactatg aaggcggtat caaggcg 417

<210> 79
<211> 417
<212> DNA
<213> Erwinia rhapontici

<400> 79
gaaggtattt ctgccgctga agtgatcatg accgtgctgc acgcaggcgg aaagttcgat 60

gataactcct ataaagtgtc cggcggcctg catggtgtgg gtgtttccgt ggttaacgcc 120

ctgtcggaaa aactggaact gaccattcgt cgtgaaggca aagttcacca gcagatctac 180

gtccatggtg tgccgcaggc gccgttaagc gttagcggtg aaaccgatct gaccggaaca 240

cgcgtgcgtt tctggccaag ccatcagacc tttaccaacg tcgttgaatt cgaatacgaa 300

attctggcaa aacgcctgcg cgagctgtcg ttcctgaact ctggcgtctc tatcaagctg 360

gaagataagc gtgatggtaa aagcgaccat taccactatg aaggtggtat taaggcg 417

<210> 80
<211> 417
<212> DNA
<213> Erwinia toletana

<400> 80
gagggtgtct ctgccgctga agttatcatg acggtattgc atgccggcgg taagttcgat 60

gataactcgt ataaagtttc cggtggtctg catggcgtag gtgtgtcagt ggttaacgcc 120

ctgtcacaga agctggagct gaccattcgc cgtgaaggca aagttcatca gcaggtttac 180

gtacatggcg tgccacaggc gccgctggcg gtctcgggtg aaaccgacct taccggtact 240

cgcgtgcgtt tctggccaag ccatgaaacc tttaccaacg tcattgaatt tgaatatgaa 300

attctggcga agcgtctgcg tgaactttcg ttcctgaact ccggcgtttc cattcgtctg 360

gaagataagc gcgacggcaa aagcgatcac tatcactacg aaggcggtat caaggcg 417

<210> 81
<211> 417
<212> DNA
<213> Escherichia albertii

<400> 81
gagggcgtat cggcagcgga agtgatcatg accgtgctgc acgcaggcgg taaatttgac 60

gataattcct ataaagtgtc cggcggtctg cacggcgttg gtgtttcggt agtaaacgcc 120

ctgtcgcaaa aactggagct ggttattcag cgcgagggta aaattcaccg tcagatctac 180

gaacacggtg ttccgcaggc accgctggcg gttactggtg acaccgacaa aaccggcacc 240

atggtgcgtt tctggccaag ccttgaaact ttcaccaatg tgaccgagtt cgaatacgat 300

attctggcga agcgtctgcg tgagctgtcc ttcctcaatt ccggcgtgtc tattcgtctg 360

aaagacaaac gcgacggcaa agaagaccac ttccactatg aaggcggcat taaggcg 417

<210> 82
<211> 417
<212> DNA
<213> Escherichia blattae

<400> 82
gagggcgtgt ccgctgccga agtgattatg actgtgctgc acgctggcgg taaattcgac 60

gataactcct ataaagtctc cggcggtctg cacggcgtgg gggtatctgt tgtgaacgcc 120

ctgtctcaga agctggagct gaccatccgc cgcgacggga aagtgcacca ccagatttat 180

cagcacgggg agccccaggc acccctgtcc gttacgggcg acaccgaagt gaccggcacc 240

atggtgcgtt tctggccgag cctggaaact ttcaccaatg tgaccgagtt tgaatatgaa 300

gtgctggcaa aacgcctgcg tgaactttcg ttcctgaact ccggcgtctc tattcgcctt 360

atcgacaagc gcgacggcaa agaagaccat ttccactacg aaggcggtat caaagcc 417

<210> 83
<211> 417
<212> DNA
<213> Escherichia coli T

<400> 83
gagggcgtat cggcggcgga agtgatcatg accgttctgc acgcaggcgg taaatttgac 60

gataactcct ataaagtgtc cggcggtctg cacggcgttg gtgtttcggt agtaaacgcc 120

ctgtcgcaaa aactggagct ggttatccag cgcgagggta aaattcaccg tcagatctac 180

gaacacggtg taccgcaggc cccgctggcg gttaccggcg agactgaaaa aaccggcact 240

atggtgcgtt tctggccaag ccttgaaacc ttcaccaatg tgaccgagtt cgaatatgac 300

attctggcga aacgtctgcg tgagttgtcg ttcctcaact ccggcgtttc cattcgtctg 360

cgcgacaagc gcgacggcaa agaagaccac ttccactatg aaggcggcat caaggcg 417

<210> 84
<211> 417
<212> DNA
<213> Escherichia fergusonii

<400> 84
gagggcgtat cggcggcgga agtgatcatg accgtcctgc acgcaggcgg taaatttgac 60

gataactcct ataaagtgtc cggcggtctg cacggcgttg gtgtttcggt agtaaacgcc 120

ctgtcgcaaa aactggagct ggttatccag cgcgagggta aaattcaccg tcagatctac 180

gaacacggtg taccgcaggc tccgctggcg gttaccggcg agactgaaaa aaccggcacc 240

atggtgcgtt tctggccaag cctcgaaacc ttcaccaatg tgaccgaatt cgaatatgac 300

attctggcga aacgcctgcg tgagttgtcg ttcctcaact ctggcgtttc cattcgtctg 360

cgcgacaagc gcgacggcaa agaagaccac ttccactatg aaggcggtat caaggcg 417

<210> 85
<211> 417
<212> DNA
<213> Escherichia hermannii

<400> 85
gaaggggtgt ctgcggcgga agttatcatg acggtgctcc acgcgggcgg taagttcgac 60

gataactcct ataaagtttc tggcggcctg cacggcgtgg gtgtatcggt ggttaacgcc 120

ctttcccaga aactggagct ggtgatccgc cgtgaaggca aagtgcacca gcaaacctac 180

gtacacggcg tgccgcaagc gccgctggcg gtaacgggcg ataccgaagc aacagggacc 240

caggtgcgtt tctggccgag cctggaaacc tttaccaacg ttgtcgaatt tgaatacgaa 300

attctggcga agcgcctgcg tgagctctcc ttcctgaact ccggcgtgtc cattcgtctg 360

cgcgataagc gtgacggcaa agaagatcat ttccactacg aaggcggtat caaggct 417

<210> 86
<211> 417
<212> DNA
<213> Escherichia vulneris

<400> 86
gagggcgtct ccgccgctga agttatcatg accgtgctgc acgcaggcgg taaattcgat 60

gataactcct ataaagtgtc cggcggcctg cacggcgtgg gggtctccgt ggttaacgcc 120

ctgtcgcaaa agcttgagct gctgatccgc cgcgaaggca aagtgcacca gcagacctac 180

gtgcacggtg tgccgcaggc cccgctggca gtgaccggcg atactgacgt caccggtacg 240

gtggtgcgtt tctggcctag cctggagacc ttcaccaacg tcaccgaatt tgagtatgag 300

atcctggcca agcgcctgcg cgagctgtca ttcctgaact ctggcgtttc cattcgcctg 360

caagacaagc gcgacggtaa agaagatcac ttccactacg aaggcggcat caaggcg 417

<210> 87
<211> 417
<212> DNA
<213> Ewingella americana T

<400> 87
gagggcgttt ctgctgctca ggtcatcatg accgttctgc atgccggcgg taaattcgat 60

gataactcgt acaaagtctc tggcggcttg cacggcgtgg gtgtttccgt cgttaacgcc 120

ctgtcagaaa aactcgagtt ggttatccgc cgcgaaggca aagtgcatga gcaaacttac 180

gtgcacggcg agccacaaac cccgctgaaa gtggttggtg aaactgaaac taccggtacc 240

accgtgcgtt tctggccgag cttccagacc tttactaacc aaaccgaatt ccagtacgac 300

attctggcga aacgcctgcg tgaactgtcg ttcctcaact ccggtgtggc aattcgtctg 360

ctggacaagc gtgacgataa gagcgatcac ttccactacg aaggcggtat caaggct 417

<210> 88
<211> 417
<212> DNA

<213>  Hafnia alvei T

<400>  88
gaaggtgtgt ctgctgccga agttatcatg accgtgctgc acgccggtgg taagttcgat      60

gataactctt ataaagtttc cggtggtttg cacggcgttg gtgtttccgt agttaacgcc     120

ctgtctgaaa aattggaatt ggttattcgc cgtgaaggta aagttcacga gcagacctac     180

gcgcacggtg ttccccagtt cccgctgaaa gtggttggtg atactgaaat taccggtacc     240

accgtacgtt tctggccaag cctgcagacg ttctctaacc ataccgaatt ccagtacgat     300

attctggcta agcgcctgcg tgagctctcc ttcttgaact ccggcgtgtc tattcgtctg     360

cgcgacaaac gtgacaaccg tgaagatcat ttccactatg aaggcggtat caaagcg        417

<210>  89
<211>  417
<212>  DNA
<213>  Klebsiella oxytoca

<400>  89
gagggcgtgt cggcggcgga agtgatcatg accgttctgc acgcaggcgg taaattcgat      60

gataactcct ataaagtttc cggcggtctg cacggcgtag gcgtttccgt tgttaacgcc     120

ctgtcgcaga agctggagct ggttattcag cgcgataaca aaattcaccg tcaggtttat     180

gcccacggcg tgccgcaggc tccgctggcg gttaccggcg aaaccgaaaa aaccggtacc     240

atggtgcgct tctggccgag ctacgaaacc tttaccaacg ttgtcgaatt cgaatacgag     300

attctggcaa aacgcctgcg cgagctgtcg ttcctgaact ccggagtgtc gattcgcctg     360

cgcgacaagc gcgacggcaa agaagatcat ttccactacg aaggcggtat caaagcc        417

<210>  90
<211>  417
<212>  DNA
<213>  Klebsiella pneumoniae sbsp. ozaenae

<400>  90
gagggcgtct ccgcagcgga agttatcatg accgtcctgc acgctggcgg taaattcgat      60

gataactcct ataaagtttc cggcggtctg cacggcgtag gcgtctcggt ggttaacgcc     120

ctgtcgcaga agctggagct ggttatccag cgcgataaca aagttcacaa gcagatgtac     180

gaacatggtg tgccgcaggc accgctggcc gtgactggcg aaaccgacaa aaccggcacc     240

atggtgcgtt tctggccaag cctggaaacc ttcaccaatg tcaccgaatt tgaatatgac     300

atcctggcga aacgcctgcg cgagctgtcg ttcctgaact ccggcgtctc tatccgtctg     360

cgcgataagc gcgatggcaa agaagaccat ttccactacg aaggcggcat caaggcg        417

<210>  91
<211>  417
<212>  DNA
<213>  Klebsiella pneumoniae sbsp. pneumoniae T

<400>  91
gagggcgtct ccgcagcgga agttatcatg accgtcctgc acgctggcgg taaattcgat      60

gataactcct ataaagtttc cggcggtctg cacggcgtag gcgtctcggt ggttaacgcc     120

ctgtcgcaga agctggagct ggttatccag cgcgataaca aagttcacaa gcagatgtac     180

gaacacggtg tgccgcaggc accgctggcc gtgactggcg aaaccgacaa aaccggcacc     240

atggtgcgtt tctggccaag cctggaaacc ttcaccaatg tcaccgaatt tgaatatgac     300

atcctggcga aacgcctgcg cgagctgtcg ttcctgaact ccggcgtctc tatccgcctg     360

cgcgataagc gcgacggcaa agaagaccat ttccactacg aaggcggcat caaggcg        417

<210>  92
<211>  417
<212>  DNA
<213>  Klebsiella pneumoniae sbsp. rhinoscleromatis

<400>  92
gagggcgtct ccgcagcgga agttatcatg accgtcctgc acgctggcgg taaattcgat      60

gataactcct ataaagtttc cggcggtctg cacggcgtag gcgtctcggt ggttaacgcc     120

ctgtcgcaga agctggagct ggttatccag cgcgataaca aagttcacaa gcagatgtac     180

gaacacggtg tgccgcaggc accgctggcc gtgactggcg aaaccgacaa aaccggcacc     240

atggtgcgtt tctggccaag cctggaaacc ttcaccaatg tcaccgaatt tgaatatgac     300

atcctggtga aacgcctgcg cgagctgtcg ttcctgaact ccggcgtctc tatccgtctg     360

cgcgataagc gcgacggcaa agaagaccat ttccactacg aaggcggcat caaggcg        417

<210>  93
<211>  417
<212>  DNA
<213>  Klebsiella singaporensis

<400>  93
gagggcgtct ccgcagcgga agttatcatg accgtcctgc acgccggcgg taaatttgat      60

gataactcct ataaagtgtc cggcggtctg cacggcgtag gcgtctcggt ggttaacgcc     120

ctgtcgcaga agctggagct cgttatccag cgcgataaca aagtccacaa acagatttat     180

gtgcacggtg taccgcaggc gccgctggcc gtgactggcg aaaccgacaa aaccggcacc     240

atggtgcgtt tctggccaag cctggaaacc ttcaccaatg tcaccgaatt tgaatatgac     300

atcctggcga aacgcctgcg cgagctgtcg ttcctgaact ccggcgtctc tatccgcctg     360

cgcgataagc gcgatggcaa agaagaccat ttccactacg aaggcggcat caaggcg        417

<210>  94
<211>  417
<212>  DNA
<213>  Klebsiella variicola

<400>  94
gagggcgtct ccgcagcgga agttatcatg accgtcctgc acgccggcgg taaatttgat      60

gataactcct ataaagtgtc cggcggtctg cacggcgtag gcgtctcggt ggttaacgcc     120

ctgtcgcaga agctggagct ggttatccag cgcgataaca aagtccacaa acagatttat     180

gtgcacggtg taccgcaggc gccgctggcg gtgaccggcg agaccgataa aaccggcacc     240

atggtgcgtt tctggccgag ccatgaaacc ttcaccaacg ttactgaatt cgaatatgac 300

atcctggcaa aacgtctgcg cgagctgtcg ttcctgaact caggggtctc tatccgcctg 360

cgcgataagc gcgacggcaa agaagatcat ttccactacg aaggcggcat caaggcg 417

<210> 95
<211> 417
<212> DNA
<213> Kluyvera ascorbata T

<400> 95
gagggcgttt ctgctgcgga agtcatcatg accgttctgc acgcaggcgg taagttcgac 60

gataactcct ataaagtctc cggcggtctg cacggcgtgg gcgtttccgt ggttaacgcc 120

ctgtcccaga aactggaact ggttatccag cgcgataaca aaattcaccg tcagatttac 180

gcgcacggcg tgccgcaggc accgctggcc gtcaccggcg ataccgaaaa aaccggtacc 240

atggtacgtt tctggccgag ctacgaaacc ttcactaatg tcgtcgagtt cgaatacgat 300

attctggcaa aacgtctgcg tgagctgtcg ttcctgaact ccggcgtttc tattcgtctg 360

cgcgataagc gtgacggtaa agaagaccat ttccactacg aaggcggcat caaggcg 417

<210> 96
<211> 417
<212> DNA
<213> Kluyvera cochleae

<400> 96
gaaggcgttt ctgcggccga agttatcatg accgttctgc atgcaggcgg taaatttgat 60

gataactcct ataaagtgtc cggtggtttg cacggcgtgg gtgtttcggt tgttaacgcc 120

ctgtcgcaga aactggaatt gctgatccgc cgcgaaggca aagtacatca gcaaacttat 180

gtccacggtg tgccacaggc tccgctggcg gtaaccggtg aaacggaagc gaccggtaca 240

caggtgcgtt tctggccaag ccacgaaacc ttcaccaacg ttatcgaatt cgaatatgac 300

attctggcaa aacgcctgcg cgagctgtcg ttcctgaact ccggcgtttc catccgtctg 360

cgcgacaagc gcgatggcaa agaagatcat ttccactacg aaggcggcat caaggcg 417

<210> 97
<211> 417
<212> DNA
<213> Kluyvera cryocrescens

<400> 97
gagggcgtat cggcggcgga agtgatcatg accgttctgc acgcaggcgg taaattcgat 60

gataactcct acaaagtttc cggtggtctg cacggcgtcg gcgtgtcggt tgtaaacgcc 120

ctgtcgcaga agctggaact ggttatccag cgcgataaca aaatccacaa acagatttat 180

gcgcacggcg tgccgcaggc tccgctagcg gtcactggcg ataccgagaa aaccggtacc 240

atggtgcgtt tctggccgag ctacgaaacc ttcaccaatg tcattgaatt cgaatatgac 300

attctggcaa aacgcctgcg cgagctgtca ttcctgaact ccggcgtgtc tatccgtctg 360

cgcgataagc gcgacggcaa agaagatcat ttccactacg aaggtggcat caaggca 417

<210> 98
<211> 417
<212> DNA
<213> Kluyvera georgiana

<400> 98
gagggcgtct ccgcagcgga agttatcatg accgtgctgc acgccggcgg taaattcgat 60

gataactcct ataaagtgtc cggcggtctg cacggcgtag gcgtctcggt cgttaacgcc 120

ctgtcccaga agctggaact ggttatccag cgcgataaca aagttcaccg ccagatctat 180

gagcacggcg tgccgcaggc accgctggcc gtcacgggtg ataccgaaaa aaccggtacc 240

atggtgcgtt tctggcctag ctatgaaact ttcaccaacg ttaccgaatt cgaatatgac 300

atcctggcaa aacgtctgcg cgagctgtcg ttcctgaact ccggcgtgtc catccgtctg 360

cgcgacaaac gcgacggcaa agaagatcat ttccactacg aaggcggcat caaggcg 417

<210> 99
<211> 417
<212> DNA
<213> Kluyvera intermedia

<400> 99
gaaggcgttt ctgcggccga agttatcatg accgttctgc acgcaggcgg taaatttgat 60

gataactcct ataaagtgtc cggtggtttg cacggcgtag gtgtttcggt tgttaacgcc 120

ctgtcgcaga aactggaatt gctgatccgc cgcgaaggca aagtacatca gcaaacttat 180

gtccacggtg tgccacaggc tccgctggcg gtaaccggtg aaacggaagc gaccggtaca 240

caggtgcgtt tctggccaag ccacgaaacc ttcaccaacg taatcgaatt cgaatatgag 300

attctggcaa aacgtctgcg cgagctgtca ttcctgaact ccggcgtttc catccgtctg 360

cgcgataagc gtgacggcaa agaagaccat ttccactatg aaggtggtat caaggcg 417

<210> 100
<211> 417
<212> DNA
<213> Leclercia adecarboxylata T

<400> 100
gagggcgtct ctgcagcgga agttatcatg accgtcctgc acgcaggcgg taagttcgat 60

gataactcct ataaagtatc cggcggcctg cacggcgtgg gtgtctctgt ggttaacgcc 120

ctgtcgcaga agctggaact gctgatccgt cgcgaaggca aagtgcatca gcaaacctac 180

gtgcacggcg tacctcaggc tccgctggcg gttaccggtg aaactgacgt aaccgggact 240

caggtccgtt tctggccgag ccatgaaacc ttcaccaacg tcaccgagtt tgaatacgat 300

atcctcgcca aacgtctgcg cgagctgtca ttcctgaact ccggtgtctc tattcgcctg 360

cgcgacaagc gcgacggcaa agaagaccac ttccactacg aaggcggcat caaggcg 417

<210> 101
<211> 417

<212>  DNA
<213>  Leminorella grimontii T

<400>  101
gagggcgtgt cggcggcgga ggttatcatg accgtactgc acgccggcgg caagtttgac      60

gataactcgt ataaagtttc cggcggcctg cacggcgtag gggtatcggt agtgaacgcg      120

ctgtccgata agctggagat ggttatcgat cgcggcggca aggtctaccg acaaatctat      180

cgtcacggcg tgccggaagc gccgcttgcg cctatcggcg agagcgataa aaccggtacc      240

cgcattcgct tctggccaag tccggacacc ttttccagca atctggaatt ccagtacgac      300

attctggcca agcgcctgcg cgagctgtcg tttttgaact ccggcgtttc tatcaagcta      360

aacgatgagc gcgacgataa gcacgatcac ttccactatg aaggcggcat caaggcg      417

<210>  102
<211>  417
<212>  DNA
<213>  Leminorella richardii

<400>  102
gagggcgtat cggcagcgga agtcatcatg actgtgctgc atgctggcgg caagtttgac      60

gataactcgt ataaagtttc cggtggtctg cacggcgtag gtgtttcagt ggtaaacgcc      120

ctgtccgaaa agctggaaat ggtcatcgac cgcggcggca aagtttataa gcagatctat      180

cgccacggcg ttcctgaggc gccgctggct cctttcgccg acagcgaaaa aaccggtact      240

cgtattcgct tttggccaag cgcggacact ttctccagca atctggagtt tcagtacgag      300

atcctggcca agcgcctgcg cgagctgtcg tttttgaact ctggcgtttc tatcaagctg      360

aacgacgagc gcgatgataa gcacgatcac ttccactacg aaggcggcat caaggcg      417

<210>  103
<211>  417
<212>  DNA
<213>  Moellerella wisconsensis T

<400>  103
gaaggagttt cggcggcgca agttatcatg accgtcctgc atgcgggagg caaattcgat      60

gataactcct ataaagtctc cggcggtttg cacggtgtag gggtatctgt tgttaacgcc      120

ttgtctgaaa aactggaact ggttatccgt cgtgatggta aagtccatga acaaacttat      180

cgtcatggtg agccacaaag tccattagcc gtcgtcggtg aaactgattt gaccggaacc      240

atggttcgct tctggccaag ccatgagacc ttcaaaggca ttaccgaata ccaatatgat      300

attctcgcta aacgactgcg tgaactgtca ttcctgaatt caggcgtatc cattcgatta      360

atcgatgaac gtgataatcg ccaagatcat ttccattatg aaggtggtat taaggcc      417

<210>  104
<211>  417
<212>  DNA
<213>  Morganella morganii sbsp. morganii T

<400>  104
gaaggcgtct ccgccgcaga agttatcatg actgttctgc acgccggcgg gaagttcgat      60

gataactcct ataaagtctc aggcggcctg cacggcgtcg gggtctctgt tgttaacgcc 120

ctgtctgaaa aactggaact ggttatccgc cgtgacggca aagttcacga gcagatctac 180

cgccacggtg aaccgcagga tcgcctgact gttgtcggcg aaactgacaa aaccgggaca 240

cgcgtgcgtt tctggccgag catggacacc ttcaaaggcg agactgaatt ccagtacgac 300

attctggcaa aacgcctgcg cgaactctcc ttcctgaact ccggtgtatc gatccgtctg 360

atcgataaac gcgacggcaa agaagatcac ttccactacg aaggcggtat caaagcg 417

<210> 105
<211> 417
<212> DNA
<213> Morganella morganii sbsp. sibonii

<400> 105
gaaggcgtct ccgccgcaga agttatcatg actgttctgc atgccggcgg gaagttcgat 60

gataactcct ataaagtctc gggcggcctg cacggcgtcg gggtctctgt tgttaacgcc 120

ctgtctgaaa aactggaact ggttatccgc cgtgacggca aagtccacga gcagatctac 180

cgccacggtg agccgcagga tcgcctgacc attgtcggcg aaaccgataa aaccggaaca 240

cgcgtgcgtt tctggccgag catggacacc ttcaaaggcg agactgaatt ccagtacgat 300

attctggcaa aacgcctgcg tgaactttcc ttcctgaact ccggtgtgtc gatccgtctg 360

gtcgacaaac gcgacggcaa agaagaccat ttccattacg aaggcggtat caaagca 417

<210> 106
<211> 417
<212> DNA
<213> Obesumbacterium proteus T

<400> 106
gaaggtgtgt ctgctgccga agttatcatg accgtgctgc acgccggcgg taagttcgat 60

gacaactctt ataaagtttc cggtggtctg cacggcgttg gtgtttccgt ggttaacgcc 120

ctgtctgaaa aattggaatt gattatccgc cgtgaaggta aagttcacga gcagacttac 180

gcccacggcg ttcctcagtt cccgctgaaa gtggttggcg atactgaaat taccggtacc 240

accgtgcgtt tctggccgag catggagacg ttcagcaacc atactgaatt ccaatacgat 300

attctggcta agcgcctgcg tgagctctcc ttcttgaact ccggcgtctc tattcgcctg 360

cgcgacaagc gtgataaccg tgaagatcat ttccactatg aaggcggtat taaagcg 417

<210> 107
<211> 417
<212> DNA
<213> Pantoea agglomerans T

<400> 107
gagggcattt ctgccgctga agtgatcatg accgtgctgc atgctggcgg taagttcgat 60

gataactcct ataaagtctc cggcggcctg cacggcgtgg gcgtctccgt ggttaacgcc 120

ctgtcgcaga agctggagct gaccattcgt cgcgaaggca aagtgcatca gcagatttac 180

gtccatggcg taccgcaggc accactggcg gtgacgggtg agaccgacat taccgggacc 240

cgtgtgcgtt tctggccaag ccacgaaacc tttaccaacg tgcgcgattt tgagtatgac 300

atcctggcga aacgcctgcg tgaactctca ttcctgaact ccggcgtgtc gattcgtctg 360

gatgataagc gtgatggtaa aagtgatcac ttccactatg aaggcggcat ccgggcg 417

<210> 108
<211> 417
<212> DNA
<213> Pantoea ananatis

<400> 108
gaaggtgtgt cggcggctga agttatcatg accgtgctgc acgctggcgg taagtttgac 60

gacaactcct acaaagtgtc tggcggcctg cacggcgtag gtgtttccgt tgtgaacgcc 120

ctgtcccaga aactggaact gaccattcgt cgtgaaggca aagtgcatca gcagatttat 180

ctgcacggcg tacctgaagc gccgctggcg gttacgggag ataccgatgc aaccggtacc 240

cgcattcgtt tctggccaag ccatgacacc ttcaccaacg ttaccgattt cgaatatgac 300

attctggcga aacgcctgcg cgaactgtcc ttcctgaact ctggcgtatc gattcgtctg 360

gaagacaagc gtgatgcaag aaacgaccac tttcactacg aaggtggtat ccgcgcc 417

<210> 109
<211> 417
<212> DNA
<213> Pantoea citrea

<400> 109
gaaggtgttt ctgctgccga agttatcatg acggtactgc atgccggcgg taagtttgat 60

gataactcgt ataaagtgtc aggcggcctg catggtgtcg gtgtttcggt ggtgaatgcc 120

ttatccgaaa aactggaact gacgattcgt cgtgatggta aagtccatca gcaaacctat 180

ctgcacggcg taccaagtgc accactgaat gtggtaggcg aaactgattc taccgggact 240

tcagttcgtt tctgggccag ccatcagacg tttaccaatg tcactgactt tgagtatgac 300

atcctggcga agcgcctgcg tgaactgtcg ttcctgaatt caggggtctc tatccggctg 360

atcgacaagc gttcagacaa gagtgatcat tatcactatg aaggcggcat taaagcg 417

<210> 110
<211> 417
<212> DNA
<213> Pantoea dispersa

<400> 110
gagggcgtct ctgctgccga agtgatcatg accgtactgc atgccggcgg taagtttgat 60

gacaactctt ataaagtttc cggtggtctg cacggcgtcg gcgtatcggt ggtgaacgcc 120

ctgtcgcaga agctggaact gaccattcgt cgcgacggca aagtgcatca gcaaacctac 180

gtgcacggtg tgccgcaggc gccactggcg gtgaccggcg ataccgacgc taccggtacg 240

cgcgtgcgtt tctggccgag ccacgatacg ttcaccaacg tcaccgattt tgaatatgaa 300

atcctggcga agcgtctgcg cgaactgtca ttccttaact ccggcgtgtc gattcgcctg 360

gaagacaagc gcgacgataa aaacgaccat taccactacg aaggtggtat caaagcg        417


<210>  111
<211>  417
<212>  DNA
<213>  Pantoea punctata

<400>  111
gaaggggtat cggctgccga agtcattatg acggtactgc atgccggggg gaaatttgac        60

gataactcct ataaagtctc cggcggtttg catggtgtcg gcgtttctgt ggttaacgcc       120

ttatcggaaa aactggaact gaccattcgt cgtgatggta aagttcatca gcaaacctat       180

ctgcacggtg tcccgggcgc acctctggcc gttatcggcg aaacggacag caccgggacg       240

tcggttcgtt tctgggcaag ccaccagact ttcaccaatg tcaccgactt tgaatacgac       300

atcctggcca aacgtctgcg tgagttatcg ttccttaact ccggagtttc tatccggctg       360

atcgataaac gttctgacaa gagcgatcat tatcactacg aaggtggtat taaagcc          417


<210>  112
<211>  417
<212>  DNA
<213>  Pantoea stewartii sbsp. indologenes

<400>  112
gaaggcgtgt ccgcggcaga agtgatcatg acggtgctgc acgctggcgg taagttcgac        60

gataactcgt ataaagtctc gggcggcctg cacggcgtgg gcgtgtctgt ggttaacgcc       120

ctgtcacaga agctggagct gaccattcgt cgcgaaggca aagtgcacca gcagatttat       180

ctgcatggcg tgcccgaggc gccgctggcg gtgacaggcg ataccgacgc gaccggtacc       240

cgtatccgtt tctggccaag ccatgacacg ttcaccaacg tgaccgactt cgaatacgac       300

attctggcga agcgcctgcg tgagctctct ttcctgaact ccggtgtttc cattcgtctg       360

gaagacaaac gtgacggcaa aaacgaccat ttccactatg aaggtgggat taaagcg          417


<210>  113
<211>  417
<212>  DNA
<213>  Pantoea stewartii sbsp. stewartii

<400>  113
gaaggcgtgt ccgcggcaga agtgatcatg accgtgctgc acgctggcgg taagttcgac        60

gataactcgt ataaagtctc gggcggcctg cacggcgtgg gcgtgtctgt ggttaacgcc       120

ctgtcacaga agctggagct gaccattcgt cgcgaaggca aagtgcacca gcagatttat       180

ctgcatggcg tgcccgaggc gccgctggcg gtgacaggcg ataccgacgc gaccggtacc       240

cgtatccgtt tctggccaag ccatgacacg ttcaccaacg tgaccgactt cgaatacgac       300

attctggcga agcgcctgcg tgagctctct ttcctgaact ccggtgtttc cattcgtctg       360

gaagacaaac gtgacggcaa aaacgaccat ttccactatg aaggtgggat taaagcg          417


<210>  114

<211>  417
<212>  DNA
<213>  Pantoea terrea

<400>  114
gagggtgtct cggcggcaga agtgattatg acagtactgc acgccggcgg taagtttgat      60

gataactctt ataaagtctc cggtggtttg catggtgtcg gtgtttctgt cgtcaatgcc     120

ttgtctgaaa agctggaact gactatccgc cgtgacggta aagtccatca gcaaatttat     180

cagcacggtg taccgaccgc tccgctgact gtgatcggcg aaacagaggt gaccgggacg     240

gcggtacgtt tctgggccag tcatcagaca ttcacgaatg tcaccgattt cgaatacgac     300

attctggcca aacgcctgcg cgaactctct ttcctgaatt ccggcgtgtc tatccgcctg     360

atcgacaagc gcaccgataa gagtgatcac taccactatg aaggtggtat ccgcgcg        417


<210>  115
<211>  420
<212>  DNA
<213>  Pectobacterium atrosepticum

<400>  115
gaagggatct ctgcggctga agtcatcatg actgtgctgc atgcgggcgg taagttcgac      60

gataactcgt ataaagtctc cggcggcctg cacggcgtag gggtttctgt ggttaacgcc     120

ctgtcggaaa aactgaagct ggttatccac cgtgaaggga agcttcacga gcagacctat     180

aagcacggcg tggcgcaggc accgctggcg gtcacgggtg aaaccgacag aacgggtacg     240

acggtgcgtt tctggccgag ccacgagacg ttcagcaatg tgctggaatt cgagtatgag     300

atcctggcca agcgtctgcg tgagctatcg ttcctgaact ccggcgtctc tattcgcctg     360

attgacgatc gtgagaaaga taaagccgat cattaccatt atgatggcgg tatcaaggca     420


<210>  116
<211>  420
<212>  DNA
<213>  Pectobacterium betavasculorum

<400>  116
gaagggatct ccgctgctga agtcatcatg accgtgctgc acgcgggcgg taagttcgat      60

gataactcgt ataaagtctc cggcggcctg cacggcgtag gggtttccgt ggttaacgcc     120

ctgtcggaaa aactgacgct ggttattcac cgcgaaggga agcttcacga gcagacctat     180

aaacacggcg tggcgcaggc accgctggcg gtcacgggtg aaaccaacag aaccggtacg     240

acggtgcgtt tctggccgag ccacgagacg ttcaccaacg tggtggaatt cgagtatgag     300

attctggcca agcgtctgcg cgagctgtcg ttcctgaact ctggcgtctc tattcgcctg     360

atcgatgagc gtgagaaaga taaagccgat cattaccatt atgaaggcgg catcaaggca     420


<210>  117
<211>  420
<212>  DNA
<213>  Pectobacterium cacticida

<400>  117

gaagggattt ccgctgcgga agtcatcatg acggtgctgc atgccggcgg taagttcgat 60

gataactcgt ataaagtctc cggcggtctg cacggtgttg gggtttccgt ggtgaacgcc 120

ctgtcggaaa agctggagct gaccatttat cgtgacggta aaattcacca ccaggtttat 180

cgacacggtg tggcgcaggc gccgctggcg ataacggggg aaaccgatag aacgggtact 240

accgtgcgct tttggccgag ccatgagaca ttcaccaacg tgctggaatt tgagtatgag 300

attctggcca aacgcttgcg cgagttgtcg ttcctgaact ccggcgtctc tatccgcctg 360

agcgacgagc gcgagaaaga taaagccgac cacttccact atgagggcgg catcaaggca 420

<210> 118
<211> 417
<212> DNA
<213> Pectobacterium cypripedii

<400> 118
gagggcgtat cggcggctga agtcatcatg acggtactgc atgcgggcgg taagttcgac 60

gacaactcct ataaagtttc cggtggtctg cacggtgtgg gtgtctccgt agtaaacgcc 120

ctgtcgcaga aactggaact gaccattcgt cgcgatggca aagtgcacca gcaaacctac 180

attcacggcg tgccgcaggc accgctggcc gtggtcggtg acactgaagc caccggtacg 240

cgcgtgcgtt tctggccgag ccatgaaacc ttcaccaatg tgacggaatt tgagtacgag 300

atcctggcaa aacgcctgcg cgaactgtcg ttcctcaatt ctggcgtgtc gattcgcctt 360

gaagacaagc gcgatgacaa aaacgaccat taccactacg aaggtggtat caaggcg 417

<210> 119
<211> 420
<212> DNA
<213> Pectobacterium wasabiae

<400> 119
gaagggatct ccgctgctga agttatcatg accgtgctgc atgcgggcgg taagttcgat 60

gataactcgt ataaagtctc cggcggcctg cacggcgtag gggtttccgt ggttaacgcc 120

ctgtcggaaa aactgaaact ggttatccac cgcgaaggga aacttcacga acaaacctac 180

aaacacggtg tagcgcaggc tccgctggcg gtcacaggcg agaccaacag aaccggcacg 240

acggtgcgct tctggccgag ccacgagacg ttcaccaatg tggtggaatt cgagtatgaa 300

attctggcta agcgtctgcg cgaactgtcg ttcctgaact ctggcgtctc tatccgcctg 360

atcgacgagc gtgaaaaaga taaagctgat cattaccatt atgatggcgg catcaaggca 420

<210> 120
<211> 417
<212> DNA
<213> Photorhabdus asymbiotica sbsp. australis

<400> 120
gaaggtgttt cagcagccga agttattatg actgtgctgc atgcgggggg taaattcgat 60

gataactctt ataaagtctc cggcggtctg cacggtgtag gggtttccgt tgttaacgcc 120

ttgtctgaaa agctggaact ggttatccgc cgtgacggta aagttcatga acagacttat 180

caccttggcg tgccacaaag cccactgaaa atcgttggag aaactgaaca aacaggtact 240

cgtgtccgct tctggccaag catggatact tttcgtaaca acactgaatt tcagcatgac 300

attctggcta aacgcctgcg tgaactctcc ttcctgaatt ccggcgtatc catccgtctt 360

attgataagc gcacgaatac tgaagatcac ttccattatg aaggtggaat taaagcg 417

<210> 121
<211> 417
<212> DNA
<213> Photorhabdus luminescens sbsp. akhurstii

<400> 121
gaaggtgttt cagcagccga agttattatg accgtgctgc atgcgggggg taaattcgat 60

gataactctt ataaagtctc cggcggtttg cacggcgtag gggtttctgt tgttaacgcc 120

ttgtctgaaa agctggaatt ggttatccgc cgtgacggca aagttcatga acagacttat 180

caccttggcg taccacaaag cccactgaaa gtcgttggag aaacagaaca gacaggtacc 240

cgtgtccgtt tctggccgag catggatact ttccgtaata acacagaatt tcagcatgac 300

attctggcta aacgcttgcg tgaattatcc ttcctaaatt ccggtgtatc catccgcctt 360

attgataaac gcactaatac ggaagaccac ttccattatg aaggtggcat taaagcg 417

<210> 122
<211> 417
<212> DNA
<213> Photorhabdus luminescens sbsp. kayaii

<400> 122
gaaggtgttt cagcagctga agttattatg acggtgctgc atgcgggggg taaattcgat 60

gataactctt ataaggtctc cggcggttta catggcgtag gggtttctgt tgttaacgcc 120

ttgtctgaaa agctggagtt ggttatccgc cgtgacggta aagttcatga acagacttat 180

caccttggtg tgccacaaag cccactgaaa gtcgttggag aaactgaaca aacaggtacc 240

cgtgtccgct tctggccgag catggatact ttcagtaata acacagaatt ccagcacgac 300

attctggcta aacgcctgcg tgaactatcc ttcctgaatt ccggtgtatc catccgtctt 360

attgataagc acactaatat agaagaccac ttccattatg aaggtggaat taaagcg 417

<210> 123
<211> 417
<212> DNA
<213> Photorhabdus luminescens sbsp. thracensis

<400> 123
gaaggtgttt cagcagctga agttattatg actgtgctgc atgcgggggg taaattcgac 60

gataactctt ataaagtttc cggcggtctg cacggtgtag gggtttctgt tgttaacgcc 120

ttgtctgaaa agctggaact gattatccgc cgtgacggca aagttcatga acagacttat 180

caccttggtg tgccgcaaaa cccactaaaa gttgtcggtg aaaccgaaca gacaggtacc 240

cgtgtccgct tctggccaag catggatact ttccgtgaca acactgaatt ccagcacgac 300

attctggcta aacgtctgcg tgaattgtcc tttctgaact caggtgtatc tatccgcctg 360

cttgataaac gcaccaatat tgaagaccat ttccattatg aaggtggtat taaggcg 417

<210> 124
<211> 414
<212> DNA
<213> Plesiomonas shigelloides T

<400> 124
gaaggggtgt cagcggccga agtgatcatg accgtactgc acgccggtgg taagtttgac 60

gataactcct ataaagtgtc cggtggtctg cacggggtag gggtatcggt ggttaacgcc 120

ctgtcggata agttggagct gatgatccgc cgtgaaggca aagtgcacac ccaagaatac 180

catctgggtg agccggtagc gccgctggcg gtggtcggtg ataccaatgc caccggtact 240

caagtgcgtt tctggcccag cccaaccatc ttcaccaata tcgaattcca gtacgagatc 300

ctggctaagc gtctgcgtga gctgtcgttc ttgaactctg gcgtgtcgat tcatctgctc 360

gacaagcgta acgacaagca ggatcacttt aagtacgaag gtggtattca agcg 414

<210> 125
<211> 417
<212> DNA
<213> Pragia fontium T

<400> 125
gaaggggtat ccgcagcaga agttatcatg acggtgttac acgccggtgg taagttcgat 60

gataactcgt ataaagtatc cggcggcctg cacggtgtag gtgtgtcggt ggtaaacgcc 120

ctgtcagaca aacttgagct gattatcgat cgcgatggca aaaatcataa acaaatctat 180

cgcaatggcg tacctgaagc tccgctaaaa atcatgggtg ataccgagag aaccggtacc 240

cgcattcgtt tttggccaag tccagatacc tttacctcta atctggagtt ccagtatgag 300

attctggcga aacgcttgcg cgaactgtca ttcctgaact ctggcgtatc gatcaaatta 360

agtgatgaac gcgatggcaa gaatgaccat ttccattatg aaggtggtat taaagcc 417

<210> 126
<211> 417
<212> DNA
<213> Proteus hauseri

<400> 126
gagggtgttt ctgctgcaga agttattatg acggttctgc atgcgggcgg taagttcgat 60

gataactcct ataaagtttc tggtggactt catggtgtag gtgtttcggt tgtcaatgcg 120

ttatctgaaa aactggaact gactattcat cgtgatggaa agatccatca acaaatttat 180

cgtcatggtg ttcctgaaga tcgcttaaaa gtaattggcg atactgaaaa atcaggtaca 240

tttgttcgtt tttggccaag cctagaaacc tttcaaggtg agactgaatt ccaatatgaa 300

atcttagcaa aacgcctacg tgagctttct ttcttaaact ctggtgttgc tattcgttta 360

attgataaac gtgataaccg cgaagaccat ttccattacg aaggtggtat taaggcg 417

<210>  127
<211>  417
<212>  DNA
<213>  Proteus mirabilis

<400>  127
gagggcgttt ctgcagcaga agtgattatg acggttctgc atgcgggtgg taaattcgat      60

gataactcct ataaagtctc tggtgggctg catggtgtag gggtttccgt tgttaatgcg      120

ttgtctgaaa aacttgaact gactatccat cgtgatggta aaattcatca acaaatttac      180

cgttcaggtg tacctgacga tcgcttaaaa gtgattggtg aaacagataa gtcaggaacc      240

tttgttcgtt tctggccaag tcttgatacc tttaaaggtg agactgaatt ccaatatgat      300

attttagcga aacgtctgcg tgagctctcc ttcttaaact caggagtctc tattcgttta      360

attgataaac gtgataaccg cgaagatcat ttccactatg aaggtggtat caaagca      417

<210>  128
<211>  417
<212>  DNA
<213>  Proteus myxofaciens

<400>  128
gaaggcgttt ctgcagcaga agtgatcatg acagttctgc atgcaggcgg taagtttgat      60

gataactcct ataaagtctc tggcggactt catggtgttg gggtttctgt cgttaatgcg      120

ttgtctgaaa aactagaatt aactattcac cgtgataata aaattcatca acaaatttat      180

cgtcatggtg ttcctgatga tcgtctaaaa gtcattggcg atactgaaaa atcaggtaca      240

tatgttcgtt tttggccaag cctagaaacg tttaaaggtg aaactgaatt tcaatacgaa      300

attctagcca aacgtcttag agagctttcg ttcttaaact caggcgtatc aatccgttta      360

attgataaac gtgataaccg tgaagaccat ttccattatg aaggtggtat taaagcc      417

<210>  129
<211>  417
<212>  DNA
<213>  Proteus penneri

<400>  129
gagggtgttt cagcagcaga agttattatg acggttctgc atgcgggggg gaaattcgat      60

gataactcct ataaagtctc tggtggactt catggtgtag gggtttctgt tgttaatgcg      120

ttgtctgaaa aactcgaact gactattcat cgcgatggaa aaatccatca gcaaatttat      180

cgtcatggtg tacctgatga tcgcttaaaa gtgattggtg ataccgaaaa gtcaggcaca      240

tttgttcgct tctggccaag tcttgaaacc tttaaaggcg agactgaatt ccagtatgac      300

attttagcaa aacgtctgcg tgagttgtca ttcttaaact caggcgtttc tatccgttta      360

attgataaac gtgataaccg tgaagatcat ttccattatg aaggcggtat taaggca      417

<210>  130
<211>  417
<212>  DNA
<213>  Proteus vulgaris T

<400>  130
gagggtgttt cagcagcaga agtcattatg acggttctgc atgccggggg taaattcgat      60

gataactcct ataaagtctc tggtggactt catggtgtag gggtttctgt tgttaatgcg      120

ttgtctgaaa aacttgaact gacgattcat cgcgatggaa aaatccatca gcaaatttat      180

cgtcatggtg tacctgatga tcgcttaaaa gtgattggtg atactgaaaa atctggcaca      240

tttgttcgct tctggccaag tcttgaaacc tttaaaggtg agaccgaatt ccagtatgac      300

attttagcaa aacgtcttcg tgagttgtca ttcttaaact ccggtgtttc tatccgttta      360

atcgataaac gcgataatcg tgaagatcat ttccactata aaggcggtat taaggca      417

<210>  131
<211>  417
<212>  DNA
<213>  Providencia alcalifaciens T

<400>  131
gaaggtgttt ctgccgcaga agtcatcatg accgttctgc acgccggcgg taaatttgac      60

gacaactcct acaaagtctc cggtggtctg cacggtgtgg gtgtttcagt ggttaacgcc      120

ttatccgaaa aacttgaatt agtaatccgc cgtgacggta aagttcacga acaaatatac      180

aaacacggcg agccacaagg tcctctaaca gttgttggag aaacagacca aacagggact      240

cgtgtacgtt tctggccaag catggacacc ttcaaaggtg tgactgaatt cgaatacgat      300

attctggcaa aacgcttacg tgaactttca ttcctgaact caggcgtttc aatccgttta      360

attgataaac gagatggcaa agaagaccat ttccactatg aaggtgggat caaagcc      417

<210>  132
<211>  417
<212>  DNA
<213>  Providencia heimbachae

<400>  132
gaaggcgtgt ccgcagcaga agttattatg actgttctgc atgctggcgg taaatttgac      60

gacaacgctt ataaagtctc cggcggtttg catggagttg gtgtttctgt tgttaacgcc      120

ttatctgaaa aactcgaatt aatcattcgt cgtgacggta aagttcacga gcaaatctac      180

aaacatggtg agccacaagg cccactaacg gttgtcggtg aaactgatat aaccggaact      240

cgcgttcgct tttggccgag tatggatacc tttaaaggga tcactgagtt cgaatatgac      300

attttagcaa aacgcttacg tgagctgtca ttcctaaact caggtgtttc tatcaaactc      360

gttgataaac gtgatggaaa agaagatcac ttccactacg aaggcggaat taaagcc      417

<210>  133
<211>  417
<212>  DNA
<213>  Providencia rettgeri

<400>  133
gaaggtgtat cagccgcaga ggttatcatg accgttctgc acgcaggcgg aaaatttgat      60

gacaactcct ataaagtctc cggtggttta cacggtgttg gggtttccgt tgttaacgcc      120

ttatctgaaa aattagaatt agttatccgc cgtgatggca aagttcatga gcaaatctac 180

aaacacggtg agcctcaagg gcctctgact gttgtcggtg aaactgacca aacaggaaca 240

cgcgttcgtt tttggccaag catggacacc tttaaaggtg tgactgaatt tgaatatgat 300

gtcttagcaa aacgcttacg tgagttatcg ttcctaaact caggtgtttc tattaagctc 360

gtcgataaac gcgatggcaa agaagaccat ttccattatg aaggcggtat caaagcc 417

<210> 134
<211> 417
<212> DNA
<213> Providencia rustigianii

<400> 134
gaaggtgtct ccgcggcaga agttatcatg acagttctgc acgctggcgg taaatttgac 60

gacaactcct acaaagtttc cggcggtttg cacggtgtag gtgtttctgt ggttaacgcc 120

ttatctgaaa aacttgaatt agtgatccgc cgtgatggca aagttcacga gcaaatctac 180

aaacacggcg agccgcaagg tcctctgact gtcgttggcg aaacagacca aaccggaact 240

cgcgtccgtt tctggccaag catggatacc ttcaaaggta tcactgaatt cgaatacgaa 300

attctagcga aacgcctacg tgaactttca ttcctgaact ctggtgtttc tatccgctta 360

attgataagc gtgatggcaa agaagatcac ttccattatg acggtggtat caaggca 417

<210> 135
<211> 417
<212> DNA
<213> Providencia stuartii

<400> 135
gaaggggtat cagcagcaga agttatcatg acggtactgc atgcgggcgg gaaatttgac 60

gataactcct ataaagtctc cggcggtcta cacggagttg gggtttctgt tgttaacgcc 120

ttatccgaaa aattagagct cactatccgc cgtgatggta aagttcatga acaaacttat 180

tgccacggtg agccacaagg cccactaact gttgttggtg aaaccgatca aaccggaact 240

cgcgtccgtt tttggccaag cttagacacc ttcaaaggcg tcactgaatt tgaatacgac 300

gttttagcta agcgtctgcg cgaactgtca ttcttaaact ctggtgtctc aatcagactg 360

attgataaac gtgatggcaa agaagaccat ttccactatg aaggtgggat taaagcg 417

<210> 136
<211> 417
<212> DNA
<213> Providencia vermicola

<400> 136
gaaggtgtct ctgccgcgga agtcatcatg actgttctgc acgcaggggg gaaattcgat 60

gataactcct ataaagtttc cggcggattg cacggtgtag gggtatcagt tgttaacgcc 120

ttatctgaaa aactggaatt agtcatcaaa cgtgatggca aagttcacga acaaatctac 180

aaacacggtg aaccacaagg ccctctgtcc gttgttggtg aaactgacca aacagggact 240

cgcgtccgct tttggccaag tatggatacc tttaaaggcg tcactgagtt cgagtatgac 300

gtattagcga agcgtttacg tgagttatca ttcttaaact cgggcgtttc aatcaaactc 360

atcgataagc gagatggaaa agaagaccat ttccattatg aaggtggtat caaagcg 417

<210> 137
<211> 417
<212> DNA
<213> Rahnella aquatilis T

<400> 137
gagggcgttt ctgctgctca ggtcatcatg accgttcttc atgctggcgg taaattcgac 60

gataactcgt ataaagtctc cggcggtctg catggcgtgg gtgtttccgt cgttaacgcc 120

ttgtcggaaa aactggaact ggttatccgc cgtgaaggca aagtgcacac ccagacttac 180

gtgcacggcg aaccgcagga tccgctgaaa gtgattggcg atactgacgc aaccggcacc 240

acggtacgtt tttggccgag cttcaacacc ttcaccaatc acactgaatt cgaatatgac 300

attctggcga aacgcctgcg tgaactgtca ttcctgaact ccggcgtggc aatccgtctg 360

ctggataaac gtgatggtaa gaatgatcac ttccattatg aaggcggtat caaagcc 417

<210> 138
<211> 417
<212> DNA
<213> Raoultella ornithinolytica

<400> 138
gagggggtgt cggcagctga agtcatcatg accgtactgc acgcaggcgg taaatttgat 60

gataactcct ataaagtctc cggtggtctg cacggcgtgg gcgtctctgt cgttaacgcc 120

ctgtcacaga aactggagct ggtgattcag cgcgataaca agattcaccg tcagatctat 180

gcccacggcg taccgcaggc accgctggca gtcaccggtg ataccgaaaa aaccggtacc 240

atggtgcgtt tctggccgag ctatgaaacc ttcactaacg tcatcgaatt cgaatacgaa 300

atcctggcaa aacgcctgcg tgagctgtca ttcctgaact caggggtgtc tatccgcctg 360

cgcgacaagc gtgatggcaa agaagaccat ttccactacg aaggcggcat caaggcg 417

<210> 139
<211> 417
<212> DNA
<213> Raoultella planticola T

<400> 139
gagggtgtgt cggcagctga agtcatcatg accgtactgc acgcaggcgg taaatttgat 60

gataactcct ataaagtctc cggtggtctg cacggcgtgg gcgtctctgt agttaacgcc 120

ctgtcacaga aactggagct ggtgattcag cgcgacaaca agattcaccg ccagatctac 180

gcccacggcg taccgcaggc accgctggcc gtaaccggtg ataccgaaaa aaccggtacc 240

atggtgcgtt tctggccgag ctatgaaacc ttcaccaacg tcatcgaatt cgaatacgaa 300

atcctggcaa aacgcctgcg tgagctgtca ttcctgaact caggggtgtc tatccgcctg 360

cgcgacaagc gtgacggcaa agaagaccat ttccactacg aaggcggcat caaggcg 417

<210> 140
<211> 417
<212> DNA
<213> Raoultella terrigena

<400> 140
gagggcgtgt ccgctgccga agtcatcatg accgtcctgc acgcgggcgg taagttcgat 60

gataactcct ataaagtctc cggcggtctg cacggcgtag gcgtttctgt agttaacgcc 120

ctgtcgcaga agctggaact gttgatccgc cgtgaaggca aagtccacca gcaaacctac 180

gtgcacggcg tgccgcaggc accgctggcg gttaccggcg acactgatgc taccggtact 240

caggtgcgtt tctggccgag ccacgaaacc ttcaccaacg tgatcgaatt cgaatatgag 300

attctggcca aacgcctgcg cgagctgtcg ttcctgaact ccggcgtctc cattcgcctg 360

cgcgataagc gtgacggtaa agaagaccac ttccactacg aaggcggcat caaggcg 417

<210> 141
<211> 417
<212> DNA
<213> Salmonella bongori

<400> 141
gagggcgtat cggcggcaga agtcatcatg acggttctgc atgcgggcgg taaatttgac 60

gataactcct ataaagtgtc cggaggtctg cacggcgtag gtgtctcggt agtcaacgcc 120

ctgtcgcaaa aactggaact ggttatccag cgggatggca aaattcatcg tcagatctac 180

gagcacggtg ttccgcaagc accgcttgct gtgactggcg atactgataa aaccgggacg 240

atggtgcgtt tctggccgag cctggaaact ttcacaaatg tcaccgagtt tgaatacgag 300

atcctggcaa aacgcctgcg ggaactgtcg ttcctgaatt ccggcgtctc tatccgcctg 360

cgcgacaagc gcgacggtaa agaagaccat ttccattatg aaggcggcat caaggcg 417

<210> 142
<211> 417
<212> DNA
<213> Salmonella enterica sbsp. arizonae

<400> 142
gaaggcgtct cggcggcgga agtgatcatg accgttctgc acgcgggcgg taaatttgac 60

gataactcct ataaagtttc cggtggtctg cacggcgtgg gcgtttcggt cgtcaatgcc 120

ctgtcgcaga agctggaact ggttatccag cgcgatggca aaatccaccg tcagctctac 180

gaacacggcg taccgcaggc accgctggcc atcactggcg ataccgataa aactggcacg 240

atggtacgtt tctggccgag ccacgaaacc ttcaccaacg tcactgaatt tgaatatgag 300

atcctggcga aacgcctgcg tgaactgtca ttcctgaact ctggcgtctc catccgcctg 360

cgcgacaagc gcgacggcaa agaagatcat ttccactacg aaggcggcat caaggcg 417

<210> 143
<211> 417
<212> DNA
<213> Salmonella enterica sbsp. diarizonae

<400>  143
gaaggtgtct cagcggcgga agtgatcatg accgttctgc acgcgggcgg taaatttgac      60

gataactcct ataaagtctc cggcggtctg cacggcgtgg gtgtctcggt agtcaacgcc     120

ctgtcgcaaa aactggaact ggttatccag cgagatggca aaactcaccg tcagatctac     180

gagcacggcg tgccgcaggc accgctggcc gtcactggcg ataccgataa aaccggtacg     240

atggtccgtt tctggccgag ccacgaaacg ttcaccaatg tcactgagtt tgaatatgag     300

attctggcga aacgcctgcg tgaactgtca ttcctgaact ctggcgtctc catccgcctg     360

cgcgacaagc gcgacggcaa agaagatcat ttccactacg aaggcggcat caaggcg        417

<210>  144
<211>  417
<212>  DNA
<213>  Salmonella enterica sbsp. enterica T

<400>  144
gaaggcgtct cggcggcgga agtgatcatg accgttctgc acgcgggcgg taaatttgac      60

gataactcct ataaagtctc cggcggtctg cacggcgtgg gcgtctcggt agtcaacgct     120

ctgtcgcaaa aactggaact ggttatccag cgagatggca aaattcaccg tcagatctac     180

gagcacggcg tgccgcaggc acccctggcc gtcactggcg ataccgataa aaccggcacg     240

atggtacgtt tctggccgag ccacgaaacc ttcaccaacg tcactgaatt tgaatatgag     300

atcctggcga aacgcctgcg tgaactgtca ttcctgaact caggcgtctc catccgcctg     360

cgcgacaagc gcgatggcaa agaagatcat ttccactacg aaggcggcat caaggcg        417

<210>  145
<211>  417
<212>  DNA
<213>  Salmonella enterica sbsp. houtenae

<400>  145
gaaggtgtct cggcggcgga agtgatcatg accgttctgc acgcgggcgg taaatttgac      60

gataactcct ataaagtctc cggcggtctg cacggcgtag gcgtctcggt agtcaacgcc     120

ctgtcgcaaa aactggagct ggttatccag cgagatggca aaattcaccg tcagatctac     180

gagcacggcg tgccgcaggc acccttggcc gttactggcg ataccgataa aaccggcacg     240

atggtccgtt tctggccgag ccacgaaacc ttcaccaacg tcactgaatt tgaatatgag     300

atcctggcga aacgcctgcg tgaactgtca ttcctgaact ctggcgtctc tatccgcctg     360

cgcgacaagc gcgacggcaa agaggatcat ttccactacg aaggcggcat caaggcg        417

<210>  146
<211>  417
<212>  DNA
<213>  Salmonella enterica sbsp. indica

<400>  146
gaaggcgttt cggcggcgga agtgatcatg accgttctgc acgcgggcgg taaatttgac      60

gataactcct ataaagtctc cggcggtctg cacggcgtgg gcgtctcggt agtcaacgcc     120

ctgtcgcaaa aactggaact ggttatccag cgagatggca aaattcaccg tcagatctac 180

gagcatggcg tgccgcaggc acctctggcc gtcactggcg ataccgataa aaccggcacg 240

atggtacgtt tctggccgag ccacgaaacc ttcaccaacg tcactgaatt cgaatatgag 300

atcctggcga agcgcctgcg tgaactgtca ttcctgaact ctggcgtctc catccgcctg 360

cgcgacaagc gcgacggcaa agaagatcat ttccactacg aaggcggcat caaggcg 417

<210> 147
<211> 417
<212> DNA
<213> Salmonella enterica sbsp. salamae

<400> 147
gaaggcgtct cggcggcgga agtgatcatg accgttctgc acgcgggcgg taaatttgac 60

gataactcct ataaagtctc cggcggcctg cacggcgtgg gcgtctcggt agtcaacgcc 120

ctgtcgcaaa aactggaact ggttatccag cgagatggca aaattcaccg tcagatctac 180

gagcacggcg tgccgcaggc acccctggcc gttactggcg atactgataa aaccggcacg 240

atggtacgtt tctggccgag ccacgaaacc ttcaccaacg tcactgaatt tgaatatgag 300

attctggcga aacgcctgcg tgaactgtca ttcctgaact ctggcgtctc tatccgcctg 360

cgcgacaagc gcgacggcaa agaagaccat ttccactacg aaggcggcat caaggcg 417

<210> 148
<211> 420
<212> DNA
<213> Samsonia erythrinae T

<400> 148
gaagggattt ccgcggcgga ggtcatcatg accgtgctgc atgcgggggg caagttcgac 60

gataactcct ataaagtctc cggcggcctg cacggcgtag gggtttccgt ggttaacgcc 120

ctgtcggaaa aactgaagct ggttatccac cgtgacggga aagttcatga gcagatctac 180

agccacggtg tgtcacaggc gccgctggcg gtgacgggcg agaccaacag aaccggcacc 240

acggtgcgtt tctggccaag tctggaaacc ttcaccaacg tggtcgagtt tgagtacgac 300

attctggcta agcgccttcg cgagttgtcc ttccttaact ccggcgtgtc tattcgtttg 360

atcgacgagc gtgtgaaaga taaagccgat cacttccatt atgaaggcgg tatcaaggcg 420

<210> 149
<211> 417
<212> DNA
<213> Serratia entomophila

<400> 149
gaaggggttt ccgccgcaga ggtcatcatg accgtgctgc acgccggcgg taaattcgac 60

gacaactcct acaaggtatc cggcggcctg cacggcgtgg gcgtctcggt ggttaacgcc 120

ctgtcggaga agctggagct ggttatccgc cgcgaaggca aggtgcatga gcagacctac 180

ggccacggcg tgccgcaggc gccgctgaaa gtggttggcg aaaccgagca gaccggcacc 240

atggtgcgtt tctggccgag ccatcagacc ttcaccaacg tcaccgattt cgaatacgac 300

attctggcca agcgcctgcg cgagctgtcc ttcctgaact ccggcgtgtc tatccgcctg 360

aaggacaagc gttccgacag agaagaccac ttccactacg aaggcggcat caaggcg 417

<210> 150
<211> 417
<212> DNA
<213> Serratia ficaria

<400> 150
gaaggggttt ccgccgcaga ggtcatcatg accgtgctgc acgccggcgg taaatttgac 60

gacaactcct acaaggtgtc cggcggcctg cacggcgtgg gcgtctcggt ggttaacgcc 120

ctgtcggaga agctggagct ggtgatccgc cgcgaaggca aggtgcatga gcagacctac 180

ggccacggcg agccgcaggc gccgttgaaa gtggtgggtg aaaccgatca gaccggcacc 240

atggtgcgtt tctggccgag ccaccagacc ttcaccaacg tcaccgactt tgaatacgac 300

attctggcca agcgcctgcg cgagctgtcc ttcctcaact ccggcgtgtc tatccgcctg 360

aaggacaagc gttccgacag agaagaccac ttccactacg aaggcggcat caaggcg 417

<210> 151
<211> 417
<212> DNA
<213> Serratia fonticola

<400> 151
gaaggggtgt ctgcggcaca ggtcatcatg accgtactgc acgctggcgg taagtttgac 60

gacaactcct ataaggtttc cggcggtctg cacggcgtag gcgtttcggt tgtcaacgcc 120

ctgtcagaaa agctggagct ggtgatccgc cgcgaaggca aggtccatga gcaaacctac 180

aagcatggcg aaccacaggc cccgctgaaa gtcgtcggtg aaaccgatca gaccggtacc 240

accctgcgct tctggccgag cttccagacc tttaccaata acatcgagtt tgagtacgac 300

atcctggcca agcgcctgcg tgaactgtcg ttcctcaact cgggcgtttc tattcgcctg 360

aaagacaagc gcaacgacag agaagaccac ttccattacg aaggcggtat ccgtgcg 417

<210> 152
<211> 417
<212> DNA
<213> Serratia grimesii

<400> 152
gaaggggttt ctgcggcaca ggtgatcatg actgtgctgc atgccggcgg caaatttgat 60

gacaactcct acaaggtttc cggcggtctg cacggtgtag gcgtatcggt tgttaacgcc 120

ttgtccgaga agctggaact ggttatccgt cgtgaaggca aggtacacga gcaaacctat 180

gcgatgggcg agccactggc accgctgaaa gtggtcggtg aaaccgatct gaccggtacc 240

acagtgcgtt tctggccgag ctacgaaacc ttcaccaata atactgaatt tgagtacgac 300

attctggcca agcgcctgcg cgaactctca ttcctgaact ctggcgtgtc tatccgcctg 360

aaagacaagc gcaccgacaa agaagaccac ttccactatg aaggcggtat caaggcg 417

<210> 153
<211> 417
<212> DNA
<213> Serratia liquefaciens

<400> 153
gaaggggttt ccgcagcaca ggtgatcatg accgtgctgc atgctggcgg caaatttgat 60

gacaactcct acaaggtctc cggcggtctg cacggcgtgg gcgtctccgt tgttaacgcc 120

ctgtctgaga agctggaact ggtgatccgt cgtgaaggca aggtgcatga gcaaacctat 180

gccatgggcg agccattgtc gccgctgaaa gtggtcggtg aaaccgatca gaccggcacc 240

acggtccgtt tctggccgag ctatgaaacc ttcaccaaca ataccgaatt tgagtacgac 300

attctggcca agcgcctgcg cgaactctct ttcctgaact ccggcgtgtc tatccgcctg 360

aaagacaaac gtaccgacaa agaagaccac ttccactacg aaggcggtat caaggcg 417

<210> 154
<211> 417
<212> DNA
<213> Serratia marcescens sbsp. marcescens T

<400> 154
gaaggggttt cagccgcaga ggtcatcatg accgtgctgc acgccggcgg taaattcgac 60

gacaactcct ataaagtttc cggcggcctg cacggcgtgg gcgtctcggt ggttaacgcc 120

ctgtcggaga aactggagct ggtgatccgc cgcgaaggca aagtgcacga acaaacttac 180

agccacggcg agccgcaggc gccgctgacc gtggtgggcg aaaccgagca gaccggcact 240

atggtgcgct tctggccaag ccaccagacc tttaccaatg tgaccgattt cgagtacgac 300

attctggcca agcgcctgcg cgagctgtcc ttcctcaact ccggcgtgtc tatccgcctg 360

aaggacaagc gcactgaccg tgaagaccac ttccattacg aaggcggcat caaggcg 417

<210> 155
<211> 417
<212> DNA
<213> Serratia odorifera

<400> 155
gaaggcgttt ctgccgcaca ggtcatcatg accgtgctgc acgccggcgg caagttcgac 60

gataactcct acaaggtgtc cggcggcctg cacggcgtgg gcgtctcggt ggttaacgcc 120

ctgtccgaga agctggagct ggtgatccgc cgcgaaggca aggttcacga gcaaacctac 180

agccacggcg agccgcaaac gccgctgaaa gtggtcggcg aaaccgagca gaccggcacc 240

acggtgcgtt tctggccgag ctaccagacc tttaccaacc agaccgaatt ccagtacgac 300

attctggcga aacgcctgcg tgaactgtcg ttcctcaact ccggcgtatc tatccgcctg 360

aaagacaagc gcgatgacaa agaagaccac ttccactacg aaggcggtat caaggcg 417

<210> 156
<211> 417
<212> DNA

<213>  Serratia plymuthica

<400>  156
gaaggggttt ctgcggcgca ggtcatcatg accgtgctgc atgccggcgg caaatttgac      60

gacaactcct acaaggtatc cggcggcctg cacggcgtgg gtgtctccgt tgttaacgcc     120

ctgtccgaga agctggaact ggttattcgc cgcgaaggca aggtgcatga gcagacctac     180

gccatgggtg agccgcaggc gccgctgaaa gtggtcggtg aaaccgatca gaccggcacc     240

acggtgcgtt tctggccgag ctacgaaacc ttcaccaaca acgttgaatt tgagtacgac     300

attctggcca agcgcctgcg cgagctgtct ttccttaact ccggcgtgtc tatccgcctg     360

aaagacaagc gcacggacaa agaagaccac ttccactacg aaggcggcat caaggcg        417

<210>  157
<211>  417
<212>  DNA
<213>  Serratia proteamaculans sbsp. proteamaculans

<400>  157
gaaggggttt ctgcggcaca ggtgatcatg actgtgctgc atgccggcgg caaatttgat      60

gacaactcct acaaggtttc tggcggtctg cacggcgtgg gtgtttcggt tgttaacgcc     120

ctgtctgaaa aactggaact ggtgatccgt cgtgaaggca aggtgcacga gcaaacctac     180

gccatgggcg aaccacaggc gccgctgaaa gtggtcggtg aaaccgatca gaccggcacc     240

acggtgcgtt tctggccgag ctatgaaacc ttcaccaata atgtcgaatt tgagtacgat     300

attctggcca agcgcttgcg cgaactctca ttcctgaact ctggtgtgtc tatccgcctg     360

aaagacaaac gcacggacaa agaagaccac ttccactatg aaggcggtat caaggcg        417

<210>  158
<211>  417
<212>  DNA
<213>  Serratia rubidaea

<400>  158
gaaggcgttt ccgccgcgca ggtcatcatg accgtactgc acgccggcgg taagttcgac      60

gataactctt acaaggtttc cggcggcctg cacggcgtgg gcgtctcggt ggttaacgca     120

ctgtccgaga agctggaact ggtgatccgc cgcgacggcc aggtgcacga gcaaacctac     180

agccacggcg agccgcaggc gccgctgacc gtggtcggcg agaccgaaca gaccggcacc     240

acggtgcgtt tctggccgag ccatcagacc ttcaccaacg tggtggagtt cgaatacgac     300

atcctggcga aacgcctgcg cgaactgtcg ttcctcaact ccggcgtgtc catccgtctg     360

aaagacaagc gcagcgacaa agaggaccac ttccactacg aaggcggcat caaggcg        417

<210>  159
<211>  417
<212>  DNA
<213>  Serratia ureilytica

<400>  159
gaaggggttt cagccgcaga ggtcatcatg accgtgctgc acgccggcgg taaattcgac      60

gacaactcct ataaagtttc cggcggcctg cacggcgtgg gcgtctcggt ggttaacgcc     120

ctgtcggaga aactggagct ggtgatccgc cgcgaaggca aagtgcacga acaaacttac     180

agccacggcg agccgcaggc gccgctgaac gtggtgggcg aaaccgagca gaccggcacc     240

atggtgcgct tctggccgag tcaccagacc ttcaccaatg tgaccgaatt cgagtacgac     300

attctggcca agcgtctgcg cgagctgtcc ttcctcaact ccggcgtgtc tattcgcctg     360

aaggacaagc gcaccgaccg tgaagaccac ttccattacg aaggcggcat caaggcg        417

<210>  160
<211>  417
<212>  DNA
<213>  Shigella boydii

<400>  160
gagggcgtat cggcggcgga agtgatcatg accgttctgc acgcaggcgg taaatttgac      60

gataactcct ataaagtgtc cggcggtctg cacggcgttg gtgtttcggt agtaaacgcc     120

ctgtcgcaaa aactggagct ggttatccag cgcgagggta aaattcaccg tcagatctac     180

gaacacggtg taccgcaggc tccgctggcg gttaccggcg agactgaaaa aaccggcacc     240

atggtgcgtt tctggcccag cctcgaaacc ttcaccaatg tgaccgagtt cgaatatgaa     300

attctggcga aacgtctgcg tgagttgtcg ttcctcaact ccggcgtttc cattcgtctg     360

cgcgacaagc gtgacggcaa agaagaccac ttccactatg aaggcggcat caaggcg        417

<210>  161
<211>  417
<212>  DNA
<213>  Shigella dysenteriae T

<400>  161
gagggcgtat cggcggcgga agtgatcatg accgttctgc acgcaggcgg taaatttgac      60

gataactcct ataaagtgtc cggcggtctg cacggcgttg gtgtttcggt agtaaacgcc     120

ctgtcgcaaa aactggagct ggttatccag cgcgagggta aaattcaccg tcagatctac     180

gaacacggtg taccgcaggc tccgctggcg gttaccggcg agactgaaaa aactggcacc     240

atggtgcgtt tctggcccag cctcgaaacc ttcaccaatg tgaccgagtt cgaatatgaa     300

attctggcga aacgtctgcg tgagttgtcg ttcctcaact ccggcgtttc cattcgtctg     360

cgcgacaagc gtgacggcaa agaagaccac ttccactatg aaggcggcat caaggcg        417

<210>  162
<211>  417
<212>  DNA
<213>  Shigella flexneri

<400>  162
gagggcgtat cggcggcgga agtgatcatg accgttctgc acgcaggcgg taaatttgac      60

gataactcct ataaagtgtc cggcggtctg cacggcgttg gtgtttcggt agtaaacgcc     120

ctgtcgcaaa aactggagct ggttattcag cgcgagggta aaattcaccg tcagatctac     180

gaacacggtg taccgcaggc tccgctggcg gttaccggcg agactgaaaa aaccggcacc     240

atggtgcgtt tctggcccag cctcgaaacc ttcaccaatg tgaccgagtt cgaatatgaa 300

attctggcga aacgtctgcg tgagttgtcg ttcctcaact ccggcgtttc cattcgtctg 360

cgcgacaagc gtgacggcaa agaagaccac ttccactatg aaggcggcat caaagcg 417

<210> 163
<211> 417
<212> DNA
<213> Shigella sonnei

<400> 163
gagggcgtat cggcggcgga agtgatcatg accgttctgc acgcaggcgg taaatttgac 60

gataactcct ataaagtgtc cggcggtctg cacggcgttg gtgtttcggt agtaaacgcc 120

ctgtcgcaaa aactggagct ggttatccag cgcgagggta aaattcaccg tcagatctac 180

gaacacggtg taccgcaggc accgttggcg gttaccggcg agactgaaaa aaccggcacc 240

atggtgcgtt tctggcccag cctcgaaacc ttcaccaatg tgaccgagtt cgaatatgaa 300

attctggcga aacgtctgcg tgagttgtcg ttcctcaact ccggcgtttc cattcgtctg 360

cgcgacaagc gcgacggcaa agaagaccac ttccactatg aaggcggcat caaggcg 417

<210> 164
<211> 417
<212> DNA
<213> Tatumella ptyseos T

<400> 164
gagggcgtat cggcggcaga agtgattatg actgtgctgc atgccggggg taagtttgac 60

gataactctt ataaagtgtc cggtggttta catggcgtgg gtgtctctgt cgtcaacgca 120

ttgtctgaaa agctggaact gactatccgt cgtgacggta aagttcatca gcaaatttat 180

cagcacggtg taccgacagc accgctggcg gtgatcggcg aaacagatgt gagcgggact 240

gcggtacgtt tctgggccag tcatcagaca tttacgaatg tgaccgattt cgaatacgac 300

attctggcaa aacgcctgcg cgaactctct ttcctgaatt ccggcgtgtc tatccgcctg 360

atcgacaagc gtacagataa gagtgatcac taccactacg aaggtggtat ccgcgcg 417

<210> 165
<211> 417
<212> DNA
<213> Trabulsiella guamensis T

<400> 165
gagggcgtat ccgccgcgga agtgatcatg accgtcctgc atgcgggcgg taaattcgat 60

gacaactcct ataaagtgtc tggcggtctg catggcgtcg gcgtctccgt ggttaacgcg 120

ctgtcgcaga agctggaact gataattcac cgcgaaggca agatccatcg tcagatttac 180

gaacacggcg taccgcaggc accgctggcg gttacgggtg ataccgaaaa gaccggtacc 240

atggtgcgtt tctggccgag cctggaaacc ttcaccaacg tcaccgaatt cgaatatgaa 300

attctggcga aacgtctgcg tgagctgtcg ttcctgaact ctggcgtctc tatccgcctg 360

aaagacaagc gtgacggcaa agaagaccat ttccactacg aaggtggcat caaggcg 417

<210> 166
<211> 417
<212> DNA
<213> Yersinia aldovae

<400> 166
gaaggggtat cagccgcaga ggtcatcatg accgtattgc acgccggcgg taaatttgac 60

gataactcgt acaaagtttc cggtggtttg cacggcgtgg gtgtatcggt tgttaacgcc 120

ctgtctgaaa agctggaact ggtgatccgc cgtgacggta aagttcacga acaaacctac 180

aaaatgggtg tgccgcaggc accactgaaa atggtaggtg aaactgagct gaccgggaca 240

accgtgcgtt tctggccgag tttccagacc tttaccaata acatcgaatt ccagtacgag 300

attctggcta aacgcctgcg cgagttgtcg ttcctgaact cgggtgtgtc aatcaaactg 360

aaagataacc gtaacgacaa agaagaccac ttccattatg aaggcggtat cagggcg 417

<210> 167
<211> 417
<212> DNA
<213> Yersinia bercovieri

<400> 167
gaaggggtat cggcagctga ggtcatcatg accgtgctac atgcgggcgg taagtttgat 60

gataactctt acaaagtttc cggcggtttg catggcgtgg gtgtgtcagt tgttaacgcc 120

ctatctgaga aactggagct ggtcattcgc cgtgaaggta aagttcacga gcagacttac 180

aaaatgggtg tgccgcaagc acctctgaaa gtggtgggtg aaactgatct aaccgggact 240

accgtgcgtt tttggccgag tttcgagacc ttcaccaata acaccgaatt ccagtatgaa 300

attttagcta aacgtctgcg tgagctgtca ttcctgaact caggtgtttc aatcaagctg 360

aaagacaagc gcaacgacag agaagaccac ttccactacg aaggcggtat caaggcg 417

<210> 168
<211> 417
<212> DNA
<213> Yersinia enterocolitica sbsp. palearctica

<400> 168
gaaggggtat cggcagcaga ggtcatcatg accgtgttgc atgctggcgg taagtttgac 60

gataactcgt acaaagtttc cggtggtttg cacggcgtag gtgtatccgt tgttaacgcc 120

ctgtctgaaa aactggagct ggtgattcgc cgtgaaggta aagttcacga gcagacttac 180

aagatgggtg tgccgcaggc accattgaaa gtggtgggcg aaaccgatca aaccgggaca 240

accgtgcgtt tctggccgag cttccagacg ttcaccaata ataccgaatt ccaatatgaa 300

atcctggcaa aacgtctgcg tgagctatcg ttcctgaact ctggggtttc aatcaagctg 360

aaagacaaac gtaatgacaa agaagaccac ttccattacg aaggcggtat caaagcg 417

<210> 169
<211> 417

<212>   DNA
<213>   Yersinia frederiksenii

<400>   169
gaaggggtat cggcagctga ggtcatcatg accgtgttgc atgctggcgg taaatttgac      60

gataactcgt acaaagtttc cggcggtttg cacggcgtgg gtgtgtcagt tgttaacgct     120

ctgtctgaaa aactggagct ggtgatccgc cgtgaaggta aagttcacga gcagacttac     180

agaatgggtg tgccgcaggc accattgaaa gtggtgggtg aaaccgatca aaccgggact     240

accgtgcgct tctggccaag cttccagacc ttcaccaata acatcgaatt ccaatatgaa     300

atcctggcta aacgcctgcg cgagctgtcg ttcctgaact ccggggtttc aatcaaattg     360

agagacaaac gtaacgacaa agaagaccac ttccattatg aaggcggtat caaagcg        417

<210>   170
<211>   417
<212>   DNA
<213>   Yersinia intermedia

<400>   170
gaaggggtat cagcagcaga ggtcatcatg accgtgttgc atgctggcgg taaatttgac      60

gataactctt acaaagtttc tggcggtttg cacggtgtgg gcgtatccgt tgttaacgcc     120

ctgtctgaaa agctggaact ggtcattcgc cgtgacggca aagtacacga gcaaacctac     180

aaaatgggcg tgcctcaggc accgctgaaa atggtgggtg aaaccgatct gaccgggact     240

accgtgcgtt tctggccaag tttccagacc ttcaccaata acatcgaatt ccagtatgaa     300

attctggcta agcgcctgcg cgagctgtcg ttcctgaact ccggtgtgtc aatcaaactg     360

aaagacaaac gtaacgacag agaagaccac ttccattatg aagggggtat tcgggcg       417

<210>   171
<211>   417
<212>   DNA
<213>   Yersinia kristensenii

<400>   171
gaaggggtgt cggcagctga ggtcatcatg accgtgttgc atgctggcgg taaatttgac      60

gataactcgt ataaagtttc cggcggtttg cacggcgtgg gtgtatccgt tgttaacgcc     120

ttgtctgaaa aactggaact ggtgattcgc cgtgaaggta aagttcacga gcagacttac     180

aaaatgggtg tgccgcaggc accactgaaa gtggtgggtg aaaccgatca gaccgggaca     240

accgtgcgtt tctggccgag tttccagact ttcaccaata acaccgaatt ccagtatgaa     300

atcttggcta aacgtctgcg cgagttgtca ttcctgaact ccggggtttc aatcaaactg     360

aaagacaaac gtaacgatag agaagaccat tttcattatg aaggtgggat tcgggcg        417

<210>   172
<211>   417
<212>   DNA
<213>   Yersinia mollaretii

<400>   172
gaaggggtat cagcagctga ggtcatcatg accgtgctgc atgctggcgg taagtttgat      60

gataactctt acaaagtttc cggcggtttg catggcgtgg gtgtgtcagt tgttaacgcc     120

ttgtctgaaa aactggagct ggtcatccgc cgtgaaggta aagttcacga gcaaacttat     180

aaaatgggtg tgccgcaggc ccctctgaaa gttgtgggtg aaactgacct gaccgggact     240

accgtgcgtt tttggccgag tttcgagacg ttcaccaata acactgaatt ccagtatgaa     300

attttagcta agcgcctgcg cgagctgtca ttcctgaact ccggcgtttc aatcaagctg     360

aaagacaaac gcaatgatag agaagaccac ttccattacg aaggtggtat caaggcg        417

<210>  173
<211>  417
<212>  DNA
<213>  Yersinia pestis T

<400>  173
gaaggggtat ctgcagcaga ggtcatcatg accgtgttgc atgctggcgg taaatttgac      60

gataactcct ataaagtttc cggcggtttg catggcgtag gggtatccgt tgttaacgcc     120

ctgtctgaaa agttggaact ggttattcgc cgtgaaggta aagttcacga gcaaacctac     180

aaaatgggcg tgccacaggc accgctgaaa gtggtgggtg aaactgagca aaccgggact     240

actgtgcgtt tctggccaag tttccagacc tttaccaata acaccgaatt ccagtatgag     300

atcctggcta aacgcctgcg cgaactctct ttcctgaact cgggtgtttc aatcaaactg     360

aaagacaaac gtaacgataa agaagaccac ttccattacg aaggtggtat caaggcg        417

<210>  174
<211>  417
<212>  DNA
<213>  Yersinia pseudotuberculosis sbsp. pseudotuberculosis

<400>  174
gaaggggtat ctgcagcaga ggtcatcatg accgtgttgc atgctggcgg taaatttgac      60

gataactcct ataaagtttc cggcggtttg catggcgtag gggtatccgt tgttaacgcc     120

ctgtctgaaa agttggaact ggttattcgc cgtgaaggta aagttcacga gcaaacctac     180

aaaatgggcg tgccacaggc accgctgaaa gtggtgggtg aaactgagca aaccgggacc     240

actgtgcgtt tctggccaag tttccagacc tttaccaata acaccgaatt ccagtatgag     300

atcctggcta aacgcctgcg cgaactctct ttcctgaact cgggtgtttc aatcaaactg     360

aaagacaaac gtaacgataa agaagaccac ttccattacg aaggtggtat caaggcg        417

<210>  175
<211>  417
<212>  DNA
<213>  Yersinia rohdei

<400>  175
gaaggggtat cggccgctga ggtcatcatg accgtcttgc atgctggcgg taaatttgac      60

gataactctt ataaagtttc cggcggtttg cacggcgtgg gtgtgtcagt tgttaacgcc     120

ctgtctgaaa aactggagct ggtgattcgc cgtgaaggta aagttcacga acagacttac     180

agaatgggtg tgccgcaggc accgctgaaa gttgtgggcg aaaccgacca gaccgggaca 240

actgtgcgct tctggccgag cttccagacg ttcaccaata acaccgaatt ccagtatgaa 300

atcctggcaa aacgcctgcg tgagctgtcg ttcctgaact ccggggtttc aatcaaactg 360

agagataaac gcaacgacaa agaagaccac ttccattatg aaggcggtat caaagcg 417

<210> 176
<211> 417
<212> DNA
<213> Yersinia ruckeri

<400> 176
gaaggggtat cagccgctga ggtcatcatg accgtgttac atgctggcgg taaatttgac 60

gataactcgt acaaagtttc tggcggcttg catggcgtgg gtgtgtcagt tgttaacgct 120

ctgtctgaaa aactggagtt ggtgattcgc cgtgaaggta aagttcacga gcagacttac 180

aaaatgggtg tgccgcaagc accactaaaa atggtgggtg aaaccgatca gaccgggact 240

acggtgcgtt tctggccgag cttccagacc ttcaccaata acaccgaatt tcaatacgaa 300

attctggcga aacgcttgcg tgagctgtcg ttcctgaact ccggggtttc aatcaaactg 360

aaagacaaac gtaacgacaa agaagaccac ttccattacg aaggcggtat caaagcg 417

<210> 177
<211> 417
<212> DNA
<213> Yokenella regensburgei T

<400> 177
gagggcgtgt ctgccgcaga agtgatcatg accgttctgc acgcaggcgg taagttcgac 60

gataactcgt ataaagtttc tggtggtctg cacggcgtag gcgtgtccgt agttaacgcc 120

ctgtcgcaga agctggagct ggtcatccag cgcgaaggta aaatccatcg ccagatttat 180

acccacggcg tgccgcaggc accgctggcc gtaacgggcg ataccgaaaa gaccggtacc 240

atggtgcgtt tctggccgag tctcgaaacc ttcaccaacg tcactgagtt tgaatacgaa 300

attctggcta agcgcctgcg tgagctgtcg ttcctgaact ccggcgtctc tattcgcctg 360

cgcgacaagc gtgacaacaa agaagaccac ttccactacg aaggcggcat taaggcg 417

<210> 178
<211> 501
<212> DNA
<213> Brenneria alni

<400> 178
acccactatg gccgggtatg tcctatcgaa acgccggaag gcccgaacat cggcctgatc 60

aactccttgt ctgtttatgc gcagactaac gagtatggat ttcttgaaac cccgtatcgt 120

cgcgtgcgtg acaatgtagt gacggatgag atccattatc tgtcggcgat tgaagaaggc 180

aactttgtta tcgctcaggc gaataccaac ctggatgaag aaggccgctt cattgatgaa 240

ctggtaacct gccgtaataa aggcgagtcc agcttgttca gccgcgatca ggttgaatat 300

atggacgtat ccacccagca ggttgtatcc gtaggtgctt cactgatccc cttcctggaa 360

cacgatgacg ccaaccgtgc attgatgggg gcgaacatgc aacgtcaggc ggtgcctact 420

ctgcgagctg ataagccgct ggttggtacc ggtatggagc gtgcagttgc ggttgactcc 480

ggtgtaacgg ctgtcgcaaa a 501

<210> 179
<211> 501
<212> DNA
<213> Brenneria quercina

<400> 179
acacactacg gtcgcgtatg tcctatcgaa acgccggaag gtccgaacat cggtctgatc 60

aactctctgt ccgtttatgc gcagaccaac gaatatggtt tcctcgaaac cccttatcgt 120

ctggttcgcg acggcgttgt gactgatgag attcattacc tgtctgcgat tgaagaaggc 180

aacttcgtca tcgctcaggc gaacaccaat ctggatgaag aaggtcactt cattgatgag 240

ctggtgacct gccgtaacaa aggcgagtcc agcttgttca gccgcgatca ggttgaatac 300

atggacgttt ccacccagca ggtggtttcc gtcggtgctt ccctgatccc gttcctggaa 360

cacgatgatg ctaaccgcgc cttgatgggt gcgaacatgc aacgtcaggc cgttcctact 420

ctgcgcgccg acaagccgct ggtaggtacc ggtatggaac gcgcggtggc ggtagactcc 480

ggtgttaccg cagtagcgaa a 501

<210> 180
<211> 501
<212> DNA
<213> Brenneria rubrifaciens

<400> 180
actcactatg gccgtgtttg tccaatcgaa acgccagaag gtccgaatat cggcctgatt 60

aactccctgt ccgtttacgc acaaaccaac gaatatggtt tcctggaaac cccgtatcgt 120

cgcgtgcgtg acaatgtggt gacggatgag atccattacc tgtcagcgat agaggaaggt 180

aacttcgtta tcgcgcaggc gaacaccaat ctcgatgaag aaggtcgttt catcgatgaa 240

ctggtgacct gccgtaataa aggcgagtct agcttgttca gccgcgatca ggttgaatat 300

atggacgttt ctacacagca ggttgtctct gtcggtgctt cactgatccc attcctggaa 360

cacgatgacg ccaaccgtgc attgatgggt gcgaacatgc aacgtcaggc cgttcctact 420

ctgcgtgctg acaagccgct ggttggtacg ggtatggaac gcgccgttgc ggttgactcc 480

ggtgtaacag ccgtagcaaa a 501

<210> 181
<211> 501
<212> DNA
<213> Brenneria salicis T

<400> 181
acgcactacg gtcgcgtatg tcctatcgaa acgccggaag gtccgaacat cggtttgatc 60

aactccttgt ctgtttatgc gcaaaccaac gaatacggtt tccttgaaac cccgtatcgt 120

cgtgtgcgtg acaacgtggt gacggatgaa atccattacc tgtcggcgat tgaggaaggc 180

aacttcgtta tcgcgcaggc gaacaccaat ctggacgaag aaggccgctt tattgacgaa 240

ctggttacct gccgtaataa aggcgagtcc agcttgttca gccgcgatca ggttgaatat 300

atggacgttt ccacacagca ggtcgtttct gtgggtgcat cgttgatccc gttcctggaa 360

catgatgacg ccaaccgcgc cctgatgggt gcgaacatgc aacgtcaggc tgtgcctact 420

ctgcgtgctg ataagccgct ggtggggacc gggatggaac gtgccgttgc ggttgactcc 480

ggcgtaaccg ctgtggcgaa a 501

<210> 182
<211> 501
<212> DNA
<213> Budvicia aquatica T

<400> 182
actcactatg gtcgcgtatg tccaatcgaa acgccggaag gtccaaacat cggtctgatc 60

aactcactgt ctgtttatgc tcagaccaat gagtatggtt tcttagaaac accttaccgt 120

cgcgtacgcg aaggcgtggt gaccgatgag atccactatt tatctgccat tgaagaaggc 180

aacttcgtta tcgctcaggc gaactcaaac ttgagtgacg aaggccactt tatggaagac 240

ctggttacct gtcgtaacaa aggcgaatca agcttattca gccgtgaaca agtcgactat 300

atggacgttt caacccaaca ggttgtgtcc gtaggtgctt ctttgattcc gttcctggag 360

catgatgatg ccaaccgtgc cctgatgggt gcgaacatgc aacgtcaggc agttcctact 420

ctgcgcgctg ataagccgct ggttggtacc ggtatggaac gtgctgtagc cgttgactcc 480

ggtgtaaccg cagtggcaac g 501

<210> 183
<211> 501
<212> DNA
<213> Buttiauxella agrestis T

<400> 183
acccactacg gtcgtgtatg tccaatcgaa acccctgaag gtccaaacat cggtctgatc 60

aactccctgt ctgtgtatgc acagactaac gaatacggtt tcttagaaac tccgtatcgt 120

cgcgttgttg aaggtgttgt gactgacgag attcattacc tttctgctat tgaagaaggt 180

aactacgtta tcgctcaggc gaacaccaac ctggacgaag aaggccgttt cgttgacgac 240

ctcgtaactt gccgtagcaa aggcgaatca agcttgttca gcaacgacca ggttgactac 300

atggacgttt ccacccaaca ggtggtttcc gtcggtgcgt ccctgatccc gttcctggaa 360

cacgatgacg ccaaccgtgc attgatgggt gcgaacatgc aacgtcaggc cgttccaact 420

ctgcgcgctg ataagccgct tgttggtact ggtatggaac gtgctgttgc cgttgactcc 480

ggtgttactg cggttgctaa a 501

<210> 184
<211> 501
<212> DNA

<213>  Buttiauxella brennerae

<400>  184
acccactacg gtcgtgtatg tccaatcgaa acccctgaag gtccgaacat cggtctgatc      60

aactccctgt ctgtttatgc acagactaac gaatacggtt tcttagaaac tccgtatcgt     120

cgcgttgttg aaggtgttgt cactgacgaa atccattacc tttctgcaat tgaagaaggt     180

aactatgtca tcgctcaggc taacaccaat ctggacgaag aaggccgttt cgtagacgac     240

ctggtaactt gccgtagtaa aggcgaatcc agcttgttca gtaacgacca ggttgactac     300

atggacgttt ctacccaaca ggtagtttcc gtcggtgcgt ccctgatccc gttcctggaa     360

cacgatgacg ccaaccgtgc attgatgggt gcgaacatgc aacgtcaggc cgttccaact     420

ctgcgtgctg ataagccgct ggttggtact ggtatggaac gtgctgttgc cgttgactcc     480

ggtgttactg ccgttgctaa a                                               501

<210>  185
<211>  501
<212>  DNA
<213>  Buttiauxella ferragutiae

<400>  185
acccactacg gtcgtgtatg tccaatcgaa acccctgaag gtccgaacat cggtctgatc      60

aactccctgt ctgtttatgc acagactaac gaatacggtt tcttagaaac tccgtatcgt     120

cgcgttgttg aaggtgttgt cactgacgaa atccattacc tttctgcaat tgaagaaggt     180

aactacgtta ttgctcaggc gaacaccaat ctggacgaag aaggccgttt cgtagacgac     240

ctggtaactt gccgtagtaa aggcgaatcc agcttgttca gcaacgacca ggttgactac     300

atggacgttt ctacccaaca ggtagtttcc gtcggtgcgt ccctgatccc gttcctggaa     360

cacgatgacg ccaaccgtgc attgatgggt gcgaacatgc aacgtcaggc cgttccaact     420

ctgcgtgctg ataagccgct ggttggtact ggtatggaac gtgctgttgc cgttgactcc     480

ggtgttactg cggttgctaa a                                               501

<210>  186
<211>  501
<212>  DNA
<213>  Buttiauxella gaviniae

<400>  186
acccactacg gtcgtgtatg tccaatcgaa acccctgaag gtccgaacat cggtctgatc      60

aactccctgt ctgtttatgc acagactaac gaatacggtt tcttagaaac tccgtatcgt     120

cgcgttgttg aaggtgttgt cactgacgaa atccattacc tttctgcaat tgaagaaggt     180

aactatgtca tcgctcaggc taacaccaat ctggacgaag aaggccgttt cgtagacgac     240

ctggtaactt gccgtagtaa aggtgaatcc agcttgttca gtaacgacca ggttgactac     300

atggacgttt ctacccaaca ggtagtttcc gtcggtgcgt ccctgatccc gttcctggaa     360

cacgatgacg ccaaccgtgc attgatgggt gcgaacatgc aacgtcaggc cgttccaact     420

ttgcgtgctg ataagccgct ggttggtact ggtatggaac gtgctgttgc cgttgactcc     480

ggtgttactg cggttgctaa a                                              501

<210>  187
<211>  501
<212>  DNA
<213>  buttiauxella izardii

<400>  187
acccactacg gtcgtgtatg tccaatcgaa acccctgaag gtccgaacat cggtctgatc      60

aactccctgt ctgtttatgc acagactaac gaatacggtt tcttagaaac tccgtatcgt     120

cgcgttgttg acggtgttgt gactgacgag attcattacc tttctgcaat tgaagaaggt     180

aactacgtta ttgctcaggc gaacaccaac ctggacgaag aaggccgttt cgtagacgac     240

ctcgtaactt gccgtagcaa aggcgaatca agcttgttca gcaacgacca ggttgactac     300

atggacgttt ccacccagca ggtggtttcc gtcggtgcgt ccctgatccc gttcctggaa     360

cacgatgacg ccaaccgtgc attgatgggt gcgaacatgc aacgtcaggc cgttccaact     420

ctgcgtgctg ataagccgct ggttggtact ggtatggaac gtgctgttgc cgttgattcc     480

ggtgttactg cagttgctaa a                                              501

<210>  188
<211>  501
<212>  DNA
<213>  Buttiauxella noackiae

<400>  188
acccactacg gtcgtgtatg tccaatcgaa acccctgaag gtccaaacat cggtctgatc      60

aactccctgt ctgtttatgc acagactaac gaatacggtt tcttagaaac tccgtatcgt     120

cgcgttgttg aaggtgttgt aactgacgag attcattacc tttctgctat tgaagaaggt     180

aactacgtta tcgctcaggc gaacaccaac ctggacgaag aaggccgttt cgtagacgac     240

ctggtaactt gccgtagcaa aggcgaatcc agcttgttca gcaacgacca ggttgactac     300

atggacgttt ctacccaaca ggtagtttcc gtcggtgcgt ccctgatccc gttcctggaa     360

cacgatgacg ccaaccgtgc attgatgggt gcgaacatgc aacgtcaggc cgttccaact     420

ctgcgtgctg ataagccgct ggttggtact ggtatggaac gtgctgttgc cgttgactcc     480

ggtgttactg ctgttgctaa a                                              501

<210>  189
<211>  501
<212>  DNA
<213>  buttiauxella warmboldiae

<400>  189
acccactacg gtcgtgtatg tccaatcgaa acccctgaag gtccgaacat cggtctgatc      60

aactccctgt ctgtttatgc acagactaac gaatacggct tcttagaaac tccgtatcgt     120

cgcgttgttg acggtgttgt gactgacgag attcattacc tttctgcaat tgaagaaggt     180

aactacgtta tcgctcaggc gaacaccaac ctggacgaag aaggccgttt cgtagacgac     240

ctcgtaactt gccgtagcaa aggcgaatca agcttgttca gcaacgacca ggttgactac 300

atggacgttt ccacccagca ggtggtttcc gtcggtgcgt ccctgatccc gttcctggaa 360

cacgatgacg ccaaccgtgc attgatgggt gcgaacatgc aacgtcaggc cgttccaact 420

ctgcgtgctg ataagccgct ggttggtact ggtatggaac gtgctgttgc cgttgattcc 480

ggtgttactg cagttgctaa a 501

<210> 190
<211> 501
<212> DNA
<213> Cedecea davisae T

<400> 190
acccactacg gtcgtgtgtg tccaatcgaa acccctgaag gtccgaacat cggcctgatc 60

aactccctgt ctgtttatgc gcagactaac gaatacggct tcttagaaac cccgtatcgt 120

cgcgtagttg acggcatcgt gaccgacgaa attcattacc tgtctgctat cgaagaaggt 180

aactacgtta tcgctcaggc gaacaccaac ctggacgaag aaggccgttt cgtagacgac 240

ctggttacct gccgtagcaa aggcgaatcc agcttgttca gcaacgacca ggttgactac 300

atggacgttt ccacccagca ggtggtttcc gtcggtgcgt ccctgatccc gttcctggaa 360

cacgatgacg ccaaccgtgc cctgatgggt gcgaacatgc aacgtcaggc ggttccaact 420

ctgcgtgctg ataagccgct ggttggtacc ggtatggaac gtgctgtagc cgttgactcc 480

ggtgttactg ccgtagccaa a 501

<210> 191
<211> 501
<212> DNA
<213> Cedecea lapagei

<400> 191
acccactacg gtcgtgtatg tccaatcgaa accccggaag gtccaaacat cggcctgatc 60

aactccctgt ccgtgtacgc acagactaac gaatacggct tccttgagac tccgtatcgt 120

cgcgttgttg acggcgttgt gaccgatgaa attcattacc tgtctgctat cgaagaaggt 180

aactacgtca tcgctcaggc gaacaccaac ctgaacgaag aaggccgttt cgtagacgac 240

ctggttacct gccgtagcaa aggcgaatcc agcttgttca gcaacgacca ggttgactac 300

atggacgttt ccacccagca ggtggtttcc gtcggtgcgt ccctgatccc gttcctggaa 360

cacgatgacg ccaaccgtgc attgatgggt gcgaacatgc aacgtcaggc ggttccaacg 420

ctgcgcgctg ataagccgct ggttggtacc ggtatggagc gtgctgtagc cgttgactcc 480

ggcgttaccg ccgtagccaa a 501

<210> 192
<211> 501
<212> DNA
<213> Cedecea neteri

<400> 192
acccactacg gtcgtgtgtg tccaatcgaa accccagaag gtccaaacat cggcctgatc 60

aactccctgt ctgtgtacgc acagactaac gaatacggct tccttgagac tccgtatcgt 120

cgcgttgttg acggcgttgt gaccgacgaa attcattacc tgtctgctat tgaagaaggt 180

aactacgtta tcgctcaggc gaacaccaac ctgaacgaag aaggccgttt cgtagacgac 240

ctcgttacct gccgtagcaa aggcgaatcc agcttgttca gcaacgacca ggttgactac 300

atggacgttt ccacccagca ggtggtttcc gtcggtgcgt ccctgatccc gttcctggaa 360

cacgatgacg ccaaccgtgc attgatgggt gcgaacatgc aacgtcaggc ggttccaact 420

ctgcgtgctg acaagccgct ggttggtacc ggtatggaac gtgctgtagc cgttgactcc 480

ggcgttaccg ccgtagctaa a 501

<210> 193
<211> 501
<212> DNA
<213> Citrobacter amalonaticus

<400> 193
acgcactatg gtcgcgtatg tccaatcgaa acgcctgaag gtccgaacat cggtctgatc 60

aactccctgt ccgtgtacgc acagactaac gaatatggct tcctcgagac gccgtatcgt 120

cgcgtggttg atggcgttgt gacagatgag attcattacc tgtctgctat tgaagaaggt 180

aacttcgtta tcgctcaggc gaactccaac ctggatgacg atggccactt cgtagaagat 240

ctggtgacct gccgtagcaa aggtgaatcc agcctgttca gccgtgacca ggttgactac 300

atggacgttt ccactcagca ggtggtttcc gtcggtgcgt ccctgatccc gttcctggaa 360

cacgatgacg ccaaccgtgc attgatgggt gcgaacatgc aacgtcaggc ggttccgact 420

ctgcgcgctg ataagccgct ggtaggtacc ggtatggaac gtgctgttgc cgttgactcc 480

ggtgttactg cggtggctaa a 501

<210> 194
<211> 501
<212> DNA
<213> Citrobacter braakii

<400> 194
actcactacg gtcgcgtatg tccaatcgaa acgcctgaag gtccgaacat cggtctgatc 60

aactccttgt ctgtgtacgc acagactaac gaatacggct tcctcgagac tccgtaccgt 120

aaagtgaccg acggtgttgt aactgacgaa attcattacc tgtcagctat cgaagaaggc 180

aactacgtta tcgctcaggc gaactccaac ctggatgacg aaggccactt tgtagaagat 240

ctggttacct gccgtagcaa aggcgaatcc agcttgttca gccgtgacca ggttgactac 300

atggacgtat ccacccagca ggtggtatcc gtcggtgcgt ccctgatccc gttcctggaa 360

cacgatgacg ccaaccgtgc attgatgggt gcgaacatgc aacgtcaggc ggttccgact 420

ctgcgtgctg ataagccgct ggttggtacc ggtatggaac gtgctgttgc cgtcgactcc 480

ggtgttaccg cagttgctaa a 501

<210> 195
<211> 501
<212> DNA
<213> Citrobacter diversus

<400> 195
acccactacg gtcgcgtatg tcctatcgaa acgcctgaag gtccgaacat cggtctgatc 60

aactccctgt ccgtgtacgc acagactaac gaatacggtt tcctcgagac gccgtatcgt 120

aaagtcaccg acggcgttgt gactgacgaa attcattacc tgtctgctat cgaagaaggt 180

aactacgtta tcgctcaggc gaactccaac ctggatgacg aaggccactt tgtagaagat 240

ctggttactt gccgtagcaa aggcgaatcc agcttgttca gccgcgacca ggttgactac 300

atggacgttt ccactcaaca ggtggtttcc gtcggtgcgt ccctgatccc gttcctggaa 360

cacgatgacg ccaaccgtgc attgatgggt gcgaacatgc aacgtcaggc ggttccgact 420

ctgcgcgctg ataagccgct ggttggtacc ggtatggaac gtgctgttgc cgttgactcc 480

ggtgtaactg cggttgctaa a 501

<210> 196
<211> 501
<212> DNA
<213> Citrobacter farmeri

<400> 196
actcactatg gtcgcgtatg tcctatcgaa acgcctgaag gtccgaacat cggtctgatc 60

aactccctgt ccgtgtacgc acagactaac gaatatggct tcctcgagac gccgtaccgt 120

cgcgttgttg atggtgttgt gactgatgag attcattacc tgtctgctat tgaagaaggt 180

aacttcgtta tcgctcaggc gaactccaac ctggatgacg atggccactt cgtagaagat 240

ctggtgacct gccgtagtaa aggcgaatcc agcttgttca gccgtgatca ggttgactac 300

atggacgttt ccacccagca ggtggtttcc gtcggtgcgt ctctgatccc gttcctggaa 360

cacgatgacg ccaaccgtgc attgatgggt gcgaacatgc aacgtcaggc ggttccgact 420

ctgcgcgctg ataagccgct ggtaggtacc ggtatggaac gtgctgttgc cgttgactcc 480

ggtgtaactg cggtggctaa a 501

<210> 197
<211> 501
<212> DNA
<213> Citrobacter freundii T

<400> 197
actcactacg gtcgcgtatg tccaatcgaa acgcctgaag gtccgaacat cggtctgatc 60

aactccctgt ccgtgtatgc acagactaac gaatacggct tcctcgagac tccgtaccgt 120

aaagtgaccg acggtgttgt aactgacgaa attcattacc tgtctgctat cgaagaaggc 180

aactacgtta tcgctcaggc gaactccaac ctggatgacg aaggccactt tgtagaagat 240

ctggttacct gccgtagcaa aggcgaatcc agcttgttca gccgtgatca ggttgactac 300

atggacgtat ccacccagca ggtggtatcc gtcggtgcgt ccctgatccc gttcctggaa 360

cacgatgacg ccaaccgtgc attgatgggt gcgaacatgc aacgtcaggc ggttccgact 420

ctgcgcgctg ataagccgct ggttggtacc ggtatggaac gtgctgttgc cgttgactcc 480

ggtgttactg cagttgctaa a 501

<210> 198
<211> 501
<212> DNA
<213> Citrobacter gillenii

<400> 198
actcactacg gtcgcgtatg tccaatcgaa acccctgaag gtccgaacat cggtctgatc 60

aactccctgt ccgtgtacgc acagactaac gaatatggct tccttgagac gccgtatcgt 120

aaagtgactg acggtgttgt taccgacgaa attcattact tgtctgctat cgaagaaggc 180

aactacgtta tcgctcaggc gaactccaac ctggatgacg aaggccactt tgtagaagac 240

ctggtaacat gtcgtagcaa aggcgaatcc agcttgttca gccgtgacca ggttgactac 300

atggacgtat ccacccagca ggtggtatcc gtcggtgcgt ccctgatccc gttcctggaa 360

cacgatgacg ccaaccgtgc attgatgggt gcgaacatgc aacgtcaggc ggttccgact 420

ctgcgtgctg ataagccgct ggttggtacc ggtatggagc gtgctgttgc tgttgactcc 480

ggtgttactg ctgttgctaa g 501

<210> 199
<211> 501
<212> DNA
<213> Citrobacter koseri

<400> 199
acccactacg gtcgcgtatg tcctatcgaa acgcctgaag gtccgaacat cggtctgatc 60

aactccctgt ccgtgtacgc acagactaac gaatacggtt tccttgagac tccgtatcgt 120

aaagtcaccg acggcgtggt gactgacgaa attcattacc tgtctgctat cgaagaaggt 180

aactacgtta tcgctcaggc gaactccaac ctggatgacg aaggccactt tgtagaagat 240

ctggttacct gccgtagcaa aggcgaatcc agcttgttca gccgcgacca ggtcgactac 300

atggacgttt ccactcagca ggtggtttcc gtcggtgcgt ccctgatccc gttcctggaa 360

cacgatgacg ccaaccgtgc attgatgggt gcgaacatgc aacgtcaggc ggttccgact 420

ctgcgcgctg ataagccgct ggttggtacc ggtatggaac gtgctgttgc cgttgactcc 480

ggtgtaactg cggttgctaa a 501

<210> 200
<211> 501
<212> DNA
<213> Citrobacter murliniae

<400> 200
actcactatg gtcgcgtatg cccaatcgaa acgcctgaag gtccgaacat cggtctgatc 60

aactccctgt ccgtgtacgc acagactaac gaatacggtt tccttgagac tccgtaccgt 120

aaagtgaccg acggtgttgt aactgacgaa attcattacc tgtctgctat cgaagaaggc 180

aactacgtta tcgctcaggc gaactccaac ctggatgacg aaggccactt tgtagaagac 240

ctggtaactt gccgtagcaa aggcgaatcc agcttgttca gccgcgacca ggttgactac 300

atggacgtat ccacccaaca ggtggtttcc gtcggtgcgt ccctgatccc gttcctggaa 360

cacgatgacg ccaaccgtgc attgatgggt gcgaacatgc aacgtcaggc cgttccaact 420

ctgcgcgctg ataagccgct ggttggtacc ggtatggaac gtgctgttgc cgttgactcc 480

ggtgttactg cagttgctaa a 501

<210> 201
<211> 501
<212> DNA
<213> Citrobacter rodentium

<400> 201
actcactacg gtcgcgtatg tccaatcgaa acgccggaag gtccgaacat cggtctgatc 60

aactccctgt ccgtgtacgc gcagactaac gaatatggct tccttgagac gccgtatcgt 120

cgcgtggttg atggcgtagt aacagatgaa attcactacc tgtctgctat tgaagaaggc 180

aacttcgtta tcgctcaggc gaactccaac ctggatgacg acggccactt cgtagaagat 240

ctggtgacct gccgtagtaa aggcgaatcc agcttgttca gccgcgacca ggttgactac 300

atggacgttt ccacccagca ggtggtttcc gtcggtgcgt ccctgatccc gttcctggaa 360

cacgatgacg ccaaccgtgc gttgatgggt gcgaacatgc aacgtcaggc ggttccgacc 420

ctgcgcgctg ataagccgct ggtaggtacc ggtatggaac gtgctgttgc cgttgactcc 480

ggtgtaactg cggtcgctaa g 501

<210> 202
<211> 501
<212> DNA
<213> Citrobacter sedlakii

<400> 202
acccactacg gtcgcgtatg tccaatcgaa acgcctgaag gtccaaacat cggtctgatc 60

aactccctgt ccgtgtacgc gcagacgaac gaatatggct tccttgagac gccgtatcgt 120

cgcgtggttg atggcgtggt aaccgatgaa attcattacc tgtctgctat tgaagaaggc 180

aacttcgtta tcgctcaggc gaactccaac ctggatgaag acggccactt cgtagaagat 240

ctggtgacct gccgtagcaa aggcgaatcc agcttgttca gccgcgacca ggttgactac 300

atggacgtat ccacccagca ggtggtatcc gtcggtgcgt ccctgatccc gttcctggaa 360

cacgatgacg ccaaccgtgc attgatgggt gcgaacatgc aacgtcaggc cgttccgact 420

ctgcgtgcag ataagccgct ggttggtacc ggtatggaac gtgctgttgc cgttgactcc 480

ggtgtgactg ccgttgctaa g 501

<210> 203
<211> 501
<212> DNA
<213> Citrobacter werkmanii

<400>  203
actcactacg gtcgcgtatg tccaatcgaa acgcctgaag gtccgaacat cggtctgatc      60

aactccctgt ccgtgtatgc acagactaac gaatacggct tcctcgagac tccgtaccgt     120

aaagtgaccg acggtgttgt tactgacgaa attcattacc tgtctgctat cgaagaaggc     180

aactacgtta tcgctcaggc gaactccaac ctggatgacg aaggccactt tgtagaagat     240

ctggttacct gccgtagtaa aggcgaatcc agcttgttca gccgcgacca ggttgactac     300

atggacgtat ccacccagca ggtggtatcc gtcggtgcgt ccctgatccc gttcctggaa     360

cacgatgacg ccaaccgtgc attgatgggt gcgaacatgc aacgtcaggc ggttccgact     420

ctgcgtgctg ataagccgct ggttggtacc ggtatggaac gtgctgttgc cgttgactcc     480

ggtgttactg cagttgctaa a                                               501

<210>  204
<211>  501
<212>  DNA
<213>  Citrobacter youngae

<400>  204
actcactatg gtcgcgtatg tccaatcgaa acgcctgaag gcccgaacat cggtctgatc      60

aactccctgt ccgtgtatgc acagactaac gaatacggct tcctcgagac gccgtaccgt     120

aaagtgaccg acggtgttgt aaccgatgaa attcattacc tgtctgctat cgaagaaggc     180

aactacgtta tcgctcaggc gaactccaac ctggatgacg aagggcactt tgtagaagat     240

ctggttacct gccgtagcaa aggcgaatcc agcttgttca gccgtgatca ggttgactac     300

atggacgtat ccacccagca ggtggtatcc gtcggtgcgt ccctgatccc gttcctggaa     360

cacgatgacg ccaaccgtgc attgatgggt gcgaacatgc aacgtcaggc ggttccaact     420

ctgcgcgctg ataagccgct ggttggtacc ggtatggaac gtgctgttgc cgttgactcc     480

ggtgttactg ctgttgctaa a                                               501

<210>  205
<211>  501
<212>  DNA
<213>  Dickeya chrysanthemi T

<400>  205
actcactacg gtcgtgtatg tcctatcgaa acgccggaag gtccgaacat cggtcttatc      60

aactcgctgt ccgtgtatgc acagactaac gagtatggtt tcctggaaac cccgtaccgc     120

cgtgtacgcg atggcgtggt aaccgacgaa attcattacc tgtctgctat cgaagaaggc     180

aactacgtta ttgcccaggc gaacaccaac ctggatgacg aaggccactt catcgatgaa     240

ctggtcacct gccgtagcaa aggcgaatcc agcttgttca gccgtgatca ggttgactac     300

atggacgttt ccacccagca ggtggtttcc gtcggtgcgt cactgatccc gttcctggaa     360

cacgatgacg ccaaccgcgc cctgatgggt gcgaacatgc aacgtcaggc ggtgccgacc     420

ctgcgtgccg acaagccgct ggtaggtact ggtatggagc gtgctgttgc cgttgactcc     480

ggtgtaactg cggttgctaa a 501

<210> 206
<211> 501
<212> DNA
<213> Dickeya dadantii

<400> 206
acccattacg gtcgcgtatg tcctatcgaa acgccggaag gtccgaacat cggtctgatc 60

aactcgttgt ccgtgtacgc gcagaccaac gaatacggct tcctggaaac cccgtatcgc 120

cgtgtgcgcg atggcgtggt gaccgacgaa atccattacc tgtcggcgat tgaagaaggc 180

aattacgtta ttgctcaggc gaacaccaac ctggacgacg aaggtcactt catcgaggaa 240

ttggttacct gccgtagcaa aggcgaatcc agcttgttca gccgcgatca ggttgactac 300

atggacgttt ccacccagca ggtagtatcc gtgggtgcgt ccctgattcc gttcctggaa 360

cacgatgacg ccaaccgcgc cctgatgggt gcgaacatgc aacgtcaggc ggtaccgact 420

ctgcgtgctg acaagccgct ggtgggcacc ggtatggaac gtgctgtggc agtggactcc 480

ggtgtaaccg ctgttgctaa a 501

<210> 207
<211> 501
<212> DNA
<213> Edwardsiella hoshinae

<400> 207
actcactacg gtcgcgtatg tcctatcgag acgccggaag gcccgaacat cggtctgatc 60

aactccctgt ctgtgtacgc acagaccaac gagtatggtt tcctggaaac cccgtaccgc 120

cgcgtgcgtg acggtatcgt gaccgatgag atccattacc tatctgctat cgaagaaggc 180

aacttcgtta tcgcccaggc taactccaac ctggatgagg aaggtcgctt cgttgaagat 240

ctggttactt gccgtagcaa gggtgaatcc agcctgttca gtcgcgatca agttgactac 300

atggacgttt ccactcagca ggtggtttcc gtcggtgcgt ctctgatccc gttcctggag 360

cacgatgacg ccaaccgtgc tctgatgggt gcgaacatgc aacgtcaggc tgtaccgacc 420

ctgcgcgcgg ataagccgct ggttggtacc gggatggagc gcgcggtcgc cgtcgactcc 480

ggtgtaactg ctgtcgctaa g 501

<210> 208
<211> 501
<212> DNA
<213> Edwardsiella ictaluri

<400> 208
acccactacg gtcgcgtatg tcctatcgaa acgccggaag gtccgaacat cggtctgatc 60

aactccctgt ctgtgtacgc acagaccaac gagtatggtt tcctggaaac tccgtatcgc 120

cgcgtgcgtg acggtatcgt gaccgacgag atccattacc tgtctgccat cgaagaaggc 180

aacttcgtta tcgcccaggc gaactccaac ctggatgatg aaggccgctt tgtagaagat 240

ctggttacct gtcgcagcaa gggcgaatcc agcctgttca gccgtgacca ggtcgactac 300

atggacgttt ccacccagca ggtggtttcc gtcggtgcgt ccctgattcc gttcctggaa 360

cacgatgatg ccaaccgtgc cctgatgggt gcgaacatgc agcgtcaggc cgtgccgacc 420

ctgcgcgccg ataagccgct ggttggtacc ggtatggagc gcgccgttgc ggttgactcc 480

ggtgtaacct ccgttgccaa g 501

<210> 209
<211> 501
<212> DNA
<213> Edwardsiella tarda T

<400> 209
acccactacg gtcgcgtatg tcctatcgaa acgccggaag gtccgaacat cggtctgatc 60

aactccctgt ctgtgtacgc acagaccaac gagtatggtt tcctggaaac tccgtatcgc 120

cgcgtgcgtg acggtatcgt gaccgacgag atccattacc tgtctgccat cgaagaaggc 180

aacttcgtta tcgcccaggc gaactccaac ctggatgatg aaggccgctt cgttgaagat 240

ctggtaacgt gccgcagcaa gggcgaatcc agcttgttca gccgtgacca ggttgactac 300

atggacgttt ccacccagca ggtggtttcc gtcggtgcgt ccctgatccc gttcttggaa 360

cacgatgacg ccaaccgtgc tttgatgggt gcgaacatgc aacgtcaggc cgtaccgacc 420

ctgcgcgccg ataagccgct ggttggtacc ggtatggagc gtgcggttgc cgtcgactcc 480

ggtgtaacct ccgtcgccaa g 501

<210> 210
<211> 501
<212> DNA
<213> Enterobacter aerogenes

<400> 210
actcactacg gtcgcgtatg tccaatcgaa acgcctgaag gtccgaacat cggtctgatc 60

aactccctgt ccgtgtacgc acagactaac gaatacggtt tccttgagac cccgtaccgt 120

aaagtgaccg acggtgttgt taccgacgaa attcattacc tgtctgctat cgaagaaggt 180

aactacgtca ttgctcaggc gaactccaac ctggatgacg aaggccactt tgttgaagat 240

ctggttacct gccgtagtaa aggcgaatcc agcttgttca gccgcgacca ggttgactac 300

atggacgtat ccacccagca ggtggtatcc gtcggtgcgt ccctgatccc gttcctggaa 360

cacgatgacg ccaaccgtgc attgatgggt gcgaacatgc aacgtcaggc ggttccgact 420

ctgcgcgctg ataagccgct ggttggtacc ggtatggaac gtgctgttgc cgtcgactcc 480

ggtgttactg cggtagctaa a 501

<210> 211
<211> 501
<212> DNA
<213> Enterobacter amnigenus

<400> 211
actcactacg gtcgcgtgtg tcctatcgaa acgcctgaag gtccaaacat cggtctgatc 60

aactccttgt ccgtgtacgc acagacaaac gaatacggct tcctcgaaac gccgtaccgt 120

aaagtgactg acggtgttgt gaccgacgaa atccattacc tgtctgctat cgaagaaggt 180

aactacgtca tcgctcaggc gaacaccaac ctggacgaag aaggtcgttt cgttgacgat 240

ctcgttacct gccgtagcaa aggcgaatcc agcttgttca gcaatgatca ggtggattat 300

atggacgttt cgacccagca gatcgtttct gttggtgcgt cactgattcc attcctggaa 360

cacgatgacg ccaaccgtgc attgatgggt gcgaacatgc aacgtcaggc ggttccgact 420

ctgcgcgctg ataagccgct ggttggtacc ggtatggaac gtgctgttgc cgttgactcc 480

ggtgtaactg cggttgcgaa a 501

<210> 212
<211> 501
<212> DNA
<213> Enterobacter asburiae

<400> 212
acgcactacg gtcgcgtatg tccaatcgaa acgcctgaag gtccaaacat cggtctgatc 60

aactccctgt ccgtgtacgc acagactaac gaatacggtt tcctcgagac tccgtaccgt 120

aaagtgactg acggtgttgt aaccgacgaa attcactacc tgtctgctat cgaagaaggc 180

aactacgtta tcgctcaggc gaactccaac ctggatgacg aaggccactt tgtagaagat 240

ctggtaacct gccgtagcaa aggcgaatcc agcttgttca gccgcgacca ggttgactac 300

atggacgtat ccacccagca ggtggtatcc gtcggtgcgt ccctgatccc gttccttgaa 360

cacgatgacg ccaaccgtgc attgatgggt gcgaacatgc aacgtcaggc cgttccaact 420

ctgcgcgctg ataagccgct ggttggtacc ggtatggaac gtgctgttgc cgttgactcc 480

ggtgttactg cggttgctaa a 501

<210> 213
<211> 501
<212> DNA
<213> Enterobacter cancerogenus

<400> 213
acgcactacg gtcgcgtatg tccaatcgaa acgcctgaag gtccaaacat cggtctgatc 60

aactccctgt ccgtgtacgc acagactaac gaatacggtt tcctcgagac tccgtatcgt 120

aaagtgactg acggtgttgt aaccgacgaa attcattacc tgtctgctat cgaagaaggc 180

aactacgtta tcgctcaggc gaactccaac ctggatgacg aaggccactt tgtagaagat 240

ctcgttacct gtcgtagtaa aggcgaatcc agcctgttca gccgtgacca ggttgactac 300

atggacgttt ccacccagca ggtggtctcc gtcggtgcgt ccctgatccc gttcctggaa 360

cacgatgacg ccaaccgtgc attgatgggt gcgaacatgc aacgtcaggc cgttccaacg 420

ctgcgcgctg ataagccgct ggttggtacc ggtatggaac gtgctgttgc cgttgactcc 480

ggtgtaactg cggttgctaa a 501

<210> 214

<211>  501
<212>  DNA
<213>  Enterobacter cloacae sbsp. cloacae T

<400>  214
actcactacg gtcgcgtatg tccaatcgaa acgcctgaag gtccaaacat cggtctgatc      60

aactccctgt ccgtgtacgc acagacaaac gaatacggtt tccttgagac cccgtaccgt     120

aaagtgactg acggtgttgt taccgacgaa attcattacc tgtctgctat tgaagaaggc     180

aactacgtta tcgctcaggc gaactccaac ctggatgacg aaggccactt tgtagaagat     240

ctggttacct gccgtagcaa aggcgaatcc agcttgttca gccgcgacca ggttgactac     300

atggacgtat ccacccagca ggtggtatcc gtcggtgcgt ccctgatccc gttcctggaa     360

cacgatgacg ccaaccgtgc attgatgggt gcgaacatgc aacgtcaggc cgttccaact     420

ctgcgtgctg ataagccgct ggttggtacc ggtatggaac gtgctgttgc cgttgactcc     480

ggtgttactg cagtggctaa g                                               501

<210>  215
<211>  501
<212>  DNA
<213>  Enterobacter cloacae sbsp. dissolvens

<400>  215
acgcactacg gtcgcgtatg tccaatcgaa acccctgaag gtccaaacat cggtctgatc      60

aactccctgt ccgtgtatgc acagacaaac gaatacggtt tccttgagac cccgtaccgt     120

aaagtgactg atggtgttgt taccgacgaa attcattacc tgtctgctat tgaagaaggc     180

aactacgtta tcgctcaggc gaactccaac ctggatgacg aaggccactt tgtagaagat     240

ctggttacct gccgtagcaa aggcgaatcc agcttgttca gccgcgacca ggttgactac     300

atggacgtat ccacccagca ggtggtatcc gtcggtgcgt ccctgatccc gttcctggaa     360

cacgatgacg ccaaccgtgc attgatgggt gcgaacatgc aacgtcaggc cgttccaact     420

ctgcgtgctg ataagccgct ggttggtacc ggtatggaac gtgctgttgc cgttgactcc     480

ggtgttactg cagtggctaa g                                               501

<210>  216
<211>  501
<212>  DNA
<213>  Enterobacter cowanii

<400>  216
acccactacg gtcgcgtatg tccaatcgaa acgcctgaag gtccgaacat cggtctgatt      60

aactccctct ccgtctatgc acagactaac gaatacggct tcctcgaaac gccgtaccgt     120

aaagtgactg acggcgtggt gactgacgag atccattacc tctctgcaat tgaagagggt     180

aactacgtta tcgctcaggc gaacaccaac ctgacggaag aagggcgttt tgcagaagat     240

ctggtaacgt gccgcagcaa aggcgaatca agcctcttca gcgcagacca ggttgactac     300

atggacgtat ccacccagca ggtggtttcc gtcggtgcat ccctgattcc gttcctggaa     360

cacgatgacg ccaaccgtgc attgatgggt gcgaacatgc aacgtcaggc ggttccgact     420

ctgcgcgctg ataagccgct ggttggtacc ggtatggaac gtgctgttgc cgttgactcc 480

ggcgtaactg ccgttgcgaa a 501

<210> 217
<211> 501
<212> DNA
<213> Enterobacter gergoviae

<400> 217
actcactacg gtcgcgtctg cccaatcgaa accccggaag gtccgaacat cggtctgatc 60

aactccctgt ccgtgtacgc acagactaac gaatacggct tcctcgaaac cccgtatcgt 120

aaagtgaccg acggcgtggt gaccgacgaa attcattacc tgtctgctat tgaagagggt 180

aacttcgtta ttgcccaggc gaactccaac ctgagcgacg aaggccgttt cgtagacgat 240

ctggttacct gccgtagcaa aggcgaatcg agcctgttca gccgcgacca ggttgactac 300

atggacgtat ccacccagca ggtggtttcc gtcggtgcgt ccctgatccc gttcctggaa 360

cacgatgacg ccaaccgtgc attgatgggt gcgaacatgc aacgtcaggc ggttccgacc 420

ctgcgcgctg ataagccgct ggtaggtacc ggtatggaac gtgctgttgc cgtcgactcc 480

ggcgtaacct ccgttgccaa g 501

<210> 218
<211> 501
<212> DNA
<213> Enterobacter hormaechei

<400> 218
actcactacg gtcgcgtatg tccaatcgaa acgcctgaag gtccaaacat cggtctgatc 60

aactccctgt ccgtgtacgc acagacgaac gaatacggtt tccttgagac cccgtatcgt 120

aaagtgaccg acggtgtggt taccgacgaa attcattacc tgtctgctat cgaagaaggc 180

aactacgtta tcgctcaggc gaactccaac ctggatgacg aaggccactt tgtagaagat 240

ctggttacct gccgtagcaa aggcgaatcc agcttgttca gccgcgacca ggttgactac 300

atggacgtat ccacccagca ggtggtatcc gtcggtgcgt ccctgatccc gttcctggaa 360

cacgatgacg ccaaccgtgc attgatgggt gcgaacatgc aacgtcaggc cgttccaact 420

ctgcgtgctg ataagccgct ggttggtacc ggtatggaac gtgctgttgc cgttgactcc 480

ggtgttacgg cagtggctaa g 501

<210> 219
<211> 501
<212> DNA
<213> Enterobacter kobei

<400> 219
actcactacg gtcgcgtatg tccaatcgaa acgcctgaag gtccgaacat cggtctgatc 60

aactccctgt ccgtttatgc acagactaac gaatacggtt tccttgagac cccgtaccgt 120

aaagtcactg acggtgtggt aactgacgag atccattacc tttctgcgat tgaagaaggt 180

aactacgtta tcgctcaggc gaacaccaac ctggacgacg atggtcgctt tgttgatgat 240

ctcgtaacct gccgtagcaa aggcgaatcc agcttgttca gcaacgacca ggttgactac 300

atggacgttt ccacccagca ggtggtttcc gtcggtgcgt ccctgatccc gttcctggaa 360

cacgatgacg ccaaccgtgc attgatgggt gcgaacatgc aacgtcaggc cgttccgact 420

ctgcgcgctg acaagccgct ggttggtacc ggtatggaac gtgctgttgc cgttgactcc 480

ggtgtaaccg cagttgctaa g 501

<210> 220
<211> 501
<212> DNA
<213> Enterobacter ludwigii

<400> 220
actcactacg gtcgcgtatg tccaatcgaa acgcctgaag gtccaaacat cggtctgatc 60

aactccctgt ccgtgtacgc acagactaac gaatacggtt tccttgagac gccgtaccgt 120

aaagtgaccg acggtgttgt aactgacgaa attcactacc tgtctgctat cgaagaaggc 180

aactacgtta tcgctcaggc gaactccaac ctggatgacg aaggccactt tgtagaagat 240

ctggttacct gccgtagcaa aggcgaatcc agcttgttca gccgcgacca ggttgactac 300

atggacgtat ccacccagca ggtggtatcc gtcggtgcgt ccctgatccc gttcctggaa 360

cacgatgacg ccaaccgtgc attgatgggt gcgaacatgc aacgtcaggc cgttccaact 420

ctgcgtgctg ataagccgct ggttggtacc ggtatggaac gtgctgttgc cgttgactcc 480

ggtgttactg cggtggctaa a 501

<210> 221
<211> 501
<212> DNA
<213> Enterobacter nimipressuralis

<400> 221
actcactacg gtcgcgtatg tccaatcgaa acgcctgaag gtccaaacat cggtctgatc 60

aactccctgt ccgtgtacgc acagactaac gaatacggtt tcctcgagac tccgtaccgt 120

aaagtgactg acggtgttgt aaccgacgaa attcattacc tgtctgctat cgaagaaggt 180

aactacgtca tcgctcaggc gaacaccaac ctggacgaag aaggtcgttt cgttgacgat 240

ctcgttacct gccgtagcaa aggcgaatcc agccttttca gcaatgatca ggtggactac 300

atggacgttt ccacgcagca gatcgtttct gttggtgcgt ccctgattcc attcctggaa 360

cacgatgacg ccaaccgtgc attgatgggt gcgaacatgc aacgtcaggc ggttccgact 420

ctgcgcgctg ataagccgct ggttggtacc ggtatggaac gtgctgttgc cgttgactcc 480

ggtgtaaccg cagttgctaa a 501

<210> 222
<211> 501
<212> DNA
<213> Enterobacter pyrinus

<400>  222
actcactacg gtcgcgtgtg cccaatcgaa acccctgaag gtccgaacat cggtctgatc      60

aactccctgt ccgtgtacgc acagactaac gaatacggct tccttgaaac cccgtatcgt     120

aaagtgacag acggtgtcgt tactgacgaa attcattacc tttctgctat tgaagaaggt     180

aacttcgtta tcgcgcaggc gaactccaac ctgagcgacg ataaccgctt cgtagacgat     240

ctggttacct gccgtagcaa aggcgaatcg agcctgttca gccgcgatca ggttgactac     300

atggacgtat ccacccagca ggtggtttcc gtcggtgcgt ccctgatccc gttcctggaa     360

cacgatgacg ccaaccgtgc attgatgggt gcgaacatgc aacgtcaggc ggttccgacc     420

ctgcgcgctg ataagccgct ggtaggtacc ggtatggaac gtgctgttgc cgttgactcc     480

ggcgttaccg ccgtagcgaa a                                               501

<210>  223
<211>  501
<212>  DNA
<213>  Enterobacter radicincitans

<400>  223
acccactacg gtcgcgtatg tccgatcgaa acgcctgaag gtccgaacat cggtctgatc      60

aactccctgt ccgtgtatgc acagactaac gaatacggct tcctcgaaac gccgtaccgt     120

aaagtgactg acggcgtggt aactgacgag atccattacc tttctgcaat tgaagaaggt     180

aactatgtta tcgctcaggc gaacaccaac ctgacggaag aaggtcgttt tgtagacgat     240

ctggtgacct gccgcagcaa aggcgaatcc agcctcttca gcgcagacca ggttgactac     300

atggacgttt ctacccagca ggtggtttcc gtcggtgcgt ccctgatccc gttcctggaa     360

cacgatgacg ccaaccgtgc attgatgggt gcgaacatgc aacgtcaggc ggttccgact     420

ctgcgcgctg ataagccgct ggttggtacc ggtatggaac gtgctgttgc cgttgactcc     480

ggtgtaaccg cagttgcgaa a                                               501

<210>  224
<211>  501
<212>  DNA
<213>  Enterobacter sakazakii

<400>  224
acccactacg gtcgcgtatg tcctatcgaa acgccggaag gtccgaacat cggtctgatc      60

aactccctgt ccgtgtacgc acagactaac gagtatggct tccttgagac gccgtaccgt     120

aaagtgaccg acggtgtggt gactgatgaa attcattacc tctccgctat cgaagaaggt     180

aactacgtta tcgcgcaggc gaacaccaac ctgaccgaag aaggccgctt cgccgatgat     240

ttggtcacct gccgcagcaa aggcgaatcc agcctcttca gcgcagacca ggttgactat     300

atggacgttt ccacccagca ggtggtttct gtcggtgcgt ccctgatccc gttccttgag     360

cacgatgacg ccaaccgtgc attgatgggt gcgaacatgc aacgtcaggc ggttccgacc     420

ctgcgcgctg ataagccgct ggtaggtacc ggtatggaac gtgctgttgc cgttgactcc     480

ggtgtaaccg ccgtggccaa a                                               501

<210> 225
<211> 501
<212> DNA
<213> Erwinia amylovora T

<400> 225
actcactatg gtcgcgtatg tccaatcgaa accccggaag gtccgaacat cggtttgatc 60

aactccttgt ctgtctatgc acagaccaac gagtacggtt tcctggaaac cccataccgt 120

cgcgtgcgcg atggcgtggt gaccgacgaa attcattacc tctctgccat tgaagagggt 180

aactacgtta ttgctcaggc aaacaccaac ctcgacgacg aaggtcactt cgttgacgac 240

ctggtgacct gccgcagcaa aggcgaatcg agcctgttca gccgcgatca ggttgactac 300

atggacgttt ccacccagca ggtggtttcc gttggtgcgt cactgatccc gttcctggaa 360

cacgatgacg ccaaccgcgc attgatgggt gcaaacatgc aacgtcaggc ggtaccgact 420

ctgcgcgctg ataagccgct ggtaggtacc ggtatggaac gcgcggtagc ggtcgactcc 480

ggtgttactg ccgtagcgaa a 501

<210> 226
<211> 501
<212> DNA
<213> Erwinia aphidicola

<400> 226
actcactacg gtcgcgtatg tccaatcgaa acgccggaag gtccaaacat cggtctgatc 60

aactccctct ctgtatacgc acagacgaat gagtacggtt tccttgaaac cccatatcgt 120

cgcgtgcgcg aaggtgtggt gaccgacgaa attcattacc tctctgctat tgaagagggt 180

aactacgtta tcgctcaggc taacaccaac ctcgacgacg aaggtcactt cgtagacgat 240

ctggtgactt gccgtagcaa aggcgaatca agtctgttca gccgcgatca ggttgactac 300

atggacgttt ccacccagca ggtggtttcc gtcggtgcgt cactgatccc gttcctggaa 360

cacgatgacg ccaaccgcgc attgatgggt gcaaacatgc aacgtcaggc ggttccaact 420

ctgcgtgctg ataagccgct ggttggtacc ggtatggaac gcgcggtagc ggttgactcc 480

ggtgtaaccg ccgtagcgaa a 501

<210> 227
<211> 501
<212> DNA
<213> Erwinia billingiae

<400> 227
acgcactacg gtcgcgtgtg cccaatcgaa acgcctgaag gtccaaacat cggtctgatc 60

aactccttgt ccgtgtacgc acagaccaat gagtacggtt tcctggaaac cccataccgt 120

cgcgttcgcg aaggcgtggt gaccgacgaa attcattacc tctctgcaat tgaagagggt 180

aactacgtta tcgctcaggc taacaccaac ctcgacgacg aaggtcactt cgtagacgat 240

ctggttactt gccgtagtaa aggcgaatcg agcttgttca gccgcgatca ggttgactac 300

atggacgtat ccacccagca gatcgtttct gttggtgcgt cactgatccc gttcctggag 360

cacgatgacg ccaaccgcgc attgatgggt gcaaacatgc aacgtcaggc ggttccaact 420

ctgcgtgctg ataagccgct ggttggtacc ggtatggaac gcgcggtagc ggttgactcc 480

ggtgttacag ccgtagcaaa a 501

<210> 228
<211> 501
<212> DNA
<213> Erwinia mallotivora

<400> 228
acgcactatg gtcgcgtgtg cccaatcgaa acgcctgaag gtccaaacat cggtctgatt 60

aactccctgt ctgtttatgc gcagaccaac gagtatggtt tcctggaaac cccataccgg 120

cgcgttcgcg atggcgtggt gaccgacgaa attcattacc tctctgcgat tgaagaaggg 180

aattacgtta ttgcgcaggc taacaccaac cttgacgacg aaggccactt tgtcgatgac 240

ctggttacct gccgtagcaa aggcgaatcc agtctgttca gccgcgatca ggttgactac 300

atggacgttt ccacccagca ggtggtttct gtcggtgcgt cactgattcc gttccttgag 360

cacgatgacg ccaaccgcgc attgatgggt gcaaacatgc aacgtcaggc ggttcccact 420

ctgcgtgctg acaagccgct ggttggtacc ggtatggagc gcgcggtagc ggttgactcc 480

ggtgtaaccg cggtggcgaa a 501

<210> 229
<211> 501
<212> DNA
<213> Erwinia papayae

<400> 229
acgcactatg gtcgcgtgtg cccaatcgaa acgcctgaag gtccaaacat cggtctgatt 60

aactccctgt ctgtttatgc gcagaccaac gagtatggtt tcctggaaac cccataccgc 120

cgcgttcgcg atggcgtggt gaccgacgaa attcattacc tctctgcgat tgaagagggg 180

aattacgtta ttgcgcaggc gaacaccaac ctcgacgacg aaggccactt tgtcgatgac 240

ctggttacct gccgtagcaa aggcgaatcc agtctgttca gccgcgatca ggttgactac 300

atggacgttt ccactcagca ggtggtttct gtcggtgcgt cactgattcc gttccttgag 360

cacgatgacg ccaaccgcgc attgatgggt gcaaacatgc aacgtcaggc ggttcccact 420

ctgcgcgctg acaagccgct ggttggtacc ggtatggagc gcgcggtagc ggttgactcc 480

ggtgtaaccg cggtggcgaa a 501

<210> 230
<211> 501
<212> DNA
<213> Erwinia persicina

<400> 230
acgcactacg gtcgcgtatg tccaatcgaa acgccggaag gtccaaacat cggtctgatc 60

aactccttgt ctgtgtatgc acagaccaat gagtacggtt tcctggaaac cccataccgt 120

cgcgttcgcg aaggcgtggt gaccgacgaa attcattacc tctctgctat tgaagagggt 180

aactacgtta tcgctcaggc aaacaccaat ctcgacgacg aaggtcactt cgtagacgac 240

ctggtcacct gccgtagcaa aggcgaatcg agtctcttca accgcgatca agttgactac 300

atggacgttt ccacccagca ggtggtttcc gtcggtgcgt cactgatccc gttcctggag 360

cacgatgacg ccaaccgcgc attgatgggt gcaaacatgc aacgtcaggc ggttcctact 420

ctgcgtgctg ataagccgct ggtaggtacc ggtatggagc gtgcggttgc ggttgactcc 480

ggtgttactg ccgtagcgaa a 501

<210> 231
<211> 501
<212> DNA
<213> Erwinia psidii

<400> 231
acgcactacg gtcgcgtgtg cccaatcgaa acgcctgaag gtccaaacat cggtctgatc 60

aactcgctgt ccgtgtacgc acagaccaac gagtacggtt tcctggaaac cccataccgt 120

cgcgtacgcg aaggcgtggt gaccgacgaa attcattacc tctctgccat tgaagagggt 180

aactacgtta tcgctcaggc gaacaccaac ctcgacgacg aaggtcactt cgtggacgac 240

ctggtaacct gccgtagcaa aggcgaatca agtctgttca gccgcgatca ggttgactac 300

atggacgttt ccacccagca ggtggtttcc gtcggtgcgt cgctgatccc gttcctggaa 360

cacgatgacg ccaaccgcgc attgatgggt gcaaacatgc aacgtcaggc ggttccaact 420

ctgcgtgctg ataagccgtt ggtgggtacc ggtatggaac gcgcggtagc ggttgactcc 480

ggtgtaacag ccgtagcgaa a 501

<210> 232
<211> 501
<212> DNA
<213> Erwinia pyrifoliae

<400> 232
actcactacg gtcgcgtatg tccaatcgaa acgccagaag gtccaaacat cggtctgatc 60

aactccttgt ctgtgtatgc acagaccaac gagtacggtt tcctggaaac cccataccgc 120

cgcgtgcgcg atggcgtggt gaccgacgaa attcattacc tctctgccat tgaagagggt 180

aactacgtta tcgctcaggc aaacaccaac ctcgacgacg aaggccactt cgtagacgac 240

ctggtgacct gccgtagcaa aggcgaatcg agcctgttca gccgcgatca ggttgactac 300

atggacgttt ccacccagca ggtggtttcc gtcggtgcgt cactgatccc gttcctggaa 360

cacgatgacg ccaaccgcgc attgatgggt gcaaacatgc aacgtcaggc ggtacctact 420

ctgcgtgctg ataagccgct ggtaggaacc ggtatggaac gcgcggtagc ggtcgactcc 480

ggcgtcactg ccgtagcgaa a 501

<210> 233
<211> 501

<212>  DNA
<213>  Erwinia rhapontici

<400>  233
actcactacg gtcgcgtatg tccaatcgaa acgccggaag gtccaaacat cggtctgatc      60

aactccttgt ctgtttatgc acagaccaat gagtatggtt tcctggaaac cccataccgt     120

cgcgttcgcg aaggcgtggt gaccgacgaa attcattacc tctctgctat tgaagagggt     180

aactacgtta tcgctcaggc gaacaccaac ctcgacgacg aaggtcactt cgtagacgat     240

ctggtgacct gccgtagcaa aggcgaatcg agcctcttca accgtgacca ggttgactac     300

atggacgttt ccacccagca ggtggtttcc gtcggtgcgt cactgatccc gttcctggag     360

cacgatgacg ccaaccgcgc attgatgggt gcaaacatgc aacgtcaggc ggttccaact     420

ctgcgtgctg ataagccgct ggttggtact ggtatggaac gcgcggtagc ggtcgactcc     480

ggtgttactg ccgtagcgaa a                                               501

<210>  234
<211>  501
<212>  DNA
<213>  Erwinia toletana

<400>  234
actcactacg gtcgtgtgtg tccaatcgaa acgccggaag gtccaaacat cggtctgatc      60

aactccttgt ctgtgtatgc acagaccaat gagtacggtt tcctcgaaac cccttatcgt     120

cgcgtagtcg acggtgtggt caccgacgaa attcattacc tctctgcaat tgaagagggt     180

aactacgtta tcgctcaggc gaacaccaac ctcgacgacg aaggtcactt cgtagacgac     240

ctggtgactt gccgtagcaa aggcgaatcg agtctgttca gccgcgatca ggttgactac     300

atggacgttt ccacacagca gatcgtttct gttggtgcgt ccctgattcc gttcctggag     360

cacgatgacg ccaaccgcgc attgatgggt gcaaacatgc aacgtcaggc ggttcctact     420

ctgcgtgctg ataagccgct ggtaggtacc ggtatggaac gcgcggttgc ggttgactcc     480

ggtgtaactg ccgtagcgaa a                                               501

<210>  235
<211>  501
<212>  DNA
<213>  Erwinia tracheiphila

<400>  235
actcactacg gtcgcgtgtg cccaatcgaa acccctgaag gtccaaacat cggtctgatc      60

aactccctgt ccgtgtacgc acagaccaac gagtacggtt tcctggaaac cccataccgc     120

cgcgttcgcg acggcgtggt gaccgacgaa attcattacc tctctgccat tgaagagggc     180

aactacgtta tcgctcaggc gaacaccaac ctcgacgacg aaggtcactt cgtggacgat     240

ctggtgacct gccgcagcaa aggcgaatca agtctgttta gccgagatca ggttgactac     300

atggacgttt ccacccagca ggtggtttcc gtcggggcaa gcctgatccc gttcctggaa     360

cacgatgacg ccaaccgcgc attgatgggt gcaaacatgc aacgtcaggc ggttccaacg     420

ctgcgtgctg ataagccgct ggttggtacc ggtatggaac gcgcggtagc ggttgactcc 480

ggtgtgaccg ccgtcgcgaa a 501

<210> 236
<211> 501
<212> DNA
<213> Escherichia albertii

<400> 236
actcactacg gtcgcgtatg tcctatcgaa acgcctgaag gtccgaacat cggtctgatc 60

aactctctgt ccgtgtacgc acagactaac gaatacggct tcctcgagac tccgtatcgt 120

aaagtgaccg atggtgtggt aaccgacgaa attcactacc tgtctgctat cgaagaaggc 180

aactacgtta ttgcccaggc gaactccaac ttggatgaag aaggccgctt cgttgaggac 240

ctggtaactt gccgtagcaa aggcgaatcc agcttgttca gccgcgacca ggttgactac 300

atggacgtat ccacccaaca ggtggtatcc gtcggtgcgt ccctgatccc gttccttgaa 360

cacgatgacg ccaaccgtgc attgatgggt gcgaacatgc aacgtcaggc cgttccgact 420

ctgcgtgctg ataagccgct ggttggtacg ggtatggaac gtgctgttgc cgttgactcc 480

ggtgtaactg cggtggctaa a 501

<210> 237
<211> 501
<212> DNA
<213> Escherichia blattae

<400> 237
acccactacg gccgcgtgtg cccaatcgaa acgcctgaag gtccgaacat cggtctgatc 60

aactccctgt ccgtgtacgc acgtactaac gaatacggtt tcctcgaaac cccgtatcgt 120

ctggtgcgcg acggtgtggt gaccgatgag atccattacc tgtctgctat cgaagaaggc 180

aactacgtta tcgcccaggc gaactccaac ctggatgaag aaggccactt cgtagaagac 240

ctggttaccg cccgtaacaa aggcgaatcc agcttgttca gccgcgacca ggttgactac 300

atggacgttt ccacccagca ggtggtttct gtcggtgcgt ccctgatccc gttcctggaa 360

cacgatgacg ccaaccgtgc attgatgggt gcgaacatgc aacgtcaggc agtgccgacc 420

ctgcgctctg ataagccgct ggttggtaca ggtatggagc gcgcggtagc ggttgactcc 480

ggtgttactg cagttgccaa a 501

<210> 238
<211> 501
<212> DNA
<213> Escherichia coli T

<400> 238
actcactacg gtcgcgtatg tccaatcgaa acccctgaag gtccgaacat cggtctgatc 60

aactccctgt ccgtgtacgc acagactaac gaatacggct tccttgagac tccgtatcgt 120

aaagtgaccg acggtgttgt aactgacgaa attcactatc tgtctgctat cgaagaaggc 180

aactacgtta tcgcccaggc gaactccaac ctggatgaag aaggccactt cgtagaagac 240

ctggtaactt gccgtagcaa aggcgaatcc agcttgttca gccgcgacca ggttgactac 300

atggacgtat ccacccagca ggtggtatcc gtcggtgcgt ccctgatccc gttcctggaa 360

cacgatgacg ccaaccgtgc attgatgggt gcgaacatgc aacgtcaggc cgttccgact 420

ctgcgtgctg ataagccgct ggttggtacg ggtatggaac gtgctgttgc cgttgactcc 480

ggtgtaactg cggttgctaa a 501

<210> 239
<211> 501
<212> DNA
<213> Escherichia fergusonii

<400> 239
actcactacg gtcgcgtatg tccaatcgaa acccctgaag gtccgaacat cggtctgatc 60

aactccctgt ccgtgtacgc acagactaac gaatatggct tccttgagac gccgtaccgt 120

aaagtgattg acggtgttgt aaccgacgaa attcactacc tgtctgctat cgaagaaggc 180

aactacgtta tcgcccaggc gaactccaac ctggatgaag aaggccactt tgtagaagac 240

ctggtaactt gccgtagcaa aggcgaatcc agcttgttca gccgcgacca ggttgactac 300

atggacgtat ccacccagca ggtggtatcc gtcggtgcgt ccctgatccc gttccttgaa 360

cacgatgacg ccaaccgtgc attgatgggt gcgaacatgc aacgtcaggc cgttccgact 420

ctgcgtgctg ataagccgct ggttggtacg ggtatggaac gtgctgttgc cgttgactcc 480

ggtgtaactg cggttgctaa a 501

<210> 240
<211> 501
<212> DNA
<213> Escherichia hermannii

<400> 240
acccactacg gtcgtgtatg tcctatcgaa acccctgaag gtccgaacat cggtctgatc 60

aactccctgt ctgtgtatgc gcagactaac gaatatggct tccttgaaac cccgtatcgt 120

cgcgtagttg atggcgtggt gactgacgaa atccattacc tctccgctat cgaagaaggt 180

aactacgtca tcgctcaggc gaacaccaac ctgaccgaag aaggccgttt cgtcgacgat 240

ctggttacct gccgtagcaa aggcgaatcc agcttgttca gcaacgacca ggttgactac 300

atggacgttt ccacccagca ggtggtttcc gtcggtgcgt ccctgatccc gttcctggaa 360

cacgatgacg ccaaccgtgc attgatgggt gcgaacatgc aacgtcaggc ggttccgact 420

ctgcgcgctg ataagccgct ggttggtacc ggtatggaac gtgctgttgc cgttgactcc 480

ggtgtaactg ccgttgccaa a 501

<210> 241
<211> 501
<212> DNA
<213> Escherichia vulneris

<400> 241

acccactacg gtcgcgtatg tcctatcgaa acgcctgaag gtccgaacat cggtctgatt 60
aactccctgt ccgtgtacgc acagactaac gaatatggct tccttgagac gccgtaccgt 120
aaagttaccg acggcgttgt gaccgacgag atccattacc tctctgctat cgaagagggt 180
aactacgtta tcgcccaggc gaacaccaac ctggacgagg aaggtcgctt tgttgatgac 240
ctcgttacct gccgtagcaa aggcgaatcc agcttgttca gcaacgacca ggttgactac 300
atggacgttt ccacccagca ggtggtttcc gtcggtgcgt ccctgatccc gttcctggaa 360
cacgatgacg ccaaccgtgc attgatgggt gcgaacatgc aacgtcaggc ggttccgacc 420
ctgcgcgctg ataagccgct ggtaggtacc ggtatggaac gtgctgttgc cgttgactcc 480
ggtgtaactg cggtggctaa a 501

<210> 242
<211> 501
<212> DNA
<213> Ewingella americana T

<400> 242
actcactacg gtcgcgtatg tccaatcgaa acgccagaag gtccaaacat cggtctgatc 60
aactccttgt ctgtttatgc acagaccaac gagtatggtt tcctggaaac cccttaccgc 120
cgtgtgcgtg aaggcgtggt gactgatgaa attaactatc tgtctgccat tgaagaaggc 180
aacttcgtaa tcgctcaggc gaactccaac ttggatgacg aaggccgctt cctggaagac 240
ctggtcactt gtcgtagcaa aggcgaatca agcctgttca gccgcgacca agttgactac 300
atggacgttt ctacccagca gatcgtatcc gttggtgctt ctctgattcc attcttggaa 360
cacgatgacg ccaaccgtgc attgatgggt gcgaacatgc aacgtcaggc ggttcctact 420
ctgcgtgctg ataagccgct ggttggtacc ggtatggaac gtgctgtagc ggttgactcc 480
ggtgttactg ccgtagccaa a 501

<210> 243
<211> 501
<212> DNA
<213> Hafnia alvei T

<400> 243
actcactacg gtcgtgtatg tccaatcgaa acgcctgaag gtccaaacat cggtctgatc 60
aactccctgt ctgtttatgc gcagactaac gaatacggct tcttggaaac tccataccgc 120
cgcgttcgtg atggaattgt aactgatgag attcattacc tgtctgctat cgaagaaggc 180
aactacgtca tcgctcaggc gaactccaac ttggacgacg aaggccgctt tgtagaagat 240
ctggttactt gccgtagcaa aggcgaatca agtttgttca gccgcgatca ggttgactac 300
atggacgttt ccacccagca ggtggtttct gtcggtgctt ccctgatccc gttcttggaa 360
cacgatgacg ccaaccgtgc attgatgggt gcgaacatgc aacgtcaggc cgttccaact 420
ctgcgtgctg ataagccgct ggttggtacc ggtatggaac gtgctgtagc cgttgactcc 480
ggtgttaccg ctgttgctaa a 501

<210> 244
<211> 501
<212> DNA
<213> Klebsiella oxytoca

<400> 244
actcactacg gtcgcgtatg tccaatcgaa acgcctgaag gtccgaacat cggtctgatc 60

aactccctgt ccgtgtacgc acagacgaac gaatacggct tccttgagac tccgtatcgt 120

aaagtcaccg acggcgttgt gaccgacgaa attcattacc tgtctgctat cgaagaaggt 180

aactacgtta ttgctcaggc gaactccaac ctggatgacg aaggccactt tgtagaagat 240

ctggttacct gccgtagtaa aggcgaatcc agcttgttca gccgcgacca ggttgactac 300

atggacgtat ccacccagca ggtggtttcc gtcggtgcgt ccctgatccc gttcctggaa 360

cacgatgacg ccaaccgtgc attgatgggt gcgaacatgc aacgtcaggc cgttccgact 420

ctgcgcgctg ataagccgct ggttggtacc ggtatggaac gtgctgttgc cgttgactcc 480

ggtgtaactg cggtggctaa a 501

<210> 245
<211> 501
<212> DNA
<213> Klebsiella pneumoniae sbsp. ozaenae

<400> 245
acccactacg gtcgcgtatg tccgatcgaa acgcctgaag gtccgaacat cggtctgatt 60

aactccctgt ccgtgtacgc gcagaccaac gaatatggct tccttgagac gccgtatcgt 120

aaagtgaccg acggtgtggt tactgacgaa attcactacc tgtctgctat cgaagaaggc 180

aactacgtta tcgctcaggc gaactccaac ctggatgaaa acggccactt cgtagaagat 240

ctggttacct gccgtagcaa aggcgaatcc agcttgttca gccgcgacca ggttgactac 300

atggacgtat ccacccagca ggtggtatcc gtcggtgcgt ccctgatccc gttcctggaa 360

cacgatgacg ccaaccgtgc attgatgggt gcgaacatgc aacgtcaggc ggttccgact 420

ctgcgcgctg ataagccgct ggttggtacc ggtatggaac gtgctgttgc cgttgactcc 480

ggtgttactg ccgtggctaa a 501

<210> 246
<211> 501
<212> DNA
<213> Klebsiella pneumoniae

<400> 246
acccactacg gtcgcgtatg tccgatcgaa acgcctgaag gtccgaacat cggtctgatt 60

aactccctgt ccgtgtacgc gcagaccaac gaatatggct tccttgagac gccgtatcgt 120

aaagtgaccg acggtgtggt tactgacgaa attcactacc tgtctgctat cgaagaaggc 180

aactacgtta tcgctcaggc gaactccaac ctggatgaaa acggccactt cgtagaagat 240

ctggttacct gccgtagcaa aggcgaatcc agcttgttca gccgcgacca ggttgactac 300

atggacgtat ccacccagca ggtggtatcc gtcggtgcgt ccctgatccc gttcctggaa 360

cacgatgacg ccaaccgtgc attgatgggt gcgaacatgc aacgtcaggc ggttccgact 420

ctgcgcgctg ataagccgct ggttggtacc ggtatggaac gtgctgttgc cgttgactcc 480

ggtgttactg ccgtggctaa a 501

<210> 247
<211> 501
<212> DNA
<213> Klebsiella pneumoniae sbsp. rhinoscleromatis

<400> 247
acccactacg gtcgcgtatg tccgatcgaa acgcctgaag gtccgaacat cggtctgatt 60

aactccctgt ccgtgtacgc gcagaccaac gaatatggct tccttgagac gccgtatcgt 120

aaagtgacca acggtgtggt tactgacgaa attcactacc tgtctgctat cgaagaaggc 180

aactacgtta tcgctcaggc gaactccaac ctggatgaaa acggccactt cgtagaagat 240

ctggttacct gccgtagcaa aggcgaatcc agcttgttca gccgcgacca ggttgactac 300

atggacgtat ccacccagca ggtggtatcc gtcggtgcgt ccctgatccc gttcctggaa 360

cacgatgacg ccaaccgtgc attgatgggt gcgaacatgc aacgtcaagc ggttccgact 420

ctgcgcgctg ataagccgct ggttggtacc ggtatggaac gtgctgttgc cgttgactcc 480

ggtgttactg ccgtggctaa a 501

<210> 248
<211> 501
<212> DNA
<213> Klebsiella singaporensis

<400> 248
acgcactacg gtcgcgtatg tccaatcgaa acgcctgaag gtccgaacat cggtctgatc 60

aactccctgt ccgtgtacgc acagactaac gaatacggtt tccttgagac cccgtatcgt 120

aaagtgaccg acggtgtggt taccgatgaa attcactacc tgtctgctat cgaagaaggc 180

aactacgtta ttgctcaggc gaactccaac ctggatgaaa acggccactt cgtagaagac 240

ctggttacct gccgtagcaa aggcgaatcc agcttgttca gccgcgacca ggttgactac 300

atggacgtat ccacccagca ggtggtatcc gtcggtgcgt ccctgatccc gttcctggag 360

cacgatgacg ccaaccgtgc attgatgggt gcgaacatgc aacgtcaggc ggttccgact 420

ctgcgcgctg ataagccgct ggttggtacc ggtatggaac gtgctgttgc cgttgactcc 480

ggtgttactg cggtggctaa a 501

<210> 249
<211> 501
<212> DNA
<213> Klebsiella variicola

<400> 249
acgcactacg gtcgcgtatg tccaatcgaa acgcctgaag gtccgaacat cggtctgatc 60

aactccctgt ccgtgtacgc acagactaac gaatacggtt tccttgagac cccgtatcgt 120

aaagtgaccg acggtgtggt taccgatgaa attcactacc tgtctgctat cgaagaaggc 180

aactacgtta ttgctcaggc gaactccaac ctggatgaaa acggccactt cgtagaagac 240

ctggttacct gccgtagcaa aggcgaatcc agcttgttca gccgcgacca ggttgactac 300

atggacgtat ccacccagca ggtggtatcc gtcggtgcgt ccctgatccc gttcctggag 360

cacgatgacg ccaaccgtgc attgatgggt gcgaacatgc aacgtcaggc ggttccgact 420

ctgcgcgctg ataagccgct ggttggtacc ggtatggaac gtgctgttgc cgttgactcc 480

ggtgttactg cggtggctaa a 501

<210> 250
<211> 501
<212> DNA
<213> Kluyvera ascorbata T

<400> 250
actcactacg gtcgcgtatg tccaatcgaa acgcctgaag gtccaaacat cggtctgatc 60

aactccctgt ccgtgtacgc acagactaac gaatacggct tccttgagac tccgtaccgt 120

aaagtgaccg acggtgttgt aaccgacgaa attcattacc tgtctgctat cgaagaaggc 180

aactacgtta tcgctcaggc gaactccaac ctggatgacg aaggccactt tgtagaagac 240

ctggtaactt gccgtagcaa aggcgaatcc agcttgttca gccgcgacca ggttgactac 300

atggacgtat ccacccagca ggtggtatcc gtcggtgcgt ccctgatccc gttcctggaa 360

cacgatgacg ccaaccgtgc attgatgggt gcgaacatgc aacgtcaggc cgttccaact 420

ctgcgtgctg ataagccgct ggttggtacc ggtatggaac gtgctgttgc cgttgactcc 480

ggtgttactg cagttgctaa g 501

<210> 251
<211> 501
<212> DNA
<213> Kluyvera cochleae

<400> 251
actcactacg gtcgcgtatg tccaatcgaa acgcctgaag gtccaaacat cggtctgatc 60

aactccctgt ccgtgtacgc acagactaac gaatacggct tcctcgagac tccgtaccgt 120

aaagtgactg acggtgttgt taccgacgaa attcattacc tgtctgctat cgaagaaggc 180

aactacgtta tcgctcaggc gaactccaac ctggatgacg aaggccactt tgaagaagac 240

ctggtaacgt gccgtagcaa aggcgaatcc agcttgttca gccgtgacca ggttgactac 300

atggacgtat ccacccaaca ggtggtatcc gtcggtgcgt ccctgatccc gttccttgaa 360

cacgatgacg ccaaccgtgc attgatgggt gcgaacatgc aacgtcaggc cgttccaact 420

ctgcgtgctg ataagccgct ggttggtacc ggtatggaac gtgctgttgc cgtcgactcc 480

ggtgttactg cagtggctaa g 501

<210> 252
<211> 501
<212> DNA

<213>  Kluyvera cryocrescens

<400>  252
actcactacg gtcgcgtatg tccaatcgaa acgcctgaag gtccaaacat cggtctgatc      60

aactccttgt ccgtgtacgc acagactaac gaatacggct tcctcgagac tccgtatcgt     120

aaagtgactg acggtgtggt aaccgacgag attcattacc tgtccgctat cgaagaaggt     180

aactacgtca tcgctcaggc gaactccaac ctggatgacg aaggccactt tgtagaagac     240

ctggtaactt gccgtagcaa aggcgaatcc agcttgttca gccgcgacca ggttgactac     300

atggacgtat ccacccagca ggtggtttcc gtcggtgcgt ccctgatccc gttcctggaa     360

cacgatgacg ccaaccgtgc attgatgggt gcaaacatgc aacgtcaggc cgttccaact     420

ctgcgtgctg ataagccgct ggttggtacc ggtatggaac gtgctgtagc cgttgactcc     480

ggtgttaccg ctgttgctaa g                                               501

<210>  253
<211>  501
<212>  DNA
<213>  Kluyvera georgiana

<400>  253
actcactacg gtcgcgtatg tccaatcgaa acgcctgaag gtccgaacat cggtctgatc      60

aactccctgt ccgtgtacgc acagactaac gaatacggct tccttgagac tccgtaccgt     120

aaagtgaccg acggtgttgt aaccgacgaa attcattacc tgtctgctat cgaagaaggc     180

aactacgtta tcgctcaggc gaactccaac ctggatgacg aaggccactt tgtagaagat     240

ctggttacct gccgtagcaa aggcgaatcc agcttgttca gccgcgacca ggttgactac     300

atggacgtat ccacccagca ggtggtatcc gtcggtgcgt ccctgatccc gttcctggaa     360

cacgatgacg ccaaccgtgc attgatgggt gcgaacatgc aacgtcaggc ggttccgact     420

ctgcgcgctg ataagccgct ggttggtacc ggtatggaac gtgctgtagc cgtcgactcc     480

ggtgttactg cagttgctaa g                                               501

<210>  254
<211>  501
<212>  DNA
<213>  Kluyvera intermedia

<400>  254
actcactacg gtcgcgtatg tccaatcgaa acgcctgaag gtccaaacat cggtctgatc      60

aactccctgt ccgtgtacgc acagactaac gaatacggct tcctcgagac tccgtaccgt     120

aaagtgactg acggtgttgt taccgacgaa attcattacc tgtctgctat cgaagaaggc     180

aactacgtta tcgctcaggc gaactccaac ctggatgacg aaggccactt tgaagaagac     240

ctggtaacgt gccgtagcaa aggcgaatcc agcttgttca gccgcgacca ggttgactac     300

atggacgtat ccacccaaca ggtggtatcc gtcggtgcgt ccctgatccc gttccttgaa     360

cacgatgacg ccaaccgtgc attgatgggt gcgaacatgc aacgtcaggc cgttccaact     420

ctgcgtgctg ataagccgct ggttggtacc ggtatggaac gtgctgttgc cgtcgactcc     480

ggtgttactg cagtggctaa g 501

<210> 255
<211> 501
<212> DNA
<213> Leclercia adecarboxylata T

<400> 255
actcactacg gtcgcgtatg tccaatcgaa acgcctgaag gtccaaacat cggtctgatc 60

aactccctgt ccgtgtacgc acagactaac gaatacggtt tccttgagac cccgtatcgt 120

aaagtgactg acggtgttgt aaccgacgaa attcattacc tgtctgctat cgaagaaggt 180

aactacgtta tcgctcaggc gaactccaac ctggatgacg aaggccactt tgtagaagat 240

ctggttacct gccgtagcaa aggtgaatcg agcttgttca gtcgtgacca ggttgactac 300

atggacgttt ccacccagca ggtggtttcc gtcggtgcgt ccctgatccc gttcctggaa 360

cacgatgacg ccaaccgtgc attgatgggt gcgaacatgc aacgtcaggc ggttccaact 420

ctgcgcgctg ataagccgct ggttggtacc ggtatggaac gtgctgttgc cgttgactcc 480

ggtgttactg cagttgctaa a 501

<210> 256
<211> 501
<212> DNA
<213> Leminorella grimontii T

<400> 256
acccactacg gccgcgtgtg tcctatcgaa acgcctgaag gtccgaacat cggtctgatt 60

aactctctgt ccgtgtacgc acagaccaac gagtacggct tcctcgagac gccttaccgt 120

aaggtgactg acggcgttgt gaccgacgaa attcattacc tgtctgccat tgaagaaggc 180

aacttcgtta tcgctcaggc gaactccaac ctggatgacg aaggccactt cattgaagac 240

ctggttacct gccgtaacaa gggcgaatcc agcctgttca gccgcgatca ggttgactat 300

atggacgttt cgacccagca ggtagtttcc gtcggcgcct cgctgattcc gttccttgag 360

cacgacgatg ctaaccgcgc cctgatgggt gcgaacatgc aacgtcaggc ggttccgacc 420

ctgcgcgccg acaagccgct ggtaggtacc ggtatggagc gcgccgttgc cgttgactcg 480

ggcgttactt ctgtcgcaac g 501

<210> 257
<211> 501
<212> DNA
<213> Leminorella richardii

<400> 257
actcactacg gtcgcgtatg tcctatcgaa acgcctgaag gtccaaacat cggtctgatc 60

aactccctgt ccgtgtatgc acagactaac gaatacggct tccttgagac tccgtaccgt 120

aaagtgactg acggcgttgt caccgacgag atccactacc tgtctgccat tgaagaaggc 180

aacttcgtta tcgctcaggc gaactccaac ctgaccgacg aaggccgctt catcgaagat 240

ctggtcacct gtcgtaacaa gggcgagtcc agcctgttca gccgcgatca ggttgactat 300

atggacgtat caacccagca ggtggtttcc gttggtgctt ctctgattcc attccttgag 360

cacgacgatg ctaaccgcgc cctgatgggt gcgaacatgc aacgtcaggc ggttccgact 420

ctgcgcgctg acaagccgct ggtaggtaca ggtatggaac gcgccgttgc cgttgactcc 480

ggtgtaacct ctgttgcaac c 501

<210> 258
<211> 501
<212> DNA
<213> Moellerella wisconsensis T

<400> 258
actcactatg gtcgtgtgtg tccaatcgaa acgccagaag gtccaaacat cggtctgatt 60

aactcactgt ctgtgtacgc acaaactaac gaatatggtt tcctagaaac tccatatcgt 120

ttagtacgcg atggtattgt gactgatgag attcattacc tgtctgcgat tgaagaaggt 180

aacttcatca ttgcacaggc gaataccgta ttagatgacg aaggtcattt tgttgacgaa 240

ctggttactt gtcgtaaccg tggtgagtca agcttattca gccgtgaaca ggttgaatat 300

atggacgttt ccacacaaca ggttgtttct gttggtgcat ctttaatccc attccttgaa 360

cacgatgatg ctaaccgtgc attgatgggt gcgaacatgc aacgtcaggc tgttcctaca 420

ttacgtgctg ataagccgct agtgggtaca ggtatggaac gtgcagtagc tgttgactcc 480

ggtgtaaccg ctgtcgctaa a 501

<210> 259
<211> 501
<212> DNA
<213> Morganella morganii sbsp. morganii T

<400> 259
actcactacg gtcgcgtatg tcctatcgag acccctgaag gtccgaacat cggtctgatt 60

aactccttat ctgtttatgc acagactaac gaatatggtt tcctggaaac cccgtaccgt 120

ttagtgcgtg acgatgttgt taccgatgaa attcattacc tgtctgcaat cgaagaaggt 180

aacttcatca ttgcacaggc aaacaccgta ttagatgacg acggccactt cgttgatgaa 240

ctgatcacct gccgtaacaa aggtgaatcc agcttattca gccgtgatca ggttcagtac 300

atggacgttt ccacccagca ggtggtttct gtcggtgcat ccctgatccc gttcctggaa 360

cacgatgacg ccaaccgtgc attgatgggt gcgaacatgc aacgtcaggc ggttccgact 420

ctgcgcgctg acaagccact ggttggtacc ggtatggaac gtgcggtagc agttgactcc 480

ggtgttacag ccgtagccaa a 501

<210> 260
<211> 501
<212> DNA
<213> Morganella morganii sbsp. sibonii

<400> 260
actcactacg gccgcgtatg tcctatcgag acccctgaag gtccgaacat cggtctgatc 60

aactccttat ctgtttatgc acagactaac gaatatggtt tcctggaaac cccgtaccgt 120

ttagtgcgtg acgatgttgt taccgatgaa attcattacc tgtctgcaat cgaagaaggc 180

aacttcatca ttgcacaggc aaacaccgta ttagatgacg atggccactt cgttgatgaa 240

ctgatcacct gccgtaacaa aggtgaatcc agcttattca gccgtgatca ggttcagtac 300

atggacgttt ccacccagca ggtggtttct gtcggtgcat ccctgatccc gttcctggaa 360

cacgatgacg ccaaccgtgc attgatgggt gcgaacatgc aacgtcaggc cgttccgact 420

ctgcgcgctg ataagccgct ggttggtacc ggtatggaac gtgcggtagc ggttgactcc 480

ggtgttacag ccgtagccaa a 501

<210> 261
<211> 501
<212> DNA
<213> Obesumbacterium proteus T

<400> 261
actcactacg gtcgtgtatg tccaatcgaa acgcctgaag gtccaaacat cggtctgatc 60

aactccctgt ctgtttatgc acagactaac gaatacggct tcttggaaac tccataccgc 120

cgcgttcgtg atggaattgt aactgatgag attcattacc tgtctgctat cgaagaaggc 180

aactacgtca tcgctcaggc gaactccaac ttggacgacg aaggccgctt tgtagaagat 240

ctggttactt gccgtagcaa aggcgaatca agcttgttca gtcgcgatca ggttgactac 300

atggacgttt ccacccagca ggtggtttct gtcggtgctt ccctgatccc gttcttggaa 360

cacgatgacg ccaaccgtgc attgatgggt gcgaacatgc aacgtcaggc cgttccaact 420

ctgcgtgctg ataagccgct ggttggtacc ggtatggaac gtgctgtagc cgttgactcc 480

ggtgttaccg ctgttgctaa a 501

<210> 262
<211> 501
<212> DNA
<213> Pantoea agglomerans T

<400> 262
acgcactacg gtcgtgtctg cccaatcgaa acccctgaag gcccgaacat cggtctgatc 60

aactccttgt ctgtgtatgc acagaccaac gagtacggtt tccttgagac gccttaccgc 120

cgtgttatcg acggtctggt ttctgacgag attcactacc tctctgctat cgaagagggt 180

aactacgtta tcgctcaggc gaacgcccct cttgatgata acggcgcgtt tgttgatgac 240

ctggtcacct gccgtagcaa aggcgaatca agtctgttca gccgcgatca ggttgactac 300

atggacgttt cgactcaaca gatcgtttct gtcggtgcgt cactgatccc gttcctggaa 360

cacgatgatg ctaaccgcgc cctgatgggt gcgaacatgc aacgtcaggc cgttccgact 420

ctgcgtgctg ataagccgct ggttggtacg ggtatggaac gtgctgttgc cgttgactcc 480

ggtgtaactg ccgttgccaa a 501

<210> 263
<211> 501
<212> DNA
<213> Pantoea ananatis

<400> 263
acgcactacg gtcgtgtctg cccaatcgaa acccctgaag gtccgaacat cggtctgatc 60

aactccttgt ctgtgtacgc gcagacgaac gagtacggtt tcctggaaac cccttatcgt 120

cgcgttattg acggtgtggt ttccgatgaa attcattacc tttctgcaat cgaagaaggt 180

aactacgtta tcgcgcaggc gaacaccaat ctgtcagaag aaggtgcgtt cgttgatgac 240

ctggtcacct gccgtagcaa aggcgaatca agtctgttca gccgcgatca ggttgactac 300

atggacgttt ccacccagca ggtggtttct gtcggtgcgt cactgattcc gttcctggaa 360

cacgatgatg ctaaccgcgc cctgatgggt gcgaacatgc aacgtcaggc cgttcctact 420

ctgcgtgctg ataagccgct ggtaggtacc ggtatggagc gcgctgtagc cgttgactcc 480

ggtgttaccg ctgttgcgaa a 501

<210> 264
<211> 501
<212> DNA
<213> Pantoea citrea

<400> 264
actcactatg gtcgtgtttg tcctatcgaa actccggaag gtccaaacat cggtctgatt 60

aactcattgt ctgtttatgc tcagactaac gagtatggtt tccttgaaac cccatatcgc 120

cgtgtgattg acagccaggt aactgacgaa attcattatc tgtcagcgat tgaagaaggt 180

aacttcgtta tcgctcaggc aaacaccaac ctcgacgata atggtctgtt tgttgatgat 240

ctggtgacct gccgtaacaa aggcgagtct agcctgttca gccgtgatca ggttgactac 300

atggacgttt ctactcaaca ggttgtttct gtcggtgcgt ctctgattcc attcctggaa 360

cacgatgatg ctaaccgtgc tctgatgggt gcgaacatgc aacgtcaggc tgtaccaact 420

ctgcgtgctg ataagccgct ggtaggtacc ggtatggaac gtgctgtagc ggttgactcc 480

ggtgttactg cagtagctaa a 501

<210> 265
<211> 501
<212> DNA
<213> Pantoea dispersa

<400> 265
actcactacg gtcgcgtatg tccaatcgaa accccggaag gtccgaacat cggtctgatc 60

aactcactct ctgtttacgc gcagactaac gaatacggct tcctcgaaac gccgtaccgc 120

cgcgtggttg acggtaaagt gagtgatgaa attcattacc tctctgcaat cgaagagggt 180

aactacgtta tcgctcaggc gaacaccaac ctcgccgatg acggcagctt tgttgacgat 240

ctcgtcacct gccgtagcaa aggtgaatcg agcctgttca gccgcgacca ggttgactac 300

atggacgttt ccacccagca ggtggtttct gtcggtgcgt ccctgattcc attcctggag 360

cacgatgatg ctaaccgcgc cctgatgggt gcgaacatgc aacgtcaggc tgtacctacc 420

ctgcgtgctg acaagccgct ggttggtacc ggtatggaac gtgctgttgc ggtcgactcc 480

ggtgtaactg ccgtcgcgaa a 501

<210> 266
<211> 501
<212> DNA
<213> Pantoea punctata

<400> 266
acgcactacg gtcgtgtatg tcctatcgaa acgccggaag gtccgaacat cggtctgatc 60

aactctttat ctgtttatgc acagaccaat gagtacggtt tcctggaaac cccgtatcgc 120

cgtgttattg acagccaggt gactgacgaa attcattacc tgtccgcgat tgaagaaggt 180

aacttcgtta tcgcgcaggc gaacaccaac ctcgatgatg acggtcagtt tgttgatgac 240

ctggtcactt gtcgtaacaa aggtgagtcc agcctgttca gccgtgatca ggttgactat 300

atggacgttt ctacccagca ggttgtttct gtcggtgcgt ctctgattcc attcctggaa 360

cacgatgatg ctaaccgtgc actgatgggt gcaaacatgc aacgtcaggc ggttcctact 420

ctgcgtgctg ataagccgct ggttggtacc ggtatggaac gtgcggtagc ggtagactcc 480

ggtgttactg cggttgcgaa a 501

<210> 267
<211> 501
<212> DNA
<213> Pantoea stewartii sbsp. indologenes

<400> 267
acgcactacg gtcgtgtctg cccaatcgaa acccctgaag gtccgaacat cggtctgatc 60

aactccttgt ctgtatatgc gcagacgaac gagtacggtt tcctggaaac cccttatcgt 120

cgcgtgatcg acggtgtggt ttctgatgaa attcattacc tgtctgccat cgaagaaggt 180

aactatgtca tcgctcaggc gaacaccaat ctgtcagaag aaggtgcgtt cgttgatgac 240

ctggtgacct gccgtagcaa aggtgaatcg agtctgttca gccgcgatca ggttgactac 300

atggacgttt cgacccaaca ggttgtttcc gtcggtgcat cactgatccc gttcctggaa 360

cacgatgatg ctaaccgcgc cctgatgggt gcgaacatgc aacgtcaggc cgttcctaca 420

ctgcgtgccg ataagccgct ggtagggacc ggtatggagc gcgcggttgc cgttgactcc 480

ggtgttaccg ctgtggcgaa a 501

<210> 268
<211> 501
<212> DNA
<213> Pantoea stewartii sbsp. stewartii

<400> 268
acgcactacg gtcgtgtctg cccaatcgaa acccctgaag gtccgaacat cggtctgatc 60

aactccttgt ctgtatatgc gcagacgaac gagtacggtt tcctggaaac cccttatcgt 120

cgcttgatcg acggtgtggt ttccgatgaa attcattacc tgtctgccat cgaagaaggt 180

aactatgtca tcgctcaggc gaataccaac ctgtcagaag aaggtgcgtt cgttgatgac 240

ctggtgacct gccgtagcaa aggtgaatcg agtctgttca gccgcgatca ggttgactac 300

atggacgttt cgacccaaca ggttgtttcc gtcggtgcat cactgatccc gttcctggaa 360

cacgatgatg ctaaccgcgc cctgatgggt gcgaacatgc aacgtcaggc cgttcctaca 420

ctgcgtgccg ataagccgct ggtagggact ggtatggagc gcgcggttgc cgttgactcc 480

ggtgttaccg ctgtggcgaa a 501

<210> 269
<211> 501
<212> DNA
<213> Pantoea terrea

<400> 269
actcactacg gtcgtgtttg tcctatcgaa acgcctgaag gtccgaacat cggtcttatc 60

aactcactgt ctgtgtatgc acagaccaac gaatatggct tcctcgaaac gccataccgt 120

cgtgtggttg acagccaggt aactgacgaa atccattacc tgtctgcgat tgaagaaggc 180

aactttgtta tcgctcaggc gaacaccaac ctcgacgacg atggtcgttt cgtggatgac 240

ctggtgacct gtcgtaacaa gggtgaatcg agcctgttca gccgtgacca ggttgactac 300

atggacgttt cgacccaaca ggttgtttct gtcggtgcgt cactgatccc attcctggaa 360

cacgatgatg ctaaccgtgc attgatgggt gcaaacatgc aacgtcaggc ggttcctact 420

ctgcgtgctg ataagccgtt agtaggtacc ggtatggaac gcgcggtcgc agtcgactcc 480

ggtgtaactg cagtggctaa g 501

<210> 270
<211> 501
<212> DNA
<213> Pectobacterium atrosepticum

<400> 270
actcactatg gtcgtgtttg tcctatcgaa acgccggaag gtccgaacat cggtctgatc 60

aactccctgt ccgtttatgc gcaaactaac gaatacggtt tccttgaaac cccatatcgt 120

cgcgtgcgtg aaaacgtggt tacggatgag attcattacc tgtctgcgat tgaggaaggt 180

aacttcgtta tcgcacaggc aaacaccaat ttagacgaag aaggccgctt catcgacgaa 240

ctggttacct gccgtaacaa aggggagtcc agcttgttca gccgtgatca ggttgaatat 300

atggacgttt ccacacagca gatagtttcc gtcggtgcgt ccctgattcc gttcctggaa 360

cacgatgacg ccaaccgtgc cctgatgggt gcgaacatgc aacgtcaagc cgttccgacc 420

ttacgcgctg ataagccgct ggttggtaca ggtatggaac gcgctgttgc ggttgactcc 480

ggtgtaactg ccgtagccaa a 501

<210> 271
<211> 501
<212> DNA
<213> Pectobacterium betavasculorum

<400>  271
actcactatg gtcgtgtttg tcctatcgaa acgccggaag gtccgaacat cggtctgatc      60

aactccttgt ccgtttatgc gcaaacgaac gaatacggtt tccttgaaac cccgtatcgt     120

cgcgtgcgtg aaaacgtggt gacggatgag atccattacc tgtctgcgat tgaagaaggt     180

aacttcgtta tcgcacaggc aaacaccaat ttggacgaag aaggccgttt catcgacgaa     240

ctggttacct gccgtaacaa aggtgagtcc agcttgttca gccgcgatca ggttgaatat     300

atggacgttt ccacacagca ggtggtttct gtcggtgcat ctctgattcc gttcctggaa     360

cacgatgacg ccaaccgtgc cctgatgggt gcgaacatgc aacgtcaggc tgttccgacc     420

ttacgtgctg ataagccgct agttggtacg ggtatggaac gcgctgttgc ggttgactcc     480

ggtgtaactg ccgtagccaa a                                               501

<210>  272
<211>  501
<212>  DNA
<213>  Pectobacterium cacticida

<400>  272
acgcactacg gtcgtgtttg tcctatcgag acgccggaag gtccgaacat cggtctgatc      60

aactccctgt ctgtttatgc acaaacgaac gagtatggtt tccttgaaac cccatatcgt     120

cgtgtgcgtg acaacgtggt gacggatgag atccattatc tgtctgcaat cgaagaaggt     180

aacttcgtga ttgcgcaggc gaacaccaat ttggacgaag aaggccgttt catcgacgaa     240

ctggttacct gccgtaacaa gggcgagtcc agcttgttca gccgcgatca ggttgaatac     300

atggacgttt ccacacagca ggtcgtttcc gtcggtgcgt ctctgattcc gttcctggag     360

cacgatgatg ccaaccgcgc cttgatgggt gcgaacatgc aacgtcaggc cgtccctacc     420

ctgcgcgctg ataagccgct ggtgggaacg ggtatggaac gcgctgttgc ggttgactcc     480

ggtgttactg ccgtagccaa a                                               501

<210>  273
<211>  501
<212>  DNA
<213>  Pectobacterium carotovorum sbsp. carotovorum T

<400>  273
actcactacg gtcgcgtttg tcctatcgaa acgccggaag gtccgaacat cggtctgatc      60

aactccctgt ccgtttatgc gcaaacgaac gaatacggtt tccttgaaac cccgtatcgt     120

cgcgtgcgtg aaaatgtggt gacggatgag atccattacc tgtctgcgat tgaagaaggt     180

aacttcgtta tcgcacaggc gaacaccaat ttggacgaag aaggccgctt catcgacgaa     240

ctggttacct gccgtaacaa aggcgagtcc agcttgttca gccgcgatca ggttgaatac     300

atggacgttt ccacacagca ggtggtttcc gtcggtgcat ccctgattcc gttcctggaa     360

cacgatgacg ccaaccgtgc cctgatgggt gcgaacatgc aacgtcaggc cgttccgact     420

ctgcgcgctg ataagccgct ggttggtacc ggtatggaac gcgctgttgc ggttgactcc     480

ggtgtaactg ccgtagccaa a 501

<210> 274
<211> 501
<212> DNA
<213> Pectobacterium carotovorum sbsp. odoriferum

<400> 274
actcactacg gtcgcgtttg tcctatcgaa acgccggaag gtccgaacat cggtctgatc 60

aactccctgt ccgtttatgc gcaaacgaac gaatacggtt tccttgaaac cccgtatcgt 120

cgcgtgcgtg aaaatgtggt gacggatgag atccattacc tgtctgcgat tgaagaaggt 180

aacttcgtta tcgcacaggc gaacaccaat ttggacgaag aaggccgctt catcgacgaa 240

ctggttacct gccgtaacaa aggcgagtcc agcttgttca gccgcgatca ggttgaatac 300

atggacgttt caacacagca ggtggtttcc gtcggtgcat ccctgattcc gttcctggaa 360

cacgatgacg ccaaccgtgc cctgatgggt gcgaacatgc aacgtcaggc cgttccgacc 420

ctgcgtgctg ataagccgct ggttggtacc ggtatggaac gtgctgttgc ggttgactcc 480

ggtgtaactg ccgtagccaa a 501

<210> 275
<211> 501
<212> DNA
<213> Pectobacterium cypripedii

<400> 275
actcactacg gtcgcgtatg tccaatcgaa acgccggaag gtccgaacat cggtctgatc 60

aactcgctgt ctgtgtatgc acagaccaac gagtatggct tcctggaaac gccgtatcgt 120

cgcgtagttg acggtaaagt gagtgacgaa atccattacc tctctgcaat tgaagagggt 180

aacttcgtta tcgctcaggc gaacaccaac ctggccgacg acggttcatt cgttgacgat 240

ctggttacct gtcgtagcaa aggcgaatca agcctgttca gccgcgacca ggttgactat 300

atggacgttt cgacccagca ggttgtttct gttggtgcgt cactgatccc gttcctggaa 360

cacgatgacg ccaaccgtgc attgatgggt gcaaacatgc aacgtcaggc ggttccgact 420

ctgcgtgctg ataagccgct ggttggtacc ggtatggaac gtgctgttgc tgttgactcc 480

ggtgttaccg ccgtagccaa a 501

<210> 276
<211> 501
<212> DNA
<213> Pectobacterium wasabiae

<400> 276
actcactacg gtcgtgtttg tcctatcgaa acgccggaag gtccgaacat cggtctgatc 60

aactccttgt ccgtttatgc gcaaactaac gaatacggtt tccttgaaac cccgtatcgt 120

cgcgtgcgtg aaaacgtggt tacggatgag atccattacc tgtctgcgat tgaagaaggt 180

aacttcgtta tcgcacaggc aaacaccaat ttggacgaag aaggccgctt catcgacgaa 240

ctggttacct gtcgtaataa aggtgagtcc agcttgttca gccgcgatca ggttgaatac 300

atggacgttt ccacacagca ggtggtttcc gtcggtgcat ccctgattcc gttcctggaa 360
cacgatgacg ccaaccgtgc cctgatgggt gcgaacatgc aacgtcaggc cgttccgacc 420
ttacgcgctg ataagccgct ggttggtacg ggtatggaac gtgctgttgc ggttgactct 480
ggtgtaactg ccgtagccaa a 501

<210> 277
<211> 501
<212> DNA
<213> Photorhabdus asymbiotica sbsp. asymbiotica

<400> 277
actcactatg gccgtgtatg tccaatcgaa accccggaag gtccgaacat cggcctgatt 60
aactcattgt cggtttatgc acagactaac gagtatggtt tccttgaaac cccataccgt 120
ttggttcgtg acggcgttgt aactgatgaa attcattatc tgtctgcgat tgaagaaggt 180
aacttcgtta tcgcgcaggc aaacaccgta ttagatgatg aaggccactt tgttgaagat 240
ctgataactt gccgtaactt tggtgaatct agcttattca accgtgaaca agttgaatat 300
atggacgttt ctacccagca agtggtttcg gttggtgctt ccttgatccc attcctagaa 360
catgatgacg caaaccgtgc attgatgggt gcaaacatgc aacgtcaggc tgttcctact 420
ctgcgtgctg ataagccgtt ggttggtaca ggcatggaac gggcggtagc ggttgactca 480
ggggtaactt ctgtagctaa a 501

<210> 278
<211> 501
<212> DNA
<213> Photorhabdus asymbiotica sbsp. australis

<400> 278
acgcactatg gccgtgtatg tccaatcgaa accccggaag gtccgaacat cggtctgatt 60
aactcattgt cggtttatgc gcagactaat gagtatggtt tccttgaaac cccatatcgt 120
ctagttcgtg atggtgttgt cactgatgaa attcattatc tgtctgcgat tgaagaaggt 180
aacttcgtta tcgcacaggc aaacaccgta ttggatgatg aaggccactt tgttgaagat 240
ctgataacct gccgtaactt tggtgaatcc agcttattca accgtgaaca agttgaatat 300
atggacgttt ctacccagca ggtggtttcc gttggtgctt ccttgatccc attcttggaa 360
cacgatgacg caaaccgtgc attgatgggt gcaaacatgc aacgtcaagc tgttcctacg 420
ctgcgtgctg ataagccgct ggtcggtaca ggcatggagc gcgcagtagc ggttgactca 480
ggggtaactt ctgtagctaa a 501

<210> 279
<211> 501
<212> DNA
<213> Photorhabdus luminescens sbsp. kayaii

<400> 279
actcactatg gccgtgtatg tccaatcgaa acccctgaag gtccgaacat cggtctgatt 60

aactcattgt ctgtttatgc gcagactaac gagtatggtt tccttgaaac cccgtatcgt 120

ctggttcgtg atggtgttgt tactgatgaa attcactatc tgtctgcgat tgaagaaggt 180

aacttcgtta tcgcacaggc gaacaccgta ttagatgatg aaggtcactt cgttgaagat 240

ttgataactt gtcgtagtta tggggaatcc agcttattta accgtgagca ggttgaatat 300

atggacgttt ccactcagca ggtggtttcc gttggtgctt ccttgatccc attcttggaa 360

cacgatgacg caaaccgtgc attgatgggt gcaaacatgc aacgtcaggc tgtacctact 420

ctgcgtgctg ataagccgtt agttggtaca ggtatggaac gtgcagtagc ggttgactca 480

ggtgtaactt ctgttgctaa g 501

<210> 280
<211> 501
<212> DNA
<213> Photorhabdus luminescens sbsp. laumondii

<400> 280
acgcactatg gtcgcgtatg tccaatcgaa accccggaag gtccgaacat cggtctgatt 60

aactcattgt ctgtttatgc gcagactaac gagtatggtt tccttgaaac cccatatcgt 120

ctggttcgtg atggtgttgt tactgatgaa attcactatc tgtctgcgat tgaagaaggt 180

aacttcgtta tcgcacaggc aaacaccgta ttagatgacg aaggccactt cgttgaagat 240

ctgataactt gccgtaatta tggtgaatcc agcttattta accgtgagca ggttgaatat 300

atggacgttt ccacccagca ggtggtttcc gttggtgctt ccttgatccc attcttggaa 360

cacgatgacg caaaccgcgc attgatgggt gcaaacatgc aacgtcaggc tgttcctact 420

ctgcgtgctg ataagccgtt ggtcggaaca ggtatggaac gtgcagtagc cgttgactca 480

ggtgtaactt ctgttgctaa a 501

<210> 281
<211> 501
<212> DNA
<213> Photorhabdus luminescens sbsp. luminescens T

<400> 281
acccactatg gtcgtgtatg tccaatcgaa accccggaag gtccgaacat cggtctgatt 60

aactcattgt ctgtttatgc gcagactaac gagtatggtt tccttgaaac cccatatcgt 120

ctggttcgtg atggtgttgt tactgatgaa attcattatc tgtctgcgat tgaagaaggt 180

aacttcgtta tcgcgcaggc aaacaccgta ttagatgatg aaggccactt cgttgaagat 240

ctgataactt gccgtagcta tggtgaatcc agcttattta accgtgagca ggttgaatac 300

atggacgttt ccacccagca ggtggtttcc gttggtgctt ccttgatccc attcctggaa 360

cacgatgacg caaaccgtgc attgatgggc gcaaacatgc aacgtcaggc tgttcctact 420

ctgcgtgctg ataagccgtt ggtcggtacc ggcatggaac gtgcggtagc ggttgactca 480

ggtgtaactt ctgttgctaa a 501

<210> 282

<211>  501
<212>  DNA
<213>  Photorhabdus temperata sbsp. temperata

<400>  282
actcactatg gtcgtgtatg tccaattgaa accccggaag gtccgaacat cggtctgatt      60

aactcattgt ctgtttatgc gcagactaac gagtatggtt tccttgaaac cccataccgt     120

ctggttcgtg atggtgttgt cactgatgaa attcattact tgtctgcgat tgaagaaggt     180

aacttcgtta tcgcacaggc aaacaccgta ttggacgatg aaggtcactt cgttgaagat     240

ctgatcacct gccgtaacta tggcgaatcc agcttattca accgtgaaca ggttgaatat     300

atggacgttt ccactcaaca ggtggtttcc gtcggtgctt ccttgatccc attcctggaa     360

cacgatgacg caaaccgtgc attgatgggt gcaaacatgc aacgtcaggc tgttcctaca     420

ctgcgcgctg acaagccgct ggtagggaca ggtatggaac gtgctgtagc ggttgactca     480

ggtgtaactt ccgttgctaa a                                               501

<210>  283
<211>  501
<212>  DNA
<213>  Plesiomonas shigelloides T

<400>  283
actcactacg gtcgtgtgtg tccaatcgaa actcctgaag gtccaaacat cggtctgatc      60

aactccttgt ctgtgtatgc acagaccaac gagtatggtt tcctggaaac cccgtaccgt     120

cgcgtgaaag acggcgtggt gaccgacgag attcattacc tgtctgctat cgaagaaggt     180

aactacgtta ttgcccaggc gaactccaac ctggacgaag acggccgctt cgaagaagaa     240

ctggtaacgt gccgtagcaa aggcgaatcc agcctgttca gcaaagatca ggtggactac     300

atggacgttt cgacccaaca ggtcgtttct gtcggtgcgt ctttgatccc attcctggag     360

cacgatgatg ctaaccgcgc cctgatgggt gcgaacatgc aacgtcaggc tgtacctact     420

ctgcgtgctg ataagccgct ggttggtacc ggtatggagc gcgctgtagc ggttgactcc     480

ggtgtaaccg tagttgctaa g                                               501

<210>  284
<211>  501
<212>  DNA
<213>  Pragia fontium T

<400>  284
actcactatg gtcgcgtatg tccaatcgaa accccggaag gtccaaacat cggtttgatt      60

aactcattat ctgtgtatgc acagactaac gagtatggtt tcctggaaac cccttatcgt     120

cgcgtacgtg acggtatggt aaccgatgag attcattact tatctgccat tgaagaaggc     180

aacttcgtta tcgctcaggc taactcaaac ctggatgacg aaggccactt catggaagac     240

ctggtgacct gtcgtaacaa aggcgaatca agcttattca gccgcgaaca agttgactat     300

atggacgttt caacccaaca ggttgtgtcc gtaggtgctt ctttgattcc gttcctggag     360

catgatgacg ccaaccgtgc attgatgggt gcaaacatgc aacgtcaggc ggttcctact     420

ctgcgtgctg ataagccgct ggttggtacc ggtatggaac gtgctgtagc ggttgactcc 480

ggcgtaaccg ccgttgccac a 501

<210> 285
<211> 501
<212> DNA
<213> Proteus hauseri

<400> 285
acgcactatg gtcgtgtgtg tcctatcgaa acgcctgaag gtccaaacat cggtcttatt 60

aactcattat ctgtttatgc acagaccaat gagtacggat tccttgaaac gccttaccgt 120

gttgtagaaa acaacgctgt aacagatgaa attcactatc tgtcagcaat tgaagaaggt 180

aacttcatca ttgctcaggc taactccgta ttagacgatg ataaccactt tgtagaagag 240

ttagtgactt gccgtcatag aggcgagtca agcttattta gtcgtgatca agttcagtac 300

atggacgttt caactcaaca ggttgtttct gtcggtgcat cactgatccc attccttgaa 360

cacgatgacg ccaaccgtgc attgatgggt gcgaacatgc aacgtcaggc tgttcctaca 420

cttcgtggtg ataaaccact ggtaggtaca ggtatggagc gtgctgtagc ggttgactca 480

ggtgttactg ctgttgcaaa a 501

<210> 286
<211> 501
<212> DNA
<213> Proteus mirabilis

<400> 286
actcactatg gtcgtgtgtg tccaatcgaa acacctgaag gtccaaatat cggtctgatt 60

aactcattat ctgtttatgc acagactaac gagtacggat tcttagaaac gccttaccgt 120

gttgtagaaa acaacgctgt aacagatgaa atccactatc tgtctgcaat tgaagaaggt 180

aacttcatca ttgctcaggc gaactccgta ttagatgatg atggccactt cgtagaagaa 240

ttagtaactt gccgtcataa aggtgagtca agtttattta gtcgtgatca agttcagtac 300

atggacgttt caactcaaca ggttgtttct gtcggtgctt cactgatccc attccttgaa 360

cacgatgacg ccaaccgtgc attgatgggg gcgaacatgc aacgtcaggc tgttcctaca 420

cttcgtggtg ataaaccact ggtaggtaca ggtatggaac gtgctgtagc ggttgactcc 480

ggtgttactg cggtagcaaa a 501

<210> 287
<211> 501
<212> DNA
<213> Proteus myxofaciens

<400> 287
actcactacg gtcgtgtatg tccaatcgaa acgcctgaag gtccaaacat cggtcttatc 60

aactcattat ctgtttatgc gcagactaat gaatacggat tccttgaaac tccttaccgt 120

gttgtagaaa acaatgctgt aacagatgag attcactatc tgtctgcaat tgaagaaggt 180

aacttcatca ttgctcaggc taactccgta ttagacgatg ataatcattt cgttgaagaa 240

ttagttactt gccgtcataa aggcgagtca agtttattta gtcgtgacca agttcagtac 300

atggacgttt caactcaaca ggttgtttct gtcggtgctt cactgatccc attccttgaa 360

cacgatgatg ccaaccgtgc attgatgggt gcaaacatgc aacgtcaggc tgttcctaca 420

cttcgtggtg ataaaccact cgtaggtaca ggtatggaac gtgctgttgc ggttgactcc 480

ggtgttactg cggttgctaa a 501

<210> 288
<211> 501
<212> DNA
<213> Proteus penneri

<400> 288
actcactacg gtcgtgtgtg cccaatcgaa acgcctgaag gtccaaacat cggtctgatc 60

aactcattat ctgtttatgc acagactaac gagtacggtt tccttgaaac gccttaccgt 120

gttgttgaaa ataacgctgt aacagatgaa attcactatc tgtctgcaat cgaagaaggt 180

aacttcatca ttgctcaggc taactccgta ttagacgatg acaatcactt cgttgaagaa 240

ttagtgactt gccgtcataa aggtgagtca agcttattta gtcgtgacca agttcagtac 300

atggacgttt caacccaaca ggttgtttct gtcggtgctt cactgatccc attccttgaa 360

cacgatgacg ccaaccgtgc attgatgggt gcgaacatgc aacgtcaggc tgttcctaca 420

cttcgtggtg ataaaccact agtaggtaca ggtatggagc gtgctgtagc ggttgactcc 480

ggtgttactg cggttgctaa a 501

<210> 289
<211> 501
<212> DNA
<213> Proteus vulgaris T

<400> 289
actcactacg gtcgtgtgtg tccaatcgaa acgcctgaag gtccaaacat cggtctgatc 60

aactccttat ctgtttatgc acagactaat gagtacggtt tccttgaaac gccttaccgt 120

gttgttgaaa acaacgctgt aacagatgaa attcattatc tgtctgcaat cgaagaaggt 180

aacttcatca ttgctcaggc taactccgta ttagacgatg acaatcactt cgttgaagaa 240

ttagtaactt gccgtcataa aggtgagtca agtttattta gccgtgacca agttcagtac 300

atggacgttt caacccaaca ggttgtttct gtcggtgctt cactgatccc attccttgaa 360

cacgatgacg ccaaccgtgc attgatgggt gcgaacatgc aacgtcaagc tgttcctaca 420

cttcgtggtg ataaaccact ggtaggtaca ggtatggagc gtgctgtagc ggttgactcc 480

ggtgttactg cggttgctaa g 501

<210> 290
<211> 501
<212> DNA
<213> Providencia alcalifaciens T

<400>  290
actcactatg gtcgtgtttg tccaatcgaa acgcctgaag gtccgaacat cggtctgatc      60

aactcattat ctgtttatgc acagaccaat gagtatggtt tcttagaaac cccttaccgt     120

ttagttcgtg atggcatcgt aacggatgaa atccattact tgtctgcaat cgaagaaggt     180

aacttcatca ttgctcaggc aaacaccgta ttaagtgatg atggtcgttt catcgaagaa     240

ttgatcactt gtcgtaacaa aggtgaatca agcttattca gccgtgaaca agttgaatat     300

atggacgttt cgactcaaca ggttgtttct gttggtgcgt ctctgatccc attccttgaa     360

cacgatgatg ctaaccgtgc attgatgggt gcaaacatgc aacgtcaagc ggttcctact     420

ttacgtgctg acaaaccatt agttggtaca ggtatggaac gtgcagtagc ggttgactcc     480

ggtgttactg ctgttgctag a                                               501

<210>  291
<211>  501
<212>  DNA
<213>  Providencia heimbachae

<400>  291
actcactacg gtcgtgtttg tccaatcgaa actcctgaag gcccgaatat cggtctaatc      60

aactcattgt ctgtttatgc acagaccaac gagtatggtt tcttggaaac accttatcgt     120

ttagttcgtg acggtctcgt gactgatgaa atccactatc tgtctgcaat tgaagaaggt     180

aacttcatca ttgctcaggc caacaccgta ttaggtgaag atggtagctt tgtcgaagaa     240

ctgattacct gtcgtaaccg aggtgaatca agtttattca gccgtgaaca ggttgaatat     300

atggacgttt caacacaaca gatcgtttct gttggtgcgt ctttgattcc attccttgaa     360

cacgatgatg ctaaccgtgc attgatgggt gcgaacatgc aacgtcaagc ggttccaact     420

ttacgtgctg acaaaccatt agtcggtaca ggtatggaac gtgcggttgc tgttgactca     480

ggtgttaccg ctgttgcaag a                                               501

<210>  292
<211>  501
<212>  DNA
<213>  Providencia rettgeri

<400>  292
actcactacg gtcgtgtctg tccaatcgaa acgcctgaag gtccaaacat cggtctgatc      60

aactcattat ctgtttatgc acagaccaat gagtacggtt tcttagaaac cccataccgt     120

ttagtacgtg atggcttagt gacagacgaa atccattatc tgtctgcaat tgaagaaggt     180

aacttcatca ttgctcaggc gaacaccgtt ttaggtgatg atggtagctt tgttgaagaa     240

ctgattacgt gtcgtaacaa aggtgaatca agcttattca gccgtgaaca ggttgaatat     300

atggacgttt cgactcaaca ggttgtttct gttggtgcgt ccctgatccc attccttgaa     360

cacgatgacg ccaaccgtgc attgatgggt gcaaacatgc aacgtcaagc ggttcctact     420

ctgcgtgctg acaaaccatt agttggtaca ggtatggagc gtgcagtagc ggttgactct     480

ggtgttactg ctgttgcaag a                                               501

<210> 293
<211> 501
<212> DNA
<213> Providencia rustigianii

<400> 293
actcactacg gtcgtgtatg tccaatcgaa acgccggaag gtccaaacat cggtttgatc 60

aactcattat ctgtttatgc acagaccaat gagtacggtt tcttagaaac cccttaccgt 120

ttagttcgtg atggcatcgt aacggatgaa atccattacc tgtctgcaat tgaagaaggt 180

aacttcatca ttgctcaggc aaacaccgta ttaagtgacg atggtcgttt cattgaagaa 240

ctgattactt gtcgtaacaa aggtgaatca agcttattca gccgtgaaca agttgaatat 300

atggacgttt cgactcaaca ggttgtttct gttggtgcgt ctctgatccc attccttgaa 360

cacgatgatg ctaaccgtgc attgatgggt gcgaacatgc aacgtcaagc ggttcctact 420

ttacgtgctg acaaaccatt agtcggtact ggtatggaac gtgcagtagc ggttgactcc 480

ggtgttacag cgggtagcaa g 501

<210> 294
<211> 501
<212> DNA
<213> Providencia stuartii

<400> 294
actcactacg gtcgtgtttg tccaatcgaa acccctgaag gtccgaacat cggtctgatt 60

aactcattgt ctgtttatgc acagactaac gagtatggtt tcctagaaac accttaccgt 120

ttagttcgcg atggtatcgt tactgacgaa attcactatc tgtcagcaat tgaagaaggt 180

aacttcatca ttgctcaggc aaacaccgta ttggcggaag atggtagctt cgttgaagaa 240

ctgatcactt gtcgtaaccg tggtgaatca agcttgttca gccgcgatca agttgaatat 300

atggacgttt cgactcaaca ggttgtttct gttggtgcgt cactgatccc attccttgaa 360

cacgatgatg ctaaccgtgc attgatgggt gcgaacatgc aacgtcaagc ggttccaact 420

ttacgcgctg ataaaccatt agtgggtaca ggtatggaac gtgcggttgc tgttgactcc 480

ggtgtaacag cagttgcaaa a 501

<210> 295
<211> 501
<212> DNA
<213> Providencia vermicola

<400> 295
actcactacg gtcgtgtatg tccaatcgaa acgcctgaag gtccaaacat cggtctgatc 60

aactcattat ctgtttatgc acagaccaat gagtacggtt tcttagaaac accataccgt 120

ttagttcgtg atggcttagt cactgatgag attcactatc tgtctgcaat tgaagaaggt 180

aacttcatca ttgctcaggc aaacaccgta ttaggtgaag atggtagctt tatcgaagag 240

ttagtcactt gtcgtaacaa aggtgaatca agcttattca gccgtgaaca ggttgaatat 300

atggacgttt cgactcaaca ggttgtttct gttggtgcgt cactgatccc attccttgaa 360

cacgatgacg ccaaccgtgc attgatgggt gcgaacatgc aacgtcaagc ggttccaact 420

ttacgtgctg acaaaccatt agttggtaca ggtatggaac gtgcagtagc ggttgactcg 480

ggtgtaacgg ctgttgcaag a 501

<210> 296
<211> 501
<212> DNA
<213> Rahnella aquatilis T

<400> 296
actcactacg gtcgcgtatg tccaatcgaa acgccagaag gtccaaacat cggtctgatc 60

aactccttgt ctgtttatgc acagaccaat gagtacggtt tcctggaaac cccttaccgt 120

cgcgtacgtg atggtctggt gactgatgaa atcaactatc tgtctgctat tgaagaaggc 180

aacttcgtta tcgctcaggc gaactccaac ctggacgaag aagggcgctt cattgaagac 240

ctggtcactt gtcgtagcaa aggcgaatca agcctgttta gccgcgatca ggttgactac 300

atggacgttt ctacccagca ggtggtatcc gttggtgctt cactgattcc attcctggaa 360

cacgatgacg ccaaccgtgc attgatgggt gcgaacatgc aacgtcaggc agttccgacc 420

ctgcgcgctg ataagccgct ggtaggtact ggtatggaac gtgctgtagc ggttgactcc 480

ggtgtgactg ccgtagccaa a 501

<210> 297
<211> 501
<212> DNA
<213> Raoultella ornithinolytica

<400> 297
actcactacg gtcgcgtatg tccaatcgaa acgcctgaag gtccgaacat cggtctgatc 60

aactccctgt ccgtgtacgc acagactaac gaatacggct tcctcgagac tccgtatcgt 120

aaagtgaccg acggtgttgt gaccgacgaa attcactacc tgtctgctat cgaagaaggc 180

aactacgtta tcgctcaggc gaactccaac ctggatgacg aaggccactt tgtagaagac 240

ctggttacct gccgtagcaa aggcgaatcc agcttgttca gccgcgatca ggttgactac 300

atggacgttt ccacccagca ggtggtttct gtcggtgcgt cgctgatccc gttcctggaa 360

cacgatgacg ccaaccgtgc attgatgggt gcgaacatgc aacgtcaggc ggttccgact 420

ctgcgcgctg ataagccgct ggtgggtacc ggtatggaac gtgctgttgc cgtcgactcc 480

ggtgttactg cagttgctaa a 501

<210> 298
<211> 501
<212> DNA
<213> Raoultella planticola T

<400> 298
actcactacg gtcgcgtatg tccaatcgaa acgcctgaag gtccgaacat cggtctgatc 60

aactccctgt ccgtgtacgc acagactaac gaatacggct tcctcgagac tccgtatcgt 120

aaagtgaccg acggtgttgt gaccgacgaa attcactacc tgtctgctat cgaagaaggc 180

aactacgtta tcgctcaggc gaactccaac ctggatgacg aaggccactt tgtagaagat 240

ctggtgacct gccgtagcaa aggcgaatcc agcttgttca gccgcgatca ggttgactac 300

atggacgttt ccacccaaca ggtggtttcc gtcggtgcgt cactgatccc gttcctggaa 360

cacgatgacg ccaaccgtgc attgatgggt gcgaacatgc aacgtcaggc ggttccgact 420

ctgcgcgctg ataagccgct ggttggtacc ggtatggaac gtgctgttgc cgtcgactcc 480

ggtgttactg cagttgctaa g 501

<210> 299
<211> 501
<212> DNA
<213> Raoultella terrigena

<400> 299
acccactacg gtcgcgtatg tccaatcgaa acgcctgaag gcccgaacat cggtctgatc 60

aactccctgt ccgtgtatgc acagactaac gaatacggct tcctcgagac tccgtatcgt 120

aaagtgactg acggtgttgt aaccgacgaa attcattacc tctctgctat cgaagaaggt 180

aactacgtca tcgctcaggc gaactccaac ctggatgacg aaggccactt tgaagaagac 240

ctggtaacgt gccgtagtaa aggcgaatcc agcttgttca gccgtgacca ggttgactac 300

atggacgtat ccacccagca ggtggtttcc gtcggtgcgt ccctgatccc gttcctggaa 360

cacgatgacg ccaaccgtgc attgatgggt gcgaacatgc aacgtcaggc ggttccgact 420

ctgcgcgctg ataagccgct ggttggtacc ggtatggaac gtgctgttgc cgttgactcc 480

ggtgttactg cggtcgctaa a 501

<210> 300
<211> 501
<212> DNA
<213> Salmonella bongori

<400> 300
acgcactacg gtcgcgtatg tcctatcgaa acgcctgaag gtccgaacat cggtctgatt 60

aactccctgt cagtgtatgc acagaccaac gaatatggct tccttgagac gccgtaccgt 120

cgtgttgttg atggcgtggt cactgatgaa attcattacc tgtctgctat cgaagaaggc 180

aactacgtca tcgctcaggc gaactccaac ctggatgacg aaggccactt tgtagaagat 240

ctggtaacct gccgtagcaa aggcgagtcc agcttgttca gccgcgatca ggttgactac 300

atggacgttt ccacccagca ggtggtatcc gtcggtgcgt ccctgatccc gttcctggaa 360

cacgatgacg ccaaccgtgc attgatgggt gcgaacatgc aacgtcaggc ggttccgact 420

ctgcgcgctg ataagccgct ggttggtacc ggtatggaac gtgctgttgc tgttgactcc 480

ggtgtaactg cggtagctaa a 501

<210> 301
<211> 501

<212>  DNA
<213>  Salmonella enterica sbsp. arizonae

<400>  301
acgcactacg gtcgcgtatg tcctatcgaa acgccggaag gtccgaacat cggtctgatc      60

aactccctgt ccgtgtacgc acagactaac gaatatggct tccttgagac gccgtaccgt     120

cgtgtggttg atggcgtggt cactgacgaa attcattacc tgtctgctat cgaagaaggc     180

aactacgtta tcgctcaggc gaactccaac ctggatgacg aaggccactt tgtagaagac     240

ctggtaacgt gccgtagcaa aggcgaatcc agcttgttca gccgtgacca ggttgactac     300

atggacgttt ccacccagca ggtggtctcc gtcggtgcgt ccctgatccc gttcctggaa     360

cacgatgacg ccaaccgtgc attgatgggt gcgaacatgc aacgtcaggc ggttccgact     420

ctgcgcgctg ataagccgct ggtgggtacc ggtatggaac gtgctgtcgc cgtcgactcc     480

ggtgttactg cagtggctaa a                                               501

<210>  302
<211>  501
<212>  DNA
<213>  Salmonella enterica sbsp. diarizonae

<400>  302
acgcactacg gtcgcgtatg tcctatcgaa acgcctgaag gtccgaacat cggcctgatc      60

aactccctgt ccgtgtacgc acagactaac gaatatggct tccttgagac gccgtaccgt     120

cgtgtggttg atggcgtggt cactgacgaa attcattacc tgtctgctat cgaagaaggc     180

aattacgtta tcgctcaggc gaactccaac ctggatgacg aaggccactt tgtagaagac     240

ctggtaacgt gccgtagcaa aggcgaatcc agcttgttca gccgtgatca ggttgactac     300

atggacgttt ccacccagca ggtggtctcc gtcggtgcgt ccctgattcc attcctggaa     360

cacgatgacg ccaaccgtgc attgatgggt gcgaacatgc aacgtcaggc ggttccgacg     420

ctgcgcgctg ataagccgct ggtaggcacc ggtatggaac gtgctgtcgc cgtcgactcc     480

ggtgttactg cagtggctaa a                                               501

<210>  303
<211>  501
<212>  DNA
<213>  Salmonella enterica sbsp. enterica T

<400>  303
acgcactacg gtcgcgtatg tcctatcgaa acgccggaag gtccgaacat cggtctgatc      60

aactccctgt ccgtgtacgc acagactaac gaatatggct tccttgagac gccgtaccgt     120

cgtgtggttg atggcgtggt tactgacgaa attcattacc tgtctgctat cgaagaaggc     180

aactacgtta tcgctcaggc gaactccaac ctggatgacg aaggccactt tgtagaagat     240

ttggtgacct gccgtagcaa aggcgaatcc agcttgttca gccgcgacca ggttgactac     300

atggacgttt ccacccagca ggtggtctcc gtcggtgcgt ccctgatccc gttcctggaa     360

cacgatgacg ccaaccgtgc attgatgggt gcgaacatgc aacgtcaggc ggttccgact     420

ctgcgcgctg acaagccgct ggtgggtacc ggtatggaac gtgctgtcgc cgtcgactcc 480

ggtgttactg cagtagctaa a 501

<210> 304
<211> 501
<212> DNA
<213> Salmonella enterica sbsp. houtenae

<400> 304
acgcactacg gtcgtgtatg tcctatcgaa acgcctgaag gtccgaacat cggtctgatc 60

aactccctgt ctgtgtacgc acagactaac gaatatggct tccttgagac gccgtaccgt 120

cgtgtggttg atggcgtggt gactgacgaa attcattacc tgtctgctat cgaagaaggc 180

aactacgtta tcgctcaggc gaactccaac ctggatgacg aaggccactt tgtagaagat 240

ctggtaacgt gccgtagcaa aggcgaatcc agcttgttca gccgtgatca ggttgactac 300

atggacgttt ccacccagca ggtggtctcc gtcggtgcgt ccctgatccc gttcctggaa 360

cacgatgacg ccaaccgtgc attgatgggt gcgaacatgc aacgtcaggc ggttccgact 420

ctgcgcgctg ataagccgct ggtgggtacc ggtatggaac gtgctgtcgc cgtcgactcc 480

ggtgttactg cagtggctaa a 501

<210> 305
<211> 501
<212> DNA
<213> Salmonella enterica sbsp. indica

<400> 305
acgcactacg gtcgcgtatg tcctatcgaa acgcctgaag gtccgaacat cggtctgatc 60

aactccctgt ccgtgtatgc acagactaac gaatatggct tccttgagac gccgtaccgt 120

cgtgtggttg atggcgtggt cactgacgaa attcattacc tgtctgctat cgaagaaggc 180

aattacgtta tcgctcaggc gaactccaac ctggatgacg aaggccactt tgtagaagac 240

ctggtaacgt gccgtagcaa aggtgaatcc agcttgttca gccgtgatca ggttgactac 300

atggacgtat ccacccagca ggtggtatcc gtcggtgcgt ccctgatccc attcctggag 360

cacgatgacg ccaaccgtgc attgatgggt gcgaacatgc aacgtcaggc cgttccgact 420

ctgcgtgctg ataagccgct ggttggtact ggtatggaac gtgctgttgc cgttgactcc 480

ggtgtaactg cggtagctaa a 501

<210> 306
<211> 501
<212> DNA
<213> Salmonella enterica sbsp. salamae

<400> 306
acgcactacg gtcgcgtatg tcctatcgaa acgcctgaag gtccgaacat cggtctgatt 60

aactccctgt ccgtgtatgc acagactaac gaatatggct tccttgagac gccgtaccgt 120

cgtgtggttg atggcgtggt cactgacgaa attcattacc tgtctgctat cgaagaaggc 180

aattacgtta tcgctcaggc gaactccaac ctggatgacg aaggccactt tgtagaagac 240

ctggtaacgt gccgtagcaa aggcgaatcc agcttgttca gccgtgatca ggttgactac 300

atggacgttt ccacccagca ggtggtctcc gtcggtgcgt ccctgattcc attcctggaa 360

cacgatgacg ccaaccgtgc attgatgggg gcgaacatgc aacgtcaggc ggttccgact 420

ctgcgcgctg ataagccgct ggtaggcacc ggtatggaac gtgctgtcgc cgtcgactcc 480

ggtgttactg cagtggctaa a 501

<210> 307
<211> 501
<212> DNA
<213> Samsonia erythrinae T

<400> 307
acccattatg gtcgcgtttg tcctattgaa acgccggaag gtccgaacat cggtctgatc 60

aactccctgt ccgtttatgc gcaaaccaac gaatacggtt tccttgaaac cccatatcgt 120

cgtgtgcgcg ataacgtggt gacggatgag atccattacc tgtctgcgat tgaagaaggc 180

aacttcgtta tcgcgcaggc gaacactaat ttggacgaag aaggtcgttt catcgacgaa 240

ctggttacct gccgtaacaa aggcgagtcc agcttattca gccgcgatca ggttgaatat 300

atggacgttt ccacgcagca ggtggtttcc gttggtgcgt cactgattcc gttcctggag 360

cacgatgatg ccaaccgcgc cttgatgggt gcgaacatgc aacgtcaggc tgtgccgaca 420

ctgcgcgctg ataagccgct ggtggggacc ggtatggaac gcgcggttgc ggttgactcc 480

ggtgttaccg ccgttgccaa a 501

<210> 308
<211> 501
<212> DNA
<213> Serratia entomophila

<400> 308
actcactacg gtcgcgtgtg cccaatcgaa acgccggaag gcccgaacat cggtctgatc 60

aactccctgt ccgtgtacgc acagactaac gagtatggct tcctggaaac cccgtaccgc 120

cgcgtgcgcg acggcgtggt gaccgatgaa atcaactacc tgtctgctat tgaagaaggc 180

aacttcgtta tcgctcaggc gaactccaac ctggatgatg acggccgctt cgtagaagac 240

ctggtcacct gtcgtagcaa aggcgaatca agcctgttca gccgcgacca ggttgactat 300

atggacgtat ccacccagca ggttgtctcc gttggtgctt cactgattcc attcctggaa 360

cacgatgacg ccaaccgtgc attgatgggc gcgaacatgc aacgtcaggc ggttccaacc 420

ctgcgcgctg acaagccgct ggtaggtacc ggcatggaac gtgctgtagc ggttgactcc 480

ggcgttaccg ccgtggccaa a 501

<210> 309
<211> 501
<212> DNA
<213> Serratia ficaria

<400> 309

actcactacg gtcgcgtgtg cccaatcgaa acgccggaag gcccgaacat cggtctgatc 60

aactccctgt ccgtgtacgc acagactaac gagtatggct tcctggaaac cccgtaccgc 120

cgcgtgcgcg acggcgtggt gaccgatgaa atcaactacc tgtctgctat tgaagaaggc 180

aacttcgtta tcgctcaggc gaactccaac ctggatgatg aaggccgctt cgtagaagac 240

ctggtcacct gtcgtagcaa aggcgaatcg agcctgttca gccgcgacca ggttgactat 300

atggacgtat cgacccagca ggttgtctcc gttggtgcct cactgattcc attcctggaa 360

cacgatgacg ccaaccgtgc attgatgggt gcgaacatgc aacgtcaggc ggttccaacc 420

ctgcgcgccg acaagccgct ggtaggtacc ggtatggaac gtgctgtagc ggttgactcc 480

ggcgttaccg ccgtggccaa a 501

<210> 310
<211> 501
<212> DNA
<213> Serratia fonticola

<400> 310
actcactacg gtcgcgtgtg tccaatcgaa acgccggaag gtccaaacat cggtctgatc 60

aactccttgt ccgtgtacgc acagaccaac gagtatggct tcctggaaac cccgtaccgc 120

cgcgtgcgtg acggcgtggt taccgatgaa atcaactatc tgtctgctat tgaagaaggc 180

aacttcgtta tcgctcaggc gaactccaac ctggatgaag aaggccgctt cgaagaagac 240

ctggttacct gtcgtagcaa aggcgaatca agcctgttta gccgcgacca ggttgactat 300

atggacgtat caacccagca ggttgtttct gttggtgctt cactgatccc attcctggaa 360

cacgatgacg ccaaccgtgc attgatgggt gcgaacatgc aacgtcaagc ggtgccaacc 420

ctgcgtgctg acaagccgct ggtaggtacc ggtatggaac gtgccgtagc ggttgactcc 480

ggtgttactg ccgtagccaa a 501

<210> 311
<211> 501
<212> DNA
<213> Serratia grimesii

<400> 311
acccactacg gccgtgtgtg tccaatcgaa acgccggaag gtccaaacat cggtctgatc 60

aactccttgt ccgtgtacgc acagacgaac gagtatggct tcctggaaac cccgtaccgc 120

cgtgtgcgtg acggtttggt taccgatgaa atcaactatc tgtctgctat tgaagaaggc 180

aacttcgtta tcgctcaggc gaactccaac ctggatgatg acggccgctt cgtagaagac 240

ctggtcacct gtcgtagcaa aggcgaatca agcctgttca gccgcgacca ggttgactat 300

atggacgtat caacccagca ggttgtttcc gttggtgctt cactgattcc attcctggaa 360

cacgatgacg ccaaccgtgc attgatgggt gcgaacatgc aacgtcaggc ggttccaacc 420

ttgcgtgctg acaagccgct ggtaggtacc ggtatggaac gtgctgtagc tgttgactcc 480

ggtgttactg ccgtagctcg a 501

<210> 312
<211> 501
<212> DNA
<213> Serratia liquefaciens

<400> 312
actcactacg gtcgtgtgtg tccaatcgaa acgccggaag gtccaaacat cggtctgatc 60

aactccttgt ccgtgtacgc acagacaaac gagtatggct tcctggaaac tccgtaccgc 120

cgcgtgcgtg acggcttggt taccgatgaa atcaactatc tgtctgctat tgaagaaggc 180

aacttcgtta tcgctcaggc gaactccaac ctggacgaag acggccgctt catagaagac 240

ctggtcacct gtcgtagcaa aggcgaatca agcctgttca gccgcgatca ggttgactat 300

atggacgtat caacccagca ggttgtttcc gttggtgcct cactgattcc attcctggaa 360

cacgatgacg ccaaccgtgc attgatgggt gcgaacatgc aacgtcaggc ggttccaacc 420

ttgcgtgctg acaagccgct ggtaggtacc ggtatggaac gtgctgtagc ggttgactcc 480

ggtgttactg ccgtagctcg c 501

<210> 313
<211> 501
<212> DNA
<213> Serratia marcescens sbsp. sakuensis

<400> 313
acccactacg gccgcgtgtg cccaatcgaa acgccggaag gtccaaacat cggtctgatc 60

aactccctgt ccgtgtacgc acagactaac gagtatggct tcctggaaac cccgtaccgc 120

cgcgtgcgtg acggcgtggt gaccgatgaa atcaactacc tgtctgctat tgaagaaggc 180

aacttcgtta tcgcccaggc gaactccaac ctggatgaag aaggccgctt cgtagaagac 240

ctggtcacct gtcgtagcaa aggcgaatca agcctgttca gccgcgacca ggttgactac 300

atggacgtat ccacccagca ggtcgtttcc gttggtgctt cactgattcc attcctggaa 360

cacgatgacg ccaaccgtgc attgatgggt gcgaacatgc aacgtcaggc cgtaccaacc 420

ctgcgcgctg acaagccgct ggtcggtacc ggtatggaac gtgctgtagc ggttgactcc 480

ggcgtaaccg ccgttgccaa a 501

<210> 314
<211> 501
<212> DNA
<213> Serratia odorifera

<400> 314
actcactacg gtcgcgtatg tccaatcgaa acgccggaag gtccgaacat cggtctgatc 60

aactccttgt ccgtgtacgc acagaccaac gagtatggct tcctggaaac cccgtaccgc 120

cgcgtgcgtg acggcgtggt taccgatgaa atcaactacc tgtctgccat tgaagaaggc 180

aacttcgtta tcgcccaggc gaactccaac ctggatgaag aaggccgctt cgtggaagac 240

ctggtcacct gtcgtagcaa gggcgaatca agcctgttca gccgcgacca ggttgactat 300

atggacgtat cgacccagca ggttgtctcc gttggtgctt cactgattcc gttcctggaa 360

cacgatgacg ccaaccgtgc attgatgggt gcgaacatgc aacgtcaggc ggttcctacc 420

ctgcgtgctg acaagccgct ggttggtacc ggtatggaac gtgccgtcgc cgttgactcc 480

ggcgttaccg ccgtagccaa g 501

<210> 315
<211> 501
<212> DNA
<213> Serratia plymuthica

<400> 315
actcactatg gtcgcgtgtg tccaatcgaa acgccggaag gtccaaacat cggtctgatc 60

aactccttgt ccgtgtacgc acagaccaac gagtatggct tcctggaaac cccgtaccgc 120

cgcgtgcgtg acggtctggt taccgatgaa atcaactacc tgtctgctat tgaagaaggc 180

aacttcgtta tcgctcaggc gaactccaac ctggatgatg acggccgctt cgaagaagac 240

ctggtcacct gtcgtagcaa aggcgaatca agcctgttta gccgcgatca ggttgactat 300

atggacgtat cgacccagca ggttgtctcc gttggtgctt cactgattcc attcctggaa 360

cacgatgacg ccaaccgtgc attgatgggt gcgaacatgc aacgtcaggc ggttccgact 420

ctgcgtgctg acaagccgct ggtaggtacc ggtatggagc gtgcggttgc ggttgactcc 480

ggtgttactg ccgtagccaa a 501

<210> 316
<211> 501
<212> DNA
<213> Serratia proteamaculans sbsp. proteamaculans

<400> 316
actcactacg gtcgcgtgtg cccaatcgaa acgccggaag gtccaaacat cggtctgatc 60

aactccttgt ccgtgtacgc acagacaaac gagtatggct tcctggaaac cccgtaccgc 120

cgcgtgcgtg acggtttggt taccgatgaa atcaactatc tgtctgctat tgaagaaggc 180

aacttcgtta tcgctcaggc gaactccaac ctgagtgaag acggccgctt cgaagaagac 240

ctggtcacct gtcgtagcaa aggcgagtca agcctgttca gccgcgacca ggttgactat 300

atggacgtat caacccagca gattgtttcc gttggcgctt cactgattcc attcctggaa 360

cacgatgacg ccaaccgtgc attgatgggt gcgaacatgc aacgtcaggc ggttccaacc 420

ttgcgcgctg ataagccgct ggtaggtacc ggtatggaac gtgctgtagc tgttgactcc 480

ggtgttactg ccgtagctcg a 501

<210> 317
<211> 501
<212> DNA
<213> Serratia quinivorans

<400> 317
actcactacg gtcgcgtgtg cccaatcgaa acgccggaag gtccaaacat cggtctgatc 60

aactccttgt ccgtgtacgc acagaccaac gagtatggct tcctggaaac tccgtaccgc 120

cgcgtgcgtg acggcctggt taccgatgaa atcaactatc tgtctgctat tgaagaaggc 180

aacttcgtta tcgctcaggc gaactccaac ctggatgatg acggccgctt cgtagaagac 240

ctggtcacct gtcgtagcaa aggcgaatca agcctgttca gccgcgacca ggttgactat 300

atggacgtat caacccagca ggttgtctcc gttggtgctt cactgattcc attcctggaa 360

cacgatgacg ccaaccgtgc attgatgggt gcgaacatgc aacgtcaggc ggttccaacc 420

ttgcgtgctg acaagccgct ggtaggtacc ggtatggaac gtgctgtagc tgttgactcc 480

ggtgttactg ccgtagctcg a 501

<210> 318
<211> 501
<212> DNA
<213> Serratia rubidaea

<400> 318
acccactacg gtcgtgtatg tccaatcgaa accccggaag gtccgaacat cggtctgatc 60

aactccctgt ccgtgtacgc acagactaac gagtatggct tcctggaaac cccgtaccgc 120

cgcgtgcgtg acggcgtggt taccgatgaa atcaactacc tgtctgccat tgaagaaggc 180

aacttcgtta tcgcccaggc taactccaac ctggatgatg acggccgctt cgtggaagac 240

ctggtcacct gccgcagcaa aggcgaatcc agcctgttca gccgcgatca ggttgactat 300

atggacgtat ccacccagca ggttgtttcc gttggtgcct ctctgattcc attcctggaa 360

cacgatgacg ccaaccgtgc attgatgggt gcgaacatgc aacgtcaggc ggtgccaacc 420

ctgcgtgctg acaagccgct ggtcggtacc ggtatggaac gcgcagtagc ggttgactcc 480

ggcgttaccg ccgttgccaa a 501

<210> 319
<211> 501
<212> DNA
<213> Serratia ureilytica

<400> 319
acccactacg gccgcgtgtg cccaatcgaa acgccggaag gtccaaacat cggtctgatc 60

aactccctgt ccgtgtacgc acagactaac gagtatggct tcctggaaac cccgtaccgc 120

cgcgtgcgtg acggcgtggt gaccgatgaa atcaactacc tgtctgctat tgaagaaggc 180

aacttcgtta tcgcccaggc gaactccaac ctggatgacg aaggccgctt cgtagaagac 240

ctggtcacct gccgcagcaa aggcgaatca agcctgttca gccgcgacca ggttgactac 300

atggacgtat ccacccaaca ggtcgtttcc gttggtgcct cactgattcc attcctggaa 360

cacgatgacg ccaaccgtgc attgatgggt gcgaacatgc aacgtcaggc cgtaccaacc 420

ctgcgcgccg ataagccgct ggtcggtacc ggtatggaac gcgctgtagc ggttgactcc 480

ggcgtaaccg ccgttgccaa a 501

<210> 320
<211> 501
<212> DNA

<213>  Shigella boydii

<400>  320
actcactacg gtcgcgtatg tccaatcgaa acccctgaag gtccgaacat cggtctgatc      60

aactctctgt ccgtgtacgc acagactaac gaatacggct tccttgagac tccgtatcgt     120

aaagtgaccg acggtgttgt aactgacgaa attcactacc tgtctgctat cgaagaaggc     180

aactacgtta tcgcccaggc gaactccaac ctggatgaag aaggccactt cgtagaagac     240

ctggtaactt gccgtagcaa aggcgaatcc agcttgttca gccgcgacca ggttgactac     300

atggacgtat ccacccagca ggtggtatcc gtcggtgcgt ccctgatccc gttcctggaa     360

cacgatgacg ccaaccgtgc attgatgggt gcgaacatgc aacgtcaggc cgttccgact     420

ctgcgcgctg ataagccgct ggttggtact ggtatggaac gtgctgttgc cgttgactcc     480

ggtgtaactg cggtagctaa a                                               501

<210>  321
<211>  501
<212>  DNA
<213>  Shigella dysenteriae

<400>  321
actcactacg gtcgcgtatg tccaatcgaa acccctgaag gtccgaacat cggtctgatc      60

aactctctgt ccgtgtacgc acagactaac gaatacggct tccttgagac tccgtatcgt     120

aaagtgaccg acggtgttgt aactgacgaa attcactacc tgtctgctat cgaagaaggc     180

aactacgtta tcgcccaggc gaactccaac ctggatgaag aaggccactt cgtagaagac     240

ctggtaactt gccgtagcaa aggcgaatcc agcttgttca gccgcgacca ggttgactac     300

atggacgtat ccacccagca ggtggtatcc gtcggtgcgt ccctgatccc gttcctggaa     360

cacgatgacg ccaaccgtgc attgatgggt gcgaacatgc aacgtcaggc cgttccgact     420

ctgcgcgctg ataagccgct ggttggtact ggtatggaac gtgctgttgc cgttgactcc     480

ggtgtaactg cggtagctaa a                                               501

<210>  322
<211>  501
<212>  DNA
<213>  Shigella flexneri

<400>  322
actcactacg gtcgcgtatg tccaatcgaa acccctgaag gtccgaacat cggtctgatc      60

aactctctgt ccgtgtacgc acagactaac gaatacggct tccttgagac tccgtatcgt     120

aaagtgaccg acggtgttgt aactgacgaa attcactacc tgtctgctat cgaagaaggc     180

aactacgtta tcgcccaggc gaactccaac ctggatgaag aaggccactt cgtagaagac     240

ctggtaactt gccgtagcaa aggcgaatcc agcttgttca gccgcgacca ggttgactac     300

atggacgtat ccacccagca ggtggtatcc gtcggtgcgt ccctgatccc gttcctggaa     360

cacgatgacg ccaaccgtgc attgatgggt gcgaatatgc aacgtcaggc cgttccgact     420

ctgcgcgctg ataagccgct ggttggtact ggtatggaac gtgctgttgc cgttgactcc     480

ggtgtaactg cggtagctaa a                                              501

<210>  323
<211>  501
<212>  DNA
<213>  Shigella sonnei

<400>  323
actcattacg gtcgcgtatg tccaatcgaa acccctgaag gtccgaacat cggtctgatc      60

aactctttgt ccgtgtacgc acagactaac gaatacggct tccttgagac tccgtatcgt     120

aaggtgaccg acggtgttgt aactgacgaa attcactacc tttctgctat cgaagaaggc     180

aactacgtta tcgcccaggc gaactccaac ctggatgaag aaggccactt cgtagaagac     240

ctggtaactt gccgtagcaa aggcgaatcc agcttgttca gccgtgacca ggttgactac     300

atggacgtat ccacccagca ggtggtatcc gtcggtgcgt ccctgatccc gttcctggaa     360

cacgatgacg ccaaccgtgc attgatgggt gcgaacatgc aacgtcaggc cgttccgact     420

ctgcgcgctg ataagccgct ggttggtact ggtatggaac gtgctgttgc cgttgactcc     480

ggtgtaactg cggtagctaa a                                              501

<210>  324
<211>  501
<212>  DNA
<213>  Tatumella ptyseos T

<400>  324
actcactacg gtcgtgtttg tcctatcgaa acgcctgaag gtccgaacat cggtcttatc      60

aactcactgt ctgtgtatgc gcagactaac gaatatggct tcctcgaaac gccataccgt     120

cgtgtggttg atagccaggt gactgacgaa attcattacc tgtctgcgat tgaagaaggc     180

aacttcgtta ttgctcaggc gaacaccaac ctcgacgacg atggtcgttt cgtggatgac     240

ctggtgacct gtcgtaacaa gggtgaatcg agcctgttca gccgtgacca ggttgactac     300

atggacgttt cgacccagca ggttgtttct gtcggtgcgt cactgatccc attcctggaa     360

cacgatgatg ctaaccgtgc attgatgggt gcaaacatgc aacgtcaggc ggttcctact     420

ctgcgtgctg ataagccgtt agtaggtacc ggtatggaac gcgcggtcgc agtcgactcc     480

ggtgttactg cagtggctaa g                                              501

<210>  325
<211>  501
<212>  DNA
<213>  Trabulsiella guamensis T

<400>  325
acccactacg gacgcgtatg tcctatcgaa acgcctgaag gtccgaacat cggtctgatc      60

aactccctgt ccgtttatgc acagactaac gaatacggtt tccttgaaac cccgtatcgt     120

aaagtaactg acggtgtggt gactgacgaa attcattacc tgtccgcaat tgaggaaggt     180

aactacgtaa tcgctcaggc gaacaccaac ctgacggaag aaggtcgttt tgtagacgat     240

ctggtcacct gccgcagcaa aggcgaatcc agcctgttca gcgcagacca ggttgactac 300

atggacgttt ccactcagca ggtggtttcc gtcggtgcat ccctgatccc gttcctggaa 360

cacgatgacg ccaaccgtgc attgatgggt gcgaacatgc aacgtcaggc ggttccgacc 420

ctgcgctctg ataagccgct ggtcggtacc ggtatggaac gtgctgttgc cgttgactcc 480

ggtgttaccg cggtagctaa g 501

<210> 326
<211> 501
<212> DNA
<213> Xenorhabdus beddingii

<400> 326
actcactatg gtcgtgtatg tccaatcgaa acgcctgaag gtccaaacat cggtttgatt 60

aactcattgt ccgtttatgc gcgtaccaat gagtatggtt tccttgaaac cccttaccgc 120

ttggttcgtg atggtgtagt gactgatgaa attcactacc tgtctgcgat cgaagaaggt 180

aattacgtta tcgcacaggc taacaccgta ttggatgaag aaggccactt cgttgaagaa 240

ttggtcactt gccgtcatta cgatgaatca agtttgttca accgtgatca ggttcagtac 300

atggacgttt cgacacagca gatcgtgtcc gtcggtgctt ccttgatccc attccttgaa 360

cacgatgacg ccaaccgtgc attgatgggt gcgaacatgc aacgtcaggc ggttccaaca 420

ctgcgtgctg acaagccgct ggtcggaaca ggcatggagc gtgcggtagc ggttgactcc 480

ggggtaacct ctgttgctaa a 501

<210> 327
<211> 501
<212> DNA
<213> Xenorhabdus budapestensis

<400> 327
actcactatg gtcgtgtatg tccaattgaa acgcctgaag gtccaaacat tggtctgatt 60

aactcactgt ctgtttatgc acgtactaac gagtatggtt tccttgaaac cccttatcgt 120

ttagtacgcg acggtgtagt aacagatgag attcattacc tgtcagcgat tgaggaaggt 180

aactacgtca ttgcgcaggc taactccgca ttggacgaag aaggccactt tatcgaagaa 240

ctggtaactt gccgtcacta tgacgaatcc agtttgttta accgtgatca ggttcagtac 300

atggacgttt cgacacaaca gatcgtgtcc gtcggtgctt ccttgatccc attccttgaa 360

cacgatgacg ccaaccgtgc attgatgggt gcaaacatgc aacgtcaggc ggttccaact 420

ctgcgtgcag ataagccgct ggttggaaca ggtatggagc gtgcggtagc ggttgactct 480

ggtgtaacct ccgttgctaa a 501

<210> 328
<211> 501
<212> DNA
<213> Xenorhabdus ehlersii

<400> 328
actcactatg gtcgtgtatg tccaatcgaa acacctgaag gtccaaacat cggtttgatt 60

aactcattgt ctgtttatgc gcgtaccaat gagtatggtt tccttgaaac accttaccgc 120

ttagttcgtg atggtgtggt gactgatgaa atccactacc tgtcagcaat cgaagaaggc 180

aactacgtca ttgctcaggc gaacaccgta ttggatgaag aaggccactt cgttgaagaa 240

ttggtcactt gccgtcatta cgatgaatcc agcttgttca accgtgatca ggttcagtac 300

atggacgttt cgacacaaca gatcgtgtcc gtcggtgctt ctctgatccc attccttgaa 360

cacgatgacg ccaaccgtgc attgatgggt gcgaacatgc aacgtcaggc ggttccaact 420

ctgcgcgcag ataagccgct ggtcggaaca ggtatggagc gtgcggtagc ggttgactcc 480

ggtgtaacct ctgttgctaa a 501

<210> 329
<211> 501
<212> DNA
<213> Xenorhabdus innexi

<400> 329
actcactatg gtcgcgtatg tccaatcgaa acgcctgaag gtccgaacat cggtctgatt 60

aactcactgt ccgtttatgc tcgtaccaac gaatacggtt tccttgaaac cccttatcgt 120

ttagtacgtg acggtgtggt aacagatgag attcattacc tgtcagcgat tgaagaaggt 180

aactacgtca ttgcgcaggc taactccgca ctggacgaag aaggccactt cattgaagaa 240

ctggtaacct gccgtcatta tgacgaatcc agcttgttca gccgtgatca ggttcagtac 300

atggacgttt cgacacaaca gatcgtgtcc gtcggtgctt ccctgatccc attccttgaa 360

cacgatgacg ccaaccgtgc attgatgggt gcaaacatgc aacgtcaggc ggttccgact 420

ctgcgtgcag acaagccgct ggttggtaca ggcatggagc gtgcggtagc ggttgactcc 480

ggtgtaacct ccgttgctaa a 501

<210> 330
<211> 501
<212> DNA
<213> Xenorhabdus japonica

<400> 330
actcactatg gtcgtgtatg tccaatcgaa acgcctgaag gtccgaacat cggtctgatt 60

aactcattgt ccgtttacgc gcgtaccaat gagtatggtt tccttgaaac cccttaccgt 120

ttagttcgtg atggtgtagt gacggatgaa atccactacc tgtctgcgat cgaagaaggt 180

aactacgtta ttgcacaggc aaacactgta ttggatgaag aaggccactt cgttgaagaa 240

ttggtcactt gccgtcatta cgatgaatcc agcttgttca accgtgatca ggttcagtac 300

atggacgttt cgacacaaca gatcgtgtcc gtcggtgctt ccttgatccc attccttgaa 360

cacgatgacg ccaaccgtgc tttgatgggt gcgaacatgc aacgtcaggc ggttccgaca 420

ctgcgtgctg acaagccgtt agtgggtaca ggcatggaac gtgcagtagc ggttgactcc 480

ggtgtgacct ctgttgctaa a 501

<210> 331
<211> 501
<212> DNA
<213> Xenorhabdus poinarii

<400> 331
actcattacg gtcgtgtatg tccaatcgaa acgcctgaag ggccaaacat cggtctgatt 60

aactcattat ccgtctatgc gcgtaccaat gagtatggtt tcctggagac accttaccgc 120

ttagtgcgtg atggcgtagt cactgatgaa attcattacc tgtctgcgat cgaagaaggt 180

aactacgtta ttgctcaggc gaacaccgta ttggatgaag aaggccactt tgttgaagaa 240

ttggtcactt gccgtcatta cgatgaatcc agcttgttca accgtgatca ggttcagtac 300

atggacgttt cgacacaaca gatcgtgtcc gtcggtgctt ctctgatccc attccttgaa 360

cacgatgacg ccaaccgtgc attgatgggt gcgaacatgc aacgtcaggc ggtaccaaca 420

ctgcgtgctg ataagccgtt ggtcggaaca ggcatggagc gtgcagttgc agttgactcc 480

ggtgtcactt ccgttgctaa a 501

<210> 332
<211> 501
<212> DNA
<213> Xenorhabdus szentirmaii

<400> 332
actcactatg gccgtgtatg tccaatcgaa actcctgaag gtccaaacat cggtctgatt 60

aactcattgt ccgtttatgc acgtaccaat gagtatggtt tccttgaaac cccttaccgt 120

ttagttcgtg atggcgtagt aactgatgaa atccattacc tgtctgcgat cgaagaaggt 180

aactacgtta ttgcacaggc aaacaccgta ttggatgaag aaggccactt cgttgaagaa 240

ttagttactt gccgccatta cgatgaatcc agcttgttca accgtgatca ggttcagtac 300

atggacgttt cgacacaaca gatcgtgtcc gtcggtgctt ccctgatccc attccttgaa 360

cacgatgacg ccaaccgtgc attgatgggt gcgaacatgc aacgtcaggc tgttccaact 420

ctgcgtgctg ataagccgct ggtcggaaca ggtatggaac gtgcggtagc ggttgactcc 480

ggtgtaactt ctgttgctaa a 501

<210> 333
<211> 501
<212> DNA
<213> Yersinia aldovae

<400> 333
actcactacg gtcgcgtatg tccaatcgaa acgccagaag gtccaaacat cggtctgatt 60

aactccttgt ctgtctacgc acagaccaac gagtatggtt tcctggaaac cccttatcgt 120

cgcgtgcgcg atggtgtggt gaccgatgaa attaactatc tgtctgctat tgaagaaggc 180

aacttcgtta tcgctcaggc gaactccaac ctggatgatg aaggccgctt cttggaagac 240

ctggtcactt gtcgtagcaa aggcgaatca agcctgttta gccgcgatca ggttgactat 300

atggacgttt ccactcaaca gatcgtgtcc gttggtgctt ctctgattcc attcctggaa 360

cacgatgacg ccaaccgtgc attgatgggt gcgaacatgc aacgtcaggc ggttcctact 420

ctgcgtgctg ataagccgtt ggtgggtacc ggtatggaac gtgcggtagc ggttgactca 480

ggggtaacct ctgtagccaa a 501

<210> 334
<211> 501
<212> DNA
<213> Yersinia bercovieri

<400> 334
actcactatg gtcgtgtatg tccaatcgaa acgccagaag gtccaaacat cggtctgatc 60

aactccttgt ctgtgtacgc acagaccaat gagtatggtt tcctggaaac tccgtatcgt 120

cgtgtgcgtg acggtgtggt gactgatgaa atcaactatc tgtctgctat tgaagaaggc 180

aacttcgtta ttgctcaggc gaactccaac ctggacgaag acggccgctt catcgaagac 240

ttggtcactt gtcgtagcaa aggcgaatca agcctgttca gccgcgatca agttgactac 300

atggacgttt ccacccaaca ggtggtgtcc gttggtgctt ctctgattcc attcctggaa 360

cacgatgacg ccaaccgtgc cttgatgggt gcgaacatgc aacgtcaggc ggttcctact 420

ctgcgtgctg ataagccgct ggtaggtact ggtatggaac gtgcggtagc ggttgactca 480

ggggtaacct ctgtagccaa a 501

<210> 335
<211> 501
<212> DNA
<213> Yersinia enterocolitica sbsp. enterocolitica

<400> 335
actcactacg gtcgcgtatg tccaatcgaa acgccagaag gtccaaacat cggtctgatc 60

aactccttgt ccgtgtacgc acagaccaac gagtatggtt tcctggaaac cccttatcgt 120

cgcgtgcgcg atggtgtggt gaccgatgaa attaactatc tgtctgctat tgaagaaggc 180

aacttcgtta tcgctcaggc gaactccaac ctggacgaag acggtcgctt cattgaagac 240

ttagtcactt gtcgtagcaa aggcgaatca agcctgttta gccgcgatca agttgactac 300

atggacgttt ccactcaaca ggtggtgtcc gttggtgctt ctctgattcc attcctggaa 360

cacgatgacg ccaaccgcgc cttgatgggt gcgaacatgc aacgtcaggc agttcctact 420

ctgcgtgctg ataagccgtt ggtgggtact ggtatggaac gtgcggtagc ggttgactca 480

ggggtaacct ctgtagccaa a 501

<210> 336
<211> 501
<212> DNA
<213> Yersinia enterocolitica sbsp. palearctica

<400> 336
actcactacg gtcgcgtatg tccaatcgaa acgccagaag gtccaaacat cggtctgatc 60

aactccttgt ccgtgtacgc acagaccaac gagtatggtt tcctggaaac cccttatcgt 120

cgcgtgcgcg atggtgtggt gaccgatgaa attaactatc tgtctgctat tgaagaaggc 180

aacttcgtta tcgctcaggc gaactccaac ctggacgaag acggccgctt catcgaagac 240

ttggtcactt gtcgtagcaa aggcgaatca agcctgttca gccgcgatca agttgactac 300

atggacgttt ccactcaaca ggtggtgtcc gttggtgctt ctctgattcc attcctggaa 360

cacgatgacg ccaaccgcgc cttgatgggt gcgaacatgc aacgtcaggc agttcctact 420

ctgcgtgctg ataagccgtt ggtgggtact ggtatggaac gtgcggtagc ggttgactca 480

ggggtaacct ctgtagccaa a 501

<210> 337
<211> 501
<212> DNA
<213> Yersinia frederiksenii

<400> 337
actcactacg gtcgcgtatg tccaatcgaa acgccagaag gtccaaacat cggtctgatc 60

aactccttgt ccgtgtacgc acagaccaat gagtatggtt tcctggaaac accttatcgt 120

cgcgtgcgtg atggtgtggt gaccgatgaa attaactatt tgtctgctat tgaagaaggc 180

aacttcgtta tcgctcaggc gaactccaac ttggacgaag atggccgctt catcgaagac 240

ttagtcactt gtcgtagcaa aggcgaatca agcctgttca gccgcgacca agttgactac 300

atggacgttt ccactcaaca ggtggtgtcc gttggtgctt ctctgattcc attcttggaa 360

cacgatgacg ccaaccgtgc cttgatgggt gcgaacatgc aacgtcaggc ggttcctact 420

ctgcgtgctg ataagccgct ggtaggtacc ggtatggaac gtgcagtagc ggttgactca 480

ggggtaacct ctgtagccaa a 501

<210> 338
<211> 501
<212> DNA
<213> Yersinia intermedia

<400> 338
actcactacg gtcgcgtatg cccaatcgaa acgccagaag gtccaaacat cggtctgatc 60

aactccttgt ccgtgtacgc acagaccaac gagtatggtt tcctggaaac accttatcgt 120

cgcgtgcgcg atggtgtggt gaccgatgaa attaactatt tgtctgctat tgaagaaggc 180

aatttcgtta tcgctcaggc gaactccaac ctggacgaag acggccgctt cattgaagac 240

ttggtcactt gtcgtagcaa aggcgaatca agcctgttta gccgcgatca ggttgactat 300

atggacgttt ccactcaaca ggtcgtgtct gttggtgctt ctctgattcc attcttggaa 360

cacgatgacg ccaaccgtgc attgatgggt gcgaacatgc aacgtcaggc ggttcctact 420

ctgcgtgctg ataagccgct ggtgggtact ggtatggaac gtgcagtagc ggttgactca 480

ggggtaacct ctgtagctaa a 501

<210> 339
<211> 501
<212> DNA
<213> Yersinia kristensenii

<400> 339
actcactacg gtcgcgtatg tccaatcgaa acgccagaag gtccaaacat cggtctgatc 60

aactccttgt ccgtgtacgc acagaccaat gagtatggtt tcctggaaac accttatcgt 120

cgcgtacgtg atggtgtggt gaccgatgaa attaactatc tgtctgctat tgaagaaggc 180

aacttcgtta tcgctcaggc gaactccaac ctggacgaag acggccgctt cattgaagac 240

ttagtcactt gtcgtagcaa aggcgaatca agcctgttca gccgcgatca agttgactac 300

atggacgttt ccactcaaca ggtggtgtcc gttggtgctt ctctgattcc attcttggaa 360

cacgatgacg ccaaccgcgc cttgatgggt gcgagcatgc aacgtcaggc ggttcctact 420

ctgcgtgcag ataagccgtt agtgggtacc ggtatggaac gtgcggtagc ggttgactca 480

ggggtaacct ctgtagctaa a 501

<210> 340
<211> 501
<212> DNA
<213> Yersinia mollaretii

<400> 340
actcactacg gtcgtgtatg tccaatcgaa acgccagaag gtccaaacat cggtctgatc 60

aactccttgt ccgtgtacgc acagaccaat gagtatggtt tcctggaaac tccgtatcgt 120

cgcgtgcgtg acggtgtggt gactgatgaa atcaactacc tgtctgctat tgaagagggc 180

aacttcgtta tcgctcaggc gaactccaac ttggacgaag acggccgctt cattgaagac 240

ttagtcactt gtcgtagcaa aggcgaatca agcctgttca gccgcgatca agttgactac 300

atggacgttt ccactcaaca ggtggtgtcc gttggtgctt ctctgattcc attcttggaa 360

cacgatgacg ccaaccgtgc cttgatgggt gcgaacatgc aacgtcaggc cgttcctact 420

ctgcgtgctg ataagccgct ggtaggtacg ggtatggaac gtgcggtagc ggttgactca 480

ggggtaacct ctgtagccaa a 501

<210> 341
<211> 501
<212> DNA
<213> Yersinia pestis

<400> 341
actcactacg gtcgcgtatg tccaattgaa acgccagaag gtccaaacat cggtttgatc 60

aactccttgt ctgtatatgc acagactaac gagtatggtt tcctggaaac accttatcgt 120

cgtgtacgtg atggtgtggt gaccgatgaa attaactatc tgtctgctat tgaagaaggc 180

aacttcgtta tcgctcaggc gaactccaac ctggatgatg aaggccgctt tttagaagat 240

ttggtgactt gtcgtagcaa aggcgaatca agcctgttta gccgcgagca agttgactac 300

atggacgtat ctactcagca aatcgtatcc gttggtgctt ctctgattcc attcttggaa 360

cacgatgacg ccaaccgtgc cttgatgggc gcgaacatgc aacgtcaggc ggttcctact 420

ctgcgtgctg ataagccgtt ggtgggtact ggtatggaac gtgcagtggc ggttgactca 480

ggggtaacct ctgtagccaa a 501

<210> 342
<211> 501
<212> DNA
<213> Yersinia pseudotuberculosis sbsp. pseudotuberculosis

<400> 342
actcactacg gtcgcgtatg tccaattgaa acgccagaag gtccaaacat cggtttgatc 60

aactccttgt ctgtatatgc acagactaac gagtatggtt tcctggaaac accttatcgt 120

cgtgtacgtg atggtgtggt gaccgatgaa attaactatc tgtctgctat tgaagaaggc 180

aacttcgtta tcgctcaggc gaactccaac ctggatgatg aaggccgctt tttagaagat 240

ttggtgactt gtcgtagcaa aggcgaatca agcctgttta gccgcgagca agttgactac 300

atggacgtat ctactcagca aatcgtatcc gttggtgctt ctctgattcc attcttggaa 360

cacgatgacg ccaaccgtgc cttgatgggc gcgaacatgc aacgtcaggc ggttcctact 420

ctgcgtgctg ataagccgtt ggtgggtact ggtatggaac gtgcagtggc ggttgactca 480

ggggtaacct ctgtagccaa a 501

<210> 343
<211> 501
<212> DNA
<213> Yersinia rohdei

<400> 343
actcactacg gtcgtgtatg tccaatcgaa acgccagaag gtccaaacat cggtctgatc 60

aactccttgt ccgtgtacgc acagaccaat gagtatggtt tcctggaaac tccgtatcgt 120

cgcgtacgtg acggtgtggt gactgatgaa atcaactatc tgtctgctat tgaagaaggc 180

aacttcgtta tcgctcaggc gaactccaac ttggacgaag acggccgctt catcgaagac 240

ttagtcactt gtcgtagcaa aggcgaatca agcctgttca gccgcgacca agttgactac 300

atggacgttt ccactcaaca ggtggtgtcc gttggtgctt ctctgattcc attcttggaa 360

cacgatgacg ccaaccgcgc cttgatgggt gcgaacatgc aacgtcaggc ggttccaact 420

ctgcgtgctg ataagccgct ggtaggtacc ggtatggaac gtgcagtagc ggttgactca 480

ggggtaacct ctgtagccaa a 501

<210> 344
<211> 501
<212> DNA
<213> Yersinia ruckeri

<400> 344
actcactatg gtcgtgtatg tccaatcgaa acgccagaag gtccaaacat cggtctgatc 60

aactccttgt ccgtgtacgc acagaccaat gagtatggtt tcctggaaac tccttatcgt 120

cgcgtgcgtg acggtttggt gactgatgaa atcaactatc tgtctgctat tgaagaaggc 180

aacttcgtta tcgctcaggc gaactccaac ctggacgaag acggccgctt catcgaagac 240

ttggtcactt gtcgtagcaa aggtgaatca agcctgttta gccgcgatca agttgactat 300

atggacgttt ccactcaaca ggtggtgtcc gttggtgctt ctctgattcc gttcctggaa 360

cacgatgatg ccaaccgcgc cttgatgggt gcgaacatgc aacgtcaagc ggttcctact 420

ctgcgtgctg ataagccgct ggtgggtact ggtatggaac gtgcagtagc ggttgactct 480

ggggtaacct ctgtagccaa g 501

<210> 345
<211> 501
<212> DNA
<213> Yokenella regensburgei T

<400> 345
actcactacg gtcgcgtatg tcctatcgaa acgcctgaag gtccgaacat cggtctgatc 60

aactccttgt ccgtgtacgc acagactaac gaatacggct tccttgagac tccgtatcgt 120

aaagtgaccg acggtgttgt aaccgacgaa attcattacc tgtctgctat tgaagaaggc 180

aactacgtta tcgctcaggc gaactccaac ctggatgacg aaggccactt tgaagaagat 240

ctggttacct gccgtagcaa aggcgaatcc agcttgttca gccgtgacca ggttgactac 300

atggacgtat ccacccagca ggtggtatcc gtcggtgcgt ccctgatccc gttcctggaa 360

cacgatgacg ccaaccgtgc attgatgggt gcgaacatgc aacgtcaggc ggttccgact 420

ctgcgcgctg ataagccgct ggttggtacc ggtatggaac gtgctgttgc cgttgactcc 480

ggtgttactg cagttgctaa a 501

<210> 346
<211> 633
<212> DNA
<213> Brenneria alni

<400> 346
ggtgtaaacc acaaaatcgg tgaagttcat gacggcgcgg caactatgga ctggatggag 60

caggagcagg agcgtggtat cactatcacc tctgctgcga ctacggcttt ctggtctggt 120

atggctaaac agtttgagcc acaccgcgta aacatcatcg acacccccgg acacgttgac 180

ttcactattg aagtggaacg ttccatgcgt gttcttgacg gtgcagtaat ggtttactgt 240

gcagttggtg gtgttcagcc gcagtctgaa actgtatggc gtcaggctaa caaatataaa 300

gttccgcgta ttgcgttcgt taacaaaatg gaccgtatgg gcgcgaactt cctgcgtgtt 360

gtcagccaga tcaaaacgcg tctggcggca aacccggttc ccattcagtt ggcgatcggt 420

gcagaagaca gcttcactgg tgttgtcgac ctggtgaaaa tgaaagcgat caactggaat 480

gaagccgatc agggcgttac cttcacttat gaagagattc cggctgatat gcaggatctg 540

gcccaggaat ggcaccagaa tctggttgag tctgcagctg aagcatctga agagctgatg 600

gacaaatacc tgggcggtga agaactgacc gaa 633

<210> 347
<211> 633
<212> DNA
<213> Brenneria quercina

<400>  347
ggtgtgaatc acaaaatcgg tgaagttcat gacggcgctg ccaccatgga ctggatggcg      60
caggagcagg agcgtggtat caccatcacc tccgcggcga cgactgcctt ctggtctggc     120
atggccaagc agtatgaacc acatcgcatc aacatcatcg acaccccggg gcacgttgac     180
ttcaccatcg aagttgaacg ttccatgcgt gtactggatg gcgcggtaat ggtttactgt     240
gcggtaggtg gtgttcagcc gcagtctgaa accgtatggc gtcaggctaa caaatatcat     300
gttccgcgca tcgcgttcgt taacaaaatg gaccgtatgg gcgcgaactt cctgaaagtg     360
gtaaaccaga tcaaaacccg tctgggtgcg aatccggttc cacttcagct ggctatcggt     420
gctgaagagc acttcactgg tgttgttgac ctggtgaaaa tgaaagccat caactggaat     480
gaagctgacc acggtatcac cttcgactac gaagaaattc cagctgatat gcaggaactg     540
gctcaggaat ggcaccagaa cctgatcgag tctgctgctg aagcttccga agagctgatg     600
gataaatatc tcggcggtga agaactgacc gaa                                  633

<210>  348
<211>  633
<212>  DNA
<213>  Brenneria rubrifaciens

<400>  348
ggtgtaaacc ataagatcgg tgaagttcat gacggcgcgg ccaccatgga ctggatggcg      60
caggagcagg agcgtggtat cactatcacc tcagctgcga ctaccgcttt ttggtctggt     120
atggctaaac agtttgagcc acaccgcgtt aacatcatcg acaccccagg acacgttgac     180
ttcactatcg aagtggagcg ttctatgcgt gtactcgacg gtgcagtaat ggtttactgc     240
gcggttggtg gtgttcagcc tcagtctgaa acggtctggc gtcaggctaa caaatacaaa     300
gttccgcgta ttgcgttcgt taacaaaatg gaccgtatgg gcgcgaactt cctgaaagtc     360
gtaagtcaga tcaaaacccg tttgggtgcg aatcctgttc ctttgcagct ggctatcggc     420
gcggaagaca gcttcaccgg cgttgtcgac ttggtgaaaa tgaaagccat caactggaat     480
gacgccgatc agggcgttac cttcatttat gaagagattc cggctgaaat gcaggatctg     540
gctgaagaat ggcatcagaa cctcattgag tctgcggctg aagcaaacga agagctgatg     600
gaaaaatatc tcggcggtga agaactgtcc gag                                  633

<210>  349
<211>  633
<212>  DNA
<213>  Brenneria salicis T

<400>  349
ggtgtaaacc acaaaatcgg tgaagttcat gacggcgcgg ccaccatgga ctggatggcg      60
caggagcagg aacgtggtat cactatcacc tccgctgcta ccaccgcttt ctggtctggt     120
atggccaagc agtttgagcc gcaccgcgtt aacatcatcg ataccccggg acacgttgac     180
ttcaccatcg aagtagaacg ttccatgcgt gttcttgacg gcgcagtaat ggtttactgt     240

gcggttggtg gtgttcagcc ccagtctgaa accgtatggc gccaggctaa caaatacaaa 300

gttccgcgta ttgcgttcgt taacaaaatg gaccgtatgg gcgcgaactt cctgaaagtc 360

gtgggacaga tcaaaacccg tctgggtgcg aaccctgttc cgctgcaatt ggcaatcggc 420

gcggaagaca gcttcactgg tgttgtcgac ctggtgaaga tgaaagccat caactggaat 480

gacgccgatc agggcgtgac cttcacttat gaagagattc cggctgatat gcaggatctt 540

gctgaggaat ggcaccagaa tctgatcgag tccgcggctg aagcaaacga agaactgatg 600

gaaaaatatc tcggcggtga agaactgtcc gag 633

<210> 350
<211> 633
<212> DNA
<213> Budvicia aquatica T

<400> 350
ggtgtaaatc ataaaatcgg tgagactcac gaaggttcag caacaatgga ctggatggag 60

caagaacaag aacgtggtat taccattacc tctgcagcaa caactgcatt ctggtctggt 120

atggcgaagc agtttgatgc tcaccgcgta aatatcatcg acaccccagg acacgttgac 180

ttcaccatcg aagtagaacg ttcaatgcgc gtgttggatg gtgccgtaat ggtttattgt 240

gctgttggcg gcgttcagcc acagtcagaa acagtatggc gtcaggctaa taaatatcat 300

gtaccgcgta tcgcgttcgt taataaaatg gaccgtatgg gtgcaaactt cctgcgtgtt 360

gtcgagcagt tgaagacgcg tttagccgct aatgctgttc caattcagct gcctatcggt 420

gctgaagaga aatttaccgg cattgttgac ttgctgaaaa tgaaagcaat caactggagc 480

gaagaagatc agggcgtttc atttacttat gaagacgttc ctgctgacat gctggaagaa 540

gctaaaaaat ggcagcagtt tgcttttgaa tctgcagctg aagcatcaga agagctgatg 600

gataagtatt taggcggcga agagctgact gaa 633

<210> 351
<211> 633
<212> DNA
<213> Buttiauxella agrestis T

<400> 351
ggtgtaaacc acaaaatcgg tgaagttcat gatggcgcag ccactatgga ctggatggag 60

caggagcagg aacgtggtat taccatcacg tctgctgcga ccacctgttt ctggtctggt 120

atggccaaac agttcgaacc acaccacatc aacatcatcg acaccccagg gcacgttgac 180

ttcaccatcg aagttgaacg ttccatgcgt gttcttgatg gtgcggtaat ggtttactgt 240

gctgttggtg gtgttcagcc acagtctgag accgtatggc gtcaggcaaa caaatataaa 300

gttccacgta tcgcgttcgt taacaaaatg gaccgtatgg gtgctaactt cctgaaagtt 360

gttgatcaga ttgaaaaacg tctgggcgca actccggtgc ctctgcaatt ggctatcggc 420

gcggaagaga aattcaccgg tgttgttgac ctggtgaaaa tgaaagccat caactggaac 480

gaagccgatc agggcgtgac cttcgaatac gaagagatcc cagctgatat gcaggaactg 540

gccgaagaat ggcgccagaa actggttgaa gccgctgctg aaggttctga cgaactgatg 600

gagaaattct ttggtggcga agagctgact gaa 633

<210> 352
<211> 633
<212> DNA
<213> Buttiauxella brennerae

<400> 352
ggtgtaaacc acaaaatcgg tgaagttcat gatggcgcag ccactatgga ctggatggag 60

caggagcagg aacgtggtat taccatcacg tctgctgcga ccacctgttt ctggtctggt 120

atggccaaac agttcgaacc acaccacatc aacatcatcg acaccccagg gcacgttgac 180

ttcaccatcg aagttgaacg ttccatgcgt gttcttgatg gtgcggtaat ggtttactgt 240

gctgttggtg gtgttcagcc acagtctgag accgtatggc gtcaggcaaa caaatataaa 300

gttccacgta tcgcgttcgt taacaaaatg gaccgtatgg gtgctaactt cctgaaagtt 360

gttgaccaga tcgaaaaacg tctgggcgca actccggtgc ctttgcaatt ggcaatcggc 420

gcggaagaga aattcaccgg tgttgttgac ctggtgaaaa tgaaagccat caactggaat 480

gaagccgatc agggcgtgac cttcgaatac gaagagatcc cagctgatat gcaggaactg 540

gccgaagaat ggcgccagaa actggttgaa gccgctgctg aaggttctga cgaactgatg 600

gagaaattct ttggtggcga agagctgact gaa 633

<210> 353
<211> 633
<212> DNA
<213> Buttiauxella ferragutiae

<400> 353
ggtgtaaacc acaaaatcgg tgaagttcat gatggcgcag ccaccatgga ctggatggag 60

caggagcagg aacgtggtat taccatcacg tccgctgcga ccacctgttt ctggtcaggt 120

atggctaaac agttcgaacc acaccacatc aacatcattg ataccccagg gcacgttgac 180

ttcaccatcg aagttgaacg ttccatgcgt gttcttgatg gtgcggtaat ggtttactgt 240

gctgttggtg gtgttcagcc acagtctgag accgtatggc gtcaggcaaa caaatataaa 300

gttccacgta tcgcgttcgt taacaaaatg gaccgtatgg gtgctaactt cctgaaagtt 360

gttgatcaga ttgaaaaacg tctgggcgca actccggtgc ctctgcaact ggcaatcggc 420

gcggaagaga aattcaccgg tgttgttgac ctggtgaaaa tgaaagccat caactggaac 480

gaagccgatc agggcgtgac cttcgaatac gaagagatcc cagctgatat gcaggaactg 540

gccgaagaat ggcgccagaa actggttgaa gccgctgctg aaggttctga cgaactgatg 600

gagaaattct ttggtggcga agagctgact gaa 633

<210> 354
<211> 633
<212> DNA
<213> Buttiauxella gaviniae

<400>  354
ggtgtaaacc acaaaatcgg tgaagttcat gatggcgcag ccactatgga ctggatggag      60

caggagcagg aacgtggtat taccatcacg tctgctgcga ccacctgttt ctggtctggt     120

atggccaaac agttcgaacc acaccacatc aacatcatcg acaccccagg gcacgttgac     180

ttcaccatcg aagttgaacg ttccatgcgt gttcttgatg gtgcggtaat ggtttactgt     240

gctgttggtg gtgttcagcc acagtctgag accgtatggc gtcaggcaaa caaatataaa     300

gttccacgta tcgcgttcgt taacaaaatg gaccgtatgg gtgctaactt cctgaaagtt     360

gttgaccaga tcgaaaaacg tctgggcgca actccggtgc ctctgcaact ggcaatcggc     420

gcggaagaga aattcaccgg tgttgttgac ctggtgaaaa tgaaagccat caactggaat     480

gaagccgatc agggcgtgac cttcgaatac gaagagatcc cagctgatat gcaggaactg     540

gccgaagaat ggcgccagaa actggttgaa gccgctgctg aaggttctga cgaactgatg     600

gagaaattct ttggtggcga agagctgact gaa                                  633

<210>  355
<211>  633
<212>  DNA
<213>  Buttiauxella izardii

<400>  355
ggtgtaaacc acaaaatcgg tgaagttcat gatggcgcag ccaccatgga ctggatggag      60

caggagcagg aacgtggtat taccatcacg tccgctgcga ccacctgttt ctggtcaggt     120

atggctaaac agttcgaacc acaccacatc aacatcatcg acaccccagg gcacgttgac     180

ttcaccatcg aagttgaacg ttcaatgcgt gttcttgatg gtgcggtaat ggtttactgt     240

gctgttggtg gtgttcagcc acagtctgaa accgtatggc gtcaggcaaa caaatataaa     300

gttccacgta tcgcgttcgt taacaaaatg gaccgtatgg gtgctaactt cctgaaagtt     360

gttgatcaga ttgaaaaacg tctgggcgca actccggtgc cactacaatt ggcaatcggc     420

gcggaagaga aattcaccgg tgttgttgac ctggtgaaaa tgaaagccat caactggaac     480

gaagccgatc agggcgtgac cttcgaatac gaagagatcc cagctgatat gcaggaactg     540

gccgaagaat ggcgccagaa actggttgaa gccgctgctg aaggttctga cgaactgatg     600

gagaaattct ttggtggcga agagctgact gaa                                  633

<210>  356
<211>  633
<212>  DNA
<213>  Buttiauxella noackiae

<400>  356
ggtgtaaacc ataaaatcgg tgaagttcat gatggcgctg ctaccatgga ctggatggaa      60

caggagcagg aacgtggtat caccattact tctgctgcaa ctactgcgtt ctggtcaggt     120

atggctaagc agtatgaacc gcatcgcgta aacatcatcg acacccctgg gcacgttgac     180

ttcaccatcg aagtagaacg ttccatgcgt gttcttgacg gtgcggtaat ggtttactgc     240

gcagttggtg gtgttcagcc acagtctgag accgtatggc gtcaggctaa caaatacaaa     300

gtgccacgca ttgcgttcgt taacaaaatg gaccgcatgg gtgctaactt cctgaaagtt 360

gtaggccaga ttaaatcccg cttgggcgcg aacgcagttc cgcttcagtt ggcgattggc 420

gctgaagaag gtttcaccgg tgttgttgac ctggtgaaaa tgaaagcaat caactggaac 480

gatgccgatc agggtgtgac cttcgaatac gaagaaatcc ctgctgatat ggctgaactg 540

gctgctgaat ggcgccagaa cttggttgag tctgcagcag aagcttctga agagctgatg 600

gacaaatact tgggtggcga agagctgacc gag 633

<210> 357
<211> 633
<212> DNA
<213> Buttiauxella warmboldiae

<400> 357
ggtgtaaacc acaaaatcgg tgaagttcat gatggcgcag ccaccatgga ctggatggag 60

caggagcagg aacgtggtat taccatcacg tccgctgcga ccacctgttt ctggtcaggt 120

atggccaaac agttcgaacc acaccacatc aacatcatcg acaccccagg gcacgttgac 180

ttcaccatcg aagttgaacg ttccatgcgt gtgcttgacg gtgcggtaat ggtttactgt 240

gctgttggtg gtgttcagcc acagtctgag accgtatggc gtcaggctaa caaatacaaa 300

gttccacgta tcgcgttcgt taacaaaatg gaccgtatgg gtgctaactt cctgaaagtt 360

gttgatcaga ttgaaaaacg tctgggcgca actccggtgc ctctgcaatt ggcaatcggc 420

gcggaagaga aattcaccgg tgttgttgac ctggtgaaaa tgaaagccat caactggaac 480

gaagccgatc agggcgtgac cttcgaatac gaagagatcc cagctgatat gcaggaactg 540

gccgaagaat ggcgccagaa actggttgaa gccgctgctg aaggttctga cgaactgatg 600

gagaaattct ttggtggcga agagctgact gaa 633

<210> 358
<211> 633
<212> DNA
<213> Cedecea davisae T

<400> 358
ggtgtaaacc acaaaatcgg tgaagttcat gacggcgctg caaccatgga ctggatggag 60

caggagcagg agcgtggtat taccattacc tccgctgcaa ctactgcatt ctggtctggt 120

atggctaagc agtatgaacc gcaccgcgta aacatcatcg acaccccagg gcacgttgac 180

ttcaccatcg aagttgaacg ttccatgcgt gttcttgacg gtgcagtaat ggtttactgc 240

gcagttggtg gtgttcagcc acagtctgaa accgtatggc gtcaggctaa caaatacaaa 300

gttccacgca tcgcgttcgt taacaaaatg gaccgtatgg gtgcaaactt cctgaaagtt 360

gtaggccaga tcaaatcccg cctgggcgcg aaccctgttc cgcttcagct ggcaattggt 420

gctgaagaag gtttcaccgg cgttatcgac ctggtgaaaa tgaaagccat caactggaac 480

gatgcagatc agggcgttac cttcgaatac gaagatatcc cggctgacat gcaggacctg 540

gctgacgaat ggcaccagaa cctgatcgag tccgctgcag aagcttctga agagctgatg 600

gaaaaatacc tgggcggcga agagctgacc gag 633

<210> 359
<211> 633
<212> DNA
<213> Cedecea lapagei

<400> 359
ggtgtaaacc acaaaatcgg tgaagttcac gacggcgctg caaccatgga ctggatggag 60

caggagcagg agcgtggtat caccatcacc tccgctgcaa ctactgcatt ctggtctggt 120

atggctaagc agtatgaacc gcaccgcgta aacatcatcg acaccccagg gcacgttgac 180

ttcaccatcg aagtagaacg ttccatgcgt gttcttgacg gtgcagtaat ggtttactgc 240

gcagttggtg gtgttcagcc acagtctgaa accgtatggc gtcaggctaa caaatacaaa 300

gttccacgca tcgcgttcgt taacaaaatg gaccgtatgg gtgctaactt cctgaaagtt 360

gtaggccaga tcaaatcccg cctgggcgcg aaccctgttc cgcttcagct ggcgattggt 420

gctgaagaag gtttcaccgg cgttatcgac ctggtgaaaa tgaaagccat caactggaac 480

gatgccgatc agggcgttac cttcgaatac gaagatatcc cggctgacat gcaggacctg 540

gctgacgaat ggcaccagaa cctgattgag tccgctgcag aagcctctga agagctgatg 600

gaaaaatacc tgggcggcga agagctgacc gag 633

<210> 360
<211> 633
<212> DNA
<213> Cedecea neteri

<400> 360
ggtgtaaacc acaaaatcgg tgaagttcat gacggtgctg caaccatgga ctggatggag 60

caggagcagg agcgtggtat taccattacc tccgctgcaa ctactgcatt ctggtctggt 120

atggctaagc agtatgaacc gcaccgcgta aacatcatcg acaccccagg gcacgttgac 180

ttcaccatcg aagttgaacg ttccatgcgt gttcttgacg gtgcagtaat ggtttactgc 240

gcagttggtg gtgttcagcc acagtctgaa accgtatggc gtcaggctaa caaatacaaa 300

gttccacgca tcgcgttcgt taacaaaatg gaccgtatgg gtgcaaactt cctgaaagtt 360

gtaggccaga tcaaatcccg cctgggcgcg aacgctgttc cgcttcagct ggcgattggt 420

gctgaagaag gtttcaccgg cgttatcgac ctggtgaaaa tgaaagccat caactggaac 480

gatgcagatc agggcgttac cttcgaatac gaagatatcc cagctgacat gcaggacctg 540

gctgacgaat ggcaccagaa cctgatcgag tccgctgcag aagcctctga agagctgatg 600

gaaaaatacc tgggcggcga agagctgacc gag 633

<210> 361
<211> 633
<212> DNA
<213> Citrobacter amalonaticus

<400> 361

ggtgtaaacc ataaaatcgg tgaagttcat gacggcgcag ccaccatgga ctggatggag 60

caggagcagg aacgtggtat tactatcact tccgcagcga ctactgcatt ctggtctggt 120

atggctaagc agtatgaacc gcatcgcgta aacatcatcg acaccccggg gcacgttgac 180

ttcactatcg aagtagaacg ttccatgcgt gttcttgatg gtgcggtaat ggtttactgc 240

gcagttggtg gtgttcagcc gcagtctgaa accgtatggc gtcaggcaaa caaatataaa 300

gttccacgca ttgcgttcgt taacaaaatg gaccgtatgg gtgcgaactt cctgaaagtt 360

gttggtcaga tcaaaacccg tctgggcgcg aaccctgttc cgctgcagct ggcgattggc 420

gctgaagaag cgttcaccgg tgttgttgac ctggtgaaaa tgaaagccat caactggaac 480

gaagcagatg caggcgttac tttcacttac gaagatatcc cggctgacat gcaggaactg 540

gctgaagaat ggcaccagaa cctgatcgag tccgcagcgg aagcttctga agagctgatg 600

gagaaatacc tgggtggtga agaactgact gaa 633

<210> 362
<211> 633
<212> DNA
<213> Citrobacter braakii

<400> 362
ggtgtaaacc ataaaatcgg tgaagttcat gacggcgcag ccaccatgga ctggatggaa 60

caggagcagg agcgtggtat tactatcacc tccgcagcga ctactgcatt ctggtctggt 120

atggctaagc agtatgaacc gcatcgcgta aacatcatcg acaccccggg gcacgttgac 180

ttcactatcg aagtagaacg ttccatgcgt gtgcttgatg gtgcagtaat ggtttactgc 240

gcagttggtg gtgttcagcc tcagtctgaa accgtatggc gtcaggcaaa caaatataaa 300

gttccacgca ttgcgttcgt taacaaaatg gaccgtatgg gtgcgaactt cctgaaagtt 360

gttggtcaga tcaaaacccg tctgggcgcg aaccctgttc cgctgcagtt ggcgattggc 420

gctgaagaag cgttcaccgg tgttgttgac ctggtgaaaa tgaaagccat caactggaac 480

gaagaagatg caggcgtaac cttcacttat gaagatatcc cggctgacat gcaggaactg 540

gctgaagaat ggcaccagaa cctgatcgag tccgctgcgg aagcttcaga agagctgatg 600

gagaaatacc tgggtggtga agaactgact gaa 633

<210> 363
<211> 633
<212> DNA
<213> Citrobacter diversus

<400> 363
ggtgtaaacc ataaaatcgg tgaagttcat gacggcgcag ctaccatgga ctggatggag 60

caggagcagg agcgtggtat taccatcacc tccgcagcga ctactgcatt ctggtctggt 120

atggctaagc agtatgaacc gcatcgcgta aacatcatcg acaccccggg gcacgttgac 180

ttcactatcg aagtagaacg ttccatgcgt gttcttgatg gtgcagtaat ggtttactgc 240

gcagttggtg gtgttcagcc gcagtctgaa accgtatggc gtcaggcaaa caaatataaa 300

gttccgcgca ttgcgttcgt taacaaaatg gaccgtatgg gtgcgaactt cctgaaagtt 360

gttggtcaga tcaaaagccg tctgggcgcg aaccctgttc cgctgcaact ggcgattggc 420

gctgaagaag cgttcaccgg tgttgttgac ctggtgaaaa tgaaagccat caactggaac 480

gaagcagatg caggcgttac cttcacttac gaagatatcc cggctgacat gcaggaactg 540

gctgaagaat ggcaccagaa cctgatcgag tccgctgcgg aagcttctga agagctgatg 600

gaaaaatacc tgggtggcga agaactgact gaa 633

<210> 364
<211> 633
<212> DNA
<213> Citrobacter farmeri

<400> 364
ggtgtaaacc ataaaatcgg tgaagttcat gacggcgcgg ccaccatgga ctggatggag 60

caggagcagg aacgtggtat tactatcact tccgcagcga ctactgcatt ctggtctggt 120

atggctaagc agtatgaacc gcatcgcgta aatatcatcg acaccccggg gcacgttgac 180

ttcacaatcg aagtagaacg ttccatgcgt gttcttgatg gtgcggtaat ggtttactgc 240

gcagttggtg gtgttcagcc gcagtctgaa accgtatggc gtcaggcaaa caaatataaa 300

gttccgcgca ttgcgttcgt taacaaaatg gaccgtatgg gtgcgaactt cctgaaagtt 360

gttggtcaga tcaaaacccg tctgggcgcg aaccctgttc cgctgcagct ggcaattggc 420

gctgaagaag cgttcaccgg tgttgttgac ctggtgaaaa tgaaagccat caactggaac 480

gaagcagatg caggcgttac cttcacttac gaagatatcc cggctgacat gcaggaactg 540

gctgaagaat ggcaccagaa cctgatcgag tccgctgcgg aagcttctga agagctgatg 600

gagaaatacc tgggtggtga agaactgact gaa 633

<210> 365
<211> 633
<212> DNA
<213> Citrobacter freundii T

<400> 365
ggtgtaaacc ataaaatcgg tgaagttcat gacggcgcag ccaccatgga ctggatggag 60

caggagcagg aacgtggtat tactatcacc tccgcagcga ctactgcatt ctggtctggt 120

atggctaagc agtatgaacc gcatcgcgta aacatcatcg acaccccggg gcacgttgac 180

ttcactatcg aagtagaacg ttccatgcgt gtgcttgatg gtgcagtaat ggtttactgc 240

gcagttggtg gtgttcagcc tcagtctgaa accgtatggc gtcaggcaaa caaatataaa 300

gttccgcgca ttgcgttcgt taacaaaatg gaccgtatgg gtgcgaactt cctgaaagtt 360

gttggtcaga tcaaaacccg tctgggcgcg aaccctgttc cgctgcagct ggcaattggc 420

gctgaagaag cgttcaccgg tgttgttgac ctggtgaaaa tgaaagccat caactggaac 480

gaagaagatg caggcgtaac cttcacttat gaagatatcc cggctgacat gcaggaactg 540

gctgaagaat ggcaccagaa cctgatcgag tccgctgcag aagcttcaga agagctgatg 600

gagaaatatc tgggtggtga agaactgact gaa 633

<210> 366
<211> 633
<212> DNA
<213> Citrobacter gillenii

<400> 366
ggtgtaaacc ataaaatcgg tgaagttcat gacggcgcag ccaccatgga ctggatggaa 60

caggagcagg agcgtggtat tactatcacc tccgcagcga ctactgcatt ctggtctggt 120

atggccaagc agtatgaacc gcatcgcgta aacatcatcg acaccccggg gcacgttgac 180

ttcacaatcg aagtagaacg ttccatgcgt gtgcttgatg gtgcagtaat ggtttactgc 240

gcagttggtg gtgttcagcc gcagtctgaa accgtatggc gtcaggctaa caaatataaa 300

gttccacgca ttgcgttcgt taacaaaatg gaccgtatgg gtgcgaactt cctgaaagtt 360

gttggtcaga tcaaaacccg tctgggcgcg aaccctgttc cgctgcagtt ggctattggt 420

gctgaagaac atttcaccgg tgttgttgac ctggtgaaaa tgaaagccat caactggaac 480

gaagaagacg caggcgtaac cttcacttat gaagatattc cggcagacat gcaggaactg 540

gctgaagaat ggcaccagaa cctgatcgag tccgctgcag aagcttcaga agagctgatg 600

gagaaatacc tcggtggtga agaactgact gaa 633

<210> 367
<211> 633
<212> DNA
<213> Citrobacter koseri

<400> 367
ggtgtaaacc ataaaatcgg tgaagttcat gacggcgcag ctaccatgga ctggatggag 60

caggagcagg agcgtggtat taccatcacc tccgcagcga ctactgcatt ctggtctggt 120

atggctaagc agtatgaacc gcatcgcgta aacatcatcg acaccccggg gcacgttgac 180

ttcactatcg aagtagaacg ttccatgcgt gttcttgatg gtgcagtaat ggtttactgc 240

gcagttggtg gtgttcagcc gcagtctgaa accgtatggc gtcaggcaaa caaatataaa 300

gttccgcgca ttgcgttcgt taacaaaatg gaccgtatgg gtgcgaactt cctgaaagtt 360

gttggtcaga tcaaaagccg tctgggcgcg aaccctgttc cgctgcaact ggcgattggc 420

gctgaagaag cgttcaccgg tgttgttgac ctggtgaaaa tgaaagccat caactggaac 480

gaagcagatg caggcgttac cttcacttac gaagatatcc cggctgacat gcaggaactg 540

gctgaagaat ggcaccagaa cctgatcgag tccgctgcgg aagcttctga agagctgatg 600

gaaaaatacc tgggtggcga agaactgact gaa 633

<210> 368
<211> 633
<212> DNA
<213> Citrobacter murliniae

<400> 368
ggtgtaaacc ataaaatcgg tgaagttcat gacggcgcag ccaccatgga ctggatggaa 60

caggagcagg agcgtggtat tactatcacc tccgcagcga ctactgcatt ctggtctggt 120

atggctaagc agtatgaacc gcatcgcgta aacatcatcg acaccccggg gcacgttgac 180

ttcactatcg aagtagaacg ttccatgcgt gtgcttgatg gtgcagtaat ggtttactgc 240

gcagttggtg gtgttcagcc gcagtctgaa accgtatggc gtcaggctaa caaatataaa 300

gttccgcgca ttgcgttcgt taacaaaatg gaccgtatgg gcgcgaactt cctgaaagtt 360

gttggtcaga tcaaaacccg tctgggcgcg aaccctgttc cgctgcagct ggcaattggt 420

gctgaagaaa acttcactgg tgttgttgac ctggtgaaaa tgaaagccat caactggaac 480

gaagaagatg caggcgtaac cttcacttat gaagatatcc cggctgacat gcaggaactg 540

gctgaagaat ggcaccagaa cctgatcgag tccgccgcgg aagcttcaga agagctgatg 600

gagaaatacc tgggtggtga agaactgact gaa 633

<210> 369
<211> 633
<212> DNA
<213> Citrobacter rodentium

<400> 369
ggtgtaaacc ataaaatcgg tgaagttcat gacggcgcgg ctaccatgga ctggatggag 60

caggagcagg aacgtggtat taccatcact tccgcagcga ctactgcatt ctggtctggt 120

atggctaagc agtatgaacc gcatcgcgta aacatcatcg acaccccggg gcacgttgac 180

ttcaccatcg aagtagaacg ttccatgcgt gttcttgatg gtgcggtaat ggtttactgc 240

gcagttggtg gtgttcagcc gcagtctgaa accgtatggc gtcaggcaaa caaatataaa 300

gttccgcgca ttgcgttcgt taacaaaatg gaccgcatgg gcgcgaactt cctgaaagtt 360

gttggtcaga tcaaatcccg tctgggcgcg aaccctgttc cgctgcagct ggcgattggc 420

gctgaagaag cgttcaccgg tgttgttgac ctggtgaaaa tgaaagccat caactggaat 480

gaagcagatg ctggcgtaac cttcacctat gaagacatcc cggctgacat gcaggaactg 540

gctgaagaat ggcaccagaa cctgatcgag tccgcagcgg aagcgtctga agagctgatg 600

gaaaaatacc tgggtggtga agaactgact gaa 633

<210> 370
<211> 633
<212> DNA
<213> Citrobacter sedlakii

<400> 370
ggtgtaaacc ataaaatcgg tgaagttcat gacggcgcag ctaccatgga ctggatggag 60

caggagcagg aacgtggtat taccatcact tccgcagcga ctactgcatt ctggtctggt 120

atggctaagc agtatgaacc gcatcgcgta aacatcatcg acaccccggg gcacgttgac 180

ttcaccatcg aagtagaacg ttccatgcgt gttcttgatg gtgcggtaat ggtttactgc 240

gcagttggtg gtgttcagcc gcagtctgaa accgtatggc gtcaggcaaa caaatataaa 300

gttccgcgca ttgcgttcgt taacaaaatg gaccgtatgg gcgcgaactt cctgaaagtt 360

gttggtcaga tcaaaacccg tctgggcgcg aaccctgttc cgctgcagct ggcaattggc 420

gctgaagaag cgttcaccgg tgttgttgac ctggtgaaaa tgaaagccat caactggaac 480

gaagctgatg caggcgtgac cttcacctat gaagacatcc cggctgacat gcaggaactg 540

gctgaagaat ggcaccagaa cctgatcgag tccgcagcgg aagcgtctga agagctgatg 600

gaaaaatacc tgggtggtga agaactgact gaa 633

<210> 371
<211> 633
<212> DNA
<213> Citrobacter werkmanii

<400> 371
ggtgtaaacc ataaaatcgg tgaagttcat gacggcgctg ccaccatgga ctggatggag 60

caggagcagg agcgtggtat tactatcacc tccgcagcga ctactgcatt ctggtctggt 120

atggctaagc agtatgaacc gcatcgcgta aacatcatcg acaccccggg gcacgttgac 180

ttcactatcg aagtagaacg ttccatgcgt gtgcttgatg gtgcagtaat ggtttactgc 240

gcagttggtg gtgttcagcc tcagtctgaa accgtatggc gtcaggcaaa caaatataaa 300

gttccgcgca ttgcgttcgt taacaaaatg gaccgtatgg gtgcgaactt cctgaaagtt 360

gttggtcaga tcaaaacccg tctgggcgcg aaccctgttc cgctgcagct ggcgattggc 420

gctgaagaag cgttcaccgg tgttgttgac ctggtgaaaa tgaaagccat caactggaac 480

gaagaagatg caggcgtaac cttcacttat gaagatatcc cggctgacat gcaggaactg 540

gctgacgaat ggcaccagaa cctgatcgag tccgctgcag aagcttcaga agagctgatg 600

gagaaatacc tgggtggtga agaactgact gaa 633

<210> 372
<211> 633
<212> DNA
<213> Citrobacter youngae

<400> 372
ggtgtaaacc ataaaatcgg tgaagttcat gacggcgcag ccaccatgga ctggatggag 60

caggagcagg agcgtggtat tactatcacc tccgcagcga ctactgcatt ctggtctggt 120

atggctaagc agtacgaacc gcatcgcgta aacatcatcg acaccccggg gcacgttgac 180

ttcacaatcg aagtagaacg ttccatgcgt gtgcttgatg gtgcagtaat ggtttactgc 240

gcagttggtg gtgttcagcc tcagtctgaa accgtatggc gtcaggcaaa caaatataaa 300

gttccgcgca ttgcgttcgt taacaaaatg gaccgtatgg gtgcgaactt cctgaaagtt 360

gttggtcaga tcaaaacccg tctgggcgcg aaccctgttc cgctgcagct ggcaattggc 420

gctgaagaag cgttcaccgg tgttgttgac ctggtgaaaa tgaaagccat caactggaac 480

gaagaagatg caggcgtaac cttcacttat gaagatatcc cggctgacat gcaggaactg 540

gctgaagaat ggcaccagaa cctgatcgag tccgctgcgg aagcttcaga agagctgatg 600

gagaaatacc tgggtggtga agaactgact gaa 633

<210> 373
<211> 633
<212> DNA
<213> Dickeya chrysanthemi T

<400> 373
ggtgtgaacc acaaaatcgg tgaagttcat gatggcgccg ctactatgga ctggatggag 60

caggagcagg agcgtggtat tactattacc tccgctgcga ccactgcatt ttggtctggt 120

atggctaagc agtatgaacc gcatcgcatc aacatcatcg acaccccggg acacgttgac 180

ttcaccatcg aagtagaacg ttccatgcgt gttctggatg gcgcagtcat ggtttactgt 240

gcggtaggtg gtgttcagcc gcagtctgaa accgtatggc gtcaggcaaa caaatataaa 300

gttccgcgta ttgcgttcgt taacaaaatg gaccgtatgg gcgcgaactt cctgaaagtc 360

gttgatcaga tcaaagcacg tctgggcgcg aacccggttc cgttgcaact ggctatcggt 420

gctgaagaag cattcaccgg tgtcgttgac ctggtgaaaa tgaaagccat caactggaat 480

gaagcagacc agggcgttac cttcacttat gaagatatcc cggctgacat gcaggatctg 540

gccgctgaat ggcaccagaa tctgatcgaa tccgcagctg aagcctctga agagctgatg 600

gaaaaatacc tgggcggtga agatctgacc gag 633

<210> 374
<211> 633
<212> DNA
<213> Edwardsiella hoshinae

<400> 374
ggtgtaaacc ataagatcgg tgaagttcat gatggtgctg ccaccatgga ctggatggag 60

caggagcagg agcgtggtat caccattacc tccgctgcga ccaccgcttt ctggtctggc 120

atggccaaac agttcgaacc gcaccgtatc aacatcatcg acaccccggg gcacgttgac 180

ttcaccatcg aagtagaacg ttccatgcgt gtgctggatg gtgcggtcat ggtttactgc 240

gccgttggtg gtgttcagcc gcagtctgaa accgtatggc gtcaggccaa caagtatcat 300

gttccgcgca tcgcgttcgt taacaaaatg gaccgcatgg gtgcgaactt cctgaaagtt 360

gttagccaga tcaaaacccg tctgggtgct aacccggttc cgttgcagtt ggcaatcggt 420

gcggaagacg cattcaccgg cgttgttgac ctggttaaaa tgaaagccat caactggaac 480

gaagccgatc agggcgtgac cttcacctat gaagacatcc cggctgacat ggttgaactg 540

gctaacgaat ggcaccagtt cctggttgag tctgccgctg aagcttctga agagctgatg 600

gacaaatacc tgggcggtga agcgctgtct gaa 633

<210> 375
<211> 633
<212> DNA
<213> Edwardsiella ictaluri

<400> 375
ggtgtaaacc ataagatcgg tgaagttcat gatggtgctg ccaccatgga ctggatggag 60

caggagcagg agcgtggtat caccattacc tccgctgcga ccaccgcttt ctggtctggc 120

atggccaaac agtttgagcc gcaccgtatc aacatcatcg ataccccggg gcacgttgac 180

ttcaccatcg aagtagaacg ttccatgcgt gtactggatg gtgcggtcat ggtttactgc 240

gccgttggtg gtgttcagcc gcagtctgaa accgtatggc gtcaggccaa caagtatcat 300

gttccgcgca tcgcgttcgt taacaaaatg gaccgcatgg gtgcgaactt cctgaaagtt 360

gttaaccaga tcaaaacccg cctgggcgct aacccggttc cgttgcagtt ggcaatcggt 420

gctgaagaag cattcaccgg tatcgttgac ctggttaaaa tgaaagccat caactggaac 480

gaagccgatc agggcgtgac cttcacctat gaagacatcc cggctgacat ggttgaactg 540

gctaacgaat ggcaccagtt cctggttgag tccgcagccg aagcgtctga agagctgatg 600

gacaagtacc tgggcggtga aacgctgtct gaa 633

<210> 376
<211> 633
<212> DNA
<213> Edwardsiella tarda T

<400> 376
ggtgtaaacc ataagatcgg tgaagttcat gatggtgctg ccaccatgga ctggatggag 60

caggagcagg agcgtggtat caccattacc tccgctgcga ccaccgcttt ctggtctggc 120

atggccaaac agtttgagcc gcaccgtatc aacatcatcg ataccccggg gcacgttgac 180

ttcaccatcg aagtagaacg ttccatgcgt gtactggatg gtgcggtcat ggtttactgc 240

gccgttggtg gtgttcagcc gcagtctgaa accgtatggc gtcaggccaa caagtatcat 300

gttccgcgca tcgcgttcgt taacaaaatg gaccgcatgg gtgcgaactt cctgaaagtt 360

gttaaccaga tcaaaacccg tctgggtgct aacccggttc cgttgcagct ggcaatcggt 420

gctgaagaag ccttcaccgg tgttgttgac ctggttaaaa tgaaagccat caactggaac 480

gaagccgatc agggcgtgac cttcacctat gaagacatcc cggctgacat ggttgaactg 540

gctaacgaat ggcaccagtt cctggttgag tccgcagccg aagcgtctga agagctgatg 600

gacaagtacc tgggcggtga aacgctgtct gaa 633

<210> 377
<211> 633
<212> DNA
<213> Enterobacter aerogenes

<400> 377
ggtgtaaacc acaaaatcgg tgaagttcat gacggcgccg ctaccatgga ctggatggag 60

caggagcagg aacgtggtat caccatcact tccgctgcga ctactgcatt ctggtctggt 120

atggctaaac agtatgaacc gcaccgtgta aacatcatcg acaccccggg gcacgttgac 180

ttcactatcg aagtagaacg ttccatgcgt gttcttgatg gtgcggtaat ggtttactgc 240

gcagttggtg gtgttcagcc gcagtctgaa accgtatggc gtcaggcaaa caaatataaa 300

gttccacgca ttgcgttcgt taacaaaatg gaccgcatgg gtgcgaactt cctgaaagtt 360

gttggtcaga tcaaaacccg tctgggcgcg aacccggttc cgctgcagct ggcaattggt 420

gctgaagaag gtttcaccgg tgttgttgac ctggtgaaaa tgaaagccat caactggaac 480

gaagctgatg caggcgttac cttcgaatac gaagatatcc cggctgacat gcaggaactg 540

gctgacgaat ggcaccagaa cctgatcgag tccgctgcgg aagcttccga agagctgatg 600

gaaaaatacc tgggtggcga agaactgact gaa 633

<210> 378
<211> 633
<212> DNA
<213> Enterobacter amnigenus

<400> 378
ggtgtaaacc ataaaatcgg tgaagttcat gacggcgcag ctaccatgga ctggatggaa 60

caggagcagg agcgtggtat tactatcacc tccgcagcga ctactgcatt ctggtcaggt 120

atggctaagc agtacgaacc gcatcgcgta aatatcatcg acaccccagg ccacgttgac 180

ttcactatcg aagttgaacg ttctatgcgt gttctcgatg gtgcggtaat ggtttactgc 240

gcagttggtg gtgttcagcc acagtctgaa accgtatggc gtcaggcaaa caaatacaaa 300

gttccacgca ttgcgttcgt taacaaaatg gaccgcatgg gtgcaaactt cctgaaagtt 360

gttggtcaga tcaaaacccg tctgggcgcg acagctgttc cgcttcagtt ggcgattggt 420

gctgaagaag gtttcaccgg tgttgttgac ctggtgaaaa tgaaagccat caactggaac 480

gatgccgatc agggcgtgac cttcgaatac gaagacatcc ctgctgacat ggttgagctg 540

gcaaacgaat ggcgccagaa cctggttgaa tccgcagctg aagcttctga cgagctgatg 600

gataaatatt tgggcggcga agagctgacc gaa 633

<210> 379
<211> 633
<212> DNA
<213> Enterobacter asburiae

<400> 379
ggtgtaaacc ataaaatcgg tgaagttcat gacggcgcag ccaccatgga ctggatggaa 60

caggagcagg agcgtggtat cactatcacc tccgcagcga ctactgcatt ctggtcaggt 120

atggctaagc agtacgaacc gcatcgcgta aacatcatcg acaccccagg gcacgttgac 180

ttcaccatcg aagtagaacg ttccatgcgt gttcttgacg gcgcggtaat ggtttattgc 240

gcagttggtg gtgttcagcc acagtctgag accgtatggc gtcaggcgaa caaatataaa 300

gttccacgca tcgcgttcgt taacaaaatg gaccgtatgg gtgctaactt cctgaaagtt 360

gttggtcaga tcaaatcccg tctgggcgcg aaccctgttc cgctgcagct ggcaattggt 420

gctgaagaag gcttcaccgg cgttatcgac ctggtgaaaa tgaaagccat caactggaac 480

gatgcagacc agggcgttac cttcgaatac gaagatatcc cggctgaaat gcaggacctg 540

gctgacgaat ggcaccagaa cctgatcgaa tccgctgctg aagcttctga agagctgatg 600

gagaaatacc tgggtggtga agaactgact gaa 633

<210>  380
<211>  633
<212>  DNA
<213>  Enterobacter cancerogenus

<400>  380
ggtgtaaacc ataaaatcgg tgaagttcat gacggcgcag ccaccatgga ctggatggaa      60

caggagcagg agcgtggtat tactatcacc tccgcagcga ctactgcatt ctggtcaggt     120

atggctaagc agtacgaacc gcatcgcgta aacatcatcg acaccccagg gcacgttgac     180

ttcaccatcg aagtagaacg ttccatgcgt gttcttgacg gcgcagtaat ggtttattgc     240

gcagttggtg gtgttcagcc acagtctgaa accgtatggc gtcaggcgaa caaatataaa     300

gttccacgca tcgcgttcgt taacaaaatg gaccgtatgg gtgctaactt cctgaaagtt     360

gttggtcaga tcaaaacccg tctgggcgcg aaccctgttc cgctgcagct ggcaattggt     420

gctgaagaag ctttcaccgg cgttatcgac ctggtgaaaa tgaaagccat caactggaac     480

gaagaagacg caggcgttac cttcacttac gaagatatcc cagcagacat gcaggacctg     540

gctgacgaat ggcaccagaa cctgatcgag tccgcagcgg aagcttctga agagctgatg     600

gaaaaatacc tgggtggtga agaactgact gaa                                  633

<210>  381
<211>  633
<212>  DNA
<213>  Enterobacter cloacae sbsp. cloacae T

<400>  381
ggtgtaaacc ataaaatcgg tgaagttcat gacggcgcag ccaccatgga ctggatggaa      60

caggagcagg agcgtggtat tactatcacc tccgcagcga ctactgcatt ctggtcaggt     120

atggctaagc agtacgaacc gcatcgcgta aacatcatcg acacccctgg gcacgttgac     180

tttaccatcg aagtagaacg ttccatgcgt gttcttgacg gcgcggtaat ggtttattgc     240

gcagttggtg gtgttcagcc acagtctgaa accgtatggc gtcaggcgaa caaatataaa     300

gttccacgca tcgcgttcgt taacaaaatg gaccgtatgg gtgctaactt cctgaaagtt     360

gttggtcaga tcaaaacccg tctgggcgcg aaccctgttc cgctgcagct ggcaattggt     420

gctgaagaag gcttcaccgg cgttatcgac ctggtgaaaa tgaaagccat caactggaac     480

gatgcagacc agggcgttac cttcgaatac gaagatatcc cggctgagat gcaggacctg     540

gctgacgaat ggcaccagaa cctgatcgaa tccgctgctg aagcttctga agagctgatg     600

gagaaatacc tgggtggtga agaactgact gaa                                  633

<210>  382
<211>  633
<212>  DNA
<213>  Enterobacter cloacae sbsp. dissolvens

<400>  382
ggtgtaaacc acaaaatcgg tgaagttcat gacggcgcag ccaccatgga ctggatggaa      60

caggagcagg agcgtggtat tactatcacc tccgcagcga ctactgcatt ctggtcaggt     120

atggctaagc agtacgaacc gcatcgcgta aacatcatcg acaccccagg gcacgttgac 180

ttcaccatcg aagtagaacg ttccatgcgt gttcttgacg gcgcggtaat ggtttattgc 240

gcagttggtg gtgttcagcc acagtctgaa accgtatggc gtcaggcgaa caaatataaa 300

gttccacgca tcgcgttcgt taacaaaatg gaccgtatgg gtgctaactt cctgaaagtt 360

gttggtcaga tcaaaacccg tctgggcgcg aaccctgttc cgctgcagct ggcaattggt 420

gctgaagaag gcttcaccgg cgttatcgac ctggtgaaaa tgaaagccat caactggaac 480

gatgcagacc agggcgttac cttcgaatac gaagatatcc cggctgagat gcaggacctg 540

gctgacgaat ggcaccagaa cctgatcgaa tccgctgctg aagcttctga agagctgatg 600

gagaaatacc tgggtggtga agaactgact gaa 633

<210> 383
<211> 633
<212> DNA
<213> Enterobacter cowanii

<400> 383
ggtgtaaacc acaaaatcgg tgaagttcac gacggcgccg caaccatgga ctggatggag 60

caggagcagg agcgtggtat caccatcacc tctgcagcga ctactgcatt ctggtctggt 120

atggcgaagc agtatgaacc gcatcgcgta aacatcatcg acaccccggg gcacgttgac 180

ttcaccatcg aagtagaacg ttccatgcgt gtactggatg gtgcggtaat ggtttactgt 240

gccgttggtg gtgttcagcc acagtctgaa accgtatggc gtcaggctaa caaatataaa 300

gttccgcgca tcgcgttcgt taacaaaatg gaccgtatgg gcgctaactt cctgaaagtt 360

gttgatcaga tcaaaacccg tctgggcgcg aacccggttc cgctgcagct ggcaattggc 420

gctgaagaag gtttcaccgg tgttgttgac ctggtgaaaa tgaaagcgat caactggaac 480

gatgctgacc agggcgttac cttcgtttac gaagatatcc cggctgagat gcaggacctg 540

gctgacgaat ggcaccagaa cctgatcgaa tctgcggcgg aagcttcaga agagctgatg 600

gagaaatacc tgggtggtga agacctgact gaa 633

<210> 384
<211> 633
<212> DNA
<213> Enterobacter gergoviae

<400> 384
ggtgtaaacc ataagatcgg tgaagttcat gacggcgccg ctaccatgga ctggatggag 60

caggagcagg aacgtggtat caccatcact tccgctgcga ctactgcatt ctggtcaggt 120

atggctaaac agtatgaacc gcaccgcgta aacatcatcg acaccccggg gcacgttgac 180

ttcactatcg aagtagaacg ttccatgcgt gtgcttgatg gtgcggtaat ggtttactgc 240

gcagttggtg gtgttcagcc gcagtctgaa accgtatggc gtcaggcaaa caaatacaaa 300

gttccgcgta ttgcgttcgt taacaaaatg gaccgcatgg gcgcgaactt cctgaaagtt 360

gttggtcaga tcaaaacccg tctcggcgcg aacccggttc cgctgcagct ggcaattggt 420

gctgaagaag gtttcaccgg tgttgttgac ctggtgaaaa tgaaagccat caactggaac 480

gatgcagacc agggcgttac cttcgaatac gaagatatcc cggctgacat gcaggacctg 540

gctgaagaat ggcaccagaa cctgatcgaa tccgctgctg aagcttcaga agagctgatg 600

gagaaatacc tgggtggtga agaactgact gaa 633

<210> 385
<211> 633
<212> DNA
<213> Enterobacter hormaechei

<400> 385
ggtgtaaacc ataaaatcgg tgaagttcat gacggcgcag ccaccatgga ctggatggaa 60

caggagcagg agcgtggtat tactatcacc tccgcagcga ctactgcatt ctggtcaggt 120

atggctaagc agtacgaacc gcatcgcgta aacatcatcg acaccccagg gcacgttgac 180

ttcaccatcg aagtagaacg ttccatgcgt gttcttgacg gcgcggtaat ggtttattgc 240

gcagttggtg gtgttcagcc acagtctgaa accgtatggc gtcaggcaaa caaatataaa 300

gttccacgca tcgcgttcgt taacaaaatg gaccgtatgg gtgctaactt cctgaaagtt 360

gttggccaga tcaaaacccg tctgggcgcg aaccctgttc cgcttcagct ggcaattggt 420

gctgaagaag gcttcaccgg cgttatcgac ctggtgaaaa tgaaagccat caactggaac 480

gaagcagatg cgggcgttac cttcgaatac gaagatatcc cggcagagat gcaggacctg 540

gctgacgaat ggcaccagaa cctgatcgaa tccgcagcag aagcttctga agagctgatg 600

gagaaatacc tgggtggtga agaactgtct gag 633

<210> 386
<211> 633
<212> DNA
<213> Enterobacter kobei

<400> 386
ggtgtaaacc ataaaatcgg tgaagttcat gacggcgccg caaccatgga ctggatggag 60

caggagcagg agcgtggtat caccattacc tccgctgcga ctaccgcatt ctggtctggt 120

atggcgaagc agtatgaacc gcatcgcgta aacatcatcg acaccccggg tcacgttgac 180

ttcaccatcg aagtagaacg ttccatgcgt gttcttgacg gtgctgtaat ggtttactgc 240

gcagttggtg gtgttcagcc gcagtctgaa accgtatggc gtcaggcaaa caaatacaaa 300

gttccgcgta ttgcgttcgt taacaaaatg gaccgcatgg gcgctaactt cctgaaagtt 360

gttggtcaga tcaaatcccg tctgggcgcg aacccggttc cgctgcagct ggcgattggt 420

gctgaagaag gtttcaccgg tgttgttgac ctggtgaaaa tgaaagccat caactggaac 480

gatgccgacc agggcgtgac cttcgaatac gaagacatcc ctgcagacat ggctgacctg 540

gctgaagaat ggcaccagaa tctgatcgaa tctgctgctg aagcttctga agagctgatg 600

gaaaaatacc tgggcggcga agagctgacc gag 633

<210> 387
<211> 633
<212> DNA
<213> Enterobacter ludwigii

<400> 387
ggtgtaaacc ataaaatcgg tgaagttcat gacggcgcag ccaccatgga ctggatggaa 60

caggagcagg agcgtggtat tactatcacc tccgcagcga ctactgcatt ctggtcaggt 120

atggctaagc agtacgaacc gcatcgcgtt aacatcatcg acaccccagg gcacgttgac 180

ttcaccatcg aagtagaacg ttccatgcgt gttcttgacg gcgcggtaat ggtttattgc 240

gcagttggtg gtgttcagcc acagtctgag accgtatggc gtcaggcgaa caaatataaa 300

gttccacgca tcgcgttcgt taacaaaatg gaccgtatgg gtgctaactt cctgaaagtt 360

gttggtcaga tcaaatcccg tctgggcgcg aaccctgttc cgctgcagct ggcaattggt 420

gctgaagaag gcttcaccgg cgttatcgac ctggtgaaaa tgaaagccat caactggaac 480

gatgcagacc agggcgttac cttcgaatac gaagatatcc cagctgagat gcaggacctg 540

gctgacgaat ggcaccagaa cctgatcgaa tccgctgctg aagcttctga agagctgatg 600

gagaaatacc tgggtggtga agaactgact gaa 633

<210> 388
<211> 633
<212> DNA
<213> Enterobacter nimipressuralis

<400> 388
ggtgtaaacc ataaaatcgg tgaagttcat gacggcgcag ctaccatgga ctggatggaa 60

caggagcagg agcgtggtat taccatcacc tccgcagcga ctactgcatt ctggtcaggt 120

atggctaagc agtatgaacc gcatcgcgta aacatcatcg acacccgggg gcacgttgac 180

ttcactatcg aagttgaacg ttccatgcgt gttcttgatg gtgcagtaat ggtttactgc 240

gcagttggtg gtgttcagcc acagtctgaa accgtatggc gtcaggctaa taaatacaaa 300

gttccacgca tcgcgttcgt taacaaaatg gaccgtatgg gtgctaactt cctgaaagtt 360

gttggtcaga tcaaaacccg tctgggcgcg actccggttc cgcttcagct ggcaattggt 420

gctgaagaag gtttcaccgg tgttgttgac ctggtgaaaa tgaaagccat caactggaac 480

gaagaagatg caggcgtcac cttcgtttat gaagatattc cagcagacat gcaggacctg 540

gccgacgaat ggcaccagaa cctgatcgag tccgctgctg aagcttcaga agagctgatg 600

gagaaatacc tgggtggtga agaactgact gaa 633

<210> 389
<211> 633
<212> DNA
<213> Enterobacter pyrinus

<400> 389
ggtgtaaacc ataaaatcgg tgaagttcat gacggcgccg caaccatgga ctggatggag 60

caggagcagg agcgtggtat cactatcact tccgcagcga ctactgcatt ctggtcaggt 120

atggctaaac agtatgaacc gcaccgcgta aacatcatcg acaccccggg gcacgttgac 180

ttcactatcg aagtagaacg ttccatgcgt gtgcttgatg gtgcggtaat ggtttactgc 240

gcagtcggtg gtgttcagcc gcagtctgaa actgtatggc gtcaggcaaa caaatataaa 300

gttccgcgta ttgcgttcgt taacaaaatg gaccgcatgg gcgcgaactt cctgaaagtt 360

gttggtcaga tcaaatcccg tctgggcgcg aaccctgttc cgctgcagct ggcaattggt 420

gctgaagaag gtttcaccgg tgttgttgac ctggtgaaaa tgaaagccat caactggaac 480

gaagctgacg caggtgtaac cttcacctac gaagatattc cggctgacat gcaggacctg 540

gctgacgaat ggcaccagaa cctgatcgag tccgcagcgg aagcatctga agagctgatg 600

gaaaaatacc tcggtggtga tgaactgact gaa 633

<210> 390
<211> 633
<212> DNA
<213> Enterobacter radicincitans

<400> 390
ggtgtaaacc acaaaatcgg tgaagttcat gacggcgccg caaccatgga ctggatggag 60

caggagcagg agcgtggtat taccattacc tccgcagcaa ctactgcatt ctggtctggt 120

atggcgaagc agtatgaacc gcatcgcgta aacatcatcg acaccccggg gcacgttgac 180

ttcactatcg aagtagaacg ttccatgcgt gttcttgacg gtgcggtaat ggtttactgc 240

gcagttggtg gtgttcagcc gcagtctgaa accgtatggc gtcaggctaa caaatataaa 300

gttccgcgca tcgcgttcgt taacaaaatg gaccgtatgg gcgccaactt cctgaaagtt 360

gttggtcaga tcaaaacccg tctgggcgcg aacccggttc cgctgcagct ggcaattggt 420

gctgaagaag gtttcaccgg tgttgttgac ctggtgaaaa tgaaagccat caactggaac 480

gatgcagacc agggcgtaac cttcgaatac gaagatatcc cggctgacat gcaggatctg 540

gctgacgaat ggcaccagaa cctgatcgaa tccgctgctg aagcttctga agagctgatg 600

gagaaatacc tgggtggtga agaactgact gaa 633

<210> 391
<211> 633
<212> DNA
<213> Enterobacter sakazakii

<400> 391
ggtgtaaacc acaaaatcgg tgaagttcat gacggcgccg caactatgga ctggatggaa 60

caggagcagg agcgtggtat taccattacc tccgctgcga ctaccgcatt ctggtctggt 120

atggctaagc agtatgaacc gcaccgtatc aacatcatcg acaccccggg gcacgttgac 180

ttcaccatcg aagtagaacg ttccatgcgt gtccttgacg gtgcggtaat ggtttactgc 240

gcagttggtg gtgttcagcc gcagtctgaa accgtatggc gtcaggctaa caaatacaaa 300

gttccgcgta ttgcgttcgt taacaaaatg gaccgcatgg gcgcgaactt cctgaaagtt 360

gttggtcaga tcaaaacccg tctgggcgcg aacccggttc cgctgcagct ggcgattggt 420

gctgaagaag gtttcaccgg tgttgttgac ctggtgaaaa tgaaagccat caactggaac 480

gatgcagacc agggcgtaac cttcgaatac gaagatatcc cggctgacat gcaggacctg 540

gctgacgaat ggcaccagaa cctgatcgag tccgcagctg aagcttctga agagctgatg 600

gagaaatacc tgggtggtga agaactgact gaa 633

<210> 392
<211> 633
<212> DNA
<213> Erwinia amylovora T

<400> 392
ggtgtaaacc acaaaatcgg tgaagtgcat gatggcgcag ccaccatgga ctggatggcg 60

caggagcagg agcgtggcat caccattacc tccgcagcga ctaccgcttt ctggtctggt 120

atggctaagc agttcgagcc gcaccgcgtc aacattatcg acaccccagg acacgttgac 180

ttcaccatcg aagtagagcg ctcaatgcgt gtactcgatg gcgcagtgat ggtttactgt 240

gctgttggtg gcgtgcagcc acagtctgaa accgtatggc gtcaggctaa caaatacaaa 300

gttcctcgta tcgcgttcgt taacaaaatg gaccgtatgg gtgctaactt cctgaaagtt 360

gtcagccaga tcaaatctcg cctggcggct aacccggttc ccattcagtt ggcaatcggc 420

gcggaagaga aattcaccgg tgttgttgac ctggtgaaaa tgaaagccat caactggaac 480

gatgccgatc agggcgtgac cttcgaatac gaagagatcc cggctgatat gcaggaactg 540

gctgaagaat ggcaccagaa cctggttgaa tccgcagcag aagcctctga agagctgatg 600

gagaaatacc tgggtggtga agaactgact gaa 633

<210> 393
<211> 633
<212> DNA
<213> Erwinia aphidicola

<400> 393
ggtgtaaacc acaaaatcgg tgaagtacat gacggcgcag caaccatgga ctggatggag 60

caggaacagg aacgtggtat taccatcact tccgctgcga ccacctgtta ctggtctggt 120

atggctaagc agtttgaacc acaccacgta aacatcatcg acaccccagg acacgttgac 180

ttcaccatcg aagttgagcg ttctatgcgt gtgcttgatg gcgcggtaat ggtttactgt 240

gcagttggtg gcgttcagcc gcagtctgag accgtatggc gtcaggctaa caaatacaaa 300

gttccacgca ttgcgttcgt taacaaaatg gaccgcatgg gtgcaaactt cctgaaagtt 360

gttgaccaga tggaaaagcg cctgggtgca aacccggtgc cattgcagct ggctatcggc 420

gcagaagaga aattcaccgg tgttgttgac ctggtgaaaa tgaaagcgat caactggaac 480

gaagccgatc agggcgtcac cttcgaatac gaagatattc cagctgatat gcaggaactg 540

gccgaagaat ggcgtggcaa gctggttgaa gccgcagctg aaggctctga tgagctgatg 600

gagaagttct ttggcggcga agagctgact gaa 633

<210> 394

<211>  633
<212>  DNA
<213>  Erwinia billingiae

<400>  394
ggtgtaaacc acaaaatcgg tgaagtacat gacggcgcgg ccaccatgga ctggatggag      60

caggaacagg aacgtggtat taccatcact tccgctgcga ccacctgttt ctggtctggt     120

atggctaagc agtttgagcc acaccatgta aacatcatcg acaccccggg acacgttgac     180

ttcaccatcg aagtagaacg ttccatgcgt gtgcttgacg gcgcagtaat ggtttactgt     240

gcagttggtg gtgttcagcc acagtctgag accgtatggc gccaggctaa caaatataaa     300

gttccacgca tcgcgttcgt taacaaaatg gaccgcatgg gtgctaactt cctgaaagtt     360

gttgatcaga tgcaagtgcg cctgggtgca aacccagttc cattgcagct ggcaatcggc     420

gcagaagaga aattcaccgg tgttgttgac ctggtgaaaa tgaaagccat caactggaac     480

gatgccgatc agggcgtgac cttcgtttac gaagagatcc cagctgatat gcaggaactg     540

gctgatgaat ggcgcgccaa gctggtcgaa gccgctgctg aaggttctga cgagctgatg     600

gagaaattct ttggcggcga agagctgact gaa                                  633

<210>  395
<211>  633
<212>  DNA
<213>  Erwinia mallotivora

<400>  395
ggtgtaaacc acaaaatcgg tgaagttcat gacggcgcag ccaccatgga ctggatggcg      60

caggagcagg agcgtggtat tactatcacc tccgcagcga ctacggcttt ctggtctggt     120

atggctaagc agttcgagcc gcaccgcgta aacatcatcg acacacccgg acacgttgac     180

ttcaccatcg aagttgagcg ttctatgcgt gtcctcgacg gcgcagtaat ggtttactgt     240

gcggttggtg gtgttcagcc acagtctgag actgtatggc gtcaggcaaa caaatacaaa     300

gttccacgta tcgcgttcgt taacaaaatg gaccgtatgg gtgcgaactt cctgaaagtg     360

gttggccaga ttaaatcccg tctgtgcgct aaccctgttc cattgcagtt ggcaattggt     420

gcagaagaaa aattcactgg cgtcgttgac ttggtgaaaa tgaaagcaat caactggaac     480

gacgccgatc agggcgtaac cttcgaatac gaagatattc cagctgagat gcaggcgctg     540

gctgaagaat ggcaccagaa cctgattgaa tccgcagctg aagcgtctga agagctgatg     600

gagaaatatc tgggcggtga agaactgact gaa                                  633

<210>  396
<211>  633
<212>  DNA
<213>  Erwinia papayae

<400>  396
ggtgtaaacc acaaaatcgg tgaagttcat gacggcgcag ccaccatgga ctggatggcg      60

caggagcagg agcgtggtat tactatcacc tccgcagcga ctacggcttt ctggtctggt     120

atggctaagc agttcgagcc gcaccgcgta aacatcatcg acacccccgg acacgttgac     180

ttcaccatcg aagttgagcg ttctatgcgt gttctcgacg gcgcagtaat ggtttactgt 240

gcggttggtg gtgttcagcc acagtctgag actgtatggc gtcaggcaaa caaatacaaa 300

gttccacgta tcgcgttcgt taacaaaatg gaccgtatgg gtgcgaactt cctgaaagtg 360

gttggtcaga ttaaatcccg tctgggcgct aaccctgttc cattgcagtt ggcaattggt 420

gcagaagaaa aattcaccgg cgtcgttgac ctggtgaaaa tgaaagcaat caactggaac 480

gacgccgacc agggcgtaac cttcgaatac gaagatattc cagctgatat gcaggcgctg 540

gctgaagaat ggcaccagaa cctgattgaa tccgcagctg aagcgtctga agagctgatg 600

gagaaatatc tgggcggtga agaactgact gaa 633

<210> 397
<211> 633
<212> DNA
<213> Erwinia persicina

<400> 397
ggtgtaaacc acaaaatcgg tgaagtacat gacggcgcgg ctaccatgga ctggatggag 60

caggaacagg aacgtggtat taccatcact tccgctgcga ccacctgttt ctggtctggt 120

atggctaagc agttcgagcc acaccacgta aacatcatcg acaccccggg acacgttgac 180

ttcacaatcg aagttgagcg ttctatgcgt gtgcttgatg gcgcagtaat ggtttactgt 240

gcggttggtg gcgttcagcc acagtctgag accgtatggc gccaggctaa caaatataaa 300

gttccacgca tcgcgttcgt taacaaaatg gaccgcatgg gtgctaactt cctgaaagtc 360

gtagagcaga tgaaagcgcg cctgggtgca aacccggtgc cattgcagct ggctattggc 420

gcagaagaga aattcaccgg tgttgttgac ctggtgaaaa tgaaagcgat caactggaac 480

gatgccgatc agggcgttac cttcgtatac gaagatattc cagctgatat gcaggaacag 540

gctgaagaat ggcgtgcgaa gctggttgaa gccgcagctg aaggttctga tgagctgatg 600

gagaaattct tcggcggtga agagctgact gaa 633

<210> 398
<211> 633
<212> DNA
<213> Erwinia psidii

<400> 398
ggtgtaaacc ataaaatcgg tgaagttcat gacggcgctg ccaccatgga ctggatggcg 60

caggagcagg aacgtggtat taccatcacc tccgccgcaa cgaccgcttt ctggtctggt 120

atggccaagc agttcgagcc acaccgcgta aacatcatcg acacccccgg acacgttgac 180

ttcaccatcg aagtagaacg ttccatgcgt gttctcgacg gcgcagtaat ggtttactgt 240

gcggttggtg gtgttcagcc acagtctgaa accgtatggc gtcaggctaa caaatataaa 300

gttccgcgta tcgcgttcgt taacaaaatg gaccgcatgg gtgcgaactt cctgaaagtg 360

gttagccaga ttaaatcccg tctgggtgct aaccctgttc cattgcagct ggcaattggt 420

gcagaagaga aattcaccgg tgttgttgac ctggtgaaaa tgaaagcaat caactggaac 480

gacgccgatc agggcgtaac cttcgagtac gaagatattc cagctgatat gcaggaactg 540

gctgaagagt ggcaccagaa cctgattgaa tccgcagctg aagcttctga agagctgatg 600

gagaaatatc tgggtggtga agaactgact gaa 633

<210> 399
<211> 633
<212> DNA
<213> Erwinia pyrifoliae

<400> 399
ggtgtaaacc acaaaatcgg tgaagttcat gatggcgcag ccaccatgga ctggatggcg 60

caggagcagg agcgtggtat taccattacc tccgcagcga ctaccgcttt ctggtctggt 120

atggctaagc agttcgagcc gcaccgcgtt aacattatcg acaccccagg acacgttgac 180

ttcaccatcg aagtagagcg ctctatgcgt gtactcgatg gcgcagtgat ggtttactgt 240

gctgttggtg gcgtgcagcc acagtctgaa accgtatggc gtcaggctaa caaatataaa 300

gttcctcgta tcgcgttcgt taacaaaatg gaccgtatgg gtgctaactt cctgaaagtg 360

gtcagccaga tcaaatctcg cctggcggct aacccggtgc cgattcagtt ggcaatcggc 420

gcggaagaga aattcaccgg cgttgttgac ctggtgaaaa tgaaagcgat caactggaac 480

gatgccgatc agggtgtgac cttcgaatac gaagagatcc cagctgatat gcaggaactg 540

gctgaagaat ggcaccagaa cctggttgaa tccgcagctg aagcttctga agagctgatg 600

gagaaatacc tgggcggtga agaactgact gaa 633

<210> 400
<211> 633
<212> DNA
<213> Erwinia rhapontici

<400> 400
ggtgtaaacc acaaaatcgg tgaagtacat gacggcgcag ccaccatgga ctggatggag 60

caggaacagg aacgtggtat taccatcact tccgctgcga ccacctgttt ctggtctggt 120

atggctaagc agtttgagcc gcaccacgta aacatcatcg acaccccggg acacgttgac 180

ttcactatcg aagttgagcg ttctatgcgt gtgcttgatg gcgcagtaat ggtttactgt 240

gcagttggtg gcgttcagcc acagtctgag accgtatggc gtcaggctaa caaatataaa 300

gttccacgca ttgcgttcgt taacaaaatg gaccgcatgg gtgctaactt cctgaaagtt 360

gttgagcaga tgaaagcgcg tctgggtgca aacccggtgc cattgcagct ggccatcggc 420

gcagaagaga aattcaccgg tgttgttgac ctggtgaaaa tgaaagcgat caactggaac 480

gatgccgatc agggcgttac cttcgtttac gaagagattc cagctgatat gcaggaactg 540

gctgaagaat ggcgtgcgaa gcttgttgaa gctgcagctg aaggttctga tgagctgatg 600

gagaaattct ttggtggcga agagctgacc gaa 633

<210> 401
<211> 633

<212>  DNA
<213>  Erwinia toletana

<400>  401
ggtgtaaacc acaaaatcgg tgaagttcat gatggcgcag ccaccatgga ctggatggca      60

caggagcagg agcgtggtat taccatcacc tccgcagcga ccactgcatt ctggtcaggt     120

atggcgaagc agttcgagcc acaccgcgta aatattatcg acacccctgg acacgttgac     180

ttcaccatcg aagtagaacg ttccatgcgt gttctcgacg gcgcagtaat ggtttactgt     240

gcagttggtg gcgttcagcc acagtctgaa accgtatggc gccaggcgaa caaatataaa     300

gttccacgta tcgcgttcgt taacaaaatg gaccgtatgg gtgctaactt cctgaaagtt     360

gttggtcaga tcgaatcacg tctggcggcc actccggttc ctctgcagct ggctatcggc     420

gctgaagagc atttcaccgg cgttgttgac ctgatcaaga tgaaagcgat caactggaac     480

gatgctgatg caggcgtgac cttcgtttac gaagatatcc cggctgatat gcaggaactg     540

gctgaagaat ggcgccagaa actgatcgaa gccgcagctg aagcatctga tgagctgatg     600

gagaaattct ttggtggcga agagctgacc gaa                                  633

<210>  402
<211>  633
<212>  DNA
<213>  Erwinia tracheiphila

<400>  402
ggtgtaaatc acaaaatcgg tgaagttcat gacggcgcag ctaccatgga ctggatggcg      60

caggagcagg agcgtggtat taccattacc tccgctgcaa cgaccgcttt ttggtcaggt     120

atggctaaac agttcgagcc gcaccgtgta aacatcatcg acaccccggg acacgttgac     180

ttcaccattg aagttgagcg ttccatgcgt gttcttgacg gtgcagtcat ggtttactgt     240

gccgttggcg gtgttcagcc acagtctgaa accgtatggc gccaggcaaa caaatataaa     300

gttccacgta ttgcgttcgt taacaaaatg gaccgcatgg gtgcgaactt cctgaaagtg     360

gttggccaga ttaaatcacg tctgggcgct aaccctgttc cattgcagct ggcaattggc     420

gcagaagaaa agttcactgg tgttgtcgac ctggtgaaaa tgaaagcgat taactggaat     480

gacgccgatc agggcgtaac cttcgaatac gaagagattc catctgatat gcaggaactg     540

gccgaagaat ggcaccagaa tctgattgag tccgcagcgg aagcatctga agagctgatg     600

gaaaaatatc tgggcggtga agaactgact gaa                                  633

<210>  403
<211>  633
<212>  DNA
<213>  Escherichia albertii

<400>  403
ggtgtaaacc ataaaatcgg tgaagttcat gacggcgctg caaccatgga ctggatggag      60

caggagcagg aacgtggtat taccatcact tccgctgcga ctactgcatt ctggtctggt     120

atggctaagc agtatgagcc gcatcgcatc aacatcatcg acaccccggg acacgttgac     180

ttcacaatcg aagtagaacg ttccatgcgt gttctcgatg gtgcggtaat ggtttactgc 240

gcagttggtg gtgttcagcc gcagtctgaa accgtatggc gtcaggctaa caaatataaa 300

gttccacgca ttgcgttcgt taacaaaatg gaccgcatgg gtgcgaactt cctgaaagtt 360

gttaaccaga tcaaaacccg tctgggcgcg aacccggttc cgctgcagct ggcaattggt 420

gctgaagaac atttcactgg tgttgttgac ctggtgaaaa tgaaagctat caactggaac 480

gacgctgacc agggcgtaac cttcgaatac gaagatatcc cggcagacat ggttgaactg 540

gctaacgaat ggcaccagaa cctgatcgaa tccgcagctg aagcttctga agagctgatg 600

gaaaaatatc tgggtggtga agaactgact gaa 633

<210> 404
<211> 633
<212> DNA
<213> Escherichia blattae

<400> 404
ggtgtaaacc ataaaatcgg tgaagttcat gatggcgcag ctaccatgga ctggatggaa 60

caggaacagg aacgtggtat caccatcacg tccgctgcga ctactgcttt ctggtctggt 120

atggccaaac agtatgaacc gcatcgtatc aacatcatcg acaccccggg gcacgttgac 180

ttcactatcg aagtagaacg ttccatgcgt gtgctcgacg gtgcggtaat ggtttactgc 240

gccgttggtg gtgttcagcc gcagtctgaa actgtatggc gtcaggccaa caaatataaa 300

gttccgcgta ttgcgttcgt taacaaaatg gaccgcatgg gtgcgaactt cctgaaagtt 360

gttgatcaga tcaaaacccg tctgggcgcg actccggttc cgctgcagct ggcgattggt 420

gctgaagagc acttcaccgg tgttgttgac ctggtgaaaa tgaaagccat caactggaac 480

gaagctgatg ctggcgtaac cttcgaatac gaagatatcc cggctgacat ggttgaactg 540

gctaacgaat ggcaccagaa cctgatcgaa tccgcagctg aagcctcaga agagctgatg 600

gaaaaatacc tgggcggcga agagctgacc gag 633

<210> 405
<211> 633
<212> DNA
<213> Escherichia coli T

<400> 405
ggtgtaaacc ataaaatcgg tgaagttcat gacggcgctg caaccatgga ctggatggag 60

caggagcagg aacgtggtat taccatcact tccgctgcga ctactgcatt ctggtctggt 120

atggctaagc agtatgagcc gcatcgcatc aacatcatcg acaccccggg gcacgttgac 180

ttcacaatcg aagtagaacg ttccatgcgt gttctcgatg gtgcggtaat ggtttactgc 240

gcagttggtg gtgttcagcc gcagtctgaa accgtatggc gtcaggcaaa caaatataaa 300

gttccgcgta ttgcgttcgt taacaaaatg gaccgcatgg gtgcgaactt cctgaaagtt 360

gttaaccaga tcaaaacccg tctgggcgcg aacccggttc cgctgcagct ggcgattggt 420

gctgaagaac atttcaccgg tgttgttgac ctggtgaaaa tgaaagctat caactggaac 480

gacgctgacc agggcgtaac cttcgaatac gaagatatcc cggcagacat ggttgaactg 540

gctaacgaat ggcaccagaa cctgatcgaa tccgcagctg aagcttctga agagctgatg 600

gaaaaatacc tgggtggtga agaactgact gaa 633

<210> 406
<211> 633
<212> DNA
<213> Escherichia fergusonii

<400> 406
ggtgtaaacc ataaaatcgg tgaagttcat gacggcgctg caaccatgga ctggatggag 60

caggagcagg aacgtggtat taccatcact tccgctgcaa ctactgcatt ctggtctggt 120

atggctaagc agtatgagcc gcatcgcatc aacatcatcg acaccccggg gcacgttgac 180

ttcacaatcg aagtagaacg ttccatgcgt gttctcgatg gtgcggtaat ggtttactgc 240

gcagttggtg gtgttcagcc gcagtctgaa accgtatggc gtcaggcaaa caaatataaa 300

gttccgcgca ttgcgttcgt taacaaaatg gaccgcatgg gtgcgaactt cctgaaagtt 360

gttaaccaga tcaaaacccg tctgggcgcg aacccggttc cgctgcagct ggcgattggt 420

gctgaagaac atttcaccgg tgttgttgac ctggtgaaaa tgaaagctat caactggaac 480

gacgctgacc agggcgtaac cttcgaatac gaagatatcc cggcagacat ggttgaactg 540

gctaacgaat ggcaccagaa cctgatcgaa tccgcagctg aagcttctga agagctgatg 600

gaaaaatacc tgggtggtga agaactgact gaa 633

<210> 407
<211> 633
<212> DNA
<213> Escherichia hermannii

<400> 407
ggtgtaaacc ataaaatcgg tgaagttcat gacggcgccg ctactatgga ctggatggag 60

caggagcagg agcgtggtat caccattacc tccgcagcga ctactgcgtt ctggtctggt 120

atggctaagc agtatgaacc gcatcgcgta aacatcatcg acaccccggg gcacgttgac 180

ttcactatcg aagtagaacg ttccatgcgt gtacttgacg gtgcggtaat ggtttactgc 240

gcagttggtg gtgttcagcc gcagtctgaa accgtatggc gtcaggctaa taaatacaaa 300

gttccgcgta tcgcgttcgt taacaaaatg gaccgtatgg gtgcgaactt cctgaaagtt 360

gttggtcaga tcaaatcccg tttgggcgcg aacccggttc cgctgcagct ggcaattggt 420

gctgaagaag gtttcaccgg tgttgttgac ctggtgaaaa tgaaagccat caactggaac 480

gatgctgacc agggcgttac cttcgaatac gaagatatcc cggcagacat gcaggacctg 540

gctgaagaat ggcaccagaa cctgatcgag tccgcagctg aagcttctga agagctgatg 600

gaaaaatacc tgggtggtga agaactgact gaa 633

<210> 408
<211> 633
<212> DNA

<213>  Escherichia vulneris

<400>  408
ggtgtaaacc acaaaatcgg tgaagtacac gacggcgcgg caactatgga ctggatggag      60

caggagcagg agcgtggtat taccatcacc tccgcagcga ccactgcatt ctggtctggt     120

atggcgaagc agtatgaacc gcatcgcgta aacatcatcg acaccccggg ccacgttgac     180

ttcaccatcg aagtagaacg ttccatgcgt gttcttgacg gtgcggtaat ggtttactgc     240

gcagttggtg gtgttcagcc gcagtctgaa accgtatggc gtcaggcaaa caaatataaa     300

gttccacgta tcgcgttcgt taacaaaatg gaccgtatgg gtgcgaactt cctgaaagtt     360

gttggtcaga tcaaatcccg tctcggcgcg aacccggttc cgctgcagct ggcgattggt     420

gctgaagaag gtttcaccgg tgttgttgac ctggtgaaaa tgaaagccat caactggaac     480

gatgccgacc agggcgttac cttcgaatac gaagatatcc cggctgacat gcaggatctg     540

gctgaagaat ggcaccagaa cctcatcgaa tccgctgctg aagcttctga agagctgatg     600

gagaaatacc tgggcggtga agaactgact gaa                                  633

<210>  409
<211>  633
<212>  DNA
<213>  Ewingella americana T

<400>  409
ggtgtaaacc acaaaatcgg tgaagttcat gatggcgcag ctaccatgga ctggatggag      60

caagaacagg aacgtggtat taccatcacg tccgctgcta ccacctgtta ctgggctggt     120

atggctaagc agttcgaacc acaccacatc aacatcatcg acaccccagg gcacgttgac     180

ttcaccatcg aagttgaacg ttccatgcgt gttcttgatg gcgcggtaat ggtttactgt     240

gctgttggtg gtgttcagcc acagtctgag accgtatggc gtcaggcaaa caaatataaa     300

gttcctcgta tcgcgttcgt taacaaaatg gaccgtatgg gtgctaactt cctgaaagtt     360

gttgatcaga ttgaaaaacg tctgggcgca actccggtgc ctctgcaact ggcaatcggc     420

gcggaagaga aattcaccgg tgttgttgac ctggtgaaga tgaaagctat caactggaac     480

gaagctgatc agggcgtgac cttcgaatac gaagagatcc cagctgacat gcaagaactg     540

gctgaagaat ggcatcagaa actggttgag tccgctgctg aaggctctga tgagctgatg     600

gagaaattct ttggtggcga agagctgacc gaa                                  633

<210>  410
<211>  633
<212>  DNA
<213>  Hafnia alvei T

<400>  410
ggtgtaaacc ataaaatcgg tgaagttcat gacggcgctg ccaccatgga ctggatggaa      60

caggaacaag aacgtggtat caccatcact tccgctgcaa ctactgcgtt ctggtcaggt     120

atggctaagc agtatgaacc gcatcgcgta aacatcattg acaccccagg gcacgttgac     180

ttcaccatcg aagttgaacg ttccatgcgt gttcttgacg gtgcggtaat ggtttactgc     240

gcggttggtg gtgttcagcc acagtctgaa accgtatggc gtcaggctaa taaatacaaa 300

gttccacgca ttgcgttcgt taacaaaatg gaccgcatgg gtgctaactt cctgaaagtt 360

gtaggccaga tcaaatcccg tttgggcgcg aacgctgttc cgcttcaatt ggcaattggc 420

gctgaagaag gtttcaccgg tgttgttgac ctggtgaaaa tgaaagcgat caactggaac 480

gatgccgatc agggcgtgac cttcgaatac gaagagatcc cagctgatat ggctgagctg 540

gctgctgaat ggcaccagaa tttggttgag tctgcagcag aagcttctga agagctgatg 600

gataaatact tgggcggcga agagctgacc gag 633

<210> 411
<211> 633
<212> DNA
<213> Klebsiella oxytoca

<400> 411
ggtgtaaacc acaaaatcgg tgaagttcat gacggcgctg ccaccatgga ctggatggag 60

caggagcaag agcgtggtat tactatcacc tccgcagcga ctactgcatt ctggtcaggt 120

atggctaagc agtatgaacc gcatcgcgta aacatcatcg acaccccggg gcacgttgac 180

ttcactatcg aagtagaacg ttccatgcgt gtgcttgatg gtgcagtaat ggtttactgc 240

gcagttggtg gtgttcagcc tcagtctgaa accgtatggc gtcaggcaaa caaatataaa 300

gttccgcgca ttgcgttcgt taacaaaatg gaccgtatgg gtgcgaactt cctgaaagtt 360

gttggtcaga tcaaaacccg tctgggcgcg aaccctgttc cgctgcagct ggcgattggc 420

gctgaagaag cgttcaccgg tgttgttgac ctggtgaaaa tgaaagccat caactggaac 480

gaagaagatg caggcgtaac cttcacttac gaagatattc cggcagacat gcaggaactg 540

gccgaagaat ggcaccagaa cctgatcgag tccgctgctg aagcttctga agagctgatg 600

gaaaaatacc tgggtggcga agaactgact gaa 633

<210> 412
<211> 633
<212> DNA
<213> Klebsiella pneumoniae sbsp. ozaenae

<400> 412
ggtgtaaacc acaaaatcgg tgaagttcat gacggcgccg ctaccatgga ctggatggag 60

caggagcagg aacgtggtat caccatcact tccgctgcga ctactgcatt ctggtctggt 120

atggccaaac agtatgaacc gcaccgcgta aacatcatcg acaccccggg gcacgttgac 180

ttcactatcg aagtagaacg ttccatgcgt gttcttgatg gtgcggtaat ggtttactgc 240

gcagttggtg gtgttcagcc gcagtctgaa accgtatggc gtcaggcaaa caaatataaa 300

gttccgcgca tcgcgttcgt taacaaaatg gaccgcatgg gcgctaactt cctgaaagtt 360

gttggtcaga tcaaaacccg tctgggcgcg aacccggttc cgctgcagct ggcaattggt 420

gctgaagaag gtttcaccgg cgttgttgac ctggtgaaaa tgaaagccat caactggaac 480

gatgcagacc agggcgtaac cttcgaatac gaagatatcc cggctgacat gcaggacctg 540

gctgatgaat ggcaccagaa cctgatcgaa tccgcagctg aagcttctga agagctgatg 600

gaaaaatacc tgggtggtga agaactgact gaa 633

<210> 413
<211> 633
<212> DNA
<213> Klebsiella pneumoniae sbsp. pneumoniae T

<400> 413
ggtgtaaacc acaaaatcgg tgaagttcat gacggcgccg ctaccatgga ctggatggag 60

caggagcagg aacgtggtat caccatcact tccgctgcga ctactgcatt ctggtctggt 120

atggccaaac agtatgaacc gcaccgcgta aacatcatcg acaccccggg gcacgttgac 180

ttcactatcg aagtagaacg ttccatgcgt gttcttgatg gtgcggtaat ggtttactgc 240

gcagttggtg gtgttcagcc gcagtctgaa accgtatggc gtcaggcaaa caaatataaa 300

gttccgcgca tcgcgttcgt taacaaaatg gaccgcatgg gcgctaactt cctgaaagtt 360

gttggtcaga tcaaaacccg tctgggcgcg aacccggttc cgctgcagct ggcaattggt 420

gctgaagaag gtttcaccgg cgttgttgac ctggtgaaaa tgaaagccat caactggaac 480

gatgcagacc agggcgtaac cttcgaatac gaagatatcc cggctgacat gcaggacctg 540

gctgatgaat ggcaccagaa cctgatcgaa tccgcagctg aagcttctga agagctgatg 600

gaaaaatacc tgggtggtga agaactgact gaa 633

<210> 414
<211> 633
<212> DNA
<213> Klebsiella pneumoniae sbsp. rhinoscleromatis

<400> 414
ggtgtaaacc acaaaatcgg tgaagttcat gacggcgccg ctaccatgga ctggatggag 60

caggagcagg aacgtggtat caccatcact tccgctgcga ctactgcatt ctggtctggt 120

atggccaaac agtatgaacc gcaccgcgta aacatcatcg acaccccggg gcacgttgac 180

ttcactatcg aagtagaacg ttccatgcgt gttcttgatg gtgcggtaat ggtttactgc 240

gcagttggtg gtgttcagcc gcagtctgaa accgtatggc gtcaggcaaa caaatataaa 300

gttccgcgca tcgcgttcgt taacaaaatg gaccgcatgg gcgctaactt cctgaaagtt 360

gttggtcaga tcaaaacccg tctgggcgcg aacccggttc cgctgcagct ggcaattggt 420

gctgaagaag gtttcaccgg cgttgttgac ctggtgaaaa tgaaagccat caactggaac 480

gatgcagacc agggcgtaac cttcgaatac gaagatatcc cggctgacat gcaggacctg 540

gctgatgaat ggcaccagaa cctgatcgaa tccgcagctg aagcttctga agagctgatg 600

gaaaaatacc tgggtggtga agaactgact gaa 633

<210> 415
<211> 633
<212> DNA
<213> Klebsiella singaporensis

<400> 415
ggtgtaaacc acaaaatcgg tgaagttcat gacggcgccg ctaccatgga ctggatggag 60

caggagcagg aacgtggtat caccatcact tccgctgcga ctactgcatt ctggtctggt 120

atggctaagc agtatgaacc gcaccgtgta aacatcatcg acaccccggg gcacgttgac 180

ttcactatcg aagtagaacg ttccatgcgt gttcttgatg gtgcggtaat ggtttactgc 240

gcagttggtg gtgttcagcc acagtctgaa accgtatggc gtcaggcaaa caaatataaa 300

gttccgcgca ttgcgttcgt taacaaaatg gaccgcatgg gtgcgaactt cctgaaagtt 360

gttggtcaga tcaaaacccg tctgggcgcg aacccggttc cgctgcagct ggcgattggt 420

gctgaagaag gtttcaccgg tgttgttgac ctggtgaaaa tgaaagccat caactggaac 480

gatgcagacc agggcgttac cttcgaatac gaagatatcc cggctgacat gcaggacctg 540

gctgacgaat ggcaccagaa cctgatcgag tccgctgcgg aagcttccga agagctgatg 600

gagaaatacc tgggtggtga agaactgact gaa 633

<210> 416
<211> 633
<212> DNA
<213> Klebsiella variicola

<400> 416
ggtgtaaacc acaaaatcgg tgaagttcat gacggcgccg ctaccatgga ctggatggag 60

caggagcagg aacgtggtat caccatcact tccgctgcga ctactgcatt ctggtctggt 120

atggctaagc agtatgaacc gcaccgtgta aacatcatcg acaccccggg gcacgttgac 180

ttcactatcg aagtagaacg ttccatgcgt gttcttgatg gtgcggtaat ggtttactgc 240

gcagttggtg gtgttcagcc acagtctgaa accgtatggc gtcaggcaaa caaatataaa 300

gttccgcgca ttgcgttcgt taacaaaatg gaccgcatgg gtgcgaactt cctgaaagtt 360

gttggtcaga tcaaaacccg tctgggcgcg aacccggttc cgctgcagct ggcgattggt 420

gctgaagaag gtttcaccgg tgttgttgac ctggtgaaaa tgaaagccat caactggaac 480

gatgcagacc agggcgttac cttcgaatac gaagatatcc cggctgacat gcaggacctg 540

gctgacgaat ggcaccagaa cctgatcgag tccgctgcgg aagcttccga agagctgatg 600

gagaaatacc tgggtggtga agaactgact gaa 633

<210> 417
<211> 633
<212> DNA
<213> Kluyvera ascorbata T

<400> 417
ggtgtaaacc acaaaatcgg tgaagttcac gacggcgccg ctaccatgga ctggatggag 60

caggagcagg aacgtggtat taccatcact tccgcagcga ctactgcatt ctggtctggt 120

atggctaagc agtatgaacc gcaccgcgta aacatcatcg acaccccggg gcacgttgac 180

ttcactatcg aagtagaacg ttccatgcgt gttcttgacg gtgcggtaat ggtttactgc 240

gcagttggtg gtgttcagcc acagtctgaa accgtatggc gtcaggctaa caaatacaaa 300

gttccacgta tcgcgttcgt taacaaaatg gaccgtatgg gtgcgaactt cctgaaagtt 360

gttggtcaga tcaaaacccg tctgggcgcg aaccctgttc cgctgcagtt ggcaattggt 420

gctgaagaag gtttcaccgg tgttgttgac ctggtgaaaa tgaaagccat caactggaac 480

gaagaagacg caggcgttac cttcacttat gaagatatcc cagcagacat gcaggacctg 540

gctgacgaat ggcaccagaa cctgatcgag tccgctgcag aagcttcaga agagctgatg 600

gagaaatacc tgggtggtga agaactgact gaa 633

<210> 418
<211> 633
<212> DNA
<213> Kluyvera cochleae

<400> 418
ggtgtaaacc acaaaatcgg tgaagttcac gacggcgcgg ctaccatgga ctggatggag 60

caggagcagg aacgtggtat taccatcact tccgctgcga ctactgcatt ctggtctggt 120

atggctaagc agtatgaacc acaccgtgta aacatcatcg acaccccggg gcacgtagac 180

ttcactatcg aagttgaacg ttccatgcgt gtgcttgatg gtgcggtaat ggtttactgc 240

gcagttggtg gtgttcagcc acagtctgaa accgtatggc gtcaggcaaa caaatataaa 300

gttccacgta tcgcgttcgt taacaaaatg gaccgtatgg gtgcgaactt cctgaaagtt 360

gttggtcaga tcaaatcccg tctgggcgcg aaccctgttc cgctgcagct ggcgattggt 420

gctgaagaag gtttcaccgg tgttgttgac ctggtgaaaa tgaaagccat caactggaat 480

gaagcagatg caggcgtaac cttcacttat gaagatattc cagcagacat gcaggacctg 540

gctgacgaat ggcaccagaa cctgatcgag tccgctgctg aagcttcaga agagctgatg 600

gagaaatacc tgggtggcga agaactgact gaa 633

<210> 419
<211> 633
<212> DNA
<213> Kluyvera cryocrescens

<400> 419
ggtgtaaacc acaaaatcgg tgaagttcat gacggcgcag ctaccatgga ctggatggag 60

caggagcagg aacgtggtat caccatcact tccgctgcga ctactgcatt ctggtctggt 120

atggctaagc agtatgaacc gcaccgtgta aacatcatcg acaccccggg gcacgttgac 180

ttcacaatcg aagtagaacg ttccatgcgt gttcttgatg gtgcggtaat ggtttactgc 240

gcagttggtg gtgttcagcc acagtctgaa accgtatggc gtcaggcaaa caaatataaa 300

gttccacgca ttgcgttcgt taacaaaatg gaccgcatgg gtgcaaactt cctgaaagtt 360

gttggtcaga tcaaatcccg tctgggcgcg aaccctgttc cgctgcagct ggcaattggt 420

gctgaagaac atttcaccgg tgttgttgac ctggtgaaaa tgaaagccat caactggaac 480

gaagaagatg caggcgtaac cttcacttat gaagatattc cagctgacat gcaggaactg 540

gctgacgaat ggcaccagaa cctgatcgag tccgctgctg aagcttcaga agagctgatg 600

gagaaatacc tgggtggtga agcactgact gaa 633

<210> 420
<211> 633
<212> DNA
<213> Kluyvera georgiana

<400> 420
ggtgtaaacc ataaaatcgg tgaagttcac gacggcgccg ctaccatgga ctggatggag 60

caggagcagg aacgtggtat taccatcact tccgctgcaa ctactgcatt ctggtctggt 120

atggctaagc agtatgaacc gcatcgcgta aacatcatcg acaccccggg gcacgttgac 180

ttcaccatcg aagtagaacg ttccatgcgt gttcttgacg gtgcggtaat ggtttactgc 240

gcagttggtg gtgttcagcc acagtctgaa accgtatggc gtcaggctaa caagtataaa 300

gtaccgcgca tcgcgttcgt taacaaaatg gaccgtatgg gcgctaactt cctgaaagtt 360

gttggtcaga tcaaaacccg tctgggcgcg aaccctgttc cgctgcagct ggcaattggc 420

gctgaagaag cgttcaccgg tgttgttgac ctggtgaaaa tgaaagccat caactggaac 480

gaagaagacg caggcgtaac cttcacttat gaagatatcc cggctgacat gcaggaactg 540

gctgacgaat ggcaccagaa cctgatcgag tccgctgctg aagcttcaga agagctgatg 600

gagaaatacc tgggtggtga agaactgact gaa 633

<210> 421
<211> 633
<212> DNA
<213> Kluyvera intermedia

<400> 421
ggtgtaaacc ataaaatcgg tgaagttcat gacggcgcgg ctaccatgga ctggatggag 60

caggagcagg aacgtggtat caccatcact tccgctgcga ctactgcatt ctggtctggt 120

atggctaagc agtatgaacc acaccgtgta aacatcatcg acaccccagg gcacgttgac 180

ttcactatcg aagttgaacg ttctatgcgt gtgcttgatg gtgcggtaat ggtttactgc 240

gcagttggtg gtgttcagcc acagtctgaa accgtatggc gtcaggcaaa caaatataaa 300

gttccacgca tcgcgttcgt taacaaaatg gaccgtatgg gtgctaactt cctgaaagtt 360

gttggtcaga tcaaatcccg tctgggcgcg actccggttc cgctgcagct ggcaattggt 420

gctgaagaac atttcaccgg tgttgttgac ctggtgaaaa tgaaagccat caactggaac 480

gaagaagacg caggcgtaac cttcacttat gaagatattc cagcagacat gcaggaactg 540

gctgacgaat ggcaccagaa cctgattgag tccgctgctg aagcttcaga agagctgatg 600

gagaaatacc tgggtggtga agaactgact gaa 633

<210> 422
<211> 633
<212> DNA
<213> Leclercia adecarboxylata T

<400>  422
ggtgtaaacc ataaaatcgg tgaagttcac gacggcgccg ctaccatgga ctggatggag      60

caggagcagg agcgtggtat taccattacc tccgcagcga ctactgcatt ctggtctggt     120

atggctaagc agtatgaacc gcatcgcgta aacatcatcg acaccccggg gcacgttgac     180

ttcaccatcg aagtagaacg ttccatgcgt gttctggatg gtgcggtaat ggtttactgc     240

gcagttggtg gtgttcagcc acagtctgaa accgtatggc gtcaggcaaa caaatacaaa     300

gttccacgca tcgcgttcgt taacaaaatg gaccgtatgg gtgctaactt cctgaaagtt     360

gttggtcaga tcaaatcccg tctgggcgcg aaccctgttc cgctgcagtt ggcaattggt     420

gctgaagaag gtttcaccgg cgtaatcgac ctggtgaaaa tgaaagccat caactggaac     480

gaagaagatg caggcgtaac cttcacctac gaagatatcc cagcagacat gcaggacctg     540

gctgacgaat ggcaccagaa cctgatcgag tccgcagcgg aagcttctga agagctgatg     600

gaaaaatacc tgggtggtga agaactgact gaa                                  633

<210>  423
<211>  633
<212>  DNA
<213>  Leminorella grimontii T

<400>  423
ggtgtaaacc ataagatcgg tgaagttcac gacggcgctg cgacgatgga ctggatggaa      60

caggagcagg agcgtggtat caccatcacc tcagctgcga ctaccgcatt ctggtccggt     120

atggctaagc agtttgaagc gcaccgcgtc aacatcatcg acaccccggg gcacgttgac     180

ttcaccatcg aagtagagcg ctctatgcgc gtgcttgacg gcgctgtcat ggtttattgt     240

gccgtcggcg gcgttcagcc tcagtctgaa accgtttggc gtcaggctaa caaatataaa     300

gttccacgca tcgcgttcgt taacaaaatg gaccgcatgg gtgctaactt cctgcgcgta     360

gttgagcagt tgaaaactcg tctggctgct aacgcagtgc caattcagct gcctatcggc     420

gctgaagaca actttaccgg cgttgttgac ctgatcaaaa tgaaggccat caactggaat     480

gacgccgatc agggcgtaac cttcgaatac gaagatgttc ctgccggaat gcaggcgctt     540

gccgaagaat ggcgtcaaaa tctggtcgag tctgcggctg aggcatcaga agacctgatg     600

gacaagtatt tgggcggcga agagctgact gaa                                  633

<210>  424
<211>  633
<212>  DNA
<213>  Leminorella richardii

<400>  424
ggtgtaaacc ataagatcgg tgaaacacac gaaggcaccg cgacaatgga ctggatggcg      60

caagagcaag agcgtggtat tactatcacc tctgcggcga ccaccgcgtt ctggtccggt     120

atggccaagc agtttgatgc acaccgtatc aacattatcg acaccccagg gcacgttgac     180

ttcaccatcg aagtagagcg ctcaatgcgc gtacttgacg gcgcagtcat ggtttactgt     240

gctgtgggtg gcgttcagcc tcagtctgaa accgtatggc gtcaggccaa caagtaccag     300

gttccgcgca tcgcgttcgt taacaaaatg gaccgtatgg gtgctaactt cctgcgtgtt 360

gttgagcaga tgaaaactcg tctggcggct aatgctgttc ctcttcagct gcctatcggt 420

gcagaagaac acttcaccgg tatcgttgac ttggtcaaaa tgaaggccat caactggagc 480

gaagaagatc agggcgtaac cttcacttat gaagacgttc ctgaaagcat gctggaactg 540

gctcaagaat ggcgtcaaac gctggttgag tctgcagcgg aagcttcaga agagctgatg 600

gacaagtatc tgggcggtga agagctgtct gaa 633

<210> 425
<211> 633
<212> DNA
<213> Moellerella wisconsensis T

<400> 425
ggtgtaaacc ataaaatcgg tgagactcat gaaggttctg caactatgga ctggatggag 60

caggagcaag aacgtggtat tactatcact tcagctgcaa ctactgcatt ctggtcaggt 120

atggcaaaac agtttgagcc acatcgcata aacatcatcg acaccccagg acacgttgac 180

ttcacaatcg aagtagaacg ttctatgcgt gttcttgatg gtgcagtaat ggtttactgt 240

gcggttggtg gtgttcagcc acagtctgaa accgtatggc gtcaggctaa caaatataaa 300

gtaccacgta tcgcgttcgt aaataaaatg gaccgtatgg gtgctaactt cttgaaagtt 360

gttgagcagc ttaaaactcg cttagcagct aacccagttc cactccaatt agctattggc 420

gctgaagagt cattcacagg tgttgttgac ctggttaaaa tgaaagctat tcagtggagt 480

gaagaagatc agggcgttac ttttgtctac gaagatattc ctgcagaact gcaagaatta 540

gcagataaat ggcacagcaa cttggttgaa tctgcagcag aagcatcaga agaactgatg 600

gaaaaatatc tgggcggtga agacctgact gaa 633

<210> 426
<211> 633
<212> DNA
<213> Morganella morganii sbsp. morganii T

<400> 426
ggtgtgaacc ataaaattgg tgagacccac gaaggttcag caacgatgga ctggatggag 60

caggaacaag agcgtggtat tactatcacc tccgcagcaa ccactgcgtt ctggtccggt 120

atgggcaaac aatttgaacc acaccgcatc aacatcatcg acaccccggg gcacgttgac 180

ttcacaatcg aagtagaacg ttccatgcgt gttcttgatg gtgctgtaat ggtttactgt 240

gccgttggtg gtgttcagcc acagtctgaa accgtatggc gccaggcaaa caaatatcat 300

gtacctcgta tcgcgttcgt taacaaaatg gaccgtatgg gtgcaaactt cctgaaagtt 360

gttgatcaga tcaaaacccg cttagcggct actccggttc ctcttcagtt agctatcggc 420

gctgaagaat cattcaccgg tgttgttgac ctggttaaaa tgaaagcgat tcagtggagt 480

gaagaagacc agggcgttac cttcgtctac gaagatatcc cggcaaacat gcaggaatta 540

gctgctgaat ggcaccagaa tctggttgaa gccgcagctg aagcctcaga agagctgatg 600

gaaaaatatc tcggcggtga agaactgact gaa 633

<210> 427
<211> 633
<212> DNA
<213> Morganella morganii sbsp. sibonii

<400> 427
ggtgtgaacc ataaaattgg tgagacccac gaaggttcag caacgatgga ctggatggag 60

caggaacaag agcgtggtat tactatcacc tccgcagcaa ccactgcgtt ctggtctggt 120

atgggtaaac aatttgaacc acaccgcatc aacatcatcg acaccccggg gcacgttgac 180

ttcacaatcg aagtagaacg ttctatgcgt gttcttgatg gtgctgtaat ggtttactgt 240

gccgttggtg gtgttcagcc acagtctgaa accgtatggc gccaggcaaa caaatatcat 300

gtacctcgta tcgcgttcgt taacaaaatg gaccgtatgg gtgcaaactt cctgaaagtt 360

gttgatcaga tcaaaacccg cttagcggcc aacccggtac cacttcagtt agctatcggc 420

gctgaagagt cattcaccgg tgttgttgac ctggttaaaa tgaaagcgat tcagtggagt 480

gaagaagacc agggcgttac tttcacttac gaagatatcc cggcaaacat gcaggaatta 540

gctgctgaat ggcaccagaa tctggttgaa gccgcagctg aagcttctga agagctgatg 600

gaaaaatatc tcggcggtga agaactgact gaa 633

<210> 428
<211> 633
<212> DNA
<213> Obesumbacterium proteus T

<400> 428
ggtgtaaacc ataaaatcgg tgaagttcat gacggcgcag ccaccatgga ctggatggaa 60

caggaacaag aacgtggtat taccattacc tctgctgcga ccactgcatt ctggtctggc 120

atggctaaac agtttgaacc acaccgcgta aacatcatcg acaccccagg gcacgttgac 180

ttcactatcg aagtagaacg ttctatgcgt gttcttgatg gcgctgtaat ggtttactgt 240

gccgttggtg gtgttcagcc ccagtctgaa accgtatggc gtcaggctaa caaatataaa 300

gttccacgca tcgcgttcgt taacaaaatg gaccgtatgg gtgctaactt cctgaaagtt 360

gttggtcaga tcaaaacccg tctgggcgcg aaccctgttc cgttgcagtt ggcgattggt 420

gctgaagaag gtttcaccgg tgttgttgac ctggtgaaaa tgaaagccat caactggaac 480

gaagctgatg ctggcgttac cttcacttat gaagatatcc ctgcagacat gcaagaactg 540

gctgaagaat ggcaccagaa tctgattgaa tctgctgctg aagcttcaga agagctgatg 600

gaaaaatact tgggcggcga agagctgacc gag 633

<210> 429
<211> 633
<212> DNA
<213> Pantoea agglomerans T

<400> 429

ggtgtaaacc acaaaatcgg tgaagtacat gacggcgcag caaccatgga ctggatggag 60

caggaacagg aacgtggtat taccatcacc tccgctgcga ccacctgtta ctggtctggt 120

atggccaagc agtttgaacc acaccacgta aacattatcg acaccccagg acacgttgac 180

ttcaccatcg aagttgaacg ttctatgcgt gtgcttgatg gcgcggtaat ggtttactgt 240

gcagttggtg gcgttcagcc acagtctgag accgtatggc gtcaggctaa caaatataaa 300

gttccacgca ttgcgttcgt taacaaaatg gaccgcatgg gtgctaactt cctgaaagtt 360

gttaaacaga tggaagttcg tctgggcgca actccagttc ctctgcagct ggctatcggc 420

gcagaagaga aattcaccgg tgttgttgac ctggtgaaaa tgaaagcaat caactggaac 480

gacgcagacc agggtgtaac cttcgtctac gaagatatcc cagctgatat gcaggaactg 540

gctgaagaat ggcatgcgaa gctggttgaa gccgctgctg aaggctctga tgagctgatg 600

gagaaattct tcggtggtga agaactgact gaa 633

<210> 430
<211> 633
<212> DNA
<213> Pantoea ananatis

<400> 430
ggtgtaaacc acaaaatcgg tgaagttcat gacggcgcag caaccatgga ctggatggag 60

caggagcagg aacgtggtat taccatcacg tcagctgcga ccacctgcta ctggtctggt 120

atggctaagc agtttgaacc acaccacgta aacatcatcg acaccccggg acacgttgac 180

ttcaccatcg aagttgagcg ttctatgcgt gtgcttgatg gcgcagttat ggtttactgt 240

gcagttggtg gtgttcagcc acagtctgaa accgtatggc gccaggctaa caaatataaa 300

gttccacgca tcgcgttcgt taacaaaatg gaccgcatgg gtgctaactt cctgaaagtt 360

gttgaccaga tgaaagcgcg tctgggtgca aacccagtcc cacttcagct ggctatcggc 420

gcagaagaca gcttcaccgg tgttgttgac ctggtgaaaa tgaaagcgat caactggaac 480

gatgaagacc agggcgtgac cttcgtttac gaagacattc cagccaacat gcaggaactg 540

gctgaagaat ggcgtgcgaa actggttgaa gccgcagctg aaggttctga cgagctgatg 600

gagaaattct tcggcggtga agagctgaca gaa 633

<210> 431
<211> 633
<212> DNA
<213> Pantoea citrea

<400> 431
ggtgtaaacc ataaaatcgg tgaagttcat gatggcgctg caaccatgga ctggatggct 60

caggaacagg aacgtggtat taccatcacg tctgctgcga cgactgcttt ctggtctggt 120

atggcgaaac agtttgaacc acaccgtatc aacattattg ataccccggg acacgttgac 180

tttactatcg aagtagagcg ttcaatgcgt gttctggatg gtgcagtaat ggtttactgt 240

gcggttggcg gcgttcagcc tcagtctgag actgtatggc gtcaggctaa caaatataaa 300

gttccacgca tcgcgttcgt taacaaaatg gaccgtatgg gtgctaactt cctgaaagtt 360

gttggccaga ttaaaagccg tctgggcgct aaccctgttc cattgcagtt agcaattggc 420

gcagaagaag gtttcaccgg tgttgttgac ctggtgaaaa tgaaagcgat caactggaac 480

gaagaagacg caggcgtaac ttttgtttac gaagatatcc ctgctgatct ggctgacctg 540

gctgaagaat ggcgccagaa cctggttgaa gctgcagcag aagcttccga agagctgatg 600

gagaaatacc tcggcggtga agaactgact gaa 633

<210> 432
<211> 633
<212> DNA
<213> Pantoea dispersa

<400> 432
ggtgttaacc acaaaatcgg tgaagtgcac gatggcgctg caaccatgga ctggatggcg 60

caggagcagg agcgtggtat taccatcacc tctgcagcga ccactgcgtt ctggtcaggt 120

atggcgaagc agtttgagcc gcaccgcatc aacatcatcg acaccccggg acacgttgac 180

ttcaccatcg aagtagaacg ttctatgcgt gtgctcgacg gcgcagtaat ggtttactgt 240

gcagtaggtg gcgtgcagcc gcagtctgaa accgtatggc gtcaggcaaa caaatataaa 300

gttccacgta tcgcgttcgt taacaaaatg gaccgtatgg gtgcgaactt cctgaaagtt 360

gttggtcaga tcgaatcacg tctgggcgca actccggttc cactgcagct ggctatcggc 420

gcagaagaga acttcaccgg tgttgttgac ctgatcaaga tgaaagagat caactggaac 480

gacgccgatc agggtactac cttcgtttac gaagatatcc gtgctgagtt gcaggaactg 540

gctgaagagt ggcgtggcaa gctgattgaa gcagctgctg aagcttctga agagctgatg 600

gagaaattct ttagcggtga agaactgtct gaa 633

<210> 433
<211> 633
<212> DNA
<213> Pantoea punctata

<400> 433
ggtgtaaacc ataaaatcgg tgaagtacac gatggtgcag caactatgga ctggatggct 60

caggaacagg aacgtggtat taccatcacg tctgctgcga cgactgcttt ctggtctggt 120

atggcaaaac agtttgaacc acaccgtgta aacattatcg ataccccggg acacgttgac 180

tttactatcg aagtagagcg ttcaatgcgt gttctggatg gtgcagtaat ggtttactgt 240

gcggttggtg gcgttcagcc tcagtccgag actgtatggc gccaggctaa caaatataaa 300

gttccacgca tcgcgttcgt taacaaaatg gaccgtatgg gtgctaactt cctgaaagtt 360

gttggtcaga ttaaaagccg tctgggcgca aaccctgttc cattgcagtt agcaatcggt 420

gcagaagaag gtttcaccgg tgttgttgac ctggtgaaaa tgaaagcgat caactggaac 480

gaagcagacg ctggcgtgac cttcgaatac gaagagatcc ctgctgatat ggtcgatctg 540

gctgatgaat ggcgccagaa catggtcgag gctgctgctg aagcttctga agagctgatg 600

gagaaatacc tcggcggtga agaactgact gaa 633

<210> 434
<211> 633
<212> DNA
<213> Pantoea stewartii sbsp. indologenes

<400> 434
ggtgttaacc acaaaatcgg tgaagtgcat gatggcgcag cgaccatgga ctggatggca 60

caggagcagg aacgtggtat taccatcacg tctgctgcaa cgaccgcgtt ctggtcaggt 120

atggctaagc agtttgagcc acaccgcatc aacatcatcg acaccccggg acacgttgac 180

ttcaccatcg aagtagaacg ttccatgcgt gtgctcgatg gcgcagtaat ggtttactgt 240

gctgtaggtg gtgttcagcc acagtctgag accgtatggc gtcaggctaa caaatataaa 300

gttccacgca ttgcgttcgt taacaaaatg gaccgcatgg gtgcaaactt cctgaaagta 360

gttggtcaga tcgaatctcg tctgggcgcg actccggttc cgctgcagat cgctatcggc 420

gctgaagaaa acttcaccgg cgttgttgac ctgatcaaaa tgaaagccat caactggaac 480

gaccaggatg cgggcgtgac cttcgattac gaagagatcc cagctaacct gcaagaactg 540

gctgaagaat ggcgcggcaa gctgatcgaa gctgcagcag aagcctctga agagctgatg 600

gagaaattct tcagcggtga agagctgact gaa 633

<210> 435
<211> 633
<212> DNA
<213> Pantoea stewartii sbsp. stewartii

<400> 435
ggtgttaacc acaaaatcgg tgaagtgcat gatggcgcag cgaccatgga ctggatggca 60

caggagcagg aacgtggtat taccatcacg tctgctgcaa cgaccgcgtt ctggtcaggt 120

atggctaagc agtttgagcc acaccgcatc aacatcatcg acaccccggg acacgttgac 180

ttcaccatcg aagtagaacg ttccatgcgt gtgctcgatg gcgcagtaat ggtttactgt 240

gctgtaggtg gtgttcagcc acagtctgag accgtatggc gtcaggctaa caaatataaa 300

gttccacgca ttgcgttcgt taacaaaatg gaccgcatgg gtgcaaactt cctgaaagta 360

gttggtcaga tcgaatctcg tctgggcgcg actccggttc cgctgcagat cgctatcggc 420

gctgaagaaa acttcaccgg cgttgttgac ctgatcaaaa tgaaagccat caactggaac 480

gaccaggatg cgggcgtgac cttcgattac gaagagatcc cagctaacct gcaagaactg 540

gctgaagaat ggcgcggcaa gctgatcgaa gctgcagcag aagcctctga agagctgatg 600

gagaaattct tcagcggtga agagctgact gaa 633

<210> 436
<211> 633
<212> DNA
<213> Pantoea terrea

<400> 436
ggtgtaaacc ataaaatcgg tgaagttcat gatggtgctg caaccatgga ctggatggct 60

caggagcagg aacgtggtat tactatcacc tccgctgcta ccaccgcttt ctggtctggt 120

atggcaaaac aatttgaacc acaccgcgtc aatatcattg ataccccggg acacgttgac 180

tttactatcg aagtagagcg ttcaatgcgt gttctggatg gtgcagtcat ggtttactgc 240

gcggtcggcg gcgttcagcc tcagtctgag actgtatggc gtcaggcgaa caaatataaa 300

gttccacgta tcgcgttcgt taacaaaatg gaccgtatgg gtgctaactt cctgaaagtt 360

gttggccaga tcaaaagccg tctgggtgct aaccctgttc cattgcagct ggcaatcggt 420

gcagaagaag gcttcaccgg tgttgttgac ctggtgaaaa tgaaagccat caactggaac 480

gaagcagacg caggtgtaac cttcgaatac gaagaaatcc ctgctgatct ggtcgatctg 540

gctgacgagt ggcaccagaa cctggttgaa gccgctgcag aagcttctga agagctgatg 600

gagaaatacc tcggcggtga agaactgact gaa 633

<210> 437
<211> 633
<212> DNA
<213> Pectobacterium atrosepticum

<400> 437
ggtgtaaatc ataaaatcgg tgaagttcat gacggcgcag ctaccatgga ctggatggag 60

caggagcagg aacgtggtat tactatcact tccgcagcga ctactgcatt ctggtctggt 120

atggctaagc agtatgaacc gcatcgcgta aacatcatcg acaccccagg acacgttgac 180

ttcactatcg aagtagaacg ttccatgcgt gtactggatg gtgcggtaat ggtttactgc 240

gcagttggtg gtgttcagcc gcagtctgaa accgtatggc gtcaggcaaa caaatataaa 300

gttccacgca ttgcgttcgt taacaaaatg gaccgcatgg gtgcgaattt cctgaaagtt 360

gttggtcaga tcaaagctcg tctgggcgcg aaccctgttc cgctacagtt ggctattggt 420

gctgaagaag gtttcaccgg tgttgttgac ctggtgaaaa tgaaagccat caactggaac 480

gatgccgatc aaggcgttac cttcacttat gaagatatcc ctgctgagat gcaggaactg 540

gctgacgaat ggcaccagaa cctgatcgaa tccgcagctg aagcttctga agagctgatg 600

gagaaatacc tgggtggtga agaactgact gaa 633

<210> 438
<211> 633
<212> DNA
<213> Pectobacterium betavasculorum

<400> 438
ggtgtaaacc ataaaatcgg tgaagttcat gacggcgcag ctaccatgga ctggatggag 60

caggagcagg aacgtggtat tactatcact tccgcagcaa ctactgcatt ctggtctggt 120

atggctaagc agtatgaacc gcatcgcgta aacatcatcg acaccccagg acacgttgac 180

ttcactatcg aagtagaacg ttctatgcgt gtacttgatg gtgcggtaat ggtttactgc 240

gcagttggtg gtgttcagcc gcagtctgaa accgtatggc gtcaggcaaa caaatataaa 300

gttccacgca ttgcgttcgt taacaaaatg gaccgcatgg gtgcgaattt cctgaaagtt 360

gttggtcaga tcaaatcccg tctgggcgcg aaccctgttc cgttgcaatt ggcgattggt 420

gctgaagaag gtttcaccgg tgttgttgac ctggtgaaaa tgaaagccat caactggaat 480

gatgccgatc agggcgtgac cttcgaatac gaagatatcc ctgctgatat gcaggatctg 540

gctgacgaat ggcaccagaa cctgatcgaa tccgcagcag aagcttctga agagctgatg 600

gagaagtatc tgggtggtga agaactgact gaa 633

<210> 439
<211> 633
<212> DNA
<213> Pectobacterium cacticida

<400> 439
ggtgtaaacc ataaaatcgg tgaagtccac gacggcgcgg ctaccatgga ctggatggaa 60

caggagcagg aacgtggtat tactatcact tctgcagcga cgacggcatt ctggtctggc 120

atggccaagc agtatgaacc gcatcgcgta aacatcattg atactccggg acacgttgac 180

ttcactatcg aagtagagcg ttccatgcgt gtacttgatg gtgcggtgat ggtttactgc 240

gcagttggtg gtgttcagcc acagtctgaa accgtatggc gtcaggcaaa caaatataaa 300

gttccacgca ttgcgttcgt aaacaaaatg gaccgcatgg gtgctaactt cctaaaagtt 360

gttggtcaga ttaaaacccg tttgggcgcg aatcctgttc cgttacagct ggcaattggt 420

gctgaagaag gtttcaccgg tgttgttgat ctggtgaaaa tgaaagcgat caactggaat 480

gatgccgatc agggcgtgac cttcacatat gaagatattc cggctgatat gcaggagtta 540

gctgatgaat ggcaccaaaa tctgattgaa tccgccgccg aagcttctga agagttgatg 600

gagaaatatc tgggcggtga agaactgact gag 633

<210> 440
<211> 633
<212> DNA
<213> Pectobacterium carotovorum sbsp. carotovorum T

<400> 440
ggtgtaaacc ataaaatcgg tgaagttcat gacggcgcag ctaccatgga ctggatggag 60

caggagcagg agcgtggtat tactatcacc tccgcagcga ctactgcatt ctggtctggt 120

atggctaagc agtatgaacc gcatcgcgta aacatcatcg acaccccggg acacgttgac 180

ttcactatcg aagtagaacg ttccatgcgt gtactggatg gtgcggtaat ggtttactgc 240

gcagttggtg gtgttcagcc gcagtctgaa accgtatggc gtcaggcaaa caaatataaa 300

gttccacgca ttgcgttcgt taacaaaatg gaccgcatgg gtgcgaattt cctgaaagtt 360

gttggtcaga tcaaatcccg tctgggcgcg aaccctgttc cgctgcagct ggcaattggt 420

gctgaagaag gtttcaccgg tgttgttgac ctggtgaaaa tgaaagccat caactggaac 480

gatgccgatc agggcgtgac cttcgaatac gaagagatcc cagctgatat gcaggatctg 540

gctgaagaat ggcaccaaaa cctgatcgaa tccgcagcag aagcttctga agagctgatg 600

gagaaatacc tgggtggtga agaactgact gaa 633

<210> 441
<211> 633
<212> DNA
<213> Pectobacterium carotovorum sbsp. odoriferum

<400> 441
ggtgtaaacc ataaaatcgg tgaagttcat gacggcgcag ctaccatgga ctggatggag 60

caggagcagg agcgtggtat tactatcact tccgctgcga ctactgcatt ctggtctggc 120

atggctaagc agtatgaacc gcatcgcgta aacatcatcg acaccccggg acacgttgac 180

ttcactatcg aagtagaacg ttccatgcgt gtactggatg gtgcggtaat ggtttactgc 240

gcagttggtg gtgttcagcc gcagtctgaa accgtatggc gtcaggcaaa caaatataaa 300

gttccacgca ttgcgttcgt taacaaaatg gaccgcatgg gtgcgaattt cctgaaagtt 360

gttggtcaga tcaaatcccg tctgggcgcg aaccctgttc cgctgcaact ggcaattggt 420

gctgaagaag atttcactgg tgttgttgac ctggtgaaaa tgaaagccat caactggaac 480

gatgccgatc agggcgtaac cttcgaatac gaagagatcc cagctgattt gcaggatctg 540

gctgaagaat ggcaccaaaa cctgatcgaa tccgcagcag aagcttctga agagctgatg 600

gagaaatacc tgggtggtga agaactgact gaa 633

<210> 442
<211> 633
<212> DNA
<213> Pectobacterium cypripedii

<400> 442
ggtgttaacc acaaaatcgg tgaagtgcat gatggcgcag cgaccatgga ctggatggcc 60

caggagcagg agcgtggtat tactatcacc tctgcagcga ctaccgcatt ctggtctggt 120

atggctaagc agtttgagcc gcaccgcatc aacatcatcg acaccccggg acacgttgac 180

ttcaccatcg aagtagaacg ttccatgcgt gtgctcgacg gcgcagtaat ggtttactgt 240

gcagtaggtg gcgtgcagcc gcagtctgaa accgtatggc gtcaggctaa caaatataaa 300

gttccacgca ttgcgttcgt taacaaaatg gaccgcatgg gtgcaaactt cctgaaagtt 360

gtaggccaga ttgaaagccg tctgggcgcg actccggtac ctctgcagct ggctatcggc 420

gcagaagagc acttcaccgg tgttgttgac ctgatcaaaa tgaaagccat caactggaac 480

gatgaagatc agggcgttac cttcgtttac gaagatatcc cggctaacat gcaggaactg 540

gctgaagaat ggcgccagaa actgatcgag tctgctgctg aagcttctga cgagctgatg 600

gagaaattct ttggtggcga agagctgtct gaa 633

<210> 443
<211> 633
<212> DNA
<213> Pectobacterium wasabiae

<400> 443
ggtgtaaacc ataaaatcgg tgaagttcat gacggcgcag ctaccatgga ctggatggag 60

caggagcagg aacgtggtat tactatcact tccgcagcga ctactgcatt ctggtctggt 120

atggctaagc agtatgaacc gcatcgcgta aacatcatcg acaccccagg acacgttgac 180

ttcactatcg aagtagaacg ttctatgcgt gtgcttgatg gtgcggtaat ggtttactgc 240

gcagttggtg gtgttcagcc gcagtctgaa accgtatggc gtcaggcaaa caaatataaa 300

gttccacgca ttgcgttcgt taacaaaatg gaccgcatgg gtgcgaattt cctgaaagtt 360

gttggtcaga tcaaatctcg tctgggcgcg aaccctgttc cgctgcagtt ggcgattggt 420

gctgaagaag gtttcaccgg tgttgttgac ctggtgaaaa tgaaagccat caactggaat 480

gatgccgatc agggcgtgac cttcgaatac gaagatatcc cggctgatat gcaagatctg 540

gctgaagaat ggcaccagaa cctgatcgag tccgcagcag aagcttctga agagctgatg 600

gagaaatacc tgggtggtga agaactgact gaa 633

<210> 444
<211> 633
<212> DNA
<213> Photorhabdus asymbiotica sbsp. asymbiotica

<400> 444
ggtgtaaacc ataagatcgg tgaagttcat gatggcgctg ctactatgga ctggatggaa 60

caggagcagg agcgtggtat taccatcacc tccgcagcaa caactgcttt ctggtcaggt 120

atggctaagc agtttgacgc gcaccgtatc aatattatcg acaccccagg acacgttgac 180

ttcaccatcg aagtagaacg ttccatgcgt gtgcttgacg gtgccgtaat ggtttattgt 240

gctgttggtg gtgttcagcc acagtctgaa accgtatggc gtcaggcgaa taaatataga 300

gttccacgta tcgcgttcgt taacaaaatg gaccgtatgg gggcgaattt cttacgcgtt 360

gttgagcaga tcaaaactcg tctggcagca aacccagtac ctctgcaatt agccattggt 420

gcagaagatg cgttcaccgg tgttgttgac ctggtgaaaa tgaaagccat caactggaac 480

gatgaagatc agggaactac cttcacttat gaagatatcc cggctgatat gcaggatctg 540

gctgaagaat ggcgtaacaa cttgattgaa gccgctgcgg aagcatcaga agagctgatg 600

gaaaaatacc tcagtggtga agaactgact gaa 633

<210> 445
<211> 633
<212> DNA
<213> Photorhabdus asymbiotica sbsp. australis

<400> 445
ggtgtaaacc acaagatcgg tgaagttcat gatggcgctg ctactatgga ctggatggaa 60

caggagcagg agcgtggtat taccatcacc tccgcagcaa caactgcttt ctggtcaggc 120

atggcgaagc agtttgacgc gcaccgtatc aacattatcg acaccccagg ccacgttgac 180

ttcaccatcg aagtagaacg ttccatgcgt gtgcttgacg gtgcggtaat ggtttattgt 240

gcggttggtg gtgttcagcc acagtctgaa accgtatggc gtcaggcgaa taaatacaga 300

gttccacgta tcgcgttcgt taacaaaatg gaccgtatgg gggcgaattt cttacgcgtt 360

gttgagcaga tcaaaactcg tctggcagca aacccagtcc ctctgcaatt agccattggt 420

gcagaagatg ctttcaccgg tgttgttgac ctggtgaaaa tgaaagccat caactggaac 480

gatgaagatc agggaactac cttcgcttat gaagatatcc cagctgatat gcaggatctg 540

gcggaagaat ggcgtaacaa cttgattgaa gccgctgcgg aagcatcaga agaactgatg 600

gaaaaatacc tcggtggtga agaactgact gaa 633

<210> 446
<211> 633
<212> DNA
<213> Photorhabdus luminescens sbsp. akhurstii

<400> 446
ggtgtaaacc ataagatcgg tgaagttcat gatggcgctg ctactatgga ctggatggaa 60

caggagcagg agcgtggtat taccatcacc tccgcagcaa caactgcttt ctggtcaggt 120

atggctaagc agtttgacgc gcaccgtatt aacatcatcg acaccccagg gcacgttgac 180

ttcactatcg aagtagaacg ttccatgcgt gttcttgacg gtgcggtaat ggtttattgt 240

gcggttggtg gtgttcagcc acagtccgaa accgtatggc gtcaggcaaa taaatataga 300

gttccacgta tcgcgttcgt taacaaaatg gaccgtatgg gagcgaattt cttacgtgtt 360

gttgagcaga tcaaaactcg cttggcggca aacccagtac ctctgcaatt agccattggc 420

gcagaagatg ctttcaccgg tgttgttgac ctggtgaaaa tgaaagccat caactggaac 480

gatgaagatc agggaactac cttcacttat gaagatatcc cggctgagat gcaggatctg 540

gcggaagaat ggcgtaacaa tttgattgaa gccgctgcgg aagcatcaga agagctgatg 600

gaaaaatacc ttggcggtga agaactgact gaa 633

<210> 447
<211> 633
<212> DNA
<213> Photorhabdus luminescens sbsp. kayaii

<400> 447
ggtgtaaacc ataagatcgg tgaagttcat gatggcgctg ctactatgga ctggatggaa 60

caggagcagg agcgtggtat taccatcacc tccgcagcaa caactgcttt ctggtcaggt 120

atggctaagc agtttgatgc gcaccgtatc aacatcattg acactccagg gcacgttgac 180

ttcaccatcg aagtagaacg ttccatgcgt gttcttgacg gtgccgtaat ggtttattgt 240

gcggttggtg gtgttcagcc acagtccgaa accgtatggc gtcaggcaaa taaatataga 300

gttccacgta ttgcgttcgt taacaaaatg gaccgtatgg gagcgaattt cttacgcgtt 360

gttgagcagc tcaaaactcg cttggcggca aacccagtac ctctgcaatt agccattggt 420

gcagaagatg cgttcaccgg tgttgttgac ttggtgaaaa tgaaagccat caactggaac 480

gatgaagatc agggaaccac cttcacttac gaagatatcc cagctgatat gcaggatctg 540

gcggaagaat ggcgtagcaa tttgattgaa gccgctgcgg aagcatcaga agagctgatg 600

gaaaaatacc ttggcggtga agaactgact gaa 633

<210>  448
<211>  633
<212>  DNA
<213>  Photorhabdus luminescens sbsp. laumondii

<400>  448
ggtgtaaacc ataagatcgg tgaagttcat gatggcgctg ctactatgga ctggatggaa      60

caggagcagg agcgtggtat taccatcacc tccgcagcaa caactgcttt ctggtcaggt     120

atggctaagc agtttgatgc gcatcgtatc aacatcatcg acaccccagg gcacgttgac     180

ttcaccatcg aagtagaacg ttccatgcgt gttcttgatg gtgccgtaat ggtttattgt     240

gcggttggtg gtgttcagcc acagtccgaa accgtatggc gtcaagcaaa taaatataga     300

gttccacgta tcgcgttcgt taacaaaatg gaccgtatgg gagcgaattt cttacgcgtt     360

gttgagcagc tcaaaactcg cttggcggca aacccagtac ctctgcaatt agccattggt     420

gcagaagatg cgttcaccgg tgttgttgac ttggtgaaaa tgaaagccat caactggaac     480

gatgaagatc agggaaccac cttcacttac gaagatatcc cagctgatat gcaggatctg     540

gcggaagaat ggcgtagcaa tttgattgaa gccgctgcgg aagcatcaga agaactgatg     600

gaaaaatacc ttggcggtga agaactgact gaa                                  633

<210>  449
<211>  633
<212>  DNA
<213>  Photorhabdus luminescens sbsp. luminescens T

<400>  449
ggtgtaaacc ataagatcgg tgaagttcat gatggcgctg ctactatgga ctggatggaa      60

caggagcagg agcgtggtat taccatcacc tccgcagcga caactgcttt ctggtcaggt     120

atggctaagc agtttgacgc gcaccgtatt aacatcatcg acaccccagg acacgttgac     180

ttcactatcg aagtagaacg ttccatgcgt gttcttgacg gcgccgtaat ggtttattgt     240

gcggttggtg gtgttcagcc acagtccgaa accgtatggc gtcaggcgaa taaatataga     300

gttccacgta tcgcgttcgt taacaaaatg gaccgtatgg gagcgaattt cttacgcgtt     360

gtcgagcagc tcaaaacgcg cttggcggcg aacccagtac ctctgcaatt agccattggc     420

gcagaagatg cgttcaccgg tgttgttgac ctggtgaaaa tgaaagccat caactggaac     480

gatgaagatc agggaactac cttcacttat gaagatatcc cggctgatat gcaggatctg     540

gcggaagaat ggcgtaacaa tttgattgaa gccgctgcgg aagcatcaga agagctgatg     600

gaaaaatacc ttggcggtga agaactgact gaa                                  633

<210>  450
<211>  633
<212>  DNA
<213>  Photorhabdus luminescens sbsp. thracensis

<400>  450
ggtgtaaacc ataagatcgg tgaagttcat gatggcgctg ctactatgga ctggatggaa      60

caggagcagg agcgtggtat taccatcacc tccgcagcaa caactgcttt ctggtcaggt     120

atggctaagc agtttgacgc gcaccgtatc aatatcattg acaccccagg gcacgttgac 180

ttcactatcg aagtagaacg ttccatgcgt gttcttgacg gtgcggtcat ggtttattgt 240

gcggttggtg gtgttcagcc acagtctgaa accgtatggc gtcaggcgaa taaatataaa 300

gttccacgta ttgcgttcgt taacaaaatg gaccgtatgg gagcgaattt cttacgcgtt 360

gttgagcaga tcaaaactcg tttggctgca aacccagtac ctctgcaatt ggcaattggt 420

gcagaagatg gcttcaccgg tgttgttgac ctggtgaaaa tgaaagctat caactggaac 480

gaggaagatc agggaacttc cttcacttat gaagatgttc cggctgatat gcaggatctg 540

gctgaagaat ggcgtaacaa cttggttgaa gccgctgcgg aagcatcaga agagctgatg 600

gaaaaatacc tcggcggtga agagctgact gaa 633

<210> 451
<211> 633
<212> DNA
<213> Photorhabdus temperata sbsp. temperata

<400> 451
ggtgtaaacc ataagatcgg tgaagttcat gatggcgctg ctactatgga ctggatggaa 60

caggagcagg agcgtggtat taccatcacc tccgcagcaa caactgcttt ctggtcaggt 120

atggctaagc agtttgatgc gcaccgtatc aatatcatcg acaccccagg ccacgttgac 180

ttcactatcg aagtagaacg ttctatgcgt gtccttgacg gtgcggtcat ggtttactgt 240

gcggttggtg gtgttcagcc acagtctgaa accgtatggc gtcaggcgaa taaatataaa 300

gttccacgta tcgcgttcgt taacaaaatg gaccgtatgg gcgcgaattt cttacgcgtt 360

gttgagcaga tcaaaactcg tttggctgca aacccagtac ctctgcaatt ggcaattggt 420

gcagaagatg gcttcaccgg tgttgttgac ctggtgaaaa tgaaagctat caactggaac 480

gaggaagatc agggaactac cttcacttat gaagatattc cagctgatat gcaggatctg 540

gctgaagaat ggcgtaacaa cctggttgaa gccgctgcgg aagcatcaga agagctgatg 600

gaaaaatacc tcggcggtga agagctgacc gaa 633

<210> 452
<211> 633
<212> DNA
<213> Plesiomonas shigelloides T

<400> 452
ggtgtaaacc acaaaatcgg tgaagttcat gacggcgcag ccaccatgga ctggatggaa 60

caggaacaag agcgtggtat taccatcacc tccgctgcga ctactgcatt ctggtcaggt 120

atggctaagc agtatgaacc gcatcgcgta aacatcatcg acaccccagg acacgttgac 180

ttcaccatcg aagtagaacg ttctatgcgt gtactggacg gtgcagtaat ggtttactgt 240

gcggtaggtg gcgttcagcc acagtctgag accgtatggc gtcaggctaa caaatacaaa 300

gtacctcgta tcgcgttcgt taacaaaatg gaccgtatgg gtgctaactt cctgaaagtt 360

gttggtcaga tcaaaacccg tctgggcgcg aaccctgttc cgctgcagct ggcaattggt 420

gctgaagaag gcttcaccgg tgttatcgac ctggtgaaaa tgaaagccat caactggaac 480

gatgcagacc aaggcgtgac cttcgaatac gaagagatcc cagctgacat gcaggatctg 540

gctgacgaat ggcaccagaa cctgatcgaa tccgctgccg aagcttctga agagctgatg 600

gagaaatacc tgggtggtga agaactgact gaa 633

<210> 453
<211> 633
<212> DNA
<213> Pragia fontium T

<400> 453
ggtgtaaacc ataaaattgg tgaaactcac gaaggttctg cgactatgga ctggatggag 60

caggagcagg agcgtggtat tactatcacc tcagctgcga ctactgcgtt ctggtctggt 120

atggctaaac aatttgaacc ccatcgtgtc aatatcattg ataccccggg acacgtagac 180

tttactatcg aagtagagcg ctctatgcgt gttctggatg gtgcagtaat ggtttactgt 240

gcagttggtg gtgttcagcc tcagtctgaa actgtttggc gtcaggctaa caaatataaa 300

gttccacgca tcgcgttcgt taacaaaatg gaccgtatgg gtgctaactt cctgcgtgta 360

gttgagcagc ttaaatcacg tctggcagca aatgctgtgc ctattcagtt accaatcggt 420

gctgaagaca gctttactgg cgtaattgac ttgctgaaaa tgaaagcaat caactggagt 480

gatgctgacc aaggcgttag cttcacttat gaagatgttc cagcgaacct gttagaagaa 540

gctaaaaaat ggcagcaaaa tgcatttgaa gctgcagctg aagcatctga agagctgatg 600

gacaagtatt taggcggcga agagctgact gaa 633

<210> 454
<211> 633
<212> DNA
<213> Proteus hauseri

<400> 454
ggtgtaaacc ataaaattgg tgaaactcac gaaggttcag caacaatgga ctggatggag 60

caggagcagg agcgtggtat tactatcaca tccgcagcaa ctactgcatt ctggtctggt 120

atggctaaac agtttgagcc tcaccgtgtc aacatcatcg acaccccagg tcacgttgac 180

ttcactatcg aagttgaacg ttctatgcgt gttcttgatg gcgcggttat ggtttactgt 240

gcagttggtg gtgttcagcc tcagtcagaa acagtatggc gccaggctaa caaatatcat 300

gtaccacgta tcgcgttcgt taacaaaatg gaccgtatgg gtgcaaactt cctgcgtgtt 360

gttgagcaaa tcaaaacgcg tctggctgca aacccagttc ctcttcagat cccagtaggc 420

gctgaagaac atttcaccgg tgttgttgat ttaatcaaaa tgaaagcaat ccgttggagt 480

gaagaagatc aaggtgttac cttcgaatac gaagagattc ctgcagagct gcaagattta 540

gcagacgaat ggcacaacaa cctgatcgaa tctgctgctg aagcatcaga agaactgatg 600

gacaaatatc taggcggcga agaattgaca gaa 633

<210> 455
<211> 633
<212> DNA
<213> Proteus mirabilis

<400> 455
ggtgtaaacc ataaaattgg tgaaactcac gaaggttcag caacaatgga ctggatggag 60

caagagcagg aacgtggtat cactatcaca tccgctgcaa ctactgcatt ctggtctggt 120

atggctaaac agtttgagcc tcaccgtgta aacatcatcg acaccccagg acacgttgac 180

ttcactatcg aagtagaacg ttctatgcgt gttcttgatg gcgcggttat ggtttactgt 240

gcagttggtg gtgttcagcc tcagtcagaa acagtatggc gccaggctaa caaatatcat 300

gtaccacgta tcgcgttcgt taacaaaatg gaccgtatgg gtgcaaactt cttacgtgtt 360

gttgaacaaa tcaaaacacg tttagctgca aatccagttc cactgcaaat cccagtaggc 420

gctgaagaag atttcactgg tgttgttgac ctgatcaaaa tgaaagcgat tcgctggaat 480

gaagaagatc aaggcgtcac cttcgaatac gaagacattc ctgcagaact acaagattta 540

gcagatgagt ggcacaacaa cctgatcgaa tctgcggctg aagcatcaga agaactgatg 600

gacaaatatc taggcggtga agaactgtca gaa 633

<210> 456
<211> 633
<212> DNA
<213> Proteus myxofaciens

<400> 456
ggtgtaaacc ataaaattgg tgaaacccac gaaggttcag caacaatgga ctggatggag 60

caggagcagg aacgtggtat tactatcaca tctgcagcga ctactgcatt ctggtctggt 120

atggctaaac agtttgagcc tcaccgtgta aacatcatcg acaccccagg tcacgttgac 180

ttcacaatcg aagtagaacg ttctatgcgt gttcttgatg gcgcggttat ggtttactgt 240

gcagttggtg gtgttcagcc tcagtcagaa acagtatggc gccaggctaa caaatatcat 300

gtaccacgta tcgcgttcgt taataaaatg gaccgtatgg gtgcaaactt cctacgtgtt 360

gttgaacaaa tcaaaacacg tctggcagca actccagttc cactgcaaat tccagtaggt 420

gctgaagaac atttcaccgg tgttgttgac ttagtaaaaa tgaaagcaat ccgttggaat 480

gaagaagacc aaggtgtaac tttcgaatac gaagacattc ctgcagatct acaagattta 540

gcagaagaat ggcacaactt cctggttgaa tctgctgcag aagcatcaga agaactgatg 600

gacaaatatc tgggcggtga agaactgaca gaa 633

<210> 457
<211> 633
<212> DNA
<213> Proteus penneri

<400> 457
ggtgtaaacc ataaaattgg tgaaactcac gaaggttcag caacaatgga ctggatggag 60

caggagcagg aacgtggtat tactatcaca tccgcagcaa ctactgcatt ctggtctggt 120

atggctaaac agtttgagcc tcaccgtgta aacatcatcg acaccccagg acacgttgac 180

ttcacaatcg aagtagaacg ttctatgcgt gttcttgatg gcgcggttat ggtttactgt 240

gcagttggtg gtgttcagcc tcagtcagaa acagtatggc gccaggctaa caaatatcat 300

gtaccacgta tcgcgttcgt taacaaaatg gaccgtatgg gtgcaaactt cctgcgtgtt 360

gttgaacaaa tcaaaacacg tctggctgca aacccagttc cactgcaaat tccagtaggt 420

gctgaagaac atttcaccgg tgttgttgat ttaattaaaa tgaaatcaat ccgttggaat 480

gaagaagacc aaggtgttac cttcgaatac gaagacattc ctgcaaatct gcaagattta 540

gctgaagaat ggcacaacaa cctagtcgaa tcagcagctg aagcatcaga agaactgatg 600

gacaaatatc tgggcggcga agaactgaca gaa 633

<210> 458
<211> 633
<212> DNA
<213> Proteus vulgaris T

<400> 458
ggtgtaaacc ataaaattgg tgaaactcac gaaggttcag caacaatgga ctggatggag 60

caggagcagg agcgtggtat tactatcaca tccgcagcaa ctactgcatt ctggtctggt 120

atggctaaac agttcgagcc tcaccgtgta aacatcatcg acaccccagg acacgttgac 180

ttcacaatcg aagtagaacg ttctatgcgt gttcttgatg gcgcggttat ggtttactgt 240

gcagttggtg gtgttcagcc tcagtcagaa acagtatggc gccaggctaa caaatatcat 300

gtaccacgta tcgcgttcgt taataaaatg gaccgtatgg gtgcaaactt cctgcgtgtt 360

gttgaacaaa tcaaaacacg tctggcagca aacccagttc cactgcaaat cccagttggt 420

gctgaagaaa atttcaccgg tgttgttgat ttaatcaaaa tgaaagcaat ccgttggaat 480

gaagaagacc aaggtgttac cttcgaatac gaagacattc ctgcaaatct gcaagattta 540

gctgaagaat ggcacaataa cctggttgaa tctgcagctg aagcatcaga agaactgatg 600

gacaaatatc tgggcggtga agaactgtca gaa 633

<210> 459
<211> 633
<212> DNA
<213> Providencia alcalifaciens T

<400> 459
ggtgtaaacc ataagatcgg tgaaacccac gaaggttctg caacaatgga ctggatggag 60

caagagcaag agcgtggtat tactatcact tccgctgcga ctactgcatt ctggtctggt 120

atggctaaac agtatgaacc acaccgtatc aacatcatcg acaccccggg acacgttgac 180

ttcacaatcg aagtagaacg ttctatgcgt gttcttgatg gcgcagtaat ggtttactgt 240

gcagttggtg gtgttcagcc acagtctgaa accgtatggc gtcaggctaa caaatataaa 300

gttccacgta tcgcgttcgt taacaaaatg gaccgtatgg gtgcgaactt cttacgtgtt 360

gttgagcaat taaaaacacg tttagcagct actgcagttc cactgcaatt accagtcggc 420

gcagaagagt cgttcactgg tgttgttgac ttgctgaaaa tgaaagcaat caaatggaac 480

gacgaagacc aaggcgttac cttcgaatac gaagatatcc ctgcggacat gcaagagctg 540

gctgaagagt ggcacaacaa tctgatcgaa tccgctgcag aagcatcaga agaactgatg 600

gaaaaatatc tgggcggtga agaactgact gaa 633

<210> 460
<211> 633
<212> DNA
<213> Providencia heimbachae

<400> 460
ggtgtaaacc ataaaattgg tgaaactcac gaaggtgctg caacaatgga ctggatggag 60

caggagcaag agcgtggtat tactatcaca tctgctgcga ctactgcatt ctggtctggc 120

atggcaaaac agtatgagcc acaccgtatc aatatcatcg acaccccagg acacgttgac 180

ttcactatcg aagtagaacg ttcaatgcgt gttcttgatg gcgcagtaat ggtttactgt 240

gcagtcggtg gtgttcagcc gcagtctgaa actgtatggc gtcaagctaa taaatataaa 300

gttccacgta tcgcgttcgt taataaaatg gaccgtatgg gtgcgaacta cttacgtgtt 360

gttgagcaat tgaaaacacg tttagcggca aacccagttc cactgcaaat tccagttggc 420

gcagaagaat cattcactgg tgttgttgac ttgatcaaaa tgaaagcgat caagtggagt 480

gatgaagatc agggtgttac tttcgaatac gaagacatcc cagcaaatct gcaagaactg 540

tcagaagagt ggcacaacta cctagttgaa tccgcagcag aagcatcaga agaactgatg 600

gagaaatatc tgggcggaga agaactgact gaa 633

<210> 461
<211> 633
<212> DNA
<213> Providencia rettgeri

<400> 461
ggtgtaaacc ataaaattgg tgaaactcac gaaggttctg caacaatgga ctggatggag 60

caggagcaag agcgtggtat tactatcaca tctgcagcga ctactgcatt ctggtctggt 120

atggcaaaac agtatgagcc acaccgtatc aacatcatcg acaccccggg acacgttgac 180

ttcaccatcg aagtagaacg ttctatgcgt gttcttgatg gcgcagtaat ggtttactgt 240

gcggttggtg gtgttcagcc acagtctgaa actgtatggc gtcaggctaa caaatataaa 300

gttccacgta tcgcgttcgt taataaaatg gaccgtatgg gtgcgaactt cttacgtgtt 360

gttgagcaat taaaatcacg tttagctgct aacgcagttc cactgcaatt accagtcggt 420

gcagaagagt cgttcactgg tgttgttgac ttgctgaaaa tgaaggcaat caagtggagc 480

gacgaagacc agggcgttac cttcgaatac gaagatatcc ctgcgaacat gcaagaagct 540

gctgaagagt ggcacaacaa cctgatcgaa accgcagcag aagcatcaga agaactgatg 600

gaaaaatatc tgggcggtga agaactgacc gaa 633

<210> 462

<211>  633
<212>  DNA
<213>  Providencia rustigianii

<400>  462
ggtgtaaacc ataaaattgg tgaaactcac gaaggttctg caacaatgga ctggatggag      60

caggagcaag agcgtggtat tactatcact tccgctgcga ctacggcatt ctggtctggt     120

atggcaaaac agttcgagcc acaccgtatc aatatcatcg acaccccagg acacgttgac     180

ttcacaatcg aagtagaacg ttctatgcgt gttcttgatg gtgcagtaat ggtttactgt     240

gcggttggtg gtgttcagcc acagtctgaa actgtatggc gtcaggctaa caaatataaa     300

gttccacgta tcgcgttcgt taacaaaatg gaccgtatgg gtgcgaacta cttacgcgtt     360

gttgagcaat taaaaacgcg tttagcagct aacccagttc cacttcaaat tccagtaggc     420

gcagaagaag gcttcactgg tgttgttgac ctgatcaaaa tgaaagcgat caagtggagc     480

gatgaagacc aaggtgttac cttcgaatac gaagatatcc ctgcagatct tcaagactta     540

gctgaagaat ggcacaacta tttagttgaa tccgcagcag aagcatcaga agatctgatg     600

gaaaaatatc tgggcggtga agacctgact gaa                                  633

<210>  463
<211>  633
<212>  DNA
<213>  Providencia stuartii

<400>  463
ggtgtaaacc ataaaattgg tgaaactcac gaaggttctg caacaatgga ctggatggag      60

caggagcaag aacgtggtat tactatcacg tctgcagcga ctactgcatt ctggtctggt     120

atggcaaaac aattcgaacc acaccgtatt aacattatcg acaccccagg acacgttgac     180

ttcacaatcg aagtagaacg ttctatgcgt gttcttgatg gtgcggtcat ggtttactgt     240

gcagttggtg gtgttcagcc acagtctgaa acagtatggc gtcaggctaa caaatataaa     300

gttccacgta tcgcgttcgt taacaaaatg gaccgtatgg gtgcaaactt cctgcgtgtt     360

gttgatcaat taaaaacacg tttggctgca aacccagttc cacttcaact gccagttggt     420

gctgaagaag gcttcaccgg cgttgttgac ttgatcaaaa tgaaagcgat caagtggagc     480

gatgaagatc agggcgttac cttcgaatac gaagatatcc ctgcaaacat gcaagaacaa     540

gctgaagaat ggcataactt cctcgttgaa tctgctgctg aagcatcaga agaactgatg     600

gaaaaatatt tgggcggtga tgaactgtct gaa                                  633

<210>  464
<211>  633
<212>  DNA
<213>  Providencia vermicola

<400>  464
ggtgtaaacc ataaaattgg tgaaactcac gaaggttctg caacaatgga ctggatggag      60

caggagcaag agcgtggtat cactatcaca tctgctgcga ctactgcatt ctggtctggt     120

atggcaaaac agtatgagcc acaccgtgta aacatcatcg acaccccggg acacgttgac     180

ttcacaatcg aagtagaacg ttctatgcgt gttcttgatg gcgcagtaat ggtttactgt 240

gcggttggtg gtgttcagcc acagtctgaa actgtatggc gtcaggctaa caaatataaa 300

gttccacgta ttgcgttcgt taataaaatg gaccgtatgg gtgcgaactt cttacgtgtt 360

gttgagcaat taaaaacacg tttagcagct aacgcagttc cactgcaatt accagtcggc 420

gcagaagagt cgttcactgg tgttgttgac ttgctgaaaa tgaaagcaat caagtggagc 480

gatgaagacc aaggcgttac cttcgaatac gaagatatcc ctgcgaacat gcaagaagca 540

gctgaagagt ggcacaacaa cctgatcgaa accgcagcag aagcatcaga agaactgatg 600

gaaaaatatc tgggcggtga agaactgact gaa 633

<210> 465
<211> 633
<212> DNA
<213> Rahnella aquatilis T

<400> 465
ggtgtaaacc acaaaatcgg tgaagtgcat gatggcgcag ccaccatgga ctggatggag 60

caggagcagg aacgtggtat taccatcacg tccgctgcga ccacctgttt ctggtcaggt 120

atggctaagc agttcgaacc acaccacatc aacatcatcg acaccccagg gcacgttgac 180

ttcaccatcg aagttgaacg ttccatgcgt gttcttgatg gcgcggtaat ggtttactgt 240

gctgttggtg gtgttcagcc acagtctgaa accgtatggc gtcaggcaaa caaatataaa 300

gttcctcgta tcgcgttcgt taacaaaatg gaccgtatgg gtgctaactt cctgaaagtt 360

gttgaccaga ttgaaaaacg tctgggtgca actccggtgc ctctgcaact ggcaatcggc 420

gcggaagaaa aattcaccgg tgttgttgac ctggtgaaga tgaaagctat caactggaac 480

gaagctgatc agggcgttac cttcgaatac gaagaaatcc ctgccaacat gcaagaactg 540

gctgaagaat ggcgtcaaaa actggttgag tccgctgctg aaggctcaga tgagctgatg 600

gagaaattct ttggtggcga agagctgact gaa 633

<210> 466
<211> 633
<212> DNA
<213> Raoultella ornithinolytica

<400> 466
ggtgtaaacc acaaaatcgg tgaagttcat gacggcgcag caaccatgga ctggatggag 60

caggagcagg agcgtggtat cactatcacc tccgcagcga ctactgcatt ctggtctggt 120

atggctaagc agtatgaacc gcatcgcgtt aacatcatcg acaccccggg gcacgttgac 180

ttcactattg aagtagaacg ttccatgcgt gtgcttgatg gtgcggtaat ggtttactgc 240

gcagttggtg gtgttcagcc gcagtctgaa accgtatggc gtcaggcaaa caaatataaa 300

gttccgcgca ttgcgttcgt taacaaaatg gaccgcatgg gtgcgaactt cctgaaagtt 360

gttggtcaga tcaaaacccg tctgggcgcg aacccggttc cgctgcagct ggcaattggt 420

gctgaagaag gtttcaccgg tgttgttgac ctggtgaaaa tgaaagccat caactggaac 480

gaagaagacg caggcgtaac cttcacctat gaagatatcc cggctgacat gcaggatctg 540

gctgaagaat ggcaccagaa cctgatcgag tccgctgcag aagcttcaga agagctgatg 600

gagaaatacc tgggtggtga agaactgact gaa 633

<210> 467
<211> 633
<212> DNA
<213> Raoultella planticola T

<400> 467
ggtgtaaacc acaaaatcgg tgaagttcat gacggcgcag caaccatgga ctggatggag 60

caggagcagg agcgtggtat cactatcacc tccgcagcga ctactgcatt ctggtctggt 120

atggctaagc agtatgaacc gcatcgcgtt aacatcatcg acaccccggg gcacgttgac 180

ttcactatcg aagtagaacg ttccatgcgt gtgcttgatg gtgcggtaat ggtttactgc 240

gcagttggtg gtgttcagcc gcagtctgaa accgtatggc gtcaggcaaa caaatataaa 300

gttccgcgca ttgcgttcgt taacaaaatg gaccgcatgg gtgcgaactt cctgaaagtt 360

gttggtcaga tcaaaacccg tctgggcgcg aacccggttc cgctgcagct ggcaattggt 420

gctgaagaag gtttcaccgg tgttgttgac ctggtgaaaa tgaaagccat caactggaac 480

gaagaagacg caggcgtaac cttcacctat gaagatatcc cggctgacat gcaggatctg 540

gctgaagaat ggcaccagaa cctgatcgag tccgctgcag aagcttcaga agagctgatg 600

gagaaatacc tgggtggtga agaactgact gaa 633

<210> 468
<211> 633
<212> DNA
<213> Raoultella terrigena

<400> 468
ggtgtaaacc acaaaatcgg tgaagttcat gatggcgcag ccaccatgga ctggatggag 60

caggagcagg aacgtggtat taccatcacg tccgctgcga ctacctgttt ctggtcagga 120

atggctaaac agttcgaacc acaccacatc aacatcatcg acaccccagg gcacgttgac 180

ttcactatcg aagtagaacg ttccatgcgt gttcttgatg gtgcggtaat ggtttactgt 240

gctgttggtg gtgttcagcc acagtctgaa accgtatggc gtcaggcaaa caaatataaa 300

gttccacgta tcgcgttcgt taacaaaatg gaccgtatgg gtgctaactt cctgaaagtt 360

gttgatcaga ttgaaaagcg tctgggcgcg aacccggtcc ctctgcagct ggctatcggc 420

gcagaagaga aattcaccgg tgttgttgac ctggtgaaaa tgaaagctat caactggaac 480

gaagccgatc agggcgtgac cttcgaatac gaagagatcc cagctgatat gcaggaactg 540

gctgaagaat ggcgccagaa actggttgaa gccgctgctg aaggttctga cgaactgatg 600

gagaaattct ttggtggcga agagctgact gaa 633

<210> 469
<211> 633

<212>  DNA
<213>  Salmonella bongori

<400>  469
ggtgtaaacc acaaaatcgg tgaagttcat gaaggcgccg caaccatgga ctggatggag      60

caggagcagg agcgtggtat taccattacc tccgcagcaa ctactgcatt ctggtctggt     120

atggctaagc agtatgaacc gcaccgcatc aacatcatcg acaccccggg gcacgttgac     180

ttcactatcg aagtagaacg ttccatgcgt gttcttgacg gtgcggtaat ggtttactgt     240

gcagttggtg gtgttcagcc gcagtctgaa accgtatggc gtcaggcgaa caaatataaa     300

gttccgcgca tcgcgttcgt taacaaaatg gaccgtatgg gcgcgaactt cctgaaagtt     360

gttggtcaga tcaaaacccg tctgggcgcg aacccggttc cgctgcagtt ggcgattggc     420

gctgaagaag cgttcactgg tgttgttgac ctggtgaaaa tgaaagccat caactggaat     480

gaagaagacc gtggtgtaac cttcacttac gaagatatcc cggctgacat gcaggaactg     540

gctgaagaat ggcaccagaa cctgatcgag tccgctgcgg aagcatctga agagctgatg     600

gagaaatacc tgggcggtga agaactgact gaa                                  633

<210>  470
<211>  633
<212>  DNA
<213>  Salmonella enterica sbsp. arizonae

<400>  470
ggtgtaaacc acaaaatcgg tgaagttcat gacggcgcag ccaccatgga ctggatggag      60

caggagcagg agcgtggtat tactatcacc tccgcagcga ctactgcatt ctggtctggt     120

atggctaagc agtatgaacc gcatcgcatc aacatcatcg acaccccggg gcacgttgac     180

ttcactatcg aagtagaacg ttccatgcgt gttctcgatg gtgcggtaat ggtttactgc     240

gcagttggtg gtgttcagcc gcagtctgaa accgtatggc gtcaggcaaa caaatacaaa     300

gttccgcgta ttgcgttcgt taacaaaatg gaccgcatgg gcgcgaactt cctgaaagtt     360

gttggtcaga tcaaaacccg tctgggcgcg aacccggttc cgctgcagct ggcaattggt     420

gctgaagaag gtttcaccgg cgttgttgac ctggtgaaaa tgaaagccat caactggaac     480

gatgccgacc agggcgttac cttcgaatac gaagatatcc cggctgacat gcaggacctg     540

gctaacgaat ggcaccagaa cctgatcgag tccgccgcag aagcttctga agagctgatg     600

gaaaaatacc tgggtggtga agaactgact gaa                                  633

<210>  471
<211>  633
<212>  DNA
<213>  Salmonella enterica sbsp. diarizonae

<400>  471
ggtgtaaacc acaaaatcgg tgaagttcat gacggcgcag ctaccatgga ctggatggag      60

caggagcagg agcgtggtat tactatcacc tccgcagcga ctactgcatt ctggtctggt     120

atggctaagc agtatgaacc gcatcgcatc aacatcatcg acaccccggg gcacgttgac     180

ttcaccatcg aagtagaacg ttccatgcgt gttctcgatg gtgcggtaat ggtttactgc 240

gcagttggtg gtgttcagcc gcagtctgaa accgtatggc gtcaggcaaa caaatacaaa 300

gttccgcgta ttgcgttcgt taacaaaatg gaccgcatgg gcgcgaactt cctgaaagtt 360

gttggtcaga tcaaaacccg tctgggcgcg aacccggttc cgctgcagtt ggcaattggt 420

gctgaagaag gtttcaccgg tgttgttgac ctggtgaaaa tgaaagccat caactggaac 480

gaagcagacg caggcgttac cttcacttat gaagatatcc cggctgacat gcaggacctg 540

gctgacgaat ggcaccagaa cctgatcgag tccgctgcgg aagcttctga agagctgatg 600

gaaaaatacc tgggcggtga agaactgact gaa 633

<210> 472
<211> 633
<212> DNA
<213> Salmonella enterica sbsp. enterica T

<400> 472
ggtgtaaacc acaaaatcgg tgaagttcat gacggcgcag ctaccatgga ctggatggag 60

caggagcagg agcgtggtat tactatcacc tccgcagcga ctactgcatt ctggtctggt 120

atggctaagc agtatgaacc gcatcgcatc aacatcatcg acaccccggg gcacgttgac 180

ttcactatcg aagtagaacg ttccatgcgt gttctcgatg gtgcggtaat ggtttactgc 240

gcagttggtg gtgttcagcc gcagtctgaa accgtatggc gtcaggcaaa caaatacaaa 300

gttccgcgta ttgcgttcgt taacaaaatg gaccgcatgg gcgcgaactt cctgaaagtt 360

gttggtcaga tcaaaacccg tctgggcgcg aacccggttc cgctgcagct ggcaattggt 420

gctgaagaag gtttcaccgg cgttgttgac ctggtgaaaa tgaaagccat caactggaac 480

gatgccgacc agggcgttac cttcgaatac gaagatatcc cggctgacat gcaggacctg 540

gctaacgaat ggcaccagaa cctgatcgag tccgccgcag aagcttctga agagctgatg 600

gaaaaatacc tgggtggtga agaactgact gaa 633

<210> 473
<211> 633
<212> DNA
<213> Salmonella enterica sbsp. houtenae

<400> 473
ggtgtaaacc acaaaatcgg tgaagttcat gacggcgcag ctaccatgga ctggatggag 60

caggagcagg agcgtggtat tactatcacc tccgcagcga ctactgcatt ctggtctggt 120

atggctaagc agtatgaacc gcatcgcatc aacatcatcg acaccccggg gcacgttgac 180

ttcactatcg aagtagaacg ttctatgcgt gtgcttgatg gtgcggtaat ggtttactgc 240

gcagttggtg gtgttcagcc gcagtctgaa accgtatggc gtcaggcaaa caaatataaa 300

gttccgcgta ttgcgttcgt taacaaaatg gaccgcatgg gcgcgaactt cctgaaagtt 360

gttggtcaga tcaaaacccg tctgggcgcg aatccggttc cgctgcagtt ggcgattggt 420

gctgaagaag gtttcaccgg tgttgttgac ctggtgaaaa tgaaagccat caactggaac 480

gaagcagacg caggcgttac cttcgaatac gaagatatcc cggctgacat gcaggaactg 540

gctgaagaat ggcaccagaa cctgatcgag tccgctgcgg aagcttcaga agagctgatg 600

gaaaaatacc tgggtggtga agaactgact gaa 633

<210> 474
<211> 633
<212> DNA
<213> Salmonella enterica sbsp. indica

<400> 474
ggtgtaaacc acaaaatcgg tgaagttcat gacggcgcag ctaccatgga ctggatggag 60

caggagcagg agcgtggtat tactatcacc tccgcagcga ctactgcatt ctggtctggt 120

atggctaagc agtatgaacc gcatcgcatc aacatcatcg acaccccggg gcacgttgac 180

ttcaccatcg aagtagaacg ttccatgcgt gttctcgatg gtgcggtaat ggtttactgc 240

gcagttggtg gtgttcagcc gcagtctgaa accgtatggc gtcaggcaaa caaatacaaa 300

gttccgcgca ttgcgttcgt taacaaaatg gaccgcatgg gcgcgaactt cctgaaagtt 360

gttggtcaga tcaaaacccg tctgggcgcg aacccggttc cgctgcagct ggcaattggt 420

gctgaagaag gtttcaccgg tgttgttgac ctggtgaaaa tgaaagccat caactggaac 480

gaagcagacg caggcgttac cttcacttat gaagatatcc cggctgacat gcaggaactg 540

gctgacgaat ggcaccagaa cctgattgag tccgctgcgg aagcttctga agagctgatg 600

gaaaaatacc tgggcggtga agaactgact gaa 633

<210> 475
<211> 633
<212> DNA
<213> Salmonella enterica sbsp. salamae

<400> 475
ggtgtaaacc acaaaatcgg tgaagttcat gacggcgcag ctaccatgga ctggatggag 60

caggagcagg agcgtggtat tactatcacc tccgcagcga ctactgcatt ctggtctggt 120

atggctaagc agtatgaacc gcatcgcatc aacatcatcg acaccccggg gcacgttgac 180

ttcaccatcg aagtagaacg ttccatgcgt gttctcgatg gtgcggtaat ggtttactgc 240

gcagttggtg gtgttcagcc gcagtctgaa accgtatggc gtcaggcaaa caaatacaaa 300

gttccgcgta ttgcgttcgt taacaaaatg gaccgcatgg gcgcgaactt cctgaaagtt 360

gttggtcaga tcaaaacccg tctgggcgcg aacccggttc cgctgcagct ggcaattggt 420

gctgaagaag gtttcaccgg cgttgttgac ctggtgaaaa tgaaagccat caactggaac 480

gaagcagacg caggcgttac cttcgaatac gaagatatcc cggctgacat gcaggaactg 540

gctgacgaat ggcaccagaa cctgatcgag tccgctgcgg aagcttctga agagctgatg 600

gaaaaatacc tgggcggtga agaactgact gaa 633

<210> 476
<211> 633
<212> DNA

<213>  Samsonia erythrinae T

<400>  476
ggtgtaaatc ataaaatcgg tgaagttcat gacggcgctg ccaccatgga ctggatggag      60

caggagcagg aacgtggtat tactatcact tccgcagcga ctactgcatt ctggtctggt     120

atggccaagc agtatgaacc gcatcgcgta aacatcatcg ataccccggg acacgttgac     180

ttcactatcg aagtagaacg ttccatgcgt gttcttgatg gtgcggtaat ggtttactgc     240

gcagttggtg gtgttcagcc gcagtctgaa accgtatggc gtcaggcaaa caaatataaa     300

gttccgcgca ttgcgttcgt taacaaaatg gaccgtatgg gtgcgaactt cctgaaagtt     360

gttggtcaga tcaaaacccg tctgggcgcg aatccggttc cgcttcagtt ggcgattggc     420

gctgaagaag ggttcaccgg tgttgttgac ctggtgaaaa tgaaggccat caactggaac     480

gaagcagatg caggcgttac cttcacttac gaagatatcc cggctgacat gcaggatctg     540

gctgaagaat ggcaccagaa cctgatcgag tccgctgcgg aagcttctga agagctgatg     600

gagaaatacc tgggtggtga agaactgtct gaa                                  633

<210>  477
<211>  633
<212>  DNA
<213>  Serratia ficaria

<400>  477
ggtgtaaacc acaaaatcgg tgaagttcat gacggcgctg ccaccatgga ctggatggaa      60

caggagcagg agcgtggtat taccatcact tctgcagcga ctactgcctt ctggtctggt     120

atggccaagc agtttgaacc gcaccgcgta aacatcatcg acaccccggg acacgttgac     180

ttcaccatcg aagtagaacg ttccatgcgt gttcttgatg gtgcggtaat ggtttactgt     240

gctgttggtg gtgttcagcc acagtctgaa actgtatggc gtcaggctaa caaatacaaa     300

gttccacgca tcgcgttcgt taacaaaatg gaccgcatgg gtgccaactt cctgaaagtt     360

gttggtcaga tcaaatctcg tctgggcgct aacccggttc cactgcagct ggctatcggc     420

gcagaagaga aattcaccgg cgttatcgac ctggtgaaaa tgaaagccat caactggaac     480

gaagctgatg caggcgtgac cttcgaatac gaagagatcc cggctgacat gcaagagctg     540

gccgcagaat ggcgccagaa cctggttgaa tcggcagctg aagcttctga tgaactgatg     600

gacaagtact tgggcggcga agagctgacc gag                                  633

<210>  478
<211>  633
<212>  DNA
<213>  Serratia fonticola

<400>  478
ggtgtaaacc acaaaatcgg tgaagttcat gacggcgccg ccaccatgga ctggatggaa      60

caagagcagg agcgtggtat taccattact tctgcagcga caactgcttt ctggtctggt     120

atggccaagc agtttgagcc gcaccgtgta aacatcatcg acaccccagg gcacgttgac     180

ttcaccatcg aagtagaacg ttccatgcgt gtgcttgatg gcgcggtaat ggtttactgt     240

gctgttggtg gtgttcagcc acagtctgaa accgtatggc gtcaggctaa caaatacaaa 300

gtgccacgca tcgcgttcgt taacaaaatg gaccgtatgg gtgccaactt cctgaaagtt 360

gttggtcagc ttaaatctcg tctgggcgca aaccctgttc cattgcagct ggcaattggc 420

gcagaagaga aattcaccgg tgttgttgac ctggtgaaaa tgaaagccat caactggaac 480

gaagctgatg caggcgtgac cttcgaatac gaagagatcc cagctgacat gcaagaactg 540

gctgctgaat ggcaccagaa tctggttgag tctgctgctg aagcctctga tgagctgatg 600

gacaaatacc tgggcggcga agagctgacc gag 633

<210> 479
<211> 633
<212> DNA
<213> Serratia grimesii

<400> 479
ggtgtaaacc acaaaatcgg tgaagttcat gatggcgcag ccaccatgga ctggatggaa 60

caggagcagg agcgtggtat taccatcact tctgcagcga ctactgcttt ctggtctggt 120

atggccaagc agttcgagcc gcaccgcgta aacatcatcg acaccccagg gcacgttgac 180

ttcaccatcg aagtagaacg ttccatgcgt gttcttgatg gtgcggtaat ggtttactgt 240

gctgttggtg gtgttcagcc gcagtctgaa accgtatggc gtcaggctaa caaatacaaa 300

gttccacgca tcgcgttcgt taacaaaatg gaccgcatgg gtgctaactt cctgaaagtt 360

gttggtcaga tcaaatctcg tctgggcgcg aatccagttc cattgcaact ggcaatcggc 420

gcggaagaga aattcaccgg tgttgttgac ctggtgaaaa tgaaagctat caactggaac 480

gaagctgatg ctggcgtgac cttcgaatac gaagagatcc ctgcagacat gcaagaactg 540

gctgctgaat ggcaccagaa cctggttgaa tctgcagctg aagcttcaga tgagctgatg 600

gacaaatacc tgggcggcga agagctgacc gag 633

<210> 480
<211> 633
<212> DNA
<213> Serratia liquefaciens

<400> 480
ggtgtaaacc acaaaatcgg tgaagttcat gacggcgctg ccaccatgga ctggatggaa 60

caggagcagg agcgtggtat taccatcact tctgcagcga ctactgcttt ctggtctggt 120

atggccaagc agtttgagcc gcaccgcgta aacatcatcg acaccccagg acacgttgac 180

ttcaccatcg aagtagaacg ttccatgcgt gttcttgatg gcgctgtaat ggtttactgt 240

gctgtaggtg gtgttcagcc tcagtctgaa actgtatggc gtcaggctaa caaatacaaa 300

gttccacgca tcgcgttcgt taacaaaatg gaccgcatgg gtgctaactt cctgaaagtt 360

gttggtcaga tcaaatctcg tctgggcgcg aatccagtgc cattgcagct ggctatcggc 420

gcagaagaga aattcaccgg tgttgttgac ctggtgaaaa tgaaagcgat caactggaac 480

gaagctgatg ctggcgtgac cttcgaatac gaagacatcc ctgctgacat gcaagagctg 540

gctgaagaat ggcgccagaa cctggttgaa tctgcagctg aagcttcaga tgagctgatg 600

gacaaatacc tgggcggcga agagctgacc gag 633

<210> 481
<211> 633
<212> DNA
<213> Serratia marcescens sbsp. sakuensis

<400> 481
ggtgtaaacc acaaaatcgg tgaagttcat gacggcgctg ccaccatgga ctggatggaa 60

caggagcagg agcgtggtat taccatcact tctgcagcga ctactgcctt ctggtctggt 120

atggccaagc agttcgaacc gcatcgcgta aacatcatcg acaccccagg gcacgttgac 180

ttcaccatcg aagtagaacg ttccatgcgt gtgctggatg gcgcggtaat ggtttactgt 240

gctgttggtg gtgttcagcc acagtctgaa accgtatggc gtcaggctaa caaatacaaa 300

gtgccacgta tcgcgttcgt taacaaaatg gaccgcatgg gcgctaactt cctgaaagtt 360

gttggccaga tcaaatctcg tctgggcgcc aaccctgttc ctctgcagct ggctatcggc 420

gcagaagaca aattcaccgg cgttatcgac ctggtgaaga tgaaggcgat caactggaac 480

gaagaagacg caggcgtaac cttcgaatac gaagacgtgc cggctgacat gatggatctg 540

gctgaagaat ggcgccagaa cctgatcgag tccgctgcgg aagcttccga agagctgatg 600

gaaaaatacc tgggcggcga agagctgacc gag 633

<210> 482
<211> 633
<212> DNA
<213> Serratia odorifera

<400> 482
ggtgtaaacc acaaaatcgg tgaagttcat gacggcgcag ctaccatgga ctggatggag 60

caggagcagg agcgtggtat taccattacc tccgctgcaa ctactgcatt ctggtcaggt 120

atggccaagc agtatgaacc gcatcgcgta aacatcatcg acaccccagg gcacgttgac 180

ttcaccatcg aagttgaacg ttccatgcgt gtgcttgatg gcgcggtaat ggtttactgt 240

gctgttggtg gtgttcagcc acagtctgaa accgtatggc gtcaggctaa caaatacaaa 300

gttccacgca tcgcgttcgt taacaaaatg gaccgcatgg gcgcgaactt cctgaaagtt 360

gtaggccaga tcaaatctcg cctgggtgca aacccggttc ctctgcagct ggctatcggc 420

gcagaagaga aattcaccgg tgttgttgac ctggtgaaga tgaaagccat caactggaac 480

gaagccgatg caggcgtgac cttcgaatac gaagatatcc cggctgacat gcaggaactg 540

gctgaagaat ggcaccagaa tctggttgag tctgctgccg aagcttcaga agagctgatg 600

gaaaaatacc tgggcggcga agagctgacc gag 633

<210> 483
<211> 633
<212> DNA
<213> Serratia plymuthica

<400>  483
ggtgtaaacc acaaaatcgg tgaagttcat gacggcgctg ccaccatgga ctggatggag      60

caggagcagg agcgtggtat taccatcact tctgcagcga ctaccgcttt ctggtctggt     120

atggccaagc agtttgagcc gcaccgcgta aacatcatcg acacaccggg acacgttgac     180

ttcaccatcg aagtagaacg ttccatgcgt gttcttgatg gcgctgtaat ggtttactgt     240

gctgttggtg gtgttcagcc acagtctgaa actgtatggc gtcaggctaa caaatataaa     300

gttccacgca tcgcgttcgt taacaaaatg gaccgcatgg gtgctaactt cctgaaagtt     360

gttggtcaga tcaaatcccg tctgggcgcg aatccggttc cattgcaact ggccatcggc     420

gcagaagaga aattcaccgg tatcgttgac ctggtgaaaa tgaaagcagt caactggaac     480

gatgctgacc agggcgtgac cttcgaatac gaagagatcc ctgcggacat gcaagcgctg     540

gccgaagaat ggcaccagaa cctggttgaa tctgcagctg aagcttcaga cgagctgatg     600

gaaaaatacc tgggcggcga agagctgacc gag                                  633

<210>  484
<211>  633
<212>  DNA
<213>  Serratia quinivorans

<400>  484
ggtgtaaacc acaaaatcgg tgaagtgcac gacggcgcag ctaccatgga ctggatggag      60

caggagcagg aacgtggtat taccatcacg tccgctgcga ctacctgttt ctggtctggt     120

atggctaagc agttcgacgc acaccacatc aacatcatcg acaccccagg acacgttgac     180

ttcaccatcg aagtagaacg ttccatgcgt gttcttgatg gcgcggtaat ggtttactgt     240

gcagttggtg gcgttcagcc acagtctgaa accgtatggc gtcaggctaa caaatataaa     300

gttccacgca tcgcgttcgt taacaaaatg gaccgcatgg gtgctaactt cctgaaagtt     360

gttgatcaga ttgaaaaacg tctgggcgca actccggtcc ctctgcagct ggctatcggc     420

gcagaagaga aattcaccgg tgttgttgac ctggtgaaaa tgaaagccat caactggaac     480

gaagccgatc agggcgtgac cttcgaatac gaagagatcc cggctgatat gcaagaactg     540

gctgaagaat ggcgtcagaa gctggttgag tccgctgctg aaggttctga tgagctgatg     600

gagaaattct ttggtggcga agagctgact gaa                                  633

<210>  485
<211>  633
<212>  DNA
<213>  Serratia rubidaea

<400>  485
ggtgtaaacc ataaactcgg tgaagttcat gacggcgctg ccaccatgga ctggatggaa      60

caggagcagg agcgtggtat taccatcact tctgcagcga caactgcttt ctggtctggt     120

atggccaagc agtatgaacc gcaccgcgta aacatcatcg acaccccggg acacgttgac     180

ttcaccatcg aagtagaacg ttccatgcgt gtgttggatg gcgcagtgat ggtttactgt     240

gctgttggtg gtgttcagcc acagtctgaa accgtatggc gtcaggctaa caaatacaaa 300

gttccacgta tcgcgttcgt taacaaaatg gaccgtatgg gtgcgaactt cctgaaagtt 360

gttgaccaga tcaaaacccg tctgggcgca actccagttc cactgcagct ggcgatcggc 420

gctgaagaag cgttcaccgg cgttgttgac ctggtgaaaa tgaaagccat caactggaac 480

gacgctgacg cgggcgtaac cttcgaatac gaagacatcc ctgctgatat ggtcgaactg 540

gctgacgaat ggcaccagaa cctgatcgaa tccgcagctg aagcttctga agagctgatg 600

gaaaaatacc tgggcggcga cgagctgacc gag 633

<210> 486
<211> 633
<212> DNA
<213> Serratia ureilytica

<400> 486
ggtgtaaacc acaaaatcgg tgaagttcat gacggcgctg ccaccatgga ctggatggaa 60

caggagcagg agcgtggtat taccatcact tctgcagcga cgactgcctt ctggtctggt 120

atggccaagc agttcgaacc gcatcgcgta aacatcatcg acaccccagg gcacgttgac 180

ttcaccatcg aagtagaacg ttccatgcgt gtgctggatg gcgcggtaat ggtttactgt 240

gctgttggtg gtgttcagcc acagtctgaa accgtatggc gtcaggctaa caaatacaaa 300

gtgccacgta tcgcgttcgt taacaaaatg gaccgtatgg gcgctaactt cctgaaagtt 360

gttggccaga tcaaaactcg tctgggcgct aaccctgttc ctctgcagct ggctatcggc 420

gcagaagaca aattcaccgg cgttatcgac ctggtgaaga tgaaggcgat caactggaac 480

gaagaagacg caggcgttac cttcgaatac gaagacgtcc cggctgacat gatggaactg 540

gctgaagaat ggcgccagaa cctgatcgaa tccgctgcgg aagcttccga agagctgatg 600

gaaaaatacc tgggcggcga agagctgacc gag 633

<210> 487
<211> 633
<212> DNA
<213> Shigella boydii

<400> 487
ggtgtaaacc ataaaatagg tgaagttcat gacggcgctg caaccatgga ctggatggag 60

caggagcagg aacgtggtat taccatcact tccgctgcga ctactgcatt ctggtctggt 120

atggctaagc agtatgagcc gcatcgcatc aacatcatcg acaccccggg gcacgttgac 180

ttcacaatcg aagtagaacg ttccatgcgt gttctcgatg gtgcggtaat ggtttactgc 240

gcagttggtg gtgttcagcc gcagtctgaa accgtatggc gtcaggcaaa caaatataaa 300

gttccgcgca ttgcgttcgt taacaaaatg gaccgcatgg gtgcgaactt cctgaaagtt 360

gttaaccaga tcaaaacccg tctgggcgcg aatccggttc cgctgcagct ggcgattggt 420

gctgaagaac atttcaccgg tgttgttgac ctggtgaaaa tgaaagctat taactggaac 480

gacgctgacc agggcgtaac cttcgaatac gaagatatcc cggcagacat ggttgaactg 540

gctaacgaat ggcaccagaa cctgatcgaa tccgcagctg aagcttctga agagctgatg 600

gaaaaatacc tgggtggtga agaactgact gaa 633

<210> 488
<211> 633
<212> DNA
<213> Shigella dysenteriae T

<400> 488
ggtgtaaacc ataaaatcgg tgaagttcat gacggcgctg caaccatgga ctggatggag 60

caggagcagg aacgtggtat taccatcact tccgctgcga ctactgcatt ctggtctggt 120

atggctaagc agtatgagcc gcatcgcatc aacatcatcg acaccccggg gcacgttgac 180

ttcacaatcg aagtagaacg ttccatgcgt gtgctcgatg gtgcggtaat ggtttactgc 240

gcagttggtg gtgttcagcc gcagtctgaa accgtatggc gtcaggcaaa caaatataaa 300

gttccgcgca ttgcgttcgt taacaaaatg gaccgtatgg gtgcgaactt cctgaaagtt 360

gttaaccaga tcaaaacccg tctgggcgcg aacccggttc cgctgcagct ggcgattggt 420

gctgaagaac atttcaccgg tgttgttgac ctggtgaaaa tgaaagctat caactggaac 480

gacgctgacc agggcgtaac cttcgaatac gaagatatcc cggcagacat ggttgaactg 540

gctaacgaat ggcaccagaa cctgatcgaa tccgcagctg aagcttctga agagctgatg 600

gaaaaatacc tgggtggtga agaactgact gaa 633

<210> 489
<211> 633
<212> DNA
<213> Shigella flexneri

<400> 489
ggtgtaaacc ataaaatcgg tgaagttcat gacggcgctg caaccatgga ctggatggag 60

caggagcagg aacgtggtat taccatcact tccgctgcga ctactgcatt ctggtctggt 120

atggctaagc agtatgagcc gcatcgcatc aacatcatcg acaccccggg gcacgttgac 180

ttcacaatcg aagtagaacg ttccatgcgt gttctcgatg gtgcggtaat ggtttactgc 240

gcagttggtg gtgttcagcc gcagtctgaa accgtatggc gtcaggcaaa caaatataaa 300

gttccgcgca ttgcgttcgt taacaaaatg gaccgcgtgg gtgcgaactt cctgaaagtt 360

gttaaccaga tcaaaacccg tctgggcgcg aacccggttc cgctgcagct ggcgattggt 420

gctgaagaac atttcaccgg tgttgttgac ctggtgaaaa tgaaagctat caactggaat 480

gacgctgacc agggcgtaac cttcgaatac gaagatatcc cggcagacat ggttgaactg 540

gctaacgaat ggcaccagaa cctgatcgaa tccgcagctg aagcttctga agagctgatg 600

gaaaaatacc tgggtggtga agaactgact gaa 633

<210> 490
<211> 633
<212> DNA
<213> Shigella sonnei

<400>  490
ggtgtaaacc ataaaatcgg tgaagttcat gacggcgctg caaccatgga ctggatggag      60

caggagcagg aacgtggtat taccatcact tccgctgcga ctactgcatt ctggtctggt     120

atggctaagc agtatgagcc gcatcgcatc aacatcatcg acaccccggg gcacgttgac     180

ttcacaatcg aagtagaacg ttccatgcgt gttctcgatg gtgcggtaat ggtttactgc     240

gcagttggtg gtgttcagcc gcagtctgaa accgtatggc gtcaggcaaa caaatataaa     300

gttccgcgca ttgcgttcgt taacaaaatg gaccgcatgg gtgcgaactt cctgaaagtt     360

gttaaccaga tcaaaacccg tctgggcgcg aacccggttc cgctgcagct ggcgattggt     420

gctgaagaac atttcaccgg tgttgttgac ctggtgaaaa tgaaagctat caactggaac     480

gacgctgacc agggcgtaac cttcgaatac gaagatatcc cggcagacat ggttgaactg     540

gctaacgaat ggcaccagaa cctgatcgaa tccgcagctg aagcttctga agagctgatg     600

gaaaaatacc tgggtggtga agaactgact gaa                                  633

<210>  491
<211>  633
<212>  DNA
<213>  Tatumella ptyseos T

<400>  491
ggtgtaaacc ataaaatcgg tgaagtacat gatggtgctg caaccatgga ctggatggct      60

caggagcagg aacgtggtat tactatcacc tccgctgcta ccaccgcttt ctggtctggt     120

atggcaaaac aatttgaacc acaccgcgtc aatatcattg ataccccggg acacgttgac     180

tttactatcg aagtagagcg ttcaatgcgt gttctggatg gtgcagtaat ggtttactgc     240

gcggtcggcg gcgttcagcc tcagtctgag actgtatggc gtcaggcgaa caaatataaa     300

gttccacgta tcgcgttcgt taacaaaatg gaccgtatgg gtgcgaactt cctgaaagtt     360

gttggccaga tcaaaagccg tctgggtgct aaccctgttc cattgcagct ggcaatcggt     420

gcagaagaag gcttcaccgg tgttgttgac ctggtgaaaa tgaaagccat caactggaac     480

gaagcagacg cgggtgtgac cttcgaatac gaagaaatcc ctgctgatct ggtcgaactg     540

gctgacgagt ggcaccagaa cctggttgaa gccgccgcag aagcttctga agagctgatg     600

gagaaatacc tcggcggtga agaactgact gaa                                  633

<210>  492
<211>  633
<212>  DNA
<213>  Trabulsiella guamensis T

<400>  492
ggtgtaaacc acaaaatcgg tgaagttcat gacggcgccg caaccatgga ctggatggag      60

caggagcagg aacgtggtat caccatcact tccgctgcga ctactgcatt ctggtcaggt     120

atggctaagc agtatgaacc gcaccgtgtc aacatcatcg acaccccggg gcacgttgac     180

ttcactatcg aagtagaacg ttccatgcgt gtactcgatg gtgcggtaat ggtttactgc     240

gcagttggtg gtgttcagcc gcagtctgaa accgtatggc gtcaggcgaa caaatataaa     300

gttccacgca ttgcgttcgt taacaaaatg gaccgcatgg gtgcgaactt cctgaaagtt 360

gttggtcaga tcaaaacccg tctcggcgcg aacccggttc cgctgcagct ggcaattggt 420

gctgaagaag ctttcaccgg cgttatcgac ctggtgaaaa tgaaagccat caactggaac 480

gatgcagacc agggcgttac cttcgaatac gaagatatcc cggctaacat gcaggaactg 540

gctgacgaat ggcaccagaa cctgatcgag tccgcagctg aagcttctga agagctgatg 600

gaaaaatacc tgggcggtga agaactgact gaa 633

<210> 493
<211> 633
<212> DNA
<213> Xenorhabdus beddingii

<400> 493
ggtgtaagcc ataagatcgg tgaagttcat gatggcgctg ccactatgga ctggatggag 60

caggagcaag aacgtggtat taccatcacg tctgctgcga cgactgcatt ctggtctggt 120

atggctaaac agtatgaacc acaccgcgtc aacatcatcg acactccagg ccacgttgac 180

ttcactatcg aagtagaacg ttccatgcgt gttcttgatg gtgccgtaat ggtttactgt 240

gcagttggtg gtgttcagcc tcagtctgag actgtatggc gtcaggctaa caaatacaac 300

gtaccacgta tcgcgttcgt taacaaaatg gaccgtatgg gtgctaactt cctgcgtgtg 360

gttgagcaga tcaaaactcg tctggctgct aaccctgttc ctttgcaatt ggcaatcggt 420

gcagaagata ctttcaccgg tgttgttgat ttgctgaaaa tgaaagcaat caactggaac 480

gacgaagatc agggtattac cttcgaatac gaagatatcc cggctgacat gcttgagcag 540

gctcaggaat ggcatcagaa tctggttgaa tccgcagcag aagcatcaga agacctgatg 600

gacaaatatc tgggcggtga agagctgact gaa 633

<210> 494
<211> 633
<212> DNA
<213> Xenorhabdus budapestensis

<400> 494
ggtgtcagtc ataagatcgg tgaagttcat gatggcgctg ctactatgga ctggatggaa 60

caggagcaag aacgtggtat taccatcacg tctgctgcga ctaccgcttt ctggtctggt 120

atggctaaac agtatgagcc acaccgtgtc aacatcatcg acaccccagg ccacgttgac 180

ttcactatcg aagtagaacg ttctatgcgt gttcttgacg gtgcagtaat ggtttactgt 240

gcagttggtg gtgttcagcc acagtctgaa actgtatggc gtcaggcgaa caaatataac 300

gttccacgta tcgcgttcgt taacaaaatg gaccgtatgg gagcgaactt cctgcgcgtt 360

gttgagcaga tcaaaactcg tctggctgct aaccctgttc ctctgcaatt ggcaatcggt 420

gcagaagata ccttcaccgg tgttgttgat ttgttgaaaa tgaaagcaat caactggaac 480

gacgaagatc agggtattac cttcgaatac gaagatatcc cagctgacat gcttgagctg 540

gctcaggaat ggcatcagaa cctgatcgaa tccgcagcag aagcatcaga agaactgatg 600

gacaaatatc tgggcggtga agagctgact gaa 633

<210> 495
<211> 633
<212> DNA
<213> Xenorhabdus ehlersii

<400> 495
ggtgtaagcc ataagatcgg tgaagttcat gatggcgctg ctactatgga ctggatggag 60

caggagcaag aacgtggtat taccatcacg tctgctgcga ctaccgcatt ctggtctggt 120

atggctaaac agtatgagcc acaccgtatc aacatcatcg acaccccagg ccacgttgac 180

ttcactatcg aagtagaacg ttctatgcgt gttcttgacg gtgcagtaat ggtttactgt 240

gcagttggtg gtgttcagcc acagtctgaa actgtatggc gtcaggcgaa caaatataat 300

gttccacgta tcgcgttcgt taacaaaatg gaccgtatgg gagcgaactt cctgcgcgtt 360

gttgagcaga tcaaaactcg tctggctgct aaccctgttc ctctgcaatt ggcaattggt 420

gcagaagata ctttcaccgg tattgttgat ttgctgaaaa tgaaagcgat caactggaac 480

gacgaagatc agggtattac cttcgaatac gaagatatcc cggctgacat gcttgagcag 540

gctcaggaat ggcatcagaa cctgattgaa tccgccgcag aagcatcaga agagctgatg 600

gataaatacc tgggcggtga agagctgact gaa 633

<210> 496
<211> 633
<212> DNA
<213> Xenorhabdus innexi

<400> 496
ggtgtaaacc ataaaatcgg tgaaactcac gaaggttcag caactatgga ctggatggag 60

caggagcagg agcgtggtat taccattacc tccgcagcga ctactgcatt ctggtctggt 120

atggctaaac agtatgagcc acaccgcatc aacatcatcg acaccccagg ccacgttgac 180

ttcactatcg aagtagagcg ttctatgcgt gttcttgatg gtgcagtaat ggtttactgt 240

gcagttggtg gtgttcagcc tcagtctgaa accgtatggc gtcaggctaa caaatataat 300

gttccacgta tcgcgttcgt taacaaaatg gaccgtatgg gtgcgaactt cctgcgcgtt 360

gttgatcaga tcaaaactcg tctggcagct aacccagttc ctctgcaatt ggcaatcggt 420

gcagaagata ccttcaccgg tgttgttgac ctgctgaaaa tgaaagcaat caactggaac 480

gaagctgatc agggaactac cttcacttat gaagatatcc cagctgacat gcttgaactg 540

gcgcaggaat ggcatcagaa cctgattgaa tccgctgcag aagcatcaga agaactgatg 600

gacaaatatc tgagcggtga agagctgact gaa 633

<210> 497
<211> 633
<212> DNA
<213> Xenorhabdus japonica

<400> 497

ggtgtaaacc ataaaattgg tgaaactcac gaaggttcag caacaatgga ctggatggag 60

caggaacagg aacgtggtat tactatcacc tctgctgcga ccactgcatt ctggtctggt 120

atggctaaac agtatgagcc acaccgcatc aacatcatcg acaccccagg acacgttgac 180

ttcaccatcg aagtagaacg ttccatgcgt gttcttgacg gcgcagtaat ggtttactgt 240

gcagttggtg gtgttcagcc acagtctgag actgtatggc gtcaggctaa caaatataat 300

gttccacgca tcgcgttcgt taacaaaatg gaccgtatgg gtgcgaactt cctgcgtgtt 360

gttgaccaga tcaaaactcg tctggcagct aacccagtac cacttcaact ggctatcggc 420

gctgaagata ccttcactgg tattgttgac ctgttgaaaa tgaaagcaat caactggaac 480

gaagctgatc agggaacgac cttcacttat gaagatatcc ctgctgacat gctggaactg 540

gccgaagaat ggcatcagat tctggttgaa tccgctgcag aagcatcaga agacctgatg 600

gacaaatatc tgggcggtga agagctgact gaa 633

<210> 498
<211> 633
<212> DNA
<213> Xenorhabdus poinarii

<400> 498
ggtgtaaacc ataagattgg tgaaactcac gaaggttcag caacaatgga ctggatggag 60

caggagcagg agcgtggtat tactatcacg tccgcagcga cgactgcatt ctggtctggt 120

atggctaaac agtatgagcc acaccgtatc aacattatcg acaccccggg ccacgttgac 180

ttcactatcg aagtagaacg ttccatgcgt gttcttgacg gtgcagtaat ggtttactgt 240

gcagtaggtg gtgttcagcc tcagtctgag accgtatggc gccaggctaa caaatacaat 300

gttccacgta tcgcgttcgt taacaaaatg gaccgtatgg gggctaactt cctgcgcgtg 360

gttgagcaga tcaaaactcg tctggctgct aacccagttc ctctgcaatt ggcaatcggt 420

gcggaagact ccttcaccgg tgttgttgac ttgctgaaaa tgaaagcaat caactggaac 480

gacgcagatc agggaacaac cttcacttat gaagagatcc cagctgacat ggttgagctg 540

gcgcaggaat ggcatcagaa cctgatcgaa tccgcggcag aagcatcaga agagctgatg 600

gataaatatc tgggcggtga agagctgact gaa 633

<210> 499
<211> 633
<212> DNA
<213> Yersinia aldovae

<400> 499
ggtgtaaacc ataagatcgg tgaagttcat aacggcgcag ccactatgga ctggatggaa 60

caggagcagg agcgtggtat taccattact tctgcggcta ctacctgctt ctggtctggt 120

atggctaaac agtacgaacc acatcacgtc aatatcattg acacccctgg acacgttgac 180

ttcaccattg aagtagaacg ttccatgcgt gttcttgacg gcgcggtaat ggtttactgt 240

gcagttggtg gtgttcagcc acagtctgag accgtatggc gtcaggcgaa taaatataaa 300

gttccacgca ttgcgttcgt taacaaaatg gaccgcatgg gtgctaactt cctgaaagtt 360

gttggtcaga ttaagtctcg tctgggcgcg aatccagttc cattgcaact ggcaatcggc 420

gcggaagaga aattcaccgg tgttatcgat ctggtgaaaa tgaaagcgat caactggaat 480

gaagcggatc tgggtgtgac cttcgaatac gaagagatcc ctgcggatat ggctgagctg 540

gctgctgaat ggcaccagaa tctggttgag tctgctgctg aagcatcaga tgagctgatg 600

gataagtatt tgggcggcga agagctgact gaa 633

<210> 500
<211> 633
<212> DNA
<213> Yersinia bercovieri

<400> 500
ggtgtaaacc ataaaatcgg tgaagttcat gacggcgcag ccaccatgga ctggatggaa 60

caggagcagg agcgtggtat taccattact tctgcggcta ctacctgctt ctggtctggt 120

atggctaaac agttcgaacc acatcacgtc aatatcattg acacccctgg acacgttgac 180

ttcaccatcg aagtagaacg ttctatgcgt gttcttgacg gcgcggtaat ggtttactgt 240

gcagttggtg gtgttcagcc acagtctgag accgtatggc gtcaggcgaa taaatataaa 300

gttccgcgta tcgcgttcgt taacaaaatg gaccgtatgg gcgcgaattt cctgaaagtt 360

gttggtcaga ttaaatctcg tctgggcgcg aatccagttc cattgcaact ggctatcggc 420

gcggaagaga aattcaccgg tattattgac ctggtgaaaa tgaaagcgat caactggaac 480

gaagccgatc agggcgtgac cttcgaatac gaagagatcc ctgctgatat ggttgagctg 540

gctgctgaat ggcaccagaa cctggttgaa tctgctgcag aagcgtcaga tgagctgatg 600

gataagtact tgggtggcga tgagctgacc gaa 633

<210> 501
<211> 633
<212> DNA
<213> Yersinia enterocolitica sbsp. enterocolitica

<400> 501
ggtgtaaacc ataagatcgg tgaagttcat aacggcgcag ccaccatgga ctggatggaa 60

caagagcagg aacgtggtat taccattact tctgcggcta ctacctgctt ctggtctggt 120

atggctaaac agtatgaacc acatcacatc aatatcattg acacccctgg gcacgttgac 180

ttcaccattg aagttgaacg ttctatgcgt gttcttgacg gtgcggtaat ggtttattgt 240

gcagttggtg gtgttcagcc acagtctgaa accgtatggc gtcaggcgaa caaatataaa 300

gttccacgca ttgcgttcgt taataaaatg gaccgcatgg gtgctaactt cctgaaagtt 360

gttggtcaga ttaaatctcg tctgggcgcg aatccagttc cattgcaact ggcaatcggc 420

gcggaagaga aattcactgg tgttatcgat ctagtgaaaa tgaaagcgat caactggaat 480

gaagccgatc tgggtgtgac cttcgaatac gaagagatcc ctgctgatat ggctgagctg 540

gctgctgaat ggcaccagaa tctggttgaa tcggctgcag aagcgtctga agagttgatg 600

gacaagtatt tgggcggcga agagctgacc gaa 633

<210> 502
<211> 633
<212> DNA
<213> Yersinia enterocolitica sbsp. palearctica

<400> 502
ggtgtaaacc ataaaatcgg tgaagttcat gacggcgcag ccaccatgga ctggatggaa 60

caggagcagg agcgtggtat taccattact tctgcggcta ctacctgctt ctggtctggt 120

atggctaaac agttcgaacc acatcacgtc aatatcattg acacccctgg gcacgttgac 180

ttcaccatcg aagtagaacg ttccatgcgt gttcttgacg gcgcggtaat ggtttactgt 240

gcagttggtg gtgttcagcc acagtctgaa accgtatggc gtcaggctaa caaatataaa 300

gttccacgca ttgcgttcgt taacaaaatg gaccgcatgg gtgctaactt cctgaaagtt 360

gttggtcaga ttaaatctcg tctgggcgcg aatccagttc cattgcaact ggctatcggc 420

gcggaagaga aatttaccgg tattatcgac ctggtaaaaa tgaaagcgat caactggaac 480

gaagctgatc agggcgtgac cttcgaatac gaagagatcc cggctgatat ggctgaactg 540

gctgctgaat ggcaccagaa tctggttgaa tctgctgcag aagcgtcaga agagctgatg 600

gacaaatatt tgggcggcga agagctgacc gaa 633

<210> 503
<211> 633
<212> DNA
<213> Yersinia frederiksenii

<400> 503
ggtgtaaacc ataagatcgg tgaagttcat gacggcgcag ccaccatgga ctggatggaa 60

caggagcagg agcgtggtat taccattact tctgcagcta ctacctgctt ctggtctggt 120

atggctaaac agttcgaacc acatcacgtc aatatcattg acacccctgg gcacgttgac 180

ttcaccatcg aagtagaacg ttccatgcgt gttcttgacg gcgcggtaat ggtttactgt 240

gcagttggtg gtgttcagcc acagtctgaa accgtatggc gtcaggctaa caagtataaa 300

gttccacgta tcgcgttcgt taacaaaatg gaccgtatgg gtgcgaattt cctgaaagtt 360

gttggtcaga ttaaatctcg tctgggcgcg aatccagttc cattgcaact ggctatcggc 420

gcggaagaga aatttaccgg tattatcgac ctggtaaaaa tgaaagcgat caactggaac 480

gaagctgatc agggcgtgac cttcgaatac gaagagatcc cggctgatat ggctgagctg 540

gctgctgaat ggcaccagaa tctggttgaa tctgctgctg aagcgtcaga agagctgatg 600

gacaaatatt tgggcggcga agagctgacc gaa 633

<210> 504
<211> 633
<212> DNA
<213> Yersinia intermedia

<400> 504
ggtgtaaacc ataagatcgg tgaagttcat gacggcgcag ccaccatgga ctggatggaa 60

caggagcagg agcgtggtat taccattact tctgcggcta ctacctgctt ctggtctggt 120

atggctaaac agtacgaacc acatcacgtc aatatcattg acacccctgg gcacgttgac 180

ttcaccatcg aagtagaacg ttccatgcgt gttcttgacg gcgcggtaat ggtttactgt 240

gcagttggtg gtgttcagcc acagtctgag accgtatggc gtcaggctaa taaatataaa 300

gttccacgca ttgcgttcgt taacaaaatg gaccgcatgg gtgctaactt cctgaaagtt 360

gttggtcaga ttaaatctcg tctgggcgcg acaccagttc cattgcaact ggcaatcggc 420

gcggaagaga aattcaccgg tattatcgac ctggtgaaaa tgaaagcgat caactggaac 480

gaagctgatc agggcgtgac cttcgaatac gaagagatcc ctgctgatat ggttgagctg 540

gctgctgaat ggcaccagaa tctggttgag tctgctgcag aagcgtctga agagctgatg 600

gacaaatact tgggcggcga agagctgacc gaa 633

<210> 505
<211> 633
<212> DNA
<213> Yersinia kristensenii

<400> 505
ggtgtaaacc ataagatcgg tgaagttcat gacggcgcag ccaccatgga ctggatggaa 60

caggagcagg agcgtggtat taccattact tctgcggcta ctacctgctt ctggtctggt 120

atggctaaac agtacgaacc acatcacgtc aacatcattg acaccccagg gcacgttgac 180

ttcactatcg aagtagaacg ttccatgcgt gttcttgatg gtgcggtaat ggtttactgt 240

gcagttggtg gtgttcagcc acagtctgag accgtatggc gccaggctaa caaatataaa 300

gttccacgca ttgcgttcgt taacaaaatg gaccgcatgg gtgctaactt cctgaaagtt 360

gttggtcaga ttaaatctcg tctgggcgcg aatccagttc cattgcaact ggcaatcggc 420

gcggaagaga aattcaccgg tattatcgat ctggtgaaaa tgaaagcgat caactggaac 480

gaagctgatc agggcgtgac cttcgaatac gaagagatcc cggctgatat ggctgaactg 540

gctgctgaat ggcaccagaa cctggttgaa tctgctgcag aagcgtcaga agagctgatg 600

gataaatatt tgggcggcga agagctgact gaa 633

<210> 506
<211> 633
<212> DNA
<213> Yersinia mollaretii

<400> 506
ggtgtaaacc ataaaatcgg tgaagttcat gacggcgcag ccaccatgga ctggatggaa 60

caggagcagg agcgtggtat taccattact tctgcggcta ctacctgctt ctggtctggt 120

atggctaaac agttcgaacc acatcacgtc aatatcattg acacccctgg gcacgttgac 180

ttcaccatcg aagttgaacg ttccatgcgt gttcttgacg gcgcggtaat ggtttactgt 240

gcagttggtg gtgttcagcc acagtctgag accgtatggc gtcaggcaaa taaatataaa 300

gttccgcgta tcgcgttcgt taacaaaatg gaccgtatgg gcgcgaattt cctgaaagtt 360

gttggtcaga ttaaatctcg tctgggcgcg aatccagttc cattgcaact ggctatcggc 420

gcggaagaga aattcaccgg tattattgac ttggtgaaaa tgaaagcgat caactggaac 480

gaagccgatc agggcgtgac cttcgaatac gaagagatcc cagctgatat ggctgaactg 540

gctgctgaat ggcaccagaa tctggttgag tctgctgcag aagcgtctga tgagttgatg 600

gataagtact tgggcggcga tgagctgacc gaa 633

<210> 507
<211> 633
<212> DNA
<213> Yersinia pestis T

<400> 507
ggtgtaaatc acaaaatcgg tgaagttcat gacggcgcag ccaccatgga ctggatggaa 60

caggagcagg agcgtggtat taccattact tctgcagcta ctacctgctt ctggtctggt 120

atggctaaac agttcgaacc acatcacgtc aatatcattg acacccctgg gcacgttgac 180

ttcactatcg aagtagagcg ttccatgcgt gttcttgacg gcgcggtaat ggtttactgt 240

gcagttggtg gtgttcagcc acagtctgaa accgtatggc gtcaggctaa taaatataaa 300

gttccacgta ttgcgttcgt taacaaaatg gaccgtatgg gtgcgaactt cctgcgcgta 360

gttggtcaac tgaaatctcg ccttggtgcg aacccagttc cactgcagtt ggcaattggc 420

gcagaagaaa aattcaccgg tattatcgat ctggtgaaaa tgaaagcgat caactggaac 480

gaagctgatc agggcgtgac cttcgaatat gaagaaatcc ctgctgatat ggctgaactg 540

gctgctgaat ggcaccagaa tctggttgaa tctgcggcag aagcgtctga cgagctgatg 600

gacaaatact tgggtggcga agagctgacc gaa 633

<210> 508
<211> 633
<212> DNA
<213> Yersinia pseudotuberculosis sbsp. pseudotuberculosis

<400> 508
ggtgtaaatc acaaaatcgg tgaagttcat gacggcgcag ccaccatgga ctggatggaa 60

caggagcagg agcgtggtat taccattact tctgcagcta ctacctgctt ctggtctggt 120

atggctaaac agttcgaacc acatcacgtc aatatcattg acacccctgg gcacgttgac 180

ttcactatcg aagtagagcg ttccatgcgt gttcttgacg gcgcggtaat ggtttactgt 240

gcagttggtg gtgttcagcc acagtctgaa accgtatggc gtcaggctaa taaatataaa 300

gttccacgta ttgcgttcgt taacaaaatg gaccgtatgg gtgcgaactt cctgcgcgta 360

gttggtcaac tgaaatctcg ccttggtgcg aacccagttc cactgcagtt ggcaattggc 420

gcagaagaaa aattcaccgg tattatcgat ctggtgaaaa tgaaagcgat caactggaac 480

gaagctgatc agggcgtgac cttcgaatat gaagaaatcc ctgctgatat ggctgaactg 540

gctgctgaat ggcaccagaa tctggttgaa tctgcggcag aagcgtctga cgagctgatg 600

gacaaatact tgggtggcga agagctgacc gaa 633

<210> 509
<211> 633
<212> DNA
<213> Yersinia rohdei

<400> 509
ggtgtaaacc ataaaatcgg tgaagttcat gacggcgcag ccaccatgga ctggatggaa 60

caggagcagg agcgtggtat taccattact tctgcggcta ctacctgctt ctggtctggt 120

atggctaaac agtatgaacc acatcacgtc aatatcattg acacccctgg gcacgttgac 180

ttcaccatcg aagtagaacg ttccatgcgt gttcttgacg gcgcggtaat ggtttactgt 240

gcagttggtg gtgttcagcc acagtctgaa accgtatggc gtcaggcgaa caaatataaa 300

gttccacgca ttgcgttcgt taacaaaatg gaccgcatgg gtgctaactt cctgaaagtt 360

gttggtcaga ttaaatctcg tctgggcgcg aatccagttc cattgcaatt ggcaatcggc 420

gcggaagaga aattcaccgg tattattgac ctggtgaaaa tgaaagcgat caactggaac 480

gaagctgatc agggcgtgac cttcgaatat gaagagatcc cagctgatat ggttgagctg 540

gctgctgaat ggcaccagaa tctggttgaa tctgctgcag aagcatcaga tgagctgatg 600

gacaaatact tgggcggcga agagctgacc gag 633

<210> 510
<211> 633
<212> DNA
<213> Yersinia ruckeri

<400> 510
ggtgtaaacc ataaaatcgg tgaagttcat gacggcgctg ccaccatgga ctggatggaa 60

caggagcagg aacgtggtat taccatcact tctgctgcga ccacggcctt ctggtctggt 120

atggctaagc agtttgagcc acaccgcgtc aacatcattg acaccccagg acacgttgac 180

ttcaccattg aagtagaacg ttccatgcgt gttcttgatg gtgctgtaat ggtttactgt 240

gctgttggtg gtgttcagcc gcagtctgaa accgtatggc gtcaggctaa caaatataaa 300

gttccacgca ttgcgttcgt taacaaaatg gaccgcatgg gtgctaactt cctgaaagtt 360

gttggtcaga ttaaatctcg tctgggcgcg aatccagttc cattgcaact ggcaatcggc 420

gcggaagaga aattcaccgg tattattgac ctggtgaaaa tgaaagcgat caactggaat 480

gaagctgatc agggcgtgac cttcgaatat gaagagatcc ctgcagacat ggttgaactg 540

gctgctgaat ggcaccagaa cctggttgag tctgctgctg aagcttcaga tgagctgatg 600

gacaaatacc tgggcggcga agagctgacc gag 633

<210> 511
<211> 633
<212> DNA
<213> Yokenella regensburgei T

<400> 511
ggtgtaaacc acaaaatcgg tgaagttcat gacggcgcgg ctaccatgga ctggatggag 60

caggagcagg aacgtggtat caccatcact tccgctgcga ctactgcatt ctggtctggt 120

atggctaagc agtatgaacc gcatcgcgta aacatcatcg acaccccggg ccacgttgac 180

ttcactatcg aagtagaacg ttccatgcgt gttcttgatg gtgcggtaat ggtttactgc 240

gcagttggtg gtgttcagcc gcagtctgaa accgtatggc gtcaggcaaa caaatataaa 300

gttccacgca ttgcgttcgt taacaaaatg gaccgcatgg gcgcgaactt cctgaaagtt 360

gttggtcaga tcaaatcccg tctgggcgcg aacccggttc cgctgcagct ggcgattggt 420

gctgaagaag gtttcaccgg tgttgttgac ctggtgaaaa tgaaagccat caactggaac 480

gatgcagacc agggcgttac cttcgaatac gaagatatcc cggctgacat gcaggaactg 540

gctgaagaat ggcaccagaa cctgatcgaa tccgcagctg aagcttctga agagctgatg 600

gagaaatacc tgggtggtga agaactgact gaa 633

<210> 512
<211> 642
<212> DNA
<213> Brenneria alni

<400> 512
cagtggttta tcaaaatcac cgattacgcc gatcaactgc tcaacgatct ggatacgctg 60

gaaagctggc ccgaacaggt caaaaccatg cagcgtaact ggattggccg ttctgaaggc 120

gttgaaatca cctttgaggt ggcagacagt caggagaaac tgaacgttta taccacccgt 180

cccgatactt tcatgggcgt aacctacgtg gccgtcgccg ccggtcaccc gttggcgtta 240

caggccgcgg ccagtaattc ggcactggct gactttattg atgaatgccg taataccaaa 300

gtggccgaag ccgacatggc cactatggag aagaaaggca tggctaccgg cctgtatgcc 360

attcatccgc tgaacggtga aaaagtcgct atctgggccg ctaactttgt gctgatggac 420

tacggcacag gcgccgtcat ggctgtaccg gggcacgatc agcgtgactg ggaattcgcc 480

gccaaatacg gcctgactat caaaccggtt atcctgaaca gcgatggcag cgagccggat 540

ctatccgctc aggcgatgac ggaaaaaggc cggttgttta actccggcga gtttgatggt 600

ctggactttg aatccgcctt taacgccatt gccgacaaac tg 642

<210> 513
<211> 642
<212> DNA
<213> Brenneria quercina

<400> 513
cagtggttca tcaaaatcac ggactacgcg gaccaactgc tgaacgatct ggatacgctg 60

gaaagctggc ccgagcaggt caaaaccatg cagcgcaact ggatcggccg ctccgaaggc 120

gtcgaaatca ccttcgacat cgcggacagc gatcgccaga tgacggttta cacgacgcgc 180

cccgatacct tcatgggcgt gacctatgtc gccgtcgctg cagggcatcc tctgtctctg 240

ctggccgcag aaaaaaatcc tgcgctgaat gacttcatcg aagagtgccg caataccaaa 300

gtggccgaag ccgacatggc gacgatggag aaaaaaggca tgccgaccgg cctgtacgct 360

atccacccgc tgaacggcga aaaagtaccg gtatggatcg caaactttgt gctgatggaa 420

tacggcaccg gcgcggtcat ggccgtaccg gctcacgacc aacgcgactg ggagttcgcc 480

accaaatacg ggctgccgat caaacaggtt attcaggccg cggaaggcca agacgtcgac 540

ctgtccgctc aggcgctgac ggagaaagga cgcctgttca actccggcga attcgacggt 600

cttgattacg aagaagcctt caacgccatc gccgacacgc tg 642

<210> 514
<211> 642
<212> DNA
<213> Brenneria rubrifaciens

<400> 514
caatggttca tcaaaatcac cgactatgcc gaccaattgc tgaatgatct ggatacgctg 60

gaaagttggc ctgagcaggt caaaaccatg cagcgtaact ggattggacg ctccgaaggc 120

gtggaaatca cctttgccat cgcggatagt caggagaaat taagcgtcta tacgacgcgt 180

cccgatacat tcatgggcgt gacgtacgtt gccgttgccg ccagtcatcc tctggcgcag 240

caagccgctg caaacaatcc tgagctggct gatttcattg ccgaatgccg caacaccaaa 300

gtggcggaag cggacatggc gacgatggag aagaaaggca tggccactgg cctgtatgcc 360

atccatccgc tcaacggaga aaaagtcgcg atctgggccg caaattttgt tctgatggat 420

tacggtacag gcgcggtgat ggccgtgccg ggtcacgacc agcgcgactg ggaattcgcc 480

aacaaatatg acctgtcaat aaaaccggtc attctgaaca gcgacggtag cgaaccggat 540

ctgtcctcgc aagcgatgac ggaaaaaggc cgtttgttca actcagggga atttgacggt 600

ctggattttg aggctgcctt taacgccgtt gccgacaagc tg 642

<210> 515
<211> 642
<212> DNA
<213> Brenneria salicis T

<400> 515
caatggttta tcaaaatcac cgactatgcc gatcaactgc tgaacgatct ggatacgctg 60

gaaagctggc ctgagcaggt caaaaccatg cagcgtaact ggattggccg ttctgaaggg 120

gtggaaatca cctttgacat cgcggacagc gaagagaagc tcagtgttta taccacgcgt 180

cccgacactt tcatgggcgt gacctatgtc gccgttgccg ccggtcaccc gttggcgctg 240

caagcggcgg ccaccaatcc ggcgctggct gacttcattg ccgaatgccg caacaccaaa 300

gtcgccgaag cggatatggc gacgatggag aaaaaaggca tggccaccgg cctgtacgcc 360

atccatccgc ttagcggaga gaaagtcgcg atctgggcgg caaactttgt actgatggat 420

tacggcaccg gcgcggtgat ggcggttccg ggccatgacc agcgcgattg ggaattcgcc 480

aacaaatata gtctgccgat taaaccggtt attctgaaca gcgatggcag cgaaccggat 540

ctgtccaccc aggcgatgac cgagaaaggt cgtttgttca actctggcga atttgacggt 600

ctgaattttg acgccgcctt taacgcaatc gccgacaagc tg 642

<210> 516
<211> 642
<212> DNA
<213> Budvicia aquatica T

<400> 516
cagtggttta tcaaaatcac cgcctatgcc gatcaattgc tcaacgattt agatacgctg 60

gaaagctggc ctgagcaggt taagaccatg cagcgtaact ggattggccg ttctgaaggc 120

gtcgaaatta agttttctat cgacggttct gacgactcat tgtcagtcta taccactcgc 180

ccggatacct ttatgggggt gacctatgtg gctattgccg cgggtcatcc gttggctgca 240

caggcggcgg taaacaatcc tgtattaagt gattttatcg acgaatgccg taataccaag 300

gttgccgaag ccgaaatggc caccatggag aagaaaggcg ttgccaccgg tatgtacgtg 360

attcacccac taagcggcga aaaagtcgct atctgggtcg ctaactttgt gctgatggaa 420

tacggcacgg gtgcggttat ggccgttccg ggtcatgacc aacgtgactg ggaattcgcc 480

actaaatata atctgccaat taagccggtt attcttgctg ctgatggctc acaacctgag 540

attcatcagc aggcgatgac tgaaaaaggc gtgctgttta actctggcga gtttgacggt 600

cttgattatg aagccggttt caatgctatc gctgacaagc ta 642

<210> 517
<211> 642
<212> DNA
<213> Buttiauxella agrestis T

<400> 517
cagtggttta tcaaaatcac tgcctacgca gacgaactgt taaacgatct ggatacactt 60

gaccattggc ctgatcaggt caaaaccatg caacgtaact ggattggccg ttctgaaggc 120

gttgaaatta ctttcgacgt tgaaaacagt gctgaaaaac tgactgttta caccacgcgc 180

ccggacacct tcatgggtgt gacttacctg gctgtggctg cgggtcatcc catcgcgcaa 240

caagcggcgt tgaacaaccc tgaactggct gaattcgttg atgaatgccg taacaccaag 300

gttgccgaag ctgaaatggc aaccatggag aagaaaggcg tggcgactgg cgttaacgct 360

attcacccat tgactggcga agctgttcca gtttgggtcg ctaacttcgt cttgatggaa 420

tacggcacag gcgcggtaat ggcagttcca ggccacgacc agcgcgactg ggaatttgca 480

accaaatacg ggctgcaaat caaaccggtt attctgaatg ctgacggcag cgagccggac 540

ctgaccgctg ctgcaatgac cgaaaaaggc gtgctgttca actctggcga gtttgacggt 600

ctggacttcc agaacggttt taacgcaatt gccgataaac tg 642

<210> 518
<211> 642
<212> DNA
<213> Buttiauxella brennerae

<400> 518
cagtggttta ttaaaatcac cgcttacgca gacgagctgt taaacgatct ggatacgctg 60

gaccattggc ctgagcaggt caaaaccatg cagcgtaact ggattggccg ttccgaaggt 120

gttgagatca cattcgacgt tgaaaacagc gctgagaaac tgactgttta caccacgcgc 180

ccggatacct ttatgggcgt gacctatctg gccgttgccg caggtcaccc tatcgcgcag 240

caagccgcat tgaacaaccc ggcgctggct gaattcgttg aagagtgccg taataccaaa 300

gttgccgaag ctgaaatggc aaccatggag aagaaaggcg ttgctactgg cattaacgct 360

attcacccac tgactggcga gtctgttcca gtttgggtgg caaacttcgt cctgatggaa 420

tacggcacgg gcgcggtaat ggctgttcct ggtcacgacc agcgcgactg ggaatttgcc 480

accaaatacg gcctgcaaat caaacccgta attctgaatg ccgatggcag cgagccggac 540

ctggcggctg ccgcaatgac cgaaaaaggc gtgctgttta attcaggcga gttcgacggt 600

ctggacttcc agaatggatt taacgccatt gccgataaac tg 642

<210> 519
<211> 642
<212> DNA
<213> Buttiauxella ferragutiae

<400> 519
cagtggttta tcaaaatcac cgcttacgca gatgagttgt taaacgatct ggatacgctg 60

gaccattggc ctgatcaggt caaaaccatg cagcgtaact ggattggccg ttctgaaggc 120

gttgagatta ctttcgacgt tgaaaacagc gctgagaaac tgacggttta caccacccgc 180

ccggacacct ttatgggtgt gacttacctg gcagttgctg ctggccaccc tatcgcacaa 240

caagcggcag tgaacaaccc tgaactggct gcattcgtcg atgaatgccg taacaccaaa 300

gttgccgaag ctgaaatggc aaccatggaa aagaaaggcg ttgccactgg cgtgaacgcc 360

attcacccac tgactggcga agctgttcca gtttgggtag caaactttgt cctgatggaa 420

tacggcaccg gtgcggtgat ggcggttcct ggccacgacc agcgcgactg ggaatttgcg 480

acgaaatacg gtttgcaaat caaacctgtg attctcaatg ccgatggtag cgaacctgat 540

ttatcagccg ccgcgatgac cgaaaaaggc gcactgttca attcaggcga gttcgacggc 600

ctggacttcc agaatgcgtt caacgccatt gccgacaaac tg 642

<210> 520
<211> 642
<212> DNA
<213> Buttiauxella gaviniae

<400> 520
cagtggttta tcaaaatcac cgcttatgca gacgagttat taaacgacct cgatacgctg 60

gaccattggc ctgagcaggt caaaaccatg cagcgtaact ggattggccg ttccgaaggt 120

gttgagatca ctttcgatgt ggaaaacagc gctgagaaac tgactgttta caccacgcgt 180

ccggacacct ttatgggcgt gacctatctg gccgttgccg caggtcaccc aatcgcgcag 240

caagccgcac tgaacaaccc agcgctggct gagttcgttg aagagtgccg taacaccaaa 300

gttgccgaag cagaaatggc aacgatggag aagaaaggtg ttgcgactgg cgttaacgct 360

attcacccac tgaccggtga gtctgttcca gtttgggtag caaacttcgt cctgatggaa 420

tacggcaccg gtgctgtaat ggccgttcct ggtcatgacc agcgcgactg ggagtttgcg 480

accaaatacg gtctgcaaat caaaccggtt attctgaatg ccgatggtag cgaaccagac 540

ttgacggctt gcgcaatgac cgaaaaaggc gttctgttca attcaggtga gttcgacggt 600

ctggacttcc agaacggctt taacgctatt gccgacaaac tg 642

<210> 521
<211> 642
<212> DNA
<213> Buttiauxella izardii

<400> 521
cagtggttta tcaaaatcac cgcttacgct gacgaactgt taaacgatct ggatacgttg 60

gatcattggc ctgatcaggt caaaaccatg cagcgtaact ggattggccg ttctgaaggc 120

gttgagatta ctttcgacgt tgaaaacagc gccgagaagc tgaccgttta caccacccgc 180

ccggacacct ttatgggtgt gacttacctg gcagtggctg ccggtcaccc aatcgcccaa 240

caggcggcgc tgaacaaccc agaactggct gagttcgttg acgaatgccg taacaccaaa 300

gttgccgaag ctgaaatggc gaccatggag aagaaaggcg ttgccactgg cgttaacgcg 360

attcacccac tgactggcga atctgttcca gtttgggtag caaacttcgt gctgatggaa 420

tacggcaccg gcgcagtgat ggctgttcca ggccacgacc agcgcgactg ggaatttgcg 480

accaaatacg gcttgcagat caaaccggta attctgaatg ctgacggcag cgagcctgac 540

ttgtctgcat ccgccatgac cgaaaaaggc acactgttta actctggcga attcgacggt 600

ctggacttcc aggctggttt caacgccatt gccgacaaac tg 642

<210> 522
<211> 642
<212> DNA
<213> Buttiauxella noackiae

<400> 522
cagtggttta tcaaaatcac cgcttatgct gacgagttat taaacgatct ggatacgctg 60

gaccattggc ctgagcaggt caaaaccatg cagcgtaact ggattggccg ctctgagggt 120

gttgagatca ctttcgatgt ggaaaacagt gctgaaaagc tgactgttta caccacgcga 180

ccggacacct ttatgggtgt gacctatctg gccgttgcag cgggtcaccc aatcgcgcaa 240

caagcagcgt tgaacaaccc tgcgctggct gaattcgtcg aagagtgccg taacaccaaa 300

gttgctgaag cagaaatggc aacgatggag aagaaaggtg ttgcgactgg cgttaacgct 360

attcacccac tgacaggcga atctgttcca gtatgggtgg caaacttcgt actgatggaa 420

tacggcaccg gtgcagtaat ggccgttcca ggccacgacc aacgcgactg ggagtttgcg 480

accaaatatg gtctgcaaat caaaccggtc attctgaatg ccgatggtag tgaaccagac 540

ctgacggctt cagcaatgac cgaaaaaggc gtgctgttca attcaggtga attcgacggt 600

ctggacttcc agaacggctt taacgctatt gccgacaaac tg 642

<210> 523
<211> 642
<212> DNA
<213> Buttiauxella warmboldiae

<400> 523
cagtggttta tcaaaatcac cgcttacgcg gacgaactgt taaacgatct ggacacgctg 60

gagcactggc ccgatcaggt caaaaccatg cagcgcaact ggattggccg ttctgaaggc 120

gttgaaatta ctttcgacgt tgaaaacagc gctgaaaaac tgaccgttta caccacgcgc 180

ccggacacct tcatgggtgt gacttacctg gcggttgcgg caggtcaccc aatcgcccaa 240

caggcggcac tgaacaatcc tgaattggct gagttcgttg acgaatgccg taataccaaa 300

gttgccgaag ctgaaatggc aaccatggag aagaaaggcg tggcgaccgg cgttaacgcc 360

attcacccac tgactggcga agctgttcca gtttgggtcg ctaacttcgt cttgatggaa 420

tacggcaccg gcgcggtaat ggcggttcca ggccacgacc agcgcgactg ggaatttgcc 480

accaaatacg gcctgcaaat caaaccggtt attctgaacg ctgacgggag cgagcctgat 540

ttatctgcgg cagcaatgac cgaaaaaggc gcgctgttta actcaggcga gttcgacggt 600

ctggacttcc agaacgcgtt caacgctatc gctgataaac tg 642

<210> 524
<211> 642
<212> DNA
<213> Cedecea davisae T

<400> 524
cagtggttta ttaaaatcac cgcctatgcc gacgaactgc tgaacgacct ggacacgctg 60

gaccactggc cggatcaggt taaaaccatg cagcgcaact ggattggccg ttccgaaggc 120

gtagaaatta ccttcgacgt cgaaaacagc gccgaaaagc tgaccgttta caccacccgc 180

cctgacacct tcatgggcgt gacctacctg gccgttgccg cgggtcaccc tctggcgcag 240

caggctgcgg taaacaaccc ggctcttgcc gagtttatcg acgaatgccg caataccaaa 300

gtagccgaag ccgatatggc gactatggag aagaaaggcg ttgccacagg cattaacgct 360

attcatccgc tgaccggcga aagcgttccg gtttgggtcg caaacttcgt gctgatggaa 420

tacggcaccg gcgcggtcat ggccgttccg ggtcacgacc agcgcgactg ggaattcgcc 480

actaaatacg gcctgcaaat caaaccggtt atcctcaatg ccgacggcag cgagccggac 540

ctgtctggcg cagcaatgac cgaaaaaggc gcgctgttca actccggtga gttcaacggc 600

ctggacttcc agcaggcgtt caacgccatt gccgataagt ta 642

<210> 525
<211> 642
<212> DNA
<213> Cedecea lapagei

<400> 525
cagtggttta ttaaaatcac cgcctatgcc gacgaactgc tgaacgacct ggatacgctg 60

gatcactggc cggatcaggt caaaaccatg cagcgcaact ggattggccg ctccgaaggc 120

gtggagatta ccttcgacgt tgaaaacagc gccgagaagc tgaccgttta taccacccgc 180

cctgacacct ttatgggtgt gacctacctg gccgttgccg ccggtcaccc tctggctcag 240

caggccgcgg tcaataaccc cgcgcttggc gaatttattg aagagtgccg taacaccaaa 300

gtggccgaag ccgagatggc gaccatggag aaaaaaggtg ttgctaccgg tatcaacgct 360

attcatccgc tgaccggcga aagcattccg gtttgggtcg ctaacttcgt gctgatggaa 420

tacggcaccg gtgccgtgat ggccgttccg ggccacgatc agcgcgactg ggaattcgcc 480

accaaataca gcctgtcaat caagccggtt attctgaatg ccgacggcag cgagccggat 540

ctttccgccg ccgcaatgac cgagaaaggc acgctgttta actccggcga gttcgacggt 600

ctggacttcc agcagggctt caacgccatt gccgataagc tg 642

<210> 526
<211> 642
<212> DNA
<213> Cedecea neteri

<400> 526
cagtggttca tcaaaatcac cgcctatgct gacgaactgc tgaacgacct ggatacgctg 60

gatcactggc cggatcaggt taaaaccatg cagcgcaact ggattggccg ctccgaaggc 120

gtagaaatta ccttcgatgt agaaaacagc gccgagaagc tgaccgttta tactactcgc 180

cctgatacct tcatgggtgt gacttacctg gccgttgccg ccggccatcc tctggctcag 240

caggctgcgg taaataaccc agcgcttgcc gagttcattg aagaatgccg taacaccaaa 300

gtggccgaag ccgacatggc gaccatggag aagaaaggcg ttgcaaccgg cattaacgcc 360

attcatccgc tgaccggcga aagcgttccg gtttgggtgg ccaacttcgt gctaatggaa 420

tacggcaccg gtgccgtgat ggccgttcct ggccacgatc agcgcgactg ggaattcgcc 480

actaaatacg gtttgtcaat caagcctgtt attctgaatg ccgacggcag cgagccggac 540

ctttccgcct ccgcaatgac cgagaaaggc gcactgttta actccggcga gtttgacggt 600

ctggacttcc agcaggcgtt caacgccatt gccgataagc tg 642

<210> 527
<211> 642
<212> DNA
<213> Citrobacter amalonaticus

<400> 527
cagtggttta ttaaaatcac cgcctatgcc gatgaactgc tcaacgatct ggataaactg 60

gatcactggc ctgacaccgt gaaaacgatg cagcgcaact ggattggtcg ttctgaaggc 120

gtggaaatca ccttcaacgt gtacgattat gacaacacgc tgaccgttta cacgacccgc 180

ccggacacct ttatgggtgc cacctacctg gcggttgccg ctggtcaccc gctggcgcag 240

aaagcggcag agaacaaccc tgaactggca gcctttatcg acgaatgccg taacaccaaa 300

gtggccgaag ctgacatggc gaccatggag aaaaaaggcg tcgataccgg ctttaaggcg 360

gttcacccgc tgactggcga agcaatcccg gtctgggcgg ctaactttgt gctgatggaa 420

tacggcactg gcgcggtcat ggccgtaccg ggtcacgatc agcgcgacta cgaatttgcc 480

accaaatacg gcttaaccat taaaccggtt atcctggcag ccgatggttc agagccggat 540

ctttccgaac aggcgatgac cgataaaggc gttctgttta actctggcga atttgacggt 600

ctggccttcg acgcggcctt caacgccatt gccgataagc tg 642

<210> 528
<211> 642
<212> DNA
<213> Citrobacter braakii

<400> 528
cagtggttca tcaaaatcac cgcttacgct gacgaactgt tgcgcgatct ggataatctg 60

gatcactggc ctgacaccgt gaaaacgatg cagcgcaact ggattggccg ttctgaaggc 120

gtggaaatca ccttcgatgt gaacgattac gacaacacgt tgaccgtcta caccacacgc 180

ccggacacct ttatgggtgc gacctacctg gccgttgctg caggtcatcc gctggcgcag 240

aaagccgctg aaaataaccc agaactggct gccttcattg acgaatgccg caacaccaag 300

gttgctgaag ccgaaatggc gaccatggag aagaaaggcg tcgacactgg tttcaaagcc 360

gttcacccgc tgactggcga agagatcccg gtttgggcgg caaacttcgt gctgatggaa 420

tacggtacag gcgcagttat ggccgttccg ggtcacgatc agcgcgatta cgaatttgcg 480

actaaatatg ccctgaccat caagccggta attctgaccg ctgaaggcgc agagccggat 540

ctgtctgaac aagcgctgac cgaaaaaggc gtgctgttta actccggtga gtttgacggc 600

ctcgacttcg aagcggcctt caacgctatt gctgacaagc tg 642

<210> 529
<211> 642
<212> DNA
<213> Citrobacter diversus

<400> 529
cagtggttca ttaagatcac cgcctacgct gacgaactgc tgaacgatct ggataaactg 60

gatcactggc cagacaccgt taaaaccatg cagcgcaact ggatcggccg ttctgaaggc 120

gtcgagatca cttttgacgt gaacggctac gacaatacgc tgacggtgta caccacgcgt 180

ccagacacgt tcatgggcgc cacttacctg gcggttgccg caggtcaccc gctggcgcag 240

aaagcggctg ccaacaaccc ggaactggct gcctttatcg acgaatgccg caacaccaaa 300

gtggcggaag ccgatatggc gacgatggag aaaaaaggcg tcgacaccgg ttttaaagcc 360

attcacccac tgaccggcga agcgatcccg gtatgggccg ccaacttcgt gctgatggaa 420

tacggtaccg gtgcggtcat ggccgttccg ggccacgatc agcgtgacta tgaatttgcc 480

accaaatatg gcctgaccat caagccggtt atcctggcgg cggacggctc tgaaccggat 540

ctgtccgagc aggcgctgac cgagaaaggc gtactgttta actccggcga atttaacggt 600

ctgggctttg aagacgcctt caacgcgatt gccgataaac tg 642

<210> 530

<211>  642
<212>  DNA
<213>  Citrobacter farmeri

<400>  530
caatggttta ttaaaatcac tgcttacgcc gacgaactgc tgaacgatct ggataaactg      60

gaccactggc ctgacaccgt gaaaacgatg cagcgcaact ggattggccg ctctgaaggc     120

gtggaaatca ccttcaacgt gaacggttat gacaacacgc tgaccgtcta cacaacccgc     180

ccggacacct ttatgggtgc cacctacctg gcggttgccg caggccatcc gctggcccag     240

aaagcggcag agagcaatcc tgaactggcg gcctttatcg acgaatgccg caataccaaa     300

gtggccgaag ccgacatggc gaccatggag aaaaaaggcg tcgacaccgg ctttaaagca     360

attcatccgc tgaccggcga agcgatcccg gtctgggcgg ctaactttgt gctgatggaa     420

tatggcactg gcgcggttat ggccgtaccg ggtcacgatc agcgcgacta cgaatttgcg     480

accaaatacg gcttaaccat taaaccggtc atcctggcag ccgatggttc tgagccggac     540

ctgtccgaac aggcgatgac cgataaaggc gtcctgttta actccggtga atttgacggt     600

ctggccttcg aagcggcctt taacgccatt gccgacaagc tg                        642

<210>  531
<211>  642
<212>  DNA
<213>  Citrobacter freundii T

<400>  531
cagtggttca tcaaaatcac cgcttacgct gacgaactgc tgcgtgacct ggataacctg      60

gatcactggc ctgacaccgt gaaaacgatg cagcgcaact ggattggccg ttctgaaggc     120

gtagaaatca ccttcgatgt gaacgattac gacaacacgc tgaccgttta cacaacccgc     180

ccggacacct ttatgggtgc gacctatctg gccgttgccg caggtcaccc gctggcgcag     240

aaagccgctg caaataaccc ggaactggcc gccttcattg acgaatgccg caacaccaag     300

gttgctgaag ccgaaatggc aaccatggag aagaaaggcg tcgacacggg ctttaaagcc     360

gttcacccgc tgactggcga agagatcccg gtttgggcag caaacttcgt cctgatggaa     420

tacggtacgg gcgcagtcat ggccgttccg ggccacgatc agcgcgatta cgagtttgcg     480

actaaatatg gcctgaccat caagccagta attctgaccg ctgaaggcgc agagccggat     540

ctgtccgaac aagctctgac cgaaaaaggg gtgctgttta actccggtga gtttgacggc     600

ctcgacttcg aagcagcctt caatgccatt gctgacaagc tg                        642

<210>  532
<211>  642
<212>  DNA
<213>  Citrobacter gillenii

<400>  532
cagtggttca tcaaaatcac cgcatacgct gacgaactgc tgcgcgacct ggaccgtctg      60

gatcactggc ctgacaccgt aaaaacgatg cagcgcaact ggattggccg ctctgaaggc     120

gtggagatca cctttgatgt caacggttat gacaacacgc tgaccgttta caccacccgc     180

ccggacacct ttatgggtgc cacttatctg gccgttgccg caggtcaccc gttggcgcag 240

aaagccgcgg cgaataaccc ggaactggcg gcgtttgttg acgaatgccg caacaccaag 300

gttgccgagg ctgaaatggc gaccatggag aagaaaggcg tcgacactgg ctttaaagcg 360

attcatccgc tgaccggtga agagatccca gtttgggctg caaacttcgt gctgatggaa 420

tacggtacag gtgccgtgat ggccgtgccg ggtcacgatc agcgcgacta cgagttcgca 480

accaaatatg gtctgagcat caagccagtt atcctggcgg cggacggttc agaaccggat 540

ctgtctgaga aggcactaac tgagaaaggc gtgctgttta actctggtga gtttaacggt 600

ctcgatttcg aagcggcttt caacgccatt gccgacaagc tt 642

<210> 533
<211> 642
<212> DNA
<213> Citrobacter koseri

<400> 533
cagtggttca ttaagatcac cgcctacgct gacgaactgc tgaacgatct ggataaactg 60

gatcactggc cagacaccgt taaaaccatg cagcgcaact ggatcggccg ttctgaaggc 120

gtcgagatca cctttgacgt gaacggctac gacaatacgc tgacggtgta caccacgcgt 180

ccagacacgt tcatgggcgc cacttacctg gcggtcgccg caggtcatcc gctggcgcag 240

aaagcggctg ccaacaaccc ggaactggct gcctttatcg acgaatgccg caacaccaaa 300

gtggcggaag ccgatatggc gacgatggag aaaaaaggcg tcgacaccgg ttttaaagcc 360

attcacccgc tgaccggcga agcgatcccg gtatgggccg ccaacttcgt gctgatggag 420

tacggtaccg gtgcggtcat ggccgttccg ggccatgatc agcgtgacta tgaatttgcc 480

accaaatatg gcctgaccat caagccggtt atcctggcgg ctgacggctc tgaaccggat 540

ctgtccgagc aggcgctgac cgagaaaggc gtactgttta actccggcga atttaacggt 600

ctgggctttg aagacgcctt caacgcgatt gccgataaac tg 642

<210> 534
<211> 642
<212> DNA
<213> Citrobacter murliniae

<400> 534
cagtggttta tcaaaatcac cgcttacgct gacgaactgt tgcgcgacct ggataacctg 60

gatcactggc ctgacacagt gaaaacgatg cagcgtaact ggatcggtcg ttctgaaggc 120

gtggaaatca ccttcgatgt caccggttat gacaacacgc tcaccgttta caccacccgc 180

ccggacacct ttatgggtgc cacttacctg gcggttgcag cgggtcaccc gctggcgcag 240

aaagcggcgg caaacaatcc ggaactggcg gctttcatcg acgaatgccg caacaccaag 300

gttgccgaag ccgaaatggc aaccatggag aagaaaggcg tcaacactgg ctttaaagcg 360

attcacccgc tgactggcga agagatcccg gtatgggcgg cgaacttcgt gctgatggaa 420

tacggcacgg gtgctgtaat ggccgttcct ggccacgatc agcgcgacta cgaatttgcc 480

accaaatacg gtctgaacat taagccggtc attctggctg ctgacggttc agaaccggat 540

ctgtccgaac aagctctgac tgaaaaaggc gtgctgttca actcaggtaa atttgacggt 600

ctgtcctttg aagacgcgtt caacgccatt gccgacgaac tg 642

<210> 535
<211> 642
<212> DNA
<213> Citrobacter rodentium

<400> 535
cagtggttta tcaaaatcac cgcctacgcc gacgagctgc tgaacgatct ggataagctg 60

gatcactggc ctgacaccgt caagacgatg cagcgtaact ggatcggccg ttccgaaggc 120

gtggagatca cctttgacgt caccggttac gacaacacgc tgaccgttta caccactcgc 180

ccggacacct tcatgggcgc cacttacctg gcggtggccg ccggtcaccc gctggcgcag 240

aaagcggcgg cgaataaccc ggaactggcc gcctttatcg acgaatgccg caataccaaa 300

gtggccgaag ccgacatggc gaccatggag aaaaaaggcg tcgataccgg ctttaaagcg 360

gtgcacccgt tgaccggcga agagatcccg gtctgggcgg cgaacttcgt gctgatggag 420

tacggtactg gcgcggtaat ggccgtgccg ggtcacgatc agcgcgacta cgaatttgcc 480

accaaatatg gtttaaccat caagcccgtt attcttgccg ctgacggttc cgaaccggat 540

ctctccgaac aggcgctgac cgaaaaaggc gtactgttca actccggcga atttgacggt 600

ctggcctttg aagaggcctt taacgcgatt gccgacaagc tg 642

<210> 536
<211> 642
<212> DNA
<213> Citrobacter sedlakii

<400> 536
cagtggttta tcaaaattac cgcttatgcg gacgaactgc tgaacgatct ggacaagctc 60

gatcactggc ctgacaccgt caaaacgatg cagcgcaact ggattggccg ttctgaaggc 120

gtggaaatca cctttgatgt gaccggttat gaagatacgc tgaccgttta caccacccgc 180

ccggatacct ttatgggcgc tacttacctg gccgttgccg ccggtcaccc gctggcgcag 240

aaagccgcgg cgaacaaccc ggaactggct gcctttatcg acgaatgccg caataccaaa 300

gtcgcggaag ccgacatggc gaccatggag aagaaaggcg tcgataccgg ctttaaagcg 360

gtacatccgt taaccggcga agcgatcccg gtctgggcgg cgaacttcgt gctgatggaa 420

tacggcaccg gcgcggtcat ggcggttccg ggtcacgatc agcgcgacta cgaatttgcc 480

accaaatacg gtttaaccat caaacccgtc attcttgccg ccgacggttc cgaaccggat 540

ctgtccgaac aggcgctgac cgaaaaaggc gtgctgttta actccggcga attcgacggt 600

ctggcctttg aagacgcctt caacgcgatt gccgacaaac tg 642

<210> 537
<211> 642

<212>   DNA
<213>   Citrobacter werkmanii

<400>   537
cagtggttca ttaaaatcac cgcttacgct gacgaactgc tgcgcgatct ggataacctg      60

gatcactggc cggacaccgt taagaccatg cagcgcaact ggatcggtcg ttcggaaggt     120

gtggaaatca ccttcgacgt gaacggttac gacaacacgc tgaccgtcta taccacccgc     180

ccggatacct ttatgggtgc gacctacctg gccgttgccg ctggtcaccc gctggcgcag     240

aaagccgccg cgaacaaccc tgaactggcc gccttcattg acgaatgccg caacaccaag     300

gttgctgaag ccgaaatggc gaccatggag aagaaaggcg tcgacactgg ctttaaagcg     360

gttcacccgc tgactggcga agagatcccg gtttgggcgg caaactttgt cctgatggaa     420

tatggtacag gcgcagtgat ggccgttccg ggtcacgatc agcgcgacta cgaatttgcc     480

accaaatatg gcctgaccat caagccggtc attctgaccg ccgaaggtgc agagccggat     540

ctgtcggaac aggcactgac tgaaaaaggc gtactgttta actccggtga gtttgacggc     600

ctcgacttcg aagctgcctt caacgccatt gctgacaagc tg                        642

<210>   538
<211>   642
<212>   DNA
<213>   Citrobacter youngae

<400>   538
cagtggttca tcaaaatcac cgcttacgct gacgaactgc tgcgtgatct ggataacctg      60

gatcactggc ctgatactgt taaaaccatg cagcgcaact ggattggccg ctctgaaggt     120

gtagaaatca ccttcaacgt gaaccgttac gacaacacgc tgaccgtcta caccacccgt     180

ccggatacct ttatgggtgc gacctacctg gccgttgccg caggtcaccc gctggcgaaa     240

aaagcggcgg caaataaccc ggaactggct gccttcattg acgaatgccg taacaccaag     300

gttgctgaag ccgaaatggc gaccatggag aaaaaaggcg tcgataccgg ctttaaagcg     360

gttcatccgc tgactggcga agaaatcccg gtttgggcgg caaacttcgt cctgatggaa     420

tacggcaccg gtgcggtgat ggccgttccg ggtcacgatc agcgcgacta cgaatttgcc     480

accaagtatg gcctgtccat caagccggta attctgactt ctgaaggcgc agagccggat     540

ttgtccgagc aggcactgac cgaaaaaggc gtgctgttta actccggtga gtttgacggc     600

ctggccttcg aagacgcgtt caacgccatt gccgacaagc tg                        642

<210>   539
<211>   642
<212>   DNA
<213>   Dickeya chrysanthemi T

<400>   539
cagtggttca tcaaaatcac cgcctatgcc gatgaactgc tcaatgatct tgatacgctg      60

gaaagctggc cggaacaggt caaaaccatg caacgtaact ggatcggccg ctccgaaggg     120

gtggaaatca ccttccgtat cgccgacgcc gacgatacgc tgaccgttta cactacccgc     180

ccggatacct tcatgggcgt gacttacgtc gcggtggcag ccggtcaccc gctggcgcag 240

aaagcggcgg aaaacaaccc agcgttgcaa gactttatcg tcgaatgccg caacaccaaa 300

gtggcggaag ccgacatggc gaccatggag aaaaaaggcg tcgccaccgg cctgttcgcc 360

atccacccgc tgaccggcga aaacgtgccg gtatgggcgg ccaactttgt gctgatggaa 420

tacggcaccg gtgcggtgat ggctgtaccg ggccacgacc agcgcgactg ggagtttgcc 480

cacaaatata acctgccggt aaaaccggtt attctgttgg cagacggtag cgaaccggat 540

ctgtccgctc aggcgcaaac ggaaaaaggc tgcctgttta actccggcga attcgacggc 600

ttgaatttcg atgccgcgtt caacgccatt gccgacaaac tg 642

<210> 540
<211> 642
<212> DNA
<213> Dickeya dadantii

<400> 540
cagtggttca tcaaaatcac cgcttacgcc gacgaactgc tcaacgatct ggatacgctg 60

gaaagctggc ctgagcaggt caaaaccatg cagcgcaact ggatcggccg ttccgaaggg 120

gtggaaatca cctttgacgt ggcagacagt gaagagaagc tgagtgttta taccacccgc 180

ccggacacct tcatgggcgt gacctacgtc gccgtggcgg ccggtcaccc gctggcgcag 240

aaagcggcgg aaaataatcc ggcgctgcag gactttatcg ccgaatgtgg caacactaaa 300

atggcggaag ccgacatggc gaccatggag aaaaaaggca tcgccaccgg tttgttcgcc 360

attcacccgc tgactggcga aaatgtgccg gtgtgggccg ccaacttcgt gctgatggaa 420

tacggcaccg gcgctgtgat ggcggtaccg ggccatgacc agcgcgactg ggaattcgcc 480

cacaaataca acctgccggt gaaaccggtt attctgatgg ctgacggcag cgagccggat 540

ctgtccgcgc aggcgcagac ggaaaaaggc cgtctgttca actccggcga gttcaatggg 600

ctggatttcg acgccgcgtt caatgccatt gccgataaac tg 642

<210> 541
<211> 642
<212> DNA
<213> Edwardsiella hoshinae

<400> 541
cagtggttca ttaagatcac cgattatgcc gatcaactgc tcaacgattt ggataagctg 60

gaagactggc cggaacaggt caagaccatg cagcgtaact ggatcggtcg ctccgaaggg 120

gtggagatca ccttccgcgt cgccgatcgc gacgacacct tcgccgtcta caccacccgc 180

ccagacacct ttatgggcgt cagctacctg gcgatcgcgg cggcacaccc gctggctcag 240

gaagccgcag ccaacaaccc ggcgttggcg cagtttatcg atgagtgcaa aaacaccaag 300

gttgccgaag ccgacatggc gaccatggag aagaaaggca tggctaccgg cctgtacgct 360

gtacatccgt tgagcggtga gcgtctgccc atctgggtcg ccaacttcgt attgatggat 420

tacggtaccg gcgcagtgat gtccgttccg gctcacgacc agcgtgactg ggagttcgcc 480

gacaagtatg gcctaccaat gaaagcggtg atcctgaccg ccgatggcca cgcaccagac 540

atcagcgccg ccgccatgac cgaaaaaggg gtgctgttca attctggcga gtttgatggt 600

ctagacttcg ccgccgcctt cgacgcggta gccacccgct ta 642

<210> 542
<211> 642
<212> DNA
<213> Edwardsiella ictaluri

<400> 542
cagtggttta tcaagatcac cgactacgcc gatcagctgc tcaacgacct cgacaggctg 60

gaagagtggc cggagcaggt gaaaaccatg cagcgcaact ggatcggtcg ttctgaaggg 120

gtagagatca ccttccacgt cgccgatcgc gatgatacct ttgccgtcta caccacccgc 180

ccggacacct tcatgggcgt cagctacctg gccatcgccg ccgcacaccc gctggcccag 240

caagccgcca ccggcaatcc ggcactgacg cagttcatcg acgagtgcaa aaacaccaag 300

gttgccgagg ccgacatggc gaccatggag aagaaaggca tggccaccgg cctgtacgcc 360

atccacccgc tcaacggtga gcgtctgcct atctgggtcg ccaactttgt gctgatggac 420

tacggcaccg gcgcggtgat gtccgttccg gcccacgacc agcgcgactg ggagttcgcc 480

accagatatg acctgccgat gaagccggtg atcctgaccg ccgacggtca ggccccggac 540

atccgcgccg ccgccatgac cgacaagggc gtgctgttca attccggcga atttgacggt 600

ctcgacttca gcgccgcctt cgacgccgtc gccaaccgcc tg 642

<210> 543
<211> 642
<212> DNA
<213> Edwardsiella tarda T

<400> 543
cagtggttca tcaagatcac cgactatgcc gatcagctgc tcaacgatct ggataagctg 60

gaggattggc cggaacaggt caagaccatg cagcgtaact ggatcggccg ttccgaaggg 120

gtcgagatca ccttccgcgt cgccgatcgc gacgagacct tcgccgtcta taccacgcgc 180

ccggacacct tcatgggggt cagctatctg gcgatcgccg ccgcgcaccc gttggcccaa 240

gcggccgcgg ccaacaaccc ggcgttggcg cagtttatcg acgaatgcaa gaacaccaag 300

gtcgccgaag ccgacatggc gaccatggag aagaagggca tggccaccgg cctgtacgcc 360

gtgcacccgc tgagcggtga gcgcctcccc atctgggtcg ccaacttcgt gctgatggac 420

tacggcaccg gcgcggtgat gtccgtcccg gcccatgacc agcgtgactg ggagttcgcc 480

agcaagtatg acctgccgat gaaggcggtg atcctgaccg ccgatggcca ggtcccggat 540

atcgccgccg cggcgatgac cgaaaagggc gtgctgttca actccggcga gtttgacggg 600

ctggacttcg cggccgcctt cgacgcggta gccgcccgct ta 642

<210> 544
<211> 642
<212> DNA

<213>  Enterobacter aerogenes

<400>  544
cagtggttta ttaaaatcac cgcctacgct gacgaactgc tcaacgatct ggacaacctg      60

gatcactggc ctgacaccgt taagaccatg cagcgcaact ggatcggccg ctctgaaggg     120

gtagaaatct cctttgacgt caacgactac gccgataagc tgaccgtcta caccacccgc     180

ccggatacct tcatgggctg cacctacctt gccgtcgcag cgggtcaccc gctggcgcaa     240

caggctgcgg ctaacaatcc ggcgctggcc gcctttatcg acgaatgccg taacaccaaa     300

gttgccgaag ccgatatggc gacgatggag aaaaaaggcg tcgacaccgg ctttaaagct     360

attcacccgc tgaccggcga agccatcccg gtatgggcgg caaacttcgt actgatggag     420

tacggcaccg gcgcggtgat ggccgttccg ggtcacgacc agcgcgacta cgagtttgcc     480

agcaaatacg gcctgaacat caagccggtt atcctcgccg ccgacggttc cgagccggat     540

ctctccgagc aggcgctgac cgagaaaggc gtgctgttca actccggcga atttagcggc     600

ctggattacg aagcgggctt caacgccatt gccgacaaac tg                        642

<210>  545
<211>  642
<212>  DNA
<213>  Enterobacter amnigenus

<400>  545
cagtggttca ttaaaatcac cgcttacgct gacgaactgc tgcgcgatct cgataatctg      60

gatcactggc cagacaccgt taaaacgatg cagcgcaact ggattggccg ctctgaaggc     120

gtagaaatca ccttcaacgt tgaaaacagc gatcagacgc tcacggtgta taccacgcgt     180

cccgacacct tcatgggtgc gacttacctg gccgttgccg cgggtcatcc gctggcacag     240

caggctgctg cgaacaatcc agcgctggcc gctttcatcg acgaatgccg caataccaaa     300

gtggcagaag ccgatatggc gacgatggag aaaaaaggcg ttgcgacagg tttcaacgcc     360

attcacccac tgaccggcga agcggttccc gtttgggcag ccaactttgt gctgatggaa     420

tacggcacgg gtgcagttat ggccgttccg ggccacgatc agcgtgatta tgagtttgcg     480

accaaatacg gcctcaacat caagccggtt atcctggcgg ctgacggttc agaaccagat     540

ctgtctgagc aggcgctgac cgaaaaaggc accctgttta actctggtga attcagcgga     600

ttgagcttcg aagaaggctt caacgctatc gcggataaac tg                        642

<210>  546
<211>  642
<212>  DNA
<213>  Enterobacter asburiae

<400>  546
cagtggttta tcaaaatcac cgcttacgct gatgagctgc tgaacgatct ggataacctg      60

gatcactggc cagataccgt taagaccatg cagcgcaact ggatcggccg ttctgaaggc     120

gtggagatta ccttcaacgt tgagaactac gaccagaccc tgactgtcta caccacccgt     180

ccggacacct tcatgggcgc gacctacctg gccgtagccg caggtcaccc gctggcgcag     240

aaagcagcgg aaaacaatcc ggagctggcc accttcatcg acgagtgccg caacaccaaa 300

gtggccgaag ccgacatggc gacgatggag aaaaaagggg ttgataccgg ctttaaagcc 360

attcacccac tgactggcga agcaatccct gtctgggccg ccaacttcgt tctgatggaa 420

tacggcacag gcgccgtgat ggctgttccg ggtcacgacc agcgcgacta cgaatttgcc 480

accaaatatg gcctgaccat caagcctgtt atcctggcgg ctgacggttc agaaccagac 540

ctgtctgaac acgcgctgac tgaaaaaggc accctgttca actccggtga atttagcggt 600

ctcagcttcg aagagggctt caacgccatc gccgataagc tg 642

<210> 547
<211> 642
<212> DNA
<213> Enterobacter cancerogenus

<400> 547
cagtggttta tcaaaatcac cgcctacgct gacgagctgc tgaacgatct ggacaagctt 60

gaccactggc cagacaccgt taaaaccatg cagcgcaact ggattggccg ttctgaaggg 120

gtagagatca ccttcaacgt ggacaactac gatcggccgc tgaccgttta caccacccgt 180

ccggatacct tcatgggcgc gacctacctg gccgtggccg caggtcaccc gctggcgcag 240

aaagcggcag aaaacaatcc ggaactggcg accttcatcg acgaatgccg caacaccaaa 300

gtggccgaag ccgacatggc gacgatggag aagaaaggcg ttgcgaccgg ttactacgct 360

aagcatccgc tgactggcga agcgatcccg gtgtgggccg caaacttcgt gctgatggag 420

tacggcacgg gtgccgtgat ggccgttccg ggtcacgatc agcgcgacta cgagtttgcc 480

accaaatacg gtctggacat caagcctgtt atcctggctg ctgacggttc tgaaccggac 540

ctgtccgaac acgcgctgac cgaaaaaggc acgctgttca actccggcga gtttagcggc 600

ctgagcttcg aagacggttt taacgccatt gccgataaac tg 642

<210> 548
<211> 642
<212> DNA
<213> Enterobacter cloacae sbsp. cloacae T

<400> 548
cagtggttta tcaaaatcac cgcttacgct gacgaactgc tgaacgatct ggacacgctg 60

gaccactggc cggataccgt taagaccatg cagctcaact ggatcggccg ttctgaaggc 120

gtggagatca ccttcaacgt tgagaactac gatcagactc tgaccgtcta caccacccgt 180

cctgatacct tcatgggcgc gacctatctg gccgtggccg caggtcaccc gctggcgcag 240

aaagctgccg aaaataaccc ggagctggct atctttatcg acgaatgccg caacacgaaa 300

gtggcagaag ccgacatggc gaccatggag aagaaaggcg ttgccacggg cttctatgcg 360

aagcacccac tgactggcga agcgattccg gtctgggcag caaacttcgt gctgatggaa 420

tacggtaccg gtgccgtgat ggctgttccg ggtcacgatc agcgcgacta tgaatttgcc 480

accaaataca acctgccgat caaagccgtt attctggcgg ctgacggctc tgaacctgac 540

ctgtctgagc aagcactgac ggaaaaaggc acactgttta actctggtga gttcagcggc 600

ctgagcttcg aagagggttt caacgccatc gccgacaaac tg 642

<210> 549
<211> 642
<212> DNA
<213> Enterobacter cloacae sbsp. dissolvens

<400> 549
cagtggttta tcaaaatcac cgcttacgct gacgaactgc tgaacgatct ggacacgctg 60

gaccactggc cggataccgt taagaccatg cagcgcaact ggatcggtcg ttctgaaggc 120

gtggagatca ctttcaacgt tgagaactac gatcagaccc tgaccgtcta caccactcgt 180

ccggacacct tcatgggcgc gacctatctg gccgtggccg caggtcaccc gctggcgcag 240

aaagctgctg aaaataaccc agagctggcc accttcatcg acgaatgccg caacacgaaa 300

gtggcggaag ccgatatggc gacgatggag aaaaaaggcg ttgccacggg cttctatgcg 360

aagcacccac tgactggcga agcgattccg gtctgggcag caaacttcgt gctgatggaa 420

tacggcactg gcgcggtgat ggccgttccg ggtcatgacc agcgcgatta tgaatttgcc 480

accaaatata acctgccgat caaagccgtt attctggcag ctgacggctc tgaacctgac 540

ctgtctgagc aagcactgac ggaaaaaggt acgctgttta actctggtga gttcagcggt 600

ctgagcttcg aagacggctt caacgccatc gccgacaaac tg 642

<210> 550
<211> 642
<212> DNA
<213> Enterobacter cowanii

<400> 550
cagtggttta tcaaaatcac cgcctatgcc gacgagctgc tgaacgatct ggataaactg 60

gatcactggc cggataccgt gaaaacgatg cagcgcaact ggatcggccg ttctgaaggg 120

gtggagatca cctttgatgt gcagaacagc gtcgagaagc tgaccgttta caccacccgc 180

ccggacacct ttatgggcgt gacctatctc gccgttgccg ccggtcaccc gctggcgcag 240

caggctgccc agaccaaccc ggagctggcg agctttatcg acgaatgccg caacaccaaa 300

gtcgcggaag ccgaaatggc gacgatggag aaaaaaggcg tcgataccgg cctgaaagca 360

attcatccgc tgaccggcga agcgattccg gtctggacgg cgaactttgt gctgatggag 420

tacggcaccg gcgcggtgat ggccgttccg ggtcacgatc agcgcgacta cgagtttgcc 480

agcaaatatc agctgaacat caagccggtc atcctgaatg ccgacggcag cgagccggat 540

ctcagcgccc aggcgctgac cgaaaaaggc gtgctgttta actccggtga gtttgacggt 600

ctcgacttca acgccgcctt taacgccatt gctgacaagc tg 642

<210> 551
<211> 642
<212> DNA
<213> Enterobacter gergoviae

<400>  551
cagtggttta ttaaaatcac cgcctatgcc gacgaactgc tcaacgatct tgacacgctc      60

gaccactggc cggacacggt taagaccatg cagcgcaact ggatcggccg ctctgaaggc     120

gtggaaatca ctttcgacgt caaaaaatac gccgatacgc tgacggtcta caccacccgc     180

ccggacacct ttatgggcgc cacctacctg gcggtcgcag cgggccaccc gctggcccag     240

caggctgcgg cgaataatcc tgaactggca gcatttattg acgagtgccg caacaccaaa     300

gttgccgaag ccgacatggc aacgatggag aagaaaggca tcgacaccgg ctttaaggcc     360

gttcacccac tcaccggcga agaaatcccg gtgtgggcag ctaacttcgt gctgatggag     420

tacggcaccg gcgccgtgat ggccgttccg ggtcacgacc agcgcgacta cgagttcgcc     480

cgcaaatacg gtctgaccat caagccggtt attctcaatg ccgacggcag cgagccggac     540

ctctctgaac aggcgttgac tgaaaaaggc accctgttta actccggcga gttcagcggc     600

ctgagcttcg acgcaggctt caacgccatt gccgatcgtc tg                        642

<210>  552
<211>  642
<212>  DNA
<213>  Enterobacter hormaechei

<400>  552
cagtggttta tcaaaatcac cgcctacgct gacgaactgc tgaacgatct ggataacctg      60

gatcactggc cggacaccgt taagaccatg cagcgcaact ggattggccg ttccgaaggc     120

gtggaaatta ccttcaacgt taacgactac gaccagacgc tgaccgtcta caccacccgt     180

ccggatacct tcatgggtgc gacctacctg gctgtggctg ccggtcaccc gctggcgcaa     240

aaagcggcgg aaaacaaccc ggaactggca accttcatcg acgaatgccg caacactaaa     300

gtggccgaag ccgacatggc gacgatggag aaaaaaggcg ttgataccgg cttcaaagcg     360

atccacccgc tgacgggcga agccatccct gtctgggcgg caaactttgt gctgatggaa     420

tacggtacgg gcgccgtgat ggccgtgccg ggtcacgatc agcgtgacta cgaatttgcc     480

accaaatacg gtctgaccat caagcctgtt attcttgctg ctgatggctc cgagcccgac     540

ctctccgagc aggcgctgac tgaaaaaggc accctgttta actccggtga gttcagcggc     600

ctgagcttcg aagaaggctt caacgccatc gccgacaaac tg                        642

<210>  553
<211>  642
<212>  DNA
<213>  Enterobacter kobei

<400>  553
cagtggttca ttaaaatcac cgcctacgcc gaagagctgt taaacgatct ggataccctt      60

gatcactggc cagacaccgt taaaaccatg cagcgcaact ggattggtcg ttccgaaggc     120

gtggaaatca cctttgacgt gcagggccat gacagcaagc tgacggttta caccacccgt     180

ccggacacct ttatgggtgc cacttatctg gcggtggctg ccggtcaccc gctggcgcag     240

gccgccgcgg aaaataatcc ggcgctggca accttcatcg atgaatgccg caataccaaa 300

gtggcggaag ccgacatggc gacgatggag aaaaaaggcg ttgataccgg cttcaaagca 360

atccacccgc tgaccggcga agccatcccg gtatgggccg caaacttcgt gctgatggag 420

tacggtacag gcgccgtgat ggcggttccg ggccacgatc agcgtgacta tgagtttgcc 480

accaaatacg gcctgaccat caagccggtt attctgaccg cagacggcgc tgaaccggat 540

ctgtccgcac aggcgctgac ggaaaaaggc accctgttta actccggcga atttagcggc 600

ctgagcttcg aagaaggctt taacgccatt gccgacaagc tg 642

<210> 554
<211> 642
<212> DNA
<213> Enterobacter ludwigii

<400> 554
cagtggttta ttaaaatcac cgcctacgct gacgagctgc tgaacgatct ggataatctg 60

gatcactggc ctgataccgt caagaccatg cagcgtaact ggatcggccg ttcagaaggc 120

gtggagatca ccttcaacgt tgagaacgac gatcggaccc tgaccgtcta cactacccgt 180

ccggacacct tcatgggtgc gacctatctg gccgtggccg ctggtcaccc gctggcgctg 240

aaagccgcgg aaaacaaccc ggaactggcg gccttcatcg acgagtgccg caacaccaaa 300

gtggccgaag ccgacatggc gacgatggag aagaaaggcg ttgataccgg ctttaaggct 360

atccatcccc tgacgggcga agcaatccct gtctgggcag ccaacttcgt gctgatggag 420

tacggtacgg gcgccgtcat ggccgttcca ggccacgatc agcgtgacta cgaatttgcc 480

accaaatatg gcctgaccat taaacctgtc atcctcgcgg ctgacggttc tgagccagac 540

ctctccgcac aagccctgac cgaaaaaggc acgctgttca actcgggtga attcagcggt 600

ctgagctttg aagatggctt caacgcgatt gccgacaagt ta 642

<210> 555
<211> 642
<212> DNA
<213> Enterobacter nimipressuralis

<400> 555
cagtggttta tcaaaatcac cgcttacgcc gacgaactgc tgcgcgacct ggatacgctg 60

gatcactggc cagatactgt taaaaccatg cagcgcaact ggattggccg ttctgaaggc 120

gtcgaaatca cctttgacgt gcagaacagc gatcaaaaac tgacggttta caccacgcgc 180

cctgacacct tcatggggtc cacctacctg gccgtggctg ccggtcaccc gctggcacag 240

caggcggcgg cgaccaatcc tgaactggct gctttcatcg acgaatgccg caacaccaaa 300

gtggccgaag ccgaaatggc aaccatggag aaacgtggcg tcgcgacggg ctttaacgcc 360

attcatccgc tgaccggtga agcaatcccg gtctgggcgg ctaacttcgt gctgatggaa 420

tacggcaccg gtgccgtgat ggcggttcca ggccacgatc agcgcgatta cgaatttgcg 480

accaaatacg gcctgactat taagccggta atcctggccg ccgacggttc tgaaccggat 540

ctgtccgagc aggcgctgac tgaaaaaggc accctgttca actccggcga atttagcggc 600

ctgagcttcg aagagggctt caacgctatc gctgacaaac tg 642

<210> 556
<211> 642
<212> DNA
<213> Enterobacter pyrinus

<400> 556
cagtggttta tcaaaatcac cgcctacgcc gacgaactgc ttaacgatct ggacaccctc 60

gaccactggc cggacaccgt taaaaccatg cagcgcaact ggatcggccg ctctgaaggg 120

gttgagatca cgttcgacgt ggccgactat gccgacaagc tgacggttta caccacccgc 180

ccggacacct tcatgggtgc gacctatctg gctgtcgccg cgggccatcc gctggcccag 240

caggcggcgc agcataaccc cgagctggct gcctttatcg acgaatgccg caataccaaa 300

atggccgaag ccgatatggc gaccatggag aaaaaaggca tcgataccgg ctttaaagcg 360

attcatccgc tgaccggcga agctatcccg gtatgggccg caaactttgt actgatggag 420

tacggtaccg gcgcggtaat ggccgttccg ggtcacgacc agcgcgacta cgaatttgcc 480

agcaaatacg gccttgccat caagccggta attctggccg ccgacggcag cgagccggac 540

ctgtccgaac aggcaatgac ctacaaaggc gtgctgttta actccggcga gttcagcggc 600

ctggatttcg aagcaggctt taacgctatt gccgacaaac tg 642

<210> 557
<211> 642
<212> DNA
<213> Enterobacter radicincitans

<400> 557
cagtggttta tcaaaatcac cgcctacgcc gacgaactgc tgaacgacct ggataatctg 60

gatcactggc cggataccgt gaaaaccatg cagcgtaact ggattggccg ttccgaaggc 120

gtggaaatca cctttgacgt tcaggacagc gaccagaagc tgacggtcta tacgacccgt 180

ccggatacct ttatgggcgc gacctatctg gccgttgccg caggtcaccc gctggcgcaa 240

aaagccgcag ccagtaacgc agagctggcc gctttcatcg acgaatgccg caataccaaa 300

gtggcggaag ccgatatggc gacgatggag aaaaaaggcg ttgctaccgg ctttaaagcg 360

gttcatccgc tgaccggcga agcgatcccg gtctgggcgg caaacttcgt actgatggag 420

tacggcaccg gtgcggtcat ggccgttccg ggtcacgatc agcgcgacta tgaatttgcc 480

accaaataca acctgccgat caaggcggtt attctggcgg cggacggttc agagccggat 540

ctgtctgagc aggcgctgac tgaaaaaggc acgctgttca actctggcga gttcagcggt 600

ctgagctttg aagaaggctt caacgcgatt gccgacaagc tg 642

<210> 558
<211> 642
<212> DNA
<213> Enterobacter sakazakii

<400>  558
cagtggttta ttaaaatcac cgcctatgcc gacgagctgc taaacgatct cgataagctc      60

gatcactggc cggataccgt taaaaccatg cagcgtaact ggattggtcg ctctgaaggt     120

gtggaaatca cctttgacgt ccagaacagc gacgaaaagc tgacggttta taccacccgt     180

ccggacacct ttatgggcgt cacctatctg gccgtggcgg cgggtcatcc gctggcgcag     240

gccgcggcag ccgctaaccc ggcgctgggc gatttcattg ccgaatgccg caacaccaaa     300

gttgctgaag ccgatatagc gacgatggag aaaaaaggcg tcgatacggg cctgaaagcg     360

attcacccgc taaccggcga agctatcccg gtatgggcgg cgaacttcgt gctgatggaa     420

tatggcaccg gcgccgtcat ggccgttccg ggtcacgatc agcgcgacta tgaattcgcg     480

accaaataca gtctgccgat taagccagtt atcctgaccg cagaaggcac cgcgccggat     540

ctgagcgaac aagcgctcac cgaaaaaggc gtgctgttca actccggtga gtttgacggt     600

cttgattttg aagccgcctt taacgccatt gccgacaaac tc                        642

<210>  559
<211>  642
<212>  DNA
<213>  Erwinia amylovora T

<400>  559
cagtggtttg tcaaaatcac cgactatgct gacgaattgc tcaatgacct cgacaagctg      60

gaaagctggc ctgaacaggt gaaaaccatg cagcgtaact ggattggtcg ctccgaaggt     120

gtggaaattg agttcagcgt tattgacagc gaagagaagc tgggcgtcta taccacccgt     180

cccgacacct ttatgggcgt tacctatctg gcagtagcgg cgggtcatcc gctggcttct     240

caggcggcga tgaccaatcc ggcgctggcg gactttattg ccgaatgccg taacaccaaa     300

gttgccgagg cagatatggc aacgatggaa aagaaaggca tggcgaccgg cctgtttgca     360

acacatccgc tgacgggcga aaaagtgccg gtgtgggtgg caaactttgt gctgatggaa     420

tacggcaccg gtgcggttat ggccgtaccg ggccacgacc agcgtgactg ggagtttgcc     480

agcaaatacc gtctgccaat caaagcggtt attcttgccg ctgatggcag cgtaccggat     540

ctgagcgcct cggcgatgac ggaaaaaggc acgctgttta actctgggga atttgacggt     600

ttgtctcacg aagcgggctt tgaggccatt gccaccaagc tt                        642

<210>  560
<211>  642
<212>  DNA
<213>  Erwinia aphidicola

<400>  560
cagtggtttg ttaaaatcac cgactatgcc gacgaactgc tgaatgacct cgacaagctg      60

gaaagctggc ctgagcaggt caaaactatg cagcgcaact ggattggccg ctccgaaggg     120

gtggaaatcg agttcagcgt tatcgacagc gcagagaagc tgagcgtcta caccacgcgc     180

ccggacacct ttatgggtgt gacctacctg gccgtcgctg cgggccaccc gctggccgct     240

caggcgtccg caaccaaccc ggcgctggct gacttcatcg ccgagtgccg gaacaccaag     300

gtggccgaag ctgaaatggc gaccatggag aagaaaggca tggcgaccgg cctgtttgct 360

gaacatccac tgaccggtga gaaattgccg gtgtgggtag caaactttgt gctgatggaa 420

tacggcaccg gtgccgtaat ggccgtacct ggccacgacc agcgcgactg ggaattcgcc 480

accaagtatc acctggccat caagccggtg atccttgccg ctgacggcag cgcgccagac 540

ctgagcgaag ccgcgatgac cgaaaaaggc acgctgttta actccggtga gtttgacggt 600

ctgtcctatg aagacggttt caacgccatt gccaacaaac tg 642

<210> 561
<211> 642
<212> DNA
<213> Erwinia billingiae

<400> 561
cagtggtttg ttaaaatcac cgattacgct gatgaactgt taaatgacct ggacacgctg 60

gaaagctggc ctgagcaggt taaaaccatg cagcgtaact ggatcggccg ttctgaaggg 120

gtggaaatca cctttgacgt ggcagaaagc cccgaaaaac tgagcgttta taccacccgt 180

ccagacacct ttatgggtgt gacctatctg gccgtggctg cgggccatcc gctggctgcg 240

caggctgccg cgtcgaatcc tgcgctggcc gactttattg ccgaatgccg taataccaag 300

gttgccgaag ctgaaatggc gaccatggag aaaaaaggca tggccaccgg caagtttgcc 360

gtgcacccac tgtctggcga gaaagtgccg gtctgggtcg ccaattttgt gctgatggaa 420

tacggcaccg gtgcggtgat ggccgttcct ggccacgacc agcgtgactg ggagttcgcc 480

accaagtaca acctcactat caagcccgtg atccttaatg ctgacggaag cgagccggat 540

ctgaccgacg cggccatgac cgataaaggc acgctgttta actcaggcga gtttgacggt 600

ctgtcgtacg aagcaggctt taacgccatt gctgagaagc tg 642

<210> 562
<211> 642
<212> DNA
<213> Erwinia mallotivora

<400> 562
cagtggttta tcaaaatcac agactatgcc gatgagctgc tcaacgacct ggacacgctg 60

gaaagctggc cggaacaggt caagaccatg cagcgtaact ggattggtcg ttcggaaggg 120

gtggagatca cctttgacgt cgccgacagc agtgaaaaac tgaccgttta cactacccgt 180

ccggatacct ttatgggggc cacttacctg gctgtggctg cgggtcatcc gctggccgct 240

caggctgccg ccagccaacc ggcgctggct gattttatcg ctgaatgccg gaacaccaaa 300

gttgccgaag cagatatggc caccatggag aaaaaaggca tggcgaccgg ggtgtttgct 360

gttcatccac tgagcggtga gaaactgccg gtatgggtgg caaactttgt gctgatggaa 420

tacggcaccg gggctgttat ggcggttcca ggccatgacc agcgtgactg ggaatttgcc 480

accaaataca gcctgccggt ggtcccggtt atcctgaatg ccgatggcag cgaacctgac 540

ctgcgtgacg ccgcgatgac cgaaaaggga acgctgttca actccggcga gtttgacggt 600

ttgtctcacg aacagggctt taacgccatt gccgataggc tg 642

<210> 563
<211> 642
<212> DNA
<213> Erwinia papayae

<400> 563
cagtggttta tcaaaatcac agactatgcc gatgagctgc tcaacgacct ggatacactg 60

gaaagctggc cggaacaggt caagaccatg cagcgtaact ggattggtcg ttcggaaggg 120

gtggagatca cctttgacgt cgccgacagc agtgaaaaac tgaccgttta cactacccgt 180

ccggatacct ttatgggggc cacttacctg gctgtggctg cgggtcatcc gctggccgct 240

caggctgccg ccagccaacc ggcgctggct gattttatcg ctgaatgccg gaacaccaaa 300

gttgccgaag cagatatggc cactatggag aaaaaaggca tggcgaccgg ggtgtttgct 360

gttcatccac tgagcggtga gaaactgccg gtctgggtgg cgaactttgt gctgatggaa 420

tacggcaccg gggctgttat ggcggttcca ggccatgacc agcgtgactg ggaatttgcc 480

accaaataca gcctgccagt ggtcccggtt atcctgaatg ccgatggcag cgaacctgac 540

ctgtgtgacg ccgcgatgac cgaaaaggga acgctgttca actccggcga gtttgacggt 600

ttgtctcacg aacagggttt taacgccatt gccgacaggc tg 642

<210> 564
<211> 642
<212> DNA
<213> Erwinia persicina

<400> 564
cagtggttcg tcaaaattac cgactatgcc gatgaactgc tgaatgacct cgacacgctg 60

gaaagctggc ctgagcaggt gaaaaccatg cagcgcaact ggattggccg ctctgaaggg 120

gtggagatcg agtttgacgt gaacaacagt caggaaaagc tgcgtgttta taccacgcgt 180

cctgacacct ttatgggcgt gacctacctg gcggtagcgg cgggacaccc gctggcagcc 240

caggccgccg ccactcatcc agccctggcc gactttatcg ccgaatgccg taataccaaa 300

gtggctgaag ccgaaatggc cacgatggag aaaaaaggca tggccaccgg cctgtttgcg 360

aaacacccgc tgaccggtga agacgtcccg gtttgggtcg ccaactttgt gctgatggaa 420

tacggcaccg gtgccgttat ggctgttcct ggacatgacc agcgtgactg ggaatttgcg 480

accaaatacg gtctgcagat caaacccgtg atccttgccg ctgacggcag cgcaccggat 540

ctgagcgaag cggcaatgac cgaaaaaggc acgctgttta attccggcga gtttgacggt 600

ctgtcctatg aagacggttt caatgcgatt gccactaagc tg 642

<210> 565
<211> 642
<212> DNA
<213> Erwinia psidii

<400> 565

cagtggttta tcaaaatcac tgactatgct gatgagctgt taagcgatct ggacacgctg 60

gaaagctggc cggaacaggt caagaccatg cagcgtaact ggataggccg ttctgaaggt 120

gttgagatca cctttgacgt cgctgacagt gatgaaaagc tgaccgtcta caccacccgc 180

ccggatacct ttatgggagc cacctacctg gccgtagctg cggggcaccc actggccgca 240

cacgctgcgg tcagtcaacc cgcgctggcc gattttatcg ctgaatgccg taacaccaaa 300

gtggctgaag cggaaatggc caccatggag aaaaaaggca tggcaaccgg gacgtttgcg 360

attcatccac tgagtggtga aaagctgccg gtgtgggtcg ctaactttgt gctgatggag 420

tatggcaccg gcgcggtaat ggcggttccg ggccacgacc agcgtgactg ggagtttgcc 480

actaaatatg gcctgcctgt aatccctgtc attctgaatg ccgatggcag caaacccgat 540

gtgcgcgaat cagcgatgac cgagaaaggc acgctgttta actccggcga gtttgacggc 600

atgtcatatg aagatggctt taatgcgatc gccaacaggc tg 642

<210> 566
<211> 642
<212> DNA
<213> Erwinia rhapontici

<400> 566
cagtggtttg taaaaatcac ggattatgct gacgaactgc tgaatgacct cgacacgctg 60

gaaagctggc ctgaacaggt taaaaccatg cagcgtaact ggattggccg ttctgaaggc 120

gtggaaatcg agtttaatgt cagcaacagc ccggaaaagc tgcgcgtata taccacacgt 180

cctgacacgt tcatgggtgt gacttacctc gctgtggctg caggccatcc tctggctgca 240

caagcagcag caagcaaccc tgcgctggct gactttatcg ctgaatgccg taacaccaaa 300

gttgctgaag cagaaatggc aaccatggag aaaaaaggca tggccaccgg cctgtacgca 360

caacacccgc tgacgggtga ggacgtgccg gtatgggtcg ctaacttcgt gctgatggaa 420

tacggcacag gtgctgtgat ggccgttcct ggccacgacc agcgcgactg ggagttcgcc 480

actaaatacg acctgcagat caaaccggtt atcctggctg cagacggcag cgcaccggat 540

ctgagcgaag cggcaatgac cgaaaaaggc acgctgttta actccggtga gtttgacggt 600

ttgtcttatg aagaaggctt caacgccatc gcgacgaaac tg 642

<210> 567
<211> 642
<212> DNA
<213> Erwinia toletana

<400> 567
cagtggtttg ttaaaatcac tgactacgct gatgaactgc tgaacgacct cgacaagctg 60

gaagactggc cagagcaggt caaaaccatg cagcgtaact ggattggccg ttctgaaggg 120

gtggagatca cctttaatgt ggtcgacagc gaagagaagc tgaccgttta taccacgcgt 180

ccggacacct ttatcggcgc cacctatctg gcggtcgcgg cgggtcaccc actggcggcg 240

caggcggcgg ccagcaaccc ggcgctggca gactttatag cagaatgccg taataccaaa 300

gttgccgaag ccgagatggc gaccatggag aaaaagggca tggccaccgg cctgttcgcg 360

ctccatccgc tgactggcga aaaagtgccg gtatgggtcg ctaactttgt gctgatggaa 420

tacggtactg gcgcagtgat ggcggtaccg gcgcacgacc agcgcgactg ggagttcgcc 480

accaaatatc acctgacgat taaaccggtg atcctcaatc tggatggcag cgagccggac 540

gtcagcttag cggcgatgac cgacaaaggt acgctgttta actccggtga gtttagcggc 600

ctgtcgcacg aagaaggctt taacgcgatt gccagccagc tg 642

<210> 568
<211> 642
<212> DNA
<213> Erwinia tracheiphila

<400> 568
cagtggttta tcaaaatcac cgcctatgcc gatgagttgc tgaacgattt ggatacgctg 60

gaaaactggc cagaacaggt caagaccatg cagcgtaact ggataggccg ttctgagggt 120

gtagaaatca cctttaacat agctgacagc catgaaacgc tgaccgttta taccactcgc 180

ccggatacct ttatgggggc aacttacctg gcggtcgcag caggacatca tctggccgct 240

caggcgtctg ccagcaaccc tgcgctggca gactttatcg ctgaatgtcg taataccaag 300

gtggctgaag cggacatggc caccatggag aaaaaaggcg tggcgacagg cgtgtttgcc 360

gttcatccac tgagcggtga aaagctgccg gtatgggttg ctaactttgt actgatggaa 420

tacggcaccg gtgctgttat ggccgtcccc ggccacgatc agcgcgactg ggagttcgcc 480

acaaaatatg cattgcccgt cgtccctgtc attcttaatg ccgatggcag tgaaccagac 540

ctgcgtgaat cagccatgac cgaaaaaggg acgctgttca actcaggtga attcgacggc 600

ctgtcttatg aggatggttt taatgcgatt gctagcaaac tg 642

<210> 569
<211> 642
<212> DNA
<213> Escherichia albertii

<400> 569
cagtggttta tcaaaatcac cgcttacgct gacgagctgc tcaacgatct ggataaactg 60

gatcactggc cagacaccgt aaaaaccatg cagcgtaact ggattggtcg ttccgaaggt 120

gtggaaatca ctttcaacgt taacgactat gacaacacgc tgaccgttta tacgacccgc 180

ccggacacct ttatgggctg tacttatctg gcggtagcgg caggtcaccc actggcgcag 240

aaagcggcgg aaaataatcc tgaactggcg gcctttattg acgaatgccg taacaccaaa 300

gttgccgaag cagaaatggc gacgatggag aaaaaaggcg tcgatacggg ctttaaagct 360

gttcacccat taaccggcga agaaattccg gtttgggcag caaacttcgt attgatggaa 420

tatggcaccg gcgcagtcat ggcggttccg ggtcacgacc agcgtgacta cgaatttgcc 480

tctaaatacg gcctgaacat caaaccggtt atcctagcgg ctgacggttc tgaaccagat 540

ctctcccagc aggcactgac tgaaaaaggc gtgctgttca actctggcga gttcaacggt 600

cttgaccatg aggcggcctt taacgccatt gccgacaaac tg 642

<210> 570
<211> 642
<212> DNA
<213> Escherichia blattae

<400> 570
cagtggttta ttaaaatcac cgactacgca gaagagctgc tcaacgacct ggataagctg 60

gatcactggc cggatcaggt taagaccatg cagcgtaact ggattggccg ttctgaaggg 120

gtagaaatta cctttgacgt aaaagacagc gaccagaaac tcaccgtcta caccacccgc 180

ccggacacct ttatgggggc cacttatctg gccgtggccg ccggtcaccc gctggcgcaa 240

caggctgccg ccggtaaccc gcagctggcc gccttcgttg aagagtgccg caataccaaa 300

gtggccgaag ccgacatggc aaccatggag aaaaaaggga ttgccaccgg ctattacgcc 360

atccacccgc tgactggtga agccatcccg gtgtgggcgg caaacttcgt gctgatggag 420

tacggcaccg gtgccgttat ggccgtacct ggtcacgacc agcgcgacta cgaatttgcc 480

accaaatatc atctgccggt taaagctgtc attctggcgg cagacggcac cgcgccggat 540

ctctccagcc aggccatgac cgagaaaggc gtgctgttca actcaggtga gtttgacggt 600

ctggacttta ccgctggttt taacgccatt gccgataagc tg 642

<210> 571
<211> 642
<212> DNA
<213> Escherichia coli T

<400> 571
cagtggttta tcaaaatcac tgcttacgct gacgagctgc tcaacgatct ggataaactg 60

gatcactggc cagacaccgt taaaacaatg cagcgtaact ggatcggtcg ttccgaaggc 120

gtggaaatca ccttcaacgt taaggactat gacaacacgc tgaccgttta cactacccgc 180

ccggacacct ttatgggttg tacctacctg gcggtggctg cgggtcatcc gctggcacag 240

aaagcggcgg aaaataatcc tgaactggcc gcctttattg acgaatgccg taacaccaaa 300

gttgccgaag ctgaaatggc gacgatggag aaaaaaggcg tcgatactgg ctttaaagcg 360

gttcacccat taacgggcga agaaattccc gtttgggcag caaacttcgt attgatggaa 420

tacggcacgg gcgcagttat ggcggttccg ggtcacgacc agcgcgacta cgagtttgcc 480

tctaaatacg gcctgaacat caaaccggtt atcctggcag ctgacggctc tgagccagat 540

ctttctcagc aagccctgac tgaaaaaggc gtgctgttca actctggcga gtttaatggt 600

cttgaccatg aagcggcctt caacgccatt gccgacaaac tg 642

<210> 572
<211> 642
<212> DNA
<213> Escherichia fergusonii

<400> 572
cagtggttta tcaaaatcac cgcttacgct gacgagctgc tcaacgatct ggataaactg 60

gatcactggc cagacaccgt taaaaccatg cagcgtaact ggatcggtcg ttccgaaggc 120

gtggaaatca ccttcaacgt taacgactat gacaacacgc tgaccgttta cactacccgc 180

ccggacacct ttatgggttg tacctacctg gcggtagcgg caggtcatcc gctggcacaa 240

aaagcggcgg aaaataatcc tgaactggcg gcctttattg acgaatgccg taacaccaaa 300

gttgcagaag ctgaaatggc aacgatggag aaaaaaggcg tcgatactgg ctttaaagcg 360

gttcatccat taacgggcga agaaattccg gtttgggcag caaacttcgt attgatggaa 420

tacggcaccg gcgcagtcat ggcggttccg ggtcacgacc agcgcgacta cgagtttgcc 480

tctaaatacg gcctcaacat caagccggtt atcctggcag ctgacggctc tgagccagat 540

ctttctcagc aagccctgac cgaaaaaggc gtgctgttca actctggcga gttcaacggt 600

cttgatcatg aagcagcctt caacgccatc gccgataaac tg 642

<210> 573
<211> 642
<212> DNA
<213> Escherichia hermannii

<400> 573
cagtggttta tcaaaatcac cgcttatgcc gacgaactgc tgaatgattt ggataagctg 60

gatcagtggc cagacaccgt caaaaccatg cagcgtaact ggattggccg ttctgaaggc 120

gtggaaatca ccttcgacgt ggaaaaccgt gccgataaac tgacggtgta caccacccgc 180

ccggacacct tcatgggcgt cacctatctg gctgtggctg cgggccaccc gctggcgcag 240

gccgctgcag cgactaaccc ggcgctggct gatttcatcg ccgaatgccg taataccaaa 300

gttgccgaag ctgacatggc gacgatggag aaaaaaggca tggcgaccgg cttcaacgcc 360

attcatccgc tgactggcga agccgttccg gtatgggttg ccaacttcgt actgatggaa 420

tacggcactg gcgcagtcat ggccgttccc ggccacgatc agcgcgactg ggaattcgcc 480

accaaatacg gtctggcgat caaacccgtc atcctcgcgc aggacggcac ggcgccggat 540

ctgtcccagc aggcgctgac tgaaaaaggc gtgctgttca actccggaga attcgacggc 600

ctgccgtttg accaggcgtt taacgccatt gccgacaagc tg 642

<210> 574
<211> 642
<212> DNA
<213> Escherichia vulneris

<400> 574
cagtggttta tcaaaatcac cgcctacgct gacgagctgc tgaacgacct ggaccatatg 60

gaccagtggc cggacaccgt taaaaccatg cagcgcaact ggatcggccg ctcggaaggc 120

gttgagatca cctttgacgt cgagaacagc gacgacaagc tgaccgtcta caccacccgt 180

ccagacacct ttatgggcgt cacctacctg gcggttgccg cgggccaccc gctgagcacc 240

caggcggcgg cctataaccc ggcgctgggc gacttcattg ccgaatgccg caacaccaag 300

gttgccgaag cagatatggc gaccatggag aaaaaaggcg tcgacaccgg cgtgcgcgct 360

attcacccgc tgaccggcga agcgatcccg gtctgggcag cgaacttcgt gctgatggag 420

tacggcaccg gcgcagtgat ggccgttccg ggccacgatc agcgcgacta cgagtttgcc 480

accaaatatg gcttagagat caagccggtt atcctggccg ctgacggcac gccgccggat 540

ctcagcgcgc aggcgctgac cgaaaaaggc gtgctgttta actccggcga gtttgacggt 600

ctgggctttg aagccgcgtt taacgccatt gccgacaagc tg 642

<210> 575
<211> 642
<212> DNA
<213> Ewingella americana T

<400> 575
cagtggttca ttaaaatcac ggcctatgcc gaccaactgc ttaacgattt ggatccgctg 60

gaaagctgcc ctgagcaggt caaaaccatg cagcgtaact ggattggccg ctctgaaggc 120

gttgagatca ccttcgacgt cgaaaacagt gaagagaaag ccaccgtgta caccacgcgt 180

cctgacacct tcctgggcgc gacctacgtt gcggttgcag caggtcaccc actctcttta 240

caagctgcgg caactaaccc tgccttggct gatttcatcg acgaatgccg caataccaaa 300

gttgccgaag cagaaatggc gaccatggaa aaacgcggca tggacaccgg cctgtttgct 360

attcacccgc tgactggcaa gaaagtggcc atctgggtcg ctaacttcgt cttgatggaa 420

tacggcactg gcgccgtgat ggcggttccg ggccacgacc agcgtgactg ggagttcgca 480

accaaataca acctgccaat cgtgcccgtt atccttaacg ccgacggcac agagccaaac 540

gtcactgccg aagcgctgac tgaaaaaggc accttgtata actcaggtga gtttgacggt 600

ctgagccacg aagcaggctt caatgccatc gccgataagc tg 642

<210> 576
<211> 642
<212> DNA
<213> Hafnia alvei T

<400> 576
cagtggttca ttaaaatcac cgcttatgca gaccaactgc taaacgatct tgatacgctg 60

gaaaactggc ctgaacaggt caaaaccatg cagcgtaact ggatcggccg ttctgaaggc 120

gtcgaaatta ctttcgacgt cgcagaaagc gatgagaaag tgaccgttta tactacgcgt 180

ccagatacct tcatgggcgc aacttacgtt gccgtcgcgg cgggccatcc tctggctcag 240

cttggcgctg ctaacaatcc tgaactggtt caattcatcg acgaatgccg caataccaaa 300

gttgccgaag cagaaatggc aaccatggag aaaaaaggca tggccaccgg cctgtttgcg 360

atccacccac tgacgggcga aaaactgcca gtttgggtag caaacttcgt tctgatggaa 420

tacggcaccg gcgccgtgat ggccgttcct gctcacgatc agcgtgactg ggagttcgcc 480

accaaatacg gtttgcagat taaacctgtg atcctgaatc tggacggtag cgagcctgac 540

gttcatgcag aagccatgac cgataaaggc gcgctgttca attccggtga atttgacggt 600

ttgagccatg aagctggctt taacgccatc gcagacaagc tg 642

<210> 577
<211> 642
<212> DNA
<213> Klebsiella oxytoca

<400> 577
cagtggttta ttaaaattac cgcctacgct gacgagctgc tgaacgatct ggacaacctg 60

gatcactggc cggataccgt gaagaccatg cagcgtaact ggattggccg ctccgaaggc 120

gtcgaaatct ccttcgatgt taacgactac gccgacaagc tgaccgtcta caccacccgc 180

ccggacacct ttatgggctg tacctatctg gccgtggcgg cgggtcaccc gctggcgcag 240

caggcagcag ccaataaccc cgcgctggcg acgtttatcg acgaatgccg caataccaaa 300

gttgccgaag ccgaaatggc aactatggag aaaaaaggcg tcgataccgg ttataaagcg 360

attcatccgc tgaccggcga agcgatcccg gtctgggcgg caaactttgt cctgatggag 420

tacggcaccg gcgcggtaat ggccgttccg ggtcacgatc agcgcgacta cgagtttgcc 480

agtaaatacg gtctggagat caagccggtc attctggccg ccgacggttc cgaaccggat 540

ctctccgaac aggcgctgac cgaaaaaggc gtgctgttca actctggcga attcgatggt 600

ctggatcacg aagctggctt taacgccatc gccgataagc tg 642

<210> 578
<211> 642
<212> DNA
<213> Klebsiella pneumoniae sbsp. ozaenae

<400> 578
cagtggttta ttaaaatcac cgcctacgcc gatgaactgc tgaacgatct ggataagctg 60

gaccactggc cggacaccgt gaagaccatg cagcgcaact ggatcggccg ttcggaaggg 120

gtagaaatct cctttgacgt caacgactac gctgacaaac tgactgtcta caccacccgt 180

ccggacacct ttatgggttg tacctacctg gccgtcgcgg cgggtcaccc gctggcgcag 240

caggctgcgg cgaacaatcc ggcgctggcc gcctttatcg acgagtgccg caataccaaa 300

gtggccgaag ccgacatggc gacgatggag aaaaaaggcg tcgataccgg ctttaaagcc 360

attcatccgc tgaccggtga acaaattcca gtctgggcgg caaactttgt gctgatggaa 420

tacggcaccg gcgcggtaat ggccgttccg ggtcacgacc agcgcgacta cgagttcgcc 480

agcaagtacg gtctgaacat taagccggtt atcctcgctg ccgacggctc cgagccggat 540

ctctccgagc aggcgctgac cgagaaaggc gtcctgttta actccggcga gttcagcggt 600

cttgattatg aagcgggctt caacgccatt gccgacaaac tg 642

<210> 579
<211> 642
<212> DNA
<213> Klebsiella pneumoniae sbsp. pneumoniae T

<400> 579
cagtggttta ttaaaatcac cgcctacgcc gatgaactgc tgaacgatct ggataagctg 60

gaccactggc cggacaccgt gaagaccatg cagcgcaact ggatcggccg ttcggaaggg 120

gtagaaatct cctttgacgt caacgactac gctgacaaac tgactgtcta caccacccgt 180

ccggacacct ttatgggttg tacctacctg gccgtcgcgg cgggtcaccc gctggcgcag 240

caggctacgg cgaacaatcc ggcgctggcc gcctttatcg acgagtgccg caataccaaa 300

gtggccgaag ccgacatggc gacgatggag aaaaaaggcg tcgataccgg ctttaaagcc 360

attcatccgc tgaccggtga agaaattcca gtctgggcgg caaactttgt gctgatggaa 420

tacggcaccg gcgcggtaat ggccgttccg ggtcacgacc agcgcgacta cgagttcgcc 480

agcaagtacg gtctgaacat taagccggtt atcctcgctg ccgacggctc cgagccggat 540

ctctccgagc aggcgctgac cgagaaaggc gtcctgttta actccggcga gttcagcggt 600

cttgattatg aagcgggctt caacgccatt gccgacaaac tg 642

<210> 580
<211> 642
<212> DNA
<213> Klebsiella pneumoniae sbsp. rhinoscleromatis

<400> 580
cagtggttta ttaaaatcac cgcctacgcc gatgaactgc tgaacgatct ggataagctg 60

gaccactggc cggacaccgt gaagaccatg cagcgcaact ggatcggccg ttcggaaggg 120

gtagaaatct cctttgacgt caacgactac gctgacaaac tgactgtcta caccacccgt 180

ccggacacct ttatgggttg tacctacctg gccgtcgcgg cgggtcaccc gctggcgcag 240

caggctgcgg cgaacaatcc ggcgctggcc gcctttatcg acgagtgccg caataccaaa 300

gtggccgaag ccgacatggc gacgatggag aaaaaaggcg tcgataccgg ctttaaagcc 360

attcatccgc tgaccggtga agaaattccg gtctgggcgg caaactttgt gctgatggaa 420

tacggcaccg gcgcggtaat ggccgttccg ggtcacgacc agcgcgacta cgagttcgcc 480

agcaagtacg gtctgaacat taagccggtt atcctcgctg ccgacggctc cgagccggat 540

ctctccgagc aggcgctgac cgagaaaggt gtcctgttta actccggcga gttcagcggt 600

cttgattatg aagcgggctt caacgccatt gccgacaaac tg 642

<210> 581
<211> 642
<212> DNA
<213> Klebsiella singaporensis

<400> 581
cagtggttta ttaaaatcac cgcctacgcc gatgaactgc tgaacgatct ggataaactg 60

gatcactggc cagacaccgt aaagaccatg cagcgcaact ggatcggccg ttccgaaggg 120

gtggaaatct cctttgacgt caacaactac gccgataagc tgaccgtcta caccacccgt 180

ccggacacct ttatgggctg cacctacctg gccgtcgcgg cgggtcaccc gctggcgcag 240

caggctgcag cgaacaaccc ggcgctggct gcctttatcg acgaatgccg taataccaaa 300

gtggccgaag ccgacatggc gacgatggag aaaaaaggcg tcgataccgg ctttaaagcc 360

attcacccgc tgaccggcga agaaattccc gtctgggcgg caaacttcgt gctgatggaa 420

tacggcaccg gcgcggtcat ggccgttccg ggtcacgacc agcgtgacta tgagttcgcc 480

agcaagtacg gtctgaacat caagccggtt atcctcgccg ctgacggcag cgagccggat 540

ctctccgcgc aggcgctgac cgagaaaggc gtcctgttta actccggcga gttcagcggt 600

ctcgattatg aagccggctt caacgccatt gccgacaaac tg 642

<210> 582
<211> 642
<212> DNA
<213> Klebsiella variicola

<400> 582
cagtggttta ttaaaatcac cgcctacgcc gatgaactgc tgaacgatct ggataaactg 60

gatcactggc cagacaccgt aaagaccatg cagcgcaact ggatcggccg ttccgaaggg 120

gtggaaatct cctttgacgt caacaactac gccgataagc tgaccgtcta caccacccgt 180

ccggacacct ttatgggctg cacctacctg gccgtcgcgg cgggtcaccc gctggcgcag 240

caggctgcag cgaacaatcc ggcgctggct gcctttatcg acgaatgccg taataccaaa 300

gtagccgaag ccgacatggc gacgatggag aaaaaaggcg tcgataccgg gtttaaagcc 360

attcacccgc tgaccggcga agaaattcct gtctgggcgg caaacttcgt gctgatggaa 420

tatggcaccg gcgcggtcat ggccgttccg ggtcacgacc agcgtgacta tgagttcgcc 480

agcaagtacg gtctgaacat caagccggtt atcctcgccg ctgacggcag cgagccggat 540

ctctccgcgc aggcgctgac cgagaaaggc gtcctgttta actccggcga gttcagcggc 600

ctcgattatg aagccggctt caacgccatt gccgacaaac tg 642

<210> 583
<211> 642
<212> DNA
<213> Kluyvera ascorbata T

<400> 583
cagtggttta tcaaaatcac cgcttacgcc gacgaactgc tgagcgatct ggacaatctg 60

gatcactggc ctgacaccgt gaaaacgatg cagcgtaact ggattggccg ttccgaaggg 120

gttgagatct cctttgacgt taacgactac gccgacaagt tgaccgttta caccacccgt 180

ccagatacct tcatgggttg cacctacctg gccgttgctg caggccaccc gttggcacag 240

caggctgctg cgaacaaccc agccctggct gcttttatcg acgaatgccg caacaccaaa 300

gtggctgaag ccgacatggc gaccatggag aaaaaaggcg tcgacaccgg ctttaaagcg 360

attcacccac tgactggcga agccattcca gtctgggctg caaacttcgt gctgatggag 420

tacggtactg gcgcagtgat ggccgttcct ggtcacgatc agcgcgacta tgaatttgca 480

agcaaatacg gtctgaacat taagccggtt attcttgctg ccgacggcag ccagcccgat 540

ctctccgagc aggcgctgac tgaaaaaggc actctgttta actccggcga attcgacggc 600

ctgagctacg aagacggctt caatgcgatt gctgacaaac tg 642

<210> 584
<211> 642
<212> DNA
<213> Kluyvera cochleae

<400> 584
cagtggttta tcaaaatcac cgcctacgct gacgaactgt taaacgatct ggataacctg 60

gaccactggc ctgacaccgt gaaaacgatg cagcgcaact ggatcggccg ttctgaaggt 120

gtggaaatca ctttcgacgt gaaaggttat gacaacacgc tgaccgttta caccacccgc 180

cctgacacct ttatgggtgc cacatacctg gcggttgcag ccggtcaccc tctggcgcag 240

aaagccgcgg cgaacaacgc ggaactggct gctttcatcg acgaatgccg taacaccaaa 300

atggccgaag ccgaaatggc gaccatggag aaaaaaggcg tcgatactgg ctttaaagcg 360

gttcacccac tgactggcga agaaattcca gtctgggcag caaacttcgt gctgatggaa 420

tacggcaccg gtgccgtgat ggccgttccg ggccacgatc agcgcgacta cgagtttgcg 480

accaaataca gcctgaacat caaaccagtt atcctcgccg ctgacggctc cgccccggat 540

ctttgtgaac aggcgctgac cgaaaaaggc gtactgttta actctggtga atttgatgga 600

ctgagcttcg aagcaggctt taacgctatt gccgacaaac tg 642

<210> 585
<211> 642
<212> DNA
<213> Kluyvera cryocrescens

<400> 585
cagtggttca tcaaaatcac cgcttacgca gacgaactac tgcgtgacct ggataatttg 60

gatcactggc ctgacaccgt gaaaacgatg cagcgtaact ggattggccg ttccgaaggc 120

gtggaaatct ccttcaacgt aaacgactac gccgacaagc tgaccgtcta caccactcgt 180

ccagacacct ttatgggttg tacttacctg gccgttgctg cgggtcaccc gctggcacag 240

caggctgctg ccaataaccc agagctggcg gcctttatcg atgaatgccg taatactaaa 300

gtggccgaag ccgatatggc gaccatggag aaaaaaggca tcgataccgg ctttaaagcg 360

attcatccgc tgaccggcga agcaattcct gtttgggcgg ctaacttcgt actgatggaa 420

tatggcacag gcgcagtcat ggccgttcca ggtcacgatc agcgcgacta cgaatttgcc 480

accaaatacg gtctgaacat caagccggtt atcctggccg ctgacggttc tgaacctgac 540

ctgtccgagc aggcgctgac cgaaaaaggc accctgttta actccggcga attcgacggt 600

ctgagctatg aagctggctt taatgccatc gctgacaaac tg 642

<210> 586
<211> 642
<212> DNA
<213> Kluyvera georgiana

<400> 586
cagtggttta tcaaaatcac cgcctacgct gacgagctgc tgaacgatct ggataacctg 60

gatcactggc ctgacaccgt gaaaacgatg cagcgtaact ggatcggccg ctctgaaggc 120

gtggaaatca ccttcgacgt gaccggttac gacaacacgc tgaccgtcta caccacccgt 180

ccggacacct tcatgggtgc aacctacctg gcggtggctg caggtcatcc gctggcgcag 240

aaagcggcag agaataaccc aacgctggcg gctttcatcg acgaatgccg taataccaaa 300

gtggccgaag ccgatatggc gaccatggag aaaaaaggcg tcgacaccgg ctttaaagcg 360

gttcatccgc tgactggtga agagatcccg gtttgggcgg ctaacttcgt gctgatggaa 420

tacggtaccg gcgcggtcat ggcggttccg ggtcacgatc agcgcgatta cgaattcgcg 480

accaaatacg gtctgaccat caaaccggtt attctggccg ccgacggcac cgcgccggat 540

ctctcagagc aggcgctgac cgagaaaggc gtgttgttta actccggcga attcgacggt 600

ctggactttg aagcgggctt caacgccatt gctgacaagc tg 642

<210> 587
<211> 642
<212> DNA
<213> Kluyvera intermedia

<400> 587
cagtggttta tcaaaatcac cgcttacgct gacgaactgt taaacgatct ggataatctc 60

gatcactggc ctgacaccgt gaaaacgatg cagcgcaact ggatcggccg ttctgaaggt 120

gtcgaaatca cctttgacgt gaaaggctac gacaacacgc tgaccgttta cactacccgc 180

ccggacacct ttatgggtgc cacttatctg gcggttgctg ccggtcaccc tctggcgcaa 240

aaagccgccg cggacaacgc ggaactggct gctttcatcg acgaatgccg taacaccaaa 300

atggccgaag ccgaaatggc gaccatggag aaaaaaggcg tcgatactgg ctttaaagcg 360

gttcacccac tgactggcga agaaattcca gtctgggcag caaacttcgt gctgatggaa 420

tatggcaccg gtgccgtgat ggccgttccg ggccacgatc agcgcgacta cgagttcgcg 480

accaaataca gcctgaacat caaaccagtt atcctcgccg ctgacggttc cgccccggat 540

ctttgtgaac aggcgctgac cgaaaaaggc gtactattta actctggtga atttgatgga 600

ctgagcttcg aagcaggctt taacgctatt gccgacaaac tg 642

<210> 588
<211> 642
<212> DNA
<213> Leclercia adecarboxylata T

<400> 588
cagtggttta tcaaaatcac cgcttacgcc gacgaactgc tgagcgatct ggataatctg 60

gatcactggc cagataccgt taaaaccatg cagcgcaact ggatcggccg ttctgaaggg 120

gtggaaatca ccttcaacgt ggaaaattac gatcagaagc tgaccgttta caccacccgc 180

ccggacacct tcatgggtgc cacctacctg gccgtggcgg caggtcaccc gctggcgcag 240

aaagcggcag agaacaatcc agaactggct gcctttatcg acgaatgccg caacaccaaa 300

gtggccgaag ccgacatggc gaccatggag aagaaaggcg ttgctaccgg tttctacgcc 360

acgcacccgc tgactggcga agcgatccct gtctgggcag ccaacttcgt gctgatggag 420

tacggtacgg gcgcagtcat ggccgttccg ggtcacgacc agcgcgacta cgaatttgcc 480

actaaatacc acctggcgat caagccagtt atcctcgcgg cagacggctc tgagccggac 540

ctgtccgaac aggcgctgac cgaaaaaggc accctgttca actctggcga attcagcggc 600

ctgagcttcg aagagggctt caacgccatt gccgataaac tg 642

<210> 589
<211> 642
<212> DNA
<213> Leminorella grimontii T

<400> 589
cagtggttta tcaaaatcac cgcctacgcc gaacagctgc ttaacgatct ggacacgctt 60

gaaagctggc cagagcaggt taagaccatg cagcgcaact ggattggccg ctctgaaggg 120

gttgaaatta cctttgccgt tgacggcagc gatgatgcgc tgtccgttta caccacgcgc 180

cccgatacct ttatgggcgt ctcctacgtc gctatcgccg ccggccaccc gctggccgct 240

caggcggcca aaaacagccc cgagctgagc cgctttatcg acgaatgccg caacactaaa 300

gttgccgaag ccgacatggc gaccatggag aaaaaaggcg tcgcgaccgg cctgtacgcg 360

atccacccgc tcaatggcga aaagctgcct gtttgggtcg ccaactttgt cttgatggac 420

tacggcaccg gcgcggtgat ggcagtcccc ggccacgacc agcgcgactg ggaatttgcc 480

actaaatata gtttgcccat caagccggtt attctggccg ccgacggtac tccgccggat 540

ctgtcagaaa gcgccaatac ggaaaaaggc gtgctgttca actccggcga attcgacgga 600

ctggactttg acgcagcgtt caacgccatt gccgacaagc tg 642

<210> 590
<211> 642
<212> DNA
<213> Leminorella richardii

<400> 590
cagtggttta ttaaaatcac cgcctatgcc gaccagctgc ttaacgatct ggatacgctg 60

gagagctggc ctgagcaggt taagaccatg cagcgcaact ggattggccg ctctgaaggg 120

gttgaaatta cctttgctgt cgacggcagc gatgacaccc tgtccatcta taccacccgc 180

ccggatacct ttatgggggt ctcttacgta gcgatcgccg cgggtcaccc tcttgccgct 240

cgcgcagcgc agaacaaccc aacgctgagc agctttatcg acgagtgccg caataccaaa 300

gttgccgaag ccgacatggc gaccatggag aaaaaaggcg ttgccactgg cctgttcgct 360

gttcacccgc tgagcggcga gaaactgccg gtctgggtcg ccaactttgt tctgatggac 420

tacggcaccg gtgccgtcat ggcggtacct ggtcacgacc agcgcgactg ggagtttgcc 480

accaaataca gtctgccgct taagccggtt atcttgaccg ctgacggcgc agctcccgat 540

ctttccgaga gcgccagcac cgaaaaaggc gtgctgttta attctggtga attcgacggc 600

ctcgactttg atgctgcctt taacgccatt gttgaacgtc tg 642

<210> 591
<211> 642
<212> DNA
<213> Moellerella wisconsensis T

<400> 591
caatggttta ttaaaattac cgattatgcg gaagaactgc tgaatgattt agataagcta 60

gatgaatggc ctgaacaggt taaaaccatg cagcgtaact ggatcggtcg ttctgaaggg 120

gtagaaatca ccttcaatgt agctgaccgc gatgacaagt taacggttta taccactcgc 180

ccagatacct ttatgggagc gacttatgtt gccgtagctg ctgggcatcc gttagctcaa 240

gaagcggcag cgaataatcc tcaagtagct ggttttattg aagaatgtcg tgcgactaaa 300

atggcagaag ctgacatggc gaccatggag aaaaaaggtt tagccaccgg cttttatgct 360

gtccatcctt taactcaaga aaaactacct atttgggtag ctaactttgt gctgatggaa 420

tacggcaccg gagcagttat ggctgttcct gcacatgacc aacgtgattg ggaatttgca 480

cataaatatt cattacctat taaagcagta atcgctgatg ccgaaggaaa tcaacctgat 540

atcagcgaag ctgcaatgac ggataaaaac agcctgatta actcgggtga gtttgacggc 600

ttatcaaacg aagccggctt caatgcgatt gcagataaac tg 642

<210> 592
<211> 642
<212> DNA
<213> Morganella morganii sbsp. morganii T

<400> 592
cagtggttta tcaaaatcac cgcttatgcc gaagaactcc tcaatgatct ggatacgctg 60

gaagactggc cggagcaggt caaaaccatg cagcgcaact ggatcggccg ttccgaaggt 120

gtggaaatca cctttgatgt cgcggacagc accgaaaaac tgagtgtcta caccacccgt 180

ccggatacct ttatgggtgt cacttatctg gcggtagcgg cgggtcatcc gctggcacag 240

caggctgcac agaacaatcc tgaactgaaa acctttatcg atgagtgccg taataccaaa 300

gtggccgaag ctgaaatggc cacgatggag aaaaaaggca tggcaaccgg tatgtttgcc 360

attcacccgc tgaccggtga aaaagtcgcg gtctgggtcg ccaactttgt cctgatggaa 420

tacggtaccg gcgccgttat ggccgttccg gcgcacgatc agcgcgactg ggaattcgcc 480

accaaatacg gtttaccaat caagccggtc atcttgggcg aagacggtaa cgcaccggat 540

atcagtgaag cagcaatgac cggcaaaaac accctgttca attcaggtga gttcgatggt 600

ctggattaca ccgccggttt taacgcaatt gcggacaaac tg 642

<210> 593
<211> 642
<212> DNA
<213> Morganella morganii sbsp. sibonii

<400> 593
cagtggttta tcaaaatcac cgcttatgcc gaagagctgc tcaatgacct cgatacgctg 60

gaagactggc cggagcaggt caaaaccatg cagcgcaact ggatcggccg ttccgagggt 120

gtggaaatca cctttgatgt cgcggacagc accgaaaaac tgagtgtcta caccacccgc 180

ccggatacct ttatgggtgt cacttatctg gcggtggcag caggacatcc gctggcacag 240

caggctgcac agaacaatcc tgagctgaaa acctttatcg acgagtgccg taataccaaa 300

gtggcagagg ctgaaatggc cacgatggag aaaaaaggcg tggcaaccgg gatgtttgct 360

attcacccgc tgaccggcga aaaaatccag gtctgggctg ccaacttcgt tctgatggaa 420

tacggcaccg gtgccgtgat ggcggttccg gcacacgacc agcgtgactg ggaatttgcc 480

accaaatacg atttaccgat caaacctgtc attctgaatg aagacggttc tgagccggat 540

atcagtgaag ccgcaatgac cggcaaaaac accctgttca actccggtga atttgatggc 600

ctcgattata ccaccggttt caatgccatt gccgacaaac tg 642

<210> 594
<211> 642
<212> DNA
<213> Obesumbacterium proteus T

<400> 594
cagtggttca ttaaaatcac cgcttatgca gaccaactgc taaacgatct ggatacgctg 60

gaaaactggc ctgagcaggt caaaaccatg cagcgtaact ggattggccg ttctgaaggc 120

gttgaaatta ctttcgacgt cgcagacagc gatgagaaag tgaccgttta tacaacgcgt 180

ccagatacct tcatgggtgc aacttacgtt gccgtcgctg cgggtcatcc tctggctcag 240

cttggtgcgg ttaacaaccc tgaactggtt caattcatcg acgaatgccg taataccaaa 300

gttgccgaag cagaaatggc gaccatggag aaaaaaggca tggccaccgg cctgtttgcg 360

attcacccac tgacgggcga aaaactgcct gtttgggtag caaacttcgt tctgatggaa 420

tacggcaccg gtgccgtaat ggccgttcct gctcacgatc agcgtgactg ggagttcgcc 480

accaaatacg gtttacagat taagcctgtt atcctgaatc tggacggtag cgagcctgac 540

gttcaggcag aagctatgac cgataaaggc gcgctgttca attccggtga atttgacggc 600

ctgagccacg aagctggctt taacgccatc gctgacaaac tg 642

<210> 595
<211> 642
<212> DNA
<213> Pantoea agglomerans T

<400> 595
cagtggttta tcaaaatcac cgattatgcg gaagagctgc tgaacgacct ggatacgctg 60

gaagagtggc ctgagcaggt caaaaccatg cagcgtaact ggattggccg ctctgaaggt 120

gtcgaaatca ccttcgatgt ggctgacagc gaagagaagg tcactgtcta caccacccgc 180

ccggatacct ttatcggcgc aacctatgtt gccgttgccg caggccatcc gctggcgacc 240

caggcgtcgg tcaataatcc ggcgctggct gactttatcg ctgaatgtcg caataccaaa 300

gtggctgaag ccgatatggc gaccatggag aagaaaggca tggcgaccgg cctgtctgtt 360

gttcatccgc tgaccggcga gcttatcccg gtctgggttg ccaacttcgt actgatggaa 420

tatggcaccg gcgctgtgat ggcggttccg gcgcacgatc agcgcgactg ggagtttgcc 480

actaaatatg atctgccgat taagccggtc atcctgaatc tggatggttc cattcctgat 540

gtcagcattg cggcgatgac tgacaaaggt gcgctgttca actcaggtga gtttaacggc 600

atggatcacg ccaccggttt taacgccatc gcggattcgc tg 642

<210> 596
<211> 642
<212> DNA
<213> Pantoea ananatis

<400> 596
cagtggttta tcaaaatcac cgattacgcc gaagagctgt taaacgatct ggatacgctg 60

gaagagtggc ctgagcaggt caaaaccatg cagcgtaact ggatcggccg ctctgaaggc 120

gtggaaatca cctttgacgt ggcggacagc cttgagaaag tcacggttta taccacgcgt 180

ccagatacct tctacggcgc gacctatgtc gccgtggccg ccggtcatcc gctggcgctg 240

caggcggctg ccagcaatcc tgccctcgct gactttattg ccgaatgccg caataccaaa 300

gtggccgaag ccgatatggc caccatggaa aagaaaggca tggccactgg cctgtctgcg 360

gtacacccgt taacaggtga ggctgttcct gtctgggtcg cgaactttgt gctgatggag 420

tacggcacgg gtgccgtgat ggcggttccg gcacatgacc agcgtgactg ggagtttgcc 480

acgaaatacg atctgccgat caagccggtg atcctgaacc tggacggcag cgagccggac 540

gtaagcactg gcgcgatgac cgataaaggt acgctgttta actccgccga gtgcagcggt 600

ctggatcaca gtgccggttt caacgccatt gcggatgcgc tg 642

<210> 597
<211> 642
<212> DNA
<213> Pantoea citrea

<400> 597
caatggttta ttaaaattac tgcctatgca gaagagctgt tgaacgacct ggattctctg 60

gaaggttggc ctgaacaggt taaaaccatg cagcgtaact ggattggtcg ctctgaaggg 120

gttgaaatta cctttgatgt cgccgacagc agcgaaaaac tgaccgttta tacaacccgt 180

ccggacacct ttatgggggc cacttatgtt gcagtagccg ccgggcatcc gctggcacaa 240

caagccgcag tgaataaccc tgcgctgaca gcatttattg atgagtgccg gaataccaaa 300

gttgccgaag ctgatatggc aacgatggag aaaaaaggcc accctacagg gacctttgtg 360

attcacccac tgacgggtga aaaattaccg gtttgggtag ctaattttgt actgatggaa 420

tacggcactg gtgcggtaat ggccgttccg ggacatgatc agcgtgactg ggaatttgcc 480

cgtaagtatg acctgccgat caagcctgta atcctcaatg aagatggcag tgagccggat 540

atttcagaag cagcaatgac cagtaaagga acactgttta attctgcaca gtttgatgga 600

ctctcttatc aggacggttt taatgccatt gctgatgctc tg 642

<210> 598

<211>  642
<212>  DNA
<213>  Pantoea dispersa

<400>  598
cagtggttcg tgaaaatcac cgattacgcc gaagagctgc tcaacgacct cgataagctg      60

gaagactggc cggagcaggt caagaccatg cagcgcaact ggattggccg ttctgaaggt     120

gtcgagatca cctttgacgt ggccaacagc gcggaaaaag tcacggttta caccacccgt     180

ccggacacct tcatgggggc aacctatgtc gccgtggcag ccggtcatcc tctggcacgc     240

caggctgccg tcaacaaccc ggcgctggct gacttcattg ctgaatgcag caacaccaaa     300

gtcgccgaag cggacatggc cacgatggag aagaaaggca tggccaccgg cctgcacgct     360

atccatccgc tgacgggcga ggagattccg gtctgggtcg ctaacttcgt gctgatggag     420

tacggcaccg gtgccgtgat ggccgtaccg ggtcatgacc agcgcgactg ggaattcgcc     480

accaaatatg atctcaacat caagccggtg gtactcaacc tcgacggtac cgcgccggat     540

atcagcgccg cagcgatgac cgaaaaaggc acgctgttca actccggcga gtttgacggt     600

ctggaccacg acgccggctt caatgccatc gccgatgcgc tg                        642

<210>  599
<211>  642
<212>  DNA
<213>  Pantoea punctata

<400>  599
cagtggttta tcaaaattac tgactatgcg gaagagttac tgaatgacct ggatacgctg      60

gaaggctggc ctgagcaggt taaaaccatg cagcgtaact ggattggccg ctcggaaggg     120

gtggaaatca cctttaatgt ggccgacagc gaccagaaac tgacggtcta caccacccgc     180

ccggacactt ttatgggggc gacctatgtc gctgttgccg ccggacatcc gctggcacgc     240

caggcggcca tcggtaaccc gcaactgagc gcgtttatcg acgaatgcag taataccaaa     300

gttgctgaag ctgatatggc aaccatggag aaaaaaggtc agccaacggg catgtttgtt     360

atccatcccc tgaccggtga taaattaccc ctgtgggtgg ctaactttgt cctgatggaa     420

tacggcacag gtgcggttat ggccgttccc ggccatgacc agcgtgactg ggagttcgcc     480

agcaaatatg gtctggccat aaaacccgtg atcctcaatg aggatggcag cgaaccggat     540

atcagtgacg cggcaatgac cggtaaggga acgctgttta attccggacc gttcgatgga     600

ctgtctcatc aggacggttt caacgctatt gctgatgcgc tg                        642

<210>  600
<211>  642
<212>  DNA
<213>  Pantoea stewartii sbsp. indologenes

<400>  600
cagtggttca ttaaaatcac tgattacgcc gaagagctgt taaacgatct ggatacgctg      60

gaagagtggc ctgagcaggt caaaaccatg cagcgtaact ggatcggccg ttctgaaggg     120

gtggaaatca cctttgacgt ggcagacagc agcgaaaaag tgacggttta taccacgcgt     180

ccggacacct tctacggcgc gacctatgtc gctgtcgccg ccggtcatcc gctggcggca 240

caggcggcgg ccagcaaccc tgctctggct gattttatcg cagaatgccg caacaccaag 300

gtagccgaag ccgatatggc caccatggaa aagaaaggca tggcgacagg cctgtctgtc 360

gtgcatccgt taacgggtga acgcctgcct gtctgggtcg cgaacttcgt cctgatggaa 420

tacggtacgg gtgcggtcat ggccgtgccg gcgcatgacc agcgtgactg ggaatttgcg 480

accaaatacg atctgccgat caaaccggtt atcctcaatc tggacggcag cgagcctgac 540

gtcagcgccg ccgcgatgac cgacaaaggt acgctgttta actctgccga gtgcagcggc 600

ctggatcaca gtgccggttt taacgccatt gcagatgcgc tg 642

<210> 601
<211> 642
<212> DNA
<213> Pantoea stewartii sbsp. stewartii

<400> 601
cagtggttca ttaaaatcac tgattacgcc gaagagctgt taaacgatct ggatacgctg 60

gaagagtggc ctgagcaggt caaaaccatg cagcgtaact ggatcggccg ttctgaaggg 120

gtggaaatca cctttgacgt ggcagacagc agcgaaaaag tgacggttta taccacgcgt 180

ccggacacct tctacggcgc gacctatgtc gctgtcgccg ccggtcatcc gctggcggca 240

caggcggcgg ccagcaaccc tgctctggct gattttatcg cagaatgccg caataccaag 300

gtagccgaag ccgatatggc caccatggaa aagaaaggca tggcgacagg cctgtctgtc 360

gtgcatccgt taacgggtga acgtcttcct gtgtgggtcg cgaacttcgt cctgatggaa 420

tacggtacgg gtgcggtcat ggccgtgccg gcgcatgacc agcgcgactg ggaatttgcg 480

accaaatacg atctgccgat caaaccggtt atcctcaatc tggacggcag cgagcctgag 540

gtcagcgccg ccgcgatgac cgacaaaggt acgctgttta actctgccga gtgcagcggc 600

ctggatcaca gtgccggttt taacgccatt gcagatgcgc tg 642

<210> 602
<211> 642
<212> DNA
<213> Pantoea terrea

<400> 602
cagtggttta tcaaaatcac cgactatgcc gaagaactgc tcaatgatct ggataccctg 60

gaaggctggc ctgaacaggt taagaccatg cagcgtaact ggatcgggcg ttctgaaggg 120

gtggaaatca cctttaatgt cgcagacagc gatgacacgc tgaccgtcta cacgacccgc 180

cctgatacct ttatgggggc cacctatgtg gctgttgcag ccggacaccc actggcacag 240

gaagctgcag ccggtaaccc gtcactgcag gcctttatcg aggagtgccg taacaccaaa 300

gtggctgaag cggatatggc aaccatggag aaaaaaggcc agcccaccgg ccgttttgtg 360

atccatccgc tgaccggtga acagttaccg gtctgggtcg ctaactttgt cctgatggaa 420

tacggcaccg gtgcggtgat ggccgtacct gctcatgacc agcgtgacca cgaattcgct 480

cagaaatatc agttaccggt aaaagcggtg atcctgaacg atgacggaac cgcaccggat 540

gtcagcgaaa atgcgatgac ggtgaaaggc aacctgttca attccggcgc attcgatggc 600

ttatcacatc aggacggctt taatgccatt gcggatgcac tg 642

<210> 603
<211> 642
<212> DNA
<213> Pectobacterium atrosepticum

<400> 603
cagtggttta tcaaaatcac cgcttatgca gaccaattgc tgaatgattt ggatacgctg 60

gaaagctggc ctgagcaggt caaaaccatg cagcgtaact ggatcggccg ttccgaaggt 120

gtggaaatca cctttgacgt ggcagacagt gaagagaaac tgagtgttta caccacgcgc 180

ccagatacgt tcatgggcgt gacctacgtt gctgttgccg caggccaccc tcttgcagcg 240

caggctgccg taacaaatcc agcgctagct gatttcattg cagagtgccg taataccaaa 300

gtcgctgaag ctgatatggc gacgatggag aaaaaaggca tggcgaccgg cctgtatgcc 360

attcacccac tgaacggtga aaaagtcgct atctgggttg ccaactttgt tctgatggaa 420

tacggcacag gcgccgtcat ggccgttccg ggccacgacc agcgcgactg ggaattcgcc 480

accaaatatg agctgtccat caagccggtt attctaaatg ccgacggcag cgagcccgat 540

ctttccgctc aggcgatgac cgaaaaaggc agcctgttca actcgggtga atttgacggc 600

ctggatttcg aggctggatt caatgccatt gccgataagc tg 642

<210> 604
<211> 642
<212> DNA
<213> Pectobacterium betavasculorum

<400> 604
cagtggttta ttaaaatcac cgcttacgca gaccaattgc tgaatgattt ggatacgctg 60

gaaagctggc ctgagcaggt caaaaccatg cagcgtaact ggatcggccg ttccgaaggt 120

gtggaaatca cctttgacgt ggcagacagt gaagagaaac tgagtgttta caccacacgc 180

ccagatacgt tcatgggcgt gacctacgta gctgttgccg caggccatcc tcttgcagcg 240

caggctgccg caacgaattc agcgctggct gatttcattg cagagtgccg taataccaaa 300

gtcgctgaag ccgatatggc aacgatggag aaaaaaggca tggcgaccgg cctatacgcc 360

attcacccat tgaacggtga aaaagtcgct atctgggttg ccaactttgt tctgatggaa 420

tacggcacgg gcgccgtcat ggccgttccg ggccatgacc agcgcgactg ggaattcgcc 480

accaaatatg atctgtccat caagccagtt attttgaatg ccgatggcag cgagcccgac 540

ctgtccgctc aggcgatgac agaaaaaggc agcctgttca actccggtga atttgatgga 600

ctggatttcg aggctggatt caatgccatt gccgataagc tg 642

<210> 605
<211> 642

<212>  DNA
<213>  Pectobacterium carotovorum sbsp. carotovorum T

<400>  605
cagtggttta tcaaaatcac cgcttatgca gaccaattgc tgaacgattt ggatacgctg      60

gaaagctggc ctgaacaggt caaaaccatg cagcgtaact ggattggtcg ttccgaaggt     120

gtagaaatca cctttgacgt ggcagacagc gcagagaaat tgactgtcta taccacacgt     180

ccggatacgt tcatgggcgt gacctacgtt gccgttgccg ccggtcaccc tctcgctgca     240

caagcggcag caaccaaccc agcgctggcc gatttcattg cagaatgccg taataccaaa     300

gtcgccgaag ccgatatggc gacgatggag aaaaaaggca tggccactgg cctgtatgct     360

attcacccgc tgaacggtga gaaagtcgct atctgggtcg ccaacttcgt cctgatggaa     420

tacggtacgg gcgcagtgat ggccgttccg ggccacgacc agcgcgactg ggaatttgcc     480

accaaatacg atctgtccat caagccggtt attctgaatg ccgacggcag cgagcccgat     540

ctgtccgctc aggcgatgac ggaaaaaggc aacctgttca actctgatga gtttgacggt     600

ctggatttcg aagccgggtt caacgccatt gccgataagc tg                        642

<210>  606
<211>  642
<212>  DNA
<213>  Pectobacterium carotovorum sbsp. odoriferum

<400>  606
cagtggttta tcaaaatcac cgcttatgca gaccaattgc tgaacgattt ggatacgctg      60

gaaagctggc ctgagcaggt caaaaccatg cagcgtaact ggattggccg ttccgaaggt     120

gtagaaatca cctttgacgt ggcggacagc gcagagaaac tgacggtcta taccacgcgt     180

ccggatacgt tcatgggcgt gacctacgtt gccgttgccg ccggtcaccc tctcgctgcg     240

caagctgctg caaccaaccc agcgctggcc gatttcattg ctgaatgccg taataccaaa     300

gtcgccgaag ccgatatggc gacgatggag aaaaaaggca tggccactgg cctgtatgct     360

attcacccgc tgaacggtga gaaagtcgct atctgggtcg ccaacttcgt cctgatggaa     420

tacggtacgg gcgcagtgat ggccgttccg ggccacgacc agcgtgactg ggaattcgcc     480

accaaatacg atctgtccat caagccggtt attctgaatg ccgacggcag cgagcccgat     540

ctgtccgctc aggcgatgac ggaaaaaggc aacctgttca actccggtga gtttgacggt     600

ctcgatttcg aagccgggtt caacactatt gccgataagc tg                        642

<210>  607
<211>  642
<212>  DNA
<213>  Pectobacterium cypripedii

<400>  607
cagtggttcg taaaaatcac cgattacgcc gaagaactgc tgaacgacct cgacaagctg      60

gaagactggc ctgagcaggt taaaaccatg cagcgcaact ggattggtcg ctcagaaggc     120

gtggaaatca cctttgacgt ggccttcagc gcagaaaaag tcacggtcta caccacccgt     180

ccggatacct tcctgggtgc cacctatgtt gccgtcgccg caggtcaccc gctggcacag 240

caggccgcag cgaccaatcc ggcgctgtct gacttcatcg acgaatgccg caacaccaaa 300

gtagccgaag ccgatatggc gacgatggag aaaaaaggca tggccaccgg cctgcacgtt 360

atccatccgc tgaccggcga agagatcccg gtatgggtcg ccaactttgt gttgatggaa 420

tacggcaccg gtgcggtgat ggcggttccg ggccatgacc agcgcgactg ggaatttgcc 480

actaaatact ctctgccgat caaaccagtg atcctgaacc tcgacggcac cgagccagat 540

atcagcgccg cagcgatgac cgaaaaaggc gcgctgttca actccggcga ctacaacggt 600

ctcagccacg aagctggctt taacgcgatc gctgacgcac tg 642

<210> 608
<211> 642
<212> DNA
<213> Pectobacterium wasabiae

<400> 608
cagtggttta tcaaaatcac cgcttatgca gaccaactgc tgaacgattt ggatacgctg 60

gaaagctggc ctgagcaggt caaaaccatg cagcgtaact ggattggccg ttccgaaggg 120

gtggaaatca cctttgacgt agcagacagc gcagagaaac tgagcgttta caccacacgc 180

ccggatacgt tcatgggcgt gacctacgtt gctgttgctg cgggccatcc tctcgccacg 240

caggctgccg caaccaatcc agcgttggcc gatttcattg cagagtgtcg taataccaaa 300

gtcgctgaag ccgatatggc gacgatggag aaaaaaggca tggcgaccgg cctgtatgtc 360

attcacccgc tgaacggtga gaaagtcgct atctgggttg ccaactttgt tctgatggaa 420

tacggcacag gcgcagtgat ggccgttcca ggtcacgacc agcgcgactg ggaatttgcc 480

accaaatatg acttgtccat caagccagtt attctcaata ccgacggcag cgagcccgat 540

ctgtccgctc aggcgatgac cgaaaaaggt agcctgttta actccggcga atttgacgat 600

ctggatttcg aggctggatt caatgctatt gccgataagc tg 642

<210> 609
<211> 642
<212> DNA
<213> Photorhabdus luminescens sbsp. thracensis

<400> 609
cagtggttta tcaaaatcac tgattatgcc gaagagttgc taaacgattt ggatacgctg 60

gaagagtggc ctgagcaggt caaaaccatg caacgcaact ggattggtcg ctctgaaggt 120

gtagaaatta cttttgatgt ggctgacagt caggaaaagc tgaccgtcta taccacccgt 180

cctgatacat tcctgggcgc cacttatgtc gcgattgccg caggtcatcc tctggcaaaa 240

caagcaacaa aaaacaaccc tgaactgact gctttcattg atgaatgccg taacaccaaa 300

gttgctgaag cagatatggc aacaatggag aagaaaggca tagcaaccgg catgtttgct 360

attcatccat taaccaaaga gaaagtggcg atctgggttg caaacttcgt tttgatggaa 420

tacggcaccg gtgctgtcat ggcagttcct ggtcatgacc aacgcgactg ggaatttgcg 480

acaaaatata acctgccaat caaagccgtt attctggaca gtgaaggcaa cgaaccgaat 540

gttcatgaag cgccagtgac agataagaat gtcctgttca actccggaga attcagcggc 600

ctgaatcacg aagctggttt caacgccatt gctgataaac tg 642

<210> 610
<211> 642
<212> DNA
<213> Plesiomonas shigelloides T

<400> 610
cagtggttca tcaagatcac tgactacgcc gatcagctgc tgagcgatct ggacggtctg 60

aacgaatggc cagacatggt gaaaaccatg cagcgcaact ggattggccg ctccgaaggg 120

gtcgacatca cctttgatat cgacggcatg gatgacacgc tgaccgtgta caccacccgt 180

ccagatacct tcatgggcgt gacctatgtc ggtattgccg ccggtcaccc actggcgcaa 240

aaagccgctg agaacaaccc tgagctggcg gctttcatca acgaatgccg caacaccaag 300

gtggccgaag ctgagctggc gaccatggag aaaaaaggcg tggccaccgg cctgtacgct 360

atccacccac tgaacggccg caaggtgcct gtatgggtag cgaactttgt actgatgggc 420

tacggcaccg gcgcagtcat ggcggtacca gcgcacgatc agcgcgatta tgaatttgcc 480

accaagtaca gcctgccaat tgacgcggta atcaaaccgg ctgacggtag cgagctggat 540

atctccgctc aggcatacac tgaaaaaggc attctgttta actctggtga gttcgacggt 600

ctggacttcg atggcgcgtt caatgccgtt gctgacaagc tg 642

<210> 611
<211> 642
<212> DNA
<213> Proteus hauseri

<400> 611
cagtggttta ttaaaattac tgattacgca gaagaactgt taaacgattt agatacgtta 60

gatgaatggc ctgagcaagt taagaccatg caacgtaact ggattggtcg ctctgaaggt 120

gtagaaatca cttttaaagt tgctgatagc gacgacaaaa tgactgtcta cacaacccgc 180

ccagacacct ttatgggtgt gacttatgtt gctgttgcag ctggtcaccc attagcgcaa 240

aaagcggcta aaaataaccc agcaatccaa aacttcattg atgaatgccg taatatgaaa 300

atggcagaag ctgatatggc aacaatggag aaaaaaggta tagcaacggg tatgtatgct 360

attcatcctt taacaaacga aaaagttgct atttgggtcg ctaacttcgt attaatggaa 420

tacggtacag gtgcagtaat ggcggttcca ggtcatgacg aacgtgactg ggaatttgcc 480

aataaataca atctgccaat taaagcggtt attgcagatg cagaaggtaa ccaacctgat 540

ttatcagcaa gcgcatttac tgaaaagaat agcctgatta attctggtga attcacaggg 600

cttgataacc aagctggctt taatgctatt gctgacaaat ta 642

<210> 612
<211> 642
<212> DNA

<213>  Proteus mirabilis

<400>  612
cagtggttta ttaaaatcac cgattatgct gaagagctgt taaacgactt agatacgtta      60

gatgaatggc cagaacaagt taaaaccatg caacgtaact ggattggccg gtctgaaggt     120

gtagaaatta cctttgatgt cgcagatagc gacgatacca tgaccgtata taccacacgt     180

ccagatacct ttatgggtgt cacttatgtt gctgtggccg ccggtcatcc attagctcaa     240

aaagccgctc aaaataatcc tgaaattcag cacttcattg atgaatgccg taacatgaag     300

atggcagaag ctgaaatggc aaccatggag aaaaaaggta ttgctacagg tctgtatgct     360

attcatccat taacccaaga gaaagtcgct atttgggtag ccaactttgt cctaatggaa     420

tacggtacag gtgcagttat ggccgttcca ggccacgatg aacgcgactg ggaatttgcc     480

actaaatatg gtttgccgat taaagcggtt attgctgatg cagaaggtaa ccaacctgac     540

ttatcaacag gcgctctgac tgagaaaaat agcctgatta actctggtga attctcagga     600

cttgataacc aagctggttt taatgctatt gcagataaac tt                        642

<210>  613
<211>  642
<212>  DNA
<213>  Proteus penneri

<400>  613
cagtggttta tcaaaattac cgattacgca gaagaactgt taaacgattt agatacgtta      60

gatgagtggc cagaacaagt taagaccatg caacgtaact ggattggtcg ttcagaaggt     120

gtcgaaatta aatttgatgt ggccgatagc gacgacacaa tgaccgttta tacaacgcgt     180

cctgatacat ttatgggcgt cacttatgtt gcagttgcag caggtcaccc attagcgcaa     240

aaagctgctc aaaataaccc tgaagttcag cacttcattg atgaatgccg taacatgaaa     300

atggcagaag ccgatatggc aacaatggag aaaaaaggga ttgcaacggg tctttatgct     360

atccatccat taactaaaga gaaagtagct atttgggttg ctaactttgt attaatggaa     420

tacggcacag gtgctgtaat ggcggttcca ggtcatgatg aacgtgactg ggaatttgca     480

actaaataca atttaccaat taaagctgtt attgcggatg ctgaaggtaa tcaacctgac     540

ttatctgaag gcgcattaac cgagaaaaat agcctgatta actctggtga attctctgga     600

ttagacaacc aagctggctt taacgcaatt gcagataaac ta                        642

<210>  614
<211>  642
<212>  DNA
<213>  Providencia alcalifaciens T

<400>  614
cagtggttta tcaaaatcac cgactacgct gaagagctgc tcaatgactt ggacaaactg      60

gaagattggc cagaacaagt taaaaccatg cagcgcaact ggattggtcg ctcagaaggt     120

gtggaaatca cttttgatgt tgccgaccgt gcagagaaat taactgtcta caccacccgc     180

ccagatacct ttatgggagc gacctatgta gcggttgcag caggtcaccc attagcgcaa     240

gaagctgcca aagcgaatag cgaattagcc gactttatcg atgagtgccg taataccaaa 300

actgcggaag cggatatggc aaccatggag aaaaaaggtc tggcaacagg tctgtatgtc 360

gttcatccat taacccaaga aaaattacca atttgggtcg cgaactttgt tctgatggaa 420

tacggtacag gcgcagtgat ggcggttcca gctcacgacc aacgagactg ggaatttgct 480

cataaataca atctgccaat aaaagcagtg attgcagatg ctgaaggcaa cacgcctgac 540

ttatctcaag aagcgatgac tgagaaaaat gccctgatca actcaggtga gtttactggt 600

ttaagcaacg aagctggctt caatgctatc gcagataaac ta 642

<210> 615
<211> 642
<212> DNA
<213> Providencia heimbachae

<400> 615
cagtggttta ttaaaatcac tgactatgca gaggagttgc tgaacgacct cgataaactg 60

gaagattggc cagagcaagt taagaccatg cagcgcaact ggattggtcg ctcagaagga 120

acggaaatta cctttaatgt ggcagatcgt gatgaaacat taacggttta caccactcgc 180

ccagatactt ttatgggtgc aacttatgtt gctgttgccg ctggccatcc tctggcaaaa 240

gaagctgcca cgaataaccc agaactcgcg caatttatcg atgaatgccg taacaccaaa 300

accgccgaag ctgaaatggc aacgatggat aaaaaaggta tggcgaccgg tatttatgtt 360

atccatccac tcacacaaga aaaactgcca atctgggtag ctaactttgt tttgatggaa 420

tatggaacag gcgctgtgat ggcagttcca gcacatgacc agcgtgactg ggagtttgca 480

cacaaataca atttaccgat taaagccgtc attgcaaatg ctgaaggtaa tgaaccagac 540

ctgtcagaag gcgccatgac tgaaaaaaat tcactgatta attcaggtga atttaacggt 600

ctcgataacg aagcaggctt taatgctatt tcagacaaac tg 642

<210> 616
<211> 642
<212> DNA
<213> Providencia stuartii

<400> 616
caatggttta ttaaaatcac cgattatgct gaagagttgc taaacgatct tgataagctt 60

gatgattggc ctgagcaagt caaaaccatg caacgtaact ggattgggcg ctctgagggt 120

gtcgaaatca cctttaacgt tgccgatcgt gatgacaaat taaccgtata taccactcgc 180

cctgatactt tcatgggtgc aacttatgtg gcggttgctg cgggtcaccc attagcactc 240

gaagccgcag aaaaaaatgc cgaattagcc aactttatcg atgaatgccg taataccaaa 300

acagccgaag ctgatatggc gaccatggat aaaaaaggga tgccaactgg cctttatgtt 360

attcatccac tcacccaaga aaaattacct atctgggtag ccaactttgt gttgatggaa 420

tatggcaccg gtgcggtgat ggctgtacct gctcacgacc aacgtgactg ggagtttgcc 480

cataaatata acctgccact aaaagtggtt attgccgacg ctgaaggcaa tgagcctgat 540

atttctcaag aagcgatgac tgaaaaattt gcattgatca actcaggcga atttagcggc 600

ttagataata ccgctggctt caatgctatc gcagacaaat ta 642

<210> 617
<211> 642
<212> DNA
<213> Rahnella aquatilis T

<400> 617
cagtggttca tcaaaatcac ggcttacgcc gatcagttgc tcaatgatct ggacacgctg 60

gaaagctggc cagagcaggt gaaaaccatg cagcgcaact ggattggccg ctcggaaggg 120

gtggaaatca cttttgatgt ggctgacagc gatgaaaaag tcacggttta caccacccgt 180

cctgatacgt ttatgggcgc gacctacgtc gccgtggctg ccggtcatcc gctggcgcaa 240

ctgggtgcac agaataatcc tgcgctgaac gattttatcg acgaatgccg taacaccaaa 300

gttgccgaag cagaaatggc gacgatggaa aagaaaggca tggcaaccgg tctgtttgcc 360

attcacccgc tgtccggtga aaagctgcca gtctgggtcg ctaacttcgt tctgatggaa 420

tacggcaccg gtgcggtgat ggccgttcca gctcacgatc agcgtgactg ggaattcgcc 480

accaaatata acctgcagat taaaccggtt attctcaacg ttgatggcag ccagcctgat 540

gtcaccgcag aagcgatgac tgagaaaggc acgctgtaca attcaggcga atttgatggt 600

ctgaatcacg aagacggttt caatgccatt gccgacaagc tg 642

<210> 618
<211> 642
<212> DNA
<213> Raoultella ornithinolytica

<400> 618
cagtggttta tcaaaatcac cgcctacgcc gacgaactgc tgaacgatct ggataaactg 60

gatcactggc ctgataccgt taagaccatg cagcgcaact ggatcggccg ctccgaaggg 120

gtagaaatct ccttcaacgt taacgagtat gccgacaagc tgaccgtcta caccacccgt 180

ccggacacct tcatgggctg tacctacctg gccgtagccg cgggtcatcc gctggcacag 240

caggcggcag cgaacaaccc tgagctggct gcatttatcg acgaatgccg caacaccaaa 300

gtcgccgaag cagaaatggc gacgatggag aaaaaaggtg tcgataccgg ctacaaagcg 360

attcacccgc tgaccggcga agaaattccg gtctgggcgg caaacttcgt gctgatggaa 420

tacggcaccg gcgccgtcat ggccgttccg ggtcacgatc agcgcgacta cgaatttgcc 480

agcaaatatg gcctgaacat taagccggtt attctggcgg cagacggctc agaaccggat 540

ctctccgggc aggcgctgac tgaaaaaggc accctcttca actccggcga gttcgatggc 600

ctgagctacg aagcgggctt taacgctatc gccgacaaac tg 642

<210> 619
<211> 642
<212> DNA
<213> Raoultella planticola T

<400> 619
cagtggttta tcaaaatcac cgcctacgcc gacgagctgc tcaacgatct ggataagctg 60

gatcactggc ctgataccgt taagaccatg cagcgcaact ggatcggccg ttctgaaggg 120

gtagaaatct ccttcaacgt taacgagtat gccgacaagc tgaccgtcta caccacccgt 180

ccggacacct tcatgggctg tacctacctg gccgtagccg cgggtcatcc gctggcgcag 240

caggcggcag cgaacaaccc ggagctggct gcgtttatcg acgaatgccg taacaccaaa 300

gtcgccgaag cagaaatggc gacgatggag aaaaaaggcg tcgataccgg ctacaaagcg 360

attcacccgc tgaccggcga agagattccg gtctgggcgg caaacttcgt gctgatggaa 420

tacggcaccg gcgccgtcat ggccgttccg ggtcacgatc aacgcgacta cgaatttgcc 480

agcaaatatg gtctgaacat taagccggtt attctggcgg cagacggctc agcaccggat 540

ctgtccgagc aggcgctgac tgaaaaaggc accctgttca actccggcga gttcgacggc 600

ctgagccacg aagcgggctt caacgctatt gccgacaaac tg 642

<210> 620
<211> 642
<212> DNA
<213> Raoultella terrigena

<400> 620
cagtggttta tcaaaattac cgcctacgcc gacgagctgc tgagagatct ggataatctg 60

gatcactggc ctgataccgt caagaccatg cagcgcaact ggatcggccg ttctgaaggg 120

gtcgaaatct ccttcaacgt caacgactac gccgacaagc tgaccgtcta caccacgcgt 180

cctgatacct ttatgggctg ctcctacctg gccgtagccg cgggtcaccc gttggcgcag 240

caggcggcgg ccaataaccc ggcgctggct gcatttatcg acgaatgccg caacaccaaa 300

gtcgcagaag ctgaaatggc gacgatggag aaaaaaggcg tcgacaccgg ctttaaagcc 360

atccatccgc tgaccggcga agctattccg gtatgggcgg ccaacttcgt gctgatggag 420

tacggtaccg gcgcggtgat ggcggttcca ggccacgacc agcgcgacta tgagtttgcc 480

acaaaatatg gcctcaacat caagcaggtt atcctcgcgg cagacggctc tgagcccgat 540

ctctccgagc aggcgctgac ggaaaaaggc gtgctgttca actccggcga attcagcggg 600

ctgagctttg aagcgggctt caacgccatc gccgacaagc tg 642

<210> 621
<211> 642
<212> DNA
<213> Salmonella bongori

<400> 621
cagtggttta tcaaaatcac cgcttatgcg gaagaactgc tgggcgatct ggataaactg 60

gatcactggc cggataccgt taagaccatg cagcgcaact ggatcggtcg ctcggaaggc 120

gtggaaatca ccttcgacgt taagggttac gataacacgt tgaccgtgta taccacccgt 180

ccagatacct ttatgggcgc cacttacctg gcggtggccg cgggtcaccc gcttagcgtt 240

aaagccgcgg caaataatcc tgatctggcg acgttcattg acgaatgtcg caacactaaa 300

gtggccgaag ctgaaatggc gacgatggag aaaaaaggcg tcgacactgg ctttaaagcg 360

attcacccat taaccggcga agagatcccg gtgtgggcgg caaactttgt cctgatggag 420

tacggcactg gcgcggtgat ggccgttccg ggtcacgatc agcgcgacta tgagtttgcc 480

accaaatacg gtttaaccat caaaccggtg attctggccg ctgacggctc tgagcccgat 540

ttatctgagc aggcgctgac tgaaaaaggc gtgctgttta actccggcga gttcaatggt 600

ctgggctttg aagacgcttt caacgccatt gccgataagc tg 642

<210> 622
<211> 642
<212> DNA
<213> Salmonella enterica sbsp. arizonae

<400> 622
cagtggttta tcaaaatcac cgcttacgca gacgaactgt tgagcgatct ggataagctg 60

gaccactggc ctgataccgt gaaaacgatg cagcgcaact ggattggccg ctctgaaggc 120

gtggaaatca ccttcgacgt gaaaggttac gataacacgt tgaccgtcta taccacccgc 180

cctgacactt ttatgggcgc cacctacctg gctgtggctg ccggtcatcc gctagcgcag 240

aaagcggcgg cgaacaatcc tgagctggcg gttttcattg acgaatgccg caacaccaaa 300

gttgccgaag ctgaaatggc gaccatggag aaaaaaggcg ttgataccgg ctttaaagcg 360

atccacccgt taaccggcga agagatcccg gtatgggcgg caaacttcgt gttgatggag 420

tacggcactg gcgcggtgat ggccgttccg ggccacgacc agcgcgacta tgaattcgcc 480

accaaatatg gtttgaccat caaaccggtt attctggccg ccgacggctc cgcgccggat 540

ctctccacgc aggcgctgac cgaaaaaggc gtgctgttta attccggcga gttcgacggc 600

ctggcctttg aagcggcgtt taacgccatt gccgacaagc tg 642

<210> 623
<211> 642
<212> DNA
<213> Salmonella enterica sbsp. diarizonae

<400> 623
cagtggttta tcaaaatcac cgcttacgca gacgaactgt tgcgcgatct ggataagctg 60

gaccactggc ctgataccgt gaaaacgatg cagcgcaact ggattggccg ctctgaaggc 120

gtggaaatca ccttcgacgt gaagggttac gataacacgc tgaccgtcta taccacccgt 180

ccggatacct ttatgggcgc cacttacctg gcggtggccg ccggtcaccc gctggcgcaa 240

caggccgccg cgaacaacgc cgagctggcg gcgttcatcg acgaatgccg taacaccaaa 300

gtggccgaag ccgaaatggc gaccatggag aaaaaaggcg tcgacaccgg ctttaaagcg 360

attcatccgt taaccggcga agagatcccg gtctgggcgg caaacttcgt gttgatggag 420

tacggcaccg gcgcggtgat ggccgttccg ggtcacgatc agcgcgacta cgaatttgcc 480

tccaaatacg gtttgaccat caagccggtt attctggctg ccgacggttc cgagccggat 540

ctttccgaac aggcgctgac cgaaaaaggc gtgctgttta actccggcga gttcgacggc 600

ctggcttttg aagcggcgtt taacgccatt gccgataagc tg 642

<210> 624
<211> 642
<212> DNA
<213> Salmonella enterica sbsp. enterica T

<400> 624
cagtggttta tcaaaatcac cgcttacgca gacgagctgt tgcgcgatct ggataagctg 60

gaccactggc ctgataccgt aaaaacgatg cagcgcaact ggattggccg ctctgaaggc 120

gtggaaatca ccttcgatgt gaagggttac gataacaccc tgaccgtcta taccacccgt 180

ccagatacct ttatgggcgc cacttacctg gcggtggccg ccggtcatcc gctggcgcaa 240

aaagcggcgg cgaacaacgc cgaactggcg gcgttcatcg acgaatgccg taacaccaaa 300

gtggccgaag ccgaaatggc gaccatggag aaaaaaggcg tcgacaccgg ctataaagcg 360

attcatccgt taactggcga agagatcccg gtctgggcgg caaactttgt cctgatggag 420

tacggcactg gcgcggtgat ggccgttccg ggtcacgatc agcgcgacta cgaatttgcc 480

tccaaatacg gtttgaccat caagccggtg attctggccg ccgacggttc cgagccggat 540

ctttccgaac aggcgctgac cgaaaaaggc gtgctgttta actccggcga gttcgacggc 600

ctggcctttg aagcggcgtt taacgccatt gccgataaac tg 642

<210> 625
<211> 642
<212> DNA
<213> Salmonella enterica sbsp. houtenae

<400> 625
cagtggttta ttaagatcac cgcctacgcc gatgaactgt tgaacgatct ggataagctt 60

gaccactggc ctgacactgt gaaaacgatg cagcgcaact ggatcggtcg ttccgaaggc 120

gtggaaatca ccttcgacgt gaaaggttac gataacactc tgaccgtcta cactacgcgc 180

cctgatacct ttatgggcgc cacttacctg gcggtagccg ctggtcatcc gctggcgcaa 240

aaagcggcgg caaacaacgc cgaactggcg gcgttcatcg acgaatgccg taacaccaaa 300

gttgccgaag ccgacatggc gaccatggag aaaaaaggcg ttgatacagg ctttaaagca 360

attcatccgt taactggcga agagatcccg gtgtgggcgg caaatttcgt gttgatggag 420

tacggcactg gcgcggtgat ggccgttccg gggcacgatc agcgcgacta cgaatttgcc 480

accaaatacg gtttgaccat caaaccggtt attctggccg ccgacggttc cgaaccggat 540

ctctccgcac aggcgctgac cgaaaaaggc gtgctgttta actccggcga gttcgacgga 600

ctgggctttg aagcggcgtt taacgccatt gccgataaac tg 642

<210> 626
<211> 642
<212> DNA
<213> Salmonella enterica sbsp. indica

<400> 626
cagtggttta ttaagattac cgcctatgca gatgaactgt taagcgatct ggataagctg 60

gaccactggc ctgataccgt aaaaacgatg cagcgcaact ggattggccg ctctgaaggc 120

gtggaaatca ccttcgacgt gaagggttac gataacacgc tgaccgtcta taccacccgt 180

ccggatacct ttatgggcgc cacttacctg gcagtggccg ccggtcaccc tctggcgcaa 240

aaagcggcgg cgaacaacgc cgaactggcg gcgtttatcg acgaatgccg taacaccaaa 300

gtggccgaag ccgaaatggc gaccatggag aaaaaaggcg tcgacaccgg ctttaaagcg 360

attcatccgt taactggcga agagatcccg gtctgggcgg caaactttgt cctgatggag 420

tacggcaccg gcgcggtgat ggccgttccg ggtcacgatc aacgcgacta cgaatttgcc 480

tccaaatacg gtttgaccat caagccggtt attctggccg ctgacggttc cgaaccggat 540

ctttccgaac aggcgctgac cgaaaaaggc gtgctgttta actccggcga gttcgacggc 600

ctggcctttg acgcggcgtt taacgccatt gccgataaac tg 642

<210> 627
<211> 642
<212> DNA
<213> Salmonella enterica sbsp. salamae

<400> 627
cagtggttta tcaaaatcac cgcttacgca gacgagctgt tgcgcgatct ggataagctg 60

gaccactggc ctgataccgt gaaaacgatg cagcgcaact ggattggccg ctctgaaggc 120

gtggaaatca ccttcgatgt gaagggttac gataacacgc tgaccgtcta taccacccgt 180

ccggatacct ttatgggcgc cacttatctg gcggtggccg ctggtcatcc gctggcgcaa 240

aaagcggcgg cgaacaacgc cgagctggcg gcgttcatcg acgaatgccg taacaccaaa 300

gtggccgaag ccgaaatggc gaccatggag aaaaaaggcg tcgacaccgg ctataaagcg 360

attcatccgt taactggcga agagatcccg gtctgggcgg caaactttgt cctgatggag 420

tacggcaccg gcgcggtgat ggccgttccg ggtcacgatc agcgcgacta cgaatttgcc 480

tcaaaatacg gtttgaccat caagccagtt attctggccg ccgacggttc caggccggat 540

ctctccgaac aggcgctgac cgaaaaaggc gtgctattta actccggcga gttcgacggc 600

ctggcctttg aagcggcgtt taacgccatt gccgataaac tg 642

<210> 628
<211> 642
<212> DNA
<213> Samsonia erythrinae T

<400> 628
cagtggttta tcaaaatcac cgcgtatgca gaccaactgc tgaacgattt ggatacgctg 60

gaaagctggc ctgagcaggt caaaaccatg cagcgtaact ggatcggtcg ttccgaaggc 120

gtggaaatca cctttgacgt ggcggacagc gaagagaaac tgaccgttta caccacgcgt 180

ccggatacct tcatgggcgt gacctacgtt gccgttgccg ctggccaccc tctcgccgct 240

caggctgccg caaccaatcc tgccctggcc gatttcattg ccgaatgccg taataccaaa 300

gtcgccgaag ccgacatggc gacgatggag aaaaaaggca tggccaccgg cctgtatgcg 360

attcacccgt tgaacggcga gaaagtcgct atctgggtcg ccaacttcgt cctgatggaa 420

tacggcacgg gcgcggtgat ggccgttccg ggccacgacc agcgcgactg ggagttcgcc 480

acgaaatatg cgttgtccat caagccggtt attcttaacg ccgacggcag cgagcccgac 540

ctgtccgccg aggcgatgac ggaaaaaggc cgcctgttca actccggcga gtttgacgga 600

ctggatttcg acgccgcgtt caatgcgatt gccgataaac tg 642

<210> 629
<211> 642
<212> DNA
<213> Serratia entomophila

<400> 629
cagtggttta tcaagatcac cgactacgcc gatcagctgc tgaacgatct ggatacgctg 60

gaaagctggc ctgagcaggt caaaaccatg cagcgcaact ggattggccg ttccgaaggg 120

gtggacatca cctttaacgt ggcggacagc gacgaaacgc tgacggttta caccacccgc 180

ccggacacct tcatgggcgc cacctatgtg gcggtggccg caggccatcc gttggcgcaa 240

cagggcgcga tcaacaaccc ggcgctggcc gaattcatcg aagaatgccg caacaccaaa 300

gtagccgaag cagacatggc gacgatggag aagaaaggca tgccgaccgg cctgttcgtt 360

atccatccgc tgagcggcga aaagctgccg atctgggtgg ccaacttcgt gctgatggaa 420

tacggcaccg gcgccgtgat ggcggtaccg gctcacgacc agcgcgactg ggagttcgcc 480

accaagtatc acctgccgat caacccggtc atcctcaacc tcgacggcag cgaacctgac 540

gtgcacgccg cggcgatgac cgagaaaggc gcgctgttca actccggcga gttcgatggc 600

ctggaccacg aagccggctt caacgccatc gccgacaaac tg 642

<210> 630
<211> 642
<212> DNA
<213> Serratia ficaria

<400> 630
cagtggttca tcaaaatcac cgactacgcc gatcagctgc tgaacgatct ggatacgctg 60

gaaagctggc ctgagcaggt caaaaccatg cagcgcaact ggattggccg ttccgaaggg 120

gtggatatca cctttaacgt ggcggacagc gacgaaacgc tgacggttta caccacccgt 180

ccggacacct tcatgggtgc cacctatgtg gcggtggccg caggccaccc gttggcgcag 240

cagggcgcgc tcaacaaccc ggcgctgacc gaattcatcg aagaatgccg caacaccaaa 300

gtcgccgaag cggaaatggc gacgatggag aagaaaggca tgccgaccgg cctgttcgtt 360

atccacccgc tgagcggcga aaagctgccg atctgggtgg ccaacttcgt gctgatggaa 420

tacggcaccg gcgccgtgat ggcggtgccg gcccacgacc agcgcgactg ggagttcgcc 480

accaagtatc acctgccgat caatccggtg atcctgaacc tcgacggcag cgagcctgac 540

gtgcacgccg cggcgatgac cgaaaaaggc gcgctgttca actccggcga gttcgacggc 600

ctggaccacg aggccggttt caacgccatc gccgaccaac tg 642

<210> 631
<211> 642
<212> DNA
<213> Serratia fonticola

<400> 631
cagtggttta ttaaaatcac cggttacgcc gatcaactgc tcaacgacct ggacacgctg 60

gaaagctggc cagagcaggt gaaaaccatg cagcgcaact ggattggccg ttccgaaggg 120

gtagaaatca ccttcgacgt tgccgactgc gctgagaaag ttaccgttta caccacccgc 180

ccagacacct ttatgggtgc tacctatgtc gccgttgccg caggtcaccc gctggcacag 240

cagggcgcgg tcaacaaccc ggcgctgaca gacttcatcg acgaatgccg caacaccaag 300

gttgccgaag ccgaaatggc caccatggag aagaaaggca tggccaccgg cctgtttgcc 360

attcatccgt tgagtggtga gaaactgcca atctgggtag ccaacttcgt cctgatggaa 420

tacggcaccg gtgcggtaat ggccgtccct ggccacgatc agcgtgactg ggagttcgct 480

accaaatacg atctgcagat caaaccggtc attttgaacc tggacggcag caagccagac 540

gtcagcgccg aagcgatgac cgaaaaaggc accctgttca actccggcga attcgacgga 600

ctggatcacc tggcgggctt taacgccatc gccgacaagc tg 642

<210> 632
<211> 642
<212> DNA
<213> Serratia grimesii

<400> 632
cagtggttta ttaaaatcac tgactacgcc gatcaactgc tcaacgattt ggacacgctt 60

gaaagctggc cagagcaggt aaaaaccatg cagcgtaact ggattggccg ttctgaaggg 120

gtggacatca cctttgacgt ggcgggcagc gatgagaaac tgacggttta taccacccgt 180

cctgacacct ttatgggtgc cacttacgtc gccgttgccg cgggccaccc gctggcgcag 240

caaggtgcag tgaacaaccc tgcactgact gaatttatcg aagagtgccg taacaccaaa 300

gtggccgaag ccgaaatggc gacgatggaa aagaaaggca tgcctactgg cctgttcgcc 360

atccacccac tgtctggcga gcaagtgcca atttgggttg ccaactttgt actgatggaa 420

tacggcacag gtgccgtgat ggcagtccct gctcacgatc aacgtgactg ggagttcgcg 480

accaaatacg gtttgccgat caaaccggtc gtgctggcac tcgacggcag ccaacccgat 540

gtgcaagccg aagcgatgac cgaaaaaggc acgctgttca actctggcga gtttgacggt 600

ttggatcacc aagccggttt caacgccatt gccgacaaat tg 642

<210> 633
<211> 642
<212> DNA
<213> Serratia liquefaciens

<400> 633

cagtggttta tcaaaatcac cgactacgcc gatcagctgc tcaacgatct ggacacgctg 60

gaaagctggc cagaacaggt gaaaaccatg cagcgcaact ggattggccg ttccgaaggg 120

gtggatatca cctttgacgt agcgggcagc gacgaaaagc tgacggttta caccacccgt 180

cctgacacct ttatgggcgc gacctacgtg gccgtagccg caggtcaccc attggcgcag 240

caaggcgcgg tgaacaaccc ggcgctggct gaatttatcg aagagtgccg caacaccaaa 300

gtggccgaag ccgaaatggc gacgatggag aaaaagggca tgccgaccgg cctgttcgcc 360

atccatccgc tgtccggcga gcaagtgcca atttgggtcg ccaactttgt gctgatggaa 420

tacggcaccg gcgcggtgat ggcggttccg gctcacgatc agcgcgactg ggaattcgcc 480

accaaatacg gcctgccaat caaaccggtc gttctggccc tcgacggcag ccaaccggat 540

gtacaagccg aagcgatgac cgagaaaggc acgctgttca actccggcga gtttgacagt 600

ctggatcacc aagacggttt caacgccatt gccgacaaac tg 642

<210> 634
<211> 642
<212> DNA
<213> Serratia marcescens sbsp. marcescens T

<400> 634
cagtggttca tcaaaatcac cgcttacgcc gatcagctgc tgaacgatct ggacacgctg 60

gaaagctggc cggagcaggt gaaaaccatg cagcgcaact ggattggccg ctccgaaggc 120

gtggaaatca ccttcgacgt ggcggacagc gaagagaagt tgacggttta caccacccgc 180

ccggacacct tcatgggcgc cacctacgtg gcggtcgccg cgggccaccc gctggcgcaa 240

cagggcgcgc gcaacaatcc ggcgctgacc gagttcatcg acgaatgccg caacaccaaa 300

gttgccgaag ccgaaatggc gacgatggag aagaaaggca tgccgaccgg cctgttcgtt 360

gtccacccgt tgagcggcga gaagctgccg gtgtgggtcg ccaacttcgt gctgatggaa 420

tacggcaccg gcgccgtgat ggcggtaccg gcgcacgacc agcgcgactg ggaattcgcc 480

accaaatatg atttgccgat caaaccggtg atcctgaacc tcgacggcag ccagcctgac 540

gtaagcgccg aagcgatgac cgacaaaggc gcgctgttca actccggcga gttcgacggt 600

ctggacaatg aagccggctt caacgccatc gccgacaaac tg 642

<210> 635
<211> 642
<212> DNA
<213> Serratia marcescens sbsp. sakuensis

<400> 635
cagtggttca tcaaaatcac cgcttacgcc gatcagctgc tgaacgatct ggacacgctg 60

gaaagctggc cggagcaggt gaaaaccatg cagcgcaact ggattggccg ctccgaaggc 120

gtggaaatca ccttcgacgt ggcggacagc gaagagaagt tgacggttta caccacccgc 180

ccggacacct tcatgggcgc cacctacgtg gcggtcgccg cgggccaccc gctggcgcaa 240

cagggcgcgc gcaacaatcc ggcgctgacc gagttcatcg acgaatgccg caacaccaaa 300

gttgccgaag ccgaaatggc gacgatggag aagaaaggca tgccgaccgg cctgttcgtt 360

gtccacccgc tgagcggcca gaagctgccg gtgtgggtcg ccaacttcgt gctgatggaa 420

tacggcaccg gcgccgtgat ggcggtaccg gcgcacgacc agcgcgactg ggaattcgcc 480

accaaatatg atttgccgat caaaccggtg atcctgaacc tcgacggcag ccagcctgac 540

gtaagcgccg aagcgatgac cgacaaaggc gcgctgttca actccggcga gttcgacggt 600

ctggacaatg aagccggctt caacgccatc gccgacaaac tg 642

<210> 636
<211> 642
<212> DNA
<213> Serratia odorifera

<400> 636
cagtggttca tcaagatcac cgcttacgcc gatcagttgc tgaacgatct ggacacgctt 60

gaaagctggc cagagcaggt aaaaaccatg cagcgcaact ggattggccg ttctgaaggc 120

gtggaaatca ccttcgacgt cgccgatagc gacgagaagg tcacggtgta caccacccgt 180

ccagatacct ttatgggcgc gacctacgtc gcagtcgccg cgggccaccc gctggcgcaa 240

caaggcgcca tcaacaaccc gacattggcc gagtttatcg aagaatgccg caacaccaag 300

gttgccgaag ccgaaatggc caccatggag aagaaaggca tggccaccgg cctgttcgcc 360

attcatccgt tgagcggtga aaagctgccg gtgtgggtcg ccaacttcgt gttgatggaa 420

tacggcaccg gtgcggtcat ggccgttccc gcccacgatc agcgcgactg ggaattcgcc 480

accaaatacg atttgcgcat cgcgccggtg atcctcaacc ttgacggcag ccagcctgac 540

gtgagcagcg aagccatgac cgacaaaggc gcgctgttca actccggcga gttcgacggt 600

ctggaccacg aagccggctt caatgccatt gccgacaaac tg 642

<210> 637
<211> 642
<212> DNA
<213> Serratia plymuthica

<400> 637
cagtggttca tcaaaattac cgactacgcc gatcagttgc tcaacgatct ggatacgctg 60

gaaagctggc cagaacaggt gaaaaccatg cagcgcaact ggattggccg ctctgaaggg 120

gtggacatca cctttgacgt ggcgggcagc gacgagaagc tgaccgtgta caccacccgt 180

ccggatacct tcatgggcgc cacctatgtg gccgtcgcgg cgggccaccc gctggcgcaa 240

cagggtgcgg tgaataaccc ggcgctggct gaatttatcg aagagtgccg caataccaag 300

gtggccgaag ccgacatggc gacgatggag aagaaaggca tgccgaccgg cctgttcgcc 360

attcatccgc tgtccggcga gaaggtgccg gtttgggttg ccaacttcgt cttgatggaa 420

tacggcaccg gtgcagtgat ggcggttccg gctcacgacc agcgcgattg ggagttcgcc 480

accaaatatg gcctgccgat caaaccggtg gtgctggcgc tcgacggcag ccagccggac 540

gtacacgccg aagcgatgac cgaaaaaggc acgctgttca actccggcga gttcgatggc 600

ttggatcacg aagccggttt caacgccatt gccgacaaac tg 642

<210> 638
<211> 642
<212> DNA
<213> Serratia quinivorans

<400> 638
cagtggttta tcaaaatcac cgactacgcc gatcaactgc tgaacgatct cgatacgctg 60

gaaagctggc cagagcaggt gaaaaccatg cagcgcaact ggattggccg ttccgaaggg 120

gtggatatca cctttgacgt ggcgggcagc gacgagaaac tgaccgttta caccacccgt 180

ccagacacct ttatgggcgc gacctacgtg gccgttgccg cgggtcaccc attggcgcaa 240

caaggcgcgg tgaacaaccc ggcgctggct gaatttatcg aagaatgccg caacaccaaa 300

gtggccgaag ccgaaatggc aacgatggag aagaaaggca tgccgaccgg cctgttcgcc 360

atccatccgc tgtccggcga gcaagtgccg gtttgggtcg ccaacttcgt gctgatggaa 420

tacggcaccg gcgcggtaat ggccgttccg gctcacgacc agcgtgactg ggagttcgcc 480

accaaatacg gtttgccgat caaaccggta gtgctggcgc tcgatggcag ccaaccggat 540

gtccaggccg aagccatgac cgagaaaggc acgctgttca attccggcga gtttgacggt 600

ttggaccacc aggacggttt caacgccatt gccgacaaac tg 642

<210> 639
<211> 642
<212> DNA
<213> Serratia ureilytica

<400> 639
cagtggttca tcaaaatcac cgcctacgcc gatcagctgc tgaacgatct ggacacgctg 60

gaaagctggc cggagcaggt gaaaaccatg cagcgcaact ggattggccg ctccgaaggc 120

gtggaaatca ccttcgacgt ggctggcagc gaagagaagc tgacggttta caccacccgt 180

cccgacacct tcatgggcgc cacctacgtg gcggtcgccg cgggccaccc gctggcgcaa 240

cagggcgcgc gcaacaaccc ggcgctgacc gacttcatcg acgaatgccg caacaccaaa 300

gtggccgaag ccgaaatggc gacgatggag aagaaaggca tgccgaccgg cctgttcgtt 360

gtccacccgc tgagcggcga gaagctgccg gtgtgggtcg ccaacttcgt gctgatggaa 420

tacggcaccg gcgccgtgat ggcggtaccg gcgcacgacc agcgcgactg ggaattcgcc 480

accaaatacg atttgccgat caaaccggtg atcctgaacc tcgacggcag ccagcctgac 540

gtgagcgccg aagcgatgac cgacaaaggc gcactgttca actccggcga gttcgacggc 600

ctggacaatg aagcgggctt caacgccatc gccgacaaac tg 642

<210> 640
<211> 642
<212> DNA
<213> Shigella boydii

<400> 640
cagtggttta tcaaaatcac tgcttacgct gacgagctgc tcaacgatct ggataaactg 60

gatcactggc cagacaccgt taaaagcatg cagcgtaact ggatcggtcg ttccgaaggc 120

gtggagatca ccttcaacgt taacgactat gacaacacgc tgaccgttta cactacccgc 180

ccggacacct ttatgggttg tacctacctg gcggtagctg cgggtcatcc gctggcgcag 240

aaagcggcgg aaaataatcc tgaactggcg gcctttattg acgaatgccg taacaccaaa 300

gttgccgaag ctgaaatggc gacgatggag aaaaaaggcg tcgatactgg ctttaaagct 360

gttcacccat taacgggcga agaaattccc gtttgggcag caaacttcgt attgatggag 420

tacggcacgg gcgcagttat ggcggttccg ggtcacgacc agcgcgacta cgagtttgcc 480

tctaaatacg gcctgaacat caaaccagtt atcctggctg ctgacggctc tgagccagat 540

ctttctcagc aagccctgac tgaaaaaggc gtgctgttca actctggcga attcaacggt 600

cttgaccatg aagcggcctt caacgccatc gccgataaac tg 642

<210> 641
<211> 642
<212> DNA
<213> Shigella dysenteriae T

<400> 641
cagtggttta tcaaaatcac cgcttacgct gacgaactgc tcaacgatct ggataaactg 60

gatcactggc cagacaccgt taaaaccatg cagcgtaact ggatcggtcg ttccgaaggc 120

gtggaaatca ccttcaacgt taacgactat gacaacacgc tgaccgttta cactacccgt 180

ccggacacct ttatgggttg tacctacctg gcggtagcgg cgggtcatcc gctggcgcag 240

aaagcggcgg aaaataatcc tgaactggcc gcctttattg acgaatgccg taacaccaaa 300

gttgccgaag ctgaaatggc gacgatggag aaaaaaggcg tcgatactgg ctttaaagcg 360

gttcacccat taacgggcga agaaattccc gtttgggcag caaacttcgt attgatggag 420

tacggcacgg gcgcagttat ggcggttccg ggtcacgacc agcgcgacta cgagtttgcc 480

tctaaatacg gcctgaacat caaaccggtt atcctggcag ctgacggctc tgagccagat 540

ctttctcagc aagccctgac tgaaaaaggc gtgctgttca actctggcga gttcaacggt 600

cttgaccatg aagcggcctt caacgccatc gccgataaac tg 642

<210> 642
<211> 642
<212> DNA
<213> Shigella flexneri

<400> 642
cagtggttta tcaaaatcac tgcttacgct gacgagctgc tcaacgatct ggataaactg 60

gatcactggc cagacaccgt taaaaccatg cagcgtaact ggatcggtcg ttccgaaggc 120

gtggagatca ccttcaacgt taacgactat gacaacacgc tgaccgttta cactacccgc 180

ccggacacct ttatgggttg tacctacctg gcggtagctg cgggtcatcc gctggcgcag 240

aaagcggcgg aaaataatcc tgaactggcg gcctttattg acgaatgccg taacaccaaa 300

gttgccgaag ctgaaatggc gacgatggag aaaaaaggcg tcgatactgg ctttaaagct 360

gttcacccat taacgggcga agaaattccc gtttgggcag caaacttcgt attgatggag 420

tacggcacgg gcgcagttat ggcggttccg ggtcacgacc agcgcgacta cgagtttgcc 480

tctaaatacg gcctgaacat caaaccggtt atcctggcgg ctgacggctc tgagccagat 540

ctttctcagc aagccctgac tgaaaaaggc gtgctgttca actctggcga gttcaacggt 600

cttgaccatg aagcggcctt caacgccatc gccgataaac tg 642

<210> 643
<211> 642
<212> DNA
<213> Shigella sonnei

<400> 643
cagtggttta tcaaaatcac tgcttacgct gacgagctgc tcaacgatct ggataaactg 60

gatcactggc cagacaccgt taaaaccatg cagcgtaact ggatcggtcg ttccgaaggc 120

gtggagatca ccttcaacgt taacgactat gacaacatgc tgaccgttta cactacccgc 180

ccggacacct ttatgggttg tacctacctg gcggtagctg cgggtcatcc gctggcgcag 240

aaagcggcgg aaaataatcc tgaactggcg gcctttattg acgaatgccg taacaccaaa 300

gttgccgaag ctgaaatggc gacgatggag aaaaaaggcg tcgatactgg ctttaaagct 360

gttcacccat taacgggcga agaaattccc gtttgggcag caaacttcgt attgatggag 420

tacggcacgg gcgcagttat ggcggttccg ggtcacgacc agcgcgacta cgagtttgcc 480

tctaaatacg gcctgaacat caaaccagtt atcctggctg ctgacggctc tgagccagat 540

ctttctcagc aagccctgac tgaaaaaggc gtgctgttca actctggcga attcaacggt 600

cttgaccatg aagcggcctt caacgccatc gccgataaac tg 642

<210> 644
<211> 642
<212> DNA
<213> Tatumella ptyseos T

<400> 644
cagtggttta tcaaaatcac cgactatgcc gaagaactgc tcaatgatct ggatactctg 60

gaaggctggc ctgaacaggt taagaccatg cagcgtaact ggatcggccg ttctgagggt 120

gtggaaatca cctttaatgt ggcagacagc gacgacacgc tgacggtcta cacgacccgc 180

cctgacacct ttatgggtgc cacctatgtg gctgtcgctg ccggacatcc tctggctcag 240

gaagctgcgg cgggtaaccc gtcactgcag gcttttattg acgagtgccg taacaccaaa 300

gttgctgaag cggatatggc aacgatggag aaaaagggtc agcctaccgg ccgttttgtt 360

atccatcctc tgaccggtga gcagttaccg gtctgggtcg ctaactttgt tctgatggaa 420

tacggtaccg gtgccgtcat ggccgtgcct gcccatgacc agcgtgacca cgaattcgct 480

caaaaatatc agttaccggt taaagcggtg atcctgaatg atgacgggac agcaccggat 540

gtcagcgaaa acgcgatgac ggtgaaaggt accctgttta actccggtgc cttcgacggg 600

ttatctcatc aggacggctt taatgccatt gctgatgcac tg 642

<210> 645
<211> 642
<212> DNA
<213> Trabulsiella guamensis T

<400> 645
cagtggttta tcaaaatcac cgcttacgcg gacgaactgc tcaatgatct ggataacctg 60

gatcactggc cagacaccgt taaaaccatg cagcgcaact ggatcggccg ttctgaaggg 120

gttgaaatct cgtttgacgt gaccggttat gccgacaagc tcaccgtcta caccacccgt 180

ccggacacct tcatgggttg tacctacctg gcggttgccg cgggtcatcc gctggcgcaa 240

caggcggcag cgaacaatcc tgagctggcg gcatttatcg acgaatgccg taacactaaa 300

gtggccgaag ccgatatggc gactatggag aagaaaggca tcgacaccgg gtttaaagcg 360

attcatccgc tgaccggcga agccattccg gtatgggccg ctaacttcgt gctgatggaa 420

tacggcactg gcgcagtgat ggccgttcca ggccatgatc agcgcgacta cgaatttgcc 480

agcaaatacg gtctgtccat caaaccggtc attctggcgg cagacggctc tgaaccggat 540

ctctccgaac aggcgctgac cgacaaaggc acgctgttta attccggcga gttcagcggt 600

ctgtcttatg aagacggttt caacgctatt gccgataaac tg 642

<210> 646
<211> 642
<212> DNA
<213> Xenorhabdus beddingii

<400> 646
caatggttca ttaaaatcac cgattatgct gaagaactgc tgaacgatct ggataaactg 60

gaagattggc ctgaacaggt caagatcatg caacgcaact ggattggccg ctctgaaggt 120

gtcgaaatca cgttcaatgt tgctgatagc caagaaaaac tcaccgttta taccactcgc 180

ccggatacgt tcatgggagc aacctatgtt gctgtcgccg ccggtcatcc actggcaaaa 240

caagccgcag aaaataaccc ggaactggca gaattcatca acgaatgccg caataccaaa 300

gttgccgaag cagatatggc aactatggag aaaaaaggcg tgccaacagg catgttcgcc 360

attcatccat tgaccaacga aaaaatcgcg atctgggtgg ctaactttgt cctgatggaa 420

tacggcactg gcgcggtgat ggccgtacct gcccacgatc aacgcgattg ggagtttgcg 480

accaagtaca gcctgccaat gaacgtggtg attctggaca acgacggcaa tatccccaat 540

attcaggatg ccgccgcaac agacaaaaat gcactggtta attccggtga attcagcgga 600

ctgaataatg aagagggctt caatgctatc gcagacaaac tg 642

<210> 647
<211> 642
<212> DNA
<213> Xenorhabdus innexi

<400> 647
cagtggttta ttaaaatcac cgattacgct gaagagttgc tgaatgatct ggataaactg 60

gaagaatggc cggagcaggt caaagttatg cagcgtaact ggattggccg ttcagaaggt 120

gctgaaatta cctttaatgt tgctgacagt caggaaacac tgaccgttta taccacccgt 180

ccagatacct ttatgggggc tacctatgtt gctgtagccg ctggtcatcc tctggccaaa 240

caggcagcag aaaataattc tgagctggca gactttatca acgaatgtcg taatactaaa 300

gttgctgaag ctgatatggc aacgatggag aagaaaggca tgtcaaccgg catgttcgtg 360

atccatccgt taactaacga aaaaattgct atctgggtag ccaacttcgt tctgatggaa 420

tacggcacag gtgcggttat ggcggttccg gcccatgatc agcgtgactg ggaatttgcg 480

actaaataca gcctgccaat gaaagccgtc attctggaca acgagggtaa cgtaccaaac 540

gttcaggaag gcgcatttac cgataagaac gctctggtca attccggcga attcagtggc 600

ctgagccacg aagaaggctt caatgccatc gctgataagc tg 642

<210> 648
<211> 642
<212> DNA
<213> Xenorhabdus szentirmaii

<400> 648
cagtggttca tcaaaattac cgcttacgca gaagaactgc taaacgatct ggacaagctg 60

gaagattggc ctgagcaggt caagatcatg cagcgcaact ggataggccg ttctgaaggt 120

gttgaaatca cgtttaatgt tgctgacagt caagaaaaac tcactgttta cacaactcgc 180

ccagacactt tcatgggggc aacctatgtt gctgtagcag cgggccatcc tctggctaag 240

caagcgactg aaaacaatcc ggcattggcc gagttcatta atgaatgtcg caataccaaa 300

gttgctgaag ctgatatggc gacaatggag aaaaaaggca tgtcaacggg catgttcgtg 360

attcatccat tgaccaatga aaaaatcgct atctgggttg ctaactttgt tctgatggaa 420

tacggcactg gtgcggtaat ggctgttcct gcacacgatc aacgtgactg ggaattcgcg 480

accaaatata acctgccgat gaaagccgtg attctggata atgacggtaa tgaaccaaac 540

attcaagaag ccgcactgac agacaaaaat gccctgtgca actccggtga attcgatgga 600

ttgagcaatg aagaaggttt caatgccatt gctgataaat tg 642

<210> 649
<211> 642
<212> DNA
<213> Yersinia aldovae

<400> 649
cagtggttta tcaaaatcac tgattatgct gatcagttgc tcaacgatct ggatacgctg 60

gaaagctggc cagaacaagt taagaccatg cagcgcaact ggattggccg ctccgaaggg 120

gtcgacattg tttttgatgt cgcggatagc gaagagaaac tctcagttta caccacccgc 180

ccggacacct ttatgggggt gacctacgta gccgttgcgg ccggtcaccc actggcgttg 240

caggccgctg cgactcaacc cacactggcc gattttgttg ccgaatgccg taataccaaa 300

gttgccgaag ctgaaatggc aacgatggag aaaaaaggca tggccactgg cctgtatgtc 360

atccatccac tcactggcga gaagctacct atctgggccg ctaacttcgt cttgatggat 420

tacggcaccg gtgcggtaat ggcagttcca ggccacgatg cccgtgattg ggaatttgct 480

accaaataca acctgccaat caaaccggtt attttagccg ctgatggcag cgagcctgac 540

ttaagtcagg aagccatgac cgaaaaaggc accctgttca actccggtga gtttgatggc 600

ctgaattatg aagatggctt taatgccatt gccgacaaac tg 642

<210> 650
<211> 642
<212> DNA
<213> Yersinia bercovieri

<400> 650
cagtggttta tcaaaatcac cgattacgct gatcagttgc ttaatgatct ggacacgctg 60

gaaagctggc ctgagcaggt gaaaaccatg caacgcaact ggattggccg ctccgaaggc 120

gttgatatcg tttttgaggt gctggatagc gaagagaaac tctctgttta taccacccgt 180

ccagatacct ttatgggcgt aacctacctg gcggttgctg ccggtcaccc tctgtcactg 240

caagcggctg aaaccaaccc tgcactggcc gattttgttg ctgaatgccg taacaccaag 300

gttgccgaag ctgaaatggc taccatggag aaaaaaggca tggcgaccgg tctctacgcc 360

atccacccgc tgactggcga gaaactggct atctgggctg ctaactttgt cttgatggat 420

tacggcactg gcgcagtcat gtccgttcca ggccacgatg cccgtgactg ggagttcgcc 480

accaaatacc acctgccaat caaagcggtt attttggccg cagacggcag cgaaccagat 540

gtcaccaaag aggccctgac cgaaaaaggc accctgttca attctggcga gtttgatggc 600

ctgaattatg aagatggctt taatgctatt gccgacaaac tg 642

<210> 651
<211> 642
<212> DNA
<213> Yersinia enterocolitica sbsp. palearctica

<400> 651
cagtggttta ttaaaattac tgattacgct gatcaattgc ttaatgatct ggaccggctg 60

gaaagctggc cagaacaagt taaaaccatg caacgtaact ggattggccg ctccgaaggg 120

gttgatatcg ttttcaacgt cgcagatagc gaagagaagc tctccgttta caccacgcgc 180

ccggacacct ttatgggtgt gacttacgta gctgttgctg ctggtcaccc gctgtcatta 240

caagccgcag cgactaaccc tgcactggct gattttgtcg cggaatgccg gaacaccaag 300

gttgccgaag ccgaaatggc caccatggag aaaaaaggca tggccaccgg cctgtatgcc 360

atccatccac tgacaggtga aaaattacct atctgggcgg ctaactttgt cttgatggat 420

tacggcaccg gtgcggtaat ggcggttccg ggccacgatg cccgcgactg ggaatttgcg 480

actaaatata atttgccaat caaaccggtt attttggcgg ctgatggcag tgaaccagac 540

ttgagccagg aagccatgac cgagaaaggc attctgttca actccggtga gtttgatggt 600

ctgaattatg aagatggctt caatgctgtt gccgacaaac ta 642

<210> 652
<211> 642
<212> DNA
<213> Yersinia frederiksenii

<400> 652
caatggttta tcaaaatcac ggattacgct gatcaactgc taaatgatct ggacacgctg 60

gaaagctggc cagaacaagt taaaaccatg cagcgtaact ggattggccg ctctgaaggt 120

gtcgatatcg ttttcgacgt agtcgatagc gaagacaaac tctcggtcta caccactcgc 180

ccagacacct ttatgggtgt gacttacgtg gctgtcgctg caagtcatcc tttatcactg 240

caagctgctg ccactaatcc ggcactggct gattttgttg ccgaatgccg taataccaag 300

gttgccgaag ctgaaatggc gaccatggag aaaaaaggca tgccgaccgg cctatatgcc 360

atccatccgc tgaatggcga gaagctacct atttgggcgg ctaactttgt cttgatggat 420

tacggtactg gtgcggttat ggcggttcct ggccatgatg cccgcgacta tgaatttgcg 480

accaaatata gcctgccaat caaagcggtt attctagcgg ctgatggcag tgagccagat 540

ttaagtcaag aagcattgac cgagaaaggc acattgttta actctggcga atttgatggc 600

ctgagttatg aagatggttt taatgccatt gccgataaac tg 642

<210> 653
<211> 642
<212> DNA
<213> Yersinia intermedia

<400> 653
cagtggttta ttaaaatcac cgattacgct gatcagttgc ttaatgatct ggacacgctg 60

gaaagctggc cggagcaggt gaaaaccatg caacgcaact ggattggccg ctccgaaggg 120

gttgatatcg ttttcgacgt gcaggatagc gaagaaaaac tgtcagttta tactacccgt 180

ccagacacct ttatgggcgt gacctatgtg gcggttgctg ccggtcaccc attgtcactg 240

caagcggctg ccaccaaccc agcgctggct gattttgtgg cggaatgtcg taataccaaa 300

gttgctgaag ctgaaatggc aacgatggag aaaaaaggca tggcgaccgg tctatacgcc 360

atccatccac tgacaggtga aaagctggct atttgggcag ctaactttgt cttgatggat 420

tacggtaccg gcgcagtaat ggcagttcca ggccacgatg cccgtgactg ggaatttgcc 480

accaaatacc acctgccaat caagccggtt attttagccg ctgacggcag cgaacctgat 540

ttgagtcagg aagcgatgac cgaaaaaggc accctgttca actccggcga gttcgacggt 600

ctggattatg aagcaggctt taatgccatc gccgacaagc ta 642

<210> 654
<211> 642
<212> DNA
<213> Yersinia mollaretii

<400> 654
cagtggttta tcaaaatcac cgattacgct gatcagttgc ttaatgatct ggacacgctg 60

gaaagctggc ctgagcaggt gaaaaccatg cagcgcaact ggattggccg ctccgaaggg 120

gttgatatcg ttttcgatgt cgcggatagc gaagataaac tgtctgttta taccacccgt 180

ccagatacct ttatgggcgt gacctatgtg gcggttgctg caggtcatcc tctgtcactg 240

caagcggctg aaaccaaccc tgcactggcc gattttgttg cagaatgccg taataccaag 300

gttgccgaag ctgaaatggc caccatggag aaaaaaggca tggcgacagg tctgtatgcc 360

atccatccgc taactggcga gaaattagct atctgggccg ctaactttgt cttgatggat 420

tacggtactg gtgcggtcat ggccgttcca ggccacgatg ctcgtgactg ggagttcgcc 480

accaaataca acctaccaat caaaccggtt attttggccg cagacggtag cgaacctgat 540

gtcacgaaag aagccctgac cgagaaaggc gtcctgttca actctggcga gtttgatggt 600

ttggattacg aagagggctt taatgccatc gccgacaaac tg 642

<210> 655
<211> 642
<212> DNA
<213> Yersinia pestis T

<400> 655
cagtggttta tcaaaatcac cgattacgct gagcagttgc tcaacgatct ggacacgctg 60

gaaagctggc cagagcaggt gaaaaccatg caacgtaact ggattggccg ctctgaaggg 120

gtcgatatcg ttttcgacgt cgtggatagc gaagagaaac tgtctgttta taccacccgg 180

ccagacacct ttatgggcgt gacctacgta gcggttgctg ctggtcatcc tttgtcattg 240

caagcggcgg cgactaatcc ggccctggct gatttcgttg ctgaatgccg taataccaag 300

gttgccgaag ctgaaatggc aaccatggag aaaaaaggca tggcgacggg tctgtatgcc 360

atccatccat taactggcga gaaactgcct atatgggccg ctaactttgt cttgatggat 420

tacggtactg gcgcagtgat ggccgttccg ggccacgatg cacgtgactg ggaatttgcc 480

accaaatata acctgccaat caaaccggtt attttggccg cggatggcag tgagccagac 540

ttgagtcagg aagccatgac cgagaaaggc acgctgttta actctggtga gtttgatggc 600

ctgaatcatg aagatggttt taatgccatt gccgataaac tg 642

<210> 656
<211> 642
<212> DNA
<213> Yersinia pseudotuberculosis sbsp. pseudotuberculosis

<400> 656
cagtggttta tcaaaatcac cgattacgct gagcagttgc tcaacgatct ggacacgctg 60

gaaagctggc cagagcaggt gaaaaccatg caacgtaact ggattggccg ctctgaaggg 120

gtcgatatcg ttttcgacgt cgtggatagc gaagagaaac tgtctgttta taccacccgg 180

ccagacacct ttatgggcgt gacctacgta gcggttgcag ctggtcatcc tttgtcattg 240

caagcggcgg cgactaatcc ggctctggct gatttcgttg ctgaatgccg taataccaag 300

gttgccgaag ctgaaatggc aaccatggag aaaaaaggca tggcgacggg tctgtatgcc 360

atccatccat taactggcga gaaactgcct atctgggccg ctaactttgt cttgatggat 420

tacggtactg gcgcagtgat ggccgttccg ggccacgatg cacgtgactg ggaatttgcc 480

accaaatata acctgccaat caaaccggtt attttggccg cggatggcag tgagccagac 540

ttgagtcagg aagccatgac cgagaaaggc acgctgttta actctggtga atttgatggc 600

ctgaatcatg aagatggttt taatgccatt gccgataaac tg 642

<210> 657
<211> 642
<212> DNA
<213> Yersinia rohdei

<400> 657
cagtggttta tcaaaatcac agattatgct gaccaattgc tgaacgacct ggatacgctg 60

gaaagctggc cagagcaagt taagaccatg cagcgtaact ggattggccg ctctgaaggg 120

gttgatatcg ttttcgacgt cgccgatagc gaagagaaac tctccgttta taccacacgt 180

ccagacacct ttatgggtgt gacttacctg gctgttgctg cgggtcatcc attgtcattg 240

caagccgctg ccgctaatcc tgcgctggct gattttgttg ctgaatgccg taataccaag 300

gttgccgagg ctgaaatggc gaccatggag aaaaaaggca tggcaaccgg cctgtatgca 360

atccatccac tgaccggtga aaaactgcct atctgggcgg ctaactttgt cttgatggat 420

tacggcactg gcgctgtcat ggcagttcct ggccacgatg cgcgtgacta tgaatttgcg 480

accaaatata acctgccaat caaaccggtt attttagccg ctgatggcag tgagccagac 540

ttgagtcagg aagccatgac cgaaaaaggc accttgttta actctggcga atttgatggc 600

ctgagttatg aagatggttt taatgccata gccgataaac tg 642

<210> 658
<211> 642
<212> DNA
<213> Yersinia ruckeri

<400> 658
cagtggttta tcaaaatcac tgattatgcc gatcagttgc tgaacgatct ggataagctg 60

gaaagctggc cagagcaggt gaaaaccatg cagcgcaact ggattggccg ttctgaaggg 120

gtggaaatca cctttgatgt caccgatagc gcagaaaaac tgacggtcta taccacccgc 180

ccagatacct tctttggtgt gacctatgta gccgttgctg cgggtcatcc tttggcactg 240

caagccgcta aaaccaatcc ggcactggcc gagtttgttg atgaatgccg caataccaaa 300

gtcgctgagg ccgaaatggc tacgatggag aagaaaggca tggctaccgg cctgtcggcc 360

atccatccgt taaccggtga aaccgtggct atctgggtcg ccaactttgt gttgatggac 420

tacggtactg gtgcagtaat ggccgttccg ggtcacgatc agcgcgactg ggaattcgca 480

accaaataca atctgccgat caagcccgtg attctgtccg ctgacggtag tgcaccagat 540

ttaagcactg cgccactgac cgaaaaaggc acgctgttca actcaggtga gtttgacggc 600

ctggattacc aggccagctt tgatgctatt gccgataagc tg 642

<210>  659
<211>  642
<212>  DNA
<213>  Yokenella regensburgei T

<400>  659
cagtggttta ttaaaatcac cgcctacgct gacgagctgt tgaacgacct ggataagctg      60

gatcactggc cggatacggt gaagaccatg cagcgtaact ggattggtcg ttctgaaggg     120

gttgaaatct ctttcaacgt caacaactac gccgacaaac tgactgttta caccacccgc     180

ccggatacct tcatgggctg cacctacctg gcggtagctg cgggtcaccc gctggcgcag     240

caggctgcag caaacaatcc agagctggcc gcgtttatcg atgaatgccg taataccaaa     300

atggccgaag ccgacatggc gaccatggag aaaaaaggca tcgacaccgg ctttaaagca     360

attcatccgc tgaccggtga agaaattccg gtttgggcgg caaacttcgt gctgatggaa     420

tacggcaccg gtgcggtgat ggccgttccg ggtcacgacc agcgcgatta cgaatttgcg     480

accaaatatg gtctgaacat caagccggtt attctggccg ccgatggttc tgagccggac     540

ctctccgagc aggcactgac cgagaaaggc gtactgttta actccggtga gtttgacggc     600

ctgagccatg aagcgggctt caacgcgatt gctgacaaac tg                        642

<210>  660
<211>  333
<212>  DNA
<213>  Brenneria alni

<400>  660
cgttacatcc tggcgccgaa aggccttaaa gcaggtgacc agattcaatc tggcgttgat      60

gctgcaatca aaaccggtaa caccttgccg atgcgtaaca tccctgtcgg ttcaacggtt     120

cataacgtag aaatgaaacc aggtaagggc ggccaactgg ctcgttctgc tggagcttac     180

gttcagatcg ttgctcgtga tggtgcttac gttaccctgc gtctgcgttc tggcgaaatg     240

cgtaaagtcg agtctgactg ccgcgctacg ctgggcgaag ttggtaatgc ggagcatatg     300

ctacgcgtgc tgggtaaagc aggtgctgct cgc                                  333

<210>  661
<211>  333
<212>  DNA
<213>  Brenneria quercina

<400>  661
cgttacatcc tggcacctaa aggcctgaaa gccggtgatc agattcagtc tggtgttgat      60

gctgcaatca agccgggtaa caccctgcct atgcgtaaca tcccggttgg ttcaacggtt     120

cataacgtag aaatgaaacc aggtaaaggc ggccagctgg ctcgctcagc cggtgcctac     180

gttcagatcg ttgcccgcga cggttcttac gtcaccctgc gtctgcgctc tggtgaaatg     240

cgtaaagttg aagctgactg ccgcgcaaca ctgggtgaag tcggtaacgc tgagcacatg     300

ctgcgcgttc tgggtaaagc tggtgctgct cgc                                  333

<210>  662

<211>  333
<212>  DNA
<213>  Brenneria rubrifaciens

<400>  662
cgttacatcc tggcgccgaa aggcctgaaa gcaggtgacc agattcaatc tggcgttgat      60

gctgcaatta aaaccggtaa caccttgccg atgcgtaaca tccctgtcgg ttcaacggtt     120

cataacgtag aaatgaaacc aggtaagggc ggccaactgg ctcgttccgc cggtgcctac     180

gttcagatcg ttgctcgtga cggttcctac gtgaccctgc gcctgcgttc cggcgaaatg     240

cgtaaagtcg aatctgactg tcgcgctacg ctgggcgaag taggtaacgc tgagcatatg     300

ctgcgcgttc tgggtaaagc gggcgctgca cgc                                  333

<210>  663
<211>  333
<212>  DNA
<213>  Brenneria salicis T

<400>  663
cgttacatcc tggcgccgaa aggcctgaaa gccggtgacc agattcaatc tggcgttgat      60

gctgcaatca aacccggtaa caccctgccg atgcgtaaca ttcctgtcgg ttcaacggtt     120

cacaacgtag aaatgaaacc aggtaaaggc ggccagttgg cgcgttctgc cggtgcctac     180

gttcagatcg ttgctcgcga aggcgcttac gttaccctgc gtctgcgttc cggcgaaatg     240

cgtaaagtcg aatccgactg ccgcgcgacg ctgggcgaag ttggcaatgc ggagcatatg     300

ctacgcgttc tgggtaaagc aggtgctgca cgc                                  333

<210>  664
<211>  333
<212>  DNA
<213>  Budvicia aquatica T

<400>  664
cgttatatcc tagcgcctaa gagcctaaaa gcaggtgacc aaatccaatc tggtgttgat      60

gctgcaatta aagtgggtaa ctgcctaccg atgcgtaaca tcccagtggg ttctacggtt     120

cacaacgttg aaatgaaacc aggtaaaggc ggccaaatgg ctcgttctgc tggcggatat     180

gttcaaatcg ttgcccggga aggttcctac gtaactatac gtcttcgctc cggtgaaatg     240

cgtaaagtat tgtctgattg ccgcgccacc ttaggtgaag ttggtaactc agaacatatg     300

cttcgcgtgc ttggtaaagc tggtgctagt cgt                                  333

<210>  665
<211>  333
<212>  DNA
<213>  Buttiauxella agrestis T

<400>  665
cgttacatcc tggcccctaa aggcctgaaa gctggcgacc agattcaatc tggcgttgat      60

gctgcaatca aagcaggtaa caccctgccg atgcgcaata tcccggttgg ttctaccgtt     120

cataacgtag aaatgaaacc aggtaaaggc ggtcagcttg cacgttctgc tggtacctat     180

gtgcagatcg ttgcacgtga tggtgcttac gttaccctgc gtctgcgttc tggtgaaatg 240

cgtaaagtcg aagctgactg ccgtgcaacc atgggcgaag tcggtaactc tgagcatatg 300

ctccgcgttc tgggtaaagc aggtgctgca cgc 333

<210> 666
<211> 333
<212> DNA
<213> Buttiauxella brennerae

<400> 666
cgttacatcc tggcccctaa aggcctgaaa gctggcgacc agattcaatc tggcgttgat 60

gctgcaatca aagcaggtaa caccctgccg atgcgcaata tcccggttgg ttctaccgtt 120

cataacgtag aaatgaaacc aggtaaaggc ggtcagcttg cacgttctgc tggtacctat 180

gtgcagatcg ttgcacgtga tggtgcttac gttaccctgc gtctgcgttc tggtgaaatg 240

cgtaaagtcg aagctgactg ccgtgcaacc atgggcgaag tcggcaactc tgagcatatg 300

ctccgcgttc tgggtaaagc aggtgctgct cgc 333

<210> 667
<211> 333
<212> DNA
<213> Buttiauxella ferragutiae

<400> 667
cgttacatcc tggcccctaa aggcctgaaa gctggcgacc agattcaatc tggcgttgat 60

gctgcaatca aagcaggtaa caccctgccg atgcgcaata tcccggttgg ttctaccgtt 120

cataacgtag aaatgaaacc aggtaaaggc ggtcagcttg cacgttctgc tggtacctat 180

gtgcagatcg ttgcacgtga tggtgcttac gttaccctgc gtctgcgttc tggtgaaatg 240

cgtaaagtcg aagctgactg ccgtgcaacc atgggcgaag tcggtaactc tgagcatatg 300

ctccgcgttc tgggtaaagc aggtgctgca cgc 333

<210> 668
<211> 333
<212> DNA
<213> Buttiauxella gaviniae

<400> 668
cgttacatcc tggcccctaa aggcctgaaa gctggcgacc agattcaatc tggcgttgat 60

gctgcaatca aagcaggtaa caccctgccg atgcgcaata tcccggttgg ttctaccgtt 120

cataacgtag aaatgaaacc aggtaaaggc ggtcagcttg cacgttctgc tggtacctat 180

gtgcagatcg ttgcacgtga tggtgcttac gttaccctgc gtctgcgttc tggtgaaatg 240

cgtaaagtcg aagctgactg ccgtgcaacc atgggcgaag tcggtaactc tgagcatatg 300

ctccgcgttc tgggtaaagc aggtgctgca cgc 333

<210> 669
<211> 333
<212> DNA
<213> Buttiauxella izardii

<400>  669
cgttacatcc tggcccctaa aggcctgaaa gctggcgacc agattcaatc tggcgttgat      60

gctgcaatca aagcaggtaa caccctgccg atgcgcaata tcccggttgg ttctactgtt     120

cataacgtag aaatgaaacc aggtaaaggc ggtcagcttg cacgttctgc tggtacctat     180

gtgcagatcg ttgcacgtga tggtgcttac gttaccctgc gtctgcgttc tggtgaaatg     240

cgtaaagtcg aagctgactg ccgtgcaacc atgggcgaag tcggtaactc tgagcatatg     300

ctccgcgttc tgggtaaagc aggtgctgct cgc                                  333

<210>  670
<211>  333
<212>  DNA
<213>  Buttiauxella noackiae

<400>  670
cgttacatcc tggcccctaa aggcctgaaa gctggcgacc agattcaatc tggcgttgat      60

gctgcaatca aagcaggtaa caccctgccg atgcgcaata tcccggttgg ttctaccgtt     120

cataacgtag aaatgaaacc aggtaaaggc ggtcagctgg ctcgttctgc tggtacctat     180

gtgcagatcg ttgcacgtga tggtgcttac gttaccctgc gtctgcgttc tggtgaaatg     240

cgtaaagtcg aagctgactg ccgtgcaacc atgggcgaag tcggtaactc tgagcatatg     300

ctccgcgttc tgggtaaagc aggtgctgct cgc                                  333

<210>  671
<211>  333
<212>  DNA
<213>  Buttiauxella warmboldiae

<400>  671
cgttacatcc tggcccctaa aggcctgaaa gctggcgacc agattcaatc tggcgttgat      60

gctgcaatca aagctggtaa caccctgccg atgcgcaaca tcccggttgg ttctacagtg     120

cataacgtag aaatgaaacc aggtaaaggc ggtcagctgg ctcgttccgc gggtacctat     180

gtgcagatca ttgcacgtga tggtgcttac gttaccctgc gtctgcgttc tggtgaaatg     240

cgtaaagttg aagctgactg ccgcgcaaca ctgggcgaag ttggcaatgc tgagcatatg     300

ctgcgcgttc tgggtaaagc gggtgctgct cgc                                  333

<210>  672
<211>  333
<212>  DNA
<213>  Cedecea davisae  T

<400>  672
cgttacatcc tggcccctaa aggcctgaaa gctggcgacc agattcagtc tggcgttgat      60

gctgcaatca aagctggtaa caccctgccg atgcgcaaca tcccggttgg ttctactgtt     120

cataacgtag aaatgaaacc tggtaaaggc ggtcagctgg cacgttccgc tggtacctac     180

gtgcagatcg ttgcgcgtga tggtgcttac gttaccctgc gtctgcgttc tggtgaaatg     240

cgtaaagtcg aagctgactg ccgcgcgacc ctgggcgaag ttggcaacgc tgagcatatg     300

ctgcgcgttc tgggtaaagc gggtgctgct cgc 333

<210> 673
<211> 333
<212> DNA
<213> Cedecea lapagei

<400> 673
cgttacatcc tggcccctaa aggcctgaaa gctggcgacc agattcagtc tggcgttgat 60

gctgcaatca aagcgggtaa caccctgccg atgcgcaata tcccggttgg ttctaccgtt 120

cataacgtag aaatgaaacc aggtaaaggc ggtcagctgg ctcgttccgc tggtacctac 180

gtgcagatcg ttgctcgtga tggtgcttac gttaccctgc gcctgcgctc cggtgaaatg 240

cgtaaagtcg aagctgactg ccgcgctacc ctgggcgaag ttggcaacgc tgagcatatg 300

ctgcgcgttc tgggtaaagc aggtgctgct cgc 333

<210> 674
<211> 333
<212> DNA
<213> Cedecea neteri

<400> 674
cgttacatcc tggcccctaa aggcctgaaa gctggcgacc agattcagtc tggcgttgat 60

gctgcaatca aagcgggtaa caccctgccg atgcgcaata tcccggttgg ttctaccgtt 120

cataacgtag aaatgaaacc aggtaaaggc ggtcagctgg ctcgttccgc tggtacctac 180

gtgcagatcg ttgctcgtga tggtgcttac gttaccctgc gcctgcgctc cggtgaaatg 240

cgtaaagtcg aagctgactg ccgcgctacc ctgggcgaag ttggcaacgc tgagcatatg 300

ctgcgcgttc tgggtaaagc aggtgctgct cgc 333

<210> 675
<211> 333
<212> DNA
<213> Citrobacter amalonaticus

<400> 675
cgttacatcc tggcccctaa aggcctgaaa gcgggcgacc agatccaatc tggcgttgat 60

gctgcaatca aaccaggcaa caccctgccg atgcgcaata tcccggttgg ttctaccgtt 120

cataacgtag aaatgaaacc aggtaaaggc ggtcagctgg cacgttccgc tggtacttac 180

gttcagatcg ttgctcgtga tggtgcttat gtcaccctgc gtctgcgttc tggtgaaatg 240

cgtaaagtcg aagcagactg ccgtgcaact ctgggcgaag ttggcaatgc tgagcatatg 300

ctgcgcgttc tgggtaaagc aggtgctgca cgc 333

<210> 676
<211> 333
<212> DNA
<213> Citrobacter braakii

<400> 676
cgttacatcc tggcccctaa aggcctgaaa gctggcgacc aggttcaatc tggcgttgat 60

gctgcaatca aagcaggtaa caccctgccg atgcgcaata tcccggttgg ttctaccgtt 120

cataacgtag aaatgaaacc aggtaaaggc ggtcagctgg cacgttccgc tggtacttac 180

gttcagatcg ttgctcgtga tggtgcttat gtcaccctgc gtctgcgttc tggtgaaatg 240

cgtaaagtcg aagcagactg ccgtgcaact ctgggcgaag ttggcaatgc tgagcatatg 300

ctgcgcgttc tgggtaaagc aggtgctgca cgc 333

<210> 677
<211> 333
<212> DNA
<213> Citrobacter diversus

<400> 677
cgttacatcc tggcccctaa aggcctgaaa gctggcgacc agattcaatc tggcgttgat 60

gctgcaatca aaccaggcaa caccctgccg atgcgcaaca tcccggttgg ttctactgtt 120

cataacgtag aaatgaaacc aggtaaaggc ggtcagctgg cacgttccgc tggtacttac 180

gttcagatcg ttgcgcgcga tggtgcttat gtcaccctgc gtctgcgttc tggtgaaatg 240

cgtaaagtcg aagcagactg ccgtgcaact ctgggcgaag ttggcaatgc tgagcatatg 300

ctgcgcgttc tgggtaaagc aggtgctgca cgc 333

<210> 678
<211> 333
<212> DNA
<213> Citrobacter farmeri

<400> 678
cgttacatcc tggcccctaa aggcctgaaa gctggcgacc agatccaatc tggcgttgat 60

gctgcaatca aaccaggcaa caccctgccg atgcgcaata tcccggttgg ttctaccgtt 120

cataacgtag aaatgaaacc aggtaaaggc ggtcagctgg cacgttccgc tggtacttac 180

gttcagatcg ttgcgcgtga tggtgcttat gtcaccctgc gtctgcgttc tggtgaaatg 240

cgtaaagtcg aagcagactg ccgtgcaact ctgggcgaag ttggcaatgc tgagcatatg 300

ctgcgcgttc tgggtaaagc aggtgctgca cgc 333

<210> 679
<211> 333
<212> DNA
<213> Citrobacter freundii T

<400> 679
cgttacatcc tggcccctaa aggcctgaaa gctggcgacc agattcagtc tggcgttgat 60

gctgcaatca aagcaggtaa caccctgccg atgcgcaata tcccggttgg ttctaccgtt 120

cataacgtag aaatgaaacc aggtaaaggc ggtcagctgg cacgttccgc tggtacttac 180

gttcagatcg ttgctcgtga tggtgcttat gtcaccctgc gtctgcgttc tggtgaaatg 240

cgtaaagtcg aagcagactg ccgtgcaact ctgggcgaag ttggcaatgc tgagcatatg 300

ctgcgcgttc tgggtaaagc aggtgctgca cgc 333

<210> 680
<211> 333
<212> DNA
<213> Citrobacter gillenii

<400> 680
cgttacatcc tggcccctaa aggcctgaaa gctggcgacc aggttcaatc tggcgttgat 60

gctgcaatca aagcaggtaa caccctgccg atgcgcaata tcccggttgg ttctaccgtt 120

cataacgtag aaatgaaacc aggtaaaggc ggtcagctgg cacgttccgc tggtacttac 180

gttcagatcg ttgctcgtga tggtgcttat gtcaccctgc gtctgcgttc tggtgaaatg 240

cgtaaagtcg aagcagactg ccgtgcaact ctgggcgaag ttggcaatgc tgagcatatg 300

ctgcgcgttc tgggtaaagc aggtgctgca cgc 333

<210> 681
<211> 333
<212> DNA
<213> Citrobacter koseri

<400> 681
cgttacatcc tggcccctaa aggcctgaaa gctggcgacc agattcaatc tggcgttgat 60

gctgcaatca aaccaggcaa caccctgccg atgcgcaaca tcccggttgg ttctaccgtt 120

cataacgtag aaatgaaacc aggtaaaggc ggtcagctgg cacgttccgc tggtacttac 180

gttcagatcg ttgcgcgcga tggtgcttat gtcactctgc gtctgcgttc tggtgaaatg 240

cgtaaagtcg aagcagactg ccgtgcaact ctgggcgaag ttggcaatgc tgagcatatg 300

ctgcgcgttc tgggtaaagc aggtgctgca cgc 333

<210> 682
<211> 333
<212> DNA
<213> Citrobacter murliniae

<400> 682
cgttacatcc tggcccctaa aggcctgaaa gctggcgacc agattcagtc tggcgttgat 60

gctgcaatca aagcaggtaa caccctgccg atgcgcaata tcccggttgg ttctaccgtt 120

cataacgtag aaatgaaacc aggtaaaggc ggtcagctgg ctcgttccgc tggtacttac 180

gttcagatcg ttgctcgtga tggtgcttat gtcaccctgc gtctgcgttc tggtgaaatg 240

cgtaaagtcg aagcagactg ccgtgcaact ctgggcgaag ttggcaatgc tgagcatatg 300

ctgcgcgttc tgggtaaagc aggtgctgca cgc 333

<210> 683
<211> 333
<212> DNA
<213> Citrobacter rodentium

<400> 683
cgttacattc tggcccctaa aggcctgaaa gctggcgacc agatccaatc tggcgttgat 60

gctgcaatta aaccaggcaa caccctgccg atgcgtaata tcccggttgg ttctaccgtt 120

cataacgtag aaatgaaacc aggtaaaggc ggtcagctgg cgcgttccgc gggtacttac 180

gttcagatcg tcgcacgtga cggtgcttat gtcaccctgc gtctgcgttc tggtgaaatg 240

cgtaaagtcg aagcagactg ccgtgcgact ctgggcgaag ttggcaatgc tgagcatatg 300

ctgcgcgttc tgggtaaagc aggtgctgca cgc 333

<210> 684
<211> 333
<212> DNA
<213> Citrobacter sedlakii

<400> 684
cgttacatcc tggcccctaa aggcctgaaa gctggcgacc agatccaatc tggcgttgat 60

gctgcaatca aaccaggcaa caccctgccg atgcgcaata tcccggttgg ttctaccgtt 120

cataacgtag aaatgaaacc aggtaaaggc ggtcagctgg cacgttccgc agggacttac 180

gttcagatcg ttgcgcgcga tggtgcttat gtcaccctgc gtctgcgttc tggtgaaatg 240

cgtaaagtcg aagcagactg ccgtgcaact ctgggcgaag ttggcaatgc tgagcatatg 300

ctgcgcgttc tgggtaaagc aggtgctgca cgt 333

<210> 685
<211> 333
<212> DNA
<213> Citrobacter werkmanii

<400> 685
cgttacatcc tggcccctaa aggcctgaaa gctggcgacc agattcagtc tggcgttgat 60

gctgcaatca aagcaggtaa caccctgccg atgcgcaata tcccggttgg ttctaccgtt 120

cataacgtag aaatgaaacc aggtaaaggc ggtcagctgg cacgttccgc tggtacttac 180

gttcagatcg ttgctcgtga tggtgcttat gtcaccctgc gtctgcgttc tggtgaaatg 240

cgtaaagtcg aagcagactg ccgtgcaact ctgggcgaag ttggcaatgc tgagcatatg 300

ctgcgcgttc tgggtaaagc aggtgctgca cgc 333

<210> 686
<211> 333
<212> DNA
<213> Citrobacter youngae

<400> 686
cgttacatcc tggcccctaa aggcctgaaa gctggcgacc agattcagtc tggcgttgat 60

gctgcaatca aagcaggtaa caccctgccg atgcgcaata tcccggttgg ttctaccgtt 120

cataacgtag aaatgaaacc aggtaaaggc ggtcagctgg cacgttccgc tggtacttac 180

gttcagatcg ttgctcgtga tggtgcttat gtcaccctgc gtctgcgttc tggtgaaatg 240

cgtaaagtcg aagcagactg ccgtgcaact ctgggcgaag ttggcaatgc tgagcatatg 300

ctgcgcgttc tgggtaaagc aggtgctgca cgc 333

<210> 687
<211> 333

<212>  DNA
<213>  Dickeya chrysanthemi T

<400>  687
cgttacatcc tggcgccgaa aggcctgaaa gcaggtgacc agattcagtc tggtgttgat      60

gctgcaatca aaaccggtaa taccctgccg atgcgtaaca tcccggtcgg ttcaacggtt     120

cacaacgtag aaatgaaacc gggtaaaggc ggccagttgg cgcgctctgc tggtgcctac     180

gttcagatcg ttgctcgtga aggtgcttac gttaccctgc gtctgcgctc tggcgaaatg     240

cgtaaagtcg aatccgactg ccgcgcaacg ctgggcgaag tcggcaatgc cgagcatatg     300

ctgcgcgttc tgggtaaagc aggtgctgca cgc                                  333

<210>  688
<211>  333
<212>  DNA
<213>  Dickeya dadantii

<400>  688
cgttatatcc tggcgccgaa aggcctgaaa gcaggtgacc agattcagtc tggtgttgat      60

gctgtaatca aaaccggtaa caccctgccg atgcgtaaca tcccggtggg ttcaacggtt     120

cacaacgtag aaatgaaacc gggtaaaggc ggccagttgg ctcgctcagc cggtgcctac     180

gttcagatcg ttgcccgtga aggtgcttac gttaccctgc gtctgcgttc cggcgaaatg     240

cgtaaagtcg aatccgactg ccgcgcaacg ctgggcgaag tcggcaacgc cgagcacatg     300

ctgcgcgttc tgggtaaagc tggggctgca cgc                                  333

<210>  689
<211>  333
<212>  DNA
<213>  Edwardsiella hoshinae

<400>  689
cgttacatcc tggcgccgaa aggcctgaaa gctggcgatc agattcaatc tggcgtcgac      60

gctgcgatca aggctggtaa caccctgccg atgcgcaaca ttccggttgg ttctaccgtt     120

cataacgtag aaatgaagcc gggtaaaggt ggccagctgg ctcgttccgc tggtgcttac     180

gttcagatcg ttgcgcgtga aggtgcttat gtcactctgc gtctgcgctc tggcgaaatg     240

cgtaaaatcc cggctgactg ccgcgccact ctgggcgaag tcggtaacgc tgagcacatg     300

ctgcgcgttc tgggtaaagc aggtgctgca cgc                                  333

<210>  690
<211>  333
<212>  DNA
<213>  Edwardsiella ictaluri

<400>  690
cgttatatcc tggcggcgaa aggcctgaaa gctggcgatc agattcagtc tggcgttgat      60

gctgcaatca aagcgggtaa caccctgccg atgcgcaata tcccggttgg ttctaccgtt     120

cataacgtag aaatgaagcc gggtaaaggc ggccagctgg cgcgttccgc tggtgcttac     180

gttcagatcg ttgcacgcga tggtgcctat gtcaccctgc gtctacgttc tggcgaaatg     240

cgtaaaatcc cggctgactg ccgcgccact ctgggcgaag tcggtaacgc tgagcatatg 300

ctgcgcgttc tgggtaaagc aggtgctgca cgc 333

<210> 691
<211> 333
<212> DNA
<213> Edwardsiella tarda T

<400> 691
cgttacatcc tggcaccgaa aggcctgaaa gctggcgatc agattcaatc tggcgtcgac 60

gctgcgatca aggcgggtaa caccctgccg atgcgcaaca tcccggttgg ttctaccgtt 120

cataacgtag aaatgaagcc gggtaaaggc ggccagctgg ctcgttccgc tggtgcttac 180

gttcagatcg ttgcgcgtga aggtgcttat gtcaccctgc gtctgcgttc tggcgaaatg 240

cgcaaaatcc cggctgactg ccgcgctact ctgggcgaag tcggtaacgc tgagcatatg 300

ctgcgcgttc tgggtaaagc aggtgctgca cgc 333

<210> 692
<211> 333
<212> DNA
<213> Enterobacter aerogenes

<400> 692
cgttacattc tggccccgaa aggcctgaaa gctggcgacc agattcagtc tggcgttgat 60

gctgcaatca aagcaggcaa caccctgccg atgcgcaata tcccggttgg ttctaccgtt 120

cataacgtag aaatgaaacc aggtaaaggc ggtcagctgg ctcgttccgc tggtacttac 180

gttcagatcg ttgctcgtga cggtgcttat gtcaccctgc gtctgcgttc tggtgaaatg 240

cgtaaagtcg aagcagactg ccgcgctact ctgggcgaag ttggcaatgc tgagcatatg 300

ctgcgcgttc tgggtaaagc aggtgctgca cgc 333

<210> 693
<211> 333
<212> DNA
<213> Enterobacter amnigenus

<400> 693
cgttacatcc tggcccctaa aggcctgaaa gctggcgacc agattcaatc tggcgttgat 60

gctgcaatca aagcaggcaa caccctgccg atgcgcaata tcccggttgg ttctaccgtt 120

cataacgtag aaatgaaacc aggtaaaggc ggtcagctgg cacgttccgc tggtacttac 180

gttcagatcg ttgctcgtga tggtgcttat gtcaccctgc gtttgcgttc tggtgaaatg 240

cgtaaagtcg aagcagactg ccgcgctact ctgggcgaag ttggcaatgc tgagcatatg 300

ctgcgcgttc tgggtaaagc aggtgctgca cgc 333

<210> 694
<211> 333
<212> DNA
<213> Enterobacter asburiae

<400>  694
cgttacatcc tggcccctaa aggcctgaaa gctggcgacc agattcagtc tggcgttgat      60

gctgcaatca aagcaggcaa caccctgccg atgcgcaata tcccggttgg ttctaccgtt     120

cataacgtag aaatgaaacc aggtaaaggc ggtcagctgg cacgttccgc tggtacttac     180

gttcagatcg ttgctcgtga tggtgcttat gtcaccctgc gtctgcgttc tggtgaaatg     240

cgtaaagtcg aagcagactg ccgcgctact ctgggcgaag ttggcaatgc tgagcatatg     300

ctgcgcgttc tgggtaaagc aggtgctgca cgc                                  333

<210>  695
<211>  333
<212>  DNA
<213>  Enterobacter cancerogenus

<400>  695
cgttacatcc tggcccctaa aggcctgaaa gctggcgacc agattcagtc tggcgttgat      60

gctgcaatca aagcaggtaa caccctgccg atgcgcaata tcccggttgg ttctaccgtt     120

cataacgtag aaatgaaacc aggtaaaggc ggtcagctgg cacgttccgc tggtacttac     180

gttcagatcg ttgcgcgtga tggtgcttat gtcaccctgc gtctgcgttc tggtgaaatg     240

cgtaaagtcg aagcagactg ccgcgctact ctgggcgaag ttggcaatgc tgagcatatg     300

ctgcgcgttc tgggtaaagc aggtgctgca cgc                                  333

<210>  696
<211>  333
<212>  DNA
<213>  Enterobacter cloacae sbsp. cloacae T

<400>  696
cgttacatcc tggcccctaa aggcctgaaa gctggcgacc agattcagtc tggcgttgat      60

gctgcaatca aagcaggcaa caccctgccg atgcgcaata tcccggttgg ttctaccgtt     120

cataacgtag aaatgaaacc aggtaaaggc ggtcagctgg cgcgttccgc gggtacttac     180

gttcagatcg ttgcgcgtga cggtgcttat gtcaccctgc gtctgcgttc tggtgaaatg     240

cgtaaagtcg aagcagactg ccgcgctact ctgggcgaag ttggcaatgc tgagcatatg     300

ctgcgcgttc tgggtaaagc tggtgctgca cgc                                  333

<210>  697
<211>  333
<212>  DNA
<213>  Enterobacter cloacae sbsp. dissolvens

<400>  697
cgttacatcc tggcccctaa aggcctgaaa gctggcgacc agattcagtc tggcgttgat      60

gctgcaatca aagcaggcaa caccctgccg atgcgcaata tcccggttgg ttctaccgtt     120

cataacgtag aaatgaaacc aggtaaaggc ggtcagctgg cgcgttccgc gggtacttac     180

gttcagatcg ttgcgcgtga cggtgcttat gtcaccctgc gtctgcgttc tggtgaaatg     240

cgtaaagtcg aagcagactg ccgcgctact ctgggcgaag ttggcaatgc tgagcatatg     300

ctgcgcgttc tgggtaaagc tggtgctgca cgc 333

<210> 698
<211> 333
<212> DNA
<213> Enterobacter cowanii

<400> 698
cgttacatcc tggccccgaa aggcctgaaa gctggcgacc agattcagtc tggcgttgat 60

gctgcaatca aagcaggcaa caccctgccg atgcgcaata tcccggttgg ttctaccgtt 120

cataacgtag aaatgaaacc aggtaaaggc ggtcagctgg cacgttccgc tggtacttac 180

gttcagatcg ttgctcgcga tggtgcttat gtcaccctgc gtctgcgctc tggtgaaatg 240

cgtaaagtag aagcagactg ccgcgcgact ctgggcgaag ttggcaatgc tgagcatatg 300

ctgcgcgttc tgggtaaagc aggtgctgca cgc 333

<210> 699
<211> 333
<212> DNA
<213> Enterobacter gergoviae

<400> 699
cgttacatcc tggccccgaa aggcctgaaa gctggcgacc agattcagtc tggcgttgat 60

gctgcaatca aagcaggtaa caccctgccg atgcgcaata tcccggttgg ttctaccgtt 120

cataacgtag aaatgaaacc aggtaaaggc ggtcagctgg cacgttccgc tggtacttac 180

gttcagatcg ttgcgcgcga tggtgcttat gtcaccctgc gtctgcgttc tggtgaaatg 240

cgtaaagtcg aagcagactg ccgcgctacc ctgggcgaag ttggcaatgc tgagcatatg 300

ctgcgcgttc tgggtaaagc aggtgctgca cgc 333

<210> 700
<211> 333
<212> DNA
<213> Enterobacter hormaechei

<400> 700
cgttacatcc tggcccctaa aggcctgaaa gctggcgacc agattcagtc tggcgttgat 60

gctgcaatca aagcaggcaa caccctgccg atgcgcaata tcccggttgg ttctaccgtt 120

cataacgtag aaatgaaacc aggtaaaggc ggtcagctgg cgcgttccgc gggtacttac 180

gttcagatcg ttgcgcgtga cggtgcttat gtcaccctgc gtctgcgttc tggtgaaatg 240

cgtaaagtcg aagcagactg ccgcgctact ctgggcgaag ttggcaatgc tgagcatatg 300

ctgcgcgttc tgggtaaagc tggtgctgca cgc 333

<210> 701
<211> 333
<212> DNA
<213> Enterobacter kobei

<400> 701
cgttacatcc tggcccctaa aggcctgaaa gctggcgacc agattcagtc tggcgttgat 60

gctgctatca aagcgggtaa caccctgccg atgcgcaata tcccggttgg ttctaccgtt 120

cataacgtag aaatgaaacc aggtaaaggc ggtcagctgg cgcgttctgc tggtacctat 180

gttcagatcg ttgctcgtga tggtgcttat gtcaccctgc gtctgcgttc tggcgaaatg 240

cgcaaagttg aagctgactg ccgcgcaact ctgggcgaag ttggcaatgc tgagcatatg 300

ctgcgcgttc tgggtaaagc aggtgctgca cgc 333

<210> 702
<211> 333
<212> DNA
<213> Enterobacter ludwigii

<400> 702
cgttacatcc tggcccctaa aggcctgaaa gctggcgacc agattcagtc tggcgttgat 60

gctgcaatca aagcaggcaa caccctgccg atgcgcaata tcccggttgg ttctaccgtt 120

cataacgtag aaatgaaacc aggtaaaggc ggtcagctgg cgcgttccgc gggtacttac 180

gttcagatcg ttgctcgtga cggtgcttat gtcaccctgc gtctgcgttc tggtgaaatg 240

cgtaaagtcg aagcagattg ccgcgctact ctgggcgaag ttggcaatgc tgagcatatg 300

ctgcgcgttc tgggtaaagc tggtgctgca cgc 333

<210> 703
<211> 333
<212> DNA
<213> Enterobacter nimipressuralis

<400> 703
cgttacatcc tggcccctaa aggcctgaaa gctggcgacc agattcagtc tggcgttgat 60

gctgcaatca aagcaggtaa caccctgccg atgcgcaata tcccggttgg ttctaccgtt 120

cataacgtag aaatgaaacc aggtaaaggc ggtcagctgg cacgttccgc tggtacttac 180

gttcagatcg ttgctcgtga tggtgcttat gtcaccctgc gtctgcgttc tggtgaaatg 240

cgtaaagtag aagcagactg ccgtgcaact ctgggcgaag ttggcaatgc tgagcatatg 300

ctgcgcgttc tgggtaaagc aggtgctgca cgc 333

<210> 704
<211> 333
<212> DNA
<213> Enterobacter pyrinus

<400> 704
cgttacattc tggcccctaa aggcctgaaa gccggcgacc agattcagtc tggcgttgat 60

gctgcaatca aagcaggtaa caccctgccg atgcgcaata tcccggttgg ttctactgtt 120

cataacgtag aaatgaaacc aggtaaaggc ggtcagctgg cacgttccgc tggtacttac 180

gttcagatcg ttgctcgcga tggtgcttat gtcaccctgc gtctgcgctc tggtgaaatg 240

cgtaaagtcg aagcagactg ccgcgctact ctgggcgaag ttggcaatgc tgagcatatg 300

ctgcgcgttc tgggtaaagc aggtgctgca cgc 333

<210>  705
<211>  333
<212>  DNA
<213>  Enterobacter radicincitans

<400>  705
cgttacatcc tggcccctaa aggcctgaaa gctggcgacc agattcagtc tggcgttgat      60

gctgcaatca aagcgggtaa caccctgccg atgcgcaata tcccggttgg ttctaccgtt     120

cataacgtag aaatgaaacc aggtaaaggc ggtcagctgg cacgttctgc tggtacttac     180

gttcagatcg ttgcacgtga tggtgcttat gtcaccctgc gtctgcgttc tggtgaaatg     240

cgtaaagttg aagctgagtg ccgcgctact ctgggcgaag ttggcaatgc tgagcatatg     300

ttgcgcgttc tgggtaaagc aggtgctgca cgc                                  333

<210>  706
<211>  333
<212>  DNA
<213>  Enterobacter sakazakii

<400>  706
cgttacatcc tggcccctaa aggcctgaaa gctggcgacc agattcagtc tggcgttgat      60

gctgcaatca aaccaggtaa caccctgccg atgcgcaaca tcccggttgg ttctactgtt     120

cacaacgtag aaatgaaacc aggtaaaggc ggtcagctgg cgcgttctgc tggtacctat     180

gttcagatcg ttgctcgtga cggtgcttat gtcaccctgc gtctgcgttc tggcgaaatg     240

cgtaaagtgg aagctgactg ccgcgcaact ctgggcgaag ttggtaacgc tgagcatatg     300

ctgcgcgttc tgggtaaagc aggtgctgca cgc                                  333

<210>  707
<211>  333
<212>  DNA
<213>  Erwinia amylovora T

<400>  707
cgttacatcc tggcccctaa aggcctgaaa gctggcgacc agattcagtc tggcgttgat      60

gctgcgatca aagcgggtaa caccctgccg atgcgtaaca tcccagtcgg ttctaccgtg     120

cacaacgtag aaatgaaacc aggcaaaggc ggtcagattg ctcgctcagc cggtacttac     180

gtgcagatcg tggcgcgtga aggttcttac gtcaccttgc gtctgcgttc aggtgaaatg     240

cgcaaagtcg aatctgactg ccgcgcaacc ctgggcgaag tcggtaacgc agagcacatg     300

ctgcgcgttc tgggtaaagc cggtgccgct cgt                                  333

<210>  708
<211>  333
<212>  DNA
<213>  Erwinia aphidicola

<400>  708
cgttacatcc tggcccctaa aggcctgaaa gctggcgacc agattcaatc tggcgttgat      60

gctgcgatta aagcgggtaa caccctgcca atgcgtaaca tcccagtggg ttctaccgtg     120

cataacgtag aaatgaaacc aggcaaaggc ggtcagattg ctcgctcagc tggtacttac     180

gtgcagatcg ttgcgcgtga tggttcttac gttaccctgc gtctgcgttc tggtgaaatg 240

cgtaaagtcg aaattgactg ccgcgcgacc ctgggcgaag tcggtaacgc tgagcacatg 300

cttcgcgttc tgggtaaagc cggtgcagct cgt 333

<210> 709
<211> 333
<212> DNA
<213> Erwinia billingiae

<400> 709
cgttacatcc tggcccctaa aggcctgaaa gctggcgacc agattcagtc tggcgttgat 60

gctgcgatta aagcaggtaa caccctgccg atgcgtaaca tcccagtggg ttctaccgtg 120

cataacgtag aaatgaaacc aggcaaaggc ggacagattg ctcgctcagc tggtacttac 180

gtgcagatcg ttgcgcgtga aggttcctac gtaaccctgc gtctgcgttc aggtgaaatg 240

cgtaaagtcg aaatcgactg ccgcgctacc ctgggtgaag tcggtaatgc tgagcatatg 300

cttcgcgttc tgggtaaagc cggtgctgct cgt 333

<210> 710
<211> 333
<212> DNA
<213> Erwinia mallotivora

<400> 710
cgttacatcc tggcccctaa aggcctgaaa gctggtgacc agattcagtc tggtgtcgat 60

gctgcgatta aagcaggtaa caccctgcca atgcgtaaca ttccagtagg ttctaccgta 120

cataacgtag aaatgaaacc aggtaaaggc ggtcagattg cccgctcagc aggtgcttat 180

gtgcagatcg ttgcgcgtga aggttcctac gttaccctgc gtctgcgttc tggtgaaatg 240

cgtaaagtcg aatctgactg ccgcgcgaca ctgggcgaag tcggtaacgc tgagcacatg 300

ctgcgcgttc tgggtaaagc tggtgctgct cgt 333

<210> 711
<211> 333
<212> DNA
<213> Erwinia papayae

<400> 711
cgttacatcc tggcccctaa aggcctgaaa gctggtgacc agattcagtc tggtgtcgat 60

gctgcgatta aagcaggtaa caccctgcca atgcgtaaca ttccagtagg ttctaccgta 120

cataacgtag aaatgaaacc aggtaaaggc ggtcagattg cccgctcagc aggtgcttac 180

gtgcagatcg ttgcgcgtga aggttcctac gttaccctgc gtctgcgttc tggtgaaatg 240

cgtaaagtcg aatctgactg ccgcgcgaca ctgggcgaag tcggtaacgc tgagcacatg 300

ctgcgcgttc tgggtaaagc tggtgctgct cgt 333

<210> 712
<211> 333
<212> DNA

<213>  Erwinia persicina

<400>  712
cgttacatcc tggcccctaa aggcctgaaa gctggcgacc agattcagtc tggcgttgat      60

gctgcgatta aagcaggtaa caccctgccg atgcgtaaca tcccagtggg ttctaccgtg     120

cataacgtag aaatgaaacc aggcaaaggc ggtcagattg ctcgctctgc tggtacttac     180

gtgcagatcg ttgcgcgtga aggttcttac gttaccctgc gtctgcgttc tggtgaaatg     240

cgtaaagtcg aaattgactg ccgcgcaacc ctgggcgaag tcggtaatgc tgagcatatg     300

cttcgcgttc tgggtaaagc cggtgcagct cgt                                  333

<210>  713
<211>  333
<212>  DNA
<213>  Erwinia psidii

<400>  713
cgctacatcc tggctccaaa gggcctgaaa gcaggtgacc agattcagtc tggcgttgat      60

gctgcgatca aagcaggtaa caccctgccg atgcgtaaca ttcctgtggg ttccaccgtg     120

cacaacgtag aaatgaaacc aggcaaaggc ggtcagattg cccgctcagc aggtgcttac     180

gttcagatcg ttgcgcgtga aggttcctac gttaccctgc gtctgcgctc tggtgaaatg     240

cgtaaagtcg aatctgactg ccgcgcgacc ctgggtgaag tcggtaatgc tgagcatatg     300

cttcgcgttc tgggtaaagc tggtgctgct cgt                                  333

<210>  714
<211>  333
<212>  DNA
<213>  Erwinia pyrifoliae

<400>  714
cgttacatcc tggcccctaa aggcctgaaa gctggcgacc agattcagtc tggcgttgat      60

gcggcgatta aagcgggtaa caccctgccg atgcgtaaca tcccagtcgg ttctaccgtg     120

cataacgtag aaatgaaacc aggcaaaggc ggacagattg ctcgctcagc cggtacttac     180

gtgcagatcg ttgcgcgtga aggttcttac gttaccctgc gtctgcgttc tggtgaaatg     240

cgtaaagtcg aatctgactg ccgcgcaacc ctgggcgaag tcggtaacgc tgagcatatg     300

ctgcgcgttc tgggtaaagc cggtgccgct cgt                                  333

<210>  715
<211>  333
<212>  DNA
<213>  Erwinia rhapontici

<400>  715
cgttatatcc tggcccctaa aggcctgaaa gctggcgacc agattcagtc tggcgttgat      60

gctgcgatta aagcaggtaa cactctgccg atgcgtaaca tcccagtggg ttctaccgtg     120

cataacgtag aaatgaaacc aggcaaaggc ggtcagattg ctcgctctgc tggtacttac     180

gtgcagatcg ttgcgcgtga tggttcttac gttaccctgc gtctgcgttc tggtgaaatg     240

cgtaaagtcg aaattgactg ccgcgcaacc ctgggcgaag tcggtaacgc tgagcacatg 300

cttcgcgttc tgggtaaagc cggtgcagct cgt 333

<210> 716
<211> 333
<212> DNA
<213> Erwinia toletana

<400> 716
cgttatatcc tggcccctaa aggcctgaaa gctggcgacc agattcaatc tggcgttgat 60

gctgcgatca aagcaggtaa caccctgccg atgcgtaaca ttccagtggg ttctaccgta 120

cacaacgtag aaatgaaacc aggcaaaggc ggtcagattg ctcgctctgc tggtgcttac 180

gtgcagatcg ttgcgcgtga aggttcttac gttaccctgc gtctgcgttc tggtgaaatg 240

cgtaaagtcg aacttgactg ccgcgcgacc ctgggcgaag tcggcaacgc tgagcatatg 300

ctgcgcgttc tgggtaaagc cggtgcaact cgt 333

<210> 717
<211> 333
<212> DNA
<213> Erwinia tracheiphila

<400> 717
cgctacatcc tggctccaaa aggcctgaaa gcaggtgacc agattcagtc tggcgttgat 60

gctgcgatta aagcaggcaa taccctgccg atgcgtaaca ttcctgtggg ttccaccgtg 120

cataacgtag aaatgaaacc aggtaaaggc ggtcagattg cccgttctgc cggtacttat 180

gtgcagattg ttgcacgtga aggatcctac gttaccctgc gtctgcgttc tggtgaaatg 240

cgcaaagtcg aatctgactg tcgtgcaacg ctgggcgaag tcggtaacgc tgaacatatg 300

cttcgtgttc tgggtaaagc aggtgctgca cgc 333

<210> 718
<211> 333
<212> DNA
<213> Escherichia albertii

<400> 718
cgttacatct tggcccctaa aggcctgaaa gctggcgacc agattcagtc tggcgttgat 60

gctgcaatca aaccaggcaa caccctgccg atgcgcaaca tcccggttgg ttctaccgtt 120

cacaacgtag aaatgaaacc aggtaaaggc ggtcagctgg cacgttctgc tggtacttac 180

gttcagatcg ttgctcgtga tggtgcttat gtcaccctgc gtctgcgttc tggtgaaatg 240

cgtaaagtcg aagcagactg ccgtgcaacc ctgggcgaag ttggcaatgc tgagcatatg 300

ctgcgcgttc tgggtaaagc tggtgctgca cgc 333

<210> 719
<211> 333
<212> DNA
<213> Escherichia blattae

<400> 719

cgttacatcc tggcccctaa aggcctgaaa gctggcgacc agattcagtc tggcgttgat 60

gctgcaatca aagcgggtaa caccctgccg atgcgcaata tcccggttgg ttctactgtt 120

cataacgtag aaatgaaacc aggtaaaggc ggtcagctgg cgcgttctgc tggtacttac 180

gttcagatcg ttgcgcgcga tggtgcttat gtcaccctgc gtctgcgttc tggtgaaatg 240

cgtaaagtcg aagcagactg ccgcgctacc gtgggcgaag ttggcaatgc cgagcatatg 300

ctgcgcgttc tgggtaaagc aggtgctgca cgc 333

<210> 720
<211> 333
<212> DNA
<213> Escherichia coli T

<400> 720
cgttacatcc tggcccctaa aggcctgaaa gctggcgacc agattcagtc tggcgttgat 60

gctgcaatca aaccaggtaa caccctgccg atgcgcaaca tcccggttgg ttctactgtt 120

cataacgtag aaatgaaacc aggtaaaggc ggtcagctgg cacgttccgc tggtacttac 180

gttcagatcg ttgctcgtga tggtgcttat gtcaccctgc gtctgcgttc tggtgaaatg 240

cgtaaagtag aagcagactg ccgtgcaact ctgggcgaag ttggcaatgc tgagcatatg 300

ctgcgcgttc tgggtaaagc aggtgctgca cgc 333

<210> 721
<211> 333
<212> DNA
<213> Escherichia fergusonii

<400> 721
cgttacatcc tggcccctaa aggcctgaaa gctggcgacc agattcagtc tggcgttgat 60

gctgcaatca aaccaggtaa caccctgccg atgcgcaaca tcccggttgg ttctactgtt 120

cataacgtag aaatgaaacc aggtaaaggc ggtcagctgg cacgttccgc tggtacttac 180

gttcagatcg ttgctcgtga tggtgcttat gtcaccctgc gtctgcgttc tggtgaaatg 240

cgtaaagtag aagcagactg ccgtgcaact ctgggcgaag ttggcaatgc tgagcatatg 300

ctgcgcgttc tgggtaaagc aggtgctgca cgc 333

<210> 722
<211> 333
<212> DNA
<213> Escherichia hermannii rplB

<400> 722
cgttacatcc tggccccgaa aggcctgaaa gcgggcgacc agattcagtc tggcgttgat 60

gctgcaatca aagcgggtaa caccctgccg atgcgcaata tcccggttgg ttctaccgtt 120

cataacgtag aaatgaaacc aggtaaaggc ggtcagctgg cgcgttctgc tggtacttac 180

gttcagatcg ttgcgcgtga tggtgcttat gtcaccctgc gtctgcgttc tggtgaaatg 240

cgtaaagtag aagcagattg ccgcgctact ctgggcgaag ttggcaatgc tgagcatatg 300

ctgcgcgttc tgggtaaagc aggtgctgca cgc 333

<210> 723
<211> 333
<212> DNA
<213> Escherichia vulneris

<400> 723
cgttacatcc tggcccctaa aggcctgaaa gctggcgacc agattcagtc tggcgttgat 60

gctgcaatca aagcgggtaa caccctgccg atgcgcaaca tcccggttgg ttctaccgtt 120

cataacgtag aaatgaaacc aggtaaaggc ggtcagctgg cgcgttctgc tggtacttac 180

gttcagatcg ttgcgcgcga cggtgcttat gtcaccctgc gtctgcgttc tggtgaaatg 240

cgtaaagtcg aagcagactg ccgcgctacc ctgggcgaag ttggcaatgc tgagcatatg 300

ctgcgcgttc tgggtaaagc aggtgctgca cgc 333

<210> 724
<211> 333
<212> DNA
<213> Ewingella americana T

<400> 724
cgttatatcc tggcgccgaa aggcctgaaa gtgggtgacc agatccaatc tggtgttgat 60

gcagcaatta agacaggtaa cgccctgcct atgcgcaaca tcccagtagg ttcaacggtt 120

cacaacgtag aaatgaaacc aggtaagggt ggccaaatgg ctcgttctgc tggtgcctac 180

gttcagatcg ttgcacgtga cggttcctac gtaactctgc gtctgcgctc tggcgaaatg 240

cgtaaagttc aagttgattg ccgcgccacc ttaggtgaag tcggtaacgc tgaacatatg 300

cttcgcgttc tgggtaaagc aggtgctgca cgt 333

<210> 725
<211> 333
<212> DNA
<213> Hafnia alvei T

<400> 725
cgttatatcc tggcgcctaa aggcctgaaa gcgggtgacc agattcagtc tggtgttgat 60

gctgctatca aacctggcaa caccttgccg atgcgtaaca tcccggttgg ttctaccgtt 120

cataacgtag aaatgaaacc aggcaaaggt ggtcagctag ctcgttctgc tggcgcatac 180

gttcagatcg ttgctcgcga tggtgcatat gtcaccctgc gtctgcgttc tggtgaaatg 240

cgtaaaatcc catccgactg ccgtgctacc ttaggcgaag ttggtaacgc tgagcatatg 300

ctgcgcgttc tgggtaaagc aggtgcaagc cgc 333

<210> 726
<211> 333
<212> DNA
<213> Klebsiella oxytoca

<400> 726
cgttacattc tggcccctaa aggcctgaaa gctggcgacc agattcagtc tggcgttgat 60

gctgcaatca aagcaggcaa caccctgccg atgcgcaata tcccggttgg ttctaccgtt 120

cataacgtag aaatgaaacc aggtaaaggc ggtcagctgg ctcgttccgc tggtacttac 180

gttcagatcg ttgctcgcga tggtgcttat gtcaccctgc gtctgcgttc tggtgaaatg 240

cgtaaagtcg aagcagactg ccgcgctact ctgggcgaag ttggcaatgc tgagcatatg 300

ctgcgcgttc tgggtaaagc aggtgctgca cgc 333

<210> 727
<211> 333
<212> DNA
<213> Klebsiella pneumoniae sbsp. ozaenae

<400> 727
cgttacatcc tggccccgaa aggcctgaaa gctggcgacc agattcagtc tggcgttgat 60

gctgcaatca aagcaggcaa caccctgccg atgcgcaata tcccggttgg ttctaccgtt 120

cataacgtag aaatgaaacc aggtaaaggc ggtcagctgg ctcgttccgc tggtacttac 180

gttcagatcg ttgctcgtga cggtgcttat gtcaccctgc gtctgcgttc tggtgaaatg 240

cgtaaagtcg aagcagactg ccgcgcgact ctgggcgaag ttggcaatgc tgagcatatg 300

ctgcgcgttc tgggtaaagc aggtgctgca cgc 333

<210> 728
<211> 333
<212> DNA
<213> Klebsiella pneumoniae sbsp. pneumoniae T

<400> 728
cgttacatcc tggccccgaa aggcctgaaa gctggcgacc agattcagtc tggcgttgat 60

gctgcaatca aagcaggcaa caccctgccg atgcgcaata tcccggttgg ttctaccgtt 120

cataacgtag aaatgaaacc aggtaaaggc ggtcagctgg ctcgttccgc tggtacttac 180

gttcagatcg ttgctcgcga tggtgcttat gtcaccttgc gtctgcgttc tggtgaaatg 240

cgtaaagtcg aagcagactg ccgcgctact ctgggcgaag ttggcaatgc tgagcatatg 300

ctgcgcgttc tgggtaaagc aggtgctgca cgc 333

<210> 729
<211> 333
<212> DNA
<213> Klebsiella pneumoniae sbsp. rhinoscleromatis

<400> 729
cgttacatcc tggccccgaa aggcctgaaa gctggcgacc agattcagtc tggcgttgat 60

gctgcaatca aagcaggcaa caccctgccg atgcgcaata tcccggttgg ttctaccgtt 120

cataacgtag aaatgaaacc aggtaaagga ggtcagctgg ctcgttccgc tggtacttac 180

gttcagatcg ttgctcgtga cggtgcttat gtcaccctgc gtctgcgttc tggtgaaatg 240

cgtaaagtcg aagcagactg ccgcgcgact ctgggcgaag ttggcaatgc tgagcatatg 300

ctgcgcgttc tgggtaaagc aggtgctgca cgc 333

<210> 730

<211>  333
<212>  DNA
<213>  Klebsiella singaporensis

<400>  730
cgttacatcc tggccccgaa aggcctgaaa gctggtgacc agattcagtc tggcgttgat      60

gctgcaatca aagcaggcaa caccctgccg atgcgcaata tcccggttgg ttctaccgtt     120

cataacgtag aaatgaaacc aggtaaaggc ggtcagctgg cacgttccgc tggtacttac     180

gttcagatcg ttgctcgtga tggtgcttat gtcaccctgc gtctgcgttc tggtgaaatg     240

cgtaaagtcg aagcagactg ccgcgctact ctgggcgaag ttggcaatgc tgagcatatg     300

ctgcgcgttc tgggtaaagc aggtgctgca cgc                                  333

<210>  731
<211>  333
<212>  DNA
<213>  Klebsiella variicola

<400>  731
cgttacatcc tggccccgaa aggcctgaaa gctggtgacc agattcagtc tggcgttgat      60

gctgcaatca aagcaggcaa caccctgccg atgcgcaata tcccggttgg ttctaccgtt     120

cataacgtag aaatgaaacc aggtaaaggc ggtcagctgg cacgttccgc tggtacttac     180

gttcagatcg ttgctcgtga tggtgcttat gtcaccctgc gtctgcgttc tggtgaaatg     240

cgtaaagtcg aagcagactg ccgcgctact ctgggcgaag ttggcaatgc tgagcatatg     300

ctgcgcgttc tgggtaaagc aggtgctgca cgc                                  333

<210>  732
<211>  333
<212>  DNA
<213>  Kluyvera ascorbata T

<400>  732
cgttacatcc tggcccctaa aggcctgaaa gctggcgacc agattcagtc tggcgttgat      60

gctgcaatca aagcaggtaa caccctgccg atgcgcaata tcccggttgg ttctaccgtt     120

cataacgtag aaatgaaacc aggtaaaggc ggtcagctgg cacgttccgc tggtacttac     180

gttcagatcg ttgctcgcga tggtgcttat gtcaccctgc gtctgcgttc tggtgaaatg     240

cgtaaagtcg aagcagaatg ccgtgcgacc ctgggcgaag ttggcaatgc tgagcatatg     300

ctgcgcgttc tgggtaaagc aggtgctgca cgc                                  333

<210>  733
<211>  333
<212>  DNA
<213>  Kluyvera cochleae

<400>  733
cgttacatcc tggcccctaa aggcctgaaa gctggcgacc agattcaatc tggcgttgat      60

gctgcaatca aagcaggtaa caccctgccg atgcgcaata tcccggttgg ttctaccgtt     120

cataacgtag aaatgaaacc aggtaaaggc ggtcagctgg cacgttccgc tggtacttac     180

gttcagatcg ttgctcgtga tggtgcttat gtcaccctgc gtctgcgttc tggtgaaatg 240

cgtaaagtcg aagcagactg ccgtgcaacc atgggcgaag ttggcaatgc tgagcatatg 300

ctgcgcgttc tgggtaaagc aggtgctgca cgc 333

<210> 734
<211> 333
<212> DNA
<213> Kluyvera cryocrescens

<400> 734
cgttacattc tggcccctaa aggcctgaaa gctggcgacc agattcagtc tggcgttgat 60

gctgcaatca aagcaggtaa caccctgccg atgcgcaata tcccggttgg ttctaccgtt 120

cataacgtag aaatgaaacc aggtaaaggc ggtcagctgg cacgttccgc tggtacttac 180

gttcagatcg ttgctcgcga cggtgcttat gtcaccctgc gtctgcgttc tggtgaaatg 240

cgtaaagtcg aagcagactg ccgtgcaacc atgggcgaag ttggcaatgc tgagcatatg 300

ctgcgcgttc tgggtaaagc aggtgctgca cgc 333

<210> 735
<211> 333
<212> DNA
<213> Kluyvera georgiana

<400> 735
cgttacatcc tggcccctaa aggcctgaaa gctggcgacc agattcagtc tggcgttgat 60

gctgcaatca aagcaggtaa caccctgccg atgcgcaata tcccggttgg ttctaccgtt 120

cataacgtag aaatgaaacc aggtaaaggc ggtcagctgg cacgttccgc tggtacttac 180

gttcagatcg ttgcgcgtga tggtgcttat gtcaccctgc gtctgcgttc tggtgaaatg 240

cgtaaagtcg aagcagactg ccgtgcaacc ctgggcgaag ttggcaatgc tgagcatatg 300

ctgcgcgttc tgggtaaagc aggtgctgca cgc 333

<210> 736
<211> 333
<212> DNA
<213> Kluyvera intermedia

<400> 736
cgttacatcc tggcccctaa aggcctgaaa gctggcgacc agattcaatc tggcgttgat 60

gctgcaatca aagcaggtaa caccctgccg atgcgcaata tcccggttgg ttctaccgtt 120

cataacgtag aaatgaaacc aggtaaaggc ggtcagctgg cacgttccgc tggtacttac 180

gttcagatcg ttgctcgtga tggtgcttat gtcaccctgc gtctgcgttc tggtgaaatg 240

cgtaaagtcg aagcagactg ccgtgcaacc atgggcgaag ttggcaatgc tgagcatatg 300

ctgcgcgttc tgggtaaagc aggtgctgca cgc 333

<210> 737
<211> 333
<212> DNA
<213> Leclercia adecarboxylata T

<400>  737
cgttacattc tggcccctaa aggcctgaaa gctggcgacc agattcagtc tggcgttgat      60

gctgcaatca aagcaggtaa caccctgccg atgcgcaata tcccggttgg ttctaccgtt     120

cataacgtag aaatgaaacc aggtaaaggc ggtcagctgg cacgttccgc tggtacttac     180

gttcagatcg ttgcgcgcga tggtgcttat gtcaccctgc gtctgcgttc tggtgaaatg     240

cgtaaagtcg aagcagactg ccgcgcaact ctgggcgaag ttggcaatgc tgagcatatg     300

ctgcgcgttc tgggtaaagc aggtgctgca cgc                                  333

<210>  738
<211>  333
<212>  DNA
<213>  Leminorella grimontii T

<400>  738
cgttatattc ttgcgcccaa aggcctgaag gccggtgacc aaatccaatc cggtgttgat      60

gcagccatta aggtaggtaa ctgcctgccg atgcgcaaca ttcccgttgg ttctacggtt     120

cacaacgttg aaatgaaacc aggtaaaggc ggccaaatgg ctcgctctgc cggcggttac     180

gttcaaatcg ttgcccgtga aggttcttac gtcactatcc gtcttcgttc cggcgaaatg     240

cgtaaagtgc ttgctgactg ccgcgccacc ttaggtgaag taggcaactc tgagcacatg     300

ctgcgcgtac tgggtaaagc tggcgccagc cgt                                  333

<210>  739
<211>  333
<212>  DNA
<213>  Leminorella richardii

<400>  739
cgttatattc ttgcgcccaa aggcctgaaa gccggtgacc agatccagtc tggtgttgat      60

gctgccatta aagtaggcaa ctgcctgcca atgcgtaaca tccccgttgg ttctacggtt     120

cacaacgttg aaatgaaacc aggtaaaggc ggccagatgg ctcgctcagc cggtggttac     180

gttcaaatcg ttgcccgtga tggttcttac gtcacgatcc gccttcgctc cggcgaaatg     240

cgcaaagtgt tagccgagtg ccgcgccacc ttaggtgaag tgggtaactc tgagcacatg     300

ctgcgcgtac tgggtaaagc tggcgctagc cgc                                  333

<210>  740
<211>  333
<212>  DNA
<213>  Moellerella wisconsensis T

<400>  740
cgttatattt tggcaccaaa aggcctgaaa gcaggtgatc aaatccaatc tggagttgat      60

gctgcaatca aagctggtaa tacattgcca atgcgtaaca tcccagttgg ttcaactgtg     120

cataacattg aactgaaacc tggtaaaggt ggacaactgg ctcgttcagc aggtacatat     180

gctcaaatcg ttgctcgtga cggtgcttat gttactctgc gtctgcgttc tggtgaaatg     240

cgtaaagttc tggctgattg ccgtgcaact ttaggtgaag ttggtaatgc tgagcatatg     300

ctgcgcgtac taggtaaagc tggtgcaagc cgc                                333

<210>  741
<211>  333
<212>  DNA
<213>  Morganella morganii sbsp. morganii T

<400>  741
cgttacatcc tggctcctaa aggcctgaaa gctggtgatc aaatccagtc tggtgttgat      60

gctgcaatca aagcgggtaa cgctctgcca atgcgcaaca tcccggttgg ttctactgtt     120

cataacgtag aaatgaaacc aggtaaaggc ggccagctgg cacgttctgc cggtacttac     180

gttcagatcg ttgcgcgcga tggtgcttat gtcactattc gtctgcgttc cggtgaaatg     240

cgtaaagttc cttcagattg ccgcgcgact ttaggtgaag tgggtaacgc tgaacacatg     300

ctgcgcgttc tgggtaaagc tggtgcaagt cgc                                333

<210>  742
<211>  333
<212>  DNA
<213>  Morganella morganii sbsp. sibonii

<400>  742
cgttacatcc tggctcctaa aggcctgaaa gctggtgatc aaatccagtc tggtgttgat      60

gctgcaatca aagcgggtaa cgctttgcca atgcgcaaca tcccggttgg ttctactatt     120

cataacgtag aaatgaaacc aggtaaaggc ggccagctgg cacgttctgc cggtacttac     180

gttcaaatcg ttgcgcgcga tggtgcttat gtcactattc gtctgcgttc cggtgaaatg     240

cgtaaagttc cttcagactg ccgcgcgact ttaggtgaag tgggtaacgc tgaacacatg     300

ctgcgcgttc tgggtaaagc cggtgcaagc cgc                                333

<210>  743
<211>  333
<212>  DNA
<213>  Obesumbacterium proteus T

<400>  743
cgttatatcc tggcgcctaa aggcctgaaa gcgggtgacc agattcagtc tggtgttgat      60

gctgctatca aacctggcaa caccttgccg atgcgtaaca tcccggttgg ttctaccgtt     120

cataacgtag aaatgaaacc aggcaaaggt ggtcagctag ctcgttctgc tggcgcatac     180

gttcagatcg ttgctcgcga tggtgcatat gtcaccctgc gtctgcgttc tggtgaaatg     240

cgtaaaatcc catccgactg ccgtgctact ttaggcgaag ttggtaacgc tgagcatatg     300

ctgcgcgttc tgggtaaagc aggtgcaagc cgc                                333

<210>  744
<211>  333
<212>  DNA
<213>  Pantoea agglomerans T

<400>  744
cgttacatcc tggcccctaa aggcctgaaa gccggcgacc agatccaatc tggcgttgat      60

gctgcgatta aagcaggtaa cactctgccg atgcgtaaca tcccggtggg ttcaaccgtg 120

cataacgtag aaatgaaacc aggcaaaggc ggtcagattg ctcgctcagc cggtgcttac 180

gtgcagatcg ttgcgcgtga aggttcttac gttaccctgc gtctgcgttc aggtgaaatg 240

cgtaaagtcg agtctgactg ccgcgcaaca ctgggcgaag tcggcaacgc tgagcatatg 300

ctgcgcgttc tgggtaaagc tggtgcagcc cgt 333

<210> 745
<211> 333
<212> DNA
<213> Pantoea ananatis

<400> 745
cgttacatcc tggcccctaa aggcctgaaa gccggcgacc agattcagtc tggcgttgat 60

gctgcgatca aagcaggtaa cacactgccg atgcgtaaca tcccggtggg ttctaccgtt 120

cacaacgtag aaatgaaacc aggcaaaggc ggtcagattg ctcgctcagc cggtgcttac 180

gtgcagatcg ttgcgcgtga aggttcctac gttaccctgc gtctgcgttc aggtgaaatg 240

cgtaaagtcg aagctgactg ccgcgcaaca ctgggcgaag tcggtaacgc tgagcatatg 300

ctgcgcgttc tgggtaaagc cggtgcaacc cgt 333

<210> 746
<211> 333
<212> DNA
<213> Pantoea citrea

<400> 746
cgttatatcc tggcaccaaa aggtttaaaa gctggcgacc agattcagtc tggcgttgat 60

gctgcgatta aagcaggtaa caccctgccg atgcgtaaca tcccagtagg ttcaactgta 120

cataacgtag aaatgaaacc aggcaaaggc ggtcaaattg ctcgctctgc tggtacttac 180

gtgcagatcg ttgcgcgtga tggttcctac gtaaccctgc gtctgcgttc aggtgagatg 240

cgtaaagtcg aagctgactg ccgcgcaaca ctgggcgaag tcggtaacgc tgagcatatg 300

ctgcgcgttc tgggtaaagc aggtgcaact cgc 333

<210> 747
<211> 333
<212> DNA
<213> Pantoea dispersa

<400> 747
cgttacatcc tggcccctaa aggcctgaaa gctggcgacc agattcagtc tggcgttgat 60

gctgcgatca aagcaggtaa caccctgccg atgcgtaaca tcccggttgg ttcaaccgtg 120

cataacgtag aaatgaaacc aggtaaaggc ggtcagattg cccgttctgc tggtgcttac 180

gtgcagatcg ttgcgcgtga aggttcctac gttaccctgc gtctgcgttc aggcgaaatg 240

cgtaaagtcg aagctgactg ccgcgcaacc ctgggcgaag tcggtaacgc tgagcacatg 300

ctgcgcgttc tgggtaaagc cggtgcaacc cgt 333

<210> 748
<211> 333
<212> DNA
<213> Pantoea punctata

<400> 748
cgttatatcc tggctccaaa aggtttaaaa gctggcgacc agattcagtc tggcgttgat 60

gctgcgatta aagcaggtaa caccctgccg atgcgtaaca tcccggtagg ttcaactgta 120

cataacgtag aaatgaaacc aggcaaaggc ggtcagattg ctcgctctgc tggtacttac 180

gtgcagatcg ttgcacgtga tggttcctac gtcaccctgc gtctgcgttc aggtgaaatg 240

cgtaaagtcg aagctgactg ccgcgcaaca ctgggcgaag tcggtaacgc tgagcatatg 300

ctgcgcgttc tgggtaaagc aggtgcaact cgc 333

<210> 749
<211> 333
<212> DNA
<213> Pantoea stewartii sbsp. indologenes

<400> 749
cgttacatcc tggcccctaa aggcctgaaa gccggcgacc agattcagtc tggcgttgat 60

gctgcgatca aagcaggtaa cacactgccg atgcgtaaca tcccggtggg ttctaccgtt 120

cacaacgtag aaatgaaacc aggcaaaggc ggtcagattg ctcgctcagc cggtgcttac 180

gtgcagatcg ttgcgcgtga aggttcctac gttaccctgc gtctgcgttc aggtgaaatg 240

cgtaaagtcg aagcggactg ccgcgctaca ctgggcgaag tcggtaacgc tgagcatatg 300

ctgcgcgttc tgggtaaagc tggtgcaacc cgt 333

<210> 750
<211> 333
<212> DNA
<213> Pantoea stewartii sbsp. stewartii

<400> 750
cgttacatcc tggcccctaa aggcctgaaa gccggcgacc agattcagtc tggcgttgat 60

gctgcgatca aagcaggtaa cacactgccg atgcgtaaca tcccggtggg ttctaccgtt 120

cacaacgtag aaatgaaacc aggcaaaggc ggtcagattg ctcgctcagc cggtgcttac 180

gtgcagatcg ttgcgcgtga aggttcctac gttaccctgc gtctgcgttc aggtgaaatg 240

cgtaaagtcg aagctgactg ccgcgctaca ctgggcgaag tcggtaacgc tgagcatatg 300

ctgcgcgttc tgggtaaagc tggtgcaacc cgt 333

<210> 751
<211> 333
<212> DNA
<213> Pantoea terrea

<400> 751
cgttatatcc tggcccctaa aggtctgaaa gctggcgacc agatccaatc tggtagtgat 60

gctgcgatta aagcaggtaa caccctgccg atgcgtaata tcccggtcgg ttcaactgta 120

cacaacgtag aaatgaaacc aggtaaaggc ggtcagattg ctcgctctgc tggtacttac 180

gtgcagatcg ttgcgcgtga tggttcctac gtaaccctgc gtctgcgttc aggtgaaatg 240

cgtaaagtcg aagctgactg ccgcgcaaca ctgggcgaag tcggtaacgc tgagcatatg 300

ctgcgcgttc tgggtaaagc aggtgcaact cgc 333

<210> 752
<211> 333
<212> DNA
<213> Pectobacterium atrosepticum

<400> 752
cgttacattt tggcgccgaa aggcctgaaa gccggcgacc agattcaatc tggcgttgat 60

gctgcgatta aagcgggtaa caccctgccg atgcgtaaca ttccggtcgg ttcaacggtt 120

cacaacgtag aaatgaaacc aggtaaaggc ggccaattgg ctcgttctgc tggtacttac 180

gttcagatcg ttgctcgtga cggttcttac gttaccctgc gtctgcgttc tggcgaaatg 240

cgtaaagtcg aatctgactg tcgcgctacg ctgggcgaag ttggcaacgc tgagcacatg 300

ctacgcgttc tgggtaaagc tggtgctgct cgc 333

<210> 753
<211> 333
<212> DNA
<213> Pectobacterium betavasculorum

<400> 753
cgctacatcc tggcgccgaa aggcttgaaa gccggcgacc agattcaatc tggcgttgat 60

gctgcgatta aagcgggcaa caccctgccg atgcgtaaca ttccggtcgg ttcaacggtt 120

cacaacgtag aaatgaaacc aggtaaaggc ggccaattgg ctcgttctgc tggtacatac 180

gttcagatcg ttgctcgtga cggttcttac gttaccctgc gtctgcgttc tggcgaaatg 240

cgtaaagtcg aatctgactg ccgcgctacg ctgggcgaag tcggcaacgc tgagcacatg 300

ctgcgcgttc tgggtaaagc tggtgctgca cgc 333

<210> 754
<211> 333
<212> DNA
<213> Pectobacterium cacticida

<400> 754
cgttacattc tggcgccgaa aggcctgaaa gttggcgatc agatccaatc tggtgttgat 60

gctgctatca aagcgggtaa caccctgccg atgcgtaata tcccggtagg ttcaacggtt 120

cataacgtag aacttaaacc aggcaagggc ggtcaattgg cacgttctgc tggcgcttac 180

gttcagatcg ttgcacgtga tggtgcttac gtgaccctgc gtctgcgttc tggtgaaatg 240

cgtaaagttg aatctgactg ccgcgcaacc ctgggcgaag ttggcaatgc tgagcatatg 300

ctgcgcgttt tgggtaaagc aggtgctgca cgc 333

<210> 755
<211> 333

<212>  DNA
<213>  Pectobacterium carotovorum sbsp. odoriferum

<400>  755
cgttacatcc tggcgccgaa aggcctgaaa gccggcgatc agattcaatc tggcgttgat      60

gctgcgatta aagcgggtaa caccctgccg atgcgtaaca ttccagtcgg ttcaacggtt     120

cataacgtag aaatgaaacc aggtaaaggc ggccaattgg ctcgctctgc tggtgcttac     180

gttcagatcg ttgctcgtga cggttcttac gttaccctgc gtctgcgttc tggcgaaatg     240

cgtaaagtcg aatccgactg ccgcgctacg ctgggcgaag tcggcaacgc tgagcacatg     300

cttcgcgttc tgggtaaagc tggtgctgca cgc                                  333

<210>  756
<211>  333
<212>  DNA
<213>  Pectobacterium cypripedii

<400>  756
cgttacatcc tggcccctaa aggcctgaaa gctggcgacc aggttcagtc tggcgttgat      60

gctgcgatta aagcaggtaa caccctgccg atgcgtaaca tcccagttgg ttctaccgtg     120

cacaacgtag aaatgaaacc aggcaaaggc ggtcagattg ctcgctctgc tggtacttac     180

gtgcagatcg ttgcgcgtga aggttcctac gttaccctgc gtctgcgttc aggtgaaatg     240

cgtaaagtcg aagctgactg ccgcgcaact ctgggcgaag tcggtaacgc tgagcacatg     300

ctgcgcgttc tgggtaaagc cggtgcaacc cgt                                  333

<210>  757
<211>  333
<212>  DNA
<213>  Pectobacterium wasabiae

<400>  757
cgttacattt tggcaccaaa aggcctgaaa gccggcgacc agattcaatc tggcgttgat      60

gctgcgatta aagcgggtaa taccctgccg atgcgtaaca tcccagtcgg ttcaacggtt     120

cacaacgtag aaatgaaacc aggtaaaggc ggccagttgg ctcgttctgc tggtacttac     180

gttcagatcg ttgctcgtga tggttcttac gttaccctac gcctgcgttc tggcgaaatg     240

cgtaaagtcg aatccgactg ccgcgctacg ctgggcgaag ttggcaacgc tgagcatatg     300

ctacgcgttc tgggtaaagc tggtgctgca cgc                                  333

<210>  758
<211>  333
<212>  DNA
<213>  Photorhabdus asymbiotica sbsp. australis

<400>  758
cgttatattc ttgcacctaa aggtttgaaa gctggtgacc agatccaatc tggtgctgat      60

gccgctatca aagctggtaa cacattgcca atgcgcaata tccctgttgg ttctactgta     120

cacaacgtag aaatgaaacc gggtaaaggt ggtcagttgg ctcgttctgc tggtgcatat     180

gttcagatcg ttgctcgtga tggttcttat gtgaccttgc gtctgcgttc tggtgaaatg     240

cgtaaagttc tgtctgattg tcgtgctact ttaggcgaag ttggtaatgc agaacacatg     300

ctacgcgttc tgggtaaagc tggtgcaagt cgc                                 333

<210>  759
<211>  333
<212>  DNA
<213>  Photorhabdus luminescens sbsp. akhurstii

<400>  759
cgttatatcc ttgcgcctaa aggtctgaaa gctggtgatc agatccaatc tggtgctgac      60

gcagctatca aaactggtaa cgcattgcca atgcgcaata tccctgttgg ttctaccgta     120

cataacgtag aaatgaagcc gggtaaaggt ggtcagttgg ctcgttccgc tggtgcatac     180

gttcagatcg ttgcccgtga tggttcttat gtaactttgc gtctgcgttc tggtgaaatg     240

cgtaaagttc tgtctgattg ccgcgccact ttaggtgaag ttggtaatgc agaacatatg     300

ctacgtgttc tgggtaaagc tggtgcaagt cgc                                 333

<210>  760
<211>  333
<212>  DNA
<213>  Photorhabdus luminescens sbsp. kayaii

<400>  760
cgttatatcc ttgcacctaa agggctgaaa gctggtgatc agattcaatc tggtgttgac      60

gcagctatca aaactggtaa cgcattgcca atgcgcaata tccctgttgg ttctaccgta     120

cataacgtag aaatgaaacc gggtaaaggt ggtcagttgg ctcgttccgc tggtgcatac     180

gttcagatcg ttgcccgtga tggttcttat gtaactttgc gtctgcgctc tggtgaaatg     240

cgtaaagttc tgtctgactg ccgcgccact ttaggtgagg ttggtaatgc agaacatatg     300

ctacgtgttc tgggtaaagc tggtgcaagt cgc                                 333

<210>  761
<211>  333
<212>  DNA
<213>  Photorhabdus luminescens sbsp. luminescens T

<400>  761
cgttatatcc ttgcacctaa ggggctgaaa gctggtgatc agatccaatc tggtgctgac      60

gcagctatca aaactggtaa cgcattgcca atgcgcaata tccctgttgg ttctaccgtg     120

cataacgtag aaatgaagcc gggtaaaggt ggtcaattgg ctcgttccgc tggtgcatac     180

gttcagatcg ttgcccgtga tggttcttat gtaactttgc gtctgcgttc tggtgaaatg     240

cgtaaagttc tgtctgattg ccgcgccacc ttaggtgaag ttggtaatgc agaacatatg     300

ctacgcgttc tgggtaaagc tggtgcaagt cgc                                 333

<210>  762
<211>  333
<212>  DNA
<213>  Photorhabdus temperata sbsp. temperata

<400>  762
cgttatattc ttgcacctaa aggcctgaaa gctggtgatc agatccaatc tggtgctgat      60
gcagctatca aagctggtaa tgcattgcca atgcgtaata tcccggttgg ttctactgta     120
cacaacgtag agatgaagcc gggtaaaggc ggccagttag ctcgttccgc tggtgcatat     180
gttcagatcg ttgctcgtga tggttcttat gtaaccttgc gtttgcgctc tggtgaaatg     240
cgtaaagttc tgtctgactg ccgcgctact ttaggtgaag ttggtaacgc agaacatatg     300
ctacgtgttc tgggtaaagc tggtgcaagt cgc                                  333

<210>  763
<211>  333
<212>  DNA
<213>  Pragia fontium T

<400>  763
cgttatattc tggcaccaaa aggcctgaaa gccggtgacc aaatccaatc tggtgttgat      60
gctgctatta aggcgggtaa ctgcctacca atgcgcaaca tcccagtggg ttctacggtt     120
cacaacgttg aaatgaaacc aggtaaaggc ggccaaatgg ctcgttctgc cggtggatat     180
gttcaaatcg ttgcccgtga aggttcctac gtaacgatac gtcttcgttc tggcgaaatg     240
cgtaaagtat tatctgattg ccgcgccacc ttaggtgaag tgggtaactc agagcatatg     300
ctgcgcgtac tgggtaaagc cggtgctagt cgt                                  333

<210>  764
<211>  333
<212>  DNA
<213>  Proteus mirabilis

<400>  764
cgctacatcc tggctccaaa aggcctgaaa gcgggtgata aagttcaatc tggcgttgat      60
gctgctatca aaacgggtaa caccttacca atgcgtaata tcccggttgg ttctaccgtt     120
cataacgtag aaatgaaacc aggtaaaggt ggtcagttag ctcgttcagc tggtacttac     180
gttcaaatcg ttgcgcgtga tggtgcttat gtcactattc gtctgcgttc tggtgaaatg     240
cgtaagatcc cttctgattg ccgtgcaact atcggtgaag taggtaactc tgagcacatg     300
ttacgcgttc tgggtaaagc tggtgcaagt cgc                                  333

<210>  765
<211>  333
<212>  DNA
<213>  Proteus penneri

<400>  765
cgctacattc tggctccaaa aggcctgaaa gcgggtgata aagttcagtc tggcgttgat      60
gctgctatca aagcgggtaa caccttacca atgcgtaata tcccggttgg ttctacagtt     120
cataacgtag aaatgaaacc aggtaaaggt ggtcagttag ctcgttcagc tggtacttac     180
gttcagatcg ttgctcgtga tggtgcttat gtcactatcc gtctgcgttc tggtgaaatg     240
cgtaaagttc cttctgattg ccgtgcaact atcggtgaag ttggcaactc tgagcacatg     300

ttacgcgttc tgggtaaagc tggtgcaagt cgc 333

<210> 766
<211> 333
<212> DNA
<213> Proteus vulgaris T

<400> 766
cgctacattc tggctccaaa aggcctgaaa gcgggtgata aagttcagtc tggcgttgat 60

gctgctatca aaacgggtaa caccttacca atgcgtaata tcccggttgg ttctactgtt 120

cataacgtag aaatgaaacc aggtaaaggt ggtcagttag ctcgttcagc tggtacttac 180

gttcagatcg ttgctcgtga tggtgcttat gtcactattc gtctgcgttc tggtgaaatg 240

cgtaaagttc cttctgattg ccgtgcaact atcggtgaag taggtaactc tgagcacatg 300

ttacgcgttc tgggtaaagc tggtgcaagt cgc 333

<210> 767
<211> 333
<212> DNA
<213> Providencia alcalifaciens T

<400> 767
cgttatattc ttgcgccaaa aggcctgaaa gctggtgacc agattcaatc tggcgttgat 60

tcagctatca aagctggtaa cgcactgcca atgcgcaaca tccctgttgg ttctacagtt 120

cataacattg aactgaaacc aggtaaaggt ggtcagttag cgcgttcagc aggtacttac 180

gctcaaatcg ttgctcgtga tggtgcttat gtcaccctgc gtctgcgttc tggtgaaatg 240

cgtaaagttt tagctgattg ccgtgcaacc ttaggtgaag ttggtaacgc tgagcatatg 300

ttacgtgttc tcggtaaagc tggtgctagt cgc 333

<210> 768
<211> 333
<212> DNA
<213> Providencia heimbachae

<400> 768
cgttatattc ttgcaccaaa aggcttgaaa gctggtgacc agattcaatc tggtgttgat 60

gcagcaatca aagctggtaa tacactgcct atgcgcaaca tccctgttgg ttctacagtt 120

cataatatcg aactgaaacc aggtaaaggt ggacagttag cgcgttcagc aggtacttac 180

gctcaaatcg ttgctcgtga tggtgcttat gtcaccttac gtctgcgttc tggtgaaatg 240

cgtaaagttt tagttgattg ccgagcgacc ttaggtgaag ttggtaacgc tgagcatatg 300

ttacgtgttc tgggtaaagc tggtgcaagt cgc 333

<210> 769
<211> 333
<212> DNA
<213> Providencia rettgeri

<400> 769
cgttatattc ttgcaccaaa aggcttgaaa gctggtgacc agattcaatc tggtgttgat 60

tcagcaatca aagctggtaa tgcactgccg atgcgcaata tccctgttgg ttctacagtt 120

cataatatcg aactgaaacc aggtaaaggt ggacagttag cgcgttcagc aggtacttac 180

gctcaaatcg ttgctcgtga tggcgcttat gtctcattac gtttacgttc tggtgaaatg 240

cgtaaagttt tagctgattg ccgtgcaact cttggtgaag ttggtaacgc tgaacatatg 300

ttacgtgttc tcggtaaagc tggtgctagc cgc 333

<210> 770
<211> 333
<212> DNA
<213> Providencia rustigianii

<400> 770
cgttatattc ttgcaccaaa aggcttgaaa gctggtgacc agattcaatc tggtgttgat 60

tcagcaatca aagcaggtaa cgcactgcca atgcgcaaca tccccgttgg ttctacagtt 120

cataatatcg aactgaaacc aggtaaaggt ggacagttag cgcgttcagc aggtacttac 180

gctcagatcg ttgctcgtga tggtgcttat gtcaccctgc gtttgcgttc tggtgaaatg 240

cgtaaagttt tagcggattg ccgtgcaacc ttaggtgaag tcggtaacgc tgaacatatg 300

ttacgtgttc tcggtaaagc tggtgctagt cgc 333

<210> 771
<211> 333
<212> DNA
<213> Providencia stuartii

<400> 771
cgttatattc ttgcgcctaa aggtttgaaa gctggtgacc agatccaatc tggcgttgat 60

tcagcaatca aaactggtaa cgcactgcca atgcgcaaca tccctgttgg ttctacagtt 120

cacaacatcg aactgaaacc aggtaaaggt ggtcagttag cgcgttcagc aggtacttat 180

gctcagatcg ttgcacgtga tggtgcttat gtcactctgc gtctgcgttc tggtgaaatg 240

cgtaaagttc tggctgattg ccgtgcaact ttaggtgaag ttggtaacgc tgagcacatg 300

ttacgtgttc tgggtaaagc tggtgctagc cgc 333

<210> 772
<211> 333
<212> DNA
<213> Providencia vermicola

<400> 772
cgttatattc ttgcaccaaa aggcctgaaa gctggtgacc agattcaatc tggtgttgat 60

tcagcaatca aagctggtaa tgcaatgcca atgcgcaaca tccctgttgg ttctacagtt 120

cacaacattg aactgaaacc aggtaaaggt ggtcagttag cgcgttcagc aggtacttac 180

gctcaaatcg ttgctcgtga tggtgcttat gtaacattac gtctgcgttc tggtgaaatg 240

cgtaaagtat tagcagattg ccgtgcaacc ttaggtgaag ttggtaacgc tgaacatatg 300

ttacgtgttc tcggtaaagc tggtgctagc cgc 333

<210> 773
<211> 333
<212> DNA
<213> Rahnella aquatilis T

<400> 773
cgttacatcc tggcgccgaa aggcctgaaa gtgggtgacc agatccaatc tggtgttgat 60

gctgcaatca agacaggtaa caccctgcct atgcgcaaca tcccagtagg ttcaacggtt 120

cacaacgtag aaatgaaacc aggtaaaggc ggccagatgg ctcgttcagc cggtgcctac 180

gttcagatcg ttgctcgtga cggttcctac gtgactctgc gtctgcgctc tggcgaaatg 240

cgtaaagttc aagttgattg ccgcgccacc ttaggtgaag tcggtaactc tgaacacatg 300

cttcgcgttc tgggtaaagc aggtgctgct cgt 333

<210> 774
<211> 333
<212> DNA
<213> Raoultella ornithinolytica

<400> 774
cgttacatcc tggcccctaa aggcctgaaa gctggcgacc agattcagtc tggcgttgat 60

gctgcaatca aagcaggcaa caccctgccg atgcgcaata tcccggttgg ttctaccgtt 120

cataacgtag aaatgaaacc aggtaaaggc ggtcagctgg ctcgttccgc tggtacttac 180

gttcagatcg ttgctcgcga cggtgcttat gtcaccctgc gtctgcgttc tggtgaaatg 240

cgtaaagtcg aagcagactg ccgcgctact ctgggcgaag ttggcaatgc tgagcatatg 300

ctgcgcgtac tgggtaaagc aggtgctgca cgc 333

<210> 775
<211> 333
<212> DNA
<213> Raoultella planticola T

<400> 775
cgttacatcc tggcccctaa aggcctgaaa gctggcgacc agattcagtc tggcgttgat 60

gctgcaatca aagcaggcaa caccctgccg atgcgcaata tcccggttgg ttctaccgtt 120

cataacgtag aaatgaaacc aggtaaaggc ggtcagctgg ctcgttccgc tggtacttac 180

gttcagatcg ttgctcgcga cggtgcttat gtcaccctgc gtctgcgttc tggtgaaatg 240

cgtaaagtcg aagcagactg ccgcgctact ctgggcgaag ttggcaatgc tgagcatatg 300

ctgcgcgtac tgggtaaagc aggtgctgca cgc 333

<210> 776
<211> 333
<212> DNA
<213> Raoultella terrigena

<400> 776
cgttacatcc tggcccctaa aggcctgaaa gctggcgacc agattcagtc tggcgttgat 60

gctgcaatca aagcaggcaa caccctgccg atgcgcaata tcccggttgg ttctaccgtt 120

cataacgtag aaatgaaacc aggtaaaggc ggtcagctgg cacgttccgc tggtacttac 180

gttcagatcg ttgctcgtga tggtgcttat gtcaccctgc gtctgcgttc tggtgaaatg 240

cgtaaagtcg aagcagactg ccgcgctact ctgggcgaag ttggcaatgc tgagcatatg 300

ctgcgcgttc tgggtaaagc aggtgctgca cgc 333

<210> 777
<211> 333
<212> DNA
<213> Salmonella bongori

<400> 777
cgttacatcc tggccccgaa aggcctgaaa gctggcgacc agattcagtc tggcgttgat 60

gctgcaatca aagcaggcaa caccctgccg atgcgcaata tcccggttgg ttctaccgtt 120

cataacgtag aaatgaaacc aggtaaaggc ggtcagctgg cgcgttccgc tggtacttac 180

gttcagatcg ttgctcgcga tggcgcttat gtcactctgc gtctgcgttc tggtgaaatg 240

cgtaaagtcg aagcagactg ccgtgcaacc ctgggcgaag ttggcaatgc tgagcatatg 300

ctgcgcgttc tgggtaaagc aggtgctgca cgc 333

<210> 778
<211> 333
<212> DNA
<213> Salmonella enterica sbsp. arizonae

<400> 778
cgttacatcc tggccccgaa aggcctgaaa gctggcgacc agattcagtc tggcgttgat 60

gctgcaatca aagcaggcaa caccctgccg atgcgcaata tcccggttgg ttctaccgtt 120

cataacgtag aaatgaaacc aggtaaaggc ggtcagctgg cgcgttccgc tggtacttac 180

gttcagatcg ttgctcgcga tggcgcttat gtcaccctgc gtctgcgttc tggtgaaatg 240

cgtaaagtcg aagcagactg ccgtgcaact ctgggcgaag ttggcaatgc tgagcatatg 300

ctgcgcgttc tgggtaaagc aggtgctgca cgc 333

<210> 779
<211> 333
<212> DNA
<213> Salmonella enterica sbsp. diarizonae

<400> 779
cgttacatcc tggccccgaa aggcctgaaa gctggcgacc agattcagtc tggcgttgat 60

gctgcaatca aagcaggcaa caccctgccg atgcgcaata tcccggttgg ttctaccgtt 120

cataacgtag aaatgaaacc aggtaaaggc ggtcagctgg cgcgttccgc tggtacttac 180

gttcagatcg ttgctcgcga tggcgcttat gtcaccctgc gtctgcgttc tggtgaaatg 240

cgtaaagtcg aagcagactg ccgtgcaacc ctgggcgaag ttggcaatgc tgagcatatg 300

ctgcgcgttc tgggtaaagc aggtgctgca cgc 333

<210> 780
<211> 333
<212> DNA

<213>  Salmonella enterica sbsp. enterica T

<400>  780
cgttacatcc tggccccgaa aggcctgaaa gctggcgacc agattcagtc tggcgttgat      60

gctgcaatca aagcaggcaa caccctgccg atgcgcaata tcccggttgg ttctaccgtt     120

cataacgtag aaatgaaacc aggtaaaggc ggtcagctgg cacgttccgc tggtacttac     180

gttcagatcg ttgctcgcga tggcgcttat gtcaccctgc gtctgcgttc tggtgaaatg     240

cgtaaagtcg aagcagactg ccgtgcaacc ctgggcgaag ttggcaatgc tgagcatatg     300

ctgcgcgttc tgggtaaagc aggtgctgca cgc                                  333


<210>  781
<211>  333
<212>  DNA
<213>  Salmonella enterica sbsp. houtenae

<400>  781
cgttacatcc tggccccgaa aggcctgaaa gctggcgacc agattcagtc tggcgttgat      60

gctgcaatca aagcaggcaa caccctgccg atgcgcaata tcccggttgg ttctaccgtt     120

cataacgtag aaatgaaacc aggtaaaggc ggtcagctgg cgcgttccgc tggtacttac     180

gttcagatcg ttgctcgcga tggcgcttat gtcaccctgc gtctgcgttc tggtgaaatg     240

cgtaaagtcg aagcagactg ccgtgcaacc ctgggcgaag ttggcaatgc tgagcatatg     300

ctgcgcgttc tgggtaaagc aggtgctgca cgc                                  333


<210>  782
<211>  333
<212>  DNA
<213>  Salmonella enterica sbsp. indica

<400>  782
cgttacatcc tggcccctaa aggcctgaaa gctggcgacc agattcagtc tggcgttgat      60

gctgcaatca aagcaggcaa caccctgccg atgcgcaata tcccggttgg ttctaccgtt     120

cataacgtag aaatgaaacc aggtaaaggc ggtcagctgg cgcgttccgc tggtacttac     180

gttcagatcg ttgctcgcga tggcgcttat gtcaccctgc gtcttcgttc tggtgaaatg     240

cgtaaagtcg aagcagactg ccgcgctact ctgggcgaag ttggcaatgc tgagcatatg     300

ctgcgcgttc tgggtaaagc aggtgctgca cgc                                  333


<210>  783
<211>  333
<212>  DNA
<213>  Salmonella enterica sbsp. salamae

<400>  783
cgttacatcc tggccccgaa aggcctgaaa gctggcgacc agattcagtc tggcgttgat      60

gctgcaatca aagcaggcaa caccctgccg atgcgcaata tcccggttgg ttctaccgtt     120

cataacgtag aaatgaaacc aggtaaaggc ggtcagctgg cgcgttccgc tggtacttac     180

gttcagatcg ttgctcgcga tggcgcttat gtcaccctgc gtctgcgttc tggtgaaatg     240

cgtaaagtcg aagcagactg ccgtgcaacc ctgggcgaag ttggcaatgc tgagcatatg 300

ctgcgcgttc tgggtaaagc aggtgctgca cgc 333

<210> 784
<211> 333
<212> DNA
<213> Samsonia erythrinae T

<400> 784
cgttatatcc tggcgccgaa aggcctgaaa gccggcgacc agattcaatc tggcgttgat 60

gctgcaatta aaacgggtaa caccctgccg atgcgtaaca ttcctgtcgg ttcaacggtt 120

cacaacgtag aaatgaaacc tggtaagggc ggccaactgg ctcgttctgc gggcggctac 180

gctcaaatcg ttgcacgtga cggttcttac gttaccctgc gtctgcgttc tggcgaaatg 240

cgtaaagtcg aatccgactg ccgcgcaacg ctgggcgaag ttggcaacgc tgagcatatg 300

ctgcgcgttc tgggtaaagc aggtgctgca cgc 333

<210> 785
<211> 333
<212> DNA
<213> Serratia entomophila

<400> 785
cgttacatcc tggcgccgaa aggcctgaaa gctggtgacc agattcaatc tggcgttgat 60

gctgcaatca aagcgggtaa caccctgccg atgcgcaaca tcccggttgg ttcaacggtt 120

cacaacgtag aaatgaaacc aggtaaaggc ggccagatgg ctcgttcagc cggtgcctac 180

gttcagatcg ttgctcgtga tggttcctac gtaactctgc gtctgcgctc cggcgaaatg 240

cgtaaagttc cagttgattg ccgcgccacc ctgggtgaag tcggtaacgc tgaacatatg 300

cttcgcgttc tgggtaaagc aggtgctgca cgt 333

<210> 786
<211> 333
<212> DNA
<213> Serratia ficaria

<400> 786
cgttacatcc tggcgccgaa aggcctgaaa gctggtgacc agattcaatc tggcgttgat 60

gctgcaatca aaacgggtaa caccctgccg atgcgtaaca tcccagttgg ttcaacggtt 120

cacaacgtag aaatgaaacc aggtaaaggc ggccagatgg ctcgttcagc cggtgcctac 180

gttcagatcg ttgctcgtga tggttcctac gtaactctgc gtctgcgctc aggcgaaatg 240

cgtaaagttc cagttgattg ccgcgccacc ctgggtgaag tcggtaacgc tgaacatatg 300

cttcgcgttc tgggtaaagc aggtgctgca cgt 333

<210> 787
<211> 333
<212> DNA
<213> Serratia fonticola

<400> 787

cgttatatcc tggcgccgaa aggcctgaaa gctggtgacc agatccaatc tggtgttgat 60

gctgcaatca aagcgggtaa caccctgcct atgcgcaaca tcccagtagg ttcaacggtt 120

cataacgtag aaatgaaacc aggtaaaggc ggccagctgg ctcgttctgc tggtgcctac 180

gttcagatcg tcgcacgtga tggttcctac gtaactctgc gtctgcgctc tggcgaaatg 240

cgtaaagttc cagttgattg ccgcgccacc ctgggtgaag tcggtaacgc tgaacatatg 300

cttcgcgttc tgggtaaagc aggtgctgca cgt 333

<210> 788
<211> 333
<212> DNA
<213> Serratia grimesii

<400> 788
cgttacatcc tggcgccgaa aggcctgaaa gctggtgacc agatccaatc tggtgttgat 60

gctgcaatca aagcaggtaa cgccctgcca atgcgcaaca tcccagtggg ttcaacggtt 120

cataacgtag aaatgaaacc aggtaaaggc ggccagatgg ctcgttctgc tggtgcctac 180

gttcagatcg ttgcacgtga tggttcctac gtaactctgc gtctgcgctc aggcgaaatg 240

cgtaaagttc cagttgattg ccgcgccacc ttgggtgaag tcggtaacgc tgaacatatg 300

cttcgcgttc tgggtaaagc aggtgctgca cgt 333

<210> 789
<211> 333
<212> DNA
<213> Serratia liquefaciens

<400> 789
cgttacatcc tggcgccgaa aggcctgaaa gctggtgacc agatccaatc tggcgttgat 60

gctgcaatca aggtaggtaa caccctgcca atgcgcaaca tcccagtggg ttcaacggtt 120

cacaacgtag aaatgaaacc aggtaaaggc ggccagatgg ctcgttctgc tggtgcctac 180

gttcagatcg ttgcacgtga tggttcctac gtaactctgc gtctgcgctc aggcgaaatg 240

cgtaaagttc cagttgattg ccgcgccacc ctgggtgaag tcggtaacgc tgaacatatg 300

cttcgcgttc tgggtaaagc aggtgctgca cgt 333

<210> 790
<211> 333
<212> DNA
<213> Serratia marcescens sbsp. marcescens T

<400> 790
cgttacatcc tggcgccgaa aggcctgaaa gctggtgacc agattcaatc tggcgttgat 60

gctgcaatca aagcgggtaa caccctgccg atgcgcaaca ttccagttgg ttcaacggtt 120

cacaacgtag aaatgaaacc aggtaaaggc ggccagatgg ctcgctccgc tggtgcctac 180

gttcagatcg ttgctcgtga tggttcctac gtaactctgc gtctgcgctc cggcgaaatg 240

cgtaaagttc cagctgattg ccgcgccacc ctgggtgaag tcggtaacgc tgaacacatg 300

cttcgcgttc tgggtaaagc aggtgctgca cgt 333

<210> 791
<211> 333
<212> DNA
<213> Serratia marcescens sbsp. sakuensis

<400> 791
cgttacatcc tggcgccgaa aggcctgaaa gctggtgacc agattcaatc tggcgttgat 60

gctgcaatca aagcgggtaa caccctgccg atgcgcaaca ttccagttgg ttcaacggtt 120

cacaacgtag aaatgaaacc aggtaaaggc ggccagatgg ctcgctccgc tggtgcctac 180

gttcagatcg ttgctcgtga tggttcctac gtaactctgc gtctgcgctc cggcgaaatg 240

cgtaaagttc cagctgattg ccgcgccacc ctgggtgaag tcggtaacgc tgaacacatg 300

cttcgcgttc tgggtaaagc aggtgctgca cgt 333

<210> 792
<211> 333
<212> DNA
<213> Serratia odorifera

<400> 792
cgttacatcc ttgcgccgaa aggcctgaaa gctggtgacc agattcaatc tggcgttgat 60

gctgcaatca aaacgggtaa caccctgccg atgcgcaaca ttccagtggg ttcaacggtt 120

cacaacgtag aaatgaaacc aggtaaaggc ggccagatgg ctcgttcagc cggtgcctac 180

gttcagatcg ttgcacgtga tggttcctac gtaactctgc gtctgcgctc cggcgaaatg 240

cgtaaagttc cagttgattg ccgcgccacc ctgggtgaag tcggtaacgc tgaacatatg 300

cttcgcgttc tgggtaaagc aggtgctgca cgt 333

<210> 793
<211> 339
<212> DNA
<213> Serratia plymuthica

<400> 793
cgttacatcc tggcgccgaa aggcctgaaa gctggtgacc agattcaatc tggcgttgat 60

gctgcaatca aagcgggtaa caccctgcca atgcgcaaca tcccggttgg ttcaacggtc 120

cacaacgtag aaatgaaacc aggtaaaggc ggccagctgg ctcgttctgc tggtgcctac 180

gttcaaatcg ttgctcgtga cggttcctac gtaactctgc gtctgcgtct gcgctcaggc 240

gaaatgcgta aagttccagt tgattgccgc gccaccttgg gtgaagtcgg taacgctgaa 300

catatgcttc gcgttctggg taaagcaggt gctgcacgt 339

<210> 794
<211> 333
<212> DNA
<213> Serratia proteamaculans sbsp. proteamaculans

<400> 794
cgttatatcc tggcgccgaa aggtctgaaa gctggtgacc agatccaatc tggcgttgat 60

gctgcaatca aggtaggtaa cgccctgcca atgcgcaaca tcccagtggg ttcaacggtt 120

cataacgtag aaatgaaacc aggtaaaggc ggtcaaatcg ctcgctctgc tggtgcctac 180

gttcagatcg ttgcacgtga tggttcctac gtaactctgc gtctgcgctc aggcgaaatg 240

cgtaaagttc cagttgattg ccgcgccacc ttaggtgaag tcggtaacgc tgaacatatg 300

cttcgcgttc tgggtaaagc aggtgctgca cgt 333

<210> 795
<211> 333
<212> DNA
<213> Serratia quinivorans

<400> 795
cgttatatcc tggcgccgaa aggtctgaaa gctggtgacc agatccaatc tggtgttgat 60

gctgcaatca aggtaggtaa cgccctgcca atgcgcaaca tcccagtggg ttcaacggtt 120

cataacgtag aaatgaaacc aggtaaaggc ggtcaaatcg ctcgctctgc tggtgcctac 180

gttcagatcg ttgcacgtga tggttcctac gtaactctgc gtctgcgctc aggcgaaatg 240

cgtaaagttc cagttgattg ccgcgccacc ctgggtgaag tcggtaacgc tgaacatatg 300

cttcgcgttc tgggtaaagc aggtgctgca cgt 333

<210> 796
<211> 333
<212> DNA
<213> Serratia rubidaea

<400> 796
cgttacatcc tggcgccgaa aggcctgaaa gctggtgacc agatccaatc tggtgttgat 60

gctgcgatca aagtgggcaa cgccctgcca atgcgcaaca tcccagttgg ttcaacggtt 120

cacaacgtag aaatgaaacc aggtaaaggc ggccagatgg cgcgttctgc cggtgcctac 180

gttcagatcg ttgctcgtga tggttcctac gtaactctgc gtctgcgctc tggcgaaatg 240

cgtaaagttc ctgcagattg ccgcgccacc ctgggtgaag tcggtaactc agaacacatg 300

ctgcgcgtcc tgggtaaagc aggtgctgca cgt 333

<210> 797
<211> 333
<212> DNA
<213> Serratia ureilytica

<400> 797
cgttacatcc tggcgccgaa aggcctgaaa gctggtgacc agattcaatc tggcgttgat 60

gctgcaatca aagcgggtaa caccctgccg atgcgcaaca ttccagttgg ttcaacggtt 120

cacaacgtag aaatgaaacc aggcaaaggc ggccagatgg ctcgctccgc tggtgcctac 180

gttcagatcg ttgctcgtga tggttcctac gtaactctgc gtctgcgctc cggcgaaatg 240

cgtaaagttc cagctgattg ccgcgccacc ctgggtgaag tcggtaacgc tgaacacatg 300

cttcgcgttc tgggtaaagc aggtgctgca cgt 333

<210> 798

<211>  333
<212>  DNA
<213>  Shigella boydii

<400>  798
cgttacatcc tggcccctaa aggcctgaaa gctggcgacc agattcagtc tggcgttgat      60

gctgcaatca aaccaggtaa caccctgccg atgcgcaaca tcccggttgg ttctactgtt     120

cataacgtag aaatgaaacc aggtaaaggc ggtcagctgg cacgttccgc tggtacttac     180

gttcagatcg ttgctcgtga tggtgcttat gtcaccctgc gtctgcgttc tggtgaaatg     240

cgtaaagtag aagcagactg ccgtgcaact ctgggcgaag ttggcaatgc tgagcatatg     300

ctgcgcgttc tgggtaaagc aggtgctgca cgc                                  333


<210>  799
<211>  333
<212>  DNA
<213>  Shigella dysenteriae T

<400>  799
cgttacatcc tggcccctaa aggcctgaaa gctggcgacc agattcagtc tggcgttgat      60

gctgcaatca aaccaggtaa caccctgcct atgcgcaaca tcccggttgg ttctactgtt     120

cataacgtag aaatgaaacc aggtaaaggc ggtcagctgg cacgttccgc tggtacttac     180

gttcagatcg ttgctcgtga tggtgcttat gtcaccctgc gtttgcgttc tggtgaaatg     240

cgtaaagtag aagcagactg ccgtgcaact ctgggcgaag ttggcaatgc tgagcatatg     300

ctgcgcgttc tgggtaaagc aggtgctgca cgc                                  333


<210>  800
<211>  333
<212>  DNA
<213>  Shigella flexneri

<400>  800
cgttacatcc tggcccctaa aggcctgaaa gctggcgacc agattcagtc tggcgttgat      60

gctgcaatca aaccaggtaa caccctgccg atgcgcaaca tcccggttgg ttctactgtt     120

cataacgtag aaatgaaacc aggtaaaggc ggtcagctgg cacgttccgc tggtacttac     180

gttcagatcg ttgctcgtga tggtgcttat gtcaccctgc gtctgcgttc tggtgaaatg     240

cgtaaagtag aagcagactg ccgtgcaact ctgggcgaag ttggcaatgc tgagcatatg     300

ctgcgcgttc tgggtaaagc aggtgctgca cgc                                  333


<210>  801
<211>  333
<212>  DNA
<213>  Shigella sonnei

<400>  801
cgttacatcc tggcccctaa aggcctgaaa gctggcgacc agattcagtc tggcgttgat      60

gctgcaatca aaccaggtaa caccctgccg atgcgcaaca tcccggttgg ttctactgtt     120

cataacgtag aaatgaaacc aggtaaaggc ggtcagctgg cacgttccgc tggtacttac     180

gttcagatcg ttgctcgtga tggtgcttat gtcaccctgc gtctgcgttc tggtgaaatg 240

cgtaaagtag aagcagactg ccgtgcaact ctgggcgaag ttggcaatgc tgagcatatg 300

ctgcgcgttc tgggtaaagc aggtgctgca cgc 333

<210> 802
<211> 333
<212> DNA
<213> Tatumella ptyseos T

<400> 802
cgttatatcc tggcccctaa aggtctgaaa gctggcgacc agatccaatc tggtagtgat 60

gctgcgatta aagcaggtaa caccctgccg atgcgtaata tcccggtcgg ttcaactgta 120

cataacgtag aaatgaaacc aggtaaaggc ggtcagattg ctcgctctgc tggtacttac 180

gtgcagatcg ttgcgcgtga tggttcctac gtaaccctgc gtctgcgttc aggtgaaatg 240

cgtaaagtcg aagctgactg ccgcgcaaca ctgggcgaag tcggtaacgc tgagcatatg 300

ctgcgcgttc tgggtaaagc aggtgcaact cgc 333

<210> 803
<211> 333
<212> DNA
<213> Trabulsiella guamensis T

<400> 803
cgttacatcc tggcccctaa aggcctgaaa gcgggcgacc agattcagtc tggcgttgat 60

gctgcaatca aagcgggtaa caccctgccg atgcgcaata tcccggttgg ttctaccgtt 120

cataacgtag aaatgaaacc aggtaaaggc ggtcagctgg cgcgttctgc tggtacttac 180

gttcagatcg ttgcacgtga tggtgcttat gtcaccctgc gtctgcgttc tggtgaaatg 240

cgtaaagtcg aagcagactg ccgcgcaacc ctgggcgaag ttggcaatgc tgagcatatg 300

ctgcgcgttc tgggtaaagc aggtgctgca cgt 333

<210> 804
<211> 333
<212> DNA
<213> Xenorhabdus beddingii

<400> 804
cgttatatcc ttgcgcctaa aggcctgaaa gcgggtgatc aaatccagtc tggtgctgat 60

tcagcaatca aggcaggtaa cgctctgcca atgcgcaata tcccggttgg ttctactgtt 120

cacaacatag aaatgaaacc aggtaaaggt ggtcagctgg ctcgttcagc aggtgcttat 180

gcacagatcg ttgcacgtga cggttcttat gtaaccctac gtctgcgttc tggcgaaatg 240

cgtaaagtac tgtctgattg ccgtgctact ttaggtgaag ttggcaacgc agaacatatg 300

ctgcgcgttc tgggtaaagc cggtgcaagc cgc 333

<210> 805
<211> 333
<212> DNA
<213> Xenorhabdus ehlersii

<400> 805
cgttacatcc ttgcacctaa gggcctgaaa gcgggtgatc aaatccagtc tggtgctgat 60

tcagcgatca aggcgggtaa cgccctgcca atgcgcaaca ttccggttgg ttctactgtt 120

cacaacatag aaatgaaacc aggtaaaggt ggccagttgg ctcgttcagc aggtgcttat 180

gctcagatcg ttgcacgtga tggttcttat gtgaccctgc gtctgcgttc tggtgaaatg 240

cgtaaagtac tgtctgattg ccgcgctact ttaggtgaag ttggtaacgc agaacatatg 300

ttgcgcgttc tgggtaaagc tggtgcaagc cgc 333

<210> 806
<211> 333
<212> DNA
<213> Xenorhabdus innexi

<400> 806
cgttacatcc tggctcctaa aggcctgaaa gctggcgatc aaatccagtc tggtgcagat 60

tctgcaatca aagctggtaa tgctctgcca atgcgcaata tcccggttgg ttctactgtt 120

cacaacatag aaatgaaacc aggtaaaggt ggccagctgg ctcgttcagc aggtacttat 180

gctcagatcg ttgcacgtga tggttcttat gtaaccctgc gtctgcgttc tggtgaaatg 240

cgtaaagtac tggctgactg ccgtgcgact ttaggtgaag ttggtaacgc agaacacatg 300

ctacgcgttc tgggtaaagc tggtgcaagc cgc 333

<210> 807
<211> 333
<212> DNA
<213> Xenorhabdus japonica

<400> 807
cgttacatcc ttgcgcctaa aggcctgaaa gcaggtgatc aaatccagtc tggtgctgat 60

tcagcaatca aagctggtaa cgctttacca atgcgcaata tcccggttgg ttctactgtt 120

cacaacatag aaatgaaacc aggtaaaggt ggtcaactgg ctcgttcagc aggtgcttat 180

gctcagatcg ttgcacgtga tggttcttat gtaaccctgc gtttgcgttc tggtgaaatg 240

cgtaaagtgc tgtctgactg ccgtgctact ttgggtgaag ttggtaacgc tgaacacatg 300

ctgcgcgttc tgggtaaagc tggtgcaagc cgc 333

<210> 808
<211> 333
<212> DNA
<213> Xenorhabdus poinarii

<400> 808
cgttacatcc ttgcccctaa aggcctgaaa gcaggtgatc aaatccagtc tggtgctgat 60

tcagcaatta agacgggtaa caccctgcca atgcgcaata tcccggttgg ttctactgtt 120

cataacatag aaatgaaacc aggtaaaggc ggacagctgg ctcgctcagc cggtgcttat 180

gctcaaatcg ttgcacgtga tggttcttat gtaaccctgc gtctgcgttc tggtgaaatg 240

cgtaaagtac tgtctgactg ccgtgctact ttaggtgaag ttggtaacgc agaacatatg 300

ctgcgcgttc tgggtaaagc tggtgcaagc cgc 333

<210> 809
<211> 333
<212> DNA
<213> Xenorhabdus szentirmaii

<400> 809
cgttatatcc ttgcgcctaa aggcctgaaa gcaggtgatc aaatccagtc tggtgctgat 60

tcagcaatca aagctggtaa cacactgcca atgcgcaata tcccggttgg ttctactgtt 120

cataacatag aaatgaaacc aggaaaaggt ggccagctgg ctcgttcagc aggtgcttat 180

gctcagatcg ttgctcgtga tggttcttat gtgaccctgc gtctgcgttc tggtgaaatg 240

cgtaaagtgc tgtctgactg ccgtgctact ttaggtgaag ttggtaacgc tgagcacatg 300

ctgcgtgttc tgggtaaagc tggtgcaagc cgc 333

<210> 810
<211> 333
<212> DNA
<213> Yersinia aldovae

<400> 810
cgttatatcc tggcgccgaa aggcctgaaa gctggtgacc agattcaatc tggtgttgat 60

gctgcaatta aagcgggtaa caccctgcct atgcgtaaca tcccagttgg ttcaacggtt 120

cataacgtag aaatgaaacc aggtaaaggc ggccaattgg ctcgttctgc tggtgcatac 180

gttcagatcg ttgctcgtga cggttcctac gttactctgc gtctgcgctc cggcgaaatg 240

cgcaaagttc tagctgattg ccgcgccacc ttaggtgaag tcggtaacgc tgaacatatg 300

ctgcgtgtcc tgggtaaagc aggtgccagt cgt 333

<210> 811
<211> 333
<212> DNA
<213> Yersinia bercovieri

<400> 811
cgttacatcc tggcgccgaa aggcctgaaa gctggtgacc agattcaatc tggcgttgat 60

gctgcaatta aagcaggtaa taccctgcct atgcgtaaca tcccagttgg ttcaacggtt 120

cataacgtag aaatgaaacc aggtaaaggc ggccaattgg ctcgttctgc tggtgcatac 180

gttcagatcg ttgctcgtga cggttcctac gttactctgc gtctgcgctc cggcgaaatg 240

cgcaaggttc tagctgattg ccgcgccacc ttaggtgaag tcggtaacgc tgaacacatg 300

ctgcgtgtcc tgggtaaagc aggtgctagt cgt 333

<210> 812
<211> 333
<212> DNA
<213> Yersinia enterocolitica sbsp. enterocolitica

<400> 812
cgttacatcc tggcgccgaa aggcctgaaa gctggtgacc agattcaatc tggcgtagat 60

gctgcaatta aagcaggtaa caccctgcct atgcgtaaca tcccagttgg ttcaacggtt 120

cataacgtag aaatgaaacc aggtaaaggc ggccaattgg ctcgttctgc tggtgcatac 180

gttcagatcg ttgctcgtga cggttcctac gttactctgc gtctgcgctc cggcgaaatg 240

cgcaaagttc tagctgattg ccgcgccacc ttaggtgaag tcggtaacgc tgaacacatg 300

ctgcgtgtcc tgggtaaagc aggtgctagt cgt 333

<210> 813
<211> 333
<212> DNA
<213> Yersinia frederiksenii

<400> 813
cgttatatcc tggcgcctaa aggcctgaaa gctggtgacc agattcaatc tggcgttgat 60

gctgcaatta aagcaggtaa caccctgcct atgcgtaaca tcccagttgg ttcaacggtt 120

cataacgtag aaatgaaacc aggtaaaggc ggccaactgg ctcgttctgc tggtgcatac 180

gttcagatcg ttgctcgtga cggttcctac gttactctgc gtctgcgctc cggcgaaatg 240

cgcaaggttc tagctgattg ccgcgccacc ttaggtgaag tcggtaacgc tgaacacatg 300

ctgcgtgtcc tgggtaaagc aggtgccagt cgt 333

<210> 814
<211> 333
<212> DNA
<213> Yersinia intermedia

<400> 814
cgttatatcc tggcgccaaa aggcctgaaa gctggtgacc agattcaatc tggcgttgat 60

gctgcaatta aagcaggtaa caccctgcct atgcgtaaca tcccagttgg ttcaacggtt 120

cataacgtag aaatgaaacc aggtaaaggc ggccaattgg ctcgttctgc tggtgcatac 180

gttcagatcg ttgctcgtga cggttcctac gttactctgc gtctgcgctc cggcgaaatg 240

cgcaaagttc aagctgattg ccgcgccacc ttaggtgaag tcggtaacgc tgaacacatg 300

ctgcgtgtgc tgggtaaagc aggtgctagt cgt 333

<210> 815
<211> 333
<212> DNA
<213> Yersinia kristensenii

<400> 815
cgttatatcc tggcgcctaa aggcctgaaa gctggtgacc agattcaatc tggcgttgat 60

gctgcaatta aagcaggtaa caccctgcct atgcgtaaca tcccagttgg ttcaacggtt 120

cataacgtag aaatgaaacc aggtaaaggc ggccaattgg ctcgttctgc tggtgcatac 180

gttcagatcg ttgctcgtga cggttcctac gttactctgc gtctgcgctc cggcgaaatg 240

cgcaaagttc tagctgattg ccgcgccacc ttaggtgaag tcggtaacgc tgaacacatg 300

ctgcgtgtgc tgggtaaagc aggtgctagt cgt 333

<210> 816
<211> 333
<212> DNA
<213> Yersinia mollaretii

<400> 816
cgttacatcc tggcgccgaa aggcctgaaa gctggtgacc agatccaatc tggcgttgat 60

gctgcaatta aagcgggtaa caccctgcct atgcgtaaca tcccagttgg ttcaacggtt 120

cataacgtag agatgaaacc aggtaaaggc ggccaattgg ctcgttctgc tggtgcatac 180

gttcagatcg ttgctcgtga cggttcctac gttactctgc gtctgcgctc cggcgaaatg 240

cgcaaggttc tagctgattg ccgcgccacc ttaggtgaag tcggtaacgc tgaacacatg 300

ctgcgtgtgc tgggtaaagc aggtgctagt cgt 333

<210> 817
<211> 333
<212> DNA
<213> Yersinia pestis T

<400> 817
cgttatatcc tggcgccgaa aggcctgaag gctggtgacc agattcaatc tggcgttgat 60

gctgcaatta aagcaggtaa caccctgcct atgcgtaaca tcccagttgg ttcaacggtt 120

cataacgtag aaatgaaacc aggtaaaggc ggccagttgg ctcgttctgc tggtgcatac 180

gttcagatcg ttgctcgtga cggttcctac gttactctgc gtctgcgctc cggcgaaatg 240

cgcaaggttc aagctgattg ccgcgccacc ttaggtgaag ttggtaatgc tgaacacatg 300

ctgcgtgtcc tgggtaaagc aggtgctagt cgt 333

<210> 818
<211> 333
<212> DNA
<213> Yersinia pseudotuberculosis sbsp. pseudotuberculosis

<400> 818
cgttatatcc tggcgccgaa aggcctgaag gctggtgacc agattcaatc tggcgttgat 60

gctgcaatta aagcaggtaa caccctgcct atgcgtaaca tcccagttgg ttcaacggtt 120

cataacgtag aaatgaaacc aggtaaaggc ggccagttgg ctcgttctgc tggtgcatac 180

gttcagatcg ttgctcgtga cggttcctac gttactctgc gtctgcgctc cggcgaaatg 240

cgcaaggttc aagctgattg ccgcgccacc ttaggtgaag ttggtaatgc tgaacacatg 300

ctgcgtgtcc tgggtaaagc aggtgctagt cgt 333

<210> 819
<211> 333
<212> DNA
<213> Yersinia rohdei

<400> 819
cgttatatcc tggcgcctaa aggcctgaaa gctggtgacc agattcaatc tggcgttgat 60

gctgcaatta aagcaggtaa caccctgcct atgcgtaaca tcccagttgg ttcaacggtt 120

cataacgtag aaatgaaacc aggtaaaggc ggccaattgg ctcgttctgc tggtgcatac 180

gttcagatcg ttgctcgtga cggttcctac gttactctgc gtctgcgctc cggcgaaatg 240

cgcaaggttc tagctgattg ccgcgccacc ttaggtgaag tcggtaacgc tgaacacatg 300

ctgcgtgtcc tgggtaaagc aggtgccagt cgt 333

<210> 820
<211> 333
<212> DNA
<213> Yersinia ruckeri

<400> 820
cgttatatcc tggcgcctaa aggcctgaaa gctggtgacc agatccaatc tggtgttgat 60

gctgcaatta aagcaggtaa taccctgcct atgcgtaaca tcccagttgg ttcaacggtt 120

cacaacgtag aaatgaaacc aggtaaaggc ggccagttgg cgcgttctgc tggtgcatac 180

gttcagatcg ttgctcgtga cggttcctac gttactctgc gtctgcgctc cggcgaaatg 240

cgcaaggttc tagctgattg ccgcgccacc ttaggtgaag tcggtaatgc tgaacacatg 300

ctgcgtgtgc tgggtaaagc aggtgcaagt cgt 333

<210> 821
<211> 333
<212> DNA
<213> Yokenella regensburgei T

<400> 821
cgttacattc tggcccctaa aggcctgaaa gctggcgacc agattcagtc tggcgttgat 60

gctgcaatca aagcgggtaa caccctgccg atgcgcaata tcccggttgg ttctaccgtt 120

cataacgtag aaatgaaacc aggtaaaggc ggtcagctgg cgcgttctgc tggtacttac 180

gttcagatcg ttgctcgcga tggtgcttat gtcaccctgc gtctgcgttc tggtgaaatg 240

cgtaaagtcg aagcagactg ccgcgctact ctgggcgaag ttggcaatgc tgagcatatg 300

ctgcgcgttc tgggtaaagc aggtgctgca cgc 333

<210> 822
<211> 306
<212> DNA
<213> Brenneria alni

<400> 822
attcttgaag cccgtggcct caacgttacc atcatgaaac tggacccgta tatcaacgtg 60

gatccgggca cgatgagccc gacacagcac ggtgaagtgt tcgtcaccga agacggtgcg 120

gaaaccgatc tcgatctggg ccactatgag cgcttcattc gcaccaagat gtcgcgccgt 180

aataacttca ccgctggccg tatatactct gacgtactgc gtaaagagcg ccgtggtgac 240

tatctgggcg ccactattca ggttatcccc catattacca atgcgatcaa agagcgcatc 300

cttgag 306

<210> 823
<211> 306

<212>  DNA
<213>  Brenneria quercina

<400>  823
attctcgaag cccgcggcct caacgtgacc atcatgaaac tggacccgta tatcaatgtg      60

gatccaggga caatgagtcc tacgcagcac ggtgaagtgt tcgtcaccga agatggcgcc     120

gaaaccgatc tcgacctggg ccactacgag cgttttattc gcactaaaat gacgcgccgc     180

aataacttca ccaccggacg gatctattct gacgtcctgc gcaaagagcg ccgtggtgat     240

tatctgggtg cgaccattca ggttatcccc catatcacca acgcgatcaa agagcgcatc     300

atcgaa                                                                306

<210>  824
<211>  306
<212>  DNA
<213>  Brenneria rubrifaciens

<400>  824
attcttgaag cccgtggcct caacgttacc atcatgaaac tggacccgta tatcaacgtg      60

gatccgggca cgatgagccc gacgcaacac ggcgaagtgt ttgtcaccga agacggcgcg     120

gaaaccgatc tagacttggg ccattacgag cgtttcattc gcaccaagat gtcgcgccgt     180

aacaacttca ccaccggtcg tatctactcc gacgtattgc gcaaagagcg ccgtggcgac     240

tatctgggcg ccacgattca ggttatcccg cacatcacca acgccattaa agaacgcatt     300

ctggaa                                                                306

<210>  825
<211>  306
<212>  DNA
<213>  Brenneria salicis T

<400>  825
attcttgaag cccgtggcct caacgttacc atcatgaaac tggacccgta tatcaacgtg      60

gatccgggca cgatgagccc gacgcaacac ggcgaagtgt ttgtcaccga agacggcgcc     120

gaaaccgatc ttgacctggg tcactacgaa cgtttcattc gcaccaagat gtcgcgccgc     180

aataacttca ccactggccg gatctactcc gacgtgttgc gcaaagagcg ccgcggcgac     240

tatctgggcg ccaccatcca ggttattccc catatcacca atgccatcaa agaacgcatt     300

ctggaa                                                                306

<210>  826
<211>  306
<212>  DNA
<213>  Budvicia aquatica T

<400>  826
attcttgaag ctcgtggctt aaatgtcact atgatgaaac tggatcccta tattaacctc      60

gatccaggta ccatgagccc gattcaacac ggtgaagtct ttgtgaccga agatggtgca     120

gaaaccgatc tggacttagg ccactatgag cgttttatca gaacgaagat gacgcgtcgt     180

aataacttta ctaccgggcg tatttattcc gacgtcttac gtaaagagcg ccgtggtgac     240

taccttggtg caaccgttca ggttattccc catattacta atgcgattaa agagcgcgtc 300

attgcc 306

<210> 827
<211> 306
<212> DNA
<213> Buttiauxella agrestis T

<400> 827
attctcgaag cccgtggcct caacgtatcc atcatgaaac tcgatccgta catcaacgtc 60

gatccgggta ccatgagccc tattcaacat ggggaagtgt tcgttacaga agacggcgct 120

gaaaccgacc ttgatttagg tcactacgag cgtttcattc gcaacaaaat gacacgtcgt 180

aacaacttca ccactggccg tatttactca gaagttctgc gtaaagagcg ccgtggcgac 240

tacctgggcg cgactgttca ggttattcca cacatcacga acgcaatcaa agaacgcatc 300

cttgaa 306

<210> 828
<211> 306
<212> DNA
<213> Buttiauxella brennerae

<400> 828
attctcgaag cccgtggcct caacgtatcc atcatgaaac tcgatccgta catcaacgtc 60

gatccgggta ccatgagccc tattcaacat ggggaagtgt tcgttacaga agacggcgct 120

gaaaccgacc tggatttagg tcactacgag cgtttcattc gcaacaaaat gacacgtcgt 180

aacaacttta ccactggtcg tatttactca gacgttctgc gtaaagaacg ccgtggcgac 240

tatctgggcg cgacagtcca ggttattcca cacatcacta acgctatcaa agaacgcatc 300

cttgaa 306

<210> 829
<211> 306
<212> DNA
<213> Buttiauxella ferragutiae

<400> 829
attctcgaag cccgtggcct caacgtatcc atcatgaaac tcgatccgta catcaacgtc 60

gatccgggta ccatgagccc tattcaacat ggggaagtgt tcgttacaga agacggcgct 120

gaaaccgacc tggatttagg tcactacgag cgtttcattc gcaacaaaat gacgcgtcgt 180

aacaacttca ccactggccg tatttactca gatgttctgc gtaaagaacg ccgtggcgac 240

tatctgggcg cgactgtcca ggttattcca cacatcacga acgctatcaa agaacgcatc 300

cttgaa 306

<210> 830
<211> 306
<212> DNA
<213> Buttiauxella gaviniae

<400>  830
attctcgaag cccgtggcct caacgtatcc atcatgaaac tcgatccgta tatcaacgtc      60

gatccgggta ccatgagccc tattcaacat ggggaagtgt tcgttacaga agacggcgct     120

gaaaccgacc tggatttagg tcactacgag cgtttcattc gcaacaaaat gacgcgtcgt     180

aacaacttca ccactggccg tatttactca gaagttctgc gtaaagagcg ccgtggcgac     240

tatctgggtg cgaccgtcca ggttattccg catatcacga acgctatcaa agaacgcatc     300

cttgaa                                                                306

<210>  831
<211>  306
<212>  DNA
<213>  Buttiauxella izardii

<400>  831
attctcgaag cccgtggcct caacgtatcc atcatgaaac tcgatccgta catcaacgtc      60

gatccgggta ccatgagccc tattcaacat ggggaagtgt tcgttacaga agacggcgct     120

gaaaccgacc tggatttagg tcactacgag cgtttcattc gcaacaaaat gacgcgtcgt     180

aacaacttca ccactggccg tatttactca gaagttctgc gtaaagagcg ccgtggcgac     240

tacctgggcg caaccgttca ggttattcca cacatcacga acgcaatcaa agaacgcatc     300

cttgaa                                                                306

<210>  832
<211>  306
<212>  DNA
<213>  Buttiauxella noackiae

<400>  832
attctcgaag cccgtggcct caacgtatcc atcatgaaac tcgatccgta tatcaacgtc      60

gatccgggta ccatgagccc tattcaacat ggggaagtgt tcgttacaga agacggcgct     120

gaaaccgacc tggatttagg tcactacgag cgtttcattc gcaacaaaat gacgcgtcgt     180

aacaacttca ccactggccg tatctactca gacgttctgc gtaaagaacg ccgtggcgac     240

tatctgggcg caacagtcca ggttattcca cacatcacta acgctatcaa agaacgcatc     300

cttgaa                                                                306

<210>  833
<211>  306
<212>  DNA
<213>  Buttiauxella warmboldiae

<400>  833
attctcgaag cccgtggcct caacgtatcc atcatgaaac tcgatccgta catcaatgtc      60

gatccgggta ccatgagccc tattcaacat ggggaagtgt tcgttaccga agacggcgct     120

gaaaccgacc tggatttagg tcactacgag cgtttcattc gcaacaaaat gacgcgtcgt     180

aataacttca ccactggccg tatttactca gacgtgctgc gtaaagaacg ccgtggcgac     240

tacctgggcg ctaccgttca ggttattcca cacatcacca acgccattaa agaacgcatc     300

cttgaa                                                              306

<210>  834
<211>  306
<212>  DNA
<213>  Cedecea davisae  T

<400>  834
attcttgaag cccgtggcct caacgtatcc atcatgaaac tcgatccgta catcaacgtc      60

gatccgggta ccatgagccc tatccagcat ggggaagtgt tcgttacaga agacggcgct     120

gaaaccgacc tcgatttggg tcactacgag cgttttatcc gtaacaagat gagccgccgt     180

aacaatttca ccaccggccg tatctactct gacgtactgc gtaaagagcg ccgtggtgac     240

tatcttggcg cgaccgtgca ggttatcccg cacatcacca acgccattaa agaacgtatt     300

ctggaa                                                              306

<210>  835
<211>  306
<212>  DNA
<213>  Cedecea lapagei

<400>  835
attcttgaag cccgtggcct caacgtatcc atcatgaaac tcgatccgta catcaacgtc      60

gatccgggta ccatgagccc tattcaacat ggggaagtgt tcgttaccga agacggcgct     120

gaaaccgatc tggatttggg tcactacgag cgttttatcc gtaacaagat gagccgccgt     180

aacaacttca ccaccggccg tatctactct gacgttctgc gtaaagagcg ccgtggtgac     240

tatcttggcg cgaccgtgca ggttatcccg cacatcacca acgccattaa agaacgtatt     300

ctggaa                                                              306

<210>  836
<211>  306
<212>  DNA
<213>  Cedecea neteri

<400>  836
attcttgaag cccgtggcct caacgtatcc atcatgaaac tcgatccgta tatcaacgtc      60

gatccgggta ccatgagccc tattcaacat ggggaagtgt tcgttaccga agacggcgct     120

gaaaccgacc tggatttggg tcactacgag cgttttatcc gtaacaagat gagccgccgt     180

aacaacttca ccaccggccg tatctactct gacgttctgc gtaaagagcg ccgtggtgac     240

tatcttggcg cgaccgtgca ggttatcccg cacatcacca acgccattaa agaacgtatt     300

ctggaa                                                              306

<210>  837
<211>  306
<212>  DNA
<213>  Citrobacter amalonaticus

<400>  837
attcttgaag cccgtggcct caacgtgacc atcatgaaac tggatccgta tatcaacgtc      60

gatccaggca ccatgagccc aatccaacac ggggaagtgt tcgttactga agacggcgct 120

gaaaccgacc tggacctggg tcactacgag cgtttcattc gcaccaaaat gagccgccgc 180

aacaacttca ccacgggtcg tatttactct gacgttctgc gcaaagagcg ccgtggcgac 240

tatctgggcg cgaccgtaca ggttatcccg cacatcacca atgccattaa agaacgcgtg 300

ctggaa 306

<210> 838
<211> 306
<212> DNA
<213> Citrobacter braakii

<400> 838
attcttgagg ctcgtggcct caacgtgacc atgatgaaac tggatccgta catcaacgtc 60

gatccaggta ctatgagtcc aatccaacac ggggaagtgt tcgttactga agacggcgct 120

gaaaccgacc tggacctggg tcactatgag cgtttcattc gtaccaaaat gactcgccgc 180

aataacttca ctaccggtcg aatttactcc gatgtcctgc gcaaagagcg ccgtggcgac 240

tatctgggtg caaccgtaca ggttatcccg cacatcacca acgccattaa agagcgtgtc 300

cttgcc 306

<210> 839
<211> 306
<212> DNA
<213> Citrobacter diversus

<400> 839
attcttgaag cccgtggcct caacgtgacc atcatgaaac tggatccgta catcaacgtc 60

gatcctggta ctatgagtcc aatccaacac ggggaagtgt tcgttactga agacggcgcc 120

gaaaccgatc tggacctggg tcactatgag cgcttcattc gcaccaagat gagccgccgc 180

aacaacttca ccacgggtcg tatttactct gacgttctgc gtaaagaacg ccgtggcgac 240

tatctgggcg caaccgtaca ggttatcccg cacatcacca acgcgattaa agagcgcatc 300

atcgaa 306

<210> 840
<211> 306
<212> DNA
<213> Citrobacter farmeri

<400> 840
attcttgaag cccgtggcct caacgtgact atcatgaaac tggatccgta tatcaacgtc 60

gatccgggta ccatgagccc aatccagcac ggggaagtgt tcgttactga agacggcgct 120

gaaaccgacc tggacctggg tcactacgag cgtttcattc gcaccaaaat gagccgccgc 180

aacaacttca ctacgggccg tatctattct gacgttctgc gcaaggagcg tcgtggtgac 240

tatctgggcg cgaccgtaca ggtaatcccg cacatcacca acgctatcaa agaacgcgtg 300

ctggaa 306

<210>  841
<211>  306
<212>  DNA
<213>  Citrobacter freundii T

<400>  841
attcttgaag cccgtggcct caatgtaacc atgatgaaac tggatccgta catcaacgtc      60

gatccaggta ctatgagccc aatccaacac ggggaagtgt tcgttaccga agacggcgct     120

gaaaccgacc tggacctggg tcactatgag cgcttcattc gtaccaaaat gagccgccgc     180

aacaacttca ctaccggtcg tatctactcc gacgttctgc gtaaagaacg ccgtggcgac     240

tacctgggcg cgaccgttca ggttatcccg cacatcacga atgccattaa agagcgagtt     300

ctggaa                                                                306

<210>  842
<211>  306
<212>  DNA
<213>  Citrobacter gillenii

<400>  842
attcttgaag cccgtggcct caatgtaacc atgatgaaac tggatccgta catcaacgtc      60

gatccaggta ctatgagccc aatccaacac ggggaagtgt tcgttaccga agacggcgct     120

gaaaccgatc tggacctggg tcactacgag cgtttcatcc gcaccaaaat gagccgccgc     180

aacaacttca ccaccggtcg tatttactct gacgttctgc gtaaagaacg tcgtggcgac     240

tatctgggcg caaccgttca ggttatcccg cacatcacga atgcgatcaa agaacgcgtt     300

ctggaa                                                                306

<210>  843
<211>  306
<212>  DNA
<213>  Citrobacter koseri

<400>  843
attcttgaag cccgtggcct caacgtgacc atcatgaaac tggatccgta catcaacgtc      60

gatcctggta ctatgagtcc aatccaacac ggggaagtgt tcgttactga agacggcgcc     120

gaaaccgatc tggacctggg tcactatgag cgcttcattc gcaccaagat gagccgccgc     180

aacaacttca ccacgggtcg tatttactcc gacgttctgc gtaaagaacg ccgtggcgac     240

tatctgggcg caaccgtaca ggttatcccg cacatcacca acgcgattaa agagcgcatc     300

atcgaa                                                                306

<210>  844
<211>  306
<212>  DNA
<213>  Citrobacter murliniae

<400>  844
attcttgagg cacgtggcct caacgtgacc atgatgaaac tggatccgta tatcaacgtt      60

gatccaggta ctatgagtcc aatccaacac ggggaagtgt tcgttactga agacggcgct     120

gaaaccgacc tggacctggg tcactacgag cgtttcattc gtaccaaaat gactcgccgc     180

aataacttca ctaccgggcg tatctactcc gatgtcctgc gcaaagagcg ccgtggcgac 240

tatctgggcg caaccgtaca ggttattcca cacatcacta acgcgattaa agagcgcgtc 300

cttgcc 306

<210> 845
<211> 306
<212> DNA
<213> Citrobacter rodentium

<400> 845
attcttgaag cccgtggcct caatgtgact atcatgaagc tggatccgta tatcaacgtc 60

gatccgggca ccatgagccc aatccaacac ggggaagtgt tcgttactga agacggcgct 120

gaaaccgacc tggacctggg ccactacgag cgcttcattc gcaccaaaat gagccgccgc 180

aacaacttca ccacgggccg catctactct gacgtactgc gtaaagagcg tcgtggcgac 240

tatctgggcg cgaccgtaca ggttatcccg cacatcacta acgctattaa agaacgcgta 300

ctcgaa 306

<210> 846
<211> 306
<212> DNA
<213> Citrobacter sedlakii

<400> 846
attcttgaag cccgtggcct caatgtgacc atgatgaaac tggatccgta catcaacgtc 60

gatcctggca ccatgagccc aacccaacac ggggaagtgt tcgttactga agacggcgct 120

gaaaccgatc tggatcttgg ccactacgag cgtttcatcc gtaccaaaat gacccgtcgt 180

aacaacttta cgactggccg tatctactct gatgttctgc gtaaagagcg ccgtggtgac 240

tatctgggcg caaccgtgca ggttatccca cacatcacca acgccattaa agaacgcgtc 300

cttgcc 306

<210> 847
<211> 306
<212> DNA
<213> Citrobacter werkmanii

<400> 847
attcttgaag cccgtggcct caacgtgacc atgatgaaac tggatccgta catcaacgtc 60

gatccgggta ctatgagccc aatccaacac ggggaagtgt tcgttaccga agacggcgct 120

gaaaccgacc tggacctggg tcactatgag cgtttcatcc gtacgaaaat gagccgccgc 180

aacaacttca ccacgggtcg tatctactct gacgttctgc gtaaagaacg ccgtggcgac 240

tatctgggcg ctactgttca ggttatcccg catatcacca acgcaattaa agagcgcgtt 300

ctggaa 306

<210> 848
<211> 306
<212> DNA

<213>  Citrobacter youngae

<400>  848
attcttgaag cccgtggcct caatgtaacc atgatgaaac tggatccgta catcaacgtc      60

gatccaggta ctatgagccc tatccaacac ggggaagtgt tcgttaccga agacggcgct     120

gaaaccgacc tggacctggg tcactatgag cgtttcattc gtacgaaaat gagccgccgt     180

aacaacttca ccaccggtcg tatctactcc gacgttctgc gtaaagaacg ccgtggcgac     240

tacctgggcg cgaccgttca ggttatcccg cacatcacga atgccattaa agaacgcgtt     300

ctggaa                                                                306

<210>  849
<211>  306
<212>  DNA
<213>  Dickeya chrysanthemi T

<400>  849
attcttgaag cccgtggcct caacgtgacc atcatgaaac tggacccgta tatcaatgtg      60

gatccgggca cgatgagccc gacgcagcac ggcgaagtgt tcgttaccga agatggcgcc     120

gaaaccgatc tcgacttggg tcactatgag cgttttattc gcacaaagat gacgcgccgc     180

aataacttca ccaccggacg catttattct gacgttctgc gtaaggagcg tcggggcgac     240

tatctgggtg cgaccattca ggttatcccg cacatcacca acgccattaa agaacgcatt     300

atcgaa                                                                306

<210>  850
<211>  306
<212>  DNA
<213>  Edwardsiella hoshinae

<400>  850
attcttgaag cccgtggcct caatgtatcc atgatgaagc tcgacccgta catcaacgtc      60

gatccgggta ccatgagtcc gattcagcac ggggaagttt tcgttaccga agacggcgcg     120

gaaaccgatc tggacctggg tcactacgag cgtttcatcc gcaccaagat gtcccgccgc     180

aataatttca ccaccgggcg tatctactcc gatgtgttgc gcaaagagcg ccgcggtgac     240

tatctgggcg ccaccgtaca ggtgatcccg catatcacga atgccattaa agaacgcatc     300

ctggaa                                                                306

<210>  851
<211>  306
<212>  DNA
<213>  Edwardsiella ictaluri

<400>  851
attcttgaag cccgtggcct caatgtatcc atgatgaagc tcgatccgta catcaacgtc      60

gatccgggta ccatgagtcc gatccagcac ggggaagttt tcgttaccga agacggcgcg     120

gaaaccgatc tggacctggg tcactatgag cgcttcatcc gcaccaaaat gtcccgtcgc     180

aataacttta ccaccggacg tatctactcc gatgtgctgc gtaaagagcg tcgcggcgac     240

tatctgggcg ccaccgttca ggtgatcccg catatcacta acgccatcaa agagcgcatc 300

ctggaa 306

<210> 852
<211> 306
<212> DNA
<213> Edwardsiella tarda T

<400> 852
attcttgaag cccgtggcct caatgtatcc atgatgaagc tcgacccgta catcaacgtc 60

gatccgggta ccatgagccc gatccagcac ggggaagttt tcgtcaccga agacggcgcg 120

gaaaccgatc tggacctggg tcactacgag cgtttcattc gcaccaagat gtcccgccgc 180

aataacttca ccaccgggcg tatctactcc gatgtgctgc gcaaagagcg ccgcggcgac 240

tatctgggcg ccaccgtgca ggtgatcccg catatcacta acgccatcaa agagcgcatc 300

ctggaa 306

<210> 853
<211> 306
<212> DNA
<213> Enterobacter aerogenes

<400> 853
attcttgaag cccgtgggct caatgtaact atcatgaaac tggatccgta catcaacgtc 60

gatccgggca ccatgagccc aatccagcac ggggaagtgt tcgttactga agacggcgct 120

gaaaccgatc tggacctggg tcactacgag cgtttcatcc gcaccaagat gactcgccgc 180

aacaacttca ccaccggtcg aatctactcc gacgttctgc gtaaagagcg ccgtggcgac 240

tatctgggcg caaccgtaca ggttatcccg cacatcacca acgccatcaa agagcgcgta 300

ctggct 306

<210> 854
<211> 306
<212> DNA
<213> Enterobacter amnigenus

<400> 854
attcttgaag cccgtggcct caatgtgacc atgatgaaat tggatccgta catcaacgtt 60

gatccaggca ccatgagccc aatccagcac ggcgaagtgt tcgttaccga agacggcgct 120

gaaaccgatc tggacttagg tcactacgag cgcttcattc gcaccaagat gagtcgccgc 180

aacaacttca ctacgggtcg tatctactcc gacgttctgc gtaaagagcg ccgtggcgac 240

tacttaggcg caaccgttca ggttatcccg catatcacta atgcaatcaa agagcgcatc 300

gttgcc 306

<210> 855
<211> 306
<212> DNA
<213> Enterobacter asburiae

<400> 855

attcttgaag cccgtggcct caatgtgacc atgatgaaac tggatccgta catcaacgtc 60

gatccgggca ccatgagccc aatccaacac ggggaagtgt tcgttactga agacggcgct 120

gaaaccgatc tggatcttgg ccactacgag cgtttcatcc gcaccaaaat gacccgtcgt 180

aacaacttca cgactggccg catctactcc gacgttctgc gtaaagagcg ccgtggtgac 240

tacctgggtg caaccgttca ggttatcccg cacatcacta acgcaatcaa agaacgcatt 300

gttgcg 306

<210> 856
<211> 306
<212> DNA
<213> Enterobacter cancerogenus

<400> 856
attcttgaag cccgtggcct caatgtgacc atgatgaaac tggatccgta catcaacgtc 60

gatccgggca ccatgagtcc aatccaacac ggggaagtgt tcgttactga agacggcgct 120

gaaaccgatc tggatcttgg ccactacgag cgtttcatcc gcaccaaaat gacccgtcgt 180

aacaacttca cgactggccg catctactct gacgttctgc gtaaagagcg ccgtggcgac 240

tatctgggcg cgaccgttca ggttatcccg cacattacca atgccatcaa agagcgcatt 300

gtcgcc 306

<210> 857
<211> 306
<212> DNA
<213> Enterobacter cloacae sbsp. cloacae T

<400> 857
attcttgaag cccgtggcct caatgtgacc atgatgaaac tggatccgta catcaacgtc 60

gatcctggca ccatgagccc aatccaacac ggggaagtgt tcgttactga agacggcgct 120

gaaaccgatc tggatcttgg ccactacgag cgtttcattc gcaccaaaat gacccgtcgt 180

aataacttca cgactggccg tatctactcc gacgttctgc gtaaagaacg ccgtggtgat 240

tatctgggtg caaccgtaca ggttatcccg cacatcacta acgctatcaa agaacgtatc 300

attgcg 306

<210> 858
<211> 306
<212> DNA
<213> Enterobacter cloacae sbsp. dissolvens

<400> 858
attcttgaag cccgtggcct caatgtgacc atgatgaaac tggatccgta catcaacgtc 60

gatccgggca ccatgagccc aatccaacac ggggaagtgt tcgttactga agacggcgct 120

gaaaccgatc tggatcttgg ccactacgag cgtttcattc gcaccaaaat gacccgtcgt 180

aacaacttca cgactggccg tatctactcc gacgttctgc gtaaagagcg ccgtggtgat 240

tatctgggtg cgaccgttca ggttatcccg cacatcacta acgctatcaa agaacgcatc 300

attgcg 306

<210> 859
<211> 306
<212> DNA
<213> Enterobacter cowanii

<400> 859
attcttgaag cccgtggcct caaggtcacc atgatgaaac tggatccgta catcaacgtc 60

gatccgggca caatgagccc tatccagcat ggtgaagtgt tcgttaccga agatggcgca 120

gaaaccgatc ttgatttggg tcactacgag cgtttcatcc gcaccaagat gacccgtcgc 180

aacaacttca ccaccggccg tatctactcc gaggtgctgc gtaaagagcg ccgcggcgac 240

tatctcggtg cgaccgtgca ggttatcccc catatcacca acgccatcaa agagcgcatt 300

attgcc 306

<210> 860
<211> 306
<212> DNA
<213> Enterobacter gergoviae

<400> 860
attcttgaag cccgtggcct caacgtaact atcatgaagc tggatccgta catcaacgtc 60

gatccgggca ccatgagccc gacgcagcac ggggaagtgt tcgtgacgga agacggcgct 120

gaaacagacc tggacctggg tcactacgag cgtttcatcc gcaccaagat gacccgccgc 180

aacaacttta ctaccggccg tatctactcc gaagtcctgc gcaaagagcg ccgcggcgac 240

tatctcggcg cgactattca ggttatcccg cacatcacta acgccatcaa agagcgcgtt 300

atcgcg 306

<210> 861
<211> 306
<212> DNA
<213> Enterobacter hormaechei

<400> 861
attcttgaag cccgtggcct caatgtgacc atgatgaaac tggatccgta catcaacgtc 60

gatcctggca ccatgagccc aacccaacac ggggaagtgt tcgttactga agacggcgct 120

gaaaccgatc tggatcttgg ccactacgag cgtttcattc gtaccaaaat gacccgtcgt 180

aacaacttta cgactggccg catctactct gacgttctgc gtaaagagcg ccgcggtgac 240

tatctgggcg cgaccgtgca ggttatccca cacatcacta acgctatcaa agagcgtgtc 300

cttgcc 306

<210> 862
<211> 306
<212> DNA
<213> Enterobacter kobei

<400> 862
attcttgaag cccgtggcct caatgtaagc atgatgaaac tggatccgta catcaacgtc 60

gatccgggta ccatgagccc aattcagcac ggggaagtgt tcgttacaga agacggcgct 120

gaaaccgatc ttgacctggg ccactacgag cgcttcattc gcaccaaaat gagccgtcgt 180

aacaacttca ccaccggtcg tatctactcc gacgttctgc gtaaagagcg ccgtggcgac 240

tatctgggcg cgaccgttca ggttattccg cacatcacca acgctatcaa agagcgcatc 300

atcgaa 306

<210> 863
<211> 306
<212> DNA
<213> Enterobacter ludwigii

<400> 863
attcttgaag cccgtggcct caatgtgacc atgatgaaac tggatccgta catcaacgtc 60

gatccgggca ccatgagtcc aatccaacac ggggaagtgt tcgttactga agacggcgct 120

gaaaccgatc tggatcttgg ccactacgag cgtttcatcc gcactaaaat gacccgtcgt 180

aacaacttca cgacaggccg catctactcc gacgttctgc gtaaagagcg ccgtggtgat 240

tatcttggtg ctaccgttca ggttatccca cacatcacta acgctatcaa agaacgcatt 300

attgcg 306

<210> 864
<211> 306
<212> DNA
<213> Enterobacter nimipressuralis

<400> 864
attcttgaag cccgtggcct caatgtgact atgatgaaac tggatccgta catcaacgtc 60

gatccaggca ccatgagccc aatccagcac ggcgaagtgt tcgttaccga agacggcgct 120

gaaaccgatt tggacttagg tcactacgag cgcttcatcc gtactaaaat gagtcgccgc 180

aacaacttca ccacgggtcg tatctactct gacgttctgc gtaaagaacg ccgtggcgat 240

tacttaggcg caaccgttca ggttatccca cacatcacca acgctattaa agagcgcatc 300

atcgca 306

<210> 865
<211> 306
<212> DNA
<213> Enterobacter pyrinus

<400> 865
attcttgaag cccgtggcct caacgtgacc atcatgaaac tggatccgta tatcaacgtt 60

gatccgggca ccatgagtcc gacgcagcac ggggaagtgt tcgttacgga agacggcgcc 120

gaaaccgacc tggacctggg tcactacgag cgtttcatcc gcaccaagat gactcgccgc 180

aacaacttca ccacgggtcg tatctactct gaagtcctgc gcaaagagcg ccgcggcgac 240

taccttggcg ccacgattca ggttatcccg cacatcacta acgctatcaa agagcgcgtc 300

atcgcc 306

<210> 866

<211>  306
<212>  DNA
<213>  Enterobacter radicincitans

<400>  866
attctcgaag ctcgtggcct taacgtcact atgatgaaac tggatccgta tattaacgtc      60

gatccgggga ctatgagccc aatccagcat ggcgaagtgt tcgttaccga agatggcgct     120

gaaaccgatc ttgatttggg tcactacgag cgcttcattc gcaccaaaat gacgcgtcgc     180

aacaacttca ccaccggtcg catttactcc gaggttctgc gtaaagagcg tcgtggcgac     240

tatctgggcg cgaccgtaca ggttattccg cacatcacta acgctatcaa agagcgcatc     300

attgaa                                                                306

<210>  867
<211>  306
<212>  DNA
<213>  Enterobacter sakazakii

<400>  867
attcttgaag cccgtggcct taacgtgacc atcatgaaac tggatccgta tatcaacgtc      60

gatcctggca ccatgagtcc aatccagcac ggggaagtgt tcgttactga agacggcgct     120

gaaaccgatc tggacctggg gcactatgag cgttttatcc gcacccgcat gacgcgccgc     180

aacaacttca cgacaggccg tatctactct gaagttctgc gcaaagagcg ccgtggcgac     240

tatctgggcg ccaccgttca ggttattccg cacatcacca acgctatcaa agaacgcatt     300

cttgcg                                                                306

<210>  868
<211>  306
<212>  DNA
<213>  Erwinia amylovora T

<400>  868
atccttgaag cgcgtggtct caacgtgacc atcatgaagc tggacccgta catcaacgtg      60

gatccgggca ccatgagccc gacccagcac ggtgaagtgt ttgtcactga tgacggcgcg     120

gaaaccgatc tggatctcgg ccactatgag cgctttattc gtacccggat gagccgccgt     180

aacaacttca ccactggccg tatctactcc gaagtgctgc gcaaagagcg tcgcggtgac     240

tatctgggcg cgaccattca ggtgatcccg cacattacca acgccattaa agaacgcatc     300

atcgaa                                                                306

<210>  869
<211>  306
<212>  DNA
<213>  Erwinia aphidicola

<400>  869
attctcgaag cacgtggtct taatgtgacc atcatgaagc tggacccgta catcaacgtg      60

gatccgggca ccatgagccc gactcagcac ggtgaggtct tcgtgaccga tgacggcgca     120

gagaccgatc ttgacctcgg tcactacgag cgcttcatcc gtaccaaaat gagccgtcgt     180

aataacttca ccactggccg tatctactca gaagtgctgc gcaaagagcg ccgcggtgac 240

tatctgggcg cgaccattca ggtgatccca catattacca atgcgatcaa agagcgcatc 300

atcgaa 306

<210> 870
<211> 306
<212> DNA
<213> Erwinia billingiae

<400> 870
attcttgaag cacgcggtct taacgtgacc atcatgaaac tggacccgta catcaacgtg 60

gacccaggca ccatgagccc aactcaacac ggcgaagtgt acgtcaccga cgacggcgca 120

gagaccgatc tggatttggg tcactacgag cgtttcatcc gcaccaagat gagccgtcgt 180

aacaacttca ccactggccg tatctactca gaagtcctgc gcaaagagcg ccggggcgat 240

tatctgggcg cgaccattca ggttattccg catatcacca atgccatcaa agagcgcatc 300

atcgaa 306

<210> 871
<211> 306
<212> DNA
<213> Erwinia mallotivora

<400> 871
attcttgaag cgcgtggtct taatgtgacc atcatgaagc tggacccgta tatcaacgtg 60

gaccccggca ccatgagccc tactcagcac ggtgaagttt ttgttaccga tgacggtgct 120

gaaaccgatc tggatctggg ccactacgag cgctttatcc gcactaaaat gagccgccgt 180

aacaacttca cgacaggccg tatctattca gacgtcctgc gcaaagagcg ccgcggcgac 240

tatcttggcg caaccattca ggtcattcct catatcacta atgccattaa agagcgcatc 300

atcgaa 306

<210> 872
<211> 306
<212> DNA
<213> Erwinia persicina

<400> 872
atccttgaag cacgtggtct taacgtgact atcatgaagc tggacccgta cattaacgtg 60

gatccaggta ccatgagtcc cacccagcac ggtgaagtct tcgtcaccga tgacggggcg 120

gagaccgatc tggatcttgg ccactatgag cgtttcatcc ggactaagat gagccgccgt 180

aacaacttca ccaccggtcg aatctactca gaagtgctgc gcaaagaacg tcgcggtgac 240

tacctgggcg cgactattca ggtgatccca cacattacca atgcaatcaa agaacgcatc 300

atcgaa 306

<210> 873
<211> 306
<212> DNA
<213> Erwinia psidii

<400>  873
attcttgaag cgcgtggtct taatgtgacc atcatgaagc tggaccccta tattaacgtg      60

gatcccggta ccatgagtcc tactcaacac ggcgaagtgt tcgtcaccga tgacggcgcc     120

gaaaccgatt tggatctggg ccattatgag cgctttatcc gtaccaaaat gagccgccgt     180

aacaacttca ccactggccg tatttactca gaagtcctgc gcaaagagcg tcgtggcgac     240

tatctgggcg cgaccattca ggtgatcccc catattacca atgccatcaa agagcgcatt     300

attgaa                                                                306

<210>  874
<211>  306
<212>  DNA
<213>  Erwinia pyrifoliae

<400>  874
atccttgaag cacgcggtct taacgtgacc atcatgaagc tggacccgta catcaacgtg      60

gatccgggta ccatgagccc gacccagcac ggtgaagtct tcgtcactga tgacggcgcg     120

gaaaccgatc tggatctcgg tcactacgaa cgctttatcc gtacccggat gagccgccgt     180

aacaacttca ccaccggccg tatttactcc gaagtgctgc gcaaagagcg tcgcggtgac     240

tatctgggcg caaccattca ggtgatcccg catattacca atgccatcaa agagcgcatc     300

atcgaa                                                                306

<210>  875
<211>  306
<212>  DNA
<213>  Erwinia rhapontici

<400>  875
atccttgaag cgcgtggtct taacgtgacc atcatgaagc tggacccgta catcaacgtg      60

gacccaggca ccatgagtcc aacccagcac ggtgaagttt atgtcaccga tgatggcgcg     120

gaaaccgatc tggatctcgg tcactacgag cgtttcatcc gtactaagat gagccgccgt     180

aacaacttca ccacaggccg tatctattca gaagtcctgc gcaaagagcg tcgtggcgac     240

tatctgggtg cgaccattca ggtgatcccg cacattacca atgccatcaa agaacgcatc     300

attgaa                                                                306

<210>  876
<211>  306
<212>  DNA
<213>  Erwinia toletana

<400>  876
atcctcgaag ctcgtggtct taacgtgacc atcatgaagc tggacccgta tatcaacgtg      60

gatccaggta ctatgagccc tacacagcac ggtgaagtct ttgtgaccga tgacggtgct     120

gaaaccgatc tggatttagg tcactacgag cgttttatcc gtacccggat gacacgccgc     180

aacaacttca ccactggccg tatctactcc gaagtgctgc gtaaagaacg ccgcggtgac     240

tatctgggcg cgaccattca ggttatccca catatcacta acgcgattaa agagcgcatt     300

atcgcc                                                              306

<210>  877
<211>  306
<212>  DNA
<213>  Erwinia tracheiphila

<400>  877
attctcgaag cgcgcggtct caatgtgacc atcatgaagt tggacccata tattaatgtg      60

gatccgggta cgatgagtcc aactcagcac ggcgaagtgt tcgttaccga tgacggtgct     120

gagactgatt tggatctcgg tcactacgag cgctttattc gcaccaaaat gagccgccgt     180

aataatttta ctaccgggcg tatttactcc gaagtgctgc gtaaagagcg ccgtggcgac     240

tatctgggcg cgaccgtcca ggttataccg cacatcacca atgcaatcaa agaacgcatc     300

attgaa                                                              306

<210>  878
<211>  306
<212>  DNA
<213>  Escherichia albertii

<400>  878
attcttgaag cccgtggcct caatgtgacc atcatgaagc tggatccgta catcaacgtc      60

gatccaggta ctatgagccc aatccagcac ggggaagtgt tcgttactga agacggcgct     120

gaaaccgatc tggacctggg gcactacgag cgtttcattc gcaccaaaat gagccgccgc     180

aacaacttca ccacgggtcg tatttactct gacgttctgc gtaaagaacg ccgcggcgac     240

tacctcggcg caaccgtaca ggttattccg cacatcacca atgcaatcaa agaacgcgtg     300

ctggaa                                                              306

<210>  879
<211>  306
<212>  DNA
<213>  Escherichia blattae

<400>  879
attctcgaag cccgtggcct caacgtaact atgatgaaac tggatccgta tattaacgtc      60

gatccaggca ccatgagccc tattcaacat ggggaagtgt tcgttactga agacggcgct     120

gaaaccgacc tggacctggg tcactacgag cgttttatcc gcacccggat gacgcgtcgt     180

aacaacttta ccactggccg tatttactct gatgttctgc gtaaagagcg tcgtggtgac     240

tacctgggcg ccacggtaca ggttattccg catatcacta acgccatcaa agagcgcgtt     300

attgcc                                                              306

<210>  880
<211>  306
<212>  DNA
<213>  Escherichia coli T

<400>  880
attcttgaag cccgtggcct caatgtgacc atcatgaaac tggatccgta catcaacgtc      60

gatccaggta ctatgagccc aatccaacac ggggaagtgt tcgttactga agacggcgct 120

gaaaccgacc tggacctggg gcactacgag cgtttcattc gtaccaaaat gagccgccgc 180

aacaacttca ccacgggtcg tatctactct gacgttctgc gtaaagaacg ccgcggtgac 240

tatctcggcg caaccgtaca ggttattccg cacatcacta acgctatcaa agagcgcgtg 300

ctggaa 306

<210> 881
<211> 306
<212> DNA
<213> Escherichia fergusonii

<400> 881
attcttgaag cccgtggcct caatgtgacc atcatgaaac tggatccgta catcaacgtc 60

gatccaggta ctatgagccc aatccaacac ggggaagtgt tcgttactga agacggcgct 120

gaaaccgacc tggacctggg gcattacgag cgtttcattc gtaccaaaat gagccgccgc 180

aacaacttca ccacgggtcg tatctactct gacgttctgc gtaaagaacg ccgcggtgat 240

tacctcggcg caaccgtaca ggttattccg cacatcacta acgctatcaa agagcgcgtg 300

ctggaa 306

<210> 882
<211> 306
<212> DNA
<213> Escherichia hermannii

<400> 882
attcttgaag cccgtggcct caacgtaact atcatgaaac tggatccgta tattaacgtc 60

gatccgggca ccatgagccc tatccagcac ggggaagtgt tcgttactga agacggcgct 120

gaaaccgatt tagacctcgg ccactacgag cgttttatcc gcacgcgtat gtcgcgtcgc 180

aacaacttca ccacaggtcg catctattcc gaggttctgc gtaaagaacg tcgtggcgac 240

tatcttggcg caaccgttca ggttatcccg catatcacta atgctatcaa agaacgcatt 300

cttgaa 306

<210> 883
<211> 306
<212> DNA
<213> Escherichia vulneris

<400> 883
attcttgaag cccgtggcct taacgtgacc atgatgaaac tggatccgta catcaacgtc 60

gatccgggta ccatgagtcc aatccagcac ggggaagtgt tcgttacgga agacggcgct 120

gaaaccgacc tggatctggg ccactacgag cgtttcattc gtactaagat gacgcgccgc 180

aacaacttca ctactggccg tatctattcc gacgtactgc gcaaagagcg ccgtggcgac 240

tatctgggcg caaccgttca ggttattccg cacattacca acgccattaa agagcgcatc 300

attgcc 306

<210> 884
<211> 306
<212> DNA
<213> Ewingella americana T

<400> 884
attctcgaag cccgtggcct caacgtgacc atcatgaaac tggacccgta tatcaacgtg 60

gatccaggca ccatgagccc aactcagcac ggcgaagtgt tcgtcaccga agacggcgct 120

gaaacggatc tcgatttggg tcactacgag cgcttcatcc gcaccaaaat gactcgtcgc 180

aacaacttca ccacggggcg catctactct gacgtactgc gcaaagaacg ccgtggcgac 240

tatttgggcg cgaccatcca agtaatccca cacatcacca ataacatcaa agaacgtatc 300

atcgaa 306

<210> 885
<211> 306
<212> DNA
<213> Hafnia alvei T

<400> 885
attcttgaag cccgtggcct caacgtaagc atgatgaaac tggatccgta catcaacgtc 60

gatccgggca cgatgagccc tatccagcac ggggaagttt tcgtcaccga agacggcgca 120

gaaaccgatc tggatttagg tcattacgag cgtttcatcc gtaccaaaat gtcccgccgc 180

aataacttta ctactggccg catttattct gatgtgctgc gtaaagagcg tcgtggcgat 240

tatctgggcg cgaccgttca ggttattcct catatcacta atgcaatcaa agaacgcatc 300

cttgaa 306

<210> 886
<211> 306
<212> DNA
<213> Klebsiella oxytoca

<400> 886
attcttgaag cccgtgggct taacgtgacc atgatgaaac tggatccgta catcaacgtc 60

gatccgggta ccatgagccc aatccaacac ggggaagtgt tcgttaccga agacggcgct 120

gaaaccgatc tggacttagg tcactacgag cgcttcattc gcaccaagat gactcgtcgc 180

aacaacttca ccaccggccg tatctactct gatgtcctgc gcaaagagcg tcgtggcgac 240

tatctgggcg cgaccgtgca ggtgatcccg cacatcacca acgcgattaa agagcgcgtg 300

ctggcg 306

<210> 887
<211> 306
<212> DNA
<213> Klebsiella pneumoniae sbsp. ozaenae

<400> 887
attcttgaag cccgtggtct caacgtgacc atcatgaaac tggatccgta tatcaacgtc 60

gatccgggca ccatgagccc aatccagcat ggggaagtgt tcgttactga agacggcgca 120

gaaaccgatc tggacctggg ccactatgag cgttttatcc gcaccaagat gacgcgccgc 180

aataacttca ccaccggccg tatctactcc gacgtgctgc gcaaagagcg ccgtggcgac 240

tacctgggcg cgaccgttca ggttatcccg cacatcacca acgctatcaa agagcgcgta 300

ctggcg 306

<210> 888
<211> 306
<212> DNA
<213> Klebsiella pneumoniae sbsp. pneumoniae T

<400> 888
attcttgaag cccgtggtct caacgtgacc atcatgaaac tggatccgta tatcaacgtc 60

gatccgggca ccatgagccc aatccagcat ggggaagtgt tcgttactga agacggcgca 120

gaaaccgatc tggacctggg ccactatgag cgttttatcc gcaccaagat gacgcgccgc 180

aataacttca ccaccggccg tatctactcc gacgtgctgc gcaaagagcg ccgtggcgac 240

tacctgggcg cgaccgttca ggttatcccg cacatcacca acgctatcaa agagcgcgta 300

ctggcg 306

<210> 889
<211> 306
<212> DNA
<213> Klebsiella pneumoniae sbsp. rhinoscleromatis

<400> 889
attcttgaag cccgtggtct caacgtgacc atcatgaaac tggatccgta tatcaacgtc 60

gatccgggca ccatgagccc aatccagcat ggggaagtgt tcgttactga agacggcgca 120

gaaaccgatc tggacctggg ccactatgag cgttttatcc gtaccaagat gacgcgccgc 180

aataacttca ccaccggccg tatctactcc gacgtgctgc gcaaagagcg ccgtggcgac 240

tacctgggcg cgaccgttca ggttatcccg cacatcacca acgctatcaa agagcgcgta 300

ctggcg 306

<210> 890
<211> 306
<212> DNA
<213> Klebsiella singaporensis

<400> 890
attcttgaag cccgtggtct caacgtgacc atcatgaaac tggatccgta tatcaacgtc 60

gatccgggca ccatgagccc aatccagcat ggggaagtgt tcgttactga agacggcgca 120

gaaaccgatc tggacctggg ccactatgag cgtttcatcc gtaccaagat gacgcgtcgc 180

aacaacttca ccaccggccg tatctactcc gacgtactgc gtaaagagcg ccgtggcgac 240

tatctgggtg ccaccgttca ggttattccg cacatcacca acgctatcaa agagcgcgta 300

ctggcg 306

<210> 891
<211> 306

<212>  DNA
<213>  Klebsiella variicola

<400>  891
attcttgaag cccgtggtct caacgtgacc atcatgaaac tggatccgta tatcaacgtc      60

gatccgggca ccatgagccc aatccagcat ggggaagtgt tcgttactga agacggcgca     120

gaaaccgatc tggacctggg ccactatgag cgtttcatcc gtaccaagat gacgcgtcgc     180

aacaacttca ccaccggccg tatctactcc gacgtactgc gcaaagagcg ccgtggcgac     240

tatctgggtg ccaccgttca ggttattcca cacatcacca acgctatcaa agagcgcgta     300

ctggcg                                                                306

<210>  892
<211>  306
<212>  DNA
<213>  Kluyvera ascorbata T

<400>  892
attcttgaag cccgtggcct caacgtgacc atgatgaaac tggatccgta tattaacgtc      60

gatcctggca ccatgagccc gatccaacac ggggaagtgt tcgttaccga agacggcgct     120

gaaaccgacc tggacctggg tcactatgag cgcttcattc gtaccaaaat gactcgccgc     180

aacaacttca ctaccggccg tatctactca gatgtcctgc gcaaagagcg tcgcggtgac     240

tatctgggcg caaccgtgca ggttatccca cacatcacta acgctatcaa agagcgtgtc     300

ctcgcc                                                                306

<210>  893
<211>  306
<212>  DNA
<213>  Kluyvera cochleae

<400>  893
attcttgaag cccgtggcct caacgtgacc atgatgaaac tggatccgta catcaacgtc      60

gatccaggta ccatgagccc gatccaacac ggggaagtgt tcgttacaga agacggcgct     120

gaaaccgacc tggacctggg tcactatgag cgcttcatcc gtaccaaaat gactcgccgt     180

aataacttca ctaccggtcg tatttattca gacgtcctgc gcaaagagcg ccgtggcgac     240

tatctgggcg cgaccgttca ggttatcccg cacatcacta acgcgattaa agagcgcgta     300

ctggcc                                                                306

<210>  894
<211>  306
<212>  DNA
<213>  Kluyvera cryocrescens

<400>  894
attcttgaag cccgtggcct caacgtgacc atgatgaaac tggatccgta tattaacgtc      60

gatcctggca ccatgagccc gatccaacac ggggaagtgt tcgttactga agacggcgct     120

gaaaccgacc tggacctggg tcactatgag cgcttcatcc gcaccaaaat gactcgccgc     180

aataacttca ctaccggtcg tatttactca gacgtcctgc gcaaagagcg ccgtggcgac     240

tatctgggcg caaccgtaca ggttattcca cacatcacca acgcgattaa agagcgcgtt 300

ctggca 306

<210> 895
<211> 306
<212> DNA
<213> Kluyvera georgiana

<400> 895
attcttgaag cccgtggcct taacgtcacc atgatgaaac tggatccgta tatcaacgtc 60

gatcctggta ccatgagccc gatccaacac ggggaagtgt tcgttaccga agacggcgct 120

gaaaccgacc tggacctggg ccactacgag cgtttcatcc gcaccaaaat gactcgccgt 180

aacaacttca cgactggccg tatttactca gacgtcctgc gcaaagagcg ccgtggcgac 240

tatctgggcg cgaccgtaca ggttatcccg catatcacta acgcgatcaa agagcgcgtg 300

cttgcc 306

<210> 896
<211> 306
<212> DNA
<213> Kluyvera intermedia

<400> 896
attcttgaag cccgtggcct caacgtgacc atgatgaaac tggatccgta catcaacgtc 60

gatccaggta ccatgagccc gatccaacac ggggaagtgt tcgttacaga agacggcgct 120

gaaaccgacc tggacctggg tcactatgag cgcttcatcc gtaccaaaat gactcgccgt 180

aataacttca ctaccggtcg tatttactca gacgtcctgc gcaaagagcg ccgtggtgac 240

tatctgggcg cgaccgttca ggttatcccg cacatcacta acgcgattaa agagcgcgta 300

ctggcc 306

<210> 897
<211> 306
<212> DNA
<213> Leclercia adecarboxylata T

<400> 897
attcttgaag cccgtggcct caatgtgacc atgatgaagc tggatccgta catcaacgtc 60

gatccaggta ccatgagccc gatccagcac ggggaagtgt tcgttactga agacggcgcc 120

gaaaccgatc tggacctggg tcactacgag cgcttcattc gcaccaaaat gacccgtcgt 180

aacaacttca cgactggccg tatctactcc gacgttctgc gtaaagagcg ccgtggcgac 240

tatctgggcg ccaccgtaca ggttatcccg cacatcacca acgccattaa agagcgcatc 300

attgcc 306

<210> 898
<211> 306
<212> DNA
<213> Leminorella grimontii T

<400>  898
attcttgagg ctcgtggcct aaacgtcaca atgatgaagt tggatcccta cattaacctc      60

gatccgggta ccatgagccc catccagcac ggcgaagtgt ttgttaccga ggacggtgcg     120

gaaaccgatc tggatttggg ccactatgag cgcttcattc gcaccaagat gacccgccgc     180

aataacttca ccactggccg catttattcg gacgtgctgc gcaaagagcg ccgcggcgac     240

tatctgggcg ctacagtgca ggttattccc catattacta acgccattaa agaacgcgtc     300

atcgcc                                                                306

<210>  899
<211>  306
<212>  DNA
<213>  Leminorella richardii

<400>  899
attcttgagg ctcgtggcct aaacgtcaca atgatgaagc tggatcccta cattaacctc      60

gatccaggca ccatgagtcc tattcagcac ggcgaagtgt ttgttaccga ggacggtgcc     120

gaaaccgatc tggacttggg gcactacgag cgctttattc gcaccaagat gacccgccgc     180

aacaacttca ctactggccg catttattcc gacgtgctgc gcaaagagcg ccgcggcgac     240

tatttgggcg ccactgtgca ggtgattccc catattacca acgccattaa agagcgggtt     300

attgcc                                                                306

<210>  900
<211>  306
<212>  DNA
<213>  Moellerella wisconsensis T

<400>  900
atactcgaag cccgtggact caatgtcact atcatgaaac tggatccgta tattaacgtc      60

gatccgggga ccatgagtcc aacccaacac ggggaagttt tcgttacaga agacggcgct     120

gaaactgatc ttgatttagg tcattatgag cgttttatcc gtactaaaat gacacgtcgt     180

aataacttca cgactgggcg tgtttattct gaagttctgc gtaaagaacg tcgtggcgac     240

tatctgggcg ccactattca agttattcct catatcacca atgaaattaa agaccgcatt     300

attcgc                                                                306

<210>  901
<211>  306
<212>  DNA
<213>  Morganella morganii sbsp. morganii T

<400>  901
atactcgaag cccgtggact caatgtcacc atgatgaaac tggatccgta catcaacgtc      60

gatccgggaa ccatgagccc gacccaacac ggggaagttt tcgtcaccaa cgacggcgct     120

gaaactgatc tcgacttagg tcactacgag cgcttcatcc gcaccaaaat gacccgccgc     180

aataacttca ccaccggacg tgtttactct gaagttctgc gcaaagagcg ccgcggcgac     240

tatctcggtg ccaccattca ggttatcccg cacatcacca acgaaatcaa agatcgtatc     300

atccgt                                                              306

<210>  902
<211>  306
<212>  DNA
<213>  Morganella morganii sbsp. sibonii

<400>  902
atactcgaag cccgtggact caatgtcacc atgatgaaac tggatccgta catcaacgtc      60

gatccgggaa ccatgagccc gacccaacac ggggaagttt tcgtcactaa cgacggcgct     120

gaaactgatc tcgacttagg tcactacgag cgcttcatcc gcaccaaaat gacccgccgt     180

aataacttta ctaccggacg tgtttactct gaagtcctgc gcaaagagcg ccgcggcgac     240

tatctcggtg ccaccattca ggttatcccg cacatcacca acgaaatcaa agaccgtatc     300

atccgt                                                              306

<210>  903
<211>  306
<212>  DNA
<213>  Obesumbacterium proteus T

<400>  903
attcttgaag cccgtggcct caacgtaagc atgatgaaac tggatccgta catcaacgtc      60

gatccgggca cgatgagccc tatccagcac ggagaagttt tcgtcaccga agacggcgca     120

gaaaccgatc tggatttagg tcattacgag cgtttcatcc gtaccaaaat gtcccgccgc     180

aataacttta ccactggccg catctattcc gatgtgctgc gtaaagagcg tcgtggcgat     240

tatctgggcg ctaccgttca ggttattcct catatcacta atgcaatcaa agagcgcatc     300

cttgaa                                                              306

<210>  904
<211>  306
<212>  DNA
<213>  Pantoea agglomerans T

<400>  904
attctcgaag cccgtggtct gaacgtgacc atcatgaagc tggacccgta tatcaacgtg      60

gatccaggca ccatgagccc gacgcagcac ggtgaagttt tcgtcactga tgacggcgct     120

gaaaccgatc tggatttggg tcactacgag cgcttcatcc gtaccaaaat gtcgcgtcgt     180

aacaacttca ccactggccg tatctactca gaagtcctgc gcaaagagcg ccgtggcgac     240

tatctgggcg caaccattca ggttattccg cacatcacca acgccatcaa agaacgcatt     300

attgaa                                                              306

<210>  905
<211>  306
<212>  DNA
<213>  Pantoea ananatis

<400>  905
attctcgaag cgcgtggtct gaatgtgacc atcatgaaac tggatccgta catcaacgtc      60

gatccgggta ccatgagccc tactcagcat ggtgaagtgt ttgtcaccga tgatggtgcc 120

gaaaccgatc tggatttggg tcactacgag cgcttcatcc gcactaaaat gtcgcgccgc 180

aacaacttca ccacgggccg tatctactca gaagtcctgc gcaaagagcg tcgcggtgac 240

taccttggcg ccactattca ggtcatccct catatcacga atgcgatcaa agagcgcatt 300

cttgaa 306

<210> 906
<211> 306
<212> DNA
<213> Pantoea citrea

<400> 906
attcttgaag ctcgtggtct gaaagtgacc attatgaagc ttgaccctta tattaacgtc 60

gatcctggca ccatgagccc aactcagcat ggtgaagtat ttgtcactga tgatggtgca 120

gagacggatc tggacttagg tcactacgaa cgttttatcc gtacccggat gtcccgtcgt 180

aataacttca ccaccgggcg tatctactct gaagtgctac gtaaagagcg ccgtggtgac 240

tacctgggtg cgacgattca ggtgatcccg cacattacta atgctatcaa agaacgcatt 300

attgaa 306

<210> 907
<211> 306
<212> DNA
<213> Pantoea dispersa

<400> 907
atccttgaag cacgtggtct gaatgtgacc atcatgaaac tcgatccgta tatcaacgtc 60

gatccgggta ccatgagtcc tacgcagcac ggtgaagtgt tcgtcaccga tgacggggcc 120

gagaccgatc tggatttagg tcactacgag cgcttcatcc gtaccaaaat gtcgcgccgc 180

aacaacttta ctaccggccg tatctactca gaagtgctgc gcaaagagcg tcgcggcgac 240

tatctgggcg cgaccattca ggttatccca cacatcacca acgccatcaa agagcgcatc 300

attgaa 306

<210> 908
<211> 306
<212> DNA
<213> Pantoea punctata

<400> 908
attcttgaag ctcgtggtct gaaagtgacc attatgaagc ttgaccctta tattaacgtc 60

gatccaggca ccatgagccc aacccaacat ggtgaagtat ttgtcactga tgatggtgca 120

gaaacggatc ttgacttagg acactacgag cgttttatcc gcacccgtat gtcccgtcgt 180

aataacttta ccaccggacg aatttactcc gaagtgttgc gtaaagaacg tcgtggcgac 240

tacctggggg cgactatcca ggttattcct catatcacta atgcgattaa agaacgcatt 300

attgaa 306

<210> 909
<211> 306
<212> DNA
<213> Pantoea stewartii sbsp. indologenes

<400> 909
attctcgaag cgcgtggtct gaacgtgacc atcatgaaac tggatccgta cattaacgtc 60

gatccgggta ccatgagccc gacacagcac ggtgaagtat tcgtcactga tgatggggcc 120

gaaaccgatc tggatttggg tcactatgag cgcttcatcc gcacgaaaat gtcgcgccgc 180

aacaacttca ccactggccg tatctactcg gaagtcctgc gcaaagagcg ccgtggcgac 240

tatctgggtg ccacgattca ggtcattccg catattacta atgcgattaa agagcgcatc 300

atcgaa 306

<210> 910
<211> 306
<212> DNA
<213> Pantoea stewartii sbsp. stewartii

<400> 910
attctcgaag cgcgtggtct gaacgtgacc atcatgaaac tggatccgta cattaacgtc 60

gatccgggta ccatgagccc gacacagcac ggtgaagtat tcgtcactga tgatggggcc 120

gaaaccgatc tggatttggg tcactatgag cgcttcatcc gcacgaaaat gtcgcgccgc 180

aacaacttca ccactggccg tatctactcg gaagtcctgc gcaaagagcg ccgtggcgac 240

tatctgggtg ccacgattca ggtcattccg catattacta atgcgattaa agagcgcatc 300

atcgaa 306

<210> 911
<211> 306
<212> DNA
<213> Pantoea terrea

<400> 911
attcttgaag ctcgtggtct gaaagtgacc attatgaagc tcgaccctta tattaacgtc 60

gatccaggca ccatgagccc gactcagcat ggtgaagtct ttgtcactga tgatggtgca 120

gaaacggatc ttgacttagg gcattacgag cgttttatcc gtacccgtat gtctcgccgt 180

aataacttta ctaccggacg tatttactct gaagttctgc gtaaagaacg tcgcggtgac 240

tacctgggcg cgactattca ggtgatcccg catattacta atgctatcaa agagcgcatt 300

attgaa 306

<210> 912
<211> 306
<212> DNA
<213> Pectobacterium atrosepticum

<400> 912
attcttgaag cccgtggcct caacgttacc atcatgaaac tggacccgta tatcaacgtg 60

gatccgggca cgatgagccc gacgcagcac ggtgaagtgt ttgtcaccga agacggtgcc 120

gaaaccgatc tggacttggg tcactacgag cgtttcatcc gcactaaaat gtcgcgccgc 180

aacaacttca ccactggccg tatctactct gatgtcctgc gcaaagagcg ccgtggcgac 240

tatctgggcg cgaccattca ggtgatccca catatcacca acgcgattaa agagcgcatt 300

attgaa 306

<210> 913
<211> 306
<212> DNA
<213> Pectobacterium betavasculorum

<400> 913
attcttgaag cccgtggcct caacgttacc atcatgaaac tggacccgta tatcaacgtg 60

gatccgggca cgatgagccc gacgcagcac ggtgaagtgt ttgtcaccga agacggtgcc 120

gaaaccgatc tggacttggg tcactacgag cgtttcatcc gcaccaaaat gtcgcgccgc 180

aacaacttca ccactggccg tatctactct gatgtcctgc gcaaagagcg ccgtggcgac 240

tatctgggcg cgaccattca ggttatccca cacatcacca acgcgattaa agagcgcatc 300

attgaa 306

<210> 914
<211> 306
<212> DNA
<213> Pectobacterium cacticida

<400> 914
attctcgaag cccgtggcct caacgttacc atcatgaaac tggacccgta tatcaatgtg 60

gatccgggca cgatgagtcc gacgcagcat ggcgaagtgt ttgtcaccga agacggcgct 120

gaaaccgatc tggacttggg gcactatgaa cggttcattc gcaccaaaat gtcgcgccgt 180

aataatttca ccacagggcg aatttactcc gatgtgctgc gcaaagagcg ccgtggcgac 240

tatttaggcg cgaccattca ggtgattcca catatcacca acgcgatcaa agagcgcatc 300

attgaa 306

<210> 915
<211> 306
<212> DNA
<213> Pectobacterium carotovorum sbsp. carotovorum T

<400> 915
attcttgaag cccgtggcct caacgttacc atcatgaaac tggacccgta catcaacgtg 60

gatccgggca cgatgagccc gacgcagcac ggtgaagtgt ttgtcaccga agacggcgcc 120

gaaaccgatc tggacttggg tcactacgag cggttcatcc gcaccaaaat gtcgcgccgc 180

aacaacttta ccactggccg tatctactct gatgtcctgc gcaaagagcg ccgtggcgac 240

tatctgggcg cgaccattca ggtgatcccg catatcacca acgcgattaa agagcgcatc 300

attgaa 306

<210> 916
<211> 306
<212> DNA

<213>  Pectobacterium cypripedii

<400>  916
atccttgaag cacgtggtct gaatgtgacc atcatgaaac tcgatccgta catcaacgtc      60

gatccgggta ccatgagccc tactcagcac ggtgaagttt tcgtcaccga cgacggggcc     120

gaaaccgatc tggatttagg tcactacgag cgcttcatcc gtaccaaaat gacgcgccgc     180

aacaacttta ctaccggccg tatctactca gaagtgctgc gcaaagagcg tcgtggcgac     240

tatctgggtg cgaccattca ggttattccg cacatcacca acgccatcaa agagcgcatc     300

attgaa                                                                306

<210>  917
<211>  306
<212>  DNA
<213>  Pectobacterium wasabiae

<400>  917
attcttgaag cccgtggcct caacgttacc atcatgaaac tggacccgta catcaacgtg      60

gatccgggca cgatgagccc gacgcagcac ggtgaagtgt ttgtcaccga agacggcgcc     120

gaaaccgatc tggacttggg tcactacgag cgcttcatcc gcaccaaaat gtcgcgccgt     180

aacaacttca ccactggccg tatctactct gatgtcctgc gcaaagagcg tcgtggtgac     240

tatctgggcg cgaccattca ggtaattccg catatcacca acgcgattaa agagcgcatc     300

attgaa                                                                306

<210>  918
<211>  306
<212>  DNA
<213>  Photorhabdus asymbiotica sbsp. asymbiotica

<400>  918
atactcgaag cccgtggact caatgtcact attatgaaac tggacccgta catcaacgtt      60

gatccgggaa cgatgagtcc aatccagcat ggggaagttt tcgttactga agacggcgct     120

gaaactgatc tcgatttagg tcactacgag cgtttcattc gcactaaaat gacgcgtcgt     180

aataacttca ctacaggtcg gatctattcc gaagttctgc gtaaagaacg ccggggcgat     240

tatttgggcg caaccgttca agttattcct cacatcacca atgaaattaa agatcgcatc     300

atcagc                                                                306

<210>  919
<211>  306
<212>  DNA
<213>  Photorhabdus asymbiotica sbsp. australis

<400>  919
atactcgaag cccgtggact caatgtcact attatgaaac tggacccata catcaacgtt      60

gatccgggga caatgagtcc aatccagcat ggggaagttt tcgttactga agacggcgct     120

gaaactgatc tcgatttagg tcactacgag cgtttcattc gcactaaaat gacgcgtcgc     180

aataacttca ctacgggccg gatctattcc gaagttctgc gtaaagagcg ccggggcgat     240

tatttgggtg cgaccgttca agttattcct cacatcacca atgaaattaa agatcgcatc 300

atcagc 306

<210> 920
<211> 306
<212> DNA
<213> Photorhabdus luminescens sbsp. akhurstii

<400> 920
atactcgaag cccgtggact caatgtcacg attatgaaac tggacccgta catcaatgtc 60

gatccaggta cgatgagccc aatccagcat ggggaagtct tcgttaccga agacggcgct 120

gaaactgatc tcgatttagg tcactatgag cgtttcatcc gcactaaaat gactcgccgc 180

aataacttta cgacggggcg ggtttattcc gaagtgctgc gcaaagagcg ccgtggcgac 240

tatttagggg cgaccgttca agttattcct cacatcacca atgaaatcaa agatcgcatc 300

atcagt 306

<210> 921
<211> 306
<212> DNA
<213> Photorhabdus luminescens sbsp. kayaii

<400> 921
atactcgaag cccgtggact caatgtcacg attatgaaac tggacccgta catcaatgtc 60

gatccaggta cgatgagccc aatccaacac ggggaggttt tcgttaccga agacggtgct 120

gaaactgatc tcgatttagg tcactatgag cgtttcatcc gcactaaaat gactcgtcgc 180

aataacttca ccacgggtcg ggtttattcc gaagttttgc gtaaagagcg ccgtggcgac 240

tatcttggcg cgacggttca agtcattcct cacatcacca atgaaattaa ggatcgcatc 300

atcagt 306

<210> 922
<211> 306
<212> DNA
<213> Photorhabdus luminescens sbsp. laumondii

<400> 922
atactcgaag cccgtggact caatgtcacg attatgaaac tggacccgta catcaatgtc 60

gatccaggta cgatgagccc aatccaacat ggggaggtct tcgttaccga agacggcgct 120

gaaactgatc tcgatttagg tcactatgag cgtttcatcc gcactaaaat gactcgtcgc 180

aataacttca ctacggggcg agtttattcc gaagttttgc gtaaagagcg ccgtggtgac 240

tatctggggg cgacggttca agttattcct cacatcacca atgaaattaa agatcgcatc 300

atcagt 306

<210> 923
<211> 306
<212> DNA
<213> Photorhabdus luminescens sbsp. luminescens T

<400> 923

atactcgaag cccgtggact caatgtcact attatgaaac tggacccgta catcaatgtc 60

gatccaggta cgatgagtcc aattcaacac ggggaggttt tcgttaccga agacggtgct 120

gaaactgatc tcgatttagg tcactatgag cgtttcatcc gtactaaaat gactcgccgt 180

aataacttta cgacggggcg ggtttattcc gaagttctgc gcaaagagcg ccgtggcgac 240

tatttggggg cgaccgttca agttattcct catatcacca atgaaattaa agatcgcatc 300

atcagc 306

<210> 924
<211> 306
<212> DNA
<213> Photorhabdus luminescens sbsp. thracensis

<400> 924
atactcgaag cccgtggact caatgtcact attatgaaac tggatccgta catcaacgtc 60

gatccgggga cgatgagccc aatccagcat ggggaagttt tcgttactga agacggcgct 120

gaaactgatc tcgatttagg tcactatgag cgtttcatcc gcactaaaat gactcgccgt 180

aataatttca ccactggtcg ggtttattcc gaagttttgc gtaaagagcg tcgtggcgac 240

tatctgggag cgaccgttca ggttattcct cacatcacca atgaaattaa agaccgcatc 300

atcagc 306

<210> 925
<211> 306
<212> DNA
<213> Photorhabdus temperata sbsp. temperata

<400> 925
atactcgaag cccgtggact caatgtcact attatgaaac tggatccgta catcaacgtc 60

gatccgggga cgatgagccc aatccagcat ggggaagttt tcgttactga agacggcgct 120

gaaactgatc tcgatttagg tcactatgag cgtttcatcc gcactaaaat gactcgccgt 180

aataatttca ccactggccg ggtttattcc gaagttttgc gtaaagagcg tcgtggcgac 240

tatctgggag cgaccgttca ggttattcct cacatcacca atgaaattaa agaccgcatc 300

atcagc 306

<210> 926
<211> 306
<212> DNA
<213> Plesiomonas shigelloides T

<400> 926
attctcgaag cgcgtggcct caatgtcacc atcatgaaac tggaccctta catcaacgtg 60

gatccgggca ccatgagccc aatccagcac ggtgaagtat tcgtaacgga agacggtgcg 120

gaaacagacc ttgatttggg ccactatgag cgttttattc gcactaagat gacccgccgc 180

aacaacttca ctaccggtcg cgtatacgcc gacgtgctgc gcaaagagcg ccgtggtgat 240

tacttagggg cgaccattca ggttattccg catattacta atgcgatcaa agagcgtgtt 300

atcgcc 306

<210> 927
<211> 306
<212> DNA
<213> Pragia fontium T

<400> 927
attcttgaag ctcgtggctt aaacgtcact atgatgaagc tggatccgta tattaacctc 60

gatccaggta ccatgagccc aattcaacac ggtgaagtgt tcgtcaccga agacggtgcc 120

gaaaccgatc tggatttagg tcattacgaa cgttttattc gtaccaaaat gactcgccgt 180

aataacttca ccaccggtcg catttactct gacgtattac gtaaagagcg tcgtggtgat 240

tatcttggcg caacggttca ggttatcccg catattacca atgccattaa agagcgcgtt 300

atcgct 306

<210> 928
<211> 306
<212> DNA
<213> Proteus mirabilis

<400> 928
atactagaag cccgtggact caatgtcacc atgatgaagt tagatccgta tattaacgtc 60

gatccaggta cgatgagccc aattcaacac ggagaagtct tcgtcactga cgatggagct 120

gaaactgatc ttgacttagg ccattacgag cgctttattc gcacgaaaat gacgcgtcgt 180

aataacttta cgaccggccg tgtatactca gaagtgctac gcaaagagcg tcgtggtgac 240

tatcttggtg ctactatcca agttattcct catatcacta atgaaatcaa agaacgcatc 300

atccgt 306

<210> 929
<211> 306
<212> DNA
<213> Proteus myxofaciens

<400> 929
atactcgaag cccgtggact caatgtcacc attatgaagt tggatccgta tattaacgtc 60

gatcctggta ctatgagccc aattcagcac ggggaagtct tcgtcaccga cgatggagct 120

gaaactgatc tcgacttagg ccattatgag cgctttattc gcacaagaat gactcgccgt 180

aataatttca ctacaggccg tgtatattca gaagtcctac gcaaagaacg ccgtggtgac 240

tatctgggcg cgactattca agttattcct catatcacta atgaaattaa agaacgtatt 300

attcgt 306

<210> 930
<211> 306
<212> DNA
<213> Proteus penneri

<400> 930
atactcgaag cccgtggact caatgtcacc atgatgaagt tggatccgta tatcaacgtc 60

gatccaggta ctatgagccc aattcagcac ggggaagtct tcgttactga cgatggtgct 120

gaaactgatc tcgacttagg acactatgag cgctttattc gcacgaaaat gactcgtcgt 180

aataacttca cgacaggtcg cgtatactcg gaagtactac gcaaagagcg tcgtggtgac 240

tatcttgggg ctacaattca agttattcct catatcacta atgaaatcaa agaacgcatc 300

atccgt 306

<210> 931
<211> 306
<212> DNA
<213> Providencia alcalifaciens T

<400> 931
atactcgaag cccgtggact caatgtaacc attatgaaat tggatcccta cattaacgta 60

gatccaggta caatgagccc aacccaacac ggggaagttt tcgttacaga agacggcgct 120

gaaactgact tggatttagg tcattatgag cgttttattc gtaccaaaat gacacgtcgc 180

aacaacttta cgactggacg tgtttattct gaagtcctgc gcaaagagcg tcgtggcgac 240

tacttaggcg cgaccatcca agttattcct catatcacta atgaaatcaa agatcgcatc 300

atccgt 306

<210> 932
<211> 306
<212> DNA
<213> Providencia heimbachae

<400> 932
atactcgaag cccgtggact taatgtaaca atgatgaaac tggatcccta catcaacgta 60

gatccaggaa caatgagtcc aacccaacac ggggaagtat tcgtaacaga agacggcgct 120

gaaactgact tagatttagg tcattatgag cgttttattc gtaccaaaat gacgcgtcgt 180

aataacttta cgactgggcg cgtctactca gaagtcttgc gcaaagagcg tcgtggtgat 240

tacctcggtg cgacgattca agttatccct catatcacta atgaaatcaa agaccgcatc 300

attcgt 306

<210> 933
<211> 306
<212> DNA
<213> Providencia rettgeri

<400> 933
atactcgaag cccgtggact caatgtgact attatgaaac tggatcccta catcaacgta 60

gatccaggca caatgagccc aacccaacac ggggaagttt tcgttacaga agacggcgct 120

gaaactgact tagatttggg tcattatgag cgcttcatcc gtactaaaat gactcgtcgt 180

aataacttta cgactgggcg tgtttactct gaagttctgc gcaaagagcg ccgtggcgac 240

tacttaggcg ctacaattca agttatccct catatcacta atgaaattaa agaccgcatc 300

atccgt 306

<210> 934

<211>  306
<212>  DNA
<213>  Providencia rustigianii

<400>  934
atactcgaag cccgtggact caatgtaacc attatgaaat tggatcccta cattaacgta      60

gatccaggta ctatgagccc aacccaacac ggggaagttt tcgttacaga agacggcgct     120

gaaactgact tggatttagg tcattatgag cgtttcatcc gtactaaaat gacacgtcgc     180

aataacttta cgacggggcg tgtttattct gaagtcttac gcaaagagcg ccgtggcgac     240

tacttaggcg cgactatcca agttatccct catattacta acgaaattaa agatcgcatc     300

atccgt                                                                306

<210>  935
<211>  306
<212>  DNA
<213>  Providencia stuartii

<400>  935
atactcgaag cccgtggact caatgtcacc attatgaaac tggatccgta catcaacgtt      60

gatccaggga caatgagccc aacccaacac ggggaagttt tcgttacaga agacggcgct     120

gaaactgact tggatttagg tcattatgag cgtttcatcc gtaccaaaat gacgcgtcgc     180

aacaacttta caacggggcg cgtttattca gaagtattgc gcaaagaacg tcgcggagac     240

tatcttggcg cgactatcca agttatccct catattacca atgaaattaa agaccgcatc     300

atccgt                                                                306

<210>  936
<211>  306
<212>  DNA
<213>  Providencia vermicola

<400>  936
atactcgaag cccgtggact caatgtaacg attatgaaac tggatcccta cattaacgta      60

gatccaggta caatgagccc aacccaacac ggggaagttt tcgttacaga agacggcgct     120

gaaactgact tggatttggg tcattatgag cgtttcatcc gtaccaaaat gacacgtcgt     180

aataacttta cgacaggccg tgtctactct gaagttctgc gcaaagagcg ccgtggcgac     240

tatttaggcg ctaccatcca agttatcccg catattacga atgaaatcaa agaccgcatc     300

atccgt                                                                306

<210>  937
<211>  306
<212>  DNA
<213>  Rahnella aquatilis T

<400>  937
attctcgaag cccgtggcct caacgtgact atcatgaaac tggacccgta tatcaacgtg      60

gatccgggta cgatgagccc aactcaacac ggcgaagtct ttgttaccga agatggcgct     120

gaaaccgatc tcgatctggg tcactacgag cgcttcatcc gcaccaagat gactcgtcgc     180

aacaacttca ccacgggccg tatttattcc gacgtgctgc gcaaagagcg ccgcggcgac 240

tatctcggcg caaccatcca ggttatcccg cacatcacta acaacatcaa agaacgcatc 300

attgaa 306

<210> 938
<211> 306
<212> DNA
<213> Raoultella ornithinolytica

<400> 938
attctcgaag cccgtgggct taacgtaacc atgatgaaac tggatccgta catcaacgtc 60

gatccgggca ccatgagccc aatccaacac ggggaagtgt tcgttactga agacggcgct 120

gaaaccgatc tggacctggg tcactacgag cgcttcatcc gtaccaaaat gagccgccgc 180

aacaacttca ccacgggtcg tatttactcc gacgttctgc gtaaagagcg ccgcggtgac 240

tatctgggcg caaccgtaca ggttatcccg cacatcacta acgcgattaa agagcgcgtg 300

cttgcc 306

<210> 939
<211> 306
<212> DNA
<213> Raoultella planticola T

<400> 939
attctcgaag cccgtgggct taacgtaacc atgatgaaac tggatccgta catcaacgtc 60

gatccgggca ccatgagccc aatccaacac ggggaagtgt tcgttactga agacggcgct 120

gaaaccgatc tggacctggg tcactacgag cgcttcattc gtaccaaaat gagccgccgc 180

aacaacttca ccaccgggcg tatttactcc gacgttctgc gtaaagagcg ccgcggtgac 240

tatctgggcg caaccgtaca ggttattccg catatcacta acgcgatcaa agaacgcgtt 300

ctggcg 306

<210> 940
<211> 306
<212> DNA
<213> Raoultella terrigena

<400> 940
attctcgagg cccgtgggct taacgtgacc atgatgaaac tggatccgta catcaacgtc 60

gatccgggta ccatgagtcc tatccaacac ggggaagtgt tcgttactga agacggcgct 120

gaaaccgacc tggacctggg ccattacgag cgtttcatcc gcaccaaaat gagccgccgc 180

aataacttca ctacgggtcg tatttactcc gacgtcctgc gcaaagagcg ccgtggtgat 240

tacctgggcg caaccgtaca ggttattccg cacattacga atgcaatcaa agagcgcgtg 300

cttgcc 306

<210> 941
<211> 306
<212> DNA
<213> Salmonella bongori

<400>  941
attcttgaag cccgtggtct caacgtgacc atcatgaaac tggatcccta tatcaacgtc      60

gatccaggta ctatgagccc aatccaacac ggggaagtgt tcgttactga agacggcgcc     120

gaaaccgacc tggacctggg gcactacgag cgtttcatcc gtaccaagat gtctcgccgc     180

aacaacttca ccactggccg tatctactcc gacgttctgc gtaaagaacg ccgtggcgac     240

tatctgggcg ctactgtaca ggtcatccct catatcacta acgcgattaa agagcgcgtg     300

ctggaa                                                                306

<210>  942
<211>  306
<212>  DNA
<213>  Salmonella enterica sbsp. arizonae

<400>  942
attctggaag cccgtggtct caacgtgacc atcatgaaac tggatccgta catcaacgtc      60

gatccaggta ctatgagccc aatccaacat ggggaagtgt tcgttactga agacggcgct     120

gaaaccgacc tggacctggg gcactacgag cgtttcatcc gtaccaagat gtctcgccgc     180

aacaacttca cgactggccg tatctattcc gacgttctgc gtaaagagcg ccgtggcgac     240

tatttgggcg caaccgtaca ggttatccct cacatcacta acgcgattaa agagcgcgtg     300

ctggaa                                                                306

<210>  943
<211>  306
<212>  DNA
<213>  Salmonella enterica sbsp. diarizonae

<400>  943
attctggaag cccgtggtct caacgtgacc atcatgaaac tggatccgta catcaacgtc      60

gatccgggta ctatgagccc aatccaacac ggggaagtgt tcgttactga agacggcgct     120

gaaaccgacc tggacctggg gcactacgag cgtttcatcc gcaccaagat gtctcgccgc     180

aataacttca cgactggccg tatctactcc gacgttctgc gtaaagaacg ccgtggcgac     240

tatctgggcg caaccgtaca ggtgatccct cacatcacta atgcgattaa agagcgtgtg     300

ctggaa                                                                306

<210>  944
<211>  306
<212>  DNA
<213>  Salmonella enterica sbsp. enterica T

<400>  944
attctggaag cccgtggtct caacgtgacc atcatgaaac tggatccgta catcaacgtc      60

gatccgggta ctatgagccc aatccaacac ggggaagtgt tcgttactga agacggcgct     120

gaaaccgacc tggacctggg gcactacgag cgtttcatcc gcaccaagat gtctcgccgc     180

aacaacttca cgactggccg catctactcc gacgttctgc gtaaagaacg ccgtggcgac     240

tatctgggcg caaccgtaca ggtcatccct cacatcacta acgcgattaa agagcgcgtg     300

ctggaa                                                              306


<210>  945
<211>  306
<212>  DNA
<213>  Salmonella enterica sbsp. houtenae

<400>  945
attctggaag cccgtggtct caacgtgacc atcatgaaac tggatccgta catcaacgtc      60

gatccgggta ctatgagccc aatccaacac ggggaagtgt tcgttactga agacggcgct     120

gaaaccgacc tggacctggg gcactatgag cgtttcatcc gcaccaagat gtctcgccgc     180

aacaacttca cgactggccg tatctactct gatgttctgc gtaaagaacg ccgtggcgac     240

tatcttggcg caaccgtaca ggtaatccct catatcacta acgcgattaa agagcgcgtg     300

ctggaa                                                              306


<210>  946
<211>  306
<212>  DNA
<213>  Salmonella enterica sbsp. indica

<400>  946
attctggaag cccgtggtct caacgtgacc atcatgaaac tggatccgta catcaacgtc      60

gatccgggta ctatgagccc aatccaacac ggggaagtgt tcgttactga agacggcgct     120

gaaaccgacc tggacctggg gcactacgag cgtttcatcc gcaccaagat gtctcgccgc     180

aataacttca cgactggccg tatctactcc gacgttctgc gcaaagaacg tcgtggcgac     240

tatctgggcg caaccgtaca ggtaatccct cacatcacta acgcgattaa agaacgcgtg     300

ctggaa                                                              306


<210>  947
<211>  306
<212>  DNA
<213>  Salmonella enterica sbsp. salamae

<400>  947
attctggaag cccgtggtct caacgtgacc atcatgaaac tggatccgta catcaacgtc      60

gatccgggta ctatgagccc aatccaacac ggggaagtgt tcgttactga agacggcgct     120

gaaaccgacc tggacctggg gcactacgag cgtttcatcc gcaccaagat gtctcgccgc     180

aacaacttca cgactggccg tatctactcc gacgttctgc gtaaagaacg ccgtggcgac     240

tatctgggcg caaccgtaca ggtaatccct cacatcacta acgcgattaa agaacgcgtg     300

ctggaa                                                              306


<210>  948
<211>  306
<212>  DNA
<213>  Samsonia erythrinae T

<400>  948
attcttgaag cccgtggcct caacgttacc atcatgaaac tggacccgta tatcaacgtg      60

gatccgggca cgatgagccc gacgcagcac ggtgaagtat tcgtcactga agacggcgcc 120

gaaaccgatc tggatttggg ccactatgag cgtttcatcc gcactaaaat gtcacgccgc 180

aacaacttta ccactggccg tatctattcc gatgtgctgc gcaaagagcg ccgcggtgac 240

tatcttggcg cgacgattca ggttatcccg catatcacca acgcgattaa agagcgcatt 300

atcgaa 306

<210> 949
<211> 306
<212> DNA
<213> Serratia entomophila

<400> 949
attcttgaag cccgtggcct caacgttacc atcatgaaac tggacccgta catcaacgtg 60

gatccgggca ccatgagccc gattcagcat ggggaagttt tcgtcaccga agacggcgca 120

gaaaccgatc tggatttggg tcactacgag cgcttcatcc gcaccaaaat gtcgcgccgc 180

aacaacttca ctaccggccg tatctactcc gacgttctgc gtaaagaacg ccgcggcgac 240

tacctgggcg caaccgtaca ggttatcccg cacatcacca atgctatcaa agagcgcatc 300

ctggaa 306

<210> 950
<211> 306
<212> DNA
<213> Serratia ficaria

<400> 950
attcttgaag cccgtggcct caacgttacc atcatgaaac tggacccgta catcaacgtg 60

gatccgggca ccatgagccc gattcagcat ggggaagttt tcgtcaccga agacggcgca 120

gaaaccgatc tggatttggg tcactacgag cgcttcattc gcaccaaaat gtcgcgtcgc 180

aacaacttca ccaccggtcg catctactcc gacgttctgc gtaaagaacg ccgcggcgac 240

tatctgggcg ctaccgtaca ggttatcccg cacatcacca atgctatcaa agaacgcatc 300

ctggaa 306

<210> 951
<211> 306
<212> DNA
<213> Serratia fonticola

<400> 951
attcttgaag cccgtggcct caacgttacc atcatgaaac tggacccgta catcaacgtg 60

gatccgggca ccatgagccc gattcagcat ggggaagttt tcgtcaccga agacggggca 120

gaaaccgatc tggatctggg tcactacgag cgcttcatcc gtaccaaaat gacgcgtcgt 180

aacaacttca ccaccgggcg tatctactcc gacgtgctgc gcaaagagcg ccgtggcgac 240

tacttgggtg ccaccgtaca ggtgatccca cacatcacca atgccattaa agaacgcatt 300

atcgaa 306

<210> 952
<211> 306
<212> DNA
<213> Serratia grimesii

<400> 952
attcttgaag cccgtggcct caacgttacc atcatgaaac tggacccgta catcaacgtg 60

gatccgggca ccatgagccc gattcagcat ggggaagttt tcgtcaccga agacggcgca 120

gaaaccgatc tggatttggg tcactacgag cgctacatcc gtaccaaaat gtcgcgccgc 180

aacaacttca ctaccggtcg tatttactcc gacgttctgc gtaaagaacg ccgtggcgac 240

tatctgggcg caaccgtaca ggttattccg cacatcacca acgcgatcaa agaacgcatc 300

ctcgaa 306

<210> 953
<211> 306
<212> DNA
<213> Serratia liquefaciens

<400> 953
attcttgaag cccgtggcct caacgttacc atcatgaaac tggacccgta catcaacgtg 60

gatccgggca ccatgagccc gattcagcat ggggaagttt tcgtcaccga agacggcgca 120

gaaaccgatc tggatttggg tcactacgag cgctacatcc gcaccaaaat gtcgcgccgc 180

aacaacttca ccaccggtcg catttactcc gacgttctgc gtaaagaacg ccgtggcgac 240

tatctgggcg caaccgtaca ggtcatcccg cacatcacca acgcgatcaa agagcgcatc 300

ctcgaa 306

<210> 954
<211> 306
<212> DNA
<213> Serratia marcescens sbsp. sakuensis

<400> 954
attcttgagg cccgtggcct caacgttacc atcatgaaac tggacccgta catcaacgtg 60

gatccgggca ccatgagccc gattcagcat ggggaagttt tcgtcaccga agacggcgca 120

gaaaccgatc tggatttggg tcactacgag cgcttcatcc gcaccaaaat gtcgcgtcgc 180

aacaacttca ccaccggccg catctactcc gacgtgctgc gtaaagaacg ccgcggcgac 240

tatctgggcg cgaccgtgca ggtcattccg cacatcacca acgcgatcaa agagcgcatc 300

atcgaa 306

<210> 955
<211> 306
<212> DNA
<213> Serratia odorifera

<400> 955
attcttgaag cccgtggcct caacgttacc atcatgaaac tggacccgta catcaacgtg 60

gatccgggca ccatgagccc gatccagcat ggggaagttt tcgtcaccga agacggcgct 120

gaaaccgatc tggacttggg tcactacgag cgcttcatcc gcaccaaaat gacgcgccgc 180

aacaacttca ccactggccg tatctattcc gacgtactgc gtaaagagcg ccgtggcgat 240

tacctgggcg cgaccgtaca ggttatcccg cacatcacca acgctatcaa ggaacgcatc 300

atcgaa 306

<210> 956
<211> 306
<212> DNA
<213> Serratia plymuthica

<400> 956
attctcgaag cccgtggcct caacgttacc atcatgaaac tggacccgta catcaacgtg 60

gatccgggca ccatgagccc gattcagcat ggggaagttt tcgtcaccga agacggggca 120

gaaaccgatc tggatttggg tcactacgag cgcttcatcc gcaccaaaat gtcgcgccgc 180

aacaacttca ccacgggtcg catttattcc gacgttctgc gtaaagaacg ccgtggcgac 240

tacctgggcg ccaccgtaca ggttatcccg cacatcacca acgcgatcaa agaacgcatt 300

ctcgaa 306

<210> 957
<211> 306
<212> DNA
<213> Serratia proteamaculans sbsp. proteamaculans

<400> 957
attcttgaag cccgtggcct caacgttacc atcatgaaac tggacccgta catcaacgtg 60

gatccgggca ccatgagccc gattcagcat ggggaagttt tcgtcaccga agacggcgca 120

gaaaccgatc tggatttggg tcactacgag cgctacatcc gtaccaaaat gtcgcgccgc 180

aataacttca ccaccggccg tatctactcc gacgttctgc gtaaagaacg ccgtggcgac 240

tatctgggcg caaccgtaca ggttatcccg cacatcacca acgcgatcaa agagcgtatc 300

ctcgaa 306

<210> 958
<211> 306
<212> DNA
<213> Serratia rubidaea

<400> 958
attcttgaag cccgtggcct caacgttacc atcatgaaac tggacccgta catcaacgtg 60

gatccgggca ccatgagccc gattcagcat ggggaagttt tcgtcaccga agacggcgca 120

gaaaccgacc tggacttggg tcactacgag cgcttcatcc gcaccaaaat gacgcgccgc 180

aacaacttca ccaccggccg catctactcc gacgtgctgc gtaaagaacg ccgcggcgac 240

tatctgggcg cgaccgtaca ggttattcct cacatcacca acgcgatcaa agagcgtatc 300

atcgaa 306

<210> 959
<211> 306

<212>  DNA
<213>  Serratia ureilytica

<400>  959
attcttgaag cccgtggcct caacgttacc atcatgaaac tggacccgta catcaacgtg      60

gatccgggca ccatgagccc gattcagcat ggggaagttt tcgtcaccga agacggcgca     120

gaaaccgatc tggatttggg tcactacgag cgcttcatcc gcaccaaaat gtcgcgtcgc     180

aacaacttca ccaccggccg catctactcc gacgtgctgc gtaaagaacg ccgcggcgac     240

tatctgggcg cgaccgtgca ggtcattccg cacatcacca acgcgatcaa agaacgcatc     300

atcgaa                                                                306

<210>  960
<211>  306
<212>  DNA
<213>  Shigella boydii

<400>  960
attcttgaag cccgtggcct caatgtgacc atcatgaaac tggatccgta catcaacgtc      60

gatccaggta ctatgagccc aatccaacac ggggaagtgt tcgttactga agacggcgct     120

gaaaccgacc tggacctggg gcactacgag cgtttcattc gtaccaaaat gagccgccgc     180

aacaacttca ccacgggtcg tatctactct gacgttctgc gtaaagaacg ccgcggtgac     240

tacctcggcg caaccgtgca ggttattccg cacatcacta acgcaatcaa agagcgcgtg     300

ctggaa                                                                306

<210>  961
<211>  306
<212>  DNA
<213>  Shigella dysenteriae T

<400>  961
attcttgaag cccgtggcct caatgtgacc atcatgaaac tggatccgta catcaacgtc      60

gatccaggta ctatgagccc aatccaacac ggggaagtgt tcgttactga agacggcgct     120

gaaaccgacc tggacctggg gcactacgag cgtttcatcc gtaccaaaat gagccgccgc     180

aacaacttca ccacgggtcg tatctactct gacgttctgc gtaaagaacg ccgcggtgac     240

tacctcggcg caaccgtaca ggttattccg cacatcacaa acgctatcaa agagcgtgtg     300

ctggaa                                                                306

<210>  962
<211>  306
<212>  DNA
<213>  Shigella flexneri

<400>  962
attcttgaag cccgtggcct caatgtgacc atcatgaaac tggatcctta catcaacgtc      60

gatccaggta ctatgagccc aatccaacac ggggaagtgt tcgttactga agacggcgct     120

gaaaccgact tggacctggg gcactacgag cgtttcattc gtaccaaaat gagccgccgc     180

aacaacttca ccacgggtcg tatctactct gacgttctgc gtaaagaacg ccgcggtgac     240

tacctcggcg caaccgtgca ggttattccg cacatcacta acgcaatcaa agagcgcgtg 300

ctggaa 306

<210> 963
<211> 306
<212> DNA
<213> Shigella sonnei

<400> 963
attcttgaag cccgtggcct caatgtgacc atcatgaaac tggatccgta catcaacgtc 60

gatccaggta ctatgagccc aatccaacac ggggaagtgt tcgttactga agacggcgct 120

gaaaccgacc tggacctggg gcactacgag cgtttcattc gtaccaaaat gagccgccgc 180

aacaacttca ccacgggtcg tatctactct gacgttctgc gtaaagaacg ccgcggtgac 240

tacctcggcg caaccgtgca ggttattccg cacatcacta acgcaatcaa agagcgcgtg 300

ctggaa 306

<210> 964
<211> 306
<212> DNA
<213> Tatumella ptyseos T

<400> 964
attcttgaag ctcgtggtct gaaagtgacc attatgaagc tcgaccctta tattaacgtc 60

gatccaggca ccatgagccc gactcagcat ggtgaagtat ttgtcactga tgatggtgca 120

gaaacggatc ttgacttagg gcattacgag cgttttatcc gtacccgtat gtcacgccgt 180

aataacttta ctaccggacg tatttactct gaagttctgc gtaaagaacg tcgcggtgac 240

tacctggggg cgaccattca ggtgatcccg cacatcacta atgccatcaa agagcgcatt 300

attgaa 306

<210> 965
<211> 306
<212> DNA
<213> Xenorhabdus beddingii

<400> 965
atactcgaag cccgtggact caatgtcacc attatgaaac tggacccgta catcaacgtt 60

gatccaggca caatgagccc aacccaacac ggggaagttt tcgttaccga ggacggcgct 120

gaaactgatc tcgatttagg tcattacgaa cgtttcatcc gaaccaaaat gacacgccgc 180

aataatttca ccactggccg tgtctattct gaagtgttgc gcaaagaacg ccgtggcgat 240

tatttggggg caaccattca ggttatccct cacatcacca atgaaatcaa agaccgcatc 300

atccgt 306

<210> 966
<211> 306
<212> DNA
<213> Xenorhabdus budapestensis

<400>  966
atactcgaag cccgtggact caatgtcacc attatgaaac tggacccgta tatcaacgtt      60

gatccaggta caatgagccc aacccaacac ggggaagttt tcgttaccga agatggcgct     120

gaaactgatc tcgatttagg ccattacgaa cgtttcattc gaaccaaaat gacacgtcgc     180

aataatttca cgacaggtcg tgtttactcc gaagttttgc gtaaagaacg ccgtggcgat     240

tatctgggcg caaccattca ggttattcct cacatcacta atgaaatcaa agaccgtatc     300

atccgg                                                                306

<210>  967
<211>  306
<212>  DNA
<213>  Xenorhabdus ehlersii

<400>  967
atactcgaag cccgtggact caatgtcacc attatgaaac tggacccgta tatcaacgtt      60

gatccaggca caatgagccc aacccaacac ggggaagttt tcgttaccga agatggcgct     120

gaaactgatc ttgatttagg tcattacgaa cgtttcatcc gaaccaaaat gacacgccgc     180

aataatttca cgacgggtcg tgtctattca gaagtgttgc gcaaagaacg ccgtggcgat     240

tatttgggtg caaccattca ggttatcccc cacatcacca atgaaatcaa agaccgcatc     300

atccgt                                                                306

<210>  968
<211>  306
<212>  DNA
<213>  Xenorhabdus innexi

<400>  968
atactcgaag cccgtggact caatgtcacc attatgaaac tggacccgta tatcaacgtt      60

gatccgggta caatgagccc aacccaacat ggggaagttt tcgtaaccga agacggcgct     120

gaaactgatc tcgatttagg ccattacgaa cgtttcatcc gaaccaaaat gacacgtcgc     180

aataacttca cgacgggtcg tgtttactcc gaagttctgc gcaaagaacg ccgtggcgat     240

tatctgggcg caaccattca ggttattcct cacatcacta acgaaatcaa agaccgtatc     300

atccgt                                                                306

<210>  969
<211>  306
<212>  DNA
<213>  Xenorhabdus japonica

<400>  969
atactcgaag cccgtggact caatgtcacc attatgaaac tggacccgta catcaacgtc      60

gatccaggca caatgagccc aacccaacac ggggaagttt tcgtcaccga agacggcgct     120

gaaactgatc tcgatttagg tcattacgaa cgtttcatcc gaaccaaaat gacacgccgc     180

aataacttta cgactggccg tgtctattcc gaagtcctgc gcaaagaacg ccgcggcgat     240

tatctgggcg caaccattca ggttatcccc catatcacca acgaaatcaa agaccgtatc     300

atccgt                                                              306

<210>  970
<211>  306
<212>  DNA
<213>  Xenorhabdus poinarii

<400>  970
atactcgaag cccgtggact caatgttacc attatgaaac tggacccgta tattaacgtc      60

gatccaggca caatgagccc aacccaacac ggggaagttt tcgttaccga agacggcgct     120

gaaactgatc tcgatttagg tcattacgaa cgtttcatcc gaaccaaaat gacacgtcgc     180

aataatttca cgacaggtcg tgtctattct gaagtcctgc gcaaagaacg ccgtggcgat     240

tatttaggcg ccaccattca ggtcattcct catatcacca atgaaatcaa agatcgtatt     300

atccgt                                                              306

<210>  971
<211>  306
<212>  DNA
<213>  Xenorhabdus szentirmaii

<400>  971
atactcgaag cccgtggact caatgtcacc attatgaaac tggacccgta tatcaacgtt      60

gatccaggta caatgagccc aacccaacac ggggaagttt tcgtgaccga agacggcgct     120

gaaactgatc ttgatttagg tcattacgaa cgtttcatcc gaaccaaaat gacacgccgc     180

aataacttca cgaccggccg tgtctactcc gaagtgctgc gcaaagaacg ccgtggcgat     240

tatttgggtg caaccattca ggttatccct cacatcacca atgaaatcaa agaccgcatt     300

attcgt                                                              306

<210>  972
<211>  306
<212>  DNA
<213>  Yersinia aldovae

<400>  972
atacttgaag cccgtggcct caacgttacc atcatgaaac tggacccgta tatcaacgtg      60

gatccgggca ccatgagccc gacacaacac ggggaagttt tcgtcaccga agatggtgct     120

gaaaccgatc tggatttagg gcactacgag cgcttcattc gcaccaaaat gacccgccgt     180

aataacttca ccaccggccg tatctactcg gaagttctgc gtaaagagcg ccgtggcgac     240

tacttgggtg cgaccattca ggttattcct cacatcacca atgccattaa agaacgtatc     300

atcgaa                                                              306

<210>  973
<211>  306
<212>  DNA
<213>  Yersinia bercovieri

<400>  973
atacttgaag cccgtggcct caacgttacc atcatgaaac tggacccgta tatcaacgtg      60

gatccgggca ccatgagccc gacacaacac ggggaagttt ttgtcaccga agatggtgct 120

gagaccgatc tggacttagg gcactatgag cgctttattc gcaccaaaat gacccgtcgt 180

aataacttca ctacggggcg catctattct gaagtcctgc gtaaagagcg tcgtggcgac 240

tatctgggtg caactattca ggttattcct catatcacca atgccatcaa agagcgcatc 300

atcgaa 306

<210> 974
<211> 306
<212> DNA
<213> Yersinia enterocolitica sbsp. enterocolitica

<400> 974
atacttgaag cccgtggcct caacgttacc atcatgaaac tggacccgta tatcaacgtg 60

gatccgggca ccatgagccc gacacaacac ggggaagttt ttgtcaccga agatggtgct 120

gaaaccgatc tggatttagg gcattacgag cgcttcattc gcactaaaat gacccgccgt 180

aacaacttca ctactggccg tatctactct gaagttctgc gcaaagagcg ccgcggcgac 240

tatctgggtg cgactatcca ggttattcct catatcacca atgcaatcaa agaacgtatc 300

atcgaa 306

<210> 975
<211> 306
<212> DNA
<213> Yersinia frederiksenii

<400> 975
atacttgaag cccgtggcct caacgttacc atcatgaaac tggacccgta catcaacgtg 60

gatccgggca ccatgagccc gacacaacac ggggaagttt tcgtcaccga agatggtgct 120

gaaaccgatc tggatttagg gcattacgag cgcttcattc gcaccaaaat gacccgtcgt 180

aacaacttca ccactggccg tatctattct gaagttctgc gtaaagaacg ccgtggcgac 240

tatctgggtg cgaccattca ggttattcct catatcacca atgccatcaa agaacgcatc 300

atcgaa 306

<210> 976
<211> 306
<212> DNA
<213> Yersinia intermedia

<400> 976
atacttgaag cccgtggcct caaggttact atgatgaaac tggacccata tattaacatg 60

gatccaggca ccatgagccc gactcaacac ggggaagttt atgtcaccga tgatggtgct 120

gaaaccgatc tggatttagg tcactatgag cgcttcattc gcactaaaat gacccgtcgc 180

aacaacttca ccgctggtcg tatctattcc gatgttctac gcaaagaacg ccgtggcgac 240

tacttaggtg caaccattca ggttattcct catatcacca atgctatcaa agagcgcatc 300

gttgaa 306

<210> 977
<211> 306
<212> DNA
<213> Yersinia kristensenii

<400> 977
atacttgaag cccgtggcct caacgttacc atcatgaaac tggacccgta tatcaacgtg 60

gatccgggca ccatgagccc gacacaacac ggggaagttt ttgtcaccga agatggtgct 120

gaaaccgatc tggatttagg gcattacgag cgtttcattc gcactaaaat gactcgccgt 180

aacaacttca ccactggccg tatttactct gaagttctgc gcaaagaacg tcgcggcgac 240

tatctgggtg caactattca ggttattcct catatcacca atgctatcaa agaacgcatc 300

atcgaa 306

<210> 978
<211> 306
<212> DNA
<213> Yersinia mollaretii

<400> 978
atacttgaag cccgtggcct caacgttacc atcatgaaac tggacccgta tatcaacgtg 60

gatccgggca ccatgagccc gactcaacac ggggaagttt ttgtcaccga agatggtgct 120

gagaccgatc tggacttagg gcactatgag cgcttcattc gcaccaaaat gacccgtcgc 180

aacaacttca ctacgggccg catctattcc gaagtcctgc gtaaagagcg tcgtggcgac 240

tatctgggtg caactattca ggttattcct catatcacca atgctatcaa agagcgcatc 300

atcgaa 306

<210> 979
<211> 306
<212> DNA
<213> Yersinia pestis T

<400> 979
atacttgaag cccgtggcct caacgttacc atcatgaaac tggacccgta tatcaacgtg 60

gatccgggca ccatgagccc gacacaacac ggggaggttt ttgtcaccga agatggtgct 120

gagaccgatc tggatttagg gcactatgag cgcttcattc gcaccaaaat gacccgtcgt 180

aacaacttca ccactggccg tatctattcc gaagttttgc gtaaagaacg ccgtggcgac 240

tatttgggtg cgactattca ggttattccc cacatcacca atgcaatcaa agagcgcatc 300

attgaa 306

<210> 980
<211> 306
<212> DNA
<213> Yersinia pseudotuberculosis sbsp. pseudotuberculosis

<400> 980
atacttgaag cccgtggcct caacgttacc atcatgaaac tggacccgta tatcaacgtg 60

gatccgggca ccatgagccc gacacaacac ggggaagttt ttgtcaccga agatggtgct 120

gagaccgatc tggatttagg gcactatgag cgcttcattc gcaccaaaat gacccgtcgt 180

aacaacttca ccactggccg tatctattcc gaagttctgc gtaaagaacg ccgtggcgac 240

tatttgggtg cgactattca ggttattccc cacatcacca atgcaatcaa agagcgcatc 300

attgaa 306

<210> 981
<211> 306
<212> DNA
<213> Yersinia rohdei

<400> 981
attcttgaag cccgtggcct caatgttacc atcatgaaac tggacccgta catcaacgtg 60

gatccgggca ccatgagccc gacacaacac ggggaagttt tcgtcaccga agatggtgct 120

gaaaccgatc tggatttagg gcattacgag cgcttcattc gcaccaaaat gacccgtcgt 180

aacaacttca ccactggccg tatctattct gaagttctgc gaaaagagcg ccgtggcgac 240

tacctgggcg caactattca ggttattcct catatcacca atgcaatcaa agaacgcatt 300

atcgaa 306

<210> 982
<211> 306
<212> DNA
<213> Yersinia ruckeri

<400> 982
attcttgaag cccgtggcct caaagttacc atcatgaaac tggacccgta tatcaacgtg 60

gatccgggca ccatgagccc gacacaacac ggagaagttt tcgtcaccga agatggtgct 120

gagaccgatc tggatttagg gcactatgag cgcttcattc gcaccaaaat gactcgtcgc 180

aacaatttca ctacgggtcg tatctactct gaagttctgc gtaaagaacg ccgtggtgac 240

tatctgggtg cgactatcca ggttattcct catattacca atgctatcaa agaacgtatc 300

attgaa 306

<210> 983
<211> 306
<212> DNA
<213> Yokenella regensburgei T

<400> 983
attcttgaag cccgtggcct caatgtgacc atcatgaagc tggatccgta catcaacgtc 60

gatccgggta ccatgagccc aatccaacac ggggaagtgt tcgttactga agacggcgct 120

gaaaccgatc tggacctggg tcactatgag cgtttcattc gcaccaagat gacacgccgc 180

aacaacttca ccaccggccg tatctactca gacgttctgc gcaaagagcg ccgtggcgac 240

tatctgggcg cgaccgtaca ggttattccg catatcacta acgctatcaa agagcgcatc 300

atcgca 306